# EUROPEAN PATENT APPLICATION

(11) **EP 3 960 736 A1**
(43) Date of publication of application: **02.03.2022**
(21) Application number: 20794013.1
(22) Date of filing: 26.04.2020
(51) Int. Cl.: C07D 403/02, C07D 413/02, A61K 31/445, A61K 31/454, A61P 37/02, A61P 29/00, A61P 19/02

(54) **HETEROCYCLIC COMPOUND, APPLICATION THEREOF, AND COMPOSITION CONTAINING SAME**

(30) Priority: 26.04.2019 CN 201910342107; 14.06.2019 US 201962861857 P
(71) Applicant: Generos Biopharma Ltd., Hangzhou, Zhejiang 310018 (CN)
(72) Inventor: FU, Xin-Yuan, Hangzhou, Zhejiang 310018 (CN); LU, Cenbin, Hangzhou, Zhejiang 310018 (CN); LIU, Xinyu, Hangzhou, Zhejiang 310018 (CN); LUFEI, Chengchen, Hangzhou, Zhejiang 310018 (CN); ZHOU, Yi, Hangzhou, Zhejiang 310018 (CN)
(74) Representative: Williams, Gareth Owen
(86) International application number: PCT/CN2020/087008
(87) International publication number: WO 2020/216378

(57) **Abstract**

A heterocyclic compound represented by formula XI, a pharmaceutically acceptable salt, a solvate, or a solvate of a pharmaceutically acceptable salt thereof, use thereof, and a composition containing the same. The compound is novel in structure and has good STAT5 inhibitory activity.

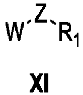

## Description

The present application claims the priority of Chinese patent application 201910342107.7 with the filing date of 2019/4/26. The present application claims the priority of US patent application 62/861,857 with the filing date of 2019/6/14. Each of the above-mentioned patent applications is incorporated in the present application by reference in its entirety.

### TECHNICAL FIELD

The present invention relates to a heterocyclic compound, use thereof and a composition containing the same.

### BACKGROUND

Rheumatoid arthritis is a chronic systemic autoimmune disease characterized by inflammation of the synovial membrane of the joints. Its incidence accounts for about 1% of all adults. The main manifestation is symmetrical multiple recurrent arthritis, and the small joints of the hands and feet are most easily affected. In the early or acute onset, the joints are mostly red, swollen, hot, painful and impaired; in the later stage, it can lead to joint destruction, stiffness, deformity and loss of function, as well as osteoporosis and skeletal muscle atrophy. Throughout the course of the disease, it can be accompanied by fever, anemia, weight loss, vasculitis and subcutaneous nodules, and can also involve multiple organs throughout the body. It is a disease with a high disability rate and significantly increases the mortality of patients. The pathogenesis of rheumatoid arthritis is not fully understood in modern medicine, and there is currently no cure for it, which causes a heavy health and financial burden on patients worldwide.

In the past two decades, people have gained a deeper understanding of the pathogenesis of arthritis. It is believed that the excessive activation of immune cells and the increase of inflammatory cytokines lead to diseases in joints and other parts, and some great progress has been made in their treatment. A series of anti-rheumatic factor drugs have been deleloped and produced, including methotrexate, tumor necrosis factor (TNFα) antibody, interleukin (IL) 1, interleukin-6 antibody and JAK Janus kinase inhibitor.

These anti-rheumatic drugs can control disease progression to a certain extent and improve patients' living conditions. However, its sustained effect is not ideal, and it is only effective for a small number of patients, accompanied by side effects of varying severity. For example, as an anti-cancer drug, methotrexate itself has significant non-specific cytotoxicity and is very harmful to the gastrointestinal tract, liver and kidney organs. Due to insufficient drug efficacy or too strong adverse reactions, approximately half of patients will stop taking the drug within one year of starting treatment. Anti-TNFa antibodies are only effective against 30-40% of patients, and are accompanied by random infections and cancer risks. There are reports that it increases the possibility of developing multiple sclerosis, and neutralizing antibodies are produced in the body after long-term use, resulting in loss of efficacy [Ann Rheum Dis. 2014 Mar;73(3):529-35]. We urgently need to find new disease pathogenesis and develop targeted and better products.

The pathogenesis of rheumatoid arthritis exhibits a severely dysregulated innate and adaptive immune response, which leads to chronic inflammation. Helper T cells play a vital role in the initiation and development of arthritis. When its function is dysregulated, the continuous activation and abnormal proliferation of T lymphocytes lead to the corresponding proliferation of fibroblasts in joint tissues, resulting in the appearance of arthritis. JAK-STAT pathway has important functions in regulating T cells and other immune cells. Among them, STAT5 factor plays a key role in the development and maturation of many T cell subtypes.

Recently, our team discovered a new helper T cell and named it as Th-GM. Such cells play a role in promoting many inflammatory diseases and represent a new mechanism for the pathogenesis of inflammation. Th-GM cells are characterized by having the STAT5 signaling pathway activated by interleukin 7 and leading to high expression of downstream colony stimulating factor (GM-CSF) and interleukin 3. At the same time, RNA sequencing experiments found that Th-GM cells exhibit a unique gene expression profile, which is very different from the established two types of helper T cells, Th-1 and Th-17. Genetic experiments have also proved that by knocking out the STAT5 gene, it can significantly reduce the severity of the nervous system inflammation and arthritis in mice with multiple sclerosis (reference CN107002037A, PCT/US2018/031217). Due to the key regulatory effect of STAT5 on Th-GM cells and its role in other inflammatory cells, we believe that STAT5 inhibitors will be very promising candidates for the treatment of arthritis.

In this patent, we clarified the design and synthesis of a series of new STAT5 inhibitors and the validation evidence for the treatment of rheumatoid arthritis.

### SUMMARY

A technical problem to be solved by the present invention is the defect that the existing STAT5 inhibitors have a relatively similar structure. For this purpose, the present invention provides a compound containing a heterocyclic ring, use thereof, and a composition containing the same. The compound is novel in structure and has good STAT5 inhibitory activity.

The present invention provides a compound represented by formula **XI,** 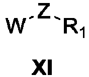 or a pharmaceutically acceptable salt, a solvate, or a solvate of a pharmaceutically acceptable salt thereof;
wherein, W is anyone of the following groups:
(1) 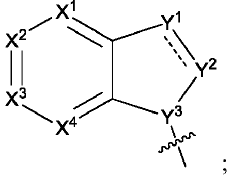
   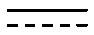 in 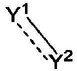 is a single bond or a double bond;
   when 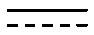 in 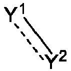 is a single bond, Y¹ is C(=O), O, S or N-R³, R³ is hydrogen, hydroxyl, CH₂COOH or C₁-C₄ alkyl, Y² is C(=N-CO-Ph), C(=N-CO-CH₃), NH, S=O, S(=O)₂, C=O or C=S;
   when 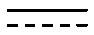 in 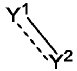 is a double bond, Y¹ is N, Y² is C-NH₂, C-NH-CN or C-O-CH₃;
   Y³ is N or CH;
   X¹ is N or CR², X² is N or CR⁴, X³ is N or CR⁵, X⁴ is N or CR⁶;
   R², R⁴, R⁵ and R⁶ are independently hydrogen, halogen, hydroxyl, amino, carboxyl, cyano, nitro, C₁-C₄ alkyl, C₁-C₄ alkyl substituted with one or more halogens, C₂-C₄ alkenyl, C₂-C₄ alkynyl, C₁-C₄ alkoxy, C₃-C₆ cycloalkyl, phenyl, 5-7 membered heterocycloalkyl containing 1, 2, or 3 atoms independently selected from N, O and S, -S(=O)₂-R²⁻¹, -NH-S(=O)₂-R²⁻², -S(=O)₂-NR²⁻³R²⁻⁴, -C(=O)-R²⁻⁵, -NH-C(=O)-R²⁻⁶, -C(=O)-NR²⁻⁷R²⁻⁸, -O-(C=O)-R²⁻⁹, or -C(=O)-O-R²⁻¹⁰;
   R²⁻¹, R²⁻² , R²⁻³, R²⁻⁴, R²⁻⁵, R²⁻⁶, R²⁻⁷, R²⁻⁸, R²⁻⁹ and R²⁻¹⁰ are independently hydrogen, C₁-C₄ alkyl, C₂-C₄ alkenyl, C₂-C₄ alkynyl, or C₁-C₄ alkyl substituted with one or more halogens;
(2) 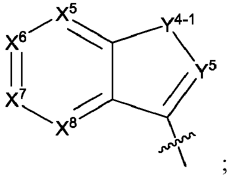
   Y⁴⁻¹ is -NH-C(=O)-CH₂- or NH;
   Y⁵ is N or C-R⁷, R⁷ is halogen;
   X⁵ is N or CR⁸, X⁶ is N or CR⁹, X⁷ is N or CR¹⁰, X⁸ is N or CR¹¹;
   R⁸, R⁹, R¹⁰ and R¹¹ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(3) 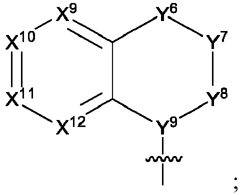
   Y⁶ is N-C(=O)-CH₃, CH₂ or NH;
   Y⁷ is NH, CH₂, C(=O), C(=S) or C(=N-OH);
   Y⁸ is CH₂, CH₂CH₂, CH(CH₃) or C(=O);
   Y⁹ is N or CH;
   X⁹ is N or CR¹², X¹⁰ is N or CR¹³, X¹¹ is N or CR¹⁴, X¹² is N or CR¹⁵;
   R¹², R¹³, R¹⁴ and R¹⁵ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(4) 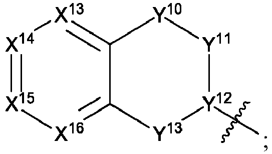
   Y¹⁰ is NH;
   Y¹¹ is C(=O);
   Y¹² is N or CH;
   Y¹³ is CH₂;
   X¹³ is N or CR¹⁶, X¹⁴ is N or CR¹⁷, X¹⁵ is N or CR¹⁸, X¹⁶ is N or CR¹⁹;
   R¹⁶, R¹⁷, R¹⁸ and R¹⁹ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(5) 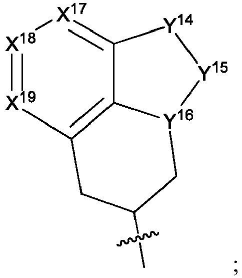
   Y¹⁴ is NH;
   Y¹⁵ is C(=O);
   Y¹⁶ is N or CH;
   X¹⁷ is N or CR²⁰, X¹⁸ is N or CR²¹, X¹⁹ is N or CR²²;
   R²⁰, R²¹ and R²² are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(6) 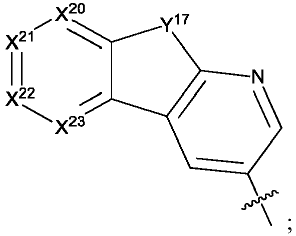
   Y¹⁷ is NH;
   X²⁰ is N or CR²³, X²¹ is N or CR²⁴, X²² is N or CR²⁵, X²³ is N or CR²⁶;
   R²³, R²⁴, R²⁵ and R²⁶ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(7) 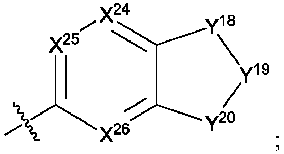
   Y¹⁸ is NH or CH2;
   Y¹⁹ is C(=O) or NH;
   Y²⁰ is CH₂ or C(=O);
   X²⁴ is N or CR²⁷, X²⁵ is N or CR²⁸, X²⁶ is N or CR²⁹;
   R²⁷, R²⁸ and R²⁹ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(8) 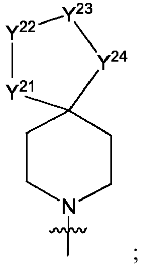
   Y²¹ is C(=O);
   Y²² is NH;
   Y²³ is CH₂ or C(=O);
   Y²⁴ is N-R³⁰, R³⁰ is hydrogen or phenyl;
(9) 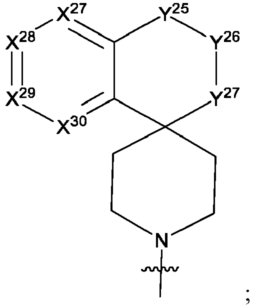
   Y²⁵ is NH;
   Y²⁶ is C(=O);
   Y²⁷ is a single bond, O or NH;
   X²⁷ is N or CR³¹, X²⁸ is N or CR³², X²⁹ is N or CR³³, X³⁰ is N or CR³⁴;
   R³¹, R³², R³³ and R³⁴ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(10) 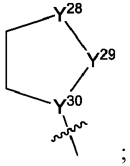
   Y²⁸ is NH;
   Y²⁹ is C(=O);
   Y³⁰ is CH or N;
(11) 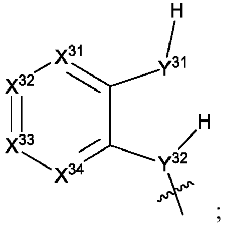
   Y³¹ is O, NH or N-C(=O)-CH₃;
   Y³² is CH or N;
   X³¹ is N or CR³⁵, X³² is N or CR³⁶, X³³ is N or CR³⁷, X³⁴ is N or CR³⁸;
   R³⁵, R³⁶, R³⁷ and R³⁸ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(12) 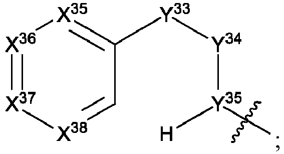
   Y³³ is NH;
   Y³⁴ is C(=O);
   Y³⁵ is CH or N;
   X³⁵ is N or CR³⁹, X³⁶ is N or CR⁴⁰, X³⁷ is N or CR⁴¹, X³⁸ is N or CR⁴²;
   R³⁹, R⁴⁰, R⁴¹ and R⁴² are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(13) 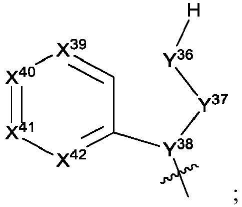
   Y³⁶ is NH;
   Y³⁷ is C(=O);
   Y³⁸ is CH or N;
   X³⁹ is N or CR⁴³, X⁴⁰ is N or CR⁴⁴, X⁴¹ is N or CR⁴⁵, X⁴² is N or CR⁴⁶;
   R⁴³, R⁴⁴, R⁴⁵ and R⁴⁶ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(14) 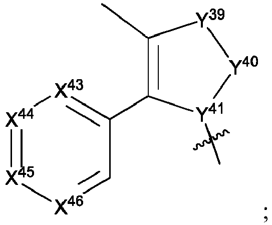
   Y³⁹ is NH;
   Y⁴⁰ is C(=O);
   Y⁴¹ is CH or N;
   X⁴³ is N or CR⁴⁷, X⁴⁴ is N or CR⁴⁸, X⁴⁵ is N or CR⁴⁹, X⁴⁶ is N or CR⁵⁰;
   R⁴⁷, R⁴⁸, R⁴⁹ and R⁵⁰ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂; and
(15)

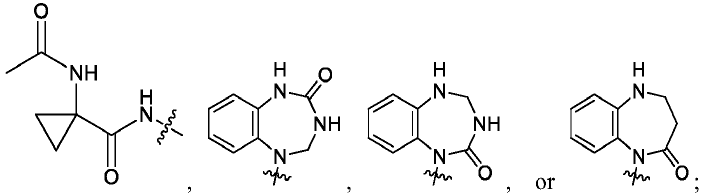

Z is anyone of the following fragments:
(1) 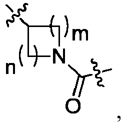 m is 1 or 2, n is 1, 2 or 3;
(2) 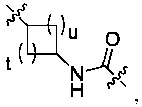 u is 1 or 2, t is 1 or 2;
(3) 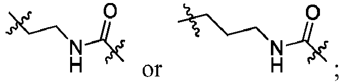
(4) 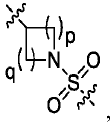 p is 1 or 2, q is 1 or 2;
(5) 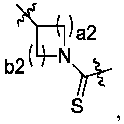 a2 is 1 or 2, b2 is 1 or 2;
(6) 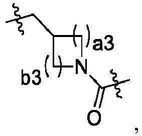 a3 is 1 or 2, b3 is 1 or 2;
(7) 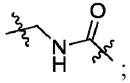
(8) -C(=O)-;
(9) 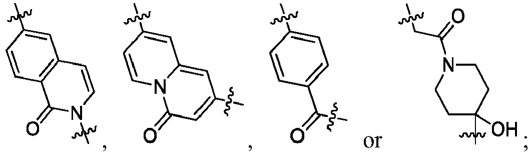
   R¹ is C₂-C₄ alkyl, C₂-C₄ alkyl substituted with one or more halogens, -(CH₂)ₛ-R¹⁻¹, -NH-R¹⁻², 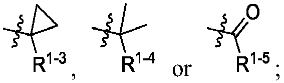 s is 0, 1 or 2;
   R¹⁻¹, R¹⁻², R¹⁻³, R¹⁻⁴ and R¹⁻⁵ are independently C₇-C₁₀ cycloalkyl, one or more R¹⁻¹⁻³ substituted C₃-C₆ cycloalkyl, one or more R¹⁻¹⁻⁶ substituted 5-6 membered heterocycloalkyl containing 1, 2, or 3 atoms independently selected from N, O and S, one or more R¹⁻¹⁻¹ substituted C₆-C₁₀ aryl, C₃-C₆ cycloalkyl, cyclopentadienyl, one or more R¹⁻¹⁻⁴ substituted cyclopentadienyl, naphthyl, 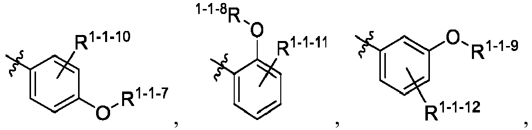 5, 10 membered heteroaryl containing 1, 2, or 3 atoms independently selected from N, O and S, pyridyl, 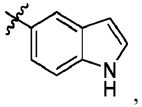 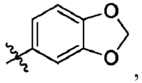 or one or more R¹⁻¹⁻² substituted 5-10 membered heteroaryl containing 1, 2, or 3 atoms independently selected from N, O and S;
   R¹⁻¹⁻¹, R¹⁻¹⁻², R¹⁻¹⁻³, R¹⁻¹⁻⁴, and R¹⁻¹⁻⁶ are independently carboxy, hydroxy, cyano, halogen, C₁-C₄ alkyl, C₁-C₄ alkyl substituted with one or more halogens, C₁-C₄ alkoxy substituted with one or more halogens, C₃-C₆ cycloalkyl, 5-6 membered heterocycloalkyl containing 1, 2, or 3 atoms independently selected from N, O and S, -N(R¹⁻¹⁻¹⁻¹)(R¹⁻¹⁻¹⁻²), or phenyl;
   R¹⁻¹⁻¹⁻¹ and R¹⁻¹⁻¹⁻² are independently hydrogen or C₁-C₄ alkyl;
   R¹⁻¹⁻⁷, R¹⁻¹⁻⁸, and R¹⁻¹⁻⁹ are independently C₁-C₄ alkyl;
   R¹⁻¹⁻¹⁰ is independently C₁-C₄ alkoxy;
   R¹⁻¹⁻¹¹ and R¹⁻¹⁻¹² are independently hydrogen or halogen.

In a certain embodiment, in the compound represented by formula **XI,** or a pharmaceutically acceptable salt, a solvate, or a solvate of a pharmaceutically acceptable salt thereof, the compound represented by formula **XI** is a compound represented by formula **I**; 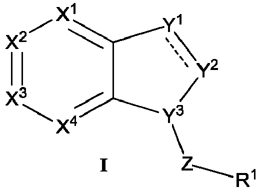
wherein, 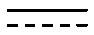 in 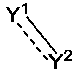 is a single bond or a double bond;
when 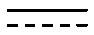 in 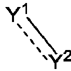 is a single bond, Y¹ is O, S or N-R³, R³ is hydrogen, hydroxyl, CH₂COOH or C₁-C₄ alkyl, Y² is C=O, C=S, S=O or S(=O)₂;
when 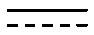 in 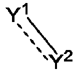 is a double bond, Y¹ is N, Y² is C-NH₂, C-NH-CN or C-O-CH₃;
Y³ is N or CH;
X¹ is N or CR², X² is N or CR⁴, X³ is N or CR⁵, X⁴ is N or CR⁶;
R², R⁴, R⁵ and R⁶ are independently hydrogen, halogen, hydroxyl, amino, carboxyl, cyano, nitro, C₁-C₄ alkyl, C₁-C₄ alkyl substituted with one or more halogens, C₂-C₄ alkenyl, C₂-C₄ alkynyl, C₁-C₄ alkoxy, C₃-C₆ cycloalkyl, phenyl, -S(=O)₂-R²⁻¹, -NH-S(=O)₂-R²⁻², -S(=O)₂-NR²⁻³R²⁻⁴, -C(=O)-R²⁻⁵, -NH-C(=O)-R²⁻⁶, -C(=O)-NR²⁻⁷R²⁻⁸, -O-(C=O)-R²⁻⁹, or -C(=O)-O-R²⁻¹⁰;
R²⁻¹, R²⁻² , R²⁻³, R²⁻⁴, R²⁻⁵, R²⁻⁶, R²⁻⁷, R²⁻⁸, R²⁻⁹ and R²⁻¹⁰ are independently C₁-C₄ alkyl, C₁-C₄ alkyl substituted with one or more halogens, C₂-C₄ alkenyl, or C₂-C₄ alkynyl;
Z is 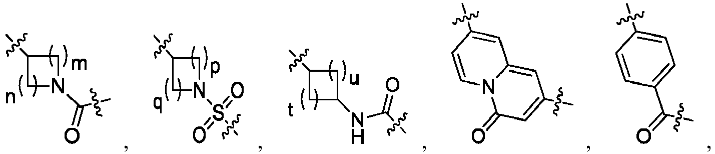 or 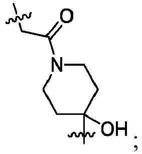 m is 1 or 2, n is 1 or 2; p is 1 or 2, q is 1 or 2; u is 1 or 2, t is 1 or 2;
R¹ is C₂-C₄ alkyl, C₂-C₄ alkyl substituted with one or more halogens, -(CH₂)ₛ-R¹⁻¹, or - NH-R¹⁻²; s is 0, 1 or 2;
R¹⁻¹ and R¹⁻² are independently C₆-C₁₀ aryl, one or more R¹⁻¹⁻¹ substituted C₆-C₁₀ aryl, 5-10 membered heteroaryl containing 1, 2, or 3 atoms independently selected from N, O and S, one or more R¹⁻¹⁻² substituted 5-10 membered heteroaryl containing 1, 2, or 3 atoms independently selected from N, O and S, C₃-C₆ cycloalkyl, one or more R¹⁻¹⁻³ substituted C₃-C₆ cycloalkyl, cyclopentadienyl, or one or more R¹⁻¹⁻⁴ substituted cyclopentadienyl;
R¹⁻¹⁻¹, R¹⁻¹⁻², R¹⁻¹⁻¹ and R¹⁻¹⁻⁴ are independently hydroxyl, cyano, halogen, carboxyl, C₁ C₄ alkyl, C₁-C₄ alkyl substituted with one or more halogens, C₁-C₄ alkoxy, C₃-C₆ cycloalkyl, 5-6 membered heterocycloalkyl containing 1, 2, or 3 atoms independently selected from N, O and S, or phenyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R³ is C₁-C₄ alkyl, said C₁-C₄ alkyl is methyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R², R⁴, R⁵ and R⁶ are independently C₁-C₄ alkyl, said C₁-C₄ alkyl is tert-butyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R², R⁴, R⁵ and R⁶ are independently C₁-C₄ alkyl substituted with a plurality of halogens, said plurality of halogens are 2 or 3 halogens.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R², R⁴, R⁵ and R⁶ are independently C₁-C₄ alkyl substituted with one or more halogens, said halogen is fluorine.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R², R⁴, R⁵ and R⁶ are independently C₁-C₄ alkyl substituted with one or more halogens, said C₁-C₄ alkyl is methyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R², R⁴, R⁵ and R⁶ are independently C₁-C₄ alkyl substituted with a plurality of halogens, said C₁-C₄ alkyl substituted with a plurality of halogens is trifluoromethyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R², R⁴, R⁵ and R⁶ are independently C₁-C₄ alkoxy, said C₁-C₄ alkoxy is methoxy.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R², R⁴, R⁵ and R⁶ are independently C₃-C₆ cycloalkyl, said C₃-C₆ cycloalkyl is cyclopropyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R²⁻¹, R²⁻², R²⁻³, R²⁻⁴, R²⁻⁵, R²⁻⁶, R²⁻⁷, R²⁻⁸, R²⁻⁹ and R²⁻¹⁰ are independently C₁-C₄ alkyl, said C₁-C₄ alkyl is methyl or tert-butyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R²⁻¹, R²⁻², R²⁻³, R²⁻⁴, R²⁻⁵, R²⁻⁶, R²⁻⁷, R²⁻⁸, R²⁻⁹ and R²⁻¹⁰ are independently C₁-C₄ alkyl substituted with a plurality of halogens, said plurality of halogens are 2 or 3 halogens.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R²⁻¹, R²⁻², R²⁻³, R²⁻⁴, R²⁻⁵, R²⁻⁶, R²⁻⁷, R²⁻⁸, R²⁻⁹ and R²⁻¹⁰ are independently C₁-C₄ alkyl substituted with one or more halogens, said halogen is fluorine.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R²⁻¹, R²⁻², R²⁻³, R²⁻⁴, R²⁻⁵, R²⁻⁶, R²⁻⁷, R²⁻⁸, R²⁻⁹ and R²⁻¹⁰ are independently C₁-C₄ alkyl substituted with one or more halogens, said C₁-C₄ alkyl is methyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R²⁻¹, R²⁻², R²⁻³, R²⁻⁴, R²⁻⁵, R²⁻⁶, R²⁻⁷, R²⁻⁸, R²⁻⁹ and R²⁻¹⁰ are independently C₁-C₄ alkyl substituted with a plurality of halogens, said C₁-C₄ alkyl substituted with a plurality of halogens is trifluoromethyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when Z is 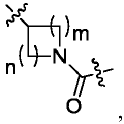 m is 1, n is 1.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when Z is 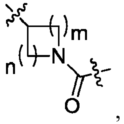 m is 2, n is 2.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when Z is 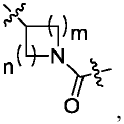 the 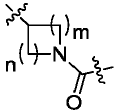 is 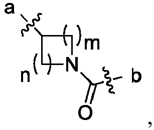 whose b end is connected with R¹.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when Z is 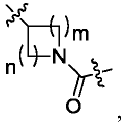 m is 1, n is 1, the 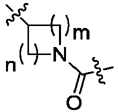 is 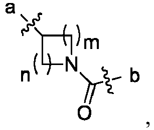 whose b end is connected with R¹.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when Z is 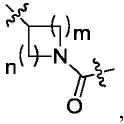 m is 2, n is 2, the 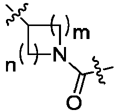 is 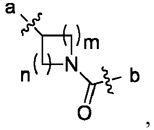 whose b end is connected with R¹.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when Z is 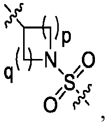 the 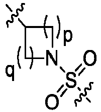 is 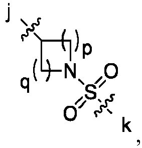 whose k end is connected with R¹.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when Z is 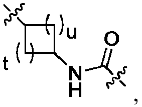 u is 1, t is 1.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when Z is 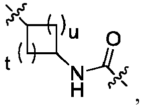 u is 2, t is 2.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when Z is 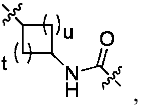 the 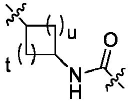 is 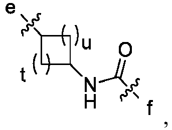 whose f end is connected with R¹;
or alternatively it can be 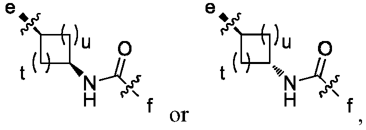 whose f end is connected with R¹.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when Z is 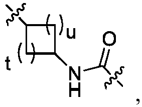 the 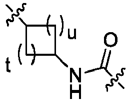 is 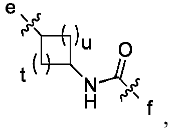 whose f end is connected with R¹, and u is 1, t is 1;
or alternatively it can be 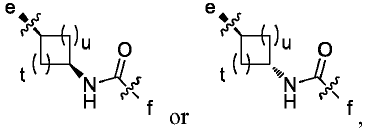 whose f end is connected with R¹, and u is 1, t is 1.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when Z is 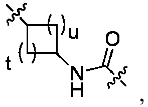 the 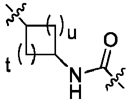 is 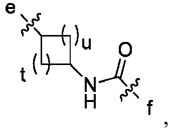 whose f end is connected with R¹, and u is 2, t is 2;
or alternatively it can be 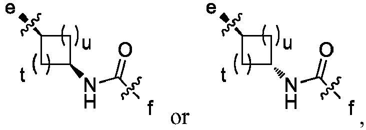 whose f end is connected with R¹, and u is 2, t is 2.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when Z is 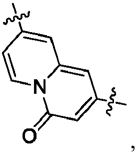 the 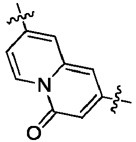 is 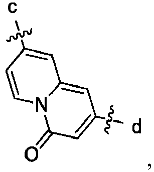 whose d end is connected with R¹.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when Z is 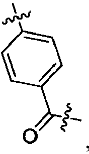 the 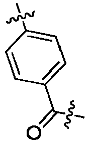 is 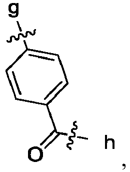 whose h end is connected with R¹.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when Z is 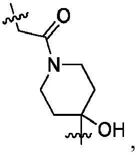 the 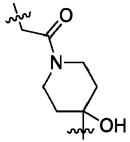 is 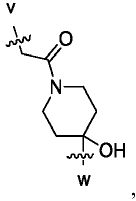 whose w end is connected with R¹.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹ is a C₂-C₄ alkyl, said C₂-C₄ alkyl is ethyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹ is a C₂-C₄ alkyl substituted with a plurality of halogens, said plurality of halogens are 2 or 3 halogens.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹ is a C₂-C₄ alkyl substituted with one or more halogens, said halogen is fluorine.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹ is a C₂-C₄ alkyl substituted with one or more halogens, the C₂-C₄ alkyl is ethyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹ is C₂-C₄ alkyl substituted with a plurality of halogens, said C₂-C₄ alkyl substituted with a plurality of halogens is 2,2,2-trifluoroethyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently C₆-C₁₀ aryl, said C₆-C₁₀ aryl is phenyl or naphthyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently one or more R¹⁻¹⁻¹ substituted C₆-C₁₀ aryl, said C₆-C₁₀ aryl is phenyl or naphthyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently one or more R¹⁻¹⁻¹ substituted C₆-C₁₀ aryl, and said C₆-C₁₀ aryl is phenyl, said R¹⁻¹⁻¹ and said -(CH₂)ₛ- or -NH- can be located in a ortho, meta or para position relative to each other, or alternatilvely can be in a para position relative to each other.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently 5-10 membered heteroaryl containing 1, 2, or 3 atoms independently selected from N, O and S, said 5-10 membered heteroaryl containing 1, 2, or 3 atoms independently selected from N, O and S can be 5-6 membered heteroaryl, indolyl, isoquinolyl or quinolyl containing one atom selected from N, O and S, or alternatilvely can be a 5-6 membered heteroaryl containing one atom selected from N, O and S.

The 5-6 membered heteroaryl containing one atom selected from N, O and S can be for example furyl, thienyl or pyridyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently one or more R¹⁻¹⁻² substituted 5-10 membered heteroaryl containing 1, 2, or 3 atoms independently selected from N, O and S, said 5-10 membered heteroaryl containing 1, 2, or 3 atoms independently selected from N, O and S can be 5-6 membered heteroaryl, indolyl, isoquinolyl or quinolyl containing one atom selected from N, O and S, or alternatively can be a 5-6 membered heteroaryl containing one atom selected from N, O and S.

The 5-6 membered heteroaryl containing one atom selected from N, O and S can be for example furyl, thienyl or pyridyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently one or more R¹⁻¹⁻² substituted 5-10 membered heteroaryl containing 1, 2, or 3 atoms independently selected from N, O and S, and said 5-10 membered heteroaryl containing 1, 2, or 3 atoms independently selected from N, O and S is a 5 membered heteroaryl containing one atom selected from N, O and S, said R¹⁻¹⁻² and said -(CH₂)ₛ-or -NH- can be located in a ortho or meta position relative to each other.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently one or more R¹⁻¹⁻² substituted 5-10 membered heteroaryl containing 1, 2, or 3 atoms independently selected from N, O and S, and said 5-10 membered heteroaryl containing 1, 2, or 3 atoms independently selected from N, O and S is a 6 membered heteroaryl containing one atom selected from N, O and S, said R¹⁻¹⁻² and said -(CH₂)ₛ-or -NH- can be located in a ortho, meta or para position relative to each other, or alternatilvely can be in a para position relative to each other.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently C₃-C₆ cycloalkyl, said C₃-C₆ cycloalkyl is cyclobutyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently one or more R¹⁻¹⁻³ substituted C₃-C₆ cycloalkyl, said C₃-C₆ cycloalkyl is cyclobutyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently halogen, said halogen is fluorine.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently C₁-C₄ alkyl, said C₁-C₄ alkyl is methyl, ethyl, n-propyl, isopropyl, n-butyl, sec-butyl, isobutyl or tert-butyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently C₁-C₄ alkyl substituted with a plurality of halogens, said plurality of halogens are 2 or 3 halogens.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently C₁-C₄ alkyl substituted with one or more halogens, said halogen is fluorine.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently C₁-C₄ alkyl substituted with one or more halogens, said C₁-C₄ alkyl is methyl or ethyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently C₁-C₄ alkyl substituted with a plurality of halogens, said C₁-C₄ alkyl substituted with a plurality of halogens is trifluoromethyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently C₁-C₄ alkoxy, said C₁-C₄ alkoxy is methoxy.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently C₃-C₆ cycloalkyl, said C₃-C₆ cycloalkyl is cyclopropyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently 5-6 membered heterocycloalkyl containing 1, 2 or 3 atoms independently selected from N, O and S, said 5-6 membered heterocycloalkyl containing 1, 2 or 3 atoms independently selected from N, O and S can be morpholinyl, or alternatively morpholin-4-yl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:

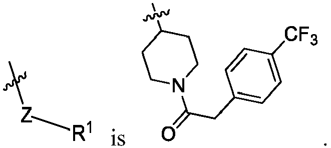

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments: 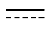
in 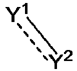 is a single bond or a double bond;
when 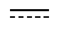 in 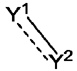 is a single bond, Y¹ is NH or N-OH, Y² is C=O;
when 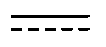 in 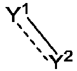 is a double bond, Y¹ is N, Y² is C-NH₂, C-NH-CN or C-O-CH₃.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
- 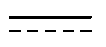 in 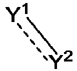 is a single bond.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments: 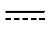 in 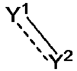 is a single bond, Y¹ is NH or N-OH, Y² is C=O.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments: 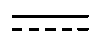 in 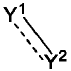 is a single bond, Y¹ is NH, Y² is C=O.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
Y³ is N.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
at most one of X¹, X², X³ and X⁴ is N.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
X¹ is CR², X² is CR⁴, X³ is CR⁵, X⁴ is CR⁶.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
X¹ is CR², X² is CR⁴, X³ is CR⁵, X⁴ is CR⁶; R², R⁴, R⁵ and R⁶ are independently hydrogen, halogen, hydroxy, amino, carboxy, cyano, nitro, C₁-C₄ alkyl, C₁-C₄ alkyl substituted with one or more halogens, C₂-C₄ alkenyl, C₂-C₄ alkynyl, C₁-C₄ alkoxy, C₃-C₆ cycloalkyl, phenyl, -S(=O)₂-R²⁻¹, -NH-S(=O)₂-R²⁻², -S(=O)₂-NR²⁻³R²⁻⁴, -C(=O)-R²⁻⁵, -NH-C(=O)-R²⁻⁶, or -C(=O)-NR²⁻⁷R²⁻⁸;
R²⁻¹, R²⁻², R²⁻³, R²⁻⁴, R²⁻⁵, R²⁻⁶, R²⁻⁷ and R²⁻⁸ are independently C₁-C₄ alkyl, or C₁-C₄ alkyl substituted with one or more halogens.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
X¹ is CR², X² is CR⁴, X³ is CR⁵, X⁴ is CR⁶; at least one of R², R⁴, R⁵ and R⁶ is halogen, hydroxyl, amino, carboxyl, cyano, nitro, C₁-C₄ alkyl, C₁-C₄ alkyl substituted with one or more halogens, C₂-C₄ alkenyl, C₂-C₄ alkynyl, C₁-C₄ alkoxy, C₃-C₆ cycloalkyl, phenyl, -S(=O)₂-R²⁻¹, - NH-S(=O)₂-R²⁻², -S(=O)₂-NR²⁻³R²⁻⁴, -C(=O)-R²⁻⁵, -NH-C(=O)-R²⁻⁶, -C(=O)-NR²⁻⁷R²⁻⁸, -O-(C=O)-R²⁻⁹, or -C(=O)-O-R²⁻¹⁰; R²⁻¹, R²⁻² , R²⁻³, R²⁻⁴, R²⁻⁵, R²⁻⁶, R²⁻⁷, R²⁻⁸, R²⁻⁹ and R²⁻¹⁰ are independently C₁-C₄ alkyl, C₁-C₄ alkyl substituted with one or more halogens, C₂-C₄ alkenyl, or C₂-C₄ alkynyl; and the rest are hydrogen.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
X¹ is CR², X² is CR⁴, X³ is CR⁵, X⁴ is CR⁶; one of R², R⁴, R⁵ and R⁶ is halogen, hydroxyl, amino, carboxyl, cyano, nitro, C₁-C₄ alkyl, C₁-C₄ alkyl substituted with one or more halogens, C₂-C₄ alkenyl, C₂-C₄ alkynyl, C₁-C₄ alkoxy, C₃-C₆ cycloalkyl, phenyl, -S(=O)₂-R²⁻¹, -NH-S(=O)2-R²⁻², -S(=O)2-NR²⁻³R²⁻⁴, -C(=O)-R²⁻⁵, -NH-C(=O)-R²⁻⁶, -C(=O)-NR²⁻⁷R²⁻⁸, -O-(C=O)-R²⁻⁹, or -C(=O)-O-R²⁻¹⁰; R²⁻¹, R²⁻², R²⁻³, R²⁻⁴, R²⁻⁵, R²⁻⁶, R²⁻⁷, R²⁻⁸, R²⁻⁹ and R²⁻¹⁰ are independently C₁-C₄ alkyl, C₁-C₄ alkyl substituted with one or more halogens, C₂-C₄ alkenyl, or C₂-C₄ alkynyl; and the rest are hydrogen.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
X¹ is CR², X² is CR⁴, X³ is CR⁵, X⁴ is CR⁶; one of R², R⁴, R⁵ and R⁶ is cyano; and the rest are hydrogen.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
X¹ is CH, X² is CH, X³ is CR⁵, X⁴ is CR⁶; one of R⁵ and R⁶ is halogen, hydroxyl, amino, carboxyl, cyano, nitro, C₁-C₄ alkyl, C₁-C₄ alkyl substituted with one or more halogens, C₂-C₄ alkenyl, C₂-C₄ alkynyl, C₁-C₄ alkoxy, C₃-C₆ cycloalkyl, phenyl, -S(=O)₂-R²⁻¹, -NH-S(=O)₂-R²⁻², -S(=O)₂-NR²⁻³R²⁻⁴, -C(=O)-R²⁻⁵, -NH-C(=O)-R²⁻⁶, -C(=O)-NR²⁻⁷R²⁻⁸, -O-(C=O)-R²⁻⁹, or - C(=O)-O-R²⁻¹⁰; R²⁻¹, R²⁻², R²⁻³, R²⁻⁴, R²⁻⁵, R²⁻⁶, R²⁻⁷, R²⁻⁸, R²⁻⁹ and R²⁻¹⁰ are independently C₁-C₄ alkyl, C₁-C₄ alkyl substituted with one or more halogens, C₂-C₄ alkenyl, or C₂-C₄ alkynyl; and the other one is hydrogen.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
X¹ is CH, X² is CH, X³ is CR⁵, X⁴ is CR⁶; one of R⁵ and R⁶ is cyano; and the other is hydrogen.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
Z is 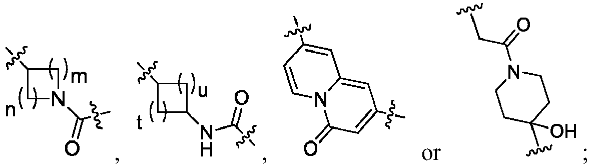 m is 1 or 2, n is 1 or 2; u is 1 or 2, t is 1 or 2.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
R¹ is -(CH₂)ₛ-R¹⁻¹; s is 0, 1 or 2;
R¹⁻¹ is C₆-C₁₀ aryl, one or more R¹⁻¹⁻¹ substituted C₆-C₁₀ aryl, 5-10 membered heteroaryl containing 1, 2, or 3 atoms independently selected from N, O and S, or one or more R¹⁻¹⁻² substituted 5-10 membered heteroaryl containing 1, 2, or 3 atoms independently selected from N, O and S;
R¹⁻¹⁻¹ and R¹⁻¹⁻² are independently C₁-C₄ alkyl, C₁-C₄ alkyl substituted with one or more halogens, C₃-C₆ cycloalkyl, or phenyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:

R¹⁻¹ and R¹⁻² are independently naphthyl, one or more R¹⁻¹⁻¹ substituted C₆-C₁₀ aryl, 10 membered heteroaryl containing 1, 2, or 3 atoms independently selected from N, O and S, one or more R¹⁻¹⁻² substituted 5-10 membered heteroaryl containing 1, 2, or 3 atoms independently selected from N, O and S, C₃-C₆ cycloalkyl, one or more R¹⁻¹⁻³ substituted C₃-C₆ cycloalkyl, cyclopentadienyl, or one or more R¹⁻¹⁻⁴ substituted cyclopentadienyl;

R¹⁻¹⁻¹, R¹⁻¹⁻², R¹⁻¹⁻³ and R¹⁻¹⁻⁴ are independently hydroxy, cyano, halogen, carboxy, C₁-C₄ alkyl, C₁-C₄ alkyl substituted with one or more halogens, C₃-C₆ cycloalkyl, 5-6 membered heterocycloalkyl containing 1, 2, or 3 atoms independently selected from N, O and S, or phenyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
wherein, 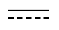 in 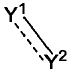 is a single bond or a double bond;
when 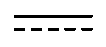 in 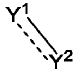 is a single bond, Y¹ is NH or N-OH, Y² is C=O;
when 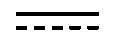 in 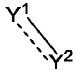 is a double bond, Y¹ is N, Y² is C-NH₂, C-NH-CN or C-O-CH₃;
Y³ is N;
X¹ is CR², X² is CR⁴, X³ is CR⁵, X⁴ is CR⁶;
R², R⁴, R⁵ and R⁶ are independently hydrogen, halogen, hydroxy, amino, carboxy, cyano, nitro, C₁-C₄ alkyl, C₁-C₄ alkyl substituted with one or more halogens, C₂-C₄ alkenyl, C₂-C₄ alkynyl, C₁-C₄ alkoxy, C₃-C₆ cycloalkyl, phenyl, -S(=O)₂-R²⁻¹, -NH-S(=O)₂-R²⁻², -S(=O)₂-NR²⁻³R²⁻⁴, -C(=O)-R²⁻⁵, -NH-C(=O)-R²⁻⁶, or -C(=O)-NR²⁻⁷R²⁻⁸;
R²⁻¹, R²⁻², R²⁻³, R²⁻⁴, R²⁻⁵, R²⁻⁶, R²⁻⁷ and R²⁻⁸ are independently C₁-C₄ alkyl, or C₁-C₄ alkyl substituted with one or more halogens;
Z is 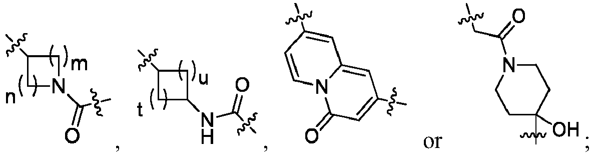 m is 1 or 2, n is 1 or 2; u is 1 or 2, t is 1 or 2;
R¹ is -(CH₂)ₛ-R¹⁻¹; s is 0, 1 or 2;
R¹⁻¹ is C₆-C₁₀ aryl, one or more R¹⁻¹⁻¹ substituted C₆-C₁₀ aryl, 5-10 membered heteroaryl containing 1, 2, or 3 atoms independently selected from N, O and S, or one or more R¹⁻¹⁻² substituted 5-10 membered heteroaryl containing 1, 2, or 3 atoms independently selected from N, O and S;
R¹⁻¹⁻¹ and R¹⁻¹⁻² are independently C₁-C₄ alkyl, C₁-C₄ alkyl substituted with one or more halogens, C₃-C₆ cycloalkyl, or phenyl.

In a certain embodiment, in the compound represented by formula **XI,** or a pharmaceutically acceptable salt, a solvate, or a solvate of a pharmaceutically acceptable salt thereof, the compound represented by formula **XI** is a compound represented by formula **III**; 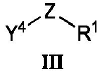
wherein, Y⁴ is 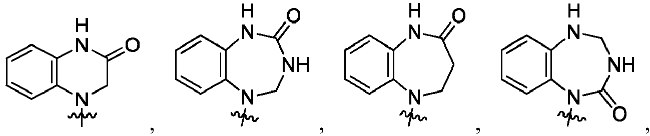 or 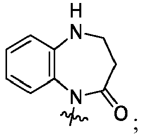
Z is 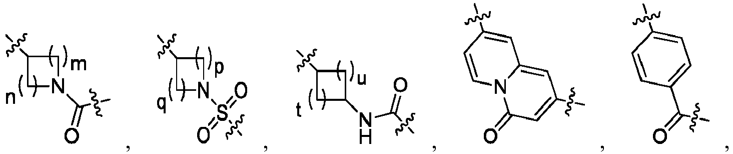 or 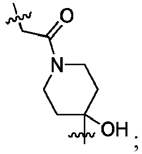 m is 1 or 2, n is 1 or 2; p is 1 or 2, q is 1 or 2; u is 1 or 2, t is 1 or 2;
R¹ is C₂-C₄ alkyl, C₂-C₄ alkyl substituted with one or more halogens, -(CH₂)ₛ-R¹⁻¹, or - NH-R¹⁻²; s is 0, 1 or 2;
R¹⁻¹ and R¹⁻² are independently C₆-C₁₀ aryl, one or more R¹⁻¹⁻¹ substituted C₆-C₁₀ aryl, 5-10 membered heteroaryl containing 1, 2, or 3 atoms independently selected from N, O and S, one or more R¹⁻¹⁻² substituted 5-10 membered heteroaryl containing 1, 2, or 3 atoms independently selected from N, O and S, C₃-C₆ cycloalkyl, one or more R¹⁻¹⁻³ substituted C₃-C₆ cycloalkyl, cyclopentadienyl, or one or more R¹⁻¹⁻⁴ substituted cyclopentadienyl;
R¹⁻¹⁻¹, R¹⁻¹⁻², R¹⁻¹⁻¹ and R¹⁻¹⁻⁴ are independently hydroxyl, cyano, halogen, carboxyl, C₁-C₄ alkyl, C₁-C₄ alkyl substituted with one or more halogens, C₁-C₄ alkoxy, C₃-C₆ cycloalkyl, 5-6 membered heterocycloalkyl containing 1, 2, or 3 atoms independently selected from N, O and S, or phenyl.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when Z is 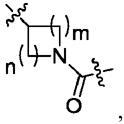 m is 1, n is 1.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when Z is 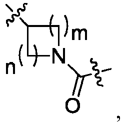 m is 2, n is 2.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when Z is 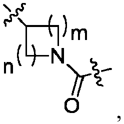 the 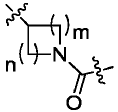 is 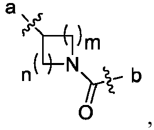 whose b end is connected with R¹.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when Z is 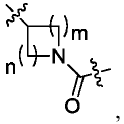 m is 1, n is 1, the 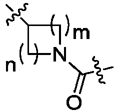 is 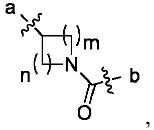 whose b end is connected with R¹.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when Z is 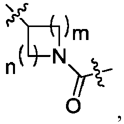 m is 2, n is 2, the 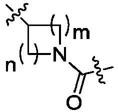 is 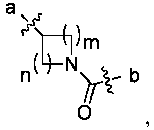 whose b end is connected with R¹.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when Z is 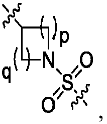 the 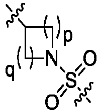 is 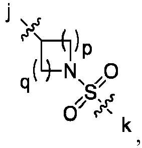 whose k end is connected with R¹.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when Z is 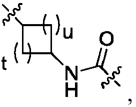 u is 1, t is 1.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when Z is 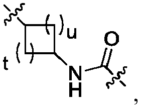 u is 2, t is 2.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when Z is 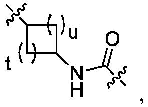 the 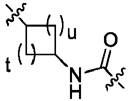 is 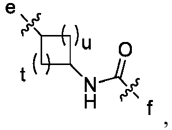 whose f end is connected with R¹;
or alternatively it can be 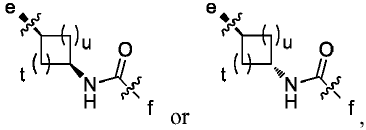 whose f end is connected with R¹.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when Z is 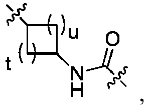 the 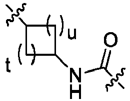 is 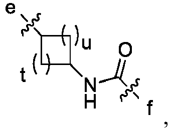 whose f end is connected with R¹, and u is 1, t is 1;
or alternatively it can be 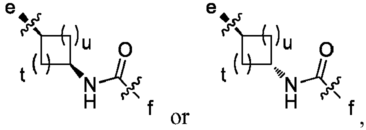 whose f end is connected with R¹, and u is 1, t is 1.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when Z is 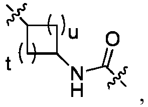 the 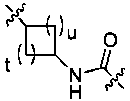 is 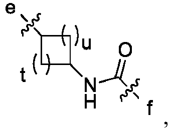 whose f end is connected with R¹, and u is 2, t is 2;
or alternatively it can be 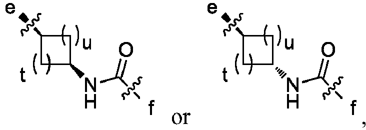 whose f end is connected with R¹, and u is 2, t is 2.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when Z is 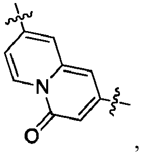 the 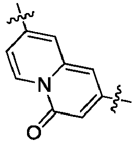 is 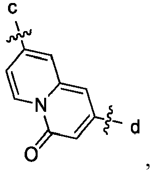 whose d end is connected with R¹.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when Z is 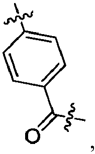 the 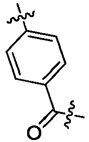 is 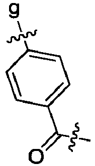 , whose h end is connected with R¹.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when Z is 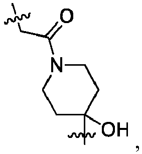 the 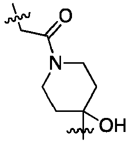 is 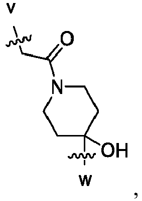 whose w end is connected with R¹.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹ is a C₂-C₄ alkyl, said C₂-C₄ alkyl is ethyl.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹ is a C₂-C₄ alkyl substituted with a plurality of halogens, said plurality of halogens are 2 or 3 halogens.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹ is a C₂-C₄ alkyl substituted with one or more halogens, said halogen is fluorine.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹ is a C₂-C₄ alkyl substituted with one or more halogens, the C₂-C₄ alkyl is ethyl.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹ is C₂-C₄ alkyl substituted with a plurality halogens, said C₂-C₄ alkyl substituted with a plurality halogens is 2,2,2-trifluoroethyl.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently C₆-C₁₀ aryl, said C₆-C₁₀ aryl is phenyl or naphthyl.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently one or more R¹⁻¹⁻¹ substituted C₆-C₁₀ aryl, said C₆-C₁₀ aryl is phenyl or naphthyl.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently one or more R¹⁻¹⁻¹ substituted C₆-C₁₀ aryl, and said C₆-C₁₀ aryl is phenyl, said R¹⁻¹⁻¹ and said -(CH₂)ₛ- or -NH- can be located in a ortho, meta or para position relative to each other, or alternatilvely can be in a para position relative to each other.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently 5-10 membered heteroaryl containing 1, 2, or 3 atoms independently selected from N, O and S, said 5-10 membered heteroaryl containing 1, 2, or 3 atoms independently selected from N, O and S can be 5-6 membered heteroaryl, indolyl, isoquinolyl or quinolyl containing one atom selected from N, O and S, or alternatilvely can be a 5-6 membered heteroaryl containing one atom selected from N, O and S.

The 5-6 membered heteroaryl containing one atom selected from N, O and S can be for example furyl, thienyl or pyridyl.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently one or more R¹⁻¹⁻² substituted 5-10 membered heteroaryl containing 1, 2, or 3 atoms independently selected from N, O and S, said 5-10 membered heteroaryl containing 1, 2, or 3 atoms independently selected from N, O and S can be 5-6 membered heteroaryl, indolyl, isoquinolyl or quinolyl containing one atom selected from N, O and S, or alternatilvely can be a 5-6 membered heteroaryl containing one atom selected from N, O and S.

The 5-6 membered heteroaryl containing one atom selected from N, O and S can be for example furyl, thienyl or pyridyl.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently one or more R¹⁻¹⁻² substituted 5-10 membered heteroaryl containing 1, 2, or 3 atoms independently selected from N, O and S, and said 5-10 membered heteroaryl containing 1, 2, or 3 atoms independently selected from N, O and S is a 5 membered heteroaryl containing one atom selected from N, O and S, said R¹⁻¹⁻² and said -(CH₂)ₛ-or -NH- can be located in a ortho or meta position relative to each other.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently one or more R¹⁻¹⁻² substituted 5-10 membered heteroaryl containing 1, 2, or 3 atoms independently selected from N, O and S, and said 5-10 membered heteroaryl containing 1, 2, or 3 atoms independently selected from N, O and S is a 6 membered heteroaryl containing one atom selected from N, O and S, said R¹⁻¹⁻² and said -(CH₂)ₛ-or -NH- can be located in a ortho, meta or para position relative to each other, or alternatilvely can be in a para position relative to each other.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently C₃-C₆ cycloalkyl, said C₃-C₆ cycloalkyl is cyclobutyl.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently one or more R¹⁻¹⁻³ substituted C₃-C₆ cycloalkyl, said C₃-C₆ cycloalkyl is cyclobutyl.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently halogen, said halogen is fluorine.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently C₁-C₄ alkyl, said C₁-C₄ alkyl is methyl, ethyl, n-propyl, isopropyl, n-butyl, sec-butyl, isobutyl or tert-butyl.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently C₁-C₄ alkyl substituted with a plurality of halogens, said plurality of halogens are 2 or 3 halogens.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently C₁-C₄ alkyl substituted with one or more halogens, said halogen is fluorine.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently C₁-C₄ alkyl substituted with one or more halogens, said C₁-C₄ alkyl is methyl or ethyl.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently C₁-C₄ alkyl substituted with a plurality of halogens, said C₁-C₄ alkyl substituted with a plurality of halogens is trifluoromethyl.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently C₁-C₄ alkoxy, said C₁-C₄ alkoxy is methoxy.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently C₃-C₆ cycloalkyl, said C₃-C₆ cycloalkyl is cyclopropyl.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:

when R¹⁻¹⁻¹, R¹⁻¹⁻², R¹⁻¹⁻³, and R¹⁻¹⁻⁴ are independently 5-6 membered heterocycloalkyl containing 1, 2 or 3 atoms independently selected from N, O and S, said 5-6 membered heterocycloalkyl containing 1, 2 or 3 atoms independently selected from N, O and S can be morpholinyl, or alternatively morpholin-4-yl.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:

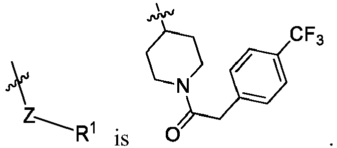

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
Y⁴ is

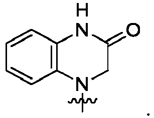

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
Y⁴ is

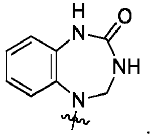

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
Y⁴ is

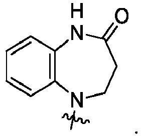

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
Y⁴ is

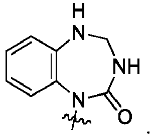

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
Y⁴ is

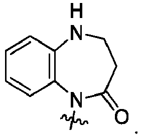

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
Z is 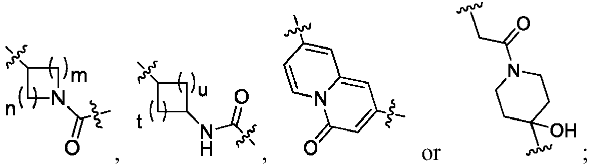 m is 1 or 2, n is 1 or 2; u is 1 or 2, t is 1 or 2.

In a certain embodiment, in the compound represented by formula **III**, or a pharmaceutically acceptable salt, a solvate, or a solvate of a pharmaceutically acceptable salt thereof, certain groups can be defined as follows, while the remaining groups are as defined in any of the above embodiments:
R¹ is -(CH₂)₅-R¹⁻¹; s is 0, 1 or 2;
R¹⁻¹ is C₆-C₁₀ aryl, one or more R¹⁻¹⁻¹ substituted C₆-C₁₀ aryl, 5-10 membered heteroaryl containing 1, 2, or 3 atoms independently selected from N, O and S, or one or more R¹⁻¹⁻² substituted 5-10 membered heteroaryl containing 1, 2, or 3 atoms independently selected from N, O and S;
R¹⁻¹⁻¹ and R¹⁻¹⁻² are independently C₁-C₄ alkyl, C₁-C₄ alkyl substituted with one or more halogens, C₃-C₆ cycloalkyl, or phenyl.

In a certain embodiment, in the compound represented by formula **XI,** or a pharmaceutically acceptable salt, a solvate, or a solvate of a pharmaceutically acceptable salt thereof, the compound represented by formula **XI** is a compound represented by formula **V;** 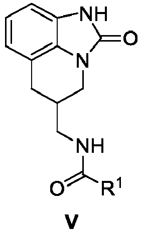
wherein, R¹ is C₂-C₄ alkyl, C₂-C₄ alkyl substituted with one or more halogens, -(CH₂)ₛ-R¹⁻¹, or -NH-R¹⁻²; s is 0, 1 or 2;
R¹⁻¹ and R¹⁻² are independently C₆-C₁₀ aryl, one or more R¹⁻¹⁻¹ substituted C₆-C₁₀ aryl, 5-10 membered heteroaryl containing 1, 2, or 3 atoms independently selected from N, O and S, one or more R¹⁻¹⁻² substituted 5-10 membered heteroaryl containing 1, 2, or 3 atoms independently selected from N, O and S, C₃-C₆ cycloalkyl, one or more R¹⁻¹⁻³ substituted C₃-C₆ cycloalkyl, cyclopentadienyl, or one or more R¹⁻¹⁻⁴ substituted cyclopentadienyl;
R¹⁻¹⁻¹, R¹⁻¹⁻², R¹⁻¹⁻³ and R¹⁻¹⁻⁴ are independently hydroxyl, cyano, halogen, carboxyl, C₁-C₄ alkyl, C₁-C₄ alkyl substituted with one or more halogens, C₁-C₄ alkoxy, C₃-C₆ cycloalkyl, 5-6 membered heterocycloalkyl containing 1, 2, or 3 atoms independently selected from N, O and S, or phenyl.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹ is a C₂-C₄ alkyl, said C₂-C₄ alkyl is ethyl.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹ is a C₂-C₄ alkyl substituted with a plurality of halogens, said plurality of halogens are 2 or 3 halogens.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹ is a C₂-C₄ alkyl substituted with one or more halogens, said halogen is fluorine.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹ is a C₂-C₄ alkyl substituted with one or more halogens, the C₂-C₄ alkyl is ethyl.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹ is C₂-C₄ alkyl substituted with a plurality halogens, said C₂-C₄ alkyl substituted with a plurality halogens is 2,2,2-trifluoroethyl.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently C₆-C₁₀ aryl, said C₆-C₁₀ aryl is phenyl or naphthyl.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently one or more R¹⁻¹⁻¹ substituted C₆-C₁₀ aryl, said C₆-C₁₀ aryl is phenyl or naphthyl.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently one or more R¹⁻¹⁻¹ substituted C₆-C₁₀ aryl, and said C₆-C₁₀ aryl is phenyl, said R¹⁻¹⁻¹ and said -(CH₂)ₛ- or -NH- can be located in a ortho, meta or para position relative to each other, or alternatilvely can be in a para position relative to each other.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently 5-10 membered heteroaryl containing 1, 2, or 3 atoms independently selected from N, O and S, said 5-10 membered heteroaryl containing 1, 2, or 3 atoms independently selected from N, O and S can be 5-6 membered heteroaryl, indolyl, isoquinolyl or quinolyl containing one atom selected from N, O and S, or alternatilvely can be a 5-6 membered heteroaryl containing one atom selected from N, O and S.

The 5-6 membered heteroaryl containing one atom selected from N, O and S can be for example furyl, thienyl or pyridyl.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently one or more R¹⁻¹⁻² substituted 5-10 membered heteroaryl containing 1, 2, or 3 atoms independently selected from N, O and S, said 5-10 membered heteroaryl containing 1, 2, or 3 atoms independently selected from N, O and S can be 5-6 membered heteroaryl, indolyl, isoquinolyl or quinolyl containing one atom selected from N, O and S, or alternatilvely can be a 5-6 membered heteroaryl containing one atom selected from N, O and S.

The 5-6 membered heteroaryl containing one atom selected from N, O and S can be for example furyl, thienyl or pyridyl.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently one or more R¹⁻¹⁻² substituted 5-10 membered heteroaryl containing 1, 2, or 3 atoms independently selected from N, O and S, and said 5-10 membered heteroaryl containing 1, 2, or 3 atoms independently selected from N, O and S is a 5 membered heteroaryl containing one atom selected from N, O and S, said R¹⁻¹⁻² and said -(CH₂)ₛ-or -NH- can be located in a ortho or meta position relative to each other.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently one or more R¹⁻¹⁻² substituted 5-10 membered heteroaryl containing 1, 2, or 3 atoms independently selected from N, O and S, and said 5-10 membered heteroaryl containing 1, 2, or 3 atoms independently selected from N, O and S is a 6 membered heteroaryl containing one atom selected from N, O and S, said R¹⁻¹⁻² and said -(CH₂)ₛ-or -NH- can be located in a ortho, meta or para position relative to each other, or alternatilvely can be in a para position relative to each other.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently C₃-C₆ cycloalkyl, said C₃-C₆ cycloalkyl is cyclobutyl.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹ and R¹⁻² are independently one or more R¹⁻¹⁻³ substituted C₃-C₆ cycloalkyl, said C₃-C₆ cycloalkyl is cyclobutyl.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently halogen, said halogen is fluorine.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently C₁-C₄ alkyl, said C₁-C₄ alkyl is methyl, ethyl, n-propyl, isopropyl, n-butyl, sec-butyl, isobutyl or tert-butyl.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently C₁-C₄ alkyl substituted with a plurality of halogens, said plurality of halogens are 2 or 3 halogens.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently C₁-C₄ alkyl substituted with one or more halogens, said halogen is fluorine.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently C₁-C₄ alkyl substituted with one or more halogens, said C₁-C₄ alkyl is methyl or ethyl.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently C₁-C₄ alkyl substituted with a plurality of halogens, said C₁-C₄ alkyl substituted with a plurality of halogens is trifluoromethyl.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently C₁-C₄ alkoxy, said C₁-C₄ alkoxy is methoxy.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently C₃-C₆ cycloalkyl, said C₃-C₆ cycloalkyl is cyclopropyl.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁴ are independently 5-6 membered heterocycloalkyl containing 1, 2 or 3 atoms independently selected from N, O and S, said 5-6 membered heterocycloalkyl containing 1, 2 or 3 atoms independently selected from N, O and S can be morpholinyl, or alternatively morpholin-4-yl.

In a certain embodiment, in the compound represented by formula **V,** or a pharmaceutically acceptable salt, a solvate, or a solvate of a pharmaceutically acceptable salt thereof, certain groups can be defined as follows, while the remaining groups are as defined in any of the above embodiments:
R¹ is -(CH₂)ₛ-R¹⁻¹; s is 0, 1 or 2;
R¹⁻¹ is C₆-C₁₀ aryl, one or more R¹⁻¹⁻¹ substituted C₆-C₁₀ aryl, 5-10 membered heteroaryl containing 1, 2, or 3 atoms independently selected from N, O and S, or one or more R¹⁻¹⁻² substituted 5-10 membered heteroaryl containing 1, 2, or 3 atoms independently selected from N, O and S;
R¹⁻¹⁻¹ and R¹⁻¹⁻² are independently C₁-C₄ alkyl, C₁-C₄ alkyl substituted with one or more halogens, C₃-C₆ cycloalkyl, or phenyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is anyone of the following groups:
(1) 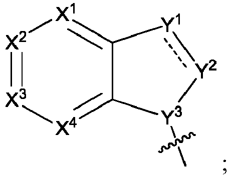 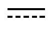
   in 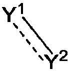 is a single bond or a double bond;
   when 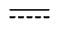 in 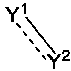 is a single bond, Y¹ is C(=O), O, or N-R³, R³ is hydrogen, hydroxyl, CH₂COOH or C₁-C₄ alkyl, Y² is C(=N-CO-Ph), C(=N-CO-CH₃), NH, S(=O)₂, C=O or C=S;
   when 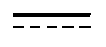 in 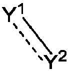 is a double bond, Y¹ is N, and Y² is C-NH₂ or C-NH-CN;
   Y³ is N or CH;
   X¹ is N or CR², X² is N or CR⁴, X³ is N or CR⁵, X⁴ is N or CR⁶;
   R², R⁴, R⁵ and R⁶ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NR²⁻³R²⁻⁴, or -C(=O)-NR²⁻⁷R²⁻⁸;
   R²⁻³, R²⁻⁴, R²⁻⁷ and R²⁻⁸ are independently hydrogen;
(2) 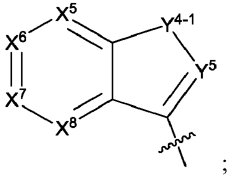
   Y⁴⁻¹ is -NH-C(=O)-CH₂- or NH;
   Y⁵ is N or C-R⁷, R⁷ is halogen;
   X⁵ is CR⁸, X⁶ is CR⁹, X⁷ is CR¹⁰, X⁸ is CR¹¹;
   R⁸, R⁹, R¹⁰ and R¹¹ are independently hydrogen;
(3) 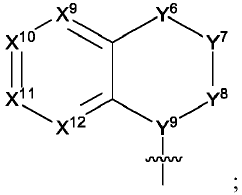
   Y⁶ is N-C(=O)-CH₃, CH₂ or NH;
   Y⁷ is NH, CH₂, C(=O), C(=S) or C(=N-OH);
   Y⁸ is CH₂, CH₂CH₂, CH(CH₃) or C(=O);
   Y⁹ is N;
   X⁹ is CR¹², X¹⁰ is CR¹³, X¹¹ is CR¹⁴, X¹² is CR¹⁵;
   R¹², R¹³, R¹⁴ and R¹⁵ are independently hydrogen, nitro, or C₁-C₄ alkyl substituted with one or more halogens;
(4) 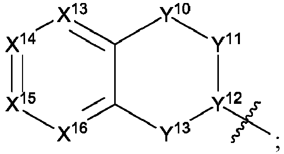
   Y¹⁰ is NH;
   Y¹¹ is C(=O);
   Y¹² is N;
   Y¹³ is CH₂;
   X¹³ is CR¹⁶, X¹⁴ is CR¹⁷, X¹⁵ is CR¹⁸, X¹⁶ is CR¹⁹;
   R¹⁶, R¹⁷, R¹⁸ and R¹⁹ are independently hydrogen;
(5) 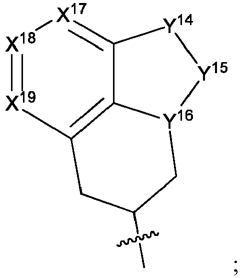
   Y¹⁴ is NH;
   Y¹⁵ is C(=O);
   Y¹⁶ is N;
   X¹⁷ is CR²⁰, X¹⁸ is CR²¹, X¹⁹ is CR²²;
   R²⁰, R²¹ and R²² are independently hydrogen;
(6) 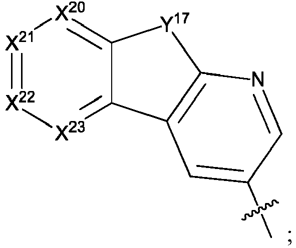
   Y¹⁷ is NH;
   X²⁰ is CR²³, X²¹ is CR²⁴, X²² is CR²⁵, X²³ is CR²⁶;
   R²³, R²⁴, R²⁵ and R²⁶ are independently hydrogen;
(7) 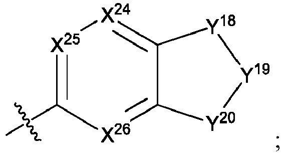
   Y¹⁸ is NH or CH₂;
   Y¹⁹ is C(=O) or NH;
   Y²⁰ is CH₂ or C(=O);
   X²⁴ is CR²⁷, X²⁵ is CR²⁸, X²⁶ is CR²⁹;
   R²⁷, R²⁸ and R²⁹ are independently hydrogen;
(8) 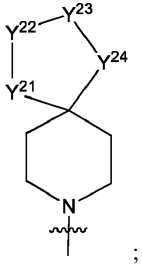
   Y²¹ is C(=O);
   Y²² is NH;
   Y²³ is CH₂ or C(=O);
   Y²⁴ is N-R³⁰, R³⁰ is hydrogen or phenyl;
(9) 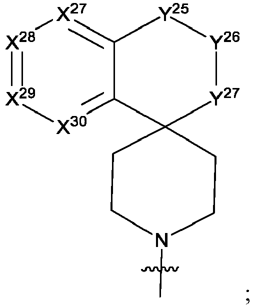
   Y²⁵ is NH;
   Y²⁶ is C(=O);
   Y²⁷ is a single bond, O or NH;
   X²⁷ is CR³¹, X²⁸ is CR³², X²⁹ is CR³³, X³⁰ is CR³⁴;
   R³¹, R³², R³³ and R³⁴ are independently hydrogen;
(10) 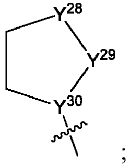
   Y²⁸ is NH;
   Y²⁹ is C(=O);
   Y³⁰ is N;
(11) 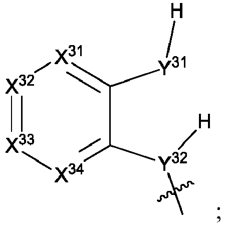
   Y³¹ is O, NH or N-C(=O)-CH₃;
   Y³² is N;
   X³¹ is CR³⁵, X³² is CR³⁶, X³³ is CR³⁷, X³⁴ is CR³⁸;
   R³⁵, R³⁶, R³⁷ and R³⁸ are independently hydrogen;
(12) 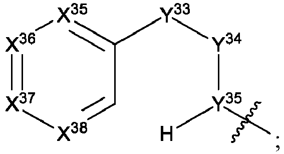
   Y³³ is NH;
   Y³⁴ is C(=O);
   Y³⁵ is N;
   X³⁵ is CR³⁹, X³⁶ is CR⁴⁰, X³⁷ is CR⁴¹, X³⁸ is CR⁴²;
   R³⁹, R⁴⁰, R⁴¹ and R⁴² are independently hydrogen;
(13) 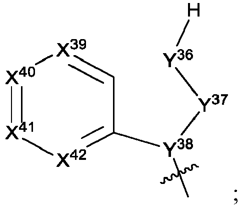
   Y³⁶ is NH;
   Y³⁷ is C(=O);
   Y³⁸ is N;
   X³⁹ is CR⁴³, X⁴⁰ is CR⁴⁴, X⁴¹ is CR⁴⁵, X⁴² is CR⁴⁶;
   R⁴³, R⁴⁴, R⁴⁵ and R⁴⁶ are independently hydrogen;
(14) 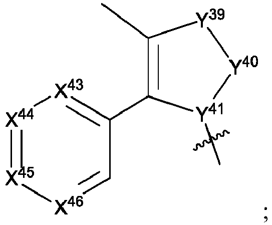
   Y³⁹ is NH;
   Y⁴⁰ is C(=O);
   Y⁴¹ is N;
   X⁴³ is CR⁴⁷, X⁴⁴ is CR⁴⁸, X⁴⁵ is CR⁴⁹, X⁴⁶ is CR⁵⁰;
   R⁴⁷, R⁴⁸, R⁴⁹ and R⁵⁰ are independently hydrogen; and
(15)

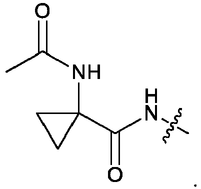

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is anyone of the following groups:
(1) 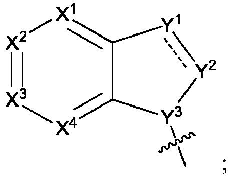 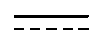
   -- in 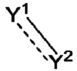 is a single bond or a double bond;
   when 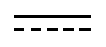 in 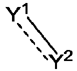 is a single bond, Y¹ is NH or N-OH, Y² is C(=N-CO-Ph), C(=N-CO-CH₃), S(=O)₂, C=O or C=S;
   when 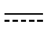 in 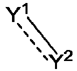 is a double bond, Y¹ is N, Y² is C-NH₂ or C-NH-CN;
   Y³ is N or CH;
   X¹ is N or CR², X² is N or CR⁴, X³ is N or CR⁵, X⁴ is N or CR⁶;
   R², R⁴, R⁵ and R⁶ are independently hydrogen, halogen, hydroxyl, amino, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NR²⁻³R²⁻⁴ or -C(=O)-NR²⁻⁷R²⁻⁸;
   R²⁻³, R²⁻⁴, R²⁻⁷ and R²⁻⁸ are independently hydrogen;
(2) 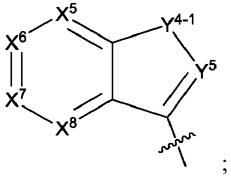
   Y⁴⁻¹ is -NH-C(=O)-CH₂- or NH;
   Y⁵ is N or C-R⁷, R⁷ is halogen;
   X⁵ is CR⁸, X⁶ is CR⁹, X⁷ is CR¹⁰, X⁸ is CR¹¹;
   R⁸, R⁹, R¹⁰ and R¹¹ are independently hydrogen;
(3) 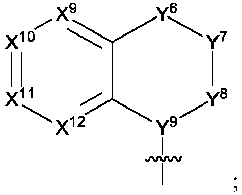
   Y⁶ is N-C(=O)-CH₃ or NH;
   Y⁷ is C(=O), C(=S) or C(=N-OH);
   Y⁸ is CH₂ or C(=O);
   Y⁹ is N;
   X⁹ is CR¹², X¹⁰ is CR¹³, X¹¹ is CR¹⁴, X¹² is CR¹⁵;
   R¹², R¹³, R¹⁴ and R¹⁵ are independently hydrogen, nitro, or C₁-C₄ alkyl substituted with one or more halogens;
(4) 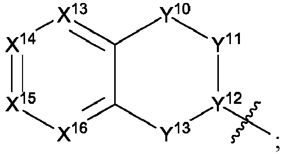
   Y¹⁰ is NH;
   Y¹¹ is C(=O);
   Y¹² is N;
   Y¹³ is CH₂;
   X¹³ is CR¹⁶, X¹⁴ is CR¹⁷, X¹⁵ is CR¹⁸, X¹⁶ is CR¹⁹;
   R¹⁶, R¹⁷ R¹⁸ and R¹⁹ are independently hydrogen;
(5) 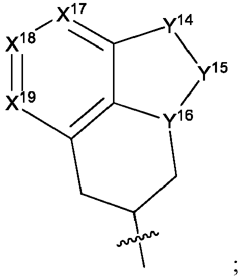
   Y¹⁴ is NH;
   Y¹⁵ is C(=O);
   Y¹⁶ is N;
   X¹⁷ is CR²⁰, X¹⁸ is CR²¹, X¹⁹ is CR²²;
   R²⁰, R²¹ and R²² are independently hydrogen;
(7) 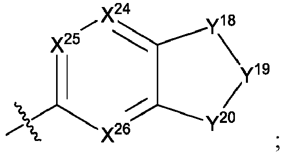
   Y¹⁸ is NH or CH₂;
   Y¹⁹ is C(=O) or NH;
   Y²⁰ is CH₂ or C(=O);
   X²⁴ is CR²⁷, X²⁵ is CR²⁸, X²⁶ is CR²⁹;
   R²⁷, R²⁸ and R²⁹ are independently hydrogen;
(8) 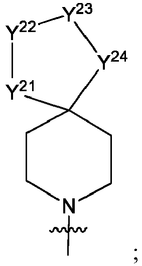
   Y²¹ is C(=O);
   Y²² is NH;
   Y²³ is CH₂;
   Y²⁴ is N-R³⁰, R³⁰ is phenyl;
(9) 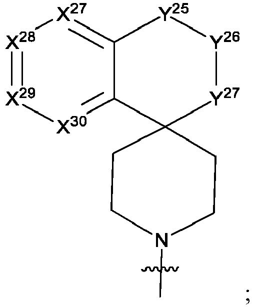
   Y²⁵ is NH;
   Y²⁶ is C(=O);
   Y²⁷ is a single bond, O or NH;
   X²⁷ is CR³¹, X²⁸ is CR³², X²⁹ is CR³³, X³⁰ is CR³⁴;
   R³¹, R³² R³³ and R³⁴ are independently hydrogen;
(11) 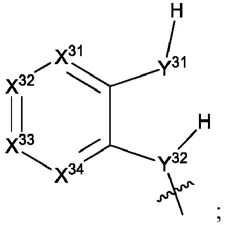
   Y³¹ is O, NH or N-C(=O)-CH₃;
   Y³² is N;
   X³¹ is CR³⁵, X³² is CR³⁶, X³³ is CR³⁷, X³⁴ is CR³⁸;
   R³⁵, R³⁶, R³⁷ and R³⁸ are independently hydrogen;
(12) 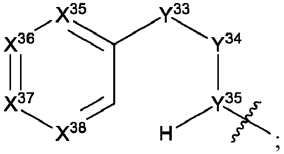
   Y³³ is NH;
   Y³⁴ is C(=O);
   Y³⁵ is N;
   X³⁵ is CR³⁹, X³⁶ is CR⁴⁰, X³⁷ is CR⁴¹, X³⁸ is CR⁴²;
   R³⁹, R⁴⁰, R⁴¹ and R⁴² are independently hydrogen;
(13) 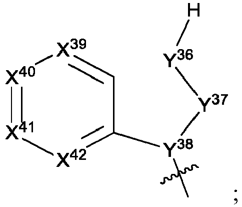
   Y³⁶ is NH;
   Y³⁷ is C(=O);
   Y³⁸ is N;
   X³⁹ is CR⁴³, X⁴⁰ is CR⁴⁴, X⁴¹ is CR⁴⁵, X⁴² is CR⁴⁶;
   R⁴³, R⁴⁴, R⁴⁵ and R⁴⁶ are independently hydrogen; and
(14) 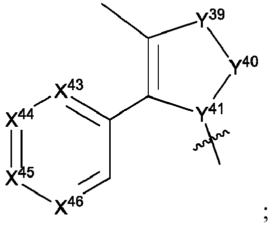
   Y³⁹ is NH;
   Y⁴⁰ is C(=O);
   Y⁴¹ is N;
   X⁴³ is CR⁴⁷, X⁴⁴ is CR⁴⁸, X⁴⁵ is CR⁴⁹, X⁴⁶ is CR⁵⁰;
   R⁴⁷, R⁴⁸, R⁴⁹ and R⁵⁰ are independently hydrogen.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
R¹ is -(CH₂)ₛ-R¹⁻¹, -NH-R¹⁻², 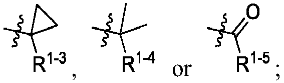 s is 0, 1 or 2;
R¹⁻¹, R¹⁻², R¹⁻³, R¹⁻⁴ and R¹⁻⁵ are independently one or more R¹⁻¹⁻³ substituted C₃-C₆ cycloalkyl, one or more R¹⁻¹⁻¹ substituted C₆-C₁₀ aryl, naphthyl, 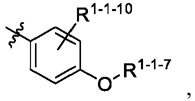 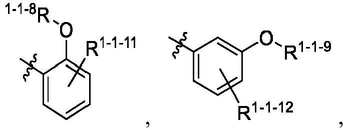 5, 10 membered heteroaryl containing 1, 2 or 3 atoms independently selected from N, O and S, pyridyl, 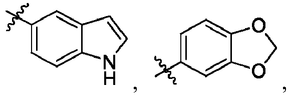 or one or more R¹⁻¹⁻² substituted 5-10 membered heteroaryl containing 1, 2 or 3 atoms independently selected from N, O and S;
R¹⁻¹⁻¹, R¹⁻¹⁻², and R¹⁻¹⁻³ are independently carboxy, hydroxy, halogen, C₁-C₄ alkyl, C₁-C₄ alkyl substituted with one or more halogens, C₁-C₄ alkoxy substituted with one or more halogens, C₃-C₆ cycloalkyl, 5-6 membered heterocycloalkyl containing 1, 2, or 3 atoms independently selected from N, O and S, -N(R¹⁻¹⁻¹⁻¹)(R¹⁻¹⁻¹⁻²), or phenyl;
R¹⁻¹⁻¹⁻¹ and R¹⁻¹⁻¹⁻² are independently hydrogen or C₁-C₄ alkyl;
R¹⁻¹⁻⁷, R¹⁻¹⁻⁸, and R¹⁻¹⁻⁹ are independently C₁-C₄ alkyl;
R¹⁻¹⁻¹⁰ is independently C₁-C₄ alkoxy;
R¹⁻¹⁻¹¹ and R¹⁻¹⁻¹² are independently hydrogen or halogen.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when W is 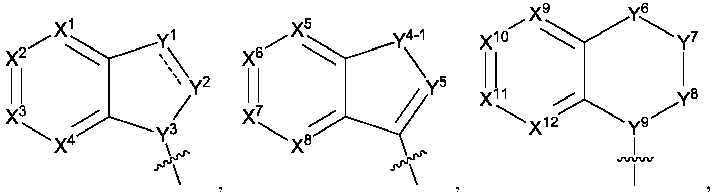 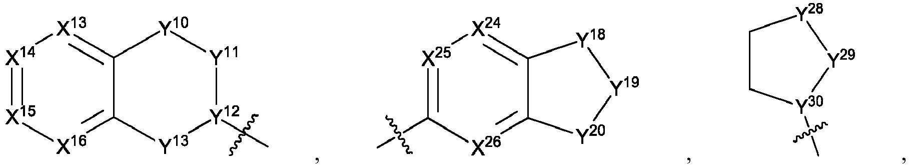 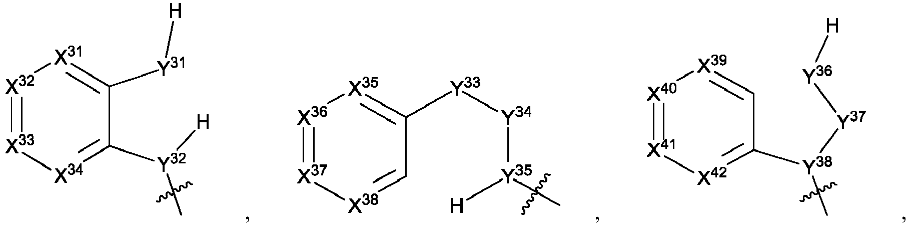 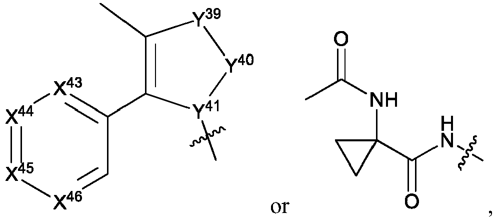 Z is anyone of the following fragments:
(1) 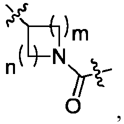 m is 1 or 2, n is 1, 2 or 3;
(2) 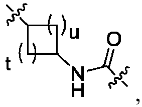 u is 1 or 2, t is 1 or 2;
(3) 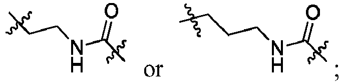
(4) 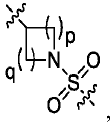 p is 1 or 2, q is 1 or 2;
(5) 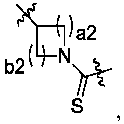 a2 is 1 or 2, b2 is 1 or 2;
(6) 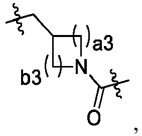 a3 is 1 or 2, b3 is 1 or 2;
(9)

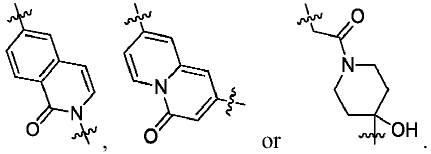

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when W is 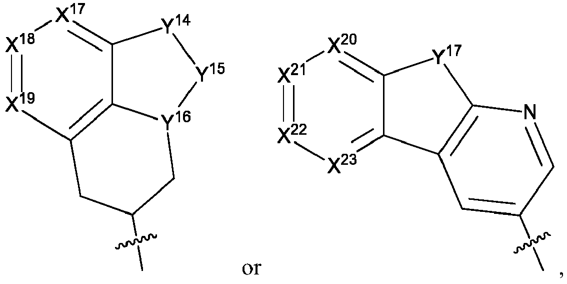 Z is

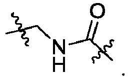

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when W is 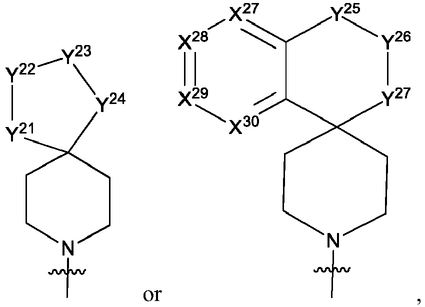 Z is -C(=O)-.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is anyone of the following groups:
(1) 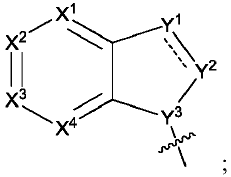 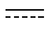
   in 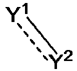 is a single bond, Y¹ is C(=O), O or N-R³, R³ is CH₂COOH or C₁-C₄ alkyl, Y² is C(=N-CO-Ph), C(=N-CO-CH₃), NH, S(=O)₂ or C=S;
   Y³ is N or CH;
   X¹ is N or CR², X² is N or CR⁴, X³ is N or CR⁵, X⁴ is N or CR⁶;
   R², R⁴, R⁵ and R⁶ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-R²⁻¹, -NH-S(=O)₂-R²⁻², -S(=O)₂-NR²⁻³R²⁻⁴, -C(=O)-R²⁻⁵, -NH-C(=O)-R²⁻⁶, or -C(=O)-NR²⁻⁷R²⁻⁸;
   R²⁻¹, R²⁻², R²⁻³, R²⁻⁴, R²⁻⁵, R²⁻⁶, R²⁻⁷, and R²⁻⁸ are independently hydrogen, C₁-C₄ alkyl, or C₁-C₄ alkyl substituted with one or more halogens;
(2) 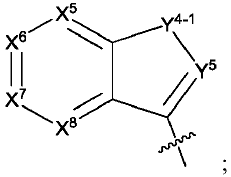
   Y⁴⁻¹ is -NH-C(=O)-CH₂- or NH;
   Y⁵ is N or C-R⁷, R⁷ is halogen;
   X⁵ is N or CR⁸, X⁶ is N or CR⁹, X⁷ is N or CR¹⁰, X⁸ is N or CR¹¹;
   R⁸, R⁹, R¹⁰ and R¹¹ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(3) 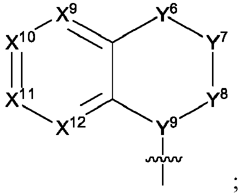
   Y⁶ is N-C(=O)-CH₃, CH₂ or NH;
   Y⁷ is NH, CH₂, C(=O), C(=S) or C(=N-OH);
   Y⁸ is CH₂, CH₂CH₂, CH(CH₃) or C(=O);
   Y⁹ is N or CH;
   X⁹ is N or CR¹² , X¹⁰ is N or CR¹³, X¹¹ is N or CR¹⁴, X¹² is N or CR¹⁵;
   R¹², R¹³, R¹⁴ and R¹⁵ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(4) 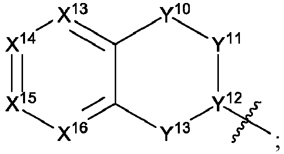
   Y¹⁰ is NH;
   Y¹¹ is C(=O);
   Y¹² is N or CH;
   Y¹³ is CH₂;
   X¹³ is N or CR¹⁶, X¹⁴ is N or CR¹⁷, X¹⁵ is N or CR¹⁸, X¹⁶ is N or CR¹⁹;
   R¹⁶, R¹⁷, R¹⁸ and R¹⁹ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(5) 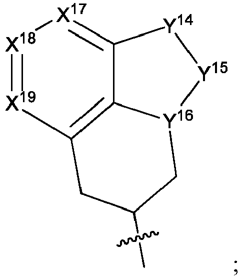
   Y¹⁴ is NH;
   Y¹⁵ is C(=O);
   Y¹⁶ is N or CH;
   X¹⁷ is N or CR²⁰, X¹⁸ is N or CR²¹, X¹⁹ is N or CR²²;
   R²⁰, R²¹ and R²² are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(6) 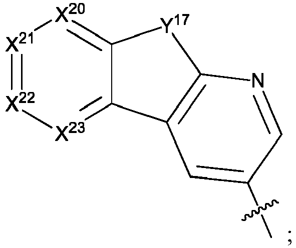
   Y¹⁷ is NH;
   X²⁰ is N or CR²³ , X²¹ is N or CR²⁴, X²² is N or CR²⁵, X²³ is N or CR²⁶;
   R²³, R²⁴, R²⁵ and R²⁶ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(7) 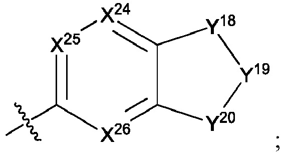
   Y¹⁸ is NH or CH2;
   Y¹⁹ is C(=O) or NH;
   Y²⁰ is CH₂ or C(=O);
   X²⁴ is N or CR²⁷, X²⁵ is N or CR²⁸, X²⁶ is N or CR²⁹;
   R²⁷, R²⁸ and R²⁹ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(8) 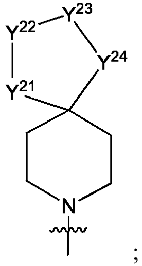
   Y²¹ is C(=O);
   Y²² is NH;
   Y²³ is CH₂ or C(=O);
   Y²⁴ is N-R³⁰, R³⁰ is hydrogen or phenyl;
(9) 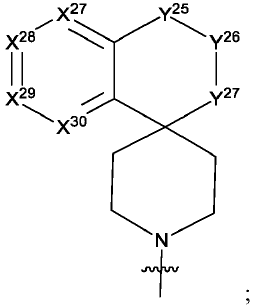
   Y²⁵ is NH;
   Y²⁶ is C(=O);
   Y²⁷ is a single bond, O or NH;
   X²⁷ is N or CR³¹, X²⁸ is N or CR³², X²⁹ is N or CR³³, X³⁰ is N or CR³⁴;
   R³¹, R³², R³³ and R³⁴ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(10) 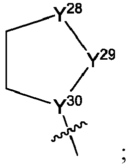
   Y²⁸ is NH;
   Y²⁹ is C(=O);
   Y³⁰ is CH or N;
(11) 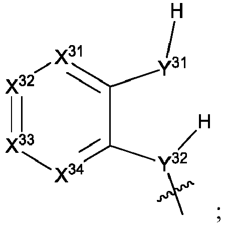
   Y³¹ is O, NH or N-C(=O)-CH₃;
   Y³² is CH or N;
   X³¹ is N or CR³⁵, X³² is N or CR³⁶, X³³ is N or CR³⁷, X³⁴ is N or CR³⁸;
   R³⁵, R³⁶, R³⁷ and R³⁸ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(12) 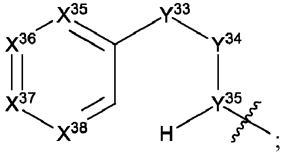
   Y³³ is NH;
   Y³⁴ is C(=O);
   Y³⁵ is CH or N;
   X³⁵ is N or CR³⁹, X³⁶ is N or CR⁴⁰, X³⁷ is N or CR⁴¹, X³⁸ is N or CR⁴²;
   R³⁹, R⁴⁰, R⁴¹ and R⁴² are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(13) 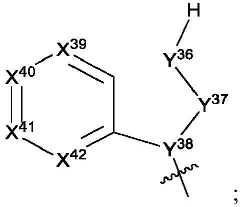
   Y³⁶ is NH;
   Y³⁷ is C(=O);
   Y³⁸ is CH or N;
   X³⁹ is N or CR⁴³, X⁴⁰ is N or CR⁴⁴, X⁴¹ is N or CR⁴⁵, X⁴² is N or CR⁴⁶;
   R⁴³, R⁴⁴, R⁴⁵ and R⁴⁶ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(14) 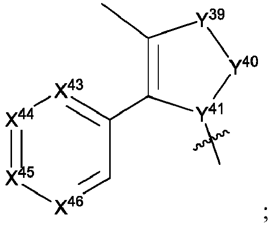
   Y³⁹ is NH;
   Y⁴⁰ is C(=O);
   Y⁴¹ is CH or N;
   X⁴³ is N or CR⁴⁷, X⁴⁴ is N or CR⁴⁸, X⁴⁵ is N or CR⁴⁹, X⁴⁶ is N or CR⁵⁰;
   R⁴⁷, R⁴⁸, R⁴⁹ and R⁵⁰ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂; and
(15)

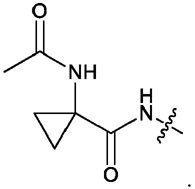

In a certain embodiment, in the compound represented by formula XI, or a pharmaceutically acceptable salt, a solvate, or a solvate of a pharmaceutically acceptable salt thereof, certain groups can be defined as follows, while the remaining groups are as defined in any of the above embodiments:
Z is anyone of the following fragments:
(1) 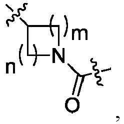 m is 1 or 2, n is 3;
(3) 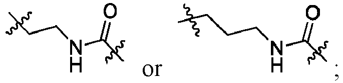
(4) 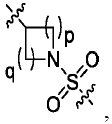 p is 1 or 2, q is 1 or 2;
(5) 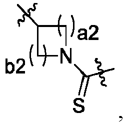 a2 is 1 or 2, b2 is 1 or 2;
(6) 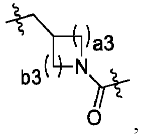 a3 is 1 or 2, b3 is 1 or 2;
(7) 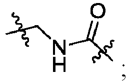
(8) -C(=O)-;
(9)

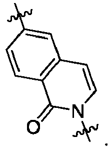

In a certain embodiment, in the compound represented by formula XI, or a pharmaceutically acceptable salt, a solvate, or a solvate of a pharmaceutically acceptable salt thereof, certain groups can be defined as follows, while the remaining groups are as defined in any of the above embodiments:
R¹ is -NH-R¹⁻², 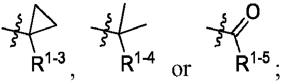
R¹⁻², R¹⁻³, R¹⁻⁴ and R¹⁻⁵ are independently C₇-C₁₀ cycloalkyl, one or more R¹⁻¹⁻³ substituted C₃-C₆ cycloalkyl, one or more R¹⁻¹⁻⁶ substituted 5-6 membered heterocycloalkyl containing 1, 2, or 3 atoms independently selected from N, O and S, one or more R¹⁻¹⁻¹ substituted C₆-C₁₀ aryl, naphthyl, 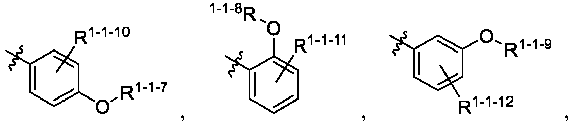 5, 10 membered heteroaryl containing 1, 2, or 3 atoms independently selected from N, O and S, pyridyl, 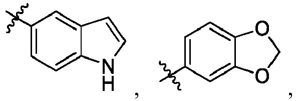 or one or more R¹⁻¹⁻² substituted 5-10 membered heteroaryl containing 1, 2, or 3 atoms independently selected from N, O and S;
R¹⁻¹⁻¹, R¹⁻¹⁻², R¹⁻¹⁻³, and R¹⁻¹⁻⁶ are independently carboxy, hydroxy, halogen, C₁-C₄ alkyl, C₁-C₄ alkyl substituted with one or more halogens, C₁-C₄ alkoxy substituted with one or more halogens, C₃-C₆ cycloalkyl, 5-6 membered heterocycloalkyl containing 1, 2, or 3 atoms independently selected from N, O and S, -N(R¹⁻¹⁻¹⁻¹)(R¹⁻¹⁻¹⁻²), or phenyl;
R¹⁻¹⁻¹ and R¹⁻¹⁻¹⁻² are independently hydrogen or C₁-C₄ alkyl;
R¹⁻¹⁻⁷ , R¹⁻¹⁻⁸, and R¹⁻¹⁻⁹ are independently C₁-C₄ alkyl;
R¹⁻¹⁻¹⁰ is independently C₁-C₄ alkoxy;
R¹⁻¹⁻¹¹ and R¹⁻¹⁻¹² are independently hydrogen or halogen.

In a certain embodiment, in the compound represented by formula **XI**, or a pharmaceutically acceptable salt, a solvate, or a solvate of a pharmaceutically acceptable salt thereof, certain groups can be defined as follows, while the remaining groups are as defined in any of the above embodiments:
R¹ is -(CH₂)ₛ-R¹⁻¹; s is 0, 1 or 2;
R¹⁻¹ is C₇-C₁₀ cycloalkyl, one or more R¹⁻¹⁻³ substituted C₃-C₆ cycloalkyl, one or more R¹⁻¹⁻⁶ substituted 5-6 membered heterocycloalkyl containing 1, 2 or 3 atoms independently selected from N, O and S, one or more R¹⁻¹⁻¹ substituted C₆-C_{1O} aryl, 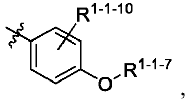 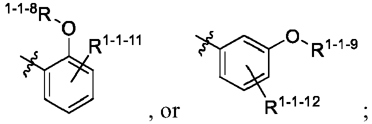
R¹⁻¹⁻¹, R¹⁻¹⁻³, and R¹⁻¹⁻⁶ are independently carboxy, hydroxy, halogen, C₁-C₄ alkoxy substituted with one or more halogens, 5-6 membered heterocycloalkyl containing 1, 2, or 3 atoms independently selected from N, O and S, or -N(R¹⁻¹⁻¹⁻¹)(R¹⁻¹⁻¹⁻²);
R¹⁻¹⁻¹⁻¹ and R¹⁻¹⁻¹⁻² are independently hydrogen or C₁-C₄ alkyl;
R¹⁻¹⁻⁷, R¹⁻¹⁻⁸, and R¹⁻¹⁻⁹ are independently C₁-C₄ alkyl;
R¹⁻¹⁻¹⁰ is independently C₁-C₄ alkoxy;
R¹⁻¹⁻¹¹ and R¹⁻¹⁻¹² are independently hydrogen or halogen.

In a certain embodiment, in the compound represented by formula **XI**, or a pharmaceutically acceptable salt, a solvate, or a solvate of a pharmaceutically acceptable salt thereof, certain groups can be defined as follows, while the remaining groups are as defined in any of the above embodiments:
W is anyone of the following groups:
(1) 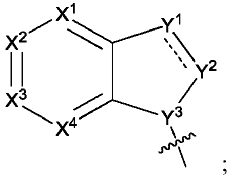 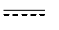
   in 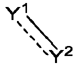 is a single bond or a double bond;
   when 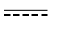 in 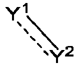 is a single bond, Y¹ is C(=O), O, or N-R³, R³ is hydrogen, hydroxyl, CH₂COOH or C₁-C₄ alkyl, Y² is C(=N-CO-Ph), C(=N-CO-CH₃), NH, S(=O)₂, C=O or C=S;
   when 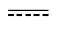 in 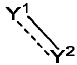 is a double bond, Y¹ is N, and Y² is C-NH₂ or C-NH-CN;
   Y³ is N or CH;
   X¹ is N or CR², X² is N or CR⁴, X³ is N or CR⁵, X⁴ is N or CR⁶;
   R², R⁴, R⁵ and R⁶ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NR²⁻³R²⁻⁴, or -C(=O)-NR²⁻⁷R²⁻⁸;
   R²⁻³, R²⁻⁴, R²⁻⁷ and R²⁻⁸ are independently hydrogen;
(2) 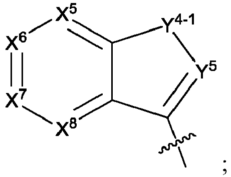
   Y⁴⁻¹ is -NH-C(=O)-CH₂- or NH;
   Y⁵ is N or C-R⁷, R⁷ is halogen;
   X⁵ is CR⁸, X⁶ is CR⁹, X⁷ is CR¹⁰, X⁸ is CR¹¹;
   R⁸, R⁹, R¹⁰ and R¹¹ are independently hydrogen;
(3) 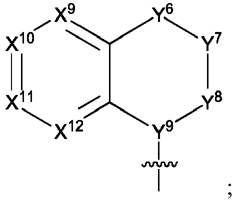
   Y⁶ is N-C(=O)-CH₃, CH₂ or NH;
   Y⁷ is NH, CH₂, C(=O), C(=S) or C(=N-OH);
   Y⁸ is CH₂, CH₂CH₂, CH(CH₃) or C(=O);
   Y⁹ is N;
   X⁹ is CR¹², X¹⁰ is CR¹³, X¹¹ is CR¹⁴, X¹² is CR¹⁵;
   R¹², R¹³, R¹⁴ and R¹⁵ are independently hydrogen, nitro, or C₁-C₄ alkyl substituted with one or more halogens;
(4) 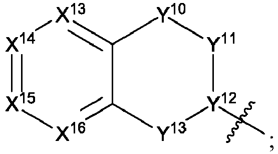
   Y¹⁰ is NH;
   Y¹¹ is C(=O);
   Y¹² is N;
   Y¹³ is CH₂;
   X¹³ is CR¹⁶, X¹⁴ is CR¹⁷, X¹⁵ is CR¹⁸, X¹⁶ is CR¹⁹;
   R¹⁶, R¹⁷, R¹⁸ and R¹⁹ are independently hydrogen;
(5) 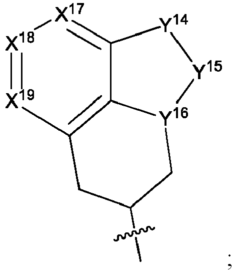
   Y¹⁴ is NH;
   Y¹⁵ is C(=O);
   Y¹⁶ is N;
   X¹⁷ is CR²⁰ , X¹⁸ is CR²¹, X¹⁹ is CR²²;
   R²⁰, R²¹ and R²² are independently hydrogen;
(6) 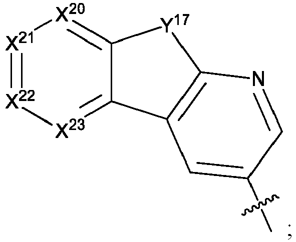
   Y¹⁷ is NH;
   X²⁰ is CR²³ , X²¹ is CR²⁴, X²² is CR²⁵, X²³ is CR²⁶;
   R²³, R²⁴, R²⁵ and R²⁶ are independently hydrogen;
(7) 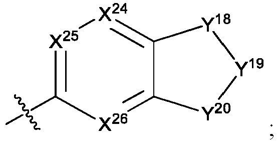
   Y¹⁸ is NH or CH₂;
   Y¹⁹ is C(=O) or NH;
   Y²⁰ is CH₂ or C(=O);
   X²⁴ is CR²⁷ , X²⁵ is CR²⁸, X²⁶ is CR²⁶;
   R²⁷, R²⁸ and R²⁹ are independently hydrogen;
(8) 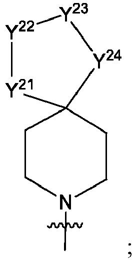
   Y²¹ is C(=O);
   Y²² is NH;
   Y²³ is CH₂ or C(=O);
   Y²⁴ is N-R³⁰, R³⁰ is hydrogen or phenyl;
(9) 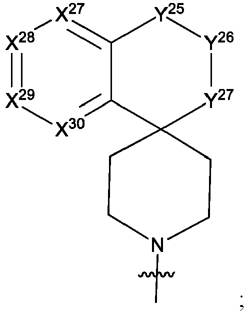
   Y²⁵ is NH;
   Y²⁶ is C(=O);
   Y²⁷ is a single bond, O or NH;
   X²⁷ is CR³¹, X²⁸ is CR³², X²⁹ is CR³³, X³⁰ is CR³⁴;
   R³¹, R³², R³³ and R³⁴ are independently hydrogen;
(10) 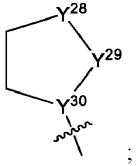
   Y²⁸ is NH;
   Y²⁹ is C(=O);
   Y³⁰ is N;
(11) 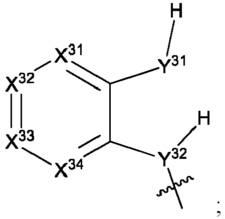
   Y³¹ is O, NH or N-C(=O)-CH₃;
   Y³² is N;
   X³¹ is CR³⁵, X³² is CR³⁶, X³³ is CR³⁷, X³⁴ is CR³⁸;
   R³⁵, R³⁶, R³⁷ and R³⁸ are independently hydrogen;
(12) 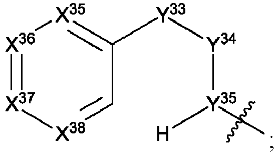
   Y³³ is NH;
   Y³⁴ is C(=O);
   Y³⁵ is N;
   X³⁵ is CR³⁹, X³⁶ is CR⁴⁰, X³⁷ is CR⁴¹, X³⁸ is CR⁴²;
   R³⁹, R⁴⁰, R⁴¹ and R⁴² are independently hydrogen;
(13) 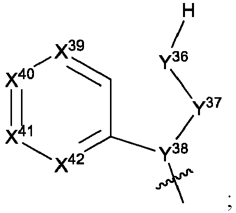
   Y³⁶ is NH;
   Y³⁷ is C(=O);
   Y³⁸ is N;
   X³⁹ is CR⁴³, X⁴⁰ is CR⁴⁴, X⁴¹ is CR⁴⁵, X⁴² is CR⁴⁶;
   R⁴³, R⁴⁴, R⁴⁵ and R⁴⁶ are independently hydrogen;
(14) 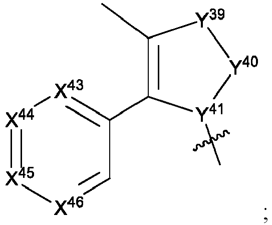
   Y³⁹ is NH;
   Y⁴⁰ is C(=O);
   Y⁴¹ is N;
   X⁴³ is CR⁴⁷, X⁴⁴ is CR⁴⁸, X⁴⁵ is CR⁴⁹, X⁴⁶ is CR⁵⁰;
   R⁴⁷, R⁴⁸, R⁴⁹ and R⁵⁰ are independently hydrogen; and
(15)

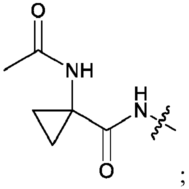

Z is anyone of the following fragments:
(1) 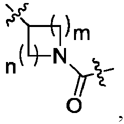 m is 1 or 2, n is 1, 2 or 3;
(2) 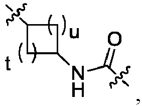 u is 1 or 2, t is 1 or 2;
(3) 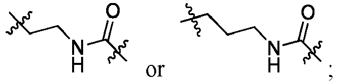
(4) 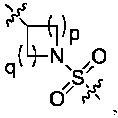 p is 1 or 2, q is 1 or 2;
(5) 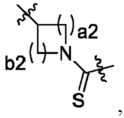 a2 is 1 or 2, b2 is 1 or 2;
(6) 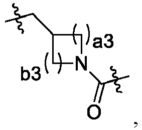 a3 is 1 or 2, b3 is 1 or 2;
(7) 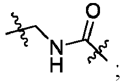
(8) -C(=O)-;
(9) 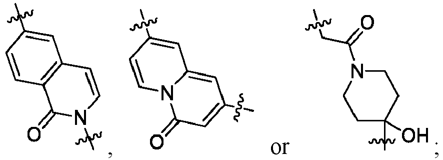
   R¹ is C₂-C₄ alkyl, C₂-C₄ alkyl substituted with one or more halogens, -(CH2)s-R¹⁻¹, -NH-R¹⁻², 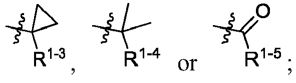 s is 0, 1 or 2;
   R¹⁻¹, R¹⁻², R¹⁻³, R¹⁻⁴ and R¹⁻⁵ are independently C₇-C₁₀ cycloalkyl, one or more R¹⁻¹⁻³ substituted C₃-C₆ cycloalkyl, one or more R¹⁻¹⁻⁶ substituted 5-6 membered heterocycloalkyl containing 1, 2, or 3 atoms independently selected from N, O and S, one or more R¹⁻¹⁻¹ substituted C₆-C_{1O} aryl, naphthyl, 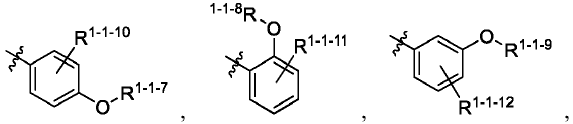 5, 10 membered heteroaryl containing 1, 2, or 3 atoms independently selected from N, O and S, pyridyl, 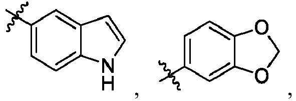 or one or more R¹⁻¹⁻² substituted 5-10 membered heteroaryl containing 1, 2, or 3 atoms independently selected from N, O and S;
   R¹⁻¹⁻¹, R¹⁻¹⁻², R¹⁻¹⁻³, and R¹⁻¹⁻⁶ are independently carboxy, hydroxy, halogen, C₁-C₄ alkyl, C₁-C₄ alkyl substituted with one or more halogens, C₁-C₄ alkoxy substituted with one or more halogens, C₃-C₆ cycloalkyl, 5-6 membered heterocycloalkyl containing 1, 2, or 3 atoms independently selected from N, O and S, -N(R¹⁻¹⁻¹⁻¹)(R¹⁻¹⁻¹⁻²), or phenyl;
   R¹⁻¹⁻¹⁻¹ and R¹⁻¹⁻¹⁻² are independently hydrogen or C₁-C₄ alkyl;
   R¹⁻¹⁻⁷, R¹⁻¹⁻⁸, and R¹⁻¹⁻⁹ are independently C₁-C₄ alkyl;
   R¹⁻¹⁻¹⁰ is independently C₁-C₄ alkoxy;
   R¹⁻¹⁻¹¹ and R¹⁻¹⁻¹² are independently hydrogen or halogen.

In a certain embodiment, in the compound represented by formula **XI**, or a pharmaceutically acceptable salt, a solvate, or a solvate of a pharmaceutically acceptable salt thereof, certain groups can be defined as follows, while the remaining groups are as defined in any of the above embodiments:
W is anyone of the following groups:
(1) 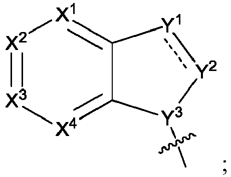 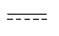
   in 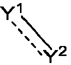 is a single bond or a double bond;
   when 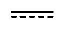 in 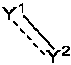 is a single bond, Y¹ is NH or N-OH, Y² is C(=N-CO-Ph), C(=N-CO-CH₃), S(=O)₂, C=O or C=S;
   when 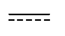 in 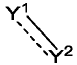 is a double bond, Y¹ is N, Y² is C-NH₂ or C-NH-CN;
   Y³ is N or CH;
   X¹ is N or CR², X² is N or CR⁴, X³ is N or CR⁵, X⁴ is N or CR⁶;
   R², R⁴, R⁵ and R⁶ are independently hydrogen, halogen, hydroxyl, amino, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NR²⁻³R²⁻⁴ or -C(=O)-NR²⁻⁷R²⁻⁸;
   R²⁻³, R²⁻⁴, R²⁻⁷ and R²⁻⁸ are independently hydrogen;
(2) 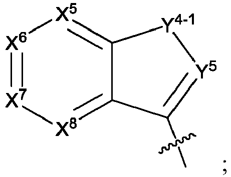
   Y⁴⁻¹ is -NH-C(=O)-CH₂- or NH;
   Y⁵ is N or C-R⁷, R⁷ is halogen;
   X⁵ is CR⁸, X⁶ is CR⁹, X⁷ is CR¹⁰, X⁸ is CR¹¹;
   R⁸, R⁹, R¹⁰ and R¹¹ are independently hydrogen;
(3) 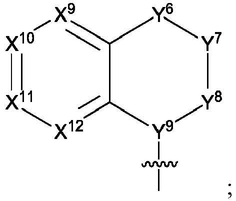
   Y⁶ is N-C(=O)-CH₃ or NH;
   Y⁷ is C(=O), C(=S) or C(=N-OH);
   Y⁸ is CH₂ or C(=O);
   Y⁹ is N;
   X⁹ is CR¹², X¹⁰ is CR¹³, X¹¹ is CR¹⁴, X¹² is CR¹⁵;
   R¹², R¹³, R¹⁴ and R¹⁵ are independently hydrogen, nitro, or C₁-C₄ alkyl substituted with one or more halogens;
(4) 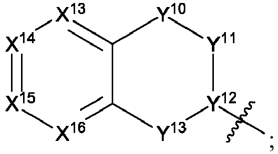
   Y¹⁰ is NH;
   Y¹¹ is C(=O);
   Y¹² is N;
   Y¹³ is CH₂;
   X¹³ is CR¹⁶, X¹⁴ is CR¹⁷, X¹⁵ is CR¹⁸, X¹⁶ is CR¹⁹;
   R¹⁶, R¹⁷ R¹⁸ and R¹⁹ are independently hydrogen;
(5) 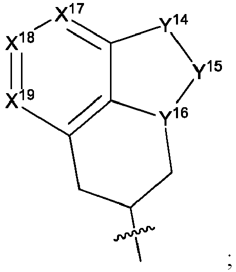
   Y¹⁴ is NH;
   Y¹⁵ is C(=O);
   Y¹⁶ is N;
   X¹⁷ is CR²⁰, X¹⁸ is CR²¹, X¹⁹ is CR²²;
   R²⁰, R²¹ and R²² are independently hydrogen;
(7) 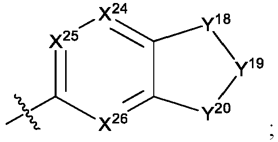
   Y¹⁸ is NH or CH₂;
   Y¹⁹ is C(=O) or NH;
   Y²⁰ is CH₂ or C(=O);
   X²⁴ is CR²⁷, X²⁵ is CR²⁸, X²⁶ is CR²⁹;
   R²⁷, R²⁸ and R²⁹ are independently hydrogen;
(8) 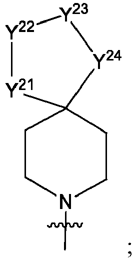
   Y²¹ is C(=O);
   Y²² is NH;
   Y²³ is CH₂;
   Y²⁴ is N-R³⁰, R³⁰ is phenyl;
(9) 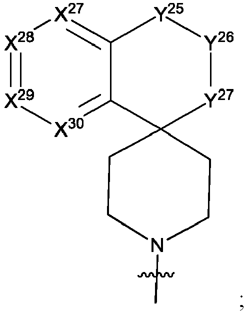
   Y²⁵ is NH;
   Y²⁶ is C(=O);
   Y²⁷ is a single bond, O or NH;
   X²⁷ is CR³¹, X²⁸ is CR³², X²⁹ is CR³³, X³⁰ is CR³⁴;
   R³¹, R³² R³³ and R³⁴ are independently hydrogen;
(11) 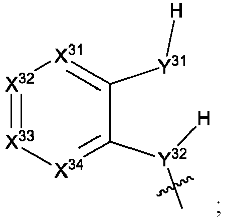
   Y³¹ is O, NH or N-C(=O)-CH₃;
   Y³² is N;
   X³¹ is CR³⁵, X³² is CR³⁶, X³³ is CR³⁷, X³⁴ is CR³⁸;
   R³⁵, R³⁶, R³⁷ and R³⁸ are independently hydrogen;
(12) 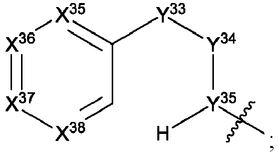
   Y³³ is NH;
   Y³⁴ is C(=O);
   Y³⁵ is N;
   X³⁵ is CR³⁹, X³⁶ is CR⁴⁰, X³⁷ is CR⁴¹, X³⁸ is CR⁴²;
   R³⁹, R⁴⁰, R⁴¹ and R⁴² are independently hydrogen;
(13) 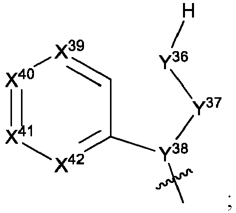
   Y³⁶ is NH;
   Y³⁷ is C(=O);
   Y³⁸ is N;
   X³⁹ is CR⁴³, X⁴⁰ is CR⁴⁴, X⁴¹ is CR⁴⁵, X⁴² is CR⁴⁶;
   R⁴³, R⁴⁴, R⁴⁵ and R⁴⁶ are independently hydrogen; and
(14) 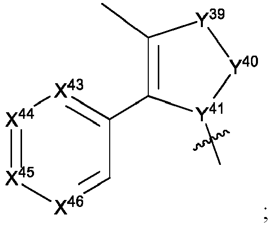
   Y³⁹ is NH;
   Y⁴⁰ is C(=O);
   Y⁴¹ is N;
   X⁴³ is CR⁴⁷, X⁴⁴ is CR⁴⁸, X⁴⁵ is CR⁴⁹, X⁴⁶ is CR⁵⁰;
   R⁴⁷, R⁴⁸, R⁴⁹ and R⁵⁰ are independently hydrogen;

Z is anyone of the following fragments:
(1) 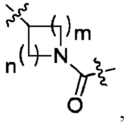 m is 1 or 2, n is 1, 2 or 3;
(2) 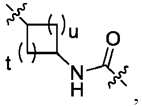 u is 1 or 2, t is 1 or 2;
(3) 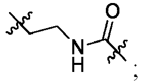
(4) 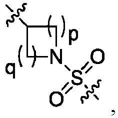 p is 1 or 2, q is 1 or 2;
(5) 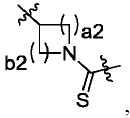 a2 is 1 or 2, b2 is 1 or 2;
(6) 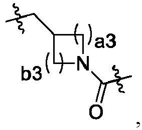 a3 is 1 or 2, b3 is 1 or 2;
(7) 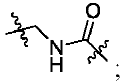
(8) -C(=O)-;
(9) 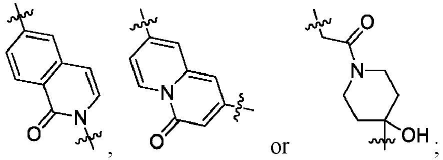
   R¹ is -(CH₂)ₛ-R¹⁻¹, -NH-R¹⁻², 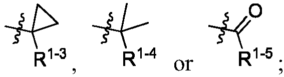 s is 0, 1 or 2;
   R¹⁻¹, R¹⁻², R¹⁻³, R¹⁻⁴ and R¹⁻⁵ are independently one or more R¹⁻¹⁻³ substituted C₃-C₆ cycloalkyl, one or more R¹⁻¹⁻¹ substituted C₆-C₁₀ aryl, naphthyl, 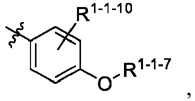 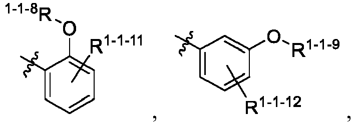 5, 10 membered heteroaryl containing 1, 2 or 3 atoms independently selected from N, O and S, pyridyl, 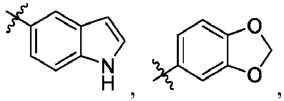 or one or more R¹⁻¹⁻² substituted 5-10 membered heteroaryl containing 1, 2 or 3 atoms independently selected from N, O and S;
   R¹⁻¹⁻¹, R¹⁻¹⁻², and R¹⁻¹⁻³ are independently carboxy, hydroxy, halogen, C₁-C₄ alkyl, C₁-C₄ alkyl substituted with one or more halogens, C₁-C₄ alkoxy substituted with one or more halogens, C₃-C₆ cycloalkyl, 5-6 membered heterocycloalkyl containing 1, 2, or 3 atoms independently selected from N, O and S, -N(R¹⁻¹⁻¹⁻¹)(R¹⁻¹⁻¹⁻²), or phenyl;
   R¹⁻¹⁻¹⁻¹ and R¹⁻¹⁻¹⁻² are independently hydrogen or C₁-C₄ alkyl;
   R¹⁻¹⁻⁷, R¹⁻¹⁻⁸, and R¹⁻¹⁻⁹ are independently C₁-C₄ alkyl;
   R¹⁻¹⁻¹⁰ is independently C₁-C₄ alkoxy;
   R¹⁻¹⁻¹¹ and R¹⁻¹⁻¹² are independently hydrogen or halogen.

In a certain embodiment, in the compound represented by formula **XI**, or a pharmaceutically acceptable salt, a solvate, or a solvate of a pharmaceutically acceptable salt thereof, certain groups can be defined as follows, while the remaining groups are as defined in any of the above embodiments:
R¹⁻¹, R¹⁻², R¹⁻³, R¹⁻⁴ and R¹⁻⁵ are independently one or more R¹⁻¹⁻³ substituted C₃-C₆ cycloalkyl, one or more R¹⁻¹⁻⁶ substituted 5-6 membered heterocycloalkyl containing 1, 2 or 3 atoms independently selected from N, O and S, one or more R¹⁻¹⁻¹ substituted C₆-C₁₀ aryl, or one or more R¹⁻¹⁻² substituted 5-10 membered heteroaryl containing 1, 2 or 3 atoms independently selected from N, O and S;
R¹⁻¹⁻¹, R¹⁻¹⁻², R¹⁻¹⁻³, and R¹⁻¹⁻⁶ are independently carboxy, hydroxy, halogen, C₁-C₄ alkyl, C₁-C₄ alkyl substituted with one or more halogens, C₁-C₄ alkoxy substituted with one or more halogens, C₃-C₆ cycloalkyl, 5-6 membered heterocycloalkyl containing 1, 2, or 3 atoms independently selected from N, O and S, -N(R¹⁻¹⁻¹⁻¹)(R¹⁻¹⁻¹⁻²), or phenyl.

In a certain embodiment, in the compound represented by formula **XI**, or a pharmaceutically acceptable salt, a solvate, or a solvate of a pharmaceutically acceptable salt thereof, certain groups can be defined as follows, while the remaining groups are as defined in any of the above embodiments:
R¹⁻¹, R¹⁻², R¹⁻³, R¹⁻⁴ and R¹⁻⁵ are independently one or more R¹⁻¹⁻³ substituted C₃-C₆ cycloalkyl, one or more R¹⁻¹⁻⁶ substituted 5-6 membered heterocycloalkyl containing 1, 2 or 3 atoms independently selected from N, O and S, one or more R¹⁻¹⁻¹ substituted C₆-C₁₀ aryl, or one or more R¹⁻¹⁻² substituted 5-10 membered heteroaryl containing 1, 2 or 3 atoms independently selected from N, O and S;
R¹⁻¹⁻¹, R¹⁻¹⁻², R¹⁻¹⁻³, and R¹⁻¹⁻⁶ are independently carboxy.

In a certain embodiment, in the compound represented by formula **XI**, or a pharmaceutically acceptable salt, a solvate, or a solvate of a pharmaceutically acceptable salt thereof, certain groups can be defined as follows, while the remaining groups are as defined in any of the above embodiments:
R¹⁻¹, R¹⁻², R¹⁻³, R¹⁻⁴ and R¹⁻⁵ are independently one or more R¹⁻¹⁻³ substituted C₃-C₆ cycloalkyl, one or more R¹⁻¹⁻⁶ substituted 5-6 membered heterocycloalkyl containing 1, 2 or 3 atoms independently selected from N, O and S, one or more R¹⁻¹⁻¹ substituted C₆-C_{1O} aryl, or one or more R¹⁻¹⁻² substituted 5-10 membered heteroaryl containing 1, 2 or 3 atoms independently selected from N, O and S;
R¹⁻¹⁻¹, R¹⁻¹⁻², R¹⁻¹⁻³, and R¹⁻¹⁻⁶ are independently hydroxy.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
R¹⁻¹, R¹⁻², R¹⁻³, R¹⁻⁴ and R¹⁻⁵ are independently one or more R¹⁻¹⁻³ substituted C₃-C₆ cycloalkyl, one or more R¹⁻¹⁻⁶ substituted 5-6 membered heterocycloalkyl containing 1, 2 or 3 atoms independently selected from N, O and S, one or more R¹⁻¹⁻¹ substituted C₆-C₁₀ aryl, or one or more R¹⁻¹⁻² substituted 5-10 membered heteroaryl containing 1, 2 or 3 atoms independently selected from N, O and S;
R¹⁻¹⁻¹, R¹⁻¹⁻², R¹⁻¹⁻³, and R¹⁻¹⁻⁶ are independently halogen.

In a certain embodiment, in the compound represented by formula **XI**, or a pharmaceutically acceptable salt, a solvate, or a solvate of a pharmaceutically acceptable salt thereof, certain groups can be defined as follows, while the remaining groups are as defined in any of the above embodiments:
R¹⁻¹, R¹⁻², R¹⁻³, R¹⁻⁴ and R¹⁻⁵ are independently one or more R¹⁻¹⁻³ substituted C₃-C₆ cycloalkyl, one or more R¹⁻¹⁻⁶ substituted 5-6 membered heterocycloalkyl containing 1, 2 or 3 atoms independently selected from N, O and S, one or more R¹⁻¹⁻¹ substituted C₆-C_{1O} aryl, or one or more R¹⁻¹⁻² substituted 5-10 membered heteroaryl containing 1, 2 or 3 atoms independently selected from N, O and S;
R¹⁻¹⁻¹, R¹⁻¹⁻², R¹⁻¹⁻³, and R¹⁻¹⁻⁶ are independently C₁-C₄ alkyl or C₁-C₄ alkyl substituted with one or more halogens.

In a certain embodiment, in the compound represented by formula **XI**, or a pharmaceutically acceptable salt, a solvate, or a solvate of a pharmaceutically acceptable salt thereof, certain groups can be defined as follows, while the remaining groups are as defined in any of the above embodiments:
R¹⁻¹, R¹⁻², R¹⁻³, R¹⁻⁴ and R¹⁻⁵ are independently one or more R¹⁻¹⁻³ substituted C₃-C₆ cycloalkyl, one or more R¹⁻¹⁻⁶ substituted 5-6 membered heterocycloalkyl containing 1, 2 or 3 atoms independently selected from N, O and S, one or more R¹⁻¹⁻¹ substituted C₆-C₁₀ aryl, or one or more R¹⁻¹⁻² substituted 5-10 membered heteroaryl containing 1, 2 or 3 atoms independently selected from N, O and S;
R¹⁻¹⁻¹, R¹⁻¹⁻², R¹⁻¹⁻³, and R¹⁻¹⁻⁶ are independently C₁-C₄ alkyl substituted with one or more halogens.

In a certain embodiment, in the compound represented by formula **XI**, or a pharmaceutically acceptable salt, a solvate, or a solvate of a pharmaceutically acceptable salt thereof, certain groups can be defined as follows, while the remaining groups are as defined in any of the above embodiments:
R¹⁻¹, R¹⁻², R¹⁻³, R¹⁻⁴ and R¹⁻⁵ are independently one or more R¹⁻¹⁻³ substituted C₃-C₆ cycloalkyl, one or more R¹⁻¹⁻⁶ substituted 5-6 membered heterocycloalkyl containing 1, 2 or 3 atoms independently selected from N, O and S, one or more R¹⁻¹⁻¹ substituted C₆-C_{1O} aryl, or one or more R¹⁻¹⁻² substituted 5-10 membered heteroaryl containing 1, 2 or 3 atoms independently selected from N, O and S;
R¹⁻¹⁻¹, R¹⁻¹⁻², R¹⁻¹⁻³, and R¹⁻¹⁻⁶ are independently trifluoromethyl.

In a certain embodiment, in the compound represented by formula **XI**, or a pharmaceutically acceptable salt, a solvate, or a solvate of a pharmaceutically acceptable salt thereof, certain groups can be defined as follows, while the remaining groups are as defined in any of the above embodiments:
R¹⁻¹, R¹⁻², R¹⁻³, R¹⁻⁴ and R¹⁻⁵ are independently one or more R¹⁻¹⁻³ substituted C₃-C₆ cycloalkyl, one or more R¹⁻¹⁻⁶ substituted 5-6 membered heterocycloalkyl containing 1, 2 or 3 atoms independently selected from N, O and S, one or more R¹⁻¹⁻¹ substituted C₆-C₁₀ aryl, or one or more R¹⁻¹⁻² substituted 5-10 membered heteroaryl containing 1, 2 or 3 atoms independently selected from N, O and S;
R¹⁻¹⁻¹, R¹⁻¹⁻², R¹⁻¹⁻³, and R¹⁻¹⁻⁶ are independently C₁-C₄ alkoxy substituted with one or more halogens.

In a certain embodiment, in the compound represented by formula **XI**, or a pharmaceutically acceptable salt, a solvate, or a solvate of a pharmaceutically acceptable salt thereof, certain groups can be defined as follows, while the remaining groups are as defined in any of the above embodiments:
R¹⁻¹, R¹⁻², R¹⁻³, R¹⁻⁴ and R¹⁻⁵ are independently one or more R¹⁻¹⁻³ substituted C₃-C₆ cycloalkyl, one or more R¹⁻¹⁻⁶ substituted 5-6 membered heterocycloalkyl containing 1, 2 or 3 atoms independently selected from N, O and S, one or more R¹⁻¹⁻¹ substituted C₆-C_{1O} aryl, or one or more R¹⁻¹⁻² substituted 5-10 membered heteroaryl containing 1, 2 or 3 atoms independently selected from N, O and S;
R¹⁻¹⁻¹, R¹⁻¹⁻², R¹⁻¹⁻³, and R¹⁻¹⁻⁶ are independently C₃-C₆ cycloalkyl or 5-6 membered heterocycloalkyl containing 1, 2, or 3 atoms independently selected from N, O and S.

In a certain embodiment, in the compound represented by formula **XI**, or a pharmaceutically acceptable salt, a solvate, or a solvate of a pharmaceutically acceptable salt thereof, certain groups can be defined as follows, while the remaining groups are as defined in any of the above embodiments:
R¹⁻¹, R¹⁻², R¹⁻³, R¹⁻⁴ and R¹⁻⁵ are independently one or more R¹⁻¹⁻³ substituted C₃-C₆ cycloalkyl, one or more R¹⁻¹⁻⁶ substituted 5-6 membered heterocycloalkyl containing 1, 2 or 3 atoms independently selected from N, O and S, one or more R¹⁻¹⁻¹ substituted C₆-C₁₀ aryl, or one or more R¹⁻¹⁻² substituted 5-10 membered heteroaryl containing 1, 2 or 3 atoms independently selected from N, O and S;
R¹⁻¹⁻¹, R¹⁻¹⁻², R¹⁻¹⁻³, and R¹⁻¹⁻⁶ are independently phenyl.

In a certain embodiment, in the compound represented by formula **XI**, or a pharmaceutically acceptable salt, a solvate, or a solvate of a pharmaceutically acceptable salt thereof, certain groups can be defined as follows, while the remaining groups are as defined in any of the above embodiments:
R¹⁻¹, R¹⁻², R¹⁻³, R¹⁻⁴ and R¹⁻⁵ are independently one or more R¹⁻¹⁻³ substituted C₃-C₆ cycloalkyl, one or more R¹⁻¹⁻⁶ substituted 5-6 membered heterocycloalkyl containing 1, 2 or 3 atoms independently selected from N, O and S, one or more R¹⁻¹⁻¹ substituted C₆-C_{1O} aryl, or one or more R¹⁻¹⁻² substituted 5-10 membered heteroaryl containing 1, 2 or 3 atoms independently selected from N, O and S;
R¹⁻¹⁻¹, R¹⁻¹⁻², R¹⁻¹⁻³, and R¹⁻¹⁻⁶ are independently -N(R¹⁻¹⁻¹⁻¹)(R¹⁻¹⁻¹⁻²).

In a certain embodiment, in the compound represented by formula **XI**, or a pharmaceutically acceptable salt, a solvate, or a solvate of a pharmaceutically acceptable salt thereof, certain groups can be defined as follows, while the remaining groups are as defined in any of the above embodiments:
R¹⁻¹, R¹⁻², R¹⁻³, R¹⁻⁴ and R¹⁻⁵ are independently C₇-C₁₀ cycloalkyl.

In a certain embodiment, in the compound represented by formula **XI**, or a pharmaceutically acceptable salt, a solvate, or a solvate of a pharmaceutically acceptable salt thereof, certain groups can be defined as follows, while the remaining groups are as defined in any of the above embodiments:
R¹⁻¹, R¹⁻², R¹⁻³, R¹⁻⁴ and R¹⁻⁵ are independently naphthyl.

In a certain embodiment, in the compound represented by formula **XI**, or a pharmaceutically acceptable salt, a solvate, or a solvate of a pharmaceutically acceptable salt thereof, certain groups can be defined as follows, while the remaining groups are as defined in any of the above embodiments:
R¹⁻¹, R¹⁻², R¹⁻³, R¹⁻⁴ and R¹⁻⁵ are independently

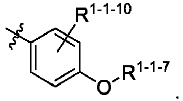

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
R¹⁻¹, R¹⁻², R¹⁻³, R¹⁻⁴ and R¹⁻⁵ are independently

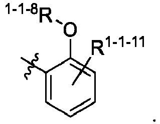

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
R¹⁻¹, R¹⁻², R¹⁻³, R¹⁻⁴ and R¹⁻⁵ are independently

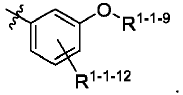

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
R¹⁻¹, R¹⁻², R¹⁻³, R¹⁻⁴ and R¹⁻⁵ are independently 5, 10 membered heteroaryl containing 1, 2 or 3 atoms independently selected from N, O and S.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
R¹⁻¹, R¹⁻², R¹⁻³, R¹⁻⁴ and R¹⁻⁵ are independently pyridyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
R¹⁻¹, R¹⁻², R¹⁻³, R¹⁻⁴ and R¹⁻⁵ are independently

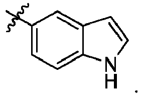

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
R¹⁻¹, R¹⁻², R¹⁻³, R¹⁻⁴ and R¹⁻⁵ are independently

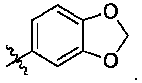

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is

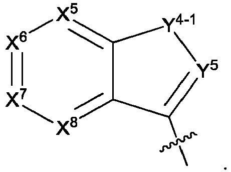

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is 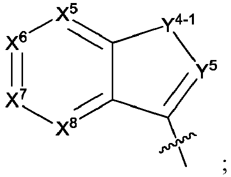
Y⁴⁻¹ is -NH-C(=O)-CH₂- or NH;
Y⁵ is N or C-R⁷, R⁷ is halogen;
X⁵ is CR⁸, X⁶ is CR⁹, X⁷ is CR¹⁰, X⁸ is CR¹¹;
R⁸, R⁹, R¹⁰ and R¹¹ are independently hydrogen.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is

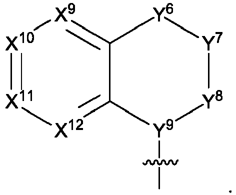

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is 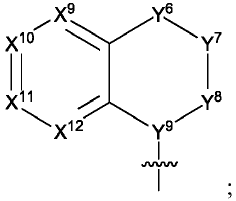
Y⁶ is N-C(=O)-CH₃, CH₂ or NH;
Y⁷ is NH, CH₂, C(=O), C(=S) or C(=N-OH);
Y⁸ is CH₂, CH₂CH₂, CH(CH₃) or C(=O);
Y⁹ is N;
X⁹ is CR¹², X¹⁰ is CR¹³, X¹¹ is CR¹⁴, X¹² is CR¹⁵;
R¹², R¹⁻³, R¹⁴ and R¹⁵ are independently hydrogen, nitro, or C₁-C₄ alkyl substituted with one or more halogens.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is

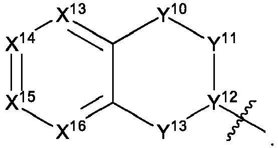

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is 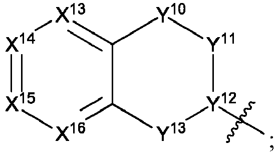
Y¹⁰ is NH;
Y¹¹ is C(=O);
Y¹² is N;
Y¹³ is CH₂;
X¹³ is CR¹⁶, X¹⁴ is CR¹⁷, X¹⁵ is CR¹⁸, X¹⁶ is CR¹⁹;
R¹⁶, R¹⁷ R¹⁸ and R¹⁹ are independently hydrogen.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is

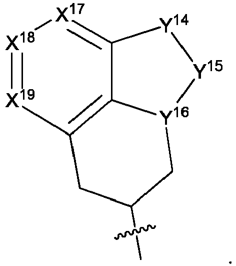

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is 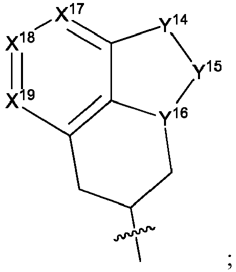
Y¹⁴ is NH;
Y¹⁵ is C(=O);
Y¹⁶ is N;
X¹⁷ is CR²⁰, X¹⁸ is CR²¹, X¹⁹ is CR²²;
R²⁰, R²¹ and R²² are independently hydrogen.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is

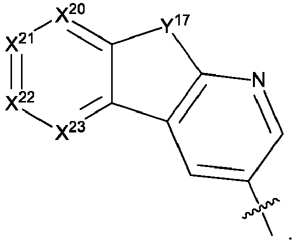

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is

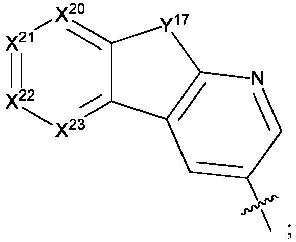

Y¹⁷ is NH;
X²⁰ is CR²³, X²¹ is CR²⁴, X²² is CR²⁵, X²³ is CR²⁶;
R²³, R²⁴, R²⁵ and R²⁶ are independently hydrogen.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is

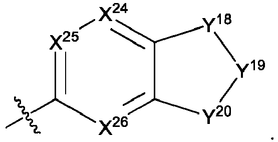

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is 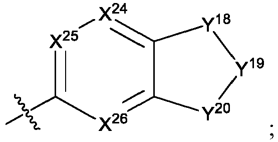
Y¹⁸ is NH or CH₂;
Y¹⁹ is C(=O) or NH;
Y²⁰ is CH₂ or C(=O);
X²⁴ is CR²⁷, X²⁵ is CR²⁸, X²⁶ is CR²⁹;
R²⁷, R²⁸ and R²⁹ are independently hydrogen.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is

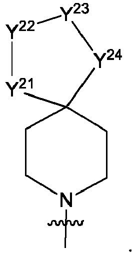

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is 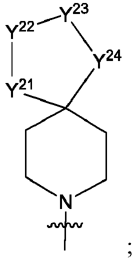
Y²¹ is C(=O);
Y²² is NH;
Y²³ is CH₂ or C(=O);
Y²⁴ is N-R³⁰, R³⁰ is hydrogen or phenyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is

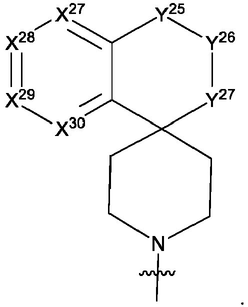

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is 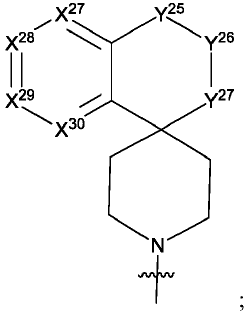
Y²⁵ is NH;
Y²⁶ is C(=O);
Y²⁷ is a single bond, O or NH;
X²⁷ is CR³¹, X²⁸ is CR³², X²⁹ is CR³³, X³⁰ is CR³⁴;
R³¹, R³², R³³ and R³⁴ are independently hydrogen.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is

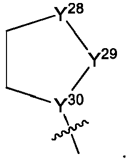

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is 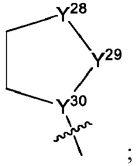
Y²⁸ is NH;
Y²⁹ is C(=O);
Y³⁰ is N.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is

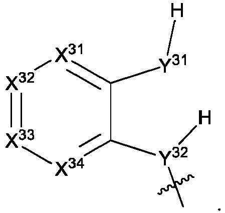

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is 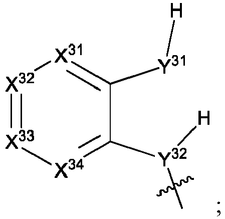
Y³¹ is O, NH or N-C(=O)-CH₃;
Y³² is N;
X³¹ is CR³⁵, X³² is CR³⁶, X³³ is CR³⁷ , X³⁴ is CR³⁸;
R³⁵, R³⁶, R³⁷ and R³⁸ are independently hydrogen.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is

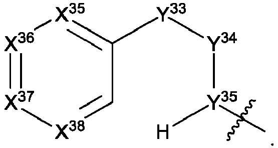

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is 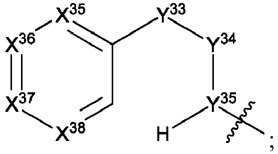
Y³³ is NH;
Y³⁴ is C(=O);
Y³⁵ is N;
X³⁵ is CR³⁹, X³⁶ is CR⁴⁰, X³⁷ is CR⁴¹, X³⁸ is CR⁴²;
R³⁹, R⁴⁰, R⁴¹ and R⁴² are independently hydrogen.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is

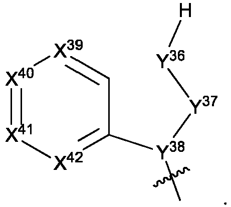

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is 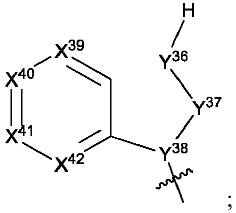
Y³⁶ is NH;
Y³⁷ is C(=O);
Y³⁸ is N;
X³⁹ is CR⁴³, X⁴⁰ is CR⁴⁴, X⁴¹ is CR⁴⁵, X⁴² is CR⁴⁶;
R⁴³, R⁴⁴, R⁴⁵ and R⁴⁶ are independently hydrogen.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is

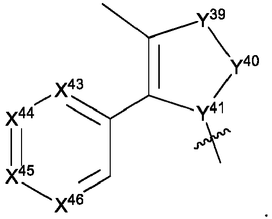

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is 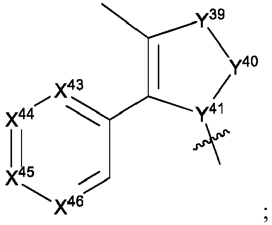
Y³⁹ is NH;
Y⁴⁰ is C(=O);
Y⁴¹ is N;
X⁴³ is CR⁴⁷, X⁴⁴ is CR⁴⁸, X⁴⁵ is CR⁴⁹, X⁴⁶ is CR⁵⁰;
R⁴⁷, R⁴⁸, R⁴⁹ and R⁵⁰ are independently hydrogen.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
W is

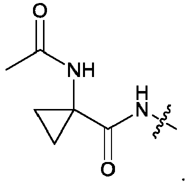

In a certain embodiment, in the compound represented by formula **XI,** or a pharmaceutically acceptable salt, a solvate, or a solvate of a pharmaceutically acceptable salt thereof, if the compound represented by formula **XI** has optical isomers, it may exist in the form of racemates.

In a certain embodiment, the atoms in the compound represented by formula **XI,** or a pharmaceutically acceptable salt, a solvate, or a solvate of a pharmaceutically acceptable salt thereof all exist in their natural abundance.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R³ is C₁-C₄ alkyl, said C₁-C₄ alkyl is methyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R², R⁴, R⁵ and R⁶ are independently halogen, said halogen is fluorine, chlorine or bromine.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R², R⁴, R⁵ and R⁶ are independently C₁-C₄ alkyl, said C₁-C₄ alkyl is methyl or tert-butyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R², R⁴, R⁵ and R⁶ are independently C₁-C₄ alkyl, said C₁-C₄ alkyl is tert-butyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R², R⁴, R⁵ and R⁶ are independently C₁-C₄ alkyl substituted with a plurality of halogens, said plurality of halogens are 2 or 3 halogens.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R², R⁴, R⁵ and R⁶ are independently C₁-C₄ alkyl substituted with one or more halogens, said halogen is fluorine.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R², R⁴, R⁵ and R⁶ are independently C₁-C₄ alkyl substituted with one or more halogens, said C₁-C₄ alkyl is methyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R², R⁴, R⁵ and R⁶ are independently C₁-C₄ alkyl substituted with a plurality of halogens, said C₁-C₄ alkyl substituted with a plurality of halogens is trifluoromethyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R², R⁴, R⁵ and R⁶ are independently C₁-C₄ alkoxy, said C₁-C₄ alkoxy is methoxy.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R², R⁴, R⁵ and R⁶ are independently C₃-C₆ cycloalkyl, said C₃-C₆ cycloalkyl is cyclopropyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R², R⁴, R⁵ and R⁶ are independently 5-7 membered heterocycloalkyl containing 1, 2 or 3 atoms independently selected from N, O and S, said 5-7 membered heterocycloalkyl containing 1, 2 or 3 atoms independently selected from N, O and S can be morpholinyl, or alternatively can be morpholin-1-yl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R²⁻¹, R²⁻², R²⁻³, R²⁻⁴, R²⁻⁵, R²⁻⁶, R²⁻⁷ and R²⁻⁸ are independently C₁-C₄ alkyl, said C₁-C₄ alkyl is methyl or tert-butyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R²⁻¹, R²⁻², R²⁻³, R²⁻⁴, R²⁻⁵, R²⁻⁶, R²⁻⁷ and R²⁻⁸ are independently C₁-C₄ alkyl substituted with a plurality of halogens, said plurality of halogens are 2 or 3 halogens.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R²⁻¹, R²⁻², R²⁻³, R²⁻⁴, R²⁻⁵, R²⁻⁶, R²⁻⁷ and R²⁻⁸ are independently C₁-C₄ alkyl substituted with one or more halogens, said halogen is fluorine.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R²⁻¹, R²⁻², R²⁻³, R²⁻⁴, R²⁻⁵, R²⁻⁶, R²⁻⁷ and R²⁻⁸ are independently C₁-C₄ alkyl substituted with one or more halogens, said C₁-C₄ alkyl is methyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R²⁻¹, R²⁻², R²⁻³, R²⁻⁴, R²⁻⁵, R²⁻⁶, R ²⁻⁷ and R²⁻⁸ are independently C₁-C₄ alkyl substituted with a plurality of halogens, said C₁-C₄ alkyl substituted with a plurality of halogens is trifluoromethyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when W is 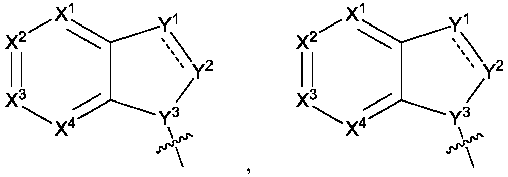 can be

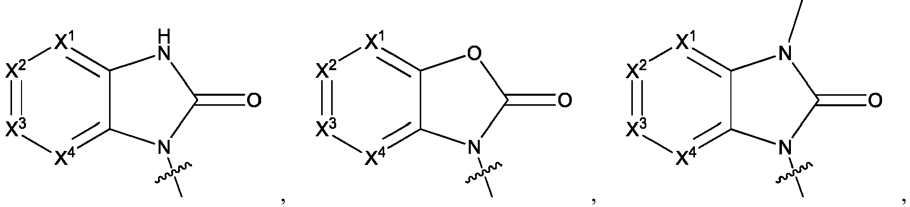

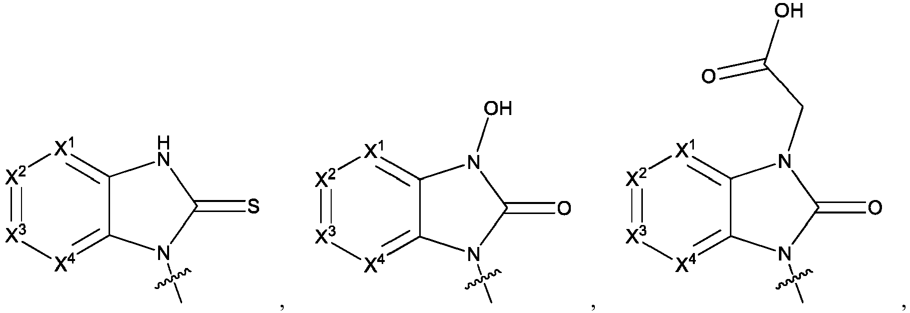

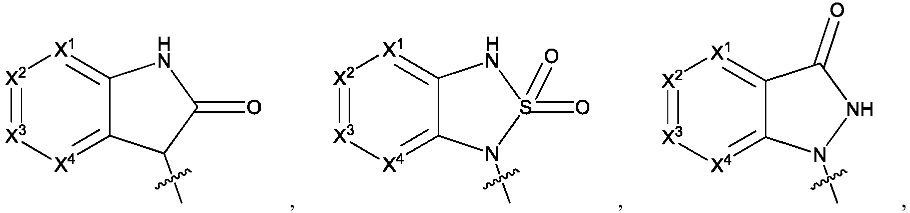

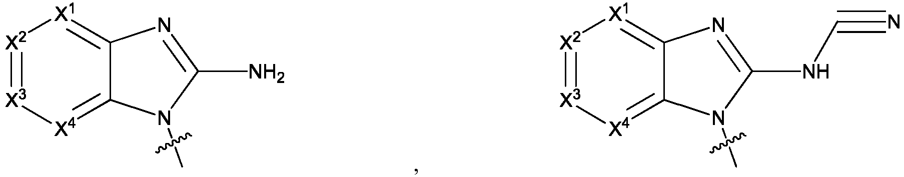

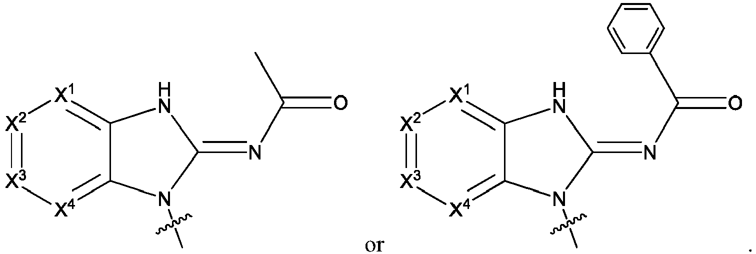

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Y⁴⁻¹ is -NH-C(=O)-CH₂-, the -CH₂- end can be connected with Y⁵.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when W is 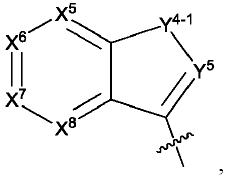 the 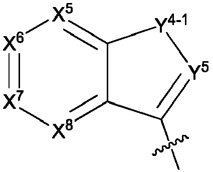 can be 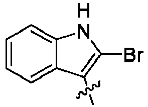 or

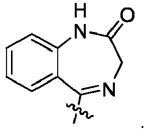

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when W is 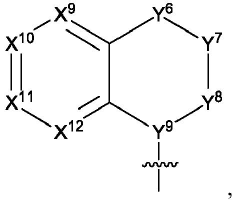 the 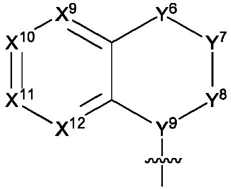 can be 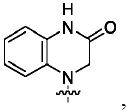 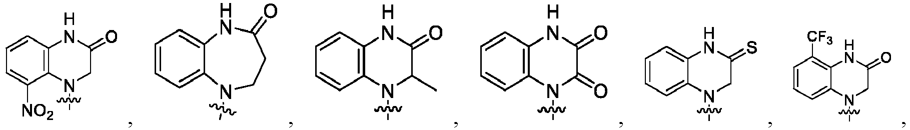 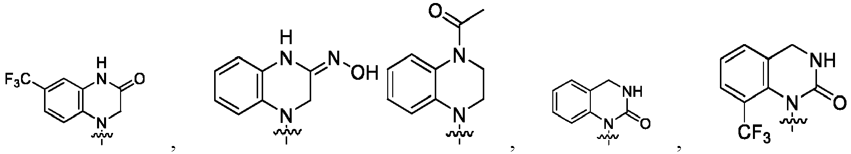 or

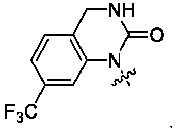

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when W is 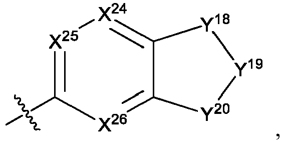 the 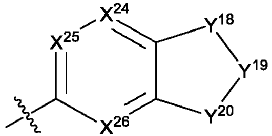 can be

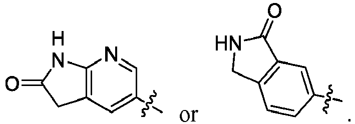

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when W is 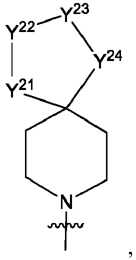 the 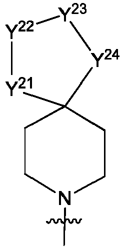 can be 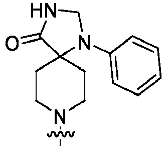 or

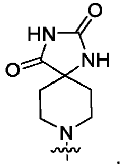

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when W is 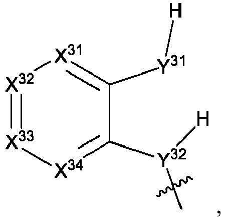 the 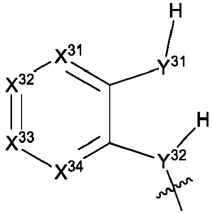 can be 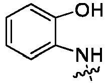 or

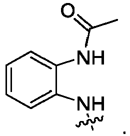

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 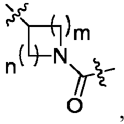 m is 1, n is 1.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 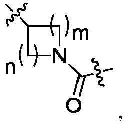 m is 2, n is 2.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 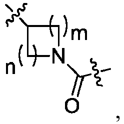 the 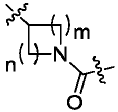 is 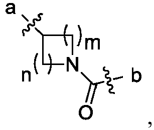 whose b end is connected with R¹.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 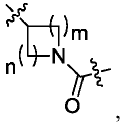 m is 1, n is 1, the 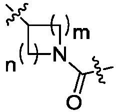 is 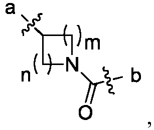 whose b end is connected with R¹.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 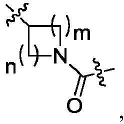 m is 2, n is 2, the 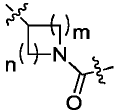 is 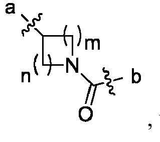 whose b end is connected with R¹.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 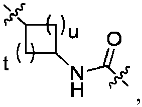 u is 1, t is 1.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 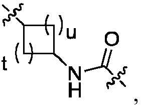 u is 2, t is 2.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 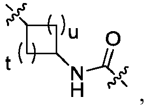 the 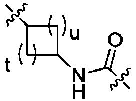 is 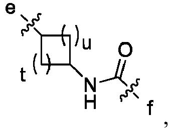 whose f end is connected with R¹;
or alternatively it can be 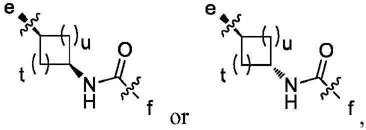 whose f end is connected with R¹.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 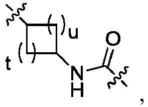 the 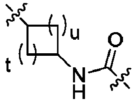 is 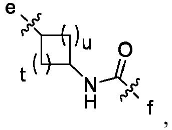 whose f end is connected with R¹, and u is 1, t is 1;
or alternatively it can be 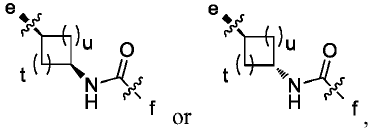 whose f end is connected with R¹, and u is 1, t is 1.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments: when Z is 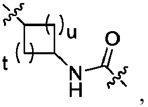 the 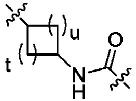 is 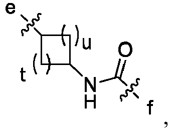 whose f end is connected with R¹, and u is 2, t is 2;
or alternatively it can be 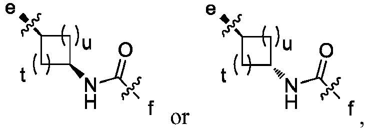 whose f end is connected with R¹, and u is 2, t is 2.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 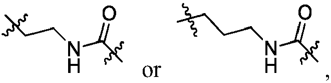 the carbonyl end can be connected with R¹.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 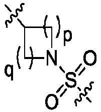 , p is 2, q is 2.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 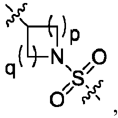 the 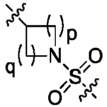 is 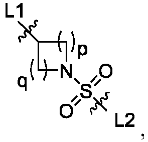 whose L2 end is connected with R¹.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 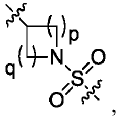 p is 2, q is 2, the 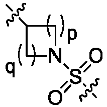 is 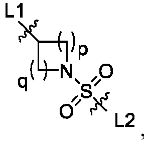 whose L2 end is connected with R¹.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 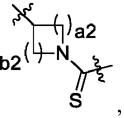 a2 is 2, b2 is 2.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 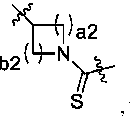 the 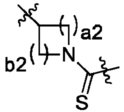 is 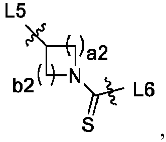 whose L6 end is connected with R¹.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 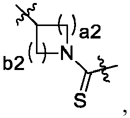 a2 is 2, b2 is 2, the 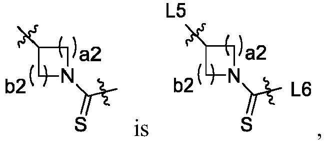 whose L6 end is connected with R¹.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 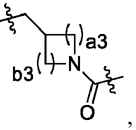 a3 is 2, b3 is 2.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 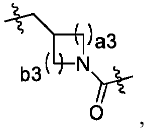 the 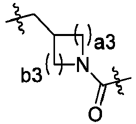 is 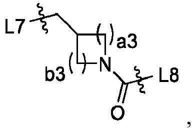 whose L8 end is connected with R¹.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 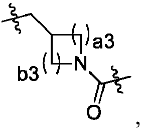 a3 is 2, b3 is 2, the 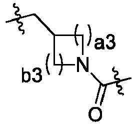 is 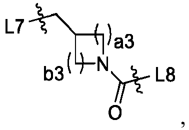 whose L8 end is connected with R¹.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 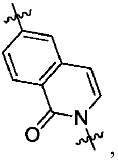 the 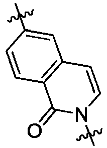 is 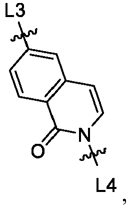 whose L4 end is connected with R¹.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 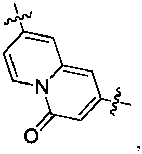 the 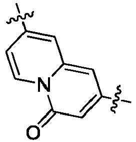 is 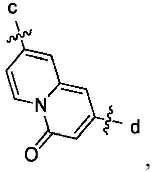 whose d end is connected with R¹.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when Z is 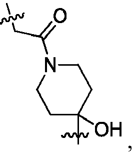 the 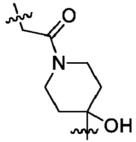 is 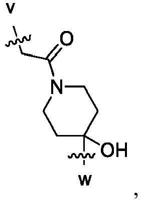 whose w end is connected with R¹.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹ is a C₂-C₄ alkyl, said C₂-C₄ alkyl is ethyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹ is a C₂-C₄ alkyl substituted with a plurality of halogens, said plurality of halogens are 2 or 3 halogens.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹ is a C₂-C₄ alkyl substituted with one or more halogens, said halogen is fluorine.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹ is a C₂-C₄ alkyl substituted with one or more halogens, the C₂-C₄ alkyl is ethyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹ is C₂-C₄ alkyl substituted with a plurality halogens, said C₂-C₄ alkyl substituted with a plurality halogens is 2,2,2-trifluoroethyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹, R¹⁻², R¹⁻³, R¹⁻⁴ and R¹⁻⁵ are independently one or more R¹⁻¹⁻¹ substituted C₆-C₁₀ aryl, said C₆-C₁₀ aryl is phenyl or naphthyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹, R¹⁻², R¹⁻³, R¹⁻⁴ and R¹⁻⁵ are independently one or more R¹⁻¹⁻¹ substituted phenyl, said R¹⁻¹⁻¹ and said -(CH₂)ₛ-, -NH-, 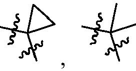 or carbonyl can be located in a ortho, meta or para position relative to each other, or alternatilvely can be in a para position relative to each other.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
R¹⁻¹, R¹⁻², R¹⁻³, R¹⁻⁴ and R¹⁻⁵ are independently 4-trifluoromethylphenyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹, R¹⁻², R¹⁻³, R¹⁻⁴ and R¹⁻⁵ are independently one or more R¹⁻¹⁻² substituted 5-10 membered heteroaryl containing 1, 2, or 3 atoms independently selected from N, O and S, said 5-10 membered heteroaryl containing 1, 2, or 3 atoms independently selected from N, O and S can be 5-6 membered heteroaryl, indolyl, isoquinolyl or quinolyl containing one atom selected from N, O and S, or alternatively can be a 5-6 membered heteroaryl containing one atom selected from N, O and S.

The 5-6 membered heteroaryl containing one atom selected from N, O and S can be for example furyl, thienyl or pyridyl, or for example pyridin-2-yl, pyridin-3-yl or pyridin-4-yl .

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹, R¹⁻², R¹⁻³, R¹⁻⁴ and R¹⁻⁵ are independently one or more R¹⁻¹⁻² substituted 5-10 membered heteroaryl containing 1, 2, or 3 atoms independently selected from N, O and S, and said 5-10 membered heteroaryl containing 1, 2, or 3 atoms independently selected from N, O and S is a 5 membered heteroaryl containing one atom selected from N, O and S, said R¹⁻¹⁻² and said -(CH₂)ₛ-, -NH-, 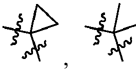 or carbonyl can be located in a ortho or meta position relative to each other.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹, R¹⁻², R¹⁻³, R¹⁻⁴ and R¹⁻⁵ are independently one or more R¹⁻¹⁻² substituted 5-10 membered heteroaryl containing 1, 2, or 3 atoms independently selected from N, O and S, and said 5-10 membered heteroaryl containing 1, 2, or 3 atoms independently selected from N, O and S is a 6 membered heteroaryl containing one N, said R¹⁻¹⁻² and said -(CH₂)ₛ-, -NH-, 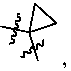 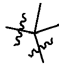 or carbonyl can be located in a ortho, meta or para position relative to each other, or alternatively can be in a para position relative to each other.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹, R¹⁻², R¹⁻³, R¹⁻⁴ and R¹⁻⁵ are independently one or more R¹⁻¹⁻² substituted pyridyl, said R¹⁻¹⁻² and said -(CH₂)ₛ-, -NH-, 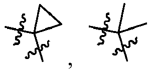 or carbonyl can be located in a ortho, meta or para position relative to each other, or alternatilvely can be in a para position relative to each other.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹, R¹⁻², R¹⁻³, R¹⁻⁴ and R¹⁻⁵ are independently C₃-C₆ cycloalkyl, said C₃-C₆ cycloalkyl is cyclobutyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹, R¹⁻², R¹⁻³, R¹⁻⁴ and R¹⁻⁵ are independently one or more R¹⁻¹⁻³ substituted C₃-C₆ cycloalkyl, said C₃-C₆ cycloalkyl is cyclobutyl or cyclohexyl.

In a certain embodiment, in the compound represented by formula **I,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹, R¹⁻², R¹⁻³, R¹⁻⁴ and R¹⁻⁵ are independently one or more R¹⁻¹⁻³ substituted C₃-C₆ cycloalkyl, said C₃-C₆ cycloalkyl is cyclohexyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹, R¹⁻², R¹⁻³, R¹⁻⁴ and R¹⁻⁵ are independently one or more R¹⁻¹⁻⁶ substituted 5-6 membered heterocycloalkyl containing 1, 2 or 3 atoms independently selected from N, O, and S, said 5-6 membered heterocycloalkyl containing 1, 2 or 3 atoms independently selected from N, O, and S is for example piperidyl, or for example piperidin-l-yl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹, R¹⁻², R¹⁻³, R¹⁻⁴ and R¹⁻⁵ are independently C₇-C₁₀ cycloalkyl, said C₇-C₁₀ cycloalkyl can be C₇-C₁₀ bridged cycloalkyl, or alternatively can be adamantyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹, R¹⁻², R¹⁻³, R¹⁻⁴ and R¹⁻⁵ are independently naphthyl, said naphthyl is naphth-2-yl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹, R¹⁻², R¹⁻³, R¹⁻⁴ and R¹⁻⁵ are independently pyridyl, said pyridyl is pyridin-4-yl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹, R¹⁻², R¹⁻³, R¹⁻⁴ and R¹⁻⁵ are independently 5, 10 membered heteroaryl containing 1, 2, or 3 atoms independently selected from N, O and S, said 5, 10 membered heteroaryl containing 1, 2, or 3 atoms independently selected from N, O and S can be a 10 membered heteroaryl containing 1 or 2 atoms independently selected from N, O and S, or alternatively can be quinolinyl (for example 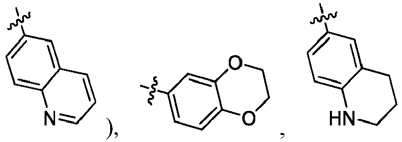 or

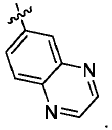

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁶ are independently C₁-C₄ alkyl, said C₁-C₄ alkyl is methyl, ethyl, n-propyl, isopropyl, n-butyl, sec-butyl, isobutyl or tert-butyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁶ are independently C₁-C₄ alkyl, said C₁-C₄ alkyl is methyl or tert-butyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁶ are independently C₁-C₄ alkyl substituted with a plurality of halogens, said plurality of halogens are 2 or 3 halogens.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁶ are independently C₁-C₄ alkyl substituted with one or more halogens, said halogen is fluorine.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁶ are independently C₁-C₄ alkyl substituted with one or more halogens, said C₁-C₄ alkyl is methyl or ethyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁶ are independently C₁-C₄ alkyl substituted with a plurality of halogens, said C₁-C₄ alkyl substituted with a plurality of halogens is trifluoromethyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁶ are independently C₃-C₆ cycloalkyl, said C₃-C₆ cycloalkyl is cyclopropyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁶ are independently halogen, said halogen is fluorine, chlorine or bromine.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁶ are independently C₁-C₄ alkoxy substituted with a plurality of halogens, said plurality of halogens are 2 or 3 halogens.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁶ are independently C₁-C₄ alkoxy substituted with one or more halogens, said halogen is fluorine.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁶ are independently C₁-C₄ alkoxy substituted with one or more halogens, said C₁-C₄ alkoxy is methoxy or ethoxy.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁶ are independently C₁-C₄ alkoxy substituted with a plurality of halogens, said C₁-C₄ alkoxy substituted with a plurality of halogens is trifluoromethoxy.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹, R¹⁻¹⁻², R¹⁻¹⁻³, and R¹⁻¹⁻⁶ are independently 5-6 membered heterocycloalkyl containing 1, 2 or 3 atoms independently selected from N, O and S, said 5-6 membered heterocycloalkyl containing 1, 2 or 3 atoms independently selected from N, O and S can be morpholinyl, or alternatively can be morpholin-1-yl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹ and R¹⁻¹⁻² are independently C₁-C₄ alkyl, said C₁-C₄ alkyl is methyl or ethyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻⁷, R¹⁻¹⁻⁸ and R¹⁻¹⁻⁹ are independently C₁-C₄ alkyl, said C₁-C₄ alkyl is methyl or ethyl.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹⁰ is independently C₁-C₄ alkoxy, said C₁-C₄ alkoxy is methoxy or ethoxy.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:
when R¹⁻¹⁻¹¹ and R¹⁻¹⁻¹² are independently halogen, said halogen is fluorine.

In a certain embodiment, in the compound represented by formula **XI,** or a pharmaceutically acceptable salt, a solvate, or a solvate of a pharmaceutically acceptable salt thereof, certain groups can be defined as follows, while the remaining groups are as defined in any of the above embodiments:

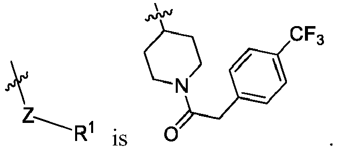

In a certain embodiment, in the compound represented by formula **XI,** or a pharmaceutically acceptable salt, a solvate, or a solvate of a pharmaceutically acceptable salt thereof, the compound represented by formula **XI** has anyone of the following structures:

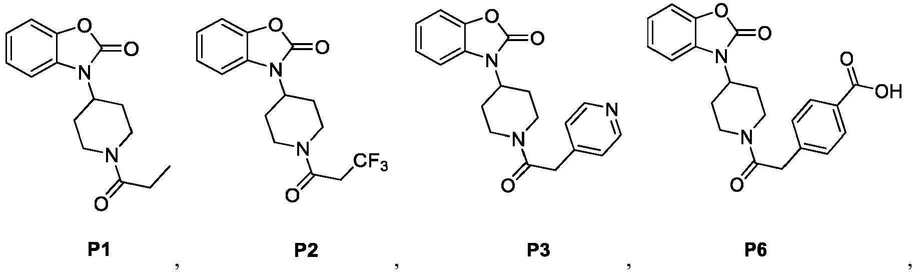

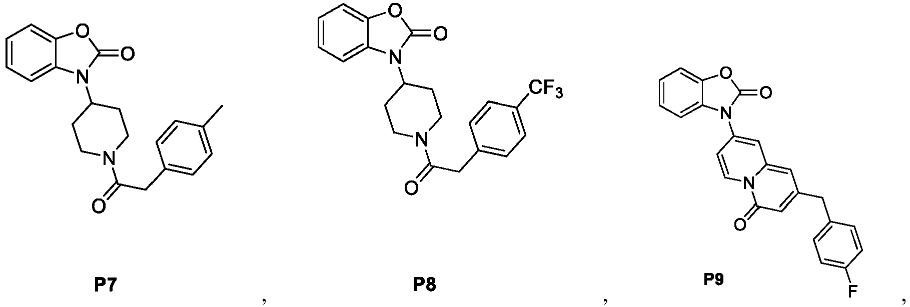

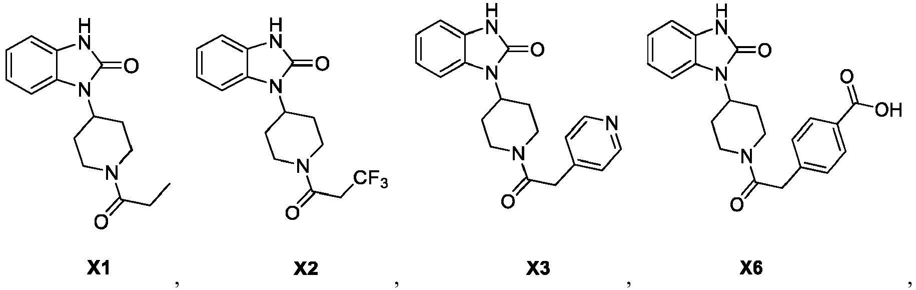

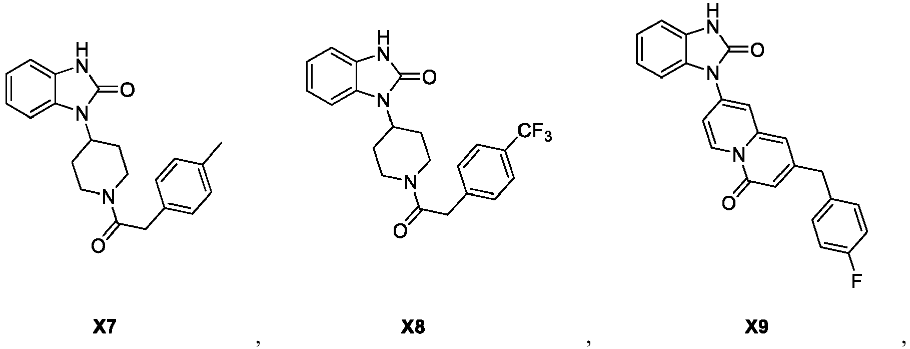

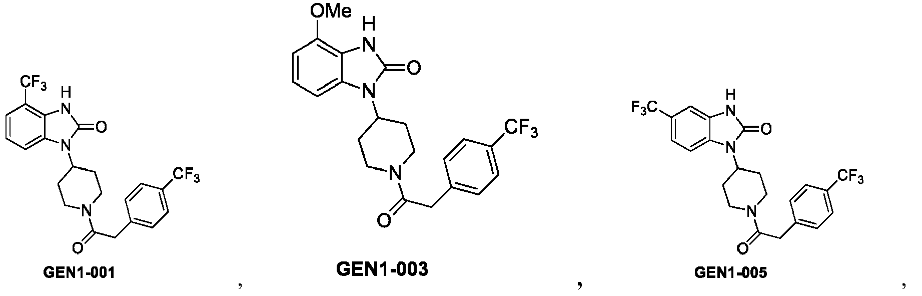

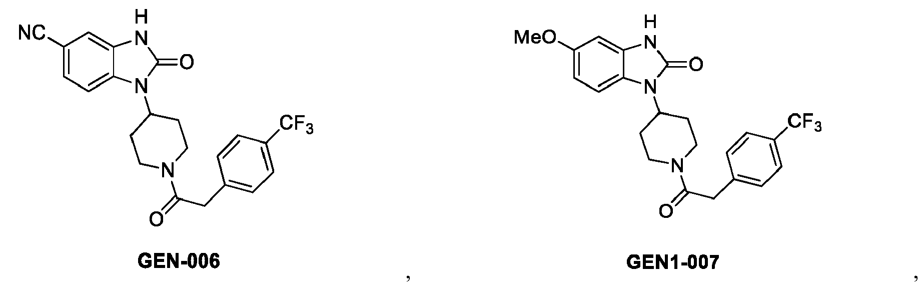

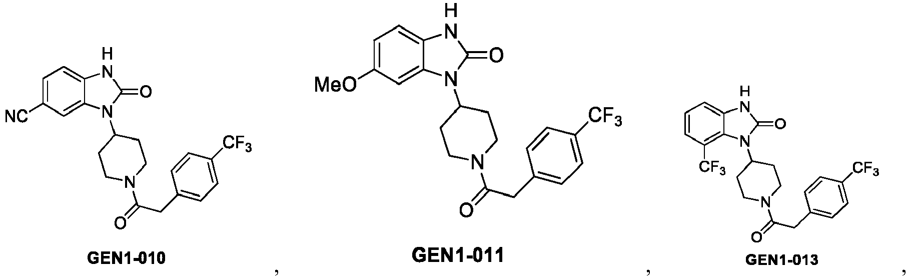

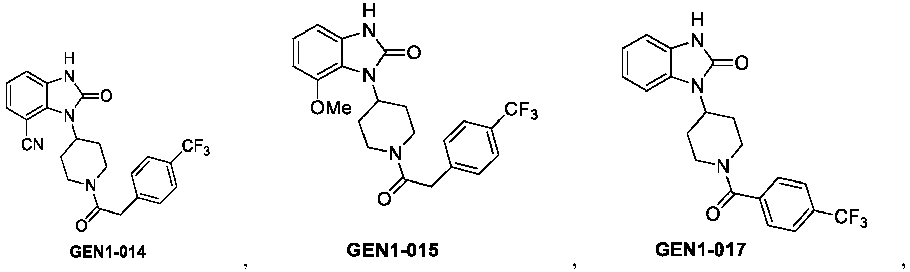

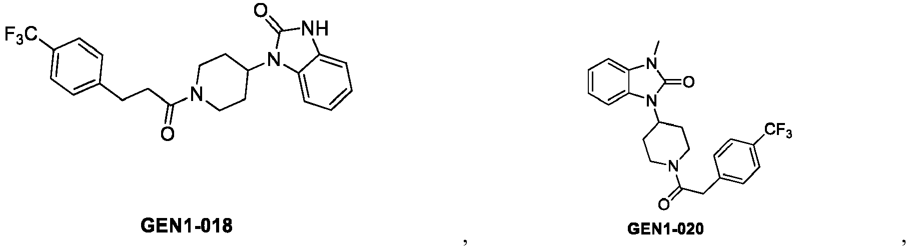

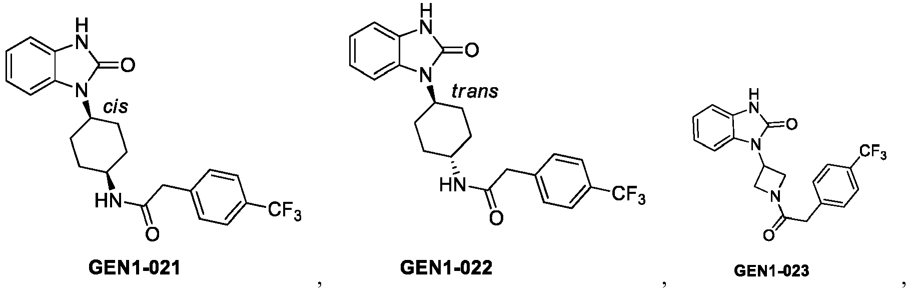

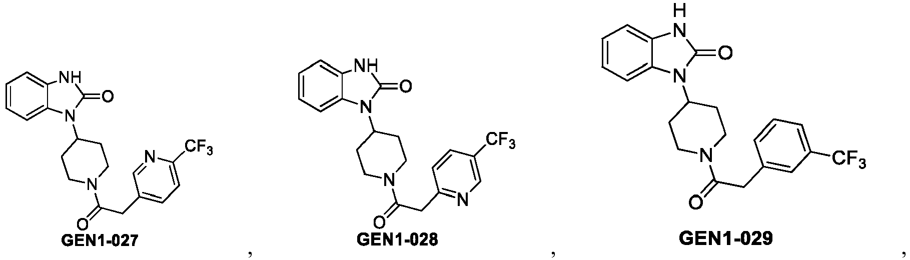

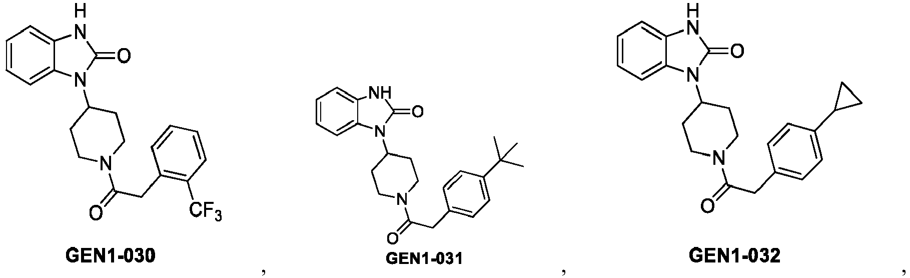

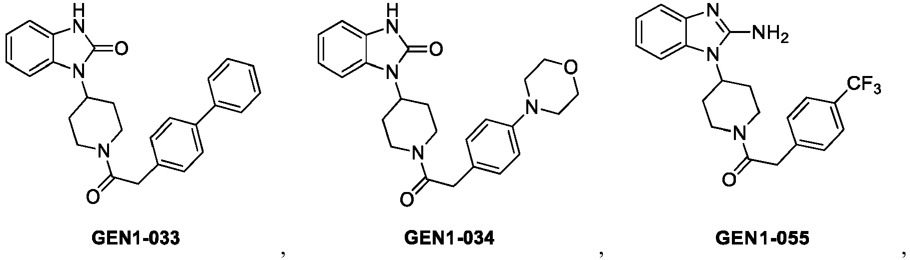

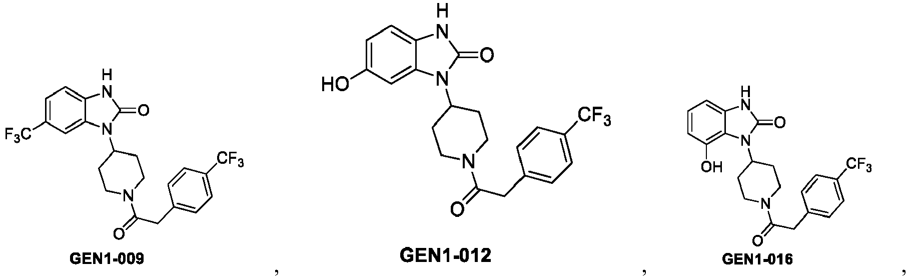

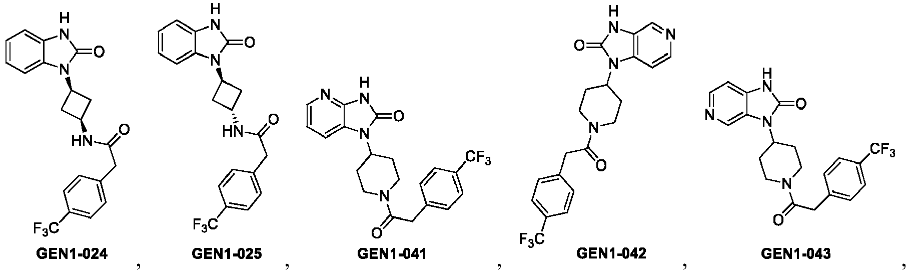

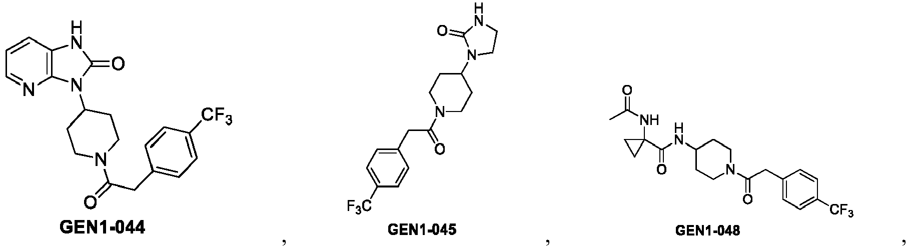

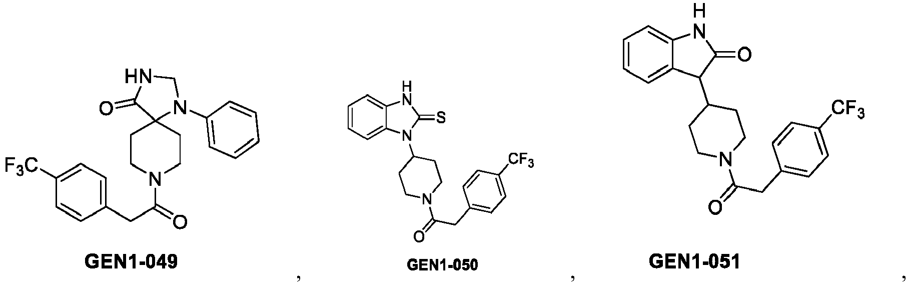

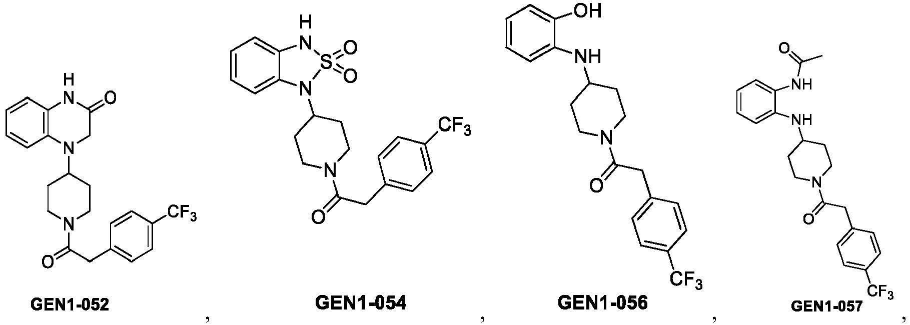

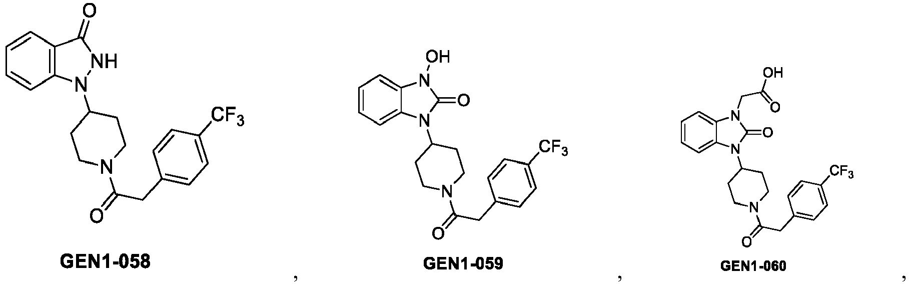

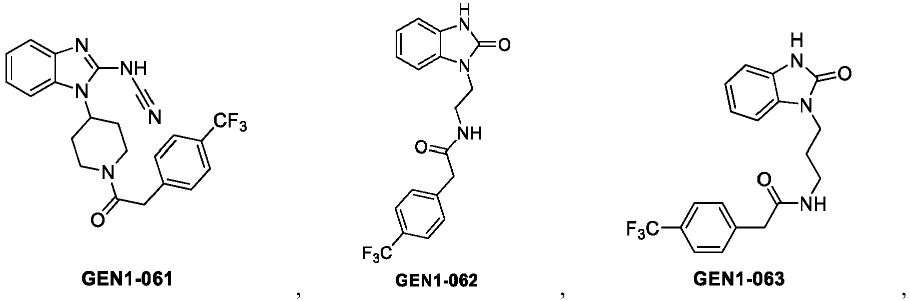

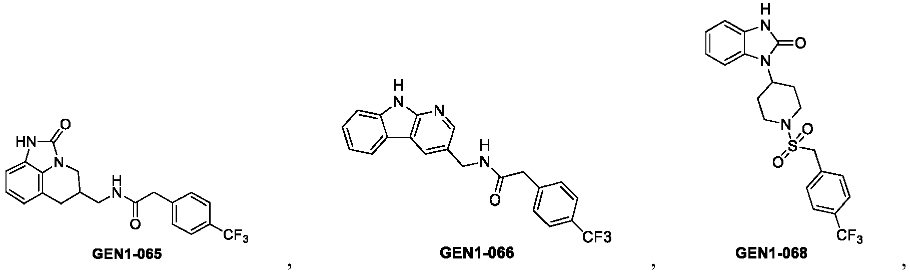

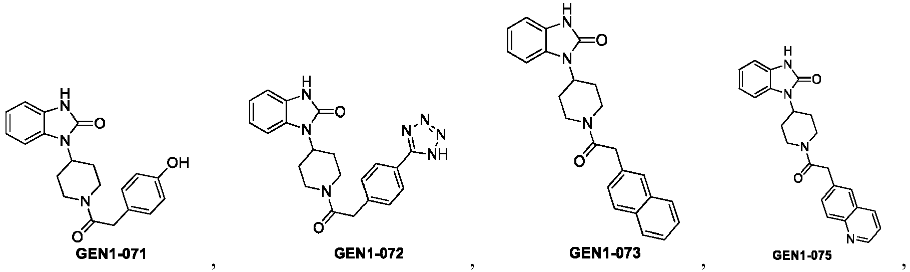

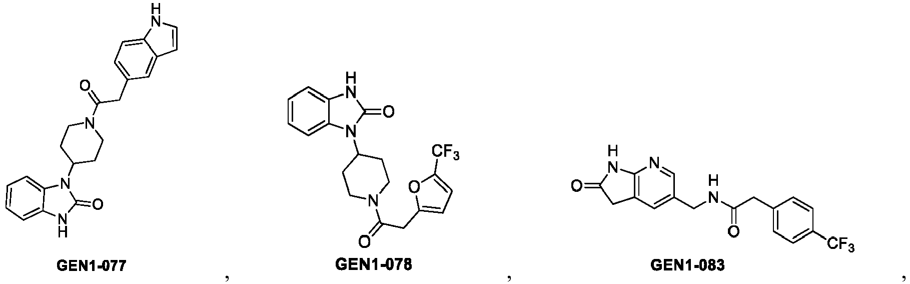

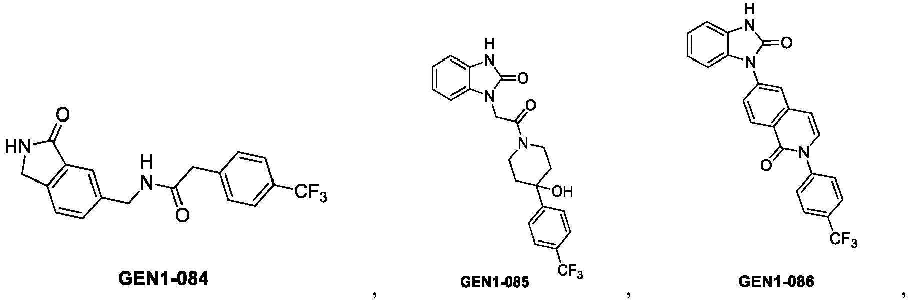

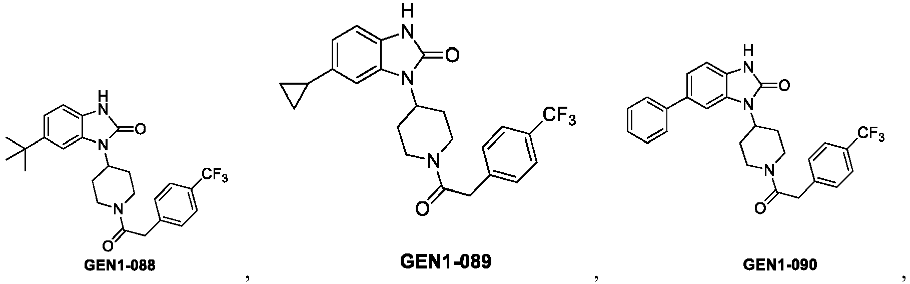

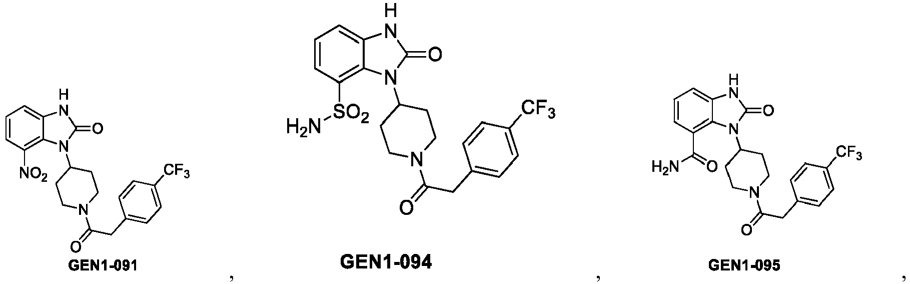

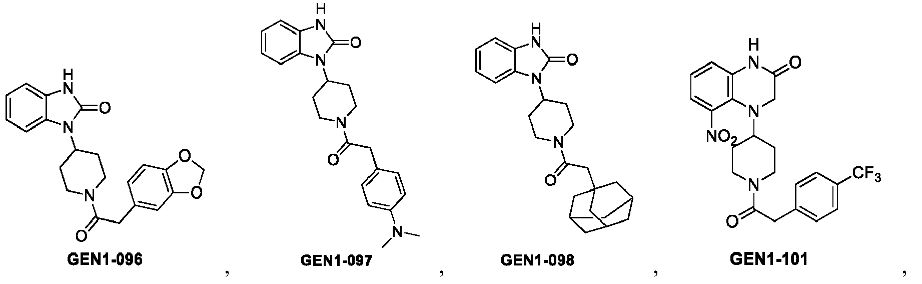

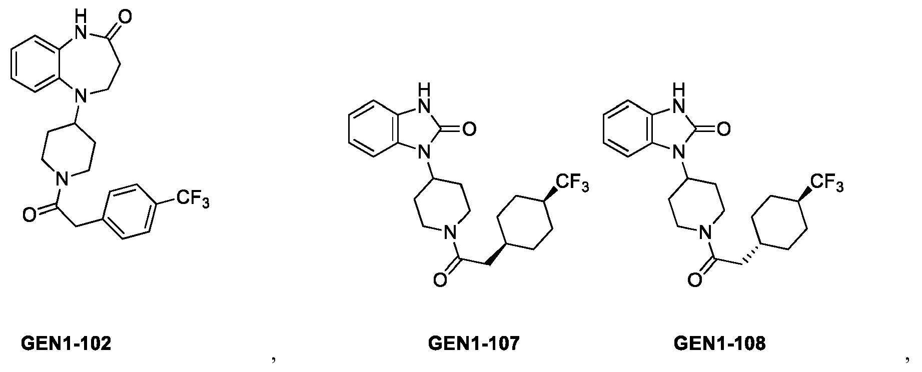

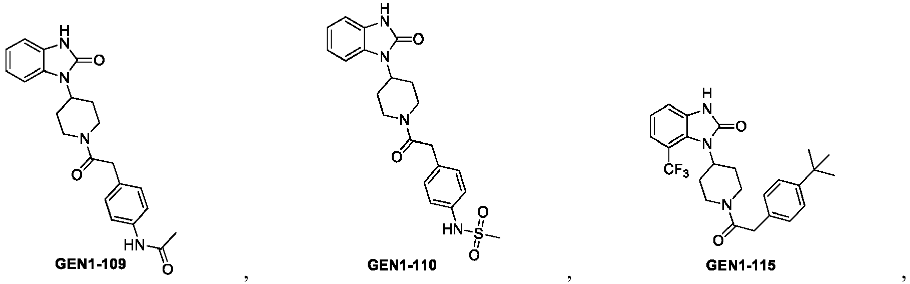

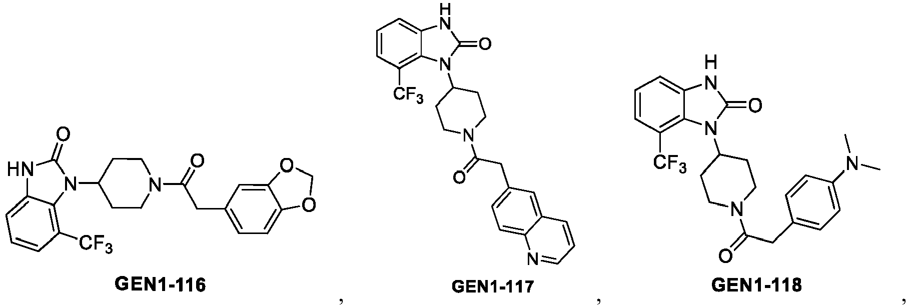

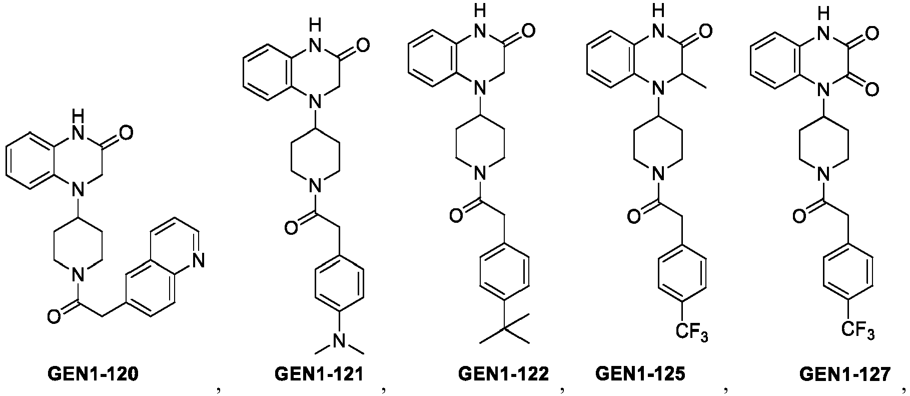

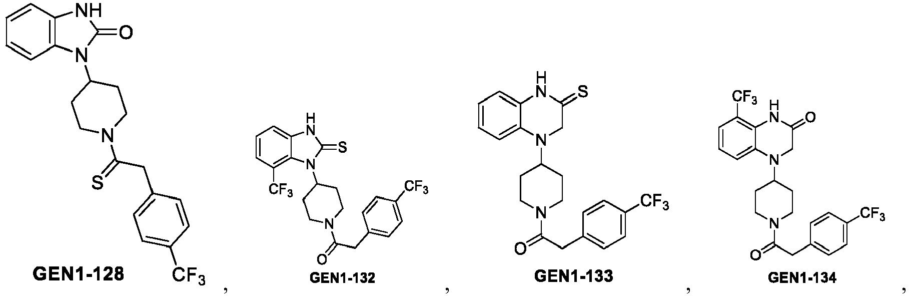

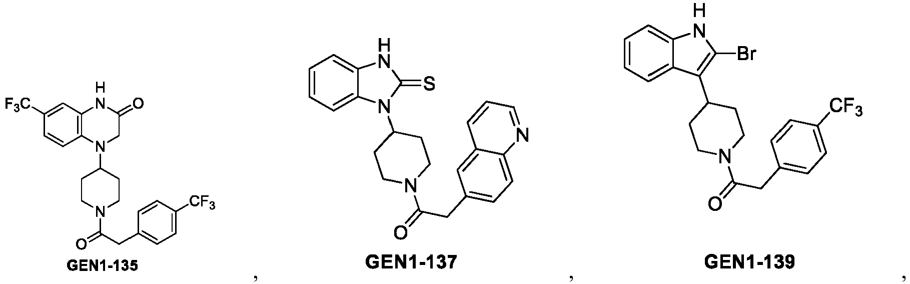

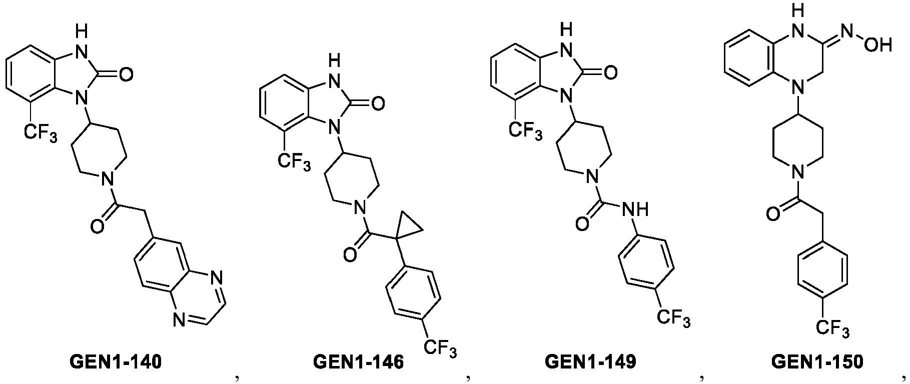

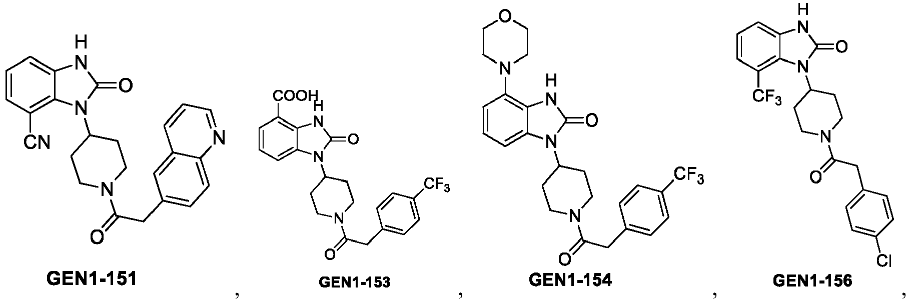

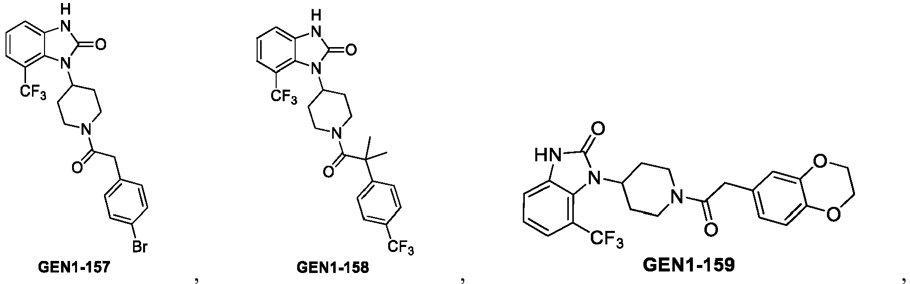

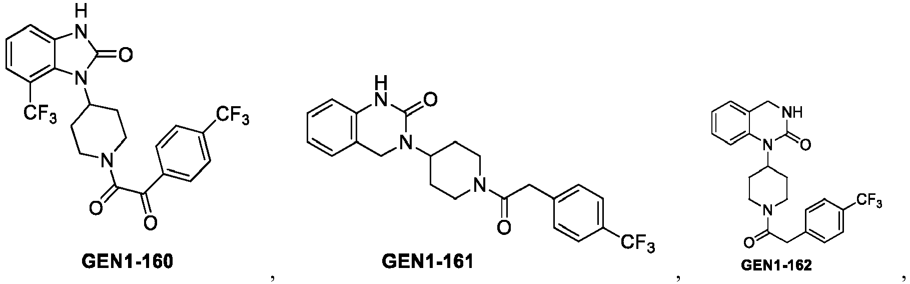

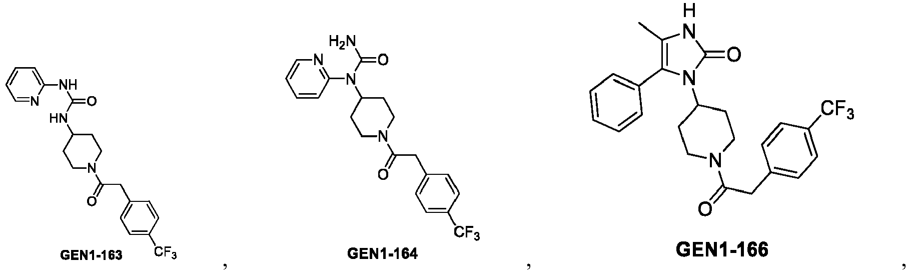

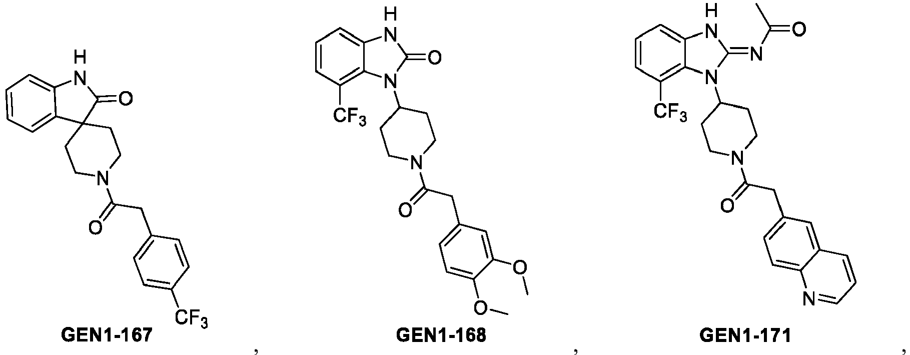

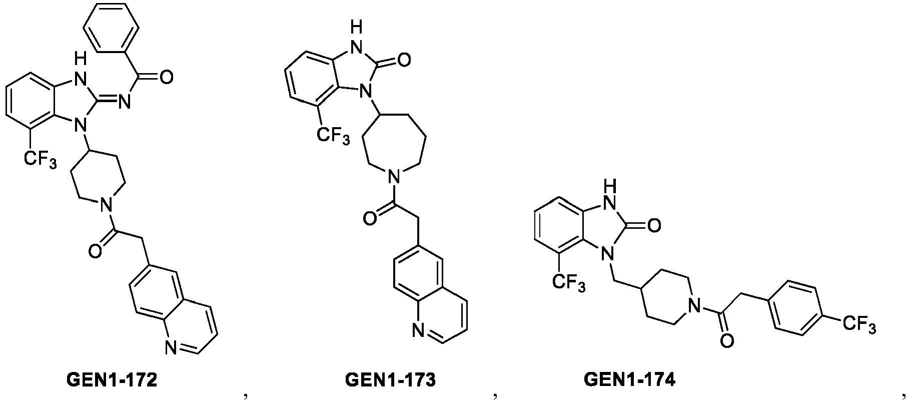

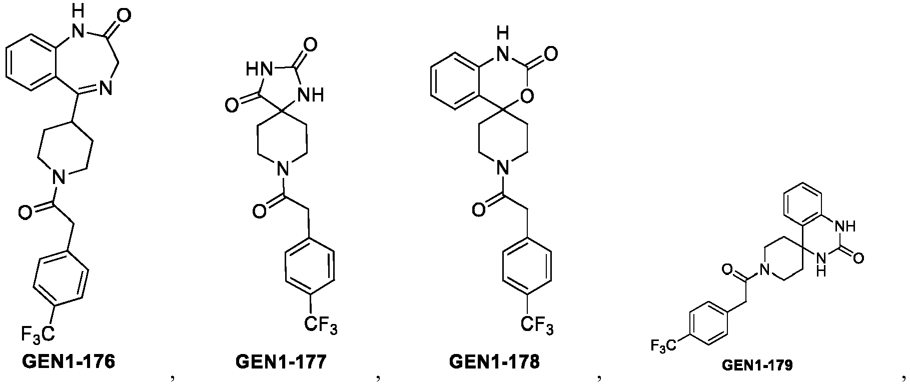

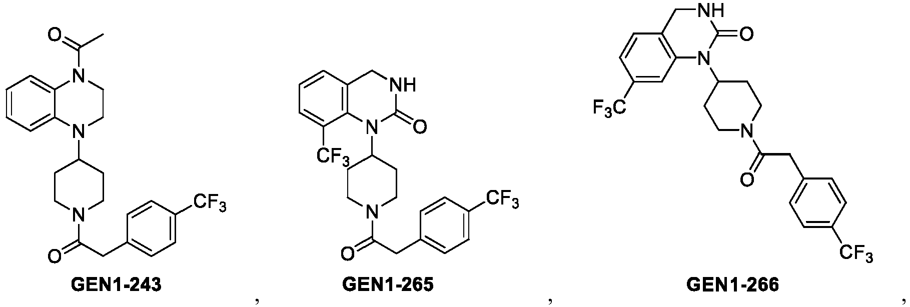

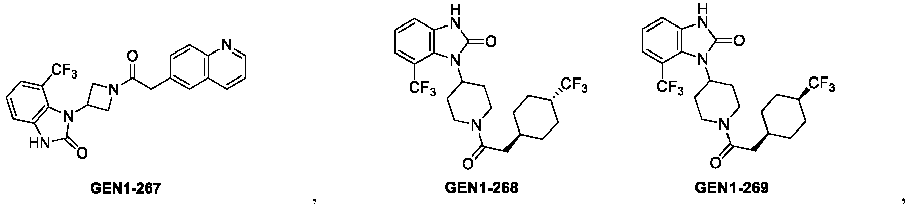

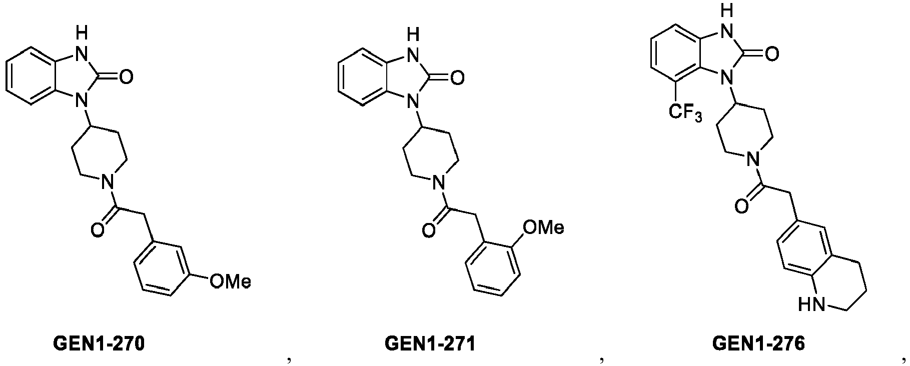

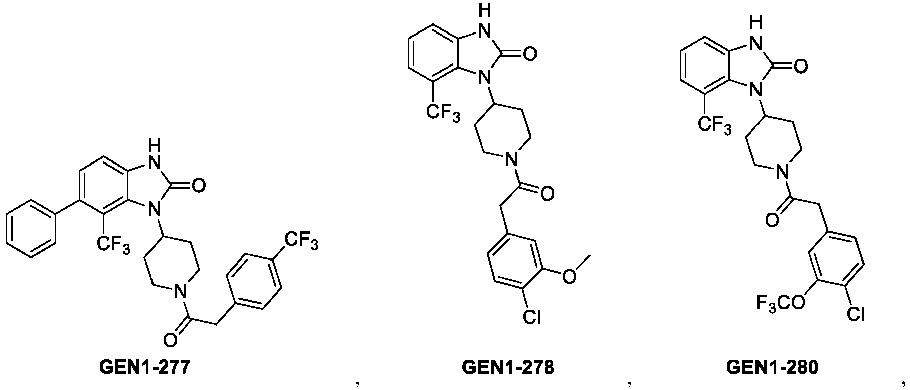

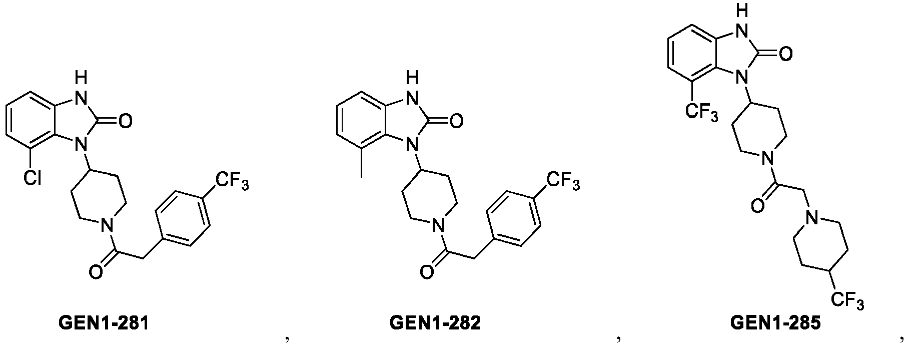

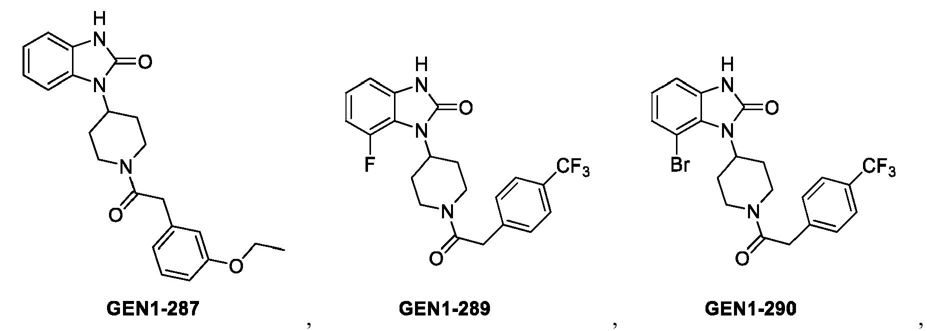

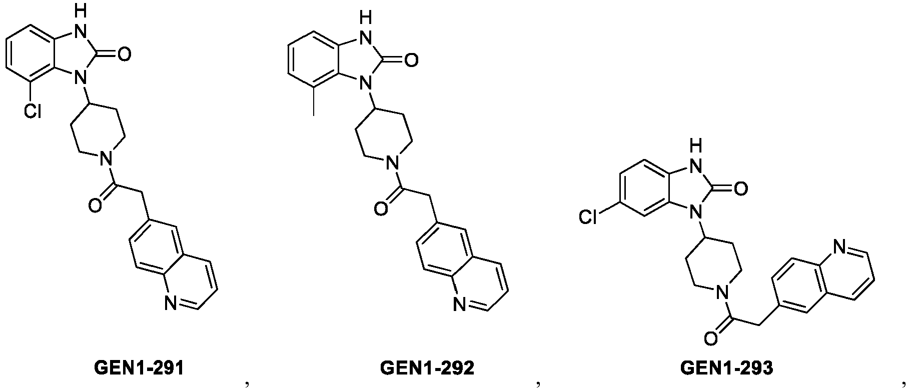

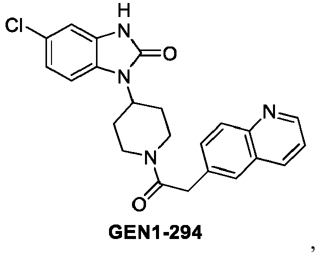

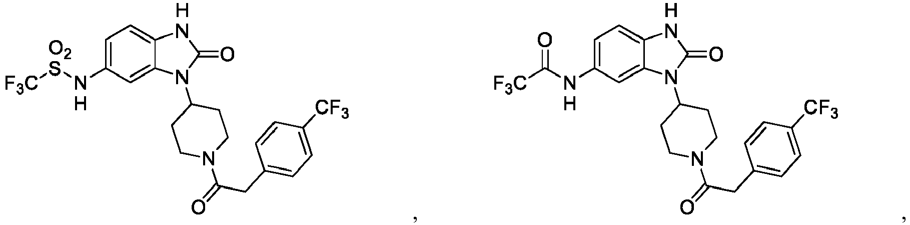

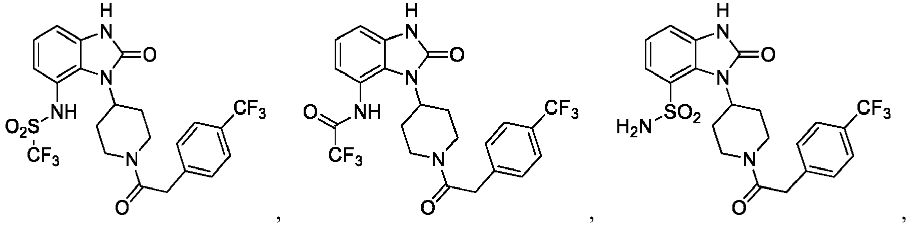

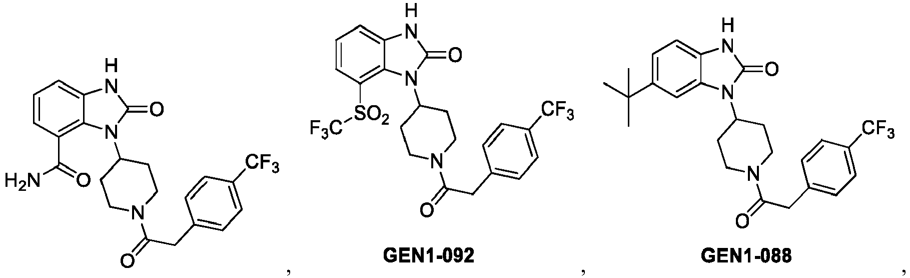

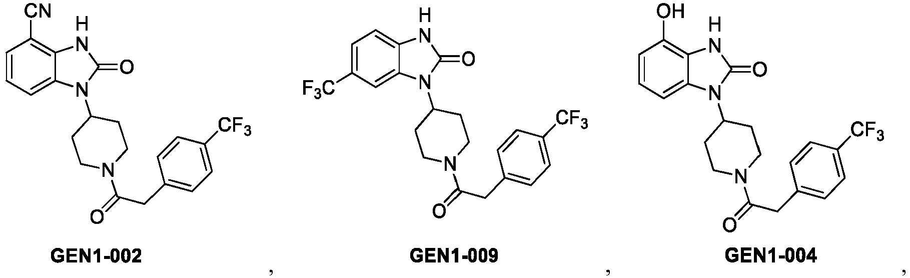

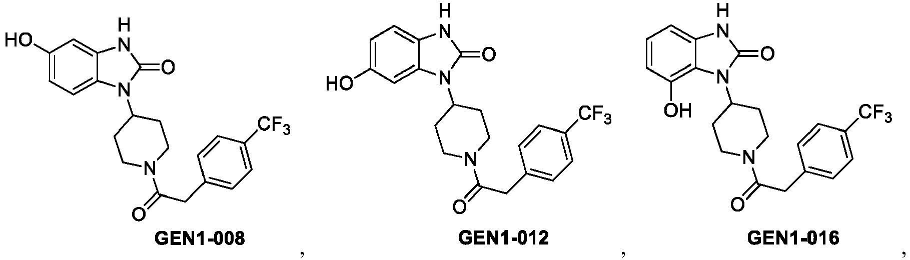

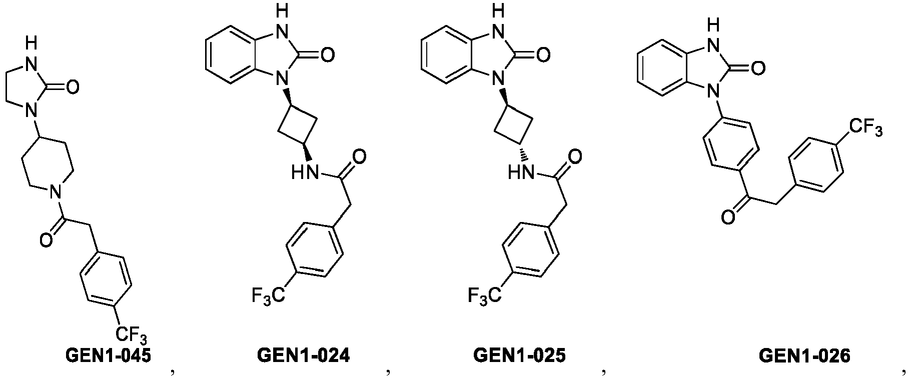

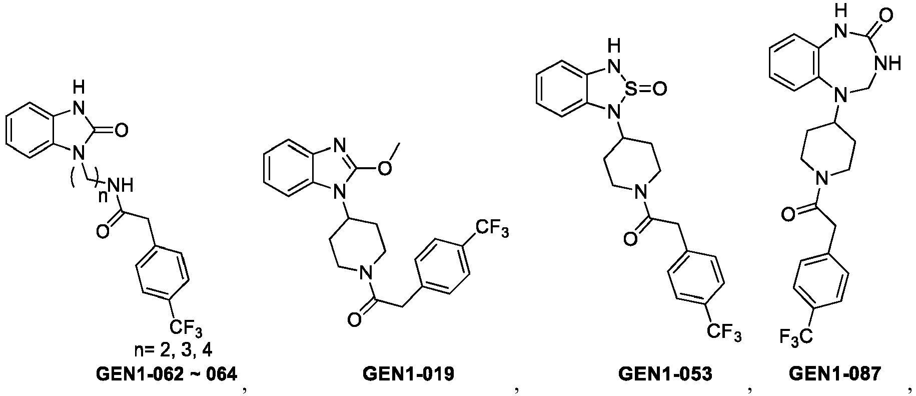

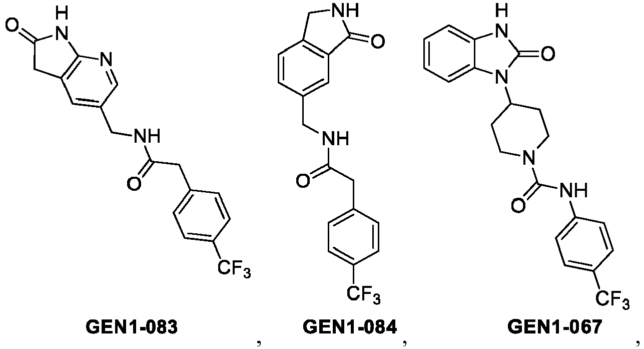

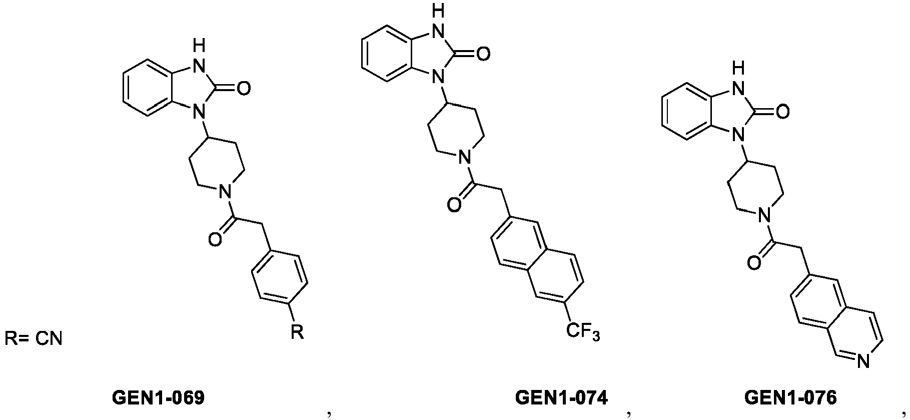

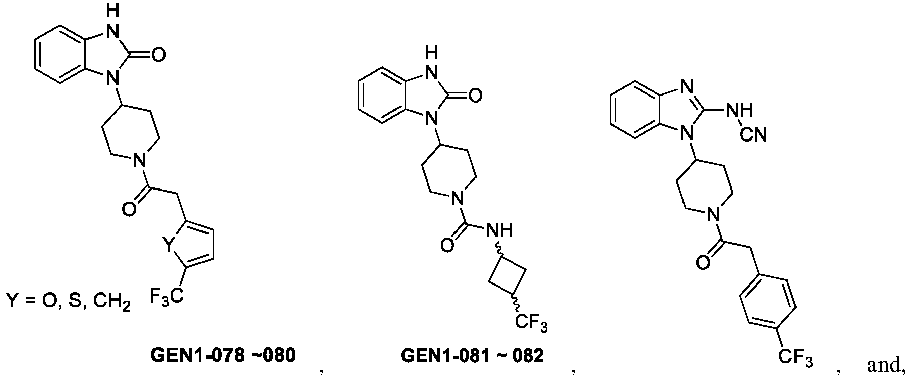

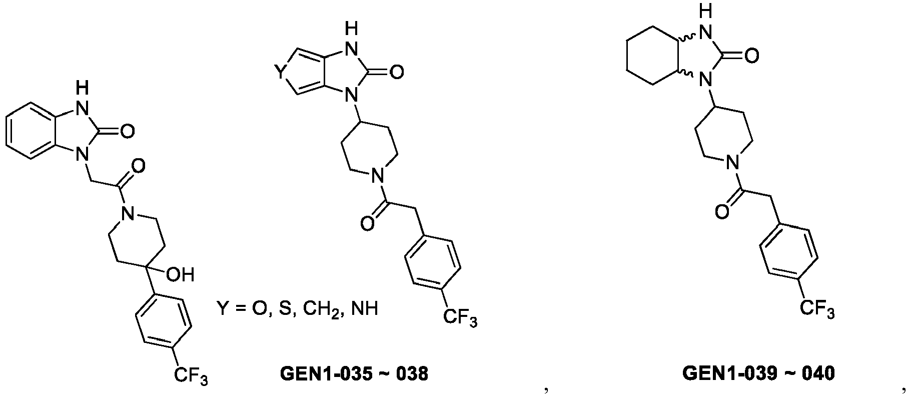

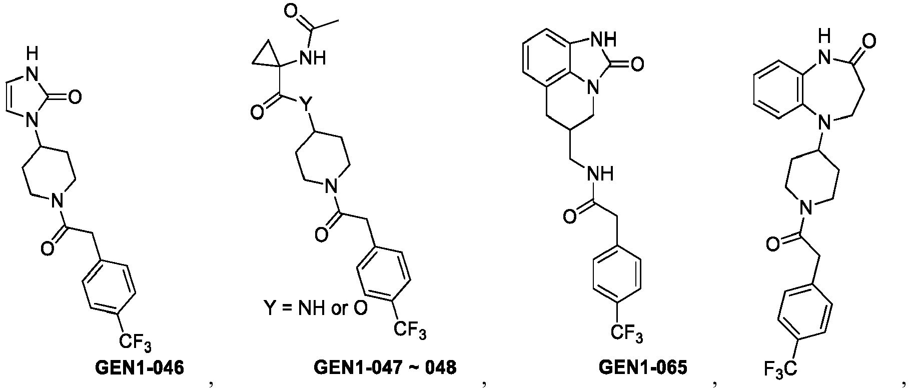

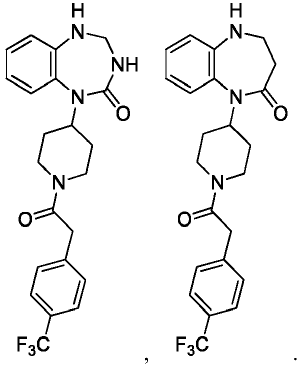

The present invention further provides a pharmaceutical composition comprising a substance **X** and a pharmaceutical excipient;
the substance **X** is the above-mentioned compound represented by formula **XI,** or a pharmaceutically acceptable salt, a solvate, or a solvate of a pharmaceutically acceptable salt thereof.

The dosage form of the pharmaceutical composition may be an oral dosage form.

In the pharmaceutical composition, the substance **X** may be the above-mentioned compound represented by formula **I,** a pharmaceutically acceptable salt of the compound represented by formula **I,** a solvent of the compound represented by formula **I,** a solvate of a pharmaceutically acceptable salt of the compound represented by formula **I,** compound represented by formula **III,** a pharmaceutically acceptable salt of the compound represented by formula **III,** a solvent of the compound represented by formula **III,** a solvate of a pharmaceutically acceptable salt of the compound represented by formula **III,** the compound represented by formula **V,** a pharmaceutically acceptable salt of the compound represented by formula **V,** a solvent of the compound represented by formula **V,** or a solvate of a pharmaceutically acceptable salt of the compound represented by formula **V.**

The present invention further provides use of a substance **X** in the manufacture of a STAT5 inhibitor;
the substance **X** is the above-mentioned compound represented by formula **XI,** or a pharmaceutically acceptable salt, a solvate, or a solvate of a pharmaceutically acceptable salt thereof.

In the use, the substance **X** may be the above-mentioned compound represented by formula **I,** a pharmaceutically acceptable salt of the compound represented by formula **I,** a solvent of the compound represented by formula **I,** a solvate of a pharmaceutically acceptable salt of the compound represented by formula **I,** compound represented by formula **III,** a pharmaceutically acceptable salt of the compound represented by formula **III,** a solvent of the compound represented by formula **III,** a solvate of a pharmaceutically acceptable salt of the compound represented by formula **III,** the compound represented by formula **V,** a pharmaceutically acceptable salt of the compound represented by formula **V,** a solvent of the compound represented by formula **V,** or a solvate of a pharmaceutically acceptable salt of the compound represented by formula **V.**

The present invention further provides use of a substance **X** in the manufacture of a medicament;
the substance **X** is the above-mentioned compound represented by formula **XI,** or a pharmaceutically acceptable salt, a solvate, or a solvate of a pharmaceutically acceptable salt thereof.

In the use, the substance **X** may be the above-mentioned compound represented by formula **I,** a pharmaceutically acceptable salt of the compound represented by formula **I,** a solvent of the compound represented by formula **I,** a solvate of a pharmaceutically acceptable salt of the compound represented by formula **I,** compound represented by formula **III,** a pharmaceutically acceptable salt of the compound represented by formula **III,** a solvent of the compound represented by formula **III,** a solvate of a pharmaceutically acceptable salt of the compound represented by formula **III,** the compound represented by formula **V,** a pharmaceutically acceptable salt of the compound represented by formula **V,** a solvent of the compound represented by formula **V,** or a solvate of a pharmaceutically acceptable salt of the compound represented by formula **V.**

The medicament may be used for the treatment and/or prevention of a disease related to STAT5.

The medicament may be used for the treatment and/or prevention for rheumatoid arthritis.

The present invention further provides a method of treating and/or preventing a disease related to STAT5, comprising administering to a patient a therapeutically effective amount of a substance **X;**
the substance **X** is the above-mentioned compound represented by formula **XI,** or a pharmaceutically acceptable salt, a solvate, or a solvate of a pharmaceutically acceptable salt thereof.

In the treatment method, the substance **X** may be the above-mentioned compound represented by formula **I,** a pharmaceutically acceptable salt of the compound represented by formula **I,** a solvent of the compound represented by formula **I,** a solvate of a pharmaceutically acceptable salt of the compound represented by formula **I,** compound represented by formula **III,** a pharmaceutically acceptable salt of the compound represented by formula **III,** a solvent of the compound represented by formula **III,** a solvate of a pharmaceutically acceptable salt of the compound represented by formula **III,** the compound represented by formula **V,** a pharmaceutically acceptable salt of the compound represented by formula **V,** a solvent of the compound represented by formula **V,** or a solvate of a pharmaceutically acceptable salt of the compound represented by formula **V.**

In the treatment method, the patient may be a mammal, such as a mouse or a human.

In the treatment method, the substance **X** is administered orally.

In the treatment method, the substance **X** can be administered according to the weight of the patient, and a non-limiting exemplary range can be 0.05 mg/kg to 1 mg/kg (single dose).

In the treatment method, the substance **X** may be administered at the frequency of twice a day, once a day, once every two days, or once a week.

The present invention further provides a method of treating and/or preventing rheumatoid arthritis, comprising administering to a patient a therapeutically effective amount of a substance **X;**
the substance **X** is the above-mentioned compound represented by formula **XI,** or a pharmaceutically acceptable salt, a solvate, or a solvate of a pharmaceutically acceptable salt thereof.

In the treatment method, the substance **X** may be the above-mentioned compound represented by formula **I,** a pharmaceutically acceptable salt of the compound represented by formula **I,** a solvent of the compound represented by formula **I,** a solvate of a pharmaceutically acceptable salt of the compound represented by formula **I,** compound represented by formula **III,** a pharmaceutically acceptable salt of the compound represented by formula **III,** a solvent of the compound represented by formula **III,** a solvate of a pharmaceutically acceptable salt of the compound represented by formula **III,** the compound represented by formula **V,** a pharmaceutically acceptable salt of the compound represented by formula **V,** a solvent of the compound represented by formula **V,** or a solvate of a pharmaceutically acceptable salt of the compound represented by formula **V.**

In the treatment method, the patient may be a mammal, such as a mouse or a human.

In the treatment method, the substance **X** is administered orally.

In the treatment method, the substance **X** can be administered according to the weight of the patient, and a non-limiting exemplary range can be 0.05 mg/kg to 1 mg/kg (single dose).

In the treatment method, the substance **X** may be administered at the frequency of twice a day, once a day, once every two days, or once a week.

Unless otherwise specified, the terms used in the present invention have the following definitions:
The term "a plurality" refers to 2, 3, 4, or 5.

The term "pharmaceutically acceptable" means that salts, solvents, excipients, etc. are generally non-toxic, safe, and suitable for use by patients. The "patient" is preferably a mammal, more preferably a human.

The term "pharmaceutically acceptable salt" refers to a salt prepared from a compound of the present invention with a relatively non-toxic, pharmaceutically acceptable acid or base. When the compound of the present invention contains a relatively acidic functional group, the base addition salt can be obtained by contacting the neutral form of the compound with a sufficient amount of a pharmaceutically acceptable base in a pure solution or a suitable inert solvent. Pharmaceutically acceptable base addition salts include, but are not limited to: lithium salt, sodium salt, potassium salt, calcium salt, aluminum salt, magnesium salt, zinc salt, bismuth salt, ammonium salt, and diethanolamine salt. When the compound of the present invention contains a relatively basic functional group, the acid addition salt can be obtained by contacting the neutral form of the compound with a sufficient amount of a pharmaceutically acceptable acid in a pure solution or a suitable inert solvent. The pharmaceutically acceptable acids include inorganic acids, including but are not limited to: hydrochloric acid, hydrobromic acid, hydroiodic acid, nitric acid, carbonic acid, phosphoric acid, phosphorous acid, sulfuric acid, and the like. The pharmaceutically acceptable acids include organic acids, including but not limited to: acetic acid, propionic acid, oxalic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, salicylic acid, tartaric acid, methanesulfonic acid, isonicotinic acid, acid citric acid, oleic acid, tannic acid, pantothenic acid, hydrogen tartrate, ascorbic acid, gentisic acid, fumaric acid, gluconic acid, sugar acid, formic acid, ethanesulfonic acid, pamoic acid (i.e., 4, 4'-methylene-bis(3-hydroxy-2-naphthoic acid)), amino acids (such as glutamic acid, arginine), etc. When the compound of the present invention contains relatively acidic and relatively basic functional groups, it can be converted into a base addition salt or an acid addition salt. For details, see Berge et al., "Pharmaceutical Salts", Journal of Pharmaceutical Science 66: 1-19 (1977) or Handbook of Pharmaceutical Salts: Properties, Selection, and Use (P. Heinrich Stahl and Camille G. Wermuth, ed., Wiley-VCH, 2002).

The term "solvate" refers to a substance formed by combining the compound of the present invention with a stoichiometric or non-stoichiometric solvent. The solvent molecules in the solvate can exist in an ordered or non-ordered arrangement. The solvents include but are not limited to: water, methanol, ethanol and the like.

The term "solvate of a pharmaceutically acceptable salt" includes the definition in the terms "pharmaceutically acceptable salt" and "solvate" are as described above, which means a substance (1) prepared by a compound of the present invention and a relatively non-toxic, pharmaceutically acceptable acid or base and (2) formed by combining a compound of the present invention with a stoichiometric or non-stoichiometric solvent. The "solvate of a pharmaceutically acceptable salt" includes, but is not limited to, the hydrochloric acid monohydrate of a compound of the present invention.

The terms "compound", "pharmaceutically acceptable salt", "solvate" and "solvate of a pharmaceutically acceptable salt" may exist in crystalline or amorphous forms. The term "crystalline form" means that the ions or molecules are arranged strictly and periodically in a three-dimensional space in a certain way, and have the regularity of periodic recurrence at a certain distance; since the above-mentioned periodic arrangement can be different, there may be multiple crystalline forms, that is, polymorphism. The term "amorphous" refers to the disordered distribution of ions or molecules, that is, there is no periodic arrangement between ions and molecules.

The terms "compound", "pharmaceutically acceptable salt", "solvate" and "solvate of a pharmaceutically acceptable salt" may exist in the form of a single stereoisomer or a mixture thereof (for example, a racemate) if they are stereoisomers. The term "stereoisomer" refers to cis-trans isomers or optical isomers. These stereoisomers can be separated, purified and enriched by asymmetric synthesis methods or chiral separation methods (including but not limited to thin layer chromatography, rotation chromatography, column chromatography, gas chromatography, high pressure liquid chromatography, etc.), and can also be obtained by chiral resolution by forming bonds (chemical bonding, etc.) or salts (physical bonding, etc.) with other chiral compounds. The term "single stereoisomer" means that the mass content of one stereoisomer of the compound of the present invention relative to all stereoisomers of the compound is not less than 95%.

The terms "compound", "pharmaceutically acceptable salt", "solvate" and "solvate of a pharmaceutically acceptable salt" can exist in the form of a single tautomer or a mixture thereof if they are tautomers, preferably in the form of relatively stable tautomers.

The atoms in the terms "compound", "pharmaceutically acceptable salt", "solvate" and "solvate of a pharmaceutically acceptable salt" may exist in their natural abundance or non-natural abundance. Taking the hydrogen atom as an example, the form of its natural abundance includes about 99.85% of protium and about 0.015% of deuterium; and the form of its unnatural abundance includes about 95% of deuterium. That is, one or more of the atoms in the terms "compound", "pharmaceutically acceptable salt", "solvate" and "solvate of a pharmaceutically acceptable salt" may be atoms that exist in an unnatural abundance.

When a variable (such as R1-1-1) appears multiple times in the definition of a compound, the definition in each position of the variable is irrelevant with the definition in the other positions, and their meanings are independent of each other and do not affect each other. Therefore, if a group is substituted by 1, 2 or 3 R1-1-1 groups, that is, the group may be substituted by up to 3 R1-1-1 groups, and the definition of R1-1-1 in this position is independent of the definition of R1-1-1 in the other positions. In addition, combinations of substituents and/or variables are only allowed if the combination results in a stable compound.

The term "halogen" refers to fluorine, chlorine, bromine or iodine.

The term "alkyl" refers to a straight or branched alkyl group having the specified number of carbon atoms. Examples of alkyl groups include methyl, ethyl, n-propyl, isopropyl, n-butyl, tert-butyl, isobutyl, sec-butyl, n-pentyl, n-hexyl, n-heptyl, n-octyl and similar alkyl groups.

The term "alkoxy" refers to the group -O-R^{X}, where R^{X} is an alkyl group as defined above.

The term "cycloalkyl" refers to a monovalent saturated cyclic alkyl group, preferably a monovalent saturated cyclic alkyl group having 3-7 ring carbon atoms, more preferably 3-6 carbon atoms, such as cyclopropyl, cyclobutyl, cyclopentyl or cyclohexyl. The cycloalkyl group may be a monocyclic ring or a polycyclic ring (for example, a bridged ring, a spiro ring).

The term "heterocycloalkyl" refers to a saturated monocyclic group containing heteroatoms, preferably a 3-7 membered saturated monocyclic group containing 1, 2, or 3 ring heteroatoms independently selected from N, O and S. Examples of heterocycloalkyl groups are: pyrrolidinyl, tetrahydrofuranyl, tetrahydropyranyl, tetrahydrothienyl, tetrahydropyridinyl, tetrahydropyrrolyl, azetidinyl, thiazolidinyl, oxazolidinyl, piperidinyl, morpholinyl, thiomorpholinyl, piperazinyl, azepanyl, diazepanyl, oxazepanyl, etc. Preferred heterocyclic groups are morpholin-4-yl, piperidin-1-yl, pyrrolidin-l-yl, thiomorpholin-4-yl and 1,1-dioxo-thiomorpholine-4 -yl.

The term "aryl" refers to phenyl or naphthyl.

The term "heteroaryl" refers to an aromatic group containing heteroatoms, preferably a aromatic 5-6 membered monocyclic ring or 9-10 membered bicyclic ring containing 1, 2, or 3 atoms independently selected from nitrogen, oxygen and sulfur, such as furyl, pyridyl, pyridazinyl, pyrimidinyl, pyrazinyl, thienyl, isoxazolyl, oxazolyl, diazolyl, imidazolyl, pyrrolyl, pyrazolyl, triazolyl, tetrazolyl, thiazolyl, isothiazolyl, thiadiazolyl, benzimidazolyl, indolyl, indazolyl, benzothiazolyl, benzisothiazolyl, benzoxazolyl, benzisozolyl, quinolyl, isoquinolyl, etc. At least one ring in the heteroaryl group has aromaticity.

The term "pharmaceutical excipient" refers to excipients and additives used in the production of medicines and formulating prescriptions, and are all substances contained in pharmaceutical preparations except for the active ingredients. See four parts of the Chinese Pharmacopoeia (2015 Edition), or Handbook of Pharmaceutical Excipients (Raymond C Rowe, 2009 Sixth Edition).

The term "treatment" refers to therapeutic therapy. When it comes to a specific disease, treatment refers to: (1) alleviating one or more biological manifestations of the disease or disorder, (2) interfering with (a) one or more points in the biological cascade leading to or causing the disease, or (b) one or more biological manifestations of the disorder, (3) improving one or more symptoms, effects or side effects related to the disorder, or one or more symptoms, effects or side effects related to the disorder or its treatment, or (4) slowing down the development of one or more biological manifestations of the disease or disorder.

The term "prevention" refers to a reduction in the risk of acquiring or developing a disease or disorder.

The term "therapeutically effective amount" refers to an amount of a compound that is sufficient to effectively treat the diseases or conditions described herein when administered to a patient. The "therapeutically effective amount" will vary according to the compound, the condition and its severity, and the age of the patient to be treated, but can be adjusted by those skilled in the art as needed.

The term "patient" refers to any animal that is about to or has received administration of the compound or composition according to an embodiment of the present invention, preferably a mammal, and most preferably a human. The term "mammal" includes any mammal. Examples of mammals include, but are not limited to, cows, horses, sheep, pigs, cats, dogs, mice, rats, rabbits, guinea pigs, monkeys, humans, etc., and most preferably humans.

The term "STAT5 inhibitor" refers to a substance that can ultimately specifically inhibit the activity of STAT5 in the in vivo environment.

On the basis of not violating common knowledge in the art, the above-mentioned preferred conditions can be combined arbitrarily to obtain preferred embodiments of the present invention.

The reagents and raw materials used in the present invention are all commercially available.

The positive and progressive effect of the present invention lies in that: the compounds are novel in structure and have good STAT5 inhibitory activity.

### DETAILED DESCRIPTION OF EMBODIMENTS

### Example 1. 3-(1-propionylpiperidin-4-yl)benzo[d]oxazole-2(3H)-one (P1)

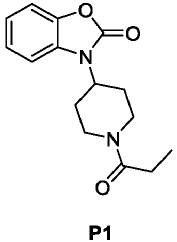

### Step 1.1. Synthesis of tert-butyl 4-(2-oxobenzo[d]oxazole-3(2H)-yl)piperidine-l-carboxylate

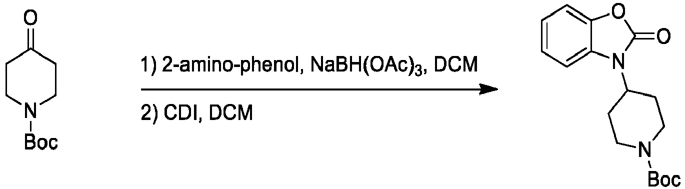

To a stirred solution of tert-butyl 4-oxy-l-piperidine carboxylate (20g, 100 mmol) and 2-aminophenol (11g, 100 mmol) in CH₂Cl₂ (300 mL), NaBH(OAc)₃ (32.4 g, 151 mmol) was added under nitrogen at room temperature. The mixture was stirred at room temperature for 14 h, diluted by adding CH₂Cl₂ (1000 mL) and saturated NaHCO₃ (1500 mL), and separated into layers. The aqueous layer was extracted with CH₂Cl₂ (2* 500ml), the combined organic layer was washed with brine, dried over Na₂SO₄, and the solvent was removed under reduced pressure. The crude material was diluted with CH₂Cl₂ (300 mL), added with carbonyldiimidazole (18 g, 110 mmol), stirred for 16 h, diluted with CH₂Cl₂ (1 L) and IN HCl (1 L), and separated into layers. The aqueous layer was extracted with CH₂Cl₂ (2* 500ml), the combined organic layer was washed with brine, dried over Na₂SO₄, and the solvent was removed under reduced pressure. Purification was performed by silica gel column chromatography (PE: EA = 15:1) to obtain a yellow solid (18.4 g, yield 38%). MS (ESI) *m*/*z* 319.0 [M + H]⁺.

### Step 1.2. Synthesis of 3-(piperidin-4-yl)benzo[d]oxazole-2(3H)-one

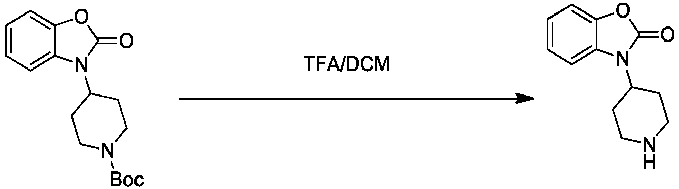

In a solution of CH₂Cl₂ (24ml) was added tert-butyl 4-(2-oxobenzo[d]oxazole-3(2H)-yl)piperidine-l-carboxylate (16.0 g, 50.3 mmol), and TFA (24ml) was slowly added at 0°C. The reaction mixture was concentrated. The residue was treated with saturated NaHCO₃ (200 mL) and CH₂Cl₂ (1000 mL). The separated organic layer was washed with brine, dried over Na₂SO₄, and the solvent was removed under reduced pressure to obtain the desired (11 g, yield: 100%) yellow solid. MS (ESI) *m*/*z* 218.9 [M + H]⁺.

### Step 1.3. Synthesis of 3-(1-propionylpiperidin-4-yl)benzo[d]oxazole-2(3H)-one (P1)

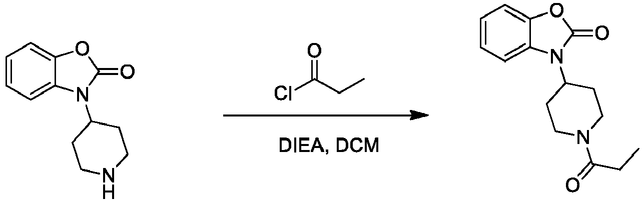

In a solution of 3-(piperidin-4-yl)benzo[d]oxazole-2(3H)-one (1.92 g, 8.8 mmol) and DIEA (1.50 g, 11.6 mmol) in DCM (25ml), propionyl chloride (1.05 g, 11.4 mmol) was added dropwise at 0°C. The reaction mixture was stirred at room temperature for 2 hours, diluted with DCM (100 mL), and washed with aqueous NH₄Cl (200 mL). The separated organic layer was dried over Na₂SO₄, concentrated under reduced pressure to obtain the crude product, which was purified by silica gel column chromatography (PE: EA: DCM = 4:1: ∼ 1:1:1) to obtain the desired white solid (1.1g, yield 46%). ¹H NMR (400 MHz, CDCl₃): δ 7.30-7.08 (m, 4H), 4.93 (d, *J* = 13.2 Hz, 1H), 4.42-4.36 (m, 1H), 4.10 (d, *J =* 13.6 Hz, 1H), 3.20 (t, *J =* 12.8 Hz, 1H), 2.68 (t, *J* = 12.8 Hz, 1H), 2.43 (q, *J* = 7.6 Hz, 2H), 2.31-2.24 (m, 2H), 2.01-1.95 (m, 2H), 1.22 (t, *J* = 7.6 Hz, 3H). MS (ESI) *m*/*z* 275.0 [M + H]⁺.

### Example 2. 3-(1-(3,3,3-trifluoropropionyl)piperidin-4-yl)benzo[d]oxazole-2(3H)-one (P2)

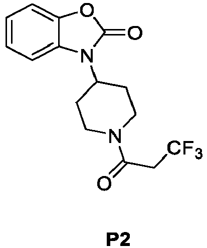

### Step 2.1. 3-(1-(3,3,3-trifluoropropionyl)piperidin-4-yl)benzo[d]oxazole-2(3H)-one (P2)

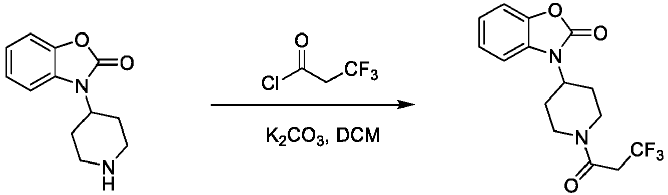

In DCM (15ml) were added 3-(piperidin-4-yl)benzo[d]oxazol-2(3H)-one (1.5 g, 6.88 mmol) and K₂CO₃ (1.99 g, 13.8 mmol), and 3,3,3-trifluoropropyl chloride (1.05 g, 11.4 mmol) was added dropwise at 0°C. The reaction mixture was stirred at room temperature for 4 hours, the reaction mixture was filtered, and the filtrate was concentrated. The product was purified by silica gel column chromatography (PE: EA: DCM = 1:1:1) to obtain the desired white solid (1.75 g, yield 77%). ¹H NMR (400 MHz, CDCl₃): δ 7.23 (dd, *J* = 5.2, 1.2 Hz, 1H), 7.21-7.12 (m, 2H), 7.04 (dd, *J* = 8.0, 1.2 Hz, 1H), 4.94-4.89 (m, 1H), 4.44-4.37 (m, 1H), 4.01-3.97 (m, 1H), 3.38-3.27 (m, 3H), 2.78-2.70 (m, 1H), 2.37-2.23 (m, 2H), 2.04-1.98 (m, 2H). MS (ESI) *m*/*z* 329.1 [M + H]⁺.

### Example 3. 3-(1-(2-(pyridin-4-yl)acetyl)piperidin-4-yl)benzo[d]oxazole-2(3H)-one (P3)

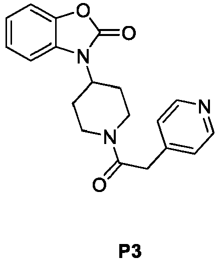

### Step 3.1. Synthesis of 3-(1-(2-(pyridin-4-yl)acetyl)piperidin-4-yl)benzo[d]oxazole-2(3H)-one (P3)

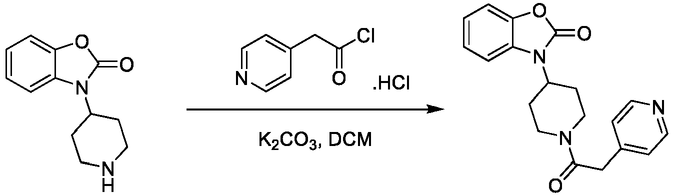

In a solution of 3-(piperidin-4-yl)benzo[d]oxazole-2(3H)-one (1.5 g, 6.88 mmol) and K₂CO₃ (2.8 g, 20.6 mmol) in DCM (20ml), 2-(pyridin-4-yl)acetyl chloride hydrochloride (2.0 g, 10.3 mmol) was added dropwise at 0°C. The reaction mixture was stirred at room temperature for 4 hours, the reaction mixture was filtered, and the filtrate was concentrated. The product was purified by high performance liquid chromatography (20-95% CH₃CN aqueous solution), and a white solid (1.07 g, yield 46%) was obtained. ¹H NMR (400 MHz, DMSO-*d*₆): δ 8.51 (d, *J* = 6.0 Hz, 2H), 7.35-7.29 (m, 4H), 7.23-7.19 (m, 1H), 7.15-7.11 (m, 1H), 4.57 (d, *J* = 13.2 Hz, 1H), 4.43-4.35 (m, 1H), 4.09 (d, *J* = 14.4 Hz, 1H), 3.85 (s, 2H), 3.23-3.16 (m, 1H), 2.76-2.69 (m, 1H), 2.09-1.98 (m, 2H), 1.86-1.81 (m, 2H). MS (ESI) *m*/*z* 338.0 [M + H]⁺.

### Example 4. 3-(1-(2-phenylacetyl)piperidin-4-yl)benzo[d]oxazole-2(3H)-one (P4)

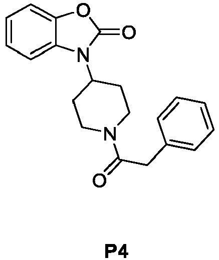

### Step 4.1. Synthesis of 3-(1-(2-phenylacetyl)piperidin-4-yl)benzo[d]oxazole-2(3H)-one (P4)

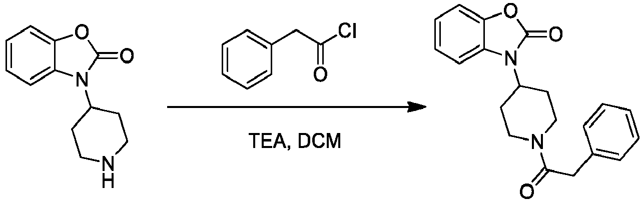

In DCM (15ml) were added 3-(piperidin-4-yl)benzo[d]oxazole-2(3H)-one (1.5 g, 6.88 mmol) and triethylamine (2.1 g, 20.6 mmol), and -phenylacetyl chloride (1.6 g, 10.3 mmol) was added dropwise at 0°C. The reaction mixture was stirred at room temperature for 1 h, and the reaction mixture was concentrated. The product was purified by silica gel column chromatography (PE: EA: DCM = 1:1:1) to obtain the desired white solid (1.16 g, yield 50%). ¹H NMR (400 MHz, CDCl₃) δ 7.39-7.35 (m, 4H), 7.29-7.26 (m, 1H), 7.20-7.18 (m, 1H), 7.11-7.09 (m, 2H), 6.79-6.75 (m, 1H), 4.91-4.88 (m, 1H), 4.39-4.30 (m, 1H), 4.08-4.04 (m, 1H), 3.82 (s, 2H), 3.16-3.08 (m, 1H), 2.70-2.63 (m, 1H), 2.18-2.09 (m, 1H), 1.91-1.78 (m, 1H), 1.77-1.73 (m, 2H). MS (ESI) *m*/*z* 337.2 [M + H]⁺.

### Example 5. 4-(2-oxo-2-(4-(2-oxobenzo[d]oxazole-3(2H)-yl)piperidin-1-yl)ethyl)benzoic acid (P6)

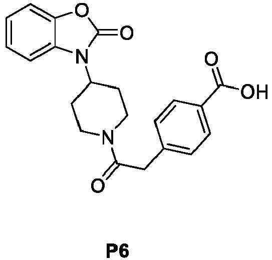

### Step 5.1. 4-(2-oxo-2-(4-(2-oxobenzo[d]oxazole-3(2H)-yl)piperidin-1-yl)ethyl)benzoic acid (P6)

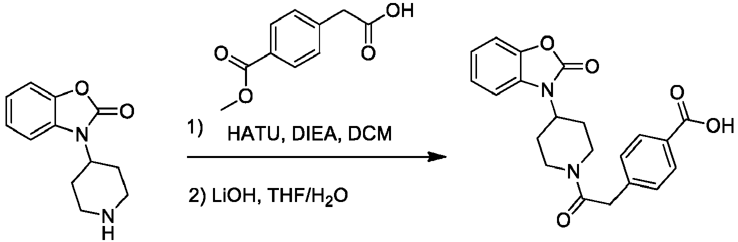

In DCM (20ml) were added 3-(piperidin-4-yl)benzo[d]oxazole-2(3H)-one (1.5 g, 6.88 mmol), 2-(4-(methoxycarbonyl)phenyl)acetic acid (1.42 g, 7.33 mmol) and HATU (3.9 g, 10.2 mmol), and DIEA (2.67 g, 20 mmol) was added at 0°C. The reaction mixture was stirred at room temperature under nitrogen for 2 hours, diluted with DCM (150 mL), and rinsed with aqueous NH₄Cl (200 mL). The separated organic layer was dried over Na₂SO₄, concentrated under reduced pressure to obtain the crude product, which was then purified by silica gel column chromatography (MeOH: DCM = 1:20 ∼ 1:10) to obtain methyl 4-(2-oxo-2-(4-(2-oxobenzo[d]oxazole-3(2H)-yl)piperidin-l-yl)ethyl)benzoate (2.5 g, yield 82%) as a brown solid. methyl 4-(2-oxo-2-(4-(2-oxobenzo[d]oxazole-3(2H)-yl)piperidin-l-yl)ethyl)benzoate (2.0 g, 5.08 mmol) and lithium hydroxide monohydrate (352 mg, 8.2 mmol) were added to THF (80 mL) and H₂O (20 mL). The mixture was stirred at room temperature for 16 h. After removing the organic solvent, the aqueous layer was adjusted to pH 2 and added with 2N HCl, and extracted with EtOAc (3*200ml). The organic layer was dried over Na₂SO₄, concentrated under reduced pressure to obtain the crude product, which was purified by silica gel column chromatography (MeOH: DCM = 1:60 ∼ 1:40) to obtain the desired brown solid (1.01 g, yield: 53%). ¹H NMR (400 MHz, DMSO-d₆): δ 12.78 (brs, 1H), 7.92 (d, *J* = 8.0 Hz, 2H), 7.42 (d, *J* = 8.0 Hz, 2H), 7.33 (d, *J* = 7.6 Hz, 1H), 7.18-7.10 (m, 3H), 4.58 (d, *J* = 13.2 Hz, 1H), 4.42-4.36 (m, 1H), 4.10 (d, *J* = 13.6 Hz, 1H), 3.94-3.83 (m, 2H), 3.21-3.15 (m, 1H), 2.75-2.68 (m, 1H), 2.00-1.76 (m, 4H). MS (ESI) *m*/*z* 381.2 [M + H]⁺.

### Example 6. 3-(1-(2-(p-tolyl)acetyl)piperidin-4-yl)benzo[d]oxazole-2(3H)-one (P7)

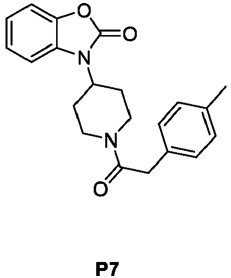

### Step 6.1. Synthesis of 3-(1-(2-(p-tolyl)acetyl)piperidin-4-yl)benzo[d]oxazole-2(3H)-one (P7)

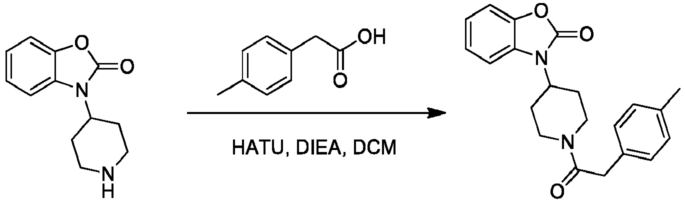

In a solution of 3-(piperidin-4-yl)benzo[d]oxazole-2(3H)-one (1.5 g, 6.88 mmol), 2-(p-tolyl)acetic acid (1.1 g, 7.32 mmol) and HATU (3.9 g, 10.2 mmol) in DCM (20ml), DIEA (2.67 g, 20 mmol) was added at 0°C. The reaction mixture was stirred at room temperature under nitrogen for 2 hours, diluted with DCM (150 mL), and rinsed with aqueous NH₄Cl (200 mL). The separated organic layer was dried over Na₂SO₄, concentrated under reduced pressure to obtain the crude product, which was purified by silica gel column chromatography (MeOH: DCM = 1:25 ∼ 1:10) to obtain the desired white solid (1.2 g, yield: 50%). ¹H NMR (400 MHz, CDCl₃): δ 7.26-7.08 (m, 7H), 6.77-6.75 (m, 1H), 4.90-4.87 (m, 1H), 4.39-4.31 (m, 1H), 4.08-4.04 (m, 1H), 3.77 (s, 2H), 3.15-3.07 (m, 1H), 2.68-2.62 (m, 1H), 2.32 (s, 3H), 2.13-2.07 (m, 1H), 1.93-1.82 (m, 1H), 1.74-1.64 (m, 2H). MS (ESI) *m*/*z* 351.2 [M + H]⁺.

### Example 7. 3-(1-(2-(4-(trifluoromethyl)phenyl)acetyl)piperidin-4-yl)benzo[d]oxazole-2(3H)-one (P8)

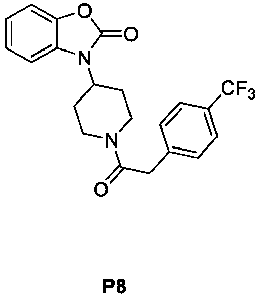

### Step 7.1. Synthesis of 3-(1-(2-(4-(trifluoromethyl)phenyl)acetyl)piperidin-4-yl)benzo[d]oxazole-2(3H)-one (P8)

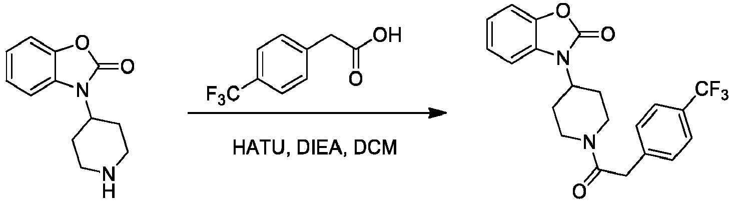

In DCM (27 mL) were added 3-(piperidin-4-yl)benzo[d]oxazole-2(3H)-one (2.0 g, 9.17 mmol), 2-(4-(trifluoromethyl)phenyl)acetic acid (2.24 g, 9.17 mmol) and HATU (5.2 g, 13.8 mmol), and DIEA (3.56 g, 27.5 mmol) was added at 0°C. The reaction mixture was stirred at room temperature under N₂ for 1 h, diluted with DCM (150 mL), and washed with aqueous NH₄Cl (200 mL). The separated organic layer was dried over Na₂SO₄, concentrated under reduced pressure to obtain the crude product, which was purified by silica gel column chromatography (MeOH: DCM = 1:30) to obtain the desired white solid (1.28 g, yield 40%). ¹H NMR (400 MHz, CDCl₃): δ 7.63 (d, *J* = 8.0 Hz, 2H), 7.44 (d, *J* = 7.6 Hz, 2H), 7.23-7.19 (m, 1H), 7.14-7.09 (m, 2H), 6.85-6.83 (m, 1H), 4.88 (d, *J* = 13.2 Hz, 1H), 4.38-4.32 (m, 1H), 4.05 (d, *J* = 14.0 Hz, 1H), 3.86 (s, 2H), 3.22-3.14 (m, 1H), 2.73-2.66 (m, 1H), 2.21-2.17 (m, 1H), 1.99-1.84 (m, 3H). MS (ESI) *m*/*z* 405.2 [M + H]⁺.

### Example 8. 3-(2-(4-fluorobenzyl)-4-oxo-4H-quinazine-8-yl)benzo[d]oxazole-2(3H)-one (P9)

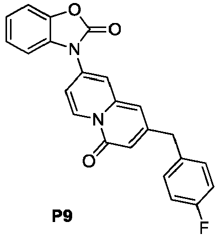

### Step 8.1. Synthesis of 4-methoxypyridinecarbaldehyde

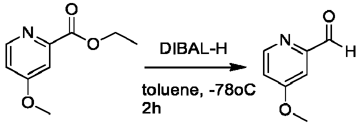

Ethyl 4-methoxypicolinate (94.3 g, 560 mmol) was added to toluene (1.70 L), and DIBAL-H (408 mL, hexane 1.0 M) was added dropwise at -78°C under nitrogen protection. After complete mixing, the mixture was stirred at this temperature for 3 hours. The reaction process was fully monitored by thin-layer chromatography. Before adding aqueous sodium hydroxide solution (2.0 M, 1.5 L), methanol was added to quench the excess DIBAL-H, and the reaction mixture was allowed to reach room temperature. The mixture was extracted with DCM (4×1 L), the mixed organic layer was dried and concentrated to obtain the crude product, which was purified by silica gel column chromatography (PE/EA = 5:1) to obtain the desired yellow gel (42.5 g, yield: 57%). MS (ESI) *m*/*z* 138.1 [M + H]⁺.

### Step 8.2. Synthesis of ethyl 8-methoxy-4-oxo-4H-quinazine-2-carboxylate

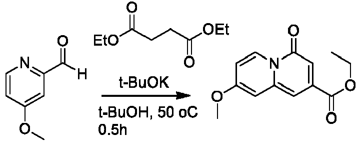

In a mixture of 4-methoxypyridine aldehyde (685 mg, 5.0 mmol) and diethyl succinate (1.74 g, 10.0 mmol) was added a suspension of potassium tert-butoxide (1.12 g, 10 mmol, 25 mL tert-butanol). The reaction mixture was stirred at 50°C for 0.5 h, 15% aqueous acetic acid (40 mL) was added, and the reaction mixture was extracted with EA (2 x 30 mL). Repeating was performed for 61 batches. The EA layers were combined and concentrated. It was stored at room temperature for 2 days until the cyclization reaction was completed. The product was purified by silica gel column chromatography (PE: EA = 2:1), and an ideal yellow solid (11.5 g, yield 15%) was obtained. ¹H NMR (400 MHz, DMSO-d₆): δ 8.96 (d, *J* = 8.0 Hz, 1 H), 7.48 (d, *J* = 2.4 Hz, 1 H), 7.13 (d, *J* = 2.4 Hz, 1 H), 7.07 (dd, *J* = 8.0 Hz, 2.8 Hz, 1 H), 6.55 (d, *J* = 2.0 Hz, 1 H), 4.34 (q, *J* = 7.2 Hz, 2 H), 3.94 (s, 3 H), 1.35 (t, *J* = 7.2 Hz, 3 H). MS (ESI) *m*/*z* 248.1 [M + H]⁺.

### Step 8.3. Synthesis of 2-(hydroxymethyl)-8-methoxy-4H-quinazin-4-one

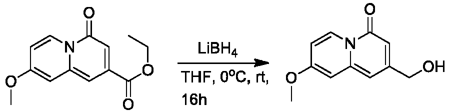

A slurry of ethyl 8-methoxy-4-oxo-4-H-quinazine-2-carboxylate (5.0 g, 20.2 mmol) was added to THF (300 mL), and LiBH4 (1.77 g, 80.4 mmol) was added gradually. The reaction mixture was stirred at room temperature for 24 hours. The reaction mixture was quenched with 15% aqueous acetic acid. After removing the solvent, the precipitate was collected by filtration and concentrated to the desired compound (3.3 g, yield: 79%) as a pale yellow solid. ¹H NMR (400 MHz, DMSO-d6): δ 8.84 (d, *J* = 8.0 Hz, 1 H), 7.11 (d, *J* = 2.8 Hz, 1 H), 6.85 (dd, *J* = 7.6 Hz, 2.4 Hz, 1 H), 6.58 (s, 1 H), 6.13 (s, 1 H), 5.39 (t, *J* = 5.6 Hz, 1 H), 4.90 (d, *J* = 2.8 Hz, 2 H), 3.90 (s, 3 H). MS (ESI) *m*/*z* 206.1 [M + H]⁺.

### Step 8.4. Synthesis of (8-methoxy-4-oxo-4H-quinolin-2-yl)methyl methanesulfonate

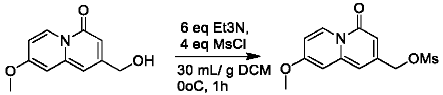

2-(hydroxymethyl)-8-methoxy-4H-quinazin-4-one (3.3 g, 16.0 mmol) and Et₃N (9.69 g, 96.0 mmol) were added in DCM (1.0 L), and MsCl (7.36 g, 64.0 mmol) was added dropwise at 0°C, and the mixture was stirred at 0°C for 1 h. Water was added and the DCM layer was separated, washed with brine, dried with Na₂SO₄ and concentrated to obtain a crude product (5.35 g), which was directly used in the next step reaction. MS (ESI) *m*/*z* 284.0 [M + H]⁺.

### Step 8.5. Synthesis of 2-(bromomethyl)-8-methoxy-4H-quinazin-4-one

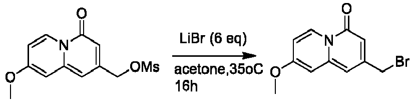

In a solution of methyl (8-methoxy-4-oxo-4H-quinazin-2-yl)methyl methanesulfonate (5.35 g, 16.0 mmol) in acetone (240 mL) was added lithium bromide (8.35 g, 96.0 mmol). The mixture was stirred overnight under nitrogen at 35°C. After the acetone was removed, the residue was dissolved in EA and water, the organic layer was separated, washed with brine, dried over Na₂SO₄ and concentrated to obtain a crude product (3.5 g) as a yellow solid, which was directly used in the next reaction. MS (ESI) *m*/*z* 267.9 and 269.9 [M + H]⁺.

### Step 8.6. Synthesis of 2-(4-fluorobenzyl)-8-methoxy-4H-quinazin-4-one

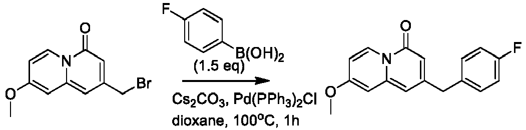

2-(bromomethyl)-8-methoxy-4H-quinazin-4-one (3.5 g, crude product, <16.0 mmol), (4-fluorophenyl)boronic acid (3.36 g, 24.0 mmol), Cs₂CO₃ (10.4 g, 32.0 mmol) and PdCl₂(PPh₃)₂ (1.12 g, 1.60 mmol) were added to dioxane (350 mL), and the mixture was stirred at 100°C for 1 hour. The reaction mixture was extracted by EA. The organic layer was washed with brine, dried with Na₂SO₄ and concentrated, and purified by silica gel column chromatography (DCM:EA = 10:1) to obtain (2.77 g, four steps yield: 48%) a pale yellow solid. ¹H NMR (400 MHz, CDCl₃): δ 8.90 (d, *J* = 8.0 Hz, 1 H), 7.15-7.08 (m, 2 H), 6.96-6.89 (m, 2 H), 6.57 (dd, *J* = 8.4 Hz, 2.8 Hz, 1 H), 6.47 (d, *J* = 2.8 Hz, 1 H), 6.19 (s, 1 H), 6.16 (s, 1 H), 3.81 (s, 3 H), 3.81 (s, 2 H). MS (ESI) *m*/*z* 284.0 [M + H]⁺.

### Step 8.7. Synthesis of 2-(4-fluorobenzyl)-8-hydroxy-4H-quinazin-4-one

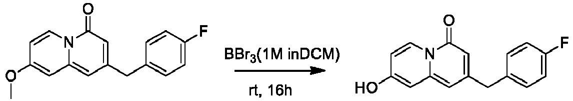

In a cooled solution of 2-(4-fluorobenzyl)-8-methoxy-4H-quinazin-4-one (2.77 g, 9.75 mmol) in dichloromethane was added 55 ml (55.0 mmol) IN boron tribromide solution. The mixture was then stirred at 35°C for 16 hours. The reaction mixture was concentrated; the residue was treated in an aqueous solution of 20 mL of acetonitrile at 0°C. After removing the acetonitrile, the precipitate was collected by filtration to obtain (2.60 g, yield 99%) a yellow solid. ¹H NMR (400 MHz, DMSO-d₆): δ 11.25 (s, 1 H), 8.86 (d, *J=* 7.6 Hz, 1 H), 7.36-7.28 (m, 2 H), 7.17-7.08 (m, 2 H), 6.83-6.75 (m, 2H), 6.38 (d, *J =* 1.2 Hz, 1 H), 5.94 (d, *J =* 1.6 Hz, 1 H), 3.83 (s, 2 H). MS (ESI) *m*/*z* 270.0 [M + H]⁺.

### Step 8.8. Synthesis of 2-(4-fluorobenzyl)-4-oxo-4H-quinoxalin-8-yl trifluoromethanesulfonate

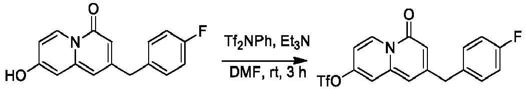

In a solution of 2-(4-fluorobenzyl)-8-hydroxy-4H-quinazin-4-one (2.60 g, 9.63 mmol) added in DMF (13ml), Et₃N (1.46 g, 14.4 mmol) and Tf₂NPh (4.13 g, 11.6 mmol) were added. Under the protection of nitrogen, the mixture was stirred at room temperature for 3 hours. 40 mL of 0°C water was added to the reaction mixture and the mixture was stirred vigorously. The precipitate was collected by filtration to obtain (3.43 g, yield: 89%) a yellow crystal. MS (ESI) *m*/*z* 402.0 [M + H]⁺.

### Step 8.9. Synthesis of trimethyl(2-((2-nitrophenoxy)methoxy)ethyl)silane

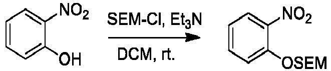

SEM-Cl (6.6 g, 39.6 mmol) was added to a cooled solution of 2-nitrophenol (5.0 g, 36.0 mmol) and Et₃N (5.4 g, 54.0 mmol) in DCM (50 mL). The reaction mixture was stirred at room temperature for 3 hours. The mixture was washed with water and NH₄Cl aqueous solution, dried over Na₂SO₄, and concentrated to obtain a crude product. The crude product was subjected to column chromatography to obtain the desired yellow oily product (5.0 g, yield 70%). ¹H NMR (400 MHz, CDCl₃): δ 7.81 (dd, *J* = 8.0 Hz, 1.6 Hz, 1 H), 7.54-7.46 (m, 1H), 7.35 (dd, *J =* 8.4 Hz, 0.8 Hz, 1 H), 7.10-7.03 (m, 1 H), 5.34 (s, 2 H), 3.84-3.77 (m, 2 H), 0.90-0.93 (m, 2 H), -0.001 (s, 9 H).

### Step 8.10. Synthesis of 2-((2-(trimethylsilyl)ethoxy)methoxy)aniline

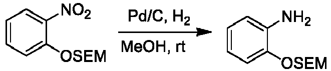

A mixture of trimethyl(2-((2-nitrophenoxy)methoxy)ethyl)silane (5.0 g, 18.6 mmol) and 10% Pd/C (2.5 g) in MeOH (50 mL The mixture in) was stirred at rt under H₂ for 16 hours. The reaction mixture was filtered through celite. After concentration, it was directly put into the next reaction. MS (ESI) *m*/*z* 240.1 [M + H]⁺.

### Step 8.11. Synthesis of 2-(4-fluorobenzyl)-8-((2-((2-(trimethylsilyl)ethoxy)methoxy)phenyl)amino)-4H-quazin-4-one

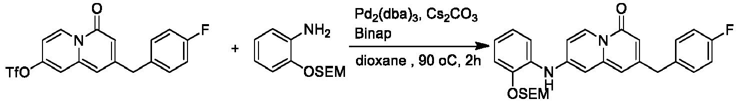

A solution of 2-(4-fluorobenzyl)-4-oxo-4H-quinazolin-8-yl trifluoromethanesulfonate (2.46g, 6.11mmol), 2-((2-(trimethylsilyl)ethoxy)methoxy)aniline (2.20g, 9.17 mmol), Cs₂CO₃ (3.98g, 12.2mmol), BINAP (380mg, 0.611mmol) and Pd₂(dba)₃ (279mg, 0.305mmol)) in dioxane (88mL) was stirred at 90°C under N₂ for 2 hours. The reaction mixture was purified by silica gel column chromatography (PE:EA from 3:1 to 1:1) to obtain the target product (2.25 g, 76% yield) as a yellow solid. MS (ESI) *m*/*z* 491.2 [M + H]⁺.

### Step 8.12. Synthesis of 2-(4-fluorobenzyl)-8-((2-hydroxyphenyl)amino)-4H-quinazin-4-one

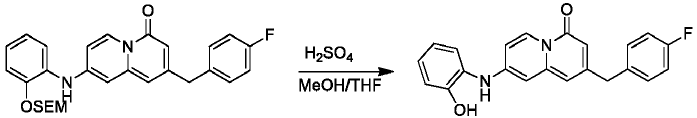

2-(4-fluorobenzyl)-8-((2-((2-(trimethylsilyl)ethoxy)methoxy)phenyl)amino)-4H-quinazine-4-one (2.25 g, 4.59 mmol) in a mixed solvent of MeOH (90 mL) and THF (90 mL) was added dropwise a solution of H₂SO₄ (4.60 mL con. H₂SO₄, 90 mL MeOH). The mixture was stirred at room temperature for 2 hours. The reaction mixture was neutralized with an ice-cold aqueous solution and NaHCO₃ was extracted by EA. The EA layer was concentrated (1.81 g, crude product) and directly put into the next reaction. MS (ESI) *m*/*z* 361.1 [M + H]⁺.

### Step 8.13. Synthesis of 3-(2-(4-fluorobenzyl)-4-oxo-4H-quinazin-8-yl)benzo[d]oxazole-2(3H)-one (P9)

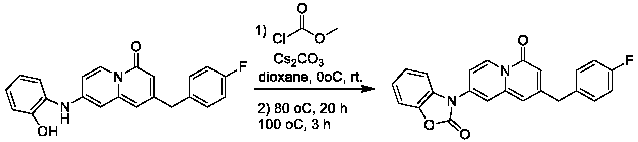

In a cooled solution of 2-(4-fluorobenzyl)-8-((2-hydroxyphenyl)amino)-4H-quinazin-4-one (1.81 g, 5.0 mmol) and Cs₂CO₃ (3.26 g, 10.0 mmol) in dioxane (250 mL), methyl chloroformate (0.95 g, 10.0 mmol) was slowly added dropwise. The mixture was stirred at room temperature overnight. The reaction mixture was then stirred at 80°C for another 20 hours. The resulting mixture was stirred at 100°C for 3 hours. LCMS monitored that the raw material was consumed completely. The reaction mixture was diluted with EA, washed with water and brine. The organic layer was concentrated by dried Na₂SO₄. The crude product was purified by silica gel column chromatography (PE/EA, 10:1 ∼ 3:1) to obtain the target compound (1.20 g, yield 63%). ¹H NMR (400 MHz, DMSO-d6) δ 8.99 (d, J = 8.0 Hz, 1H), 7.95 (d, J = 2.4 Hz, 1H), 7.52-7.46 (m, 1H), 7.46 - 7.40 (m, 2H), 7.40 - 7.33 (m, 2H), 7.33 - 7.24 (m, 2H), 7.20 - 7.11 (m, 2H), 6.83 (s, 1H), 6.38 (d, J = 1.6 Hz, 1H), 3.97 (s, 2H). MS (ESI) *m*/*z* 387.1 [M + H]⁺, purity: 99.2% @ 254 nm, 99.3% @ 214 nm.

### Example 9. 1-(1-propionylpiperidin-4-yl)-1H-benzo[d]imidazole-2(3H)-one (X1)

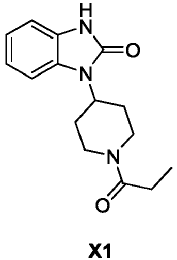

### Step 9.1. Synthesis of 1-(1-propionylpiperidin-4-yl)-1H-benzo[d]imidazole-2(3H)-one (X1)

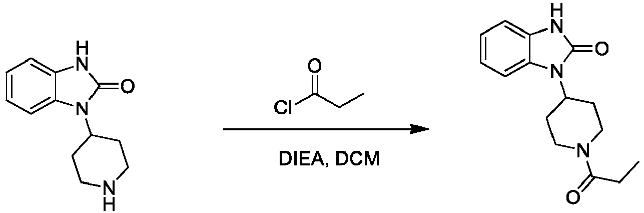

In DCM (40ml) were added 1-(piperidin-4-yl)-1H-benzo[d]imidazole-2(3H)-one hydrochloride (1.40 g, 5.52 mmol) and pyridine (1.74 g, 22.1 mmol), and propionyl chloride (1.53 g, 16.5 mmol) was added dropwise at 0°C. The reaction mixture was stirred at room temperature for 16 h, diluted with water and extracted with DCM. The combined organic layer was dried over Na₂SO₄, concentrated under reduced pressure to obtain a crude product, which was purified by high performance liquid chromatography (20-95% CH₃CN aqueous solution) to obtain the desired white solid (1.18 g, yield 69%). ¹H NMR (400 MHz, DMSO-*d*₆): δ 10.84 (s, 1H), 7.25-7.19 (m, 1H), 7.00-6.97 (m, 3H), 4.58 (d, *J* = 13.2 Hz, 1 H), 4.45-4.37 (m, 1 H), 4.01 (d, *J* = 13.6 Hz, 1 H), 3.18-3.07 (m, 1 H), 2.67-2.59 (m, 1 H), 2.42-2.39 (m, 2 H), 2.37-2.20 (m, 1 H), 2.18-2.06 (m, 1 H), 1.71 (t, *J* = 12.8 Hz, 2 H), 1.02 (m, *J* = 7.6 Hz, 3 H). MS (ESI) *m*/*z* 274.2 [M + H]⁺, purity: 99.6% @ 254 nm, 99.9% @ 214 nm.

### Example 10. 1-(1-(3,3,3-trifluoropropionyl)piperidin-4-yl)-1H-benzo[d]imidazole-2(3H)-one (X2)

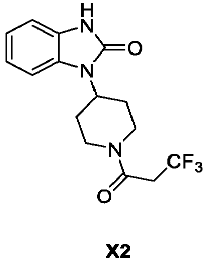

### Step 10.1. Synthesis of 1-(1-(3,3,3-trifluoropropionyl)piperidin-4-yl)-1H-benzo[d]imidazole-2(3H)-one (X2)

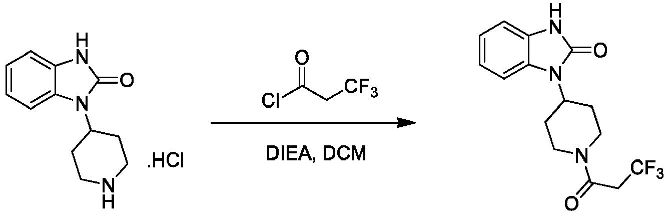

In DCM (20ml) were added 1-(piperidin-4-yl)-1H-benzo[d]imidazole-2(3H)-one hydrochloride (1.50 g, 5.90 mmol) and DIEA (3.04 g, 23.6 mmol), and 3,3,3-trifluoropropyl chloride (1.30 g, 8.86 mmol) was added dropwise at 0°C. The reaction mixture was stirred at room temperature for 2 hours, and the reaction mixture was concentrated. The product was purified by high performance liquid chromatography (10-95% CH₃CN in water) to obtain the desired white solid (1.21 g, yield 63%). ¹H NMR (400 MHz, DMSO-*d*₆): δ 10.85 (s, 1 H), 7.24-7.21 (m, 1 H), 7.00-6.97 (m, 3 H), 4.62-4.53 (m, 1 H), 4.49-4.37 (m, 1 H), 4.03-3.93 (m, 1 H), 3.76-3.68 (m, 2 H), 3.25-3.14 (m, 1 H), 2.75-2.68 (m, 1 H), 2.42-2.28 (m, 1 H), 2.21-2.07 (m, 1 H), 1.78-1.68 (m, 2 H). MS (ESI) *m*/*z* 328.1 [M + H]⁺, purity: 94.5% @ 254 nm, 99.1% @ 214 nm.

### Example 11. 1-(1-(2-(pyridin-4-yl)acetyl)piperidin-4-yl)-1H-benzo[d]imidazole-2(3H)-one (X3)

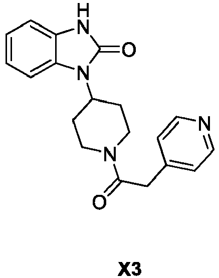

### Step 11.1. Synthesis of 1-(1-(2-(pyridin-4-yl)acetyl)piperidin-4-yl)-1H-benzo[d]imidazole-2(3H)-one (X3)

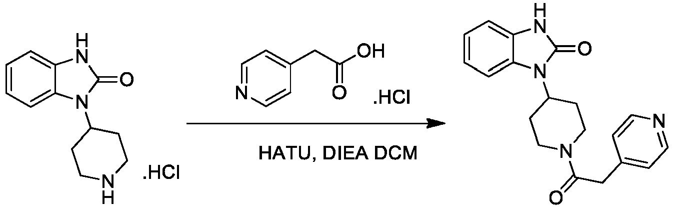

In DCM (20 mL) were added 1-(piperidin-4-yl)-1H-benzo[d]imidazole-2(3H)-one hydrochloride (1.50 g, 5.90 mmol), 2-(pyridin-4-yl)acetic acid hydrochloride (1.55 g, 8.90 mmol) and HATU (6.76 g, 17.8 mmol), and DIEA (3.81 g, 29.5 mmol) was added dropwise at 0°C. The reaction mixture was stirred at room temperature under N₂ for 2 hours, and the reaction mixture was concentrated. The product was purified by high performance liquid chromatography (10-95% CH₃CN aqueous solution) to obtain the desired white solid (1.27 g, yield: 64%). ¹H NMR (400 MHz, DMSO-*d*₆): δ 10.84 (s, 1 H), 8.72-8.35 (m, 2 H), 7.31 (d, *J* = 3.6 Hz, 2 H), 7.15-7.06 (m, 1 H), 7.02-6.89 (m, 3 H), 4.57 (d, *J* = 13.2 Hz, 1 H), 4.45-4.38 (m, 1 H), 4.08 (d, J = 13.6 Hz, 1 H), 3.91-3.79 (m, 2 H), 3.23-3.11 (m, 1 H), 2.78-2.64 (m, 1 H), 2.19-2.05 (m, 2 H), 1.73-1.62 (m, 2 H). MS (ESI) *m*/*z* 337.1 [M + H]⁺, purity: 99.8% @ 254 m, 99.9% @ 214 nm.

### Example 12. 1-(1-(2-phenylacetyl)piperidin-4-yl)-1H-benzo[d]imidazole-2(3H)-one (X4)

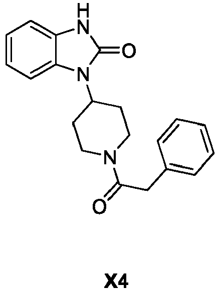

### Step 12.1. Synthesis of 1-(1-(2-phenylacetyl)piperidin-4-yl)-1H-benzo[d]imidazole-2(3H)-one (X4)

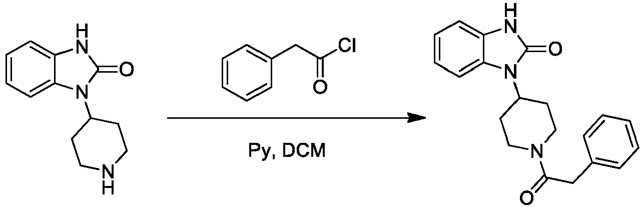

In DCM (40ml) were added 1-(piperidin-4-yl)-1H-benzo[d]imidazole-2(3H)-one hydrochloride (1.50 g, 5.90 mmol) and pyridine (1.87g, 23.6 mmol), and 2-phenylacetyl chloride (2.28 g, 14.8 mmol) was added dropwise at 0°C. The reaction mixture was stirred at room temperature for 16 h, diluted with water and extracted with DCM. The combined organic layer was dried over Na₂SO₄, concentrated under reduced pressure to obtain a crude product, which was purified by high performance liquid chromatography (10-95% CH₃CN aqueous solution) to obtain the desired white solid (1.46 g, yield 63%). ¹H NMR (400 MHz, CDCl₃) δ 10.82 (s, 1 H), 7.38-7.21 (m, 5 H), 6.96-6.95 (m, 4H), 4.57 (d, *J* = 13.2 Hz, 1 H), 4.45-4.35 (m, 1 H), 4.09 (d, *J* = 13.6 Hz, 1 H), 3.84-3.74 (m, 2 H), 3.19-3.08 (m, 1 H), 2.73-2.62 (m, 1 H), 2.11-1.88 (m, 2 H), 1.72-1.54 (m, 2 H). MS (ESI) *m*/*z* 336.1 [M + H]⁺, purity: 97.9% @ 254 nm, 99.9% @ 214 nm.

### Example 13. 4-(2-oxo-2-(4-(2-oxo-2,3-dihydro-1H-benzo[d]imidazol-1-yl)piperidine-1-yl)ethyl)benzoic acid (X6)

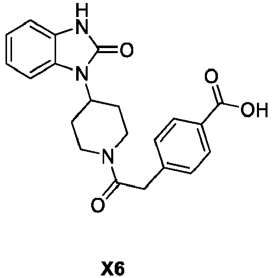

### Step 13.1. 4-(2-oxo-2-(4-(2-oxo-2,3-dihydro-1H-benzo[d]imidazol-1-yl)piperidine-1-yl)ethyl)benzoic acid (X6)

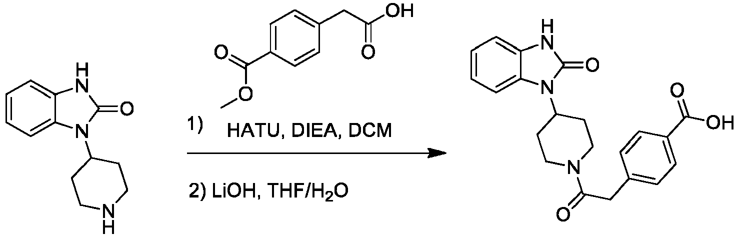

In DCM (50 mL) were added 1-(piperidin-4-yl)-1H-benzo[d]imidazole-2(3H)-one hydrochloride (2.40 g, 8.43 mmol), 2-(4-(methoxycarbonyl)phenyl)acetic acid (1.80 g, 9.28 mmol) and HATU (4.80 g, 12.6 mmol), and DIEA (3.26 g, 25.3 mmol) was added at 0°C. The reaction mixture was stirred at room temperature under N₂ for 2 h. The reaction mixture was diluted with DCM (200 mL) and washed with NH₄Cl aqueous solution (200 mL). The separated organic layer was dried over Na₂SO₄, concentrated under reduced pressure to obtain a crude product, which was purified by high performance liquid chromatography (25-95% CH₃CN aqueous solution) to obtain methyl 4-(2-oxo-2-(4-(2-oxo-2,3-dihydro-1H-benzo[d]imidazol-1-yl)piperidin-1-yl)benzoate (1.6 g, yield 37%) as a white solid. In THF (20ml) and H₂O (20ml) were added methyl 4-(2-oxo-2-(4-(2-oxo-2,3-dihydro-1H-benzo[d]imidazol-1-yl)piperidine-1-benzoate (1.6 g, 4.07 mmol), LiOH.H₂O (855 mg, 2.04 mmol). The resulting mixture was stirred at room temperature for 3 h. After removing the organic solvent, 2N hydrochloric acid was added to adjust the aqueous layer to pH 2. The precipitate was collected and dried to obtain a desired (1.15 g, yield: 75%) white solid. ¹H NMR (400 MHz, DMSO-*d*₆): δ 12.89 (brs, 1 H), 10.83 (s, 1 H), 7.93 (d, *J =* 7.2 Hz, 2 H), 7.43 (d, *J* = 7.2 Hz, 2 H), 6.95 (s, 4 H), 4.57 (d, *J* = 12.0 Hz, 1 H), 4.50-4.32 (m, 1 H), 4.10 (d, *J* = 12.8 Hz, 1 H), 3.96-3.82 (m, 2 H), 3.25-3.12 (m, 1 H), 2.75-2.62 (m, 1H), 2.13-1.88 (m, 2 H), 1.80-1.53 (m, 2 H). MS (ESI) *m*/*z* 380.0 [M + H]⁺, purity: 95.9% @ 254 nm, 98.9% @ 214 nm.

### Example 14. 1-(1-(2-(p-tolyl)acetyl)piperidin-4-yl)-1H-benzo[d]imidazole-2(3H)-one (X7)

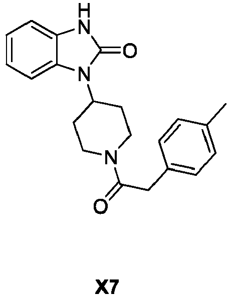

### Step 14.1. Synthesis of 1-(1-(2-(p-tolyl)acetyl)piperidin-4-yl)-1H-benzo[d]imidazole-2(3H)-one (X7)

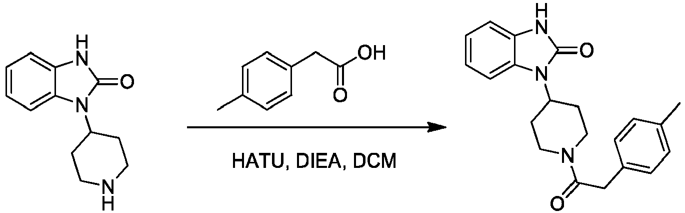

In DCM (20ml) were added 1-(piperidin-4-yl)-1H-benzo[d]imidazole-2(3H)-one hydrochloride (1.50 g, 5.90 mmol), 2-(p-tolyl)acetic acid (1.34 g, 8.90 mmol) and HATU (6.76 g, 17.8 mmol), and DIEA (3.81 g, 29.5 mmol) was added dropwise at 0°C. The reaction mixture was concentrated. The product was purified by high performance liquid chromatography (10-95% CH₃CN aqueous solution), and the desired white solid (1.16 g, yield 56%) was obtained. ¹H NMR (400 MHz, CDCl₃): δ 10.83 (s, 1H), 7.21-7.15 (m, 4H), 6.96-6.90 (m, 4H), 4.57 (d, *J* = 13.2 Hz, 1 H), 4.45-4.32 (m, 1 H), 4.07 (d, *J* = 14.0 Hz, 1 H), 3.82-3.67 (m, 2 H), 3.18-3.07 (m, 1 H), 2.72-2.62 (m, 1 H), 2.27 (s, 3 H), 2.08-1.96 (m, 1 H), 1.95-1.80 (m, 1 H), 1.66 (d, *J* = 10.8 Hz, 1 H), 1.57 (d, *J* = 10.8 Hz, 1 H). MS (ESI) *m*/*z* 350.2 [M + H]⁺, purity: 96.8% @ 254 nm, 99.6% @ 214 nm.

### Example 15. 1-(1-(2-(4-(trifluoromethyl)phenyl)acetyl)piperidin-4-yl)-1H-benzo[d]imidazole-2(3H)-one (X8)

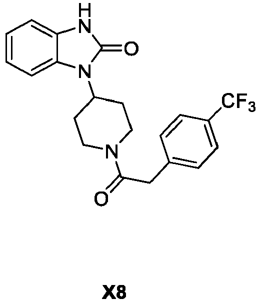

### Step 15.1. Synthesis of 1-(1-(2-(4-(trifluoromethyl)phenyl)acetyl)piperidin-4-yl)-1H-benzo[d]imidazole-2(3H)-one (X8)

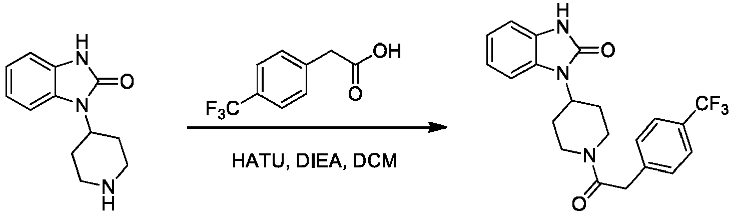

In DCM (50 mL) were added 1-(piperidin-4-yl)-1H-benzo[d]imidazole-2(3H)-one hydrochloride (1.50 g, 5.90 mmol), 2-(4-(trifluoromethyl)phenyl)acetic acid (1.45 g, 7.09 mmol) and HATU (3.37 g, 8.87 mmol), and DIEA (3.81 g, 29.6 mmol) was added at 0°C. The reaction mixture was stirred at room temperature under N₂ for 3h, the reaction mixture was diluted with DCM (100ml) and washed with NH₄Cl aqueous solution (100ml). The separated organic layer was dried over Na₂SO₄, concentrated under reduced pressure to obtain a crude product, which was purified by high performance liquid chromatography (10-50% CH₃CN aqueous solution) to obtain the desired white solid (1.75 g, yield 74%). ¹H NMR (400 MHz, CDCl₃): δ10.84 (s, 1 H), 7.71 (d, *J* = 8.0 Hz, 2 H), 7.53(d, *J* = 8.0 Hz, 2 H), 7.09-7.03 (m, 1 H), 6.98-6.92 (m, 3H), 4.57 (d, *J* = 13.2 Hz, 1 H), 4.45-4.32 (m, 1 H), 4.13 (d, *J* = 14.0 Hz, 1 H), 3.98-3.85 (m, 2 H), 3.19 (t, *J* = 12.0 Hz, 1 H), 2.71 (t, *J* = 12.0 Hz, 1 H), 2.16-2.01 (m, 2 H), 1.68 (m, t, *J* = 12.0 Hz, 2 H). MS (ESI) *m*/*z* 404.1 [M + H]⁺, purity: 92.4% @ 254 nm, 99.7% @ 214 nm.

### Example 16. 2-(4-fluorobenzyl)-8-(2-oxo-2,3-dihydro-1H-benzo[d]imidazol-1-yl)-4H-quinazine-4-one (X9)

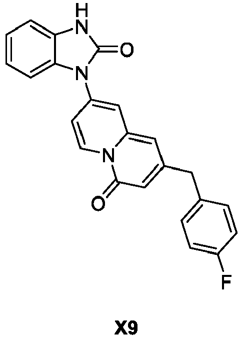

### Step 16.1. Synthesis of 2-(4-fluorobenzyl)-8-(2-oxo-2,3-dihydro-1H-benzo[d]imidazol-1-yl)-4H-quinazine-4-one (X9)

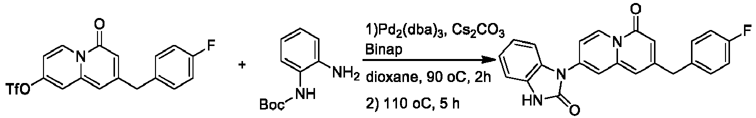

2-(4-fluorobenzyl)-4-oxo-4H-quinazine-8-yl trifluoromethanesulfonate (2.54 g, 6.32 mmol), tert-butyl (2-aminophenyl)carbamate (1.97 g, 9.48 mmol), Cs₂CO₃ (4.12 g, 9.11 mmol), BINAP (390 mg, 0.63 mmol), Pd₂(dba)₃ (270 mg, 0.32 mmol) in dioxane (70 mL) were stirred at 90°C under nitrogen for 2 hours. The reaction mixture was diluted with dioxane (0.02 M) and stirred at 110°C for 5 hours. Finally, the reaction mixture was cooled to rt, the solvent was filtered off, the filter cake was dissolved with EA, and washed with water. The EA layer was dried and concentrated to obtain a crude product. The crude product was recrystallized by EA to obtain the desired compound (1.57 g, yield 64%) as a yellow solid. ¹H NMR (400 MHz, DMSO-d6) δ 11.41 (s, 1H), 8.97 (d, J = 8.0 Hz, 1H), 7.89 (d, J = 2.4 Hz, 1H), 7.44 (dd, J = 8.0, 2.4 Hz, 1H), 7.41 - 7.26 (m, 3H), 7.21 -7.00 (m, 5H), 6.79 (s, 1H), 6.33 (d, J = 1.2 Hz, 1H), 3.95 (s, 2H). MS (ESI) *m*/*z* 386.1 [M + H]⁺, purity: 98.3% @ 254 nm, 98.9% @ 214 nm.

### Example 17. 4-(trifluoromethyl)-1-(1-(2-(4-(trifluoromethyl)phenyl)acetyl)piperidin-4-yl)-1H-benzo[d]imidazole-2(3H)-one (GEN1-001)

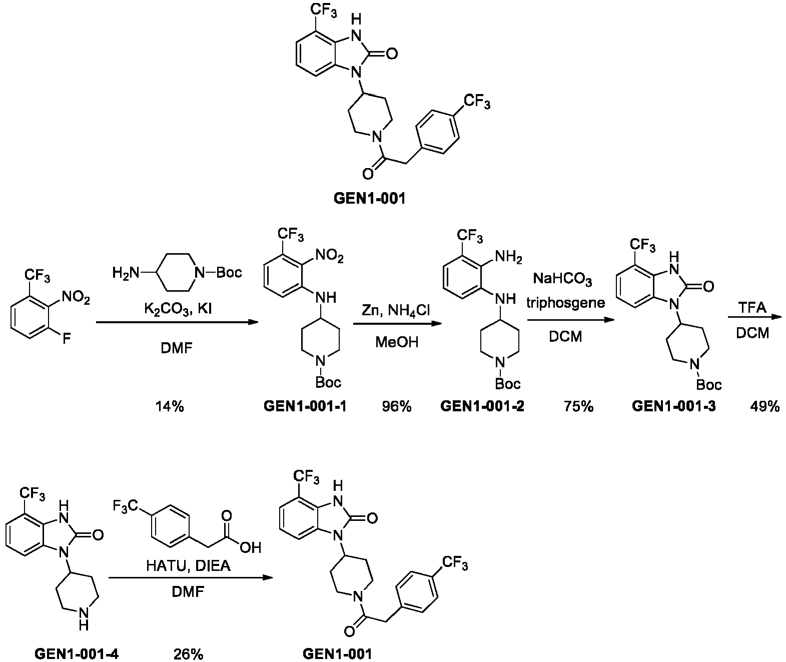

### Step 17.1. Synthesis of tert-butyl 4-((2-nitro-3-(trifluoromethyl)phenyl)amino)piperidine-1-carboxylate (GEN1-001-1)

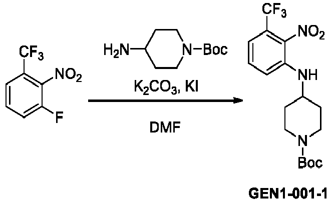

1-fluoro-2-nitro-3-(trifluoromethyl)benzene (1.50 g, 6.60 mmol), tert-butyl 4-aminopiperidine-1-carboxylate (1.60 g, 8.00 mmol), K₂CO₃ (1.80 g, 130 mmol) and KI (12 mg, 0.07 mmol) were suspended in DMF (12 mL) and stirred at 120°C for 20 hours. The reaction mixture was cooled, poured into 60 ml of water, and extracted with EtOAc (30 ml × 3). The combined organic layer was washed with 30 ml of brine, dried over MgSO₄ and concentrated. The crude product was purified by silica gel column chromatography (PE: EtOAc = 5:1 ∼ 3:1) to obtain the desired compound (360 mg, yield 14%) as an orange solid. MS (ESI) Rt = 1.85 min, *m*/*z* no [M+H]⁺ found, purity: 100% @ 254 nm, 76.33% @ 214 nm.

### Step 17.2. Synthesis of tert-butyl 4-((2-amino-3-(trifluoromethyl)phenyl)amino)piperidine-1-carboxylate (GEN1-001-2)

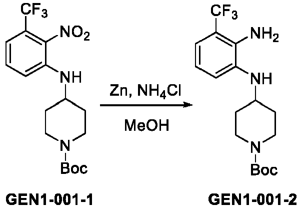

A suspension of tert-butyl 4-((2-nitro-3-(trifluoromethyl)phenyl)amino)piperidine-1-carboxylate (360 mg, 0.924 mmol), zinc powder (362 mg, 5.54 mmol) and NH₄Cl (494 mg, 9.23 mmol) was stirred in methanol (5ml) at 55°C overnight. The reaction mixture was filtered, and the filtrate was concentrated. The residue was separated between water (30ml) and ethyl acetate (30ml). The aqueous layer was extracted with EtOAc (30ml×3), the combined organic layer was washed with brine (30ml), dried over MgSO₄, filtered and concentrated to obtain the desired compound (320 mg, yield 96%) as a brown solid. ¹HNMR (400 MHz, CDCl₃): δ 7.01 (d, *J* = 8.0 Hz, 1H), 6.85-6.77 (m, 2H), 4.14-3.98 (m, 2H), 3.94 (s, 2H), 3.42-3.32 (m, 1H), 3.14 (s, 1H), 2.98-2.92 (m, 2H), 2.02-1.99 (m, 2H), 1.47 (s, 9H), 1.41-1.35 (m, 2H).

### Step 17.3. Synthesis of tert-butyl 4-(2-oxo-4-(trifluoromethyl)-2,3-dihydro-1H-benzo[d]imidazol-1-yl)piperidine-1-carboxylate (GEN1-001-3)

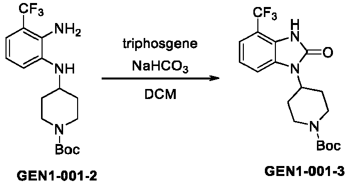

A suspension of tert-butyl 4-((2-amino-3-(trifluoromethyl)phenyl)amino)piperidine-1-carboxylate (260 mg, 0.723 mmol) and NaHCO₃ (182 mg, 2.17 mmol) in DCM (10ml) was added with triphosgene (215 mg, 0.723 mmol) under ice bath. The resulting mixture was stirred overnight. The reaction mixture was poured into 50 mL of water, extracted with DCM (30 mL×3), and the combined organic layer was washed with brine (30 mL), dried over MgSO₄, filtered and concentrated to obtain the desired compound (210 mg, yield 75%) as a brown solid. MS (ESI) Rt = 1.66 min, *m*/*z* 286.0 [M+H-Boc]⁺, purity: 34% @ 254 nm, 49% @ 214 nm.

### Step 17.4. Synthesis of 1-(piperidin-4-yl)-4-(trifluoromethyl)-1H-benzo[d]imidazole-2(3H)-one (GEN1-001-4)

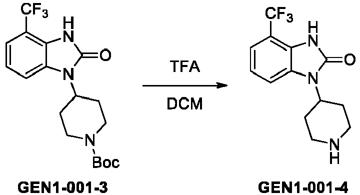

In DCM (4.5 mL) was added tert-butyl 4-(2-oxo-4-(trifluoromethyl)-2,3-dihydro-1H-benzo[d]imidazol-1-yl)piperidine -1-carboxylate (210 mg, 0.545 mmol), and TFA (1.5 mL) was added at room temperature, and the mixture was stirred overnight. The reaction mixture was concentrated and the residue was dissolved in DCM (10ml). Na₂CO₃ (sat., 20ml) was added to the solution and the mixture was stirred for 15 min. The mixture was extracted with DCM (20ml×3). The mixed organic layer was dried over MgSO₄, filtered and concentrated to obtain the desired compound (76 mg, yield 49%) as a brown oil. MS (ESI) Rt = 1.29 min, *m*/*z* 286.2 [M+H]⁺, purity: 41% @ 254 nm, 81% @ 214 nm.

### Step 17.5. Synthesis of 4-(trifluoromethyl)-1-(1-(2-(4-(trifluoromethyl)phenyl)acetyl)piperidin-4-yl)-1H-benzo[d]imidazole-2(3H)-one (GEN1-001)

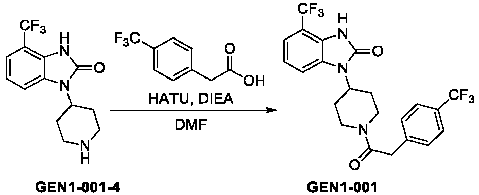

In a solution of 1-(piperidin-4-yl)-4-(trifluoromethyl)-1H-benzo[d]imidazole-2(3H)-one (76 mg, 0.27 mmol), 2-(4-(trifluoromethyl)phenyl)acetic acid (55 mg, 0.27 mmol), DIEA (105 mg, 0.810 mmol) in DMF (2 mL), HATU (156 mg, 0.410 mmol) was added in batches at room temperature . The resulting mixture was stirred at room temperature for 1.5 hours. The reaction mixture was poured into 50 mL of water, extracted with EtOAc (25 mL 3), and the combined organic layer was washed with brine (25 mL), dried over MgSO₄, filtered and concentrated. The crude product was purified by pretreated thin layer chromatography (DCM: MeOH = 15:1) to obtain the desired compound (32.6 mg, yield 26%) as a white solid. ¹H NMR (400 MHz, CDCl₃) δ 8.43 (s, 1H), 7.64 (d, *J* = 8.0 Hz, 2H), 7.46 (d, *J* = 8.0 Hz, 2H), 7.28-7.26 (m, 1H), 7.11 (t, *J* = 8.0 Hz, 1H), 6.99 (d, *J* = 8.0 Hz, 1H), 4.91-4.88 (m, 1H), 4.56-4.47 (m, 1H), 4.08-4.05 (m, 1H), 3.87 (s, 2H), 3.24-3.16 (m, 1H), 2.75-2.68 (m, 1H), 2.29-2.18 (m, 1H), 2.06-1.95 (m, 1H), 1.91-1.88 (m, 1H), 1.82-1.79 (m, 1H). MS (ESI) Rt = 3.942 min, *m*/*z* 472.2 [M+H]⁺, purity: 95.72 @ 254 nm, 99.61% @ 214 nm.

### Example 18. 4-methoxy-1-(1-(2-(4-(trifluoromethyl)phenyl)acetyl)piperidin-4-yl)-1H-benzo[d]imidazole-2(3H)-one (GEN1-003)

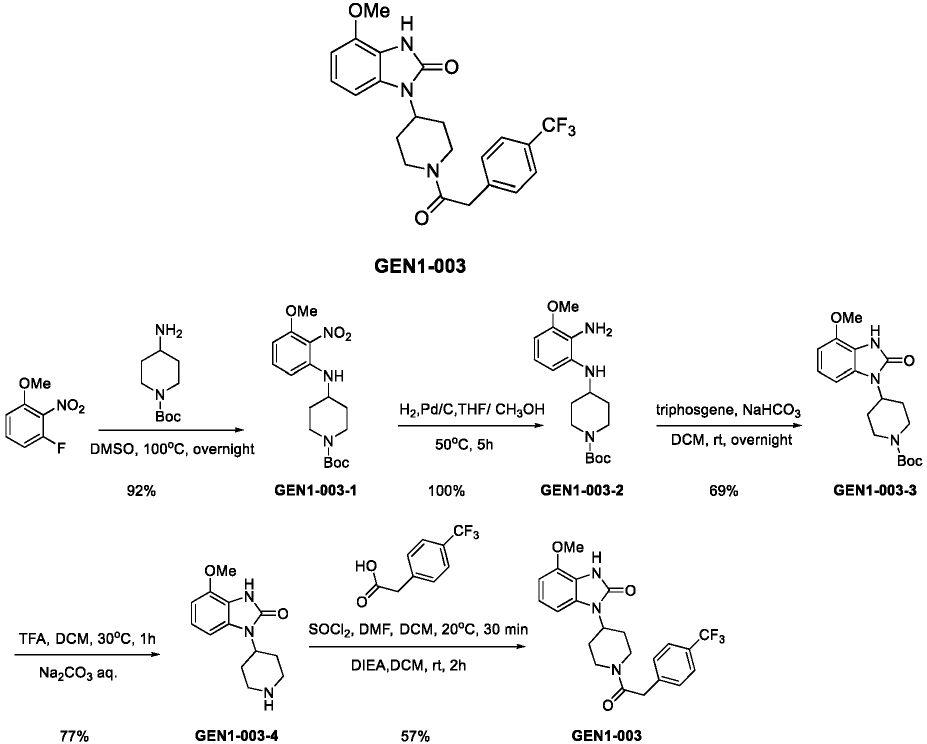

### Step 18.1. Synthesis of tert-butyl 4-((3-methoxy-2-nitrophenyl)amino)piperidine-1-carboxylate (GEN1-003-1)

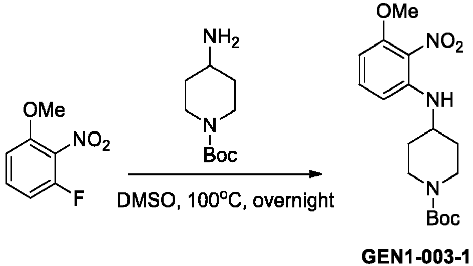

To a solution of 1-fluoro-3-methoxy-2-nitrobenzene (1.00 g, 5.85 mmol) and tert-butyl 4-aminopiperidine-1-carboxylate (1.29 g, 6.43 mmol) in DMSO (20 mL), K₂CO₃ (1.61 g, 11.7 mmol) was added. The mixture was stirred at 100°C overnight. After cooling, it was poured into 150 mL of water, and the mixture was stirred for 10 min, and extracted with EtOAc (3×30 mL). The combined organic layer was washed with brine (20 mL), dried over Na₂SO₄, and concentrated to the desired red solid (1.88 g, yield 92%). MS (ESI) Rt = 1.712 min, *m*/*z* 374.1 [M+Na]⁺, purity: 62.4% @ 214 nm.

### Step 18.2. Synthesis of tert-butyl 4-((2-amino-3-methoxyphenyl)amino)piperidine-1-carboxylate (GEN1-003-2)

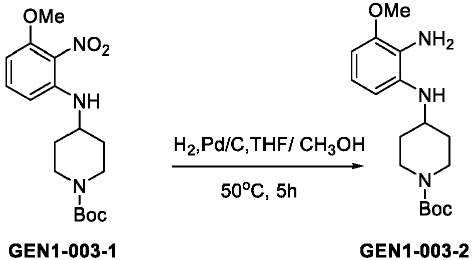

Tert-butyl 4-((3-methoxy-2-nitrophenyl)amino)piperidine-1-carboxylate (1.85 g, 5.27 mmol) was added to a solution of THF (15ml) and methanol (15ml), and Pd/C (10%, 200 mg) was added. The mixture was stirred under a hydrogen atmosphere at 50°C for 5 hours. The mixture was then filtered through celite. The filtrate was concentrated to the desired brown oil (1.69 g, 100% yield). MS (ESI) Rt = 1.660 min, *m*/*z* 322.2 [M+H]⁺, purity: 68.6% @ 214 nm.

### Step 18.3. Synthesis of tert-butyl 4-(4-methoxy-2-oxo-2,3-dihydro-1H-benzo[d]imidazol-1-yl)piperidine-1-carboxylate (GEN1-003-3)

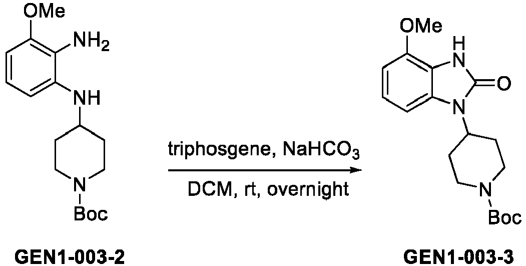

In a solution of tert-butyl 4-((2-amino-3-methoxyphenyl)amino)piperidine-1-carboxylate (1.65 g, 5.14 mmol) in DCM (50ml) was added NaHCO₃ (4.32 g, 51.4 mmol). The mixture was cooled to 0°C. Then 760 mg, 2.57 mmol triphosgene was added. The mixture was stirred at room temperature overnight. Then the mixture was poured into water (50 mL) and extracted with DCM (2 x 50 mL). The combined organic layer was washed with brine (20 mL), dried over Na₂SO₄ and concentrated. The residue was purified by silica gel column chromatography (PE: EA = 3:1 ∼ 1:1) to obtain the desired compound (1.22 g, yield 69%) as a yellow solid. ¹H NMR (400 MHz, CDCl₃): δ 8.75-8.72 (m, 1H), 6.99 (t, *J* = 8.4 Hz, 1H), 6.79 (d, *J* = 8.0 Hz, 1H), 6.63 (d, *J* = 8.0 Hz, 1H), 4.48 (t, *J* = 8.4 Hz, 1H), 4.36-4.24 (m, 2H), 3.92 (s, 3H), 2.89-2.84 (m, 2H), 2.34-2.26 (m, 2H), 1.85-1.82 (m, 2H), 1.50 (s, 9H). MS (ESI), Rt = 1.543 min, *m*/*z* 370.2 [M+Na]⁺ purity: 84.7% @ 214 nm.

### Step 18.4. Synthesis of 4-methoxy-1-(piperidin-4-yl)-1H-benzo[d]imidazole-2(3H)-one (GEN1-003-4)

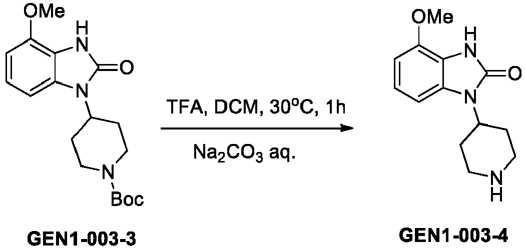

In DCM (2ml) were added tert-butyl 4-(4-methoxy-2-oxo-2,3-dihydro-1H-benzo[d]imidazol-1-yl)piperidine-1-carboxylate (200mg, 0.58 mmol) and TFA (1 mL). The mixture was stirred at 30°C for 1 hour. The mixture was concentrated. The residue was diluted with water (2 mL), and Na₂CO₃ aqueous solution (sat., 5 mL) was added. The mixture was extracted with DCM: MeOH = 10:1 (4×10 mL). The combined organic layer was washed with brine (20 mL), dried over Na₂SO₄, and concentrated to the desired (110 mg, yield: 77%) white solid. MS (ESI) Rt = 1.048 min, *m*/*z* 248.1 [M+H]⁺, purity: 95.5% @ 214 nm.

### Step 18.5. Synthesis of 4-methoxy-1-(1-(2-(4-(trifluoromethyl)phenyl)acetyl)piperidin-4-yl)-1H-benzo[d]Imidazole-2(3H)-one (GEN1-003)

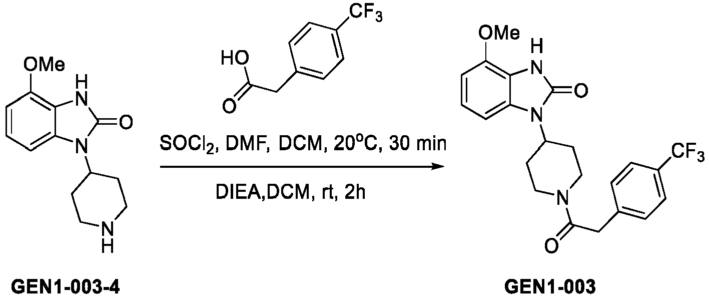

In a solution of 2-(4-(trifluoromethyl)phenyl)acetic acid (100 mg, 0.490 mmol) in DCM (2 mL) was added DMF (1 drop), and then SOCl₂ (106 mg, 0.89 mmol) was added. After stirring for 30 minutes at 20°C, the mixture was concentrated. The residue was dissolved in DCM (1 mL) and used in the next step. 4-methoxy-1-(piperidin-4-yl)-1H-benzo[d]imidazole-2(3H)-one (110 mg, 0.450 mmol) and DIEA (287 mg, 2.23 mmol) in DCM (2 mL) was stirred at room temperature for 10 min, and the previous solution was added dropwise. The mixture after addition was stirred at room temperature for 2 hours. The mixture was poured into water (10 mL) and extracted with DCM (3×10 mL). The combined organic layer was washed with brine (10ml), dried over Na₂SO₄ and concentrated. The crude product was purified using high performance liquid chromatography (Xbridge C18, 5 um, 19 x 150 mm, 20-70% B, A: H₂O (0.1% NH₄HCO₃), B: CH₃CN, uv = 214 nm, F = 15 mL/min) to obtain the desired compound (110 mg, yield 57%) as a white solid. ¹H NMR (400 MHz, CDCl₃): δ 8.19 (s, 1H), 7.63 (d, *J* = 8.0 Hz, 2H), 7.45 (d, *J* = 8.0 Hz, 2H), 6.95 (t, *J* = 8.4 Hz, 1H), 6.63 (d, *J* = 8.4 Hz, 2H), 6.50 (d, *J* = 8.0 Hz, 2H), 4.87 (d, *J* = 13.2 Hz, 1H), 4.54-4.58 (m, 1H), 4.04 (d, *J* = 13.6 Hz, 1H), 3.90 (s, 3H), 3.86 (s, 2H), 3.22-3.15 (m, H), 2.74-2.67 (m, 1H), 2.28-2.17 (m, 1H), 2.05-1.95 (m, 1H), 1.90-1.87 (m, 1H), 1.80-1.78 (m, 1H). MS (ESI) Rt = 3.448 min, *m*/*z* 434.2 [M+H]⁺, purity: 99.0% @ 254 nm, 99.6% @ 214 nm.

### Example 19. 5-(trifluoromethyl)-1-(1-(2-(4-(trifluoromethyl)phenyl)acetyl)piperidin-4-yl)-1H-benzo[d]imidazole-2(3H)-one (GEN1-005)

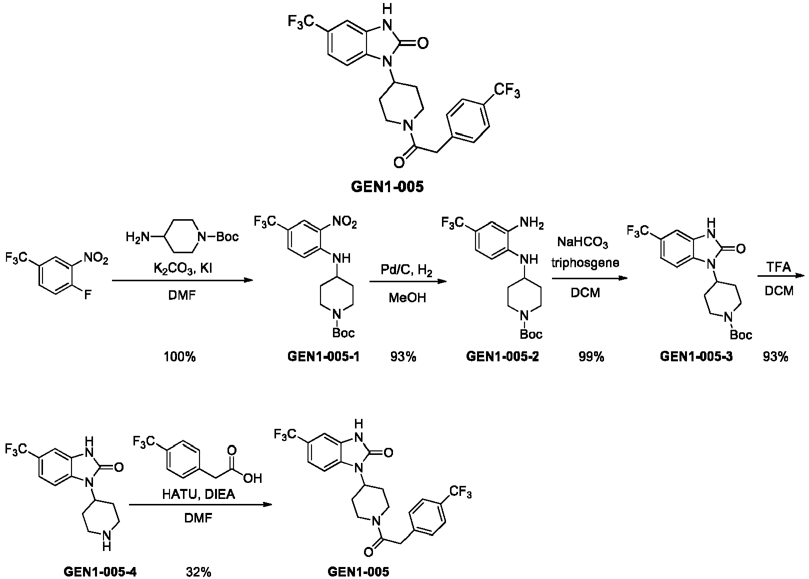

### Step 19.1. Synthesis of tert-butyl 4-((2-nitro-4-(trifluoromethyl)phenyl)amino)piperidine-1-carboxylate (GEN1-005-1)

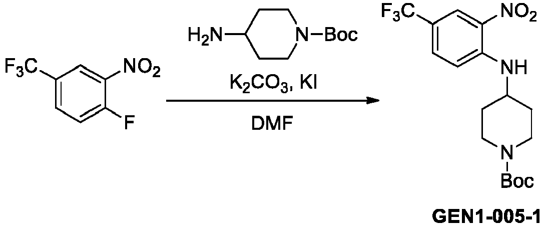

1-fluoro-2-nitro-4-(trifluoromethyl)benzene (1.00 g, 4.80 mmol), tert-butyl 4-aminopiperidine-1-carboxylate (961 mg, 4.80 mmol) and K₂CO₃ (995 mg, 7.20 mmol) were suspended in DMF (10 mL) and the mixture was stirred at 85°C for 2 hours. After cooling to room temperature, the reaction mixture was poured into water (50 mL) and extracted with EtOAc (25 mL×3). The combined organic layer was washed with water (25 mL), brine (25 mL), dried over MgSO₄ and concentrated to obtain the desired compound (1.86 g, yield 100%) as a yellow solid. ¹HNMR (400 MHz, CDCl₃): δ 8.48 (d, *J* = 1.2 Hz, 1H), 8.31 (d, *J* = 7.6 Hz, 1H), 7.61 (dd, *J* = 9.2, 2.0 Hz, 1H), 6.97 (d, *J* = 9.2 Hz, 1H), 4.06-4.03 (m, 2H), 3.76-3.67 (m, 1H), 3.09-3.04 (m, 2H), 2.09-2.04 (m, 2H), 1.61-1.54 (m, 2H), 1.48 (s, 9H).

### Step 19.2. Synthesis of tert-butyl 4-((2-amino-4-(trifluoromethyl)phenyl)amino)piperidine-1-carboxylate (GEN1-005-2)

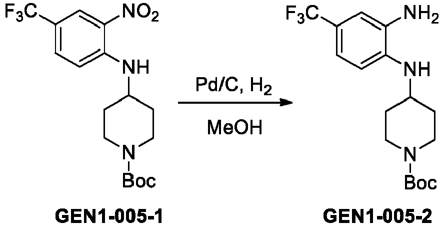

Tert-butyl 4-((2-nitro-4-(trifluoromethyl)phenyl)amino)piperidine-1-carboxylate (1.86 g, 4.78 mmol) was suspended in methanol (15ml) , and Pd/C (10%, 186 mg) was added under nitrogen atmosphere. The resulting mixture was stirred overnight under a hydrogen atmosphere at 35°C. The reaction mixture was filtered, and the filtrate was concentrated to obtain the desired compound (1.60 g, yield 93%) as a gray solid. ¹HNMR (400 MHz, CDCl₃): δ 7.08-7.06 (m, 1H), 6.94 (d, *J* = 2.0 Hz, 1H), 6.65 (d, *J* = 8.4 Hz, 1H), 4.06-4.04 (m, 2H), 3.70-3.24 (m, 4H), 3.00-2.94 (m, 2H), 2.06-2.02 (m, 2H), 1.47 (s, 9H), 1.47-1.36 (m, 2H).

### Step 19.3. Synthesis of tert-butyl 4-(2-oxo-5-(trifluoromethyl)-2,3-dihydro-1H-benzo[d]imidazol-1-yl)piperidine-1-carboxylate (GEN1-005-3)

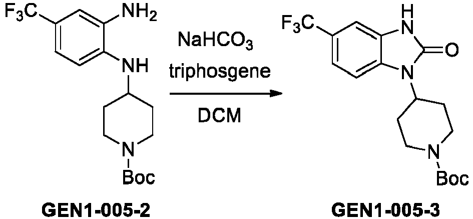

In a suspension of tert-butyl 4-((2-amino-4-(trifluoromethyl)phenyl)amino)piperidine-1-carboxylate (500 mg, 1.39 mmol) and NaHCO₃ (584 mg, 6.95 mmol) was added triphosgene (206 mg, 0.694 mmol). The resulting mixture was stirred for 2 hours. The reaction mixture was poured into water (50 mL), extracted with DCM (25 mL 3), and the combined organic layer was washed with brine (25 mL), dried over MgSO₄, and concentrated to give the desired compound (530 mg, yield 99 %) as a white solid. ¹HNMR (400 MHz, CDCl₃): δ 9.87 (s, 1H), 7.38 (s, 1H), 7.35 (d, *J* = 8.8 Hz, 1H), 7.20 (d, *J* = 8.4 Hz, 1H), 4.54-4.45 (m, 1H), 4.36-4.33 (m, 2H), 2.92-2.85 (m, 2H), 2.38-2.27 (m, 2H), 1.87-1.84 (m, 2H), 1.52 (s, 9H).

### Step 19.4. Synthesis of 1-(piperidin-4-yl)-5-(trifluoromethyl)-1H-benzo[d]imidazole-2(3H)-one (GEN1-005-4)

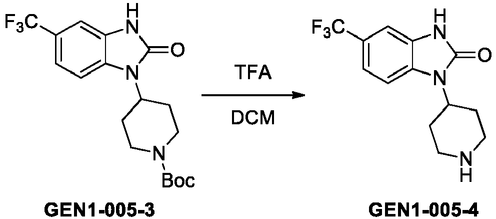

In DCM (6mL) was added tert-butyl 4-(2-oxo-5-(trifluoromethyl)-2,3-dihydro-1H-benzo[d]imidazol-1-yl)piperidine-1-carboxylate (530 mg, 1.38 mmol), and TFA (2ml) was added at room temperature. The resulting mixture was stirred overnight. The reaction mixture was concentrated, and the residue was dissolved in DCM (10 mL). Na₂CO₃ (10 mL) was added to the solution and the mixture was stirred for 20 minutes. The mixture was extracted with DCM (25 mL×3). The combined organic layer was washed with brine (25 mL), dried over MgSO₄ and concentrated to the desired compound (368 mg, yield 93%) as a white solid. ¹HNMR (400 MHz, CDCl₃): δ 7.39-7.36 (m, 3H), 4.52-4.42 (m, 1H), 3.34-3.31 (m, 2H), 2.88-2.82 (m, 2H), 2.46-2.35 (m, 2H), 1.89-1.86 (m, 2H).

### Step 19.5. Synthesis of 5-(trifluoromethyl)-1-(1-(2-(4-(trifluoromethyl)phenyl)acetyl)piperidin-4-yl)-1H-benzo[d]imidazole-2(3H)-one (GEN1-005)

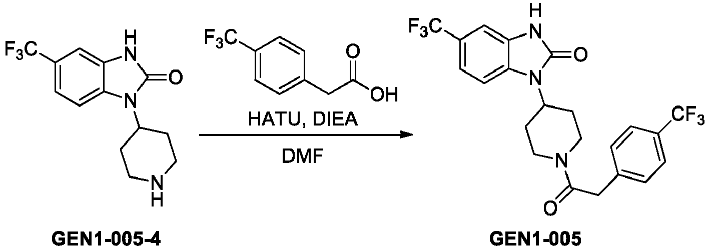

In DMF (2 mL) were added 1-(piperidin-4-yl)-5-(trifluoromethyl)-1H-benzo[d]imidazole-2(3H)-one (70 mg, 0.25 mmol), 2-[4-(trifluoromethyl)phenyl]acetic acid (60 mg, 0.29 mmol) and DIEA (95 mg, 0.74 mmol), and HATU (140 mg, 0.368 mmol) was gradually added at room temperature. The resulting mixture was stirred at room temperature for 1.5 hours. Purification of the reaction mixture: high performance liquid chromatography (Xbridge C18 5 µm 19 * 150 mm; B, A: H₂O (0.1% NH₄HCO₃), B: CH₃CN; UV: 214 nm, flow rate: 15ml/min). The desired compound (37.2 mg, yield 32%) was obtained as a white solid. ¹H NMR (400 MHz, CDCl₃) δ 7.94 (s, 1H), 7.64 (d, *J* = 8.0 Hz, 2H), 7.46 (d, *J* = 8.0 Hz, 2H), 7.34 (s, 1H), 7.31 (d, *J* = 8.4 Hz, 1H), 6.93 (d, *J* = 8.4 Hz, 1H), 4.93-4.90 (m, 1H), 4.59-4.47 (m, 1H), 4.10-4.06 (m, 1H), 3.88 (s, 2H), 3.22 (t, *J* = 12.8 Hz, 1H), 2.73 (t, *J* = 12.8 Hz, 1H), 2.32-2.21 (m, 1H), 2.09-1.98 (m, 1H), 1.92-1.89 (m, 1H), 1.84-1.81 (m, 1H). MS (ESI) Rt = 3.296 min, *m*/*z* 472.1 [M+H]⁺, purity: 98.62 @ 254 nm, 99.24% @ 214 nm.

### Example 20. 2-oxo-1-(1-(2-(4-(trifluoromethyl)phenyl)acetyl)piperidin-4-yl)-2,3-dihydro-1H-benzo[d]imidazole-5-carbonitrile (GEN1-006)

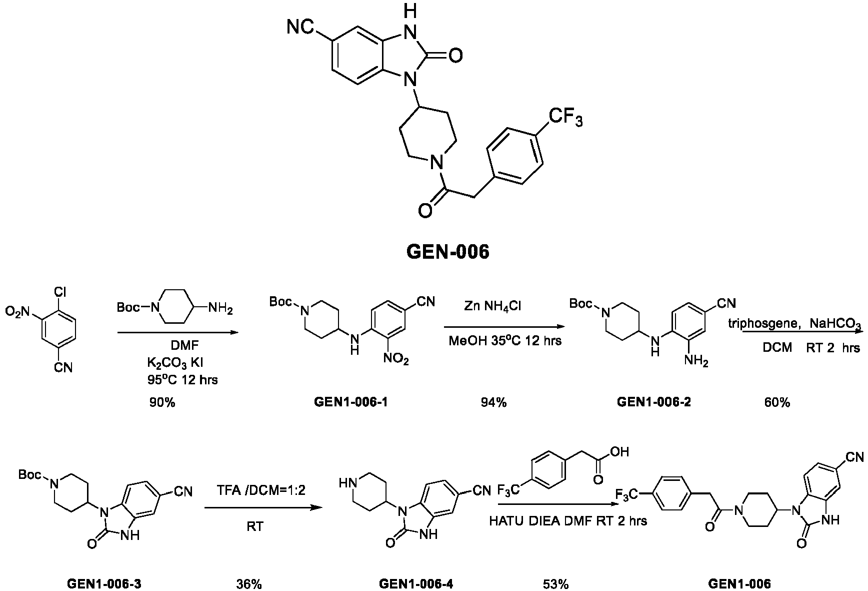

### Step 20.1. Synthesis of tert-butyl 4-((4-cyano-2-nitrophenyl)amino)piperidine-1-carboxylate (GEN1-006-1)

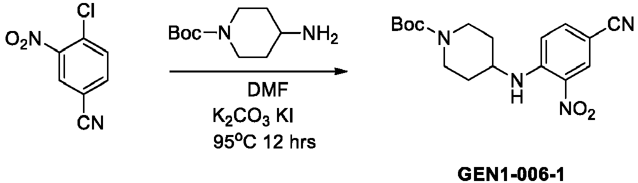

In a solution of 4-chloro-3-nitrobenzonitrile (1.00 g, 5.50 mmol), tert-butyl 4-aminopiperidine-1-carboxylate (1.1 g, 5.50 mmol) and K₂CO₃ (1.14 g, 8.25 mmol)) in DMF (20 mL) was added KI (40 mg, 0.275 mmol). After stirring for 12 hours at 95°C, the mixture was cooled to room temperature and poured into water (50 mL). EtOAc (30x2ml) was added to extract the desired compound. The combined organic layer was washed with brine, dried over Na₂SO₄, and concentrated to the desired compound (1.7 g, yield 90%) as a yellow solid. ¹H NMR (400 MHz, CDCl₃): δ 8.52 (d, *J* = 2.0 Hz, 1 H), 8.43 (d, *J* = 7.6 Hz, 1H), 7.60 (dd, *J* = 8.8, 1.6 Hz, 1H), 6.94 (d, *J* = 9.2 Hz, 1H), 4.05 (d, *J* = 12.0 Hz, 2H), 3.73-3.70 (m, 1H), 3.07-3.02 (m, 2H), 2.08-2.04 (m, 2H), 1.63-1.60 (m, 2H), 1.55 (s, 9H).

### Step 20.2. Synthesis of tert-butyl 4-((4-cyano-2-aminophenyl)amino)piperidine-1-carboxylate (GEN1-006-2)

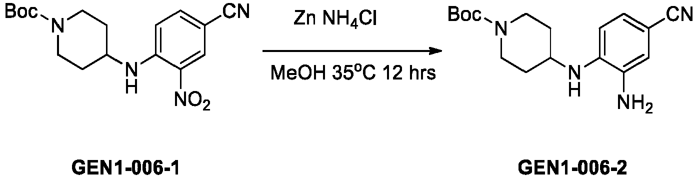

In a solution of tert-butyl 4-((4-cyano-2-nitrophenyl)amino)piperidine-1-carboxylate (560 mg, 1.60 mmol) and NH₄Cl (428 mg, 8 mmol) in MeOH (5 mL) was added zinc powder (416 mg, 6.40 mmol). The reaction mixture was stirred at 35°C for 12 hours. Then the reaction mixture was filtered, and the filtrate was concentrated to obtain the desired (482 mg, yield 94%) red oil. ¹H NMR (400 MHz, CDCl₃): δ 7.15 (dd, *J* = 8.4, 2.0 Hz, 2H), 6.95 (d, *J* = 1.6 Hz, 1H), 6.60 (d, *J* = 8.0 Hz, 1H), 4.11-4.06 (m, 3H), 3.48 (s, 1H), 3.28 (s, 2H), 2.96 (t, *J* = 11.2 Hz, 2H), 2.05-2.03 (m, 2H), 1.49 (s, 9H).

### Step 20.3. Synthesis of tert-butyl 4-(5-cyano-2-oxo-2,3-dihydro-1H-benzo[d]imidazol-1-yl)piperidine-1-carboxylate (GEN1-006-3)

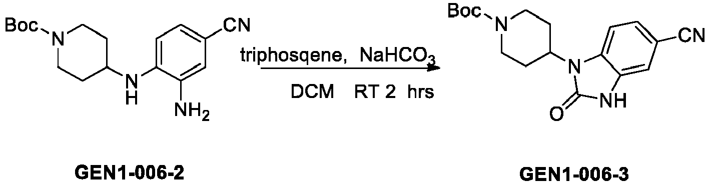

In DCM (6ml) were added tert-butyl 4-((4-cyano-2-aminophenyl)amino)piperidine-1-carboxylate (482 mg, 1.50 mmol) and NaHCO₃ (639 mg, 1.5 mmol), and triphosgene (452 mg, 1.50 mmol) was added to react under ice bath. The reaction mixture was stirred at room temperature for 2 hours. Then the mixture was poured into water (10ml) and extracted with DCM (10x2ml). The combined organic layer was washed with brine, dried over Na₂SO₄, and concentrated to the desired compound (314 mg, yield 60%) as a gray solid. MS (ESI): Rt = 0.32 min, *m*/*z* 243.0 [M+H-Boc]⁺. purity: 90% @ 254 nm, 85% @ 214 nm.

### Step 20.4. Synthesis of 2-oxo-1-(piperidin-4-yl)-2,3-dihydro-1H-benzo[d]imidazole-5-carbonitrile (GEN1-006-4)

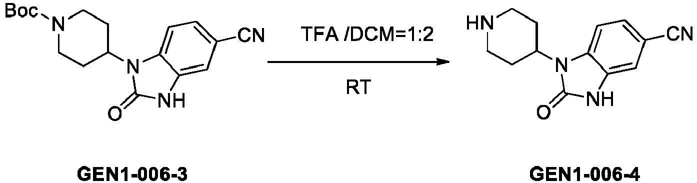

In DCM (2ml) were added tert-butyl 4-(5-cyano-2-oxo-2,3-dihydro-1H-benzo[d]imidazol-1-yl)piperidine-1-carboxylate (314 mg, 0.920 mmol) and TFA (1 mL). The reaction mixture was stirred at room temperature for 12 hours. The mixture was concentrated to obtain a crude product. Water (2ml) was added, and the pH was adjusted to 9 with saturated Na₂CO₃ aqueous solution (5ml). The mixture was extracted with DCM (10 x 4 mL). The combined organic layer was dried and concentrated to obtain the desired product (80 mg, yield 36%) as a white solid. MS (ESI) Rt = 0.87 min, *m*/*z* 243.0, [M+H]⁺. purity: 76% @ 254 nm, 81% @ 214 nm.

### Step 20.5. Synthesis of 2-oxo-1-(1-(2-(4-(trifluoromethyl)phenyl)acetyl)piperidin-4-yl)-2,3-dihydro-1H-benzo[d]imidazole-5-carbonitrile (GEN1-006)

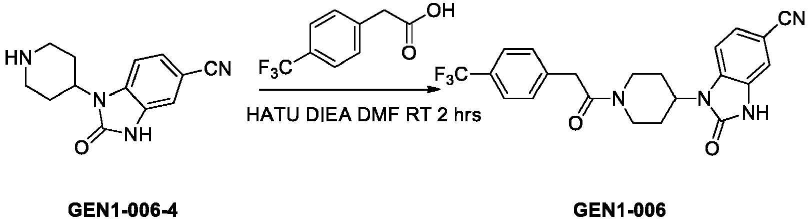

In a solution of 2-oxo-1-(piperidin-4-yl)-2,3-dihydro-1H-benzo[d]imidazole-5-carbonitrile (75 mg, 0.31 mmol), 2-(4-(trifluoromethyl)phenyl)acetic acid (63 mg, 0.31 mmol) and HATU (175 mg, 0.46 mmol) in DMF (3 mL) was added DIEA (120 mg, 0.93 mmol). The reaction mixture was stirred at room temperature for 12 hours. It was directly purified with a C18 column (CH₃CN: water = 5% ∼ 50%) to obtain the desired (70 mg, yield: 53%) yellow solid. ¹H NMR (400 MHz, CDCl₃): δ 8.96 (s, 1H), 7.63 (d, *J* = 8.0 Hz, 2H), 7.44 (d, *J* = 8.0 Hz, 2H), 7.36 (dd, *J* = 8.0, 1.2 Hz, 1H), 7.32 (d, *J* = 1.2 Hz, 1H), 6.93 (d, *J* = 8.0 Hz, 1H), 4.93-4.89 (m, 1H), 4.53-4.44 (m, 1H), 4.10-4.06 (m, 1H), 3.87 (s, 2H), 3.20 (t, *J* = 13.6 Hz, 1H), 2.72 (t, *J* = 13.6 Hz, 1H), 2.30-2.19 (m, 1H), 2.09-1.97 (m, 1H), 1.90-1.80 (m, 2H). MS (ESI), Rt = 3.748 min, *m*/*z* 429.1 [M+H]⁺, purity: 92.24% @ 254 nm, 95.70% @ 214 nm.

### Example 21. 5-methoxy-1-(1-(2-(4-(trifluoromethyl)phenyl)acetyl)piperidin-4-yl)-1H-benzo[d]imidazole-2(3H)-one (GEN1-007)

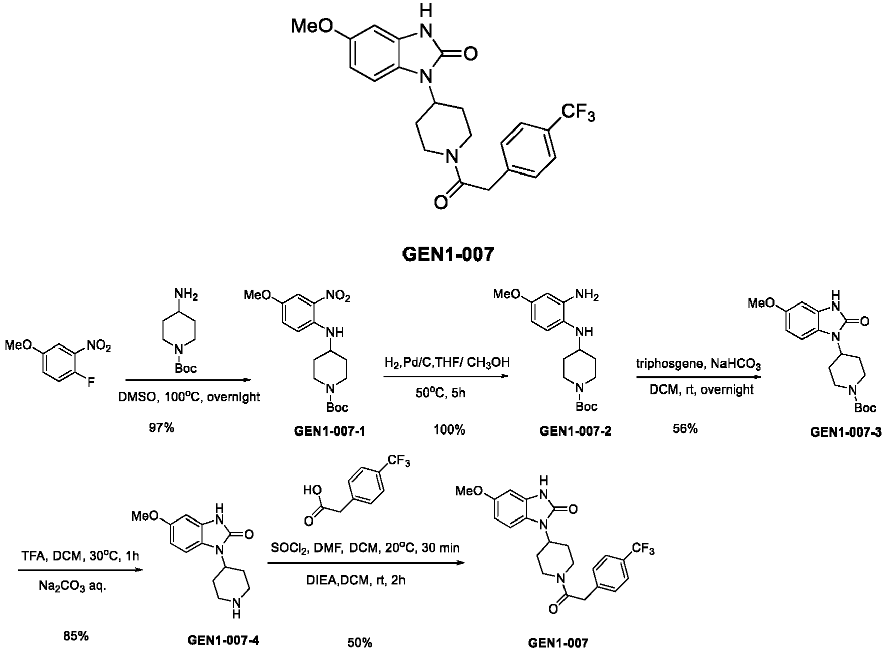

### Step 21.1. Synthesis of tert-butyl 4-((4-methoxy-2-nitrophenyl)amino)piperidine-1-carboxylate (GEN1-007-1)

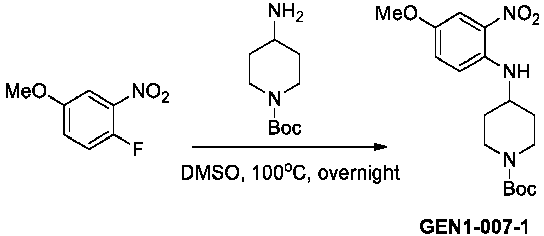

In a solution of 1-fluoro-4-methoxy-2-nitrobenzene (1.00 g, 5.85 mmol) and tert-butyl 4-aminopiperidine-1-carboxylate (1.29 g, 6.43 mmol) in DMSO (15ml) was added K₂CO₃ (1.61 g, 11.7 mmol). After stirring for 15 hours at 90°C, it was cooled to room temperature, poured into 100 mL of water, stirred for 10 min, and extracted with ethyl acetate (3×30 mL). The combined organic layer was washed with aqueous hydrochloric acid (1 M, 30 mL) and brine (30 mL), dried over Na₂SO₄, and concentrated to obtain the desired compound (1.98 g, yield 97%) as a red solid. MS (ESI) Rt = 1.746 min, *m*/*z* 296.1 [M-*t*-Bu+2H]⁺, purity: 78.6% @ 214 nm.

### Step 21.2. Synthesis of tert-butyl 4-((2-amino-4-methoxyphenyl)amino)piperidine-1-carboxylate (GEN1-007-2)

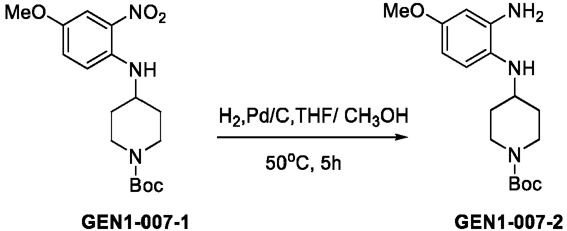

In a solution of tert-butyl 4-((4-methoxy-2-nitrophenyl)amino)piperidine-1-carboxylate (1.95 g, 5.56 mmol) in THF (15ml) and methanol (15ml) was added Pd/C (10%, 200 mg). The mixture was stirred under a hydrogen atmosphere at 50°C for 5 hours and filtered through celite. The filtrate was concentrated to obtain the desired compound (1.78 g, yield: 100%) as a brown oil. MS (ESI) Rt = 1.598 min, *m*/*z* 322.2 [M+H]⁺, purity: 71.4% @ 214 nm.

### Step 21.3. Synthesis of tert-butyl 4-(5-methoxy-2-oxo-2,3-dihydro-1H-benzo[d]imidazol-1-yl)piperidine-1-carboxylate (GEN1-007-3)

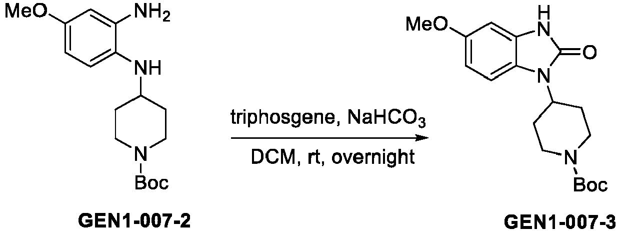

In a solution of tert-butyl (2-amino-4-methoxyphenyl)amino)piperidine-1-carboxylate (1.75 g, 5.45 mmol) in DCM (50ml) was added NaHCO₃ (4.58 g, 54.5 mmol). The mixture was cooled to 0°C. Triphosgene (810 mg, 2.73 mmol) was added step by step. The mixture was stirred at room temperature overnight. The mixture was poured into water (50 mL) and extracted with DCM (3×20 mL). The combined organic layer was washed with brine (20 mL), dried over Na₂SO₄ and concentrated. The residue was purified by silica gel column chromatography (PE: EA = 2:1 ∼ 1:1) to obtain the desired compound (1.05 g, yield 56%) as a yellow solid. ¹H NMR (400 MHz, CDCl₃): δ 10.21-9.96 (m, 1H), 7.01 (d, *J* = 8.8 Hz, 1H), 6.74 (d, *J* = 2.0 Hz, 1H), 6.62 (dd, *J* = 8.8, 2.4 Hz, 1H), 4.50-4.41 (m, 1H), 4.33-4.31 (m, 2H), 3.80 (s, 3H), 2.90-2.85 (m, 2H), 2.35-2.24 (m, 2H), 1.85-1.82 (m, 2H), 1.51 (s, 9H). MS (ESI) Rt = 1.508 min, *m*/*z* 370.2 [M+Na]⁺, purity: 54.9% @ 214 nm.

### Step 21.4. Synthesis of 5-methoxy-1-(piperidin-4-yl)-1H-benzo[d]imidazole-2(3H)-one (GEN1-007-4)

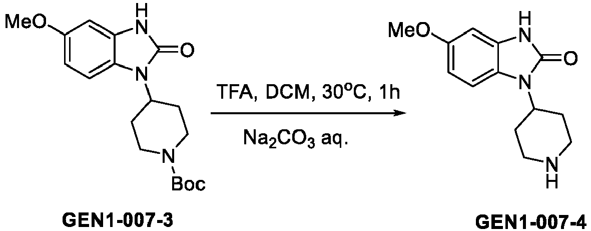

In DCM (2ml) were added tert-butyl 4-(5-methoxy-2-oxy-2,3-dihydro-1H-benzo[d]imidazol-1-yl)piperidine-1-carboxylate (200mg, 0.58 mmol) and TFA (1 mL). The mixture was stirred at 30°C for 1 hour. The mixture was concentrated. The residue was diluted with water (2 mL), and Na₂CO₃ aqueous solution (sat., 5 mL) was added. The mixture was extracted with DCM: MeOH = 10:1 (4×20 mL). The combined organic layer was washed with brine (20 mL), dried over Na₂SO₄, and concentrated to the desired yellow solid (120 mg, 85% yield). MS (ESI) Rt = 0.767 min, *m*/*z* 248.1 [M+H]⁺, purity: 95.7% @ 214 nm.

### Step 21.5. Synthesis of 5-methoxy-1-(1-(2-(4-(trifluoromethyl)phenyl)acetyl)piperidin-4-yl)-1H-benzo[d]imidazole-2(3H)-one (GEN1-007)

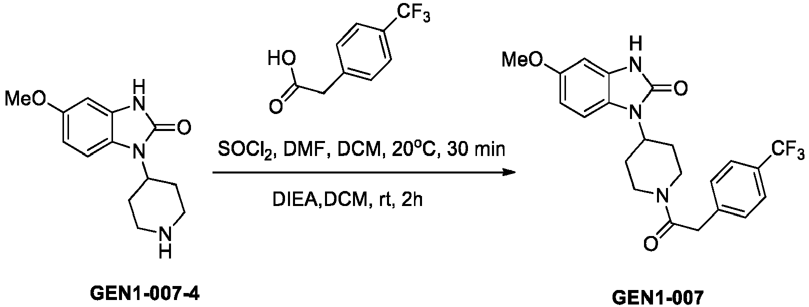

In a solution of 2-(4-(trifluoromethyl)phenyl)acetic acid (109 mg, 0.53 mmol) in DCM (2 mL) was added DMF (1 drop). SOCl₂ (116 mg, 0.97 mmol) was added. After stirring for 30 minutes at 20°C, the mixture was concentrated. The residue was dissolved in DCM (1 mL) and used in the next step. 5-methoxy-1-(piperidin-4-yl)-1H-benzo[d]imidazole-2(3H)-one (120mg, 0.490 mmol) and DIEA (313mg, 2.43 mmol) in DCM (2ml) was stirred at room temperature for 5 min, and the previous solution was added dropwise. The added mixture was stirred at room temperature for 2 hours. The mixture was poured into water (20 mL) and extracted with DCM (3×10 mL). The combined organic layer was washed with brine (10ml), dried over Na₂SO₄ and concentrated. The residue was purified using high performance liquid chromatography (Xbridge C18, 5 um, 19 x 150 mm, 20-70% B, A: H₂O (0.1 % NH₄HCO₃), B: CH₃CN, uv = 214 nm, F = 15 mL/min) to obtain the desired compound (105 mg, yield 50%) as a white solid. ¹H NMR (400 MHz, CDCl₃): δ 9.53 (s, 1H), 7.63 (d, *J* = 8.0 Hz, 2H), 7.46 (d, *J* = 8.4 Hz, 2H), 6.73-6.69 (m, 2H), 6.59-6.56 (m, 1H), 4.88 (d, *J* = 13.6 Hz, 1H), 4.52-4.46 (m, 1H), 4.05 (d, *J* = 14.4 Hz, 1H), 3.87 (s, 2H), 3.79 (s, 3H), 3.23-3.17 (m, 1H), 2.75-2.69 (m, 1H), 2.26-2.17 (m, 1H), 2.04-1.95 (m, 1H), 1.90-1.87 (m, 1H), 1.82-1.78 (m, 1H). MS (ESI) *m*/*z* 434.2 [M+H]⁺, Rt = 3.553 min, purity: 98.6% @ 254 nm, 99.6% @ 214 nm.

### Example 22. 2-oxo-3-(1-(2-(4-(trifluoromethyl)phenyl)acetyl)piperidin-4-yl)-2,3-dihydro-1H-benzo[d]imidazole-5-carbonitrile (GEN1-010)

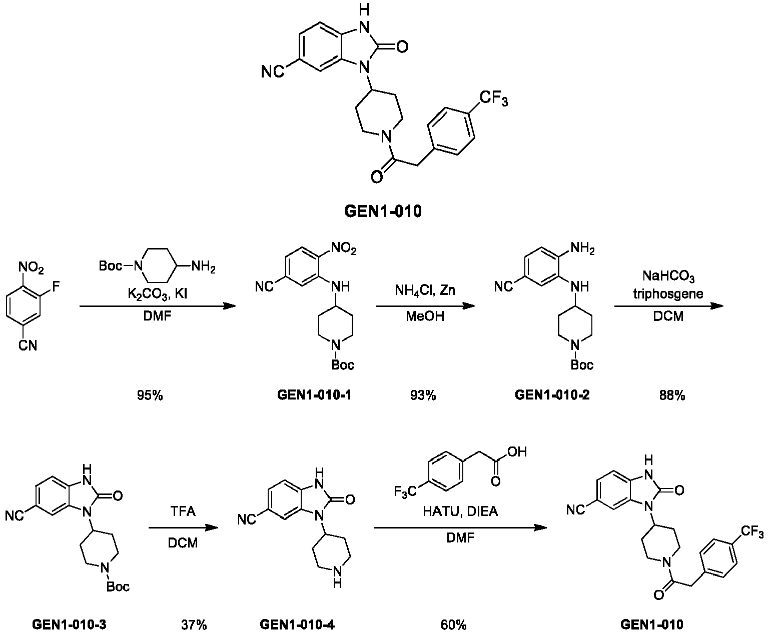

### Step 22.1. Synthesis of tert-butyl 4-((5-cyano-2-nitrophenyl)amino)piperidine-1-carboxylate (GEN1-010-1)

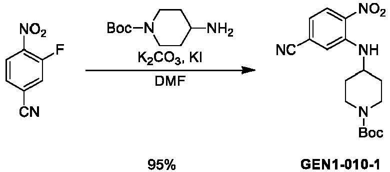

In a mixture of 3-fluoro-4-nitrobenzonitrile (1.0 g, 6.0 mmol), tert-butyl 4-aminopiperidine-1-carboxylate (1.1 g, 6.0 mmol) and K₂CO₃ (2.4 g, 18 mmol) in DMF (15 mL) was added KI (50 mg, 0.3 mmol). The resulting mixture was stirred at 95°C for 12 hours. The mixture was cooled to room temperature, poured into water (50 mL), and extracted with EtOAc (30×2 mL). The combined organic layer was washed with brine, dried over Na₂SO₄, and concentrated to the desired yellow solid (2.0 g, yield 95%). ¹H NMR (400 MHz, CDCl₃): δ 8.27 (d, *J* = 8.8 Hz, 1H), 8.06 (d, *J* = 7.6 Hz, 1H), 7.17 (d, *J* = 1.2 Hz, 1H), 6.87 (dd, *J* = 8.8, 1.6 Hz, 1H), 4.11-4.05 (m, 2H), 3.69-3.60 (m, 1H), 3.06 (t, *J* = 11.2 Hz, 2H), 2.08-2.04 (m, 2H), 1.58-1.52 (m, 2H), 1.48 (s, 9H).

### Step 22.2. Synthesis of tert-butyl 4-((2-amino-5-cyanophenyl)amino)piperidine-1-carboxylate (GEN1-010-2)

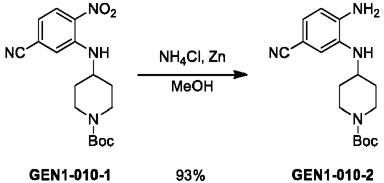

In a solution of tert-butyl 4-((5-cyano-2-nitrophenyl)amino)piperidine-1-carboxylate (1.1 g, 3.5 mmol), NH₄Cl (910 mg, 17.4 mmol) in MeOH (15 mL) was added zinc powder (905 mg, 13.9 mmol). The reaction mixture was stirred at 35°C for 12 hours. The reaction mixture was filtered and the filtrate was concentrated to the desired (930 mg, yield 93%) red solid. MS (ESI): Rt = 1.58 min, *m*/*z* 217.1, [M + H-Boc]⁺. purity: 91% @ 254 nm, 86% @ 214 nm.

### Step 22.3. Synthesis of tert-butyl 4-(6-cyano-2-oxo-2,3-dihydro-1H-benzo[d]imidazol-1-yl)piperidine-1-carboxylate (GEN1-010-3)

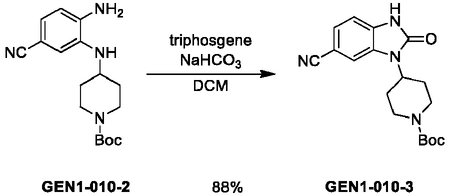

In tert-butyl 4-((2-amino-5-cyano)amino)piperidine-1-carboxylate (500 mg, 1.58 mmol) and NaHCO₃ (1.32 g, 15.8 mmol) in DCM (10ml) was added triphosgene (469 mg, 1.58 mmol) in batches under ice bath conditions. The reaction mixture was stirred at room temperature for 2 hours. The mixture was poured into 10 mL water and extracted with DCM (10×2 mL). The combined organic layer was washed with brine, dried over Na₂SO₄, and concentrated to the desired (480 mg, yield 88%) white solid. MS (ESI): Rt = 1.48 min, *m*/*z* 243.0, [M + H-Boc]⁺. purity: 100% @ 254 nm, 100% @ 214 nm.

### Step 22.4. 2-oxo-3-(piperidin-4-yl)-2,3-dihydro-1H-benzo[d]imidazole-5-carbonitrile (GEN1-010-4)

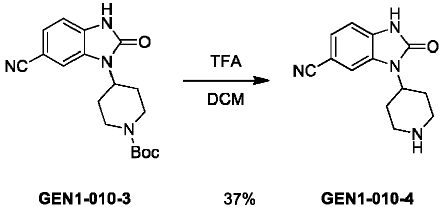

In DCM (4ml) were added tert-butyl 4-(6-cyano-2-oxy-2,3-dihydro-1H-benzo[d]imidazol-1-yl)piperidine-1-carboxylate (480 mg, 1.40 mmol) and TFA (1 mL). The reaction mixture was stirred at room temperature for 12 hours. The mixture was concentrated, and the residue was dissolved in water (2 mL). The aqueous solution was basified with aqueous Na₂CO₃ (sat., 5ml) to pH = 9, and extracted with DCM (10×4ml). The combined organic layer was dried over Na₂SO₄ and concentrated to the desired white solid (125 mg, 37% yield). MS (ESI): Rt = 0.73 min *m*/*z* 243.0, [M + H]⁺. purity: 100% @ 254 nm, 100% @ 214 nm.

### Step 22.5. Synthesis of 2-oxo-3-(1-(2-(4-(trifluoromethyl)phenyl)acetyl)piperidin-4-yl)-2,3-dihydro-1H-benzo[d]imidazole-5-carbonitrile (GEN1-010)

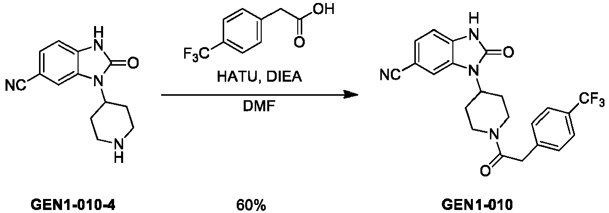

In a solution of 2-oxo-3-(piperidin-4-yl)-2,3-dihydro-1H-benzo[d]imidazole-5-carbonitrile (75 mg, 0.31 mmol), 2-(4-(trifluoromethyl)phenyl)acetic acid (63 mg, 0.31 mmol) and HATU (175 mg, 0.460 mmol) in DMF (3 mL) was added DIEA (120 mg, 0.929 mmol). The reaction mixture was stirred at room temperature for 12 hours. The mixture was purified with a C18 column (CH₃CN: water = 5% ∼ 50%) to obtain the desired (80 mg, yield 60%) white solid. ¹H NMR (400 MHz, DMSO-*d*₆): δ 11.44 (s, 1H), 7.73 - 7.68 (m, 3H), 7.51 (d, *J* = 8.4 Hz, 2H), 7.44 (dd, *J* = 8.0, 1.2 Hz, 1H), 7.11 (d, *J* = 8.0 Hz, 1H), 4.58 (d, *J* = 13.6 Hz, 1H), 4.48-4.42 (m, 1H), 4.11 (d, *J* = 13.2 Hz, 1H), 3.92-3.91 (m, 2H), 3.16 (t, *J* = 12.8 Hz, 1H), 2.72-2.67 (m, 1H), 2.21-2.12 (m, 2H), 1.72 (br s, 2H). MS (ESI): Rt = 3.55 min, *m*/*z* 429.2 [M+H]⁺, purity: 97.98% @ 254 nm, 99.10% @ 214 nm.

### Example 23. 6-methoxy-1-(1-(2-(4-(trifluoromethyl)phenyl)acetyl)piperidin-4-yl)-1H-benzo[d]imidazole-2 (3H)-one (GEN1-011)

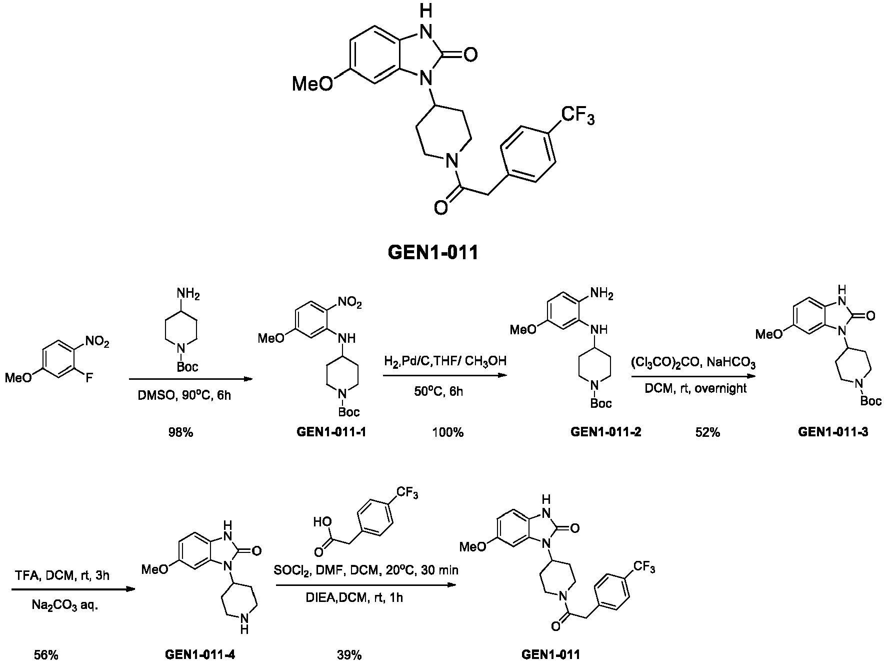

### Step 23.1. Synthesis of tert-butyl 4-((5-methoxy-2-nitrophenyl)amino)piperidine-1-carboxylate (GEN1-011-1)

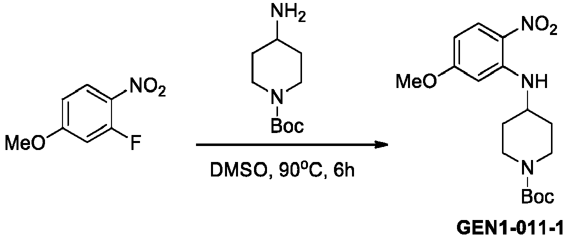

In DMSO (15ml) was added 2-fluoro-4-methoxy-1-nitrobenzene (500 mg, 2.92 mmol) and tert-butyl 4-aminopiperidine-1-carboxylate (702 mg, 3.51 mmol) solution, and K₂CO₃ (807 mg, 5.85 mmol) was added. After stirring at 90°C for 6 hours, the mixture was cooled and poured into water (100 mL). After stirring for 10 minutes, the filter cake was washed with water (2×20 mL) and dissolved in ethyl acetate (50 mL). The organic solution was washed with aqueous hydrochloric acid (1 M, 20 mL) and brine (20 mL), dried over Na₂SO₄, and concentrated to obtain the desired compound (1.01 g, yield 98%) as a yellow solid. ¹H NMR (300 MHz, CDCl₃): δ 8.34 (d, *J* = 7.2 Hz, 1H), 8.17 (d, *J* = 9.6 Hz, 1H), 6.25 (dd, *J* = 9.6, 1.5 Hz, 1H), 6.18 (s, 1H), 4.02-3.98 (m, 2H), 3.87 (s, 3H), 3.66-3.58 (m, 1H), 3.08 (t, *J* = 11.7 Hz, 2H), 2.08-2.04 (m, 2H), 1.55-1.51 (m, 2H), 1.48 (s, 9H).

### Step 23.2. Synthesis of tert-butyl 4-((2-amino-5-methoxyphenyl)amino)piperidine-1-carboxylate (GEN1-011-2)

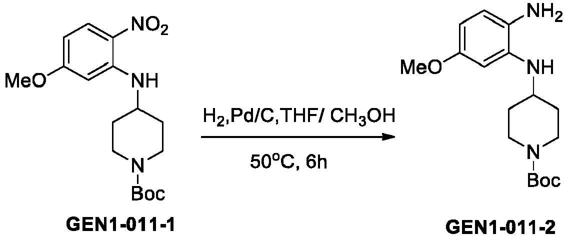

Pd/C (10%, 200 mg) was added to a solution of tert-butyl 4-((5-methoxy-2-nitrophenyl)amino)piperidine-1-carboxylate (1.00 g, 2.85 mmol ) in THF (5 mL) and methanol (15 mL). The mixture was stirred under a hydrogen atmosphere at 50°C for 6 hours. The mixture was filtered through diatomaceous earth. The filtrate was concentrated to obtain the desired compound (910 mg, yield 100%) as a brown solid. ¹H NMR (400 MHz, CDCl₃): δ 6.67 (d, *J* = 8.4 Hz, 1H), 6.25 (d, *J* = 2.4 Hz, 1H), 6.18 (dd, *J* = 8.0, 2.4 Hz, 1H), 4.02-3.99 (m, 2H), 3.75 (s, 3H), 3.40-3.35 (m, 1H), 2.95-2.92 (m, 2H), 2.05-2.02 (m, 2H), 1.47 (s, 9H), 1.43-1.26 (m, 4H).

### Step 23.3. Synthesis of tert-butyl 4-(6-methoxy-2-oxo-2,3-dihydro-1H-benzo[d]imidazol-1-yl)piperidine-1-carboxylate (GEN1-011-3)

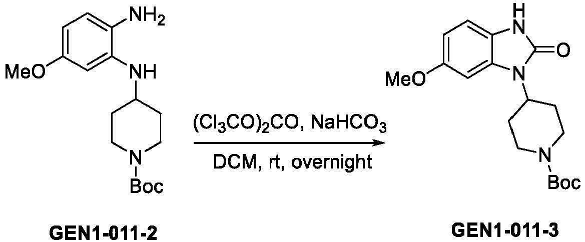

In a solution of tert-butyl 4-((2-amino-5-methoxyphenyl)amino)piperidine-1-carboxylate (900 mg, 2.80 mmol) in DCM (50ml) was added NaHCO₃ (2.36 g, 28.0 mmol). The mixture was cooled to 0°C. Then 500 mg, 1.68 mmol triphosgene was added. The mixture was stirred at room temperature overnight. The mixture was poured into water (50 mL) and extracted with DCM (2 x 30 mL). The combined organic layer was washed with brine (20 mL), dried over Na₂SO₄ and concentrated. The residue was purified by silica gel column chromatography (PE: EA = 1:1) to obtain 820 mg of crude product. It was further purified by C18 column chromatography (30∼60% CH₃CN aqueous solution) to obtain the desired yellow solid (510 mg, yield 52%). MS (ESI) Rt = 1.532 min, *m*/*z* 370.2 [M+H]⁺, purity: 82.8% @ 214 nm.

### Step 23.4. Synthesis of 6-methoxy-1-(piperidin-4-yl)-1H-benzo[d]imidazole-2(3H)-one (GEN1-011-4)

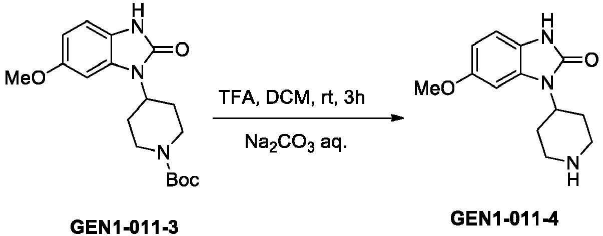

In DCM (2ml) were add tert-butyl 4-(6-methoxy-2-oxy-2,3-dihydro-1H-benzo[d]imidazol-1-yl)piperidine-1-carboxylate (500 mg, 1.44 mmol) and TFA (1 mL). The mixture was stirred at room temperature for 3 hours. Then the mixture was concentrated. The residue was diluted with 5 ml water and 10 ml Na₂CO₃ aqueous solution. The mixture was extracted with DCM: MeOH = 10:1 (4×20 mL). The combined organic layer was washed with brine (30 mL), dried over Na₂SO₄, and concentrated to the desired (200 mg, yield 56%) brown solid. MS (ESI) Rt = 1.042 min, *m*/*z* 248.1 [M+H]⁺, purity: 96.1% @ 214 nm.

### Step 23.5. Synthesis of 6-methoxy-1-(1-(2-(4-(trifluoromethyl)phenyl)acetyl)piperidin-4-yl)-1H-benzo[d]imidazole-2(3H)-one (GEN1-011)

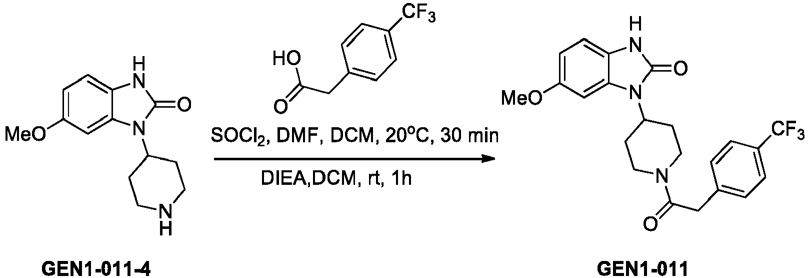

DMF (2 drops) was added to a solution of 2-(4-(trifluoromethyl)phenyl)acetic acid in DCM (3 mL), and then SOCl₂ (193 mg, 1.62 mmol) was added. After stirring for 30 minutes at 20°C, the mixture was concentrated. The concentrate was dissolved in DCM (1 mL) and directly put into the next step. 6-methoxy-1-(piperidin-4-yl)-1H-benzo[d]imidazole-2(3H)-one (200 mg, 0.81 mmol) and DIEA (522 mg, 4.05 mmol) in DCM (2 mL) was stirred at room temperature for 5 min, and the previous solution was added dropwise. The mixture was stirred at room temperature for 1 hour. The mixture was poured into water (20 mL) and extracted with DCM (2 x 20 mL). The combined organic layer was washed with brine (20 mL), dried over Na₂SO₄ and concentrated. The crude product was purified using high performance liquid chromatography (Xbridge C18, 5 um, 19 x 150 mm, 20-70% B, A: H₂O (0.1% NH₄HCO₃), B: CH₃CN, uv = 214 nm, F = 15 mL/min) to obtain the desired white solid (135 mg, yield 39%). ¹H NMR (400 MHz, CDCl₃): δ 9.46 (s, 1H), 7.65 (d, *J* = 8.0 Hz, 2H), 7.45 (d, *J* = 8.0 Hz, 2H), 6.97 (d, *J* = 8.8 Hz, 1H), 6.61 (dd, *J* = 8.8, 2.4 Hz, 1H), 6.52 (d, *J* = 2.4 Hz, 1H), 4.90 (d, *J* = 13.6 Hz, 1H), 4.51-4.45 (m, 1H), 4.03 (d, *J* = 14.0 Hz, 1H), 3.87 (s, 2H), 3.81 (s, 3H), 3.19 (t, *J* = 12.0 Hz, 1H), 2.71 (t, *J=* 12.0 Hz, 1H), 2.31-2.26 (m 1H), 2.04-2.00 (m, 1H), 1.90-1.87 (m, 1H), 1.79-1.72 (m, 1H). MS (ESI) Rt = 2.995 min, *m*/*z* 434.2 [M+H]⁺, purity: 99.4% @ 254 nm, 99.8% @ 214 nm.

### Example 24. 7-(trifluoromethyl)-1-(1-(2-(4-(trifluoromethyl)phenyl)acetyl)piperidin-4-yl)-1H-benzo[d]imidazole-2(3H)-one (GEN1-013)

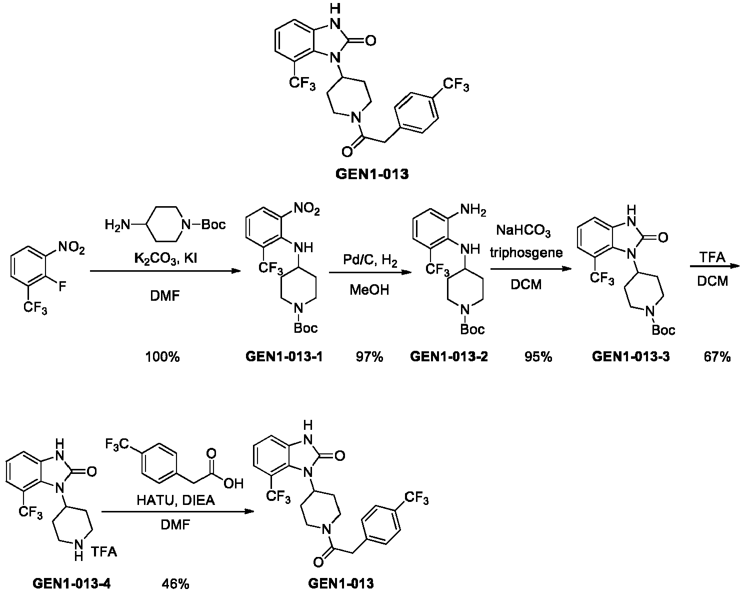

### Step 24.1. Synthesis of tert-butyl 4-((2-nitro-6-(trifluoromethyl)phenyl)amino)piperidine-1-carboxylate (GEN1-013-1)

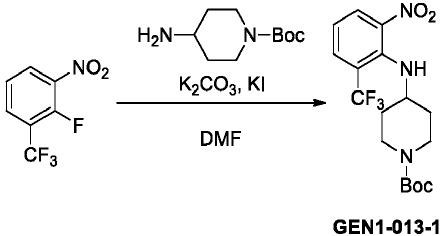

2-fluoro-1-nitro-3-(trifluoromethyl)benzene (1.00 g, 4.80 mmol), tert-butyl 4-aminopiperidine-1-carboxylate (961 mg, 4.80 mmol) and K₂CO₃ (995 mg, 7.20 mmol) were suspended in DMF (10 mL) and the mixture was stirred at 85°C for 2 hours. After cooling to room temperature, the reaction mixture was poured into water (50 mL) and extracted with EA (25 mL×3). The combined organic layer was washed with water (25 mL), brine (25 mL), dried over MgSO₄ and concentrated to obtain the desired compound (1.86 g, yield 100%) as a yellow solid. ¹HNMR (400 MHz, CDCl₃): δ 8.08 (dd, *J* = 8.0, 1.2 Hz, 1H), 7.78 (dd, *J* = 7.6, 1.2 Hz, 1H), 6.96 (t, *J* = 8.0 Hz, 1H), 5.93 (d, *J* = 9.6 Hz, 1H), 4.10-3.93 (m, 2H), 3.46-3.36 (m, 1H), 2.84-2.78 (m, 2H), 1.94-1.91 (m, 2H), 1.45 (s, 9H), 1.39-1.29 (m, 2H).

### Step 24.2. Synthesis of tert-butyl 4-((2-amino-6-(trifluoromethyl)phenyl)amino)piperidine-1-carboxylate (GEN1-013-2)

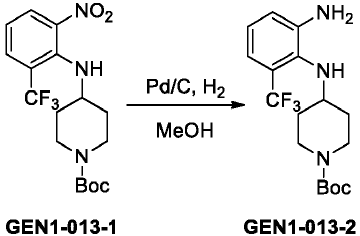

Pd/C (10%, 186 mg) was added to a solution of tert-butyl 4-((2-nitro-6-(trifluoromethyl)phenyl)amino)piperidine-1-carboxylate (1.86 g, 4.78 mmol) in methanol (20 mL) under N₂ atmosphere. The resulting mixture was degassed with H₂ and stirred overnight under a hydrogen atmosphere at 35°C. The reaction mixture was filtered, and the filtrate was concentrated to obtain the desired compound (1.66 g, yield 97%) as a brown oil. ¹HNMR (400 MHz, CDCl₃): δ 6.97 (dd, *J* = 7.6, 1.6 Hz, 1H), 6.92 (t, *J* = 8.0 Hz, 1H), 6.86 (dd, *J* = 8.0, 1.6 Hz, 1H), 4.11 (br s, 2H), 3.88 (s, 2H), 3.38-3.24 (m, 2H), 2.73-2.66 (m, 2H), 1.84-1.81 (m, 2H), 1.46 (s, 9H), 1.40-1.31 (m, 2H).

### Step 24.3. Synthesis of tert-butyl 4-(2-oxo-7-(trifluoromethyl)-2,3-dihydro-1H-benzo[d]imidazol-1-yl)piperidine-1-carboxylate (GEN1-013-3)

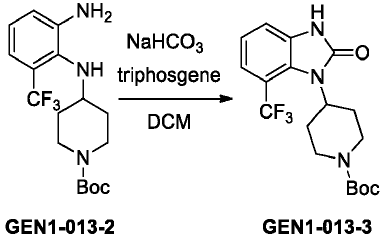

In a suspension of tert-butyl 4-((2-amino-6-(trifluoromethyl)phenyl)amino)piperidine-1-carboxylate (500 mg, 1.39 mmol) and NaHCO₃ (584 mg, 6.95 mmol) in DCM (25ml) was added triphosgene (206 mg, 0.694 mmol) at 7°C. The mixture was stirred for 2 hours. The reaction mixture was poured into water (50 mL), extracted with DCM (25 mL×3), and the combined organic layer was washed with brine (25 mL), dried over MgSO₄, and concentrated to give the desired compound (510 mg, yield 95%) as a white solid. ¹HNMR (400 MHz, CDCl₃): δ 10.16 (s, 1H), 7.40 (d, *J* = 8.0 Hz, 1H), 7.30 (d, *J* = 7.6 Hz, 1H), 7.14 (t, *J* = 8.0 Hz, 1H), 4.42-4.25 (m, 3H), 2.82-2.70 (m, 4H), 1.76-1.73 (m, 2H), 1.50 (s, 9H).

### Step 24.4. Synthesis of 1-(piperidin-4-yl)-7-(trifluoromethyl)-1H-benzo[d]imidazole-2(3H)-one (GEN1-013-4)

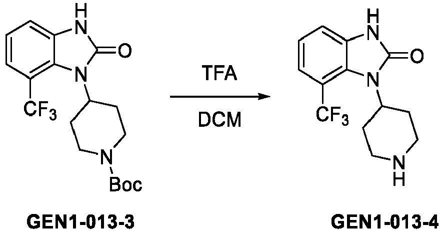

In DCM (6ml) was added tert-butyl 4-(2-oxo-7-(trifluoromethyl)-2,3-dihydro-1H-benzo[d]imidazol-1-yl)piperidine-1-carboxylate (510 mg, 1.32 mmol), and TFA (2ml) was added at room temperature. The mixture was stirred at room temperature overnight. The reaction mixture was concentrated and the residue was dissolved in DCM (10ml). Na₂CO₃ (sat., 10 mL) was added to the solution and the mixture was stirred for 20 minutes. The mixture was filtered and the filter cake was dried to obtain the desired compound (340 mg, yield 67%) as a white solid. ¹HNMR (400 MHz, DMSO-*d*₆): δ 8.41 (br s, 1H), 7.37 (d, *J* = 8.0 Hz, 1H), 7.31 (d, *J* = 7.2 Hz, 1H), 7.18 (t, *J* = 8.0 Hz, 1H), 4.29-4.18 (m, 1H), 3.43-3.40 (m, 2H), 2.97-2.80 (m, 4H), 1.88-1.85 (m, 2H). MS (ESI) Rt = 1.50 min, *m*/*z* 286.3 [M+H]⁺, purity: 98% @ 254 nm, 100% @ 214 nm.

### Step 24.5. Synthesis of 7-(trifluoromethyl)-1-(1-(2-(4-(trifluoromethyl)phenyl)acetyl)piperidin-4-yl)-1H-benzo[d]imidazole-2(3H)-one (GEN1-013)

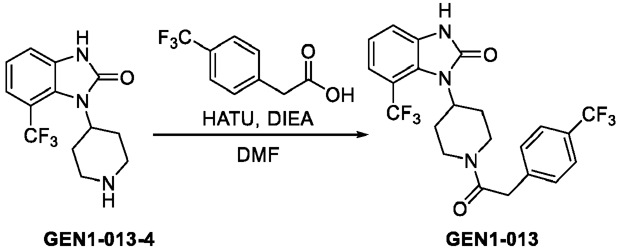

In DMF (2 mL) were added 1-(piperidin-4-yl)-7-(trifluoromethyl)-1H-benzo[d]imidazole-2(3H)-one (70 mg, 0.25 mmol), 2-[4-(trifluoromethyl)phenyl]acetic acid (60 mg, 0.29 mmol) and DIEA (95 mg, 0.74 mmol), and HATU (140 mg, 0.368 mmol) was gradually added at room temperature. The resulting mixture was stirred at room temperature for 1.5 hours. The reaction mixture was purified by C18 (CH₃CN: water = 5% ∼ 50%) to obtain the desired compound (53.1 mg, yield 46%) as a white solid. ¹H NMR (400 MHz, CDCl₃) δ 9.66 (s, 1H), 7.60 (d, *J* = 8.0 Hz, 2H), 7.42-7.40 (m, 3H), 7.28-7.26 (m, 1H), 7.15 (t, *J* = 8.0 Hz, 1H), 4.91-4.88 (m, 1H), 4.47-4.40 (m, 1H), 4.05-4.02 (m, 1H), 3.85 (s, 2H), 3.13-3.06 (m, 1H), 2.82-2.60 (m, 3H), 1.85-1.80 (m, 2H). MS (ESI) Rt = 3.437 min, *m*/*z* 472.2 [M+H]⁺, purity: 94.12% @ 254 nm, 99.57% @ 214 nm.

### Example 25. 2-oxo-3-(1-(2-(4-(trifluoromethyl)phenyl)acetyl)piperidin-4-yl)-2,3-dihydro- 1H-benzo[d]imidazole-4-carbonitrile (GEN1-014)

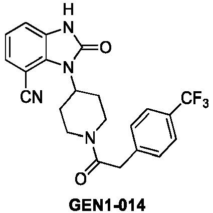

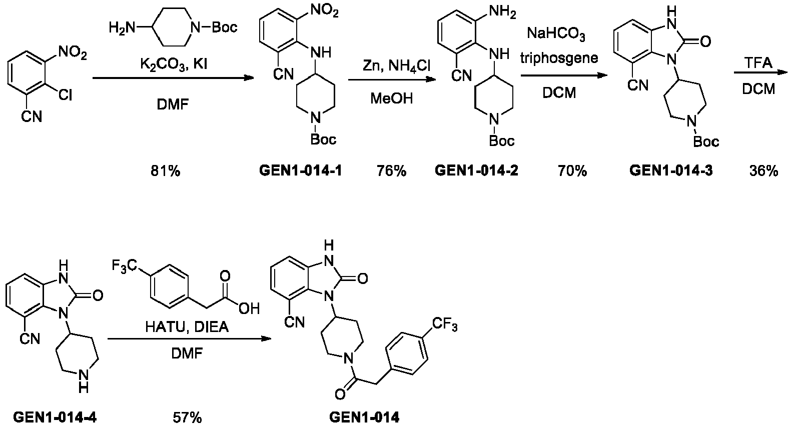

### Step 25.1. Synthesis of tert-butyl 4-((2-cyano-6-nitrophenyl)amino)piperidine-1-carboxylate (GEN1-014-1)

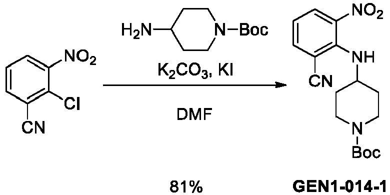

2-chloro-3-nitrobenzonitrile (875 mg, 4.80 mmol), tert-butyl 4-aminopiperidine-1-carboxylate (958 mg, 4.80 mmol) and K₂CO₃ (993 mg, 7.18 mmol), KI (50 mg, 0.30 mmol) were added to DMF (15 mL). The resulting mixture was stirred at 95°C for 12 hours. The mixture was cooled to room temperature, poured into water (50 mL), and extracted with EtOAc (30×2 mL). The organic layer was washed with brine, dried over Na₂SO₄, and concentrated to the desired (1.3 g, yield 81%) yellow solid. ¹H NMR (400 MHz, CDCl₃): δ 8.46 (d, *J* = 8.4 Hz, 1 H), 8.41 (dd, *J* = 8.4, 1.6 Hz, 1H), 7.75 (dd, *J* = 7.6, 2.0 Hz, 1 H), 6.78-6.74 (m, 1H), 4.56-4.51 (m, 1H), 4.08-4.05 (m, 2H), 3.04 (t, *J* = 11.6 Hz, 2H), 2.17-2.13 (m, 2 H), 1.55-1.51 (m, 2H), 1.47 (s, 9H).

### Step 25.2. Synthesis of tert-butyl 4-((2-amino-6-cyanophenyl)amino)piperidine-1-carboxylate (GEN1-014-2)

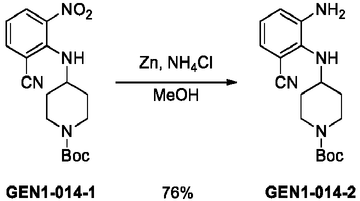

In a solution of tert-butyl 4-((2-cyano-6-nitrophenyl)amino)piperidine-1-carboxylate (1.3 g, 3.8 mmol) and NH₄Cl (0.81 g, 15 mmol) in MeOH (15 mL) was added Zn powder (0.73 g, 11 mmol). The reaction mixture was stirred at 35°C for 12 hours. The reaction mixture was filtered, and the filtrate was concentrated to obtain the desired (900 mg, yield 76%) red oil. ¹H NMR (400 MHz, CDCl₃): δ 6.98-6.95 (m, 1H), 6.90-6.88 (m, 2H), 4.23-4.00 (m, 2H), 3.84 (s, 2H), 3.43-3.31 (m, 2H), 2.77 (t, *J* = 12.4 Hz, 2H), 1.93-1.90 (m, 2H), 1.46 (s, 9H), 1.43-1.36 (m, 2H).

### Step 25.3. Synthesis of tert-butyl 4-(7-cyano-2-oxo-2,3-dihydro-1H-benzo[d]imidazol-1-yl)piperidine-1-carboxylate (GEN1-014-3)

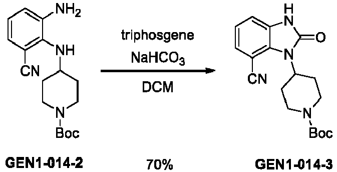

In DCM (12ml) were added tert-butyl 4-((2-amino-6-cyano)amino)piperidine-1-carboxylate (900 mg, 2.85 mmol) and NaHCO₃ (2.40 g, 28.5 mmol), and then triphosgene (592 mg, 2.28 mmol) was added under ice bath conditions. The reaction mixture was stirred at room temperature for 2 hours. The mixture was poured into 10 mL water and extracted with DCM (10×2 mL). The organic layer was washed with brine, dried over Na₂SO₄, and concentrated to the desired yellow solid (680 mg, yield 70%). MS (ESI): Rt = 1.69 min, *m*/*z* 243.2 [M + H-Boc]⁺. purity: 100% @ 254 nm, 94% @ 214 nm.

### Step 25.4. Synthesis of 2-oxo-3-(piperidin-4-yl)-2,3-dihydro-1H-benzo[d]imidazole-4-carbonitrile (GEN1-014-4)

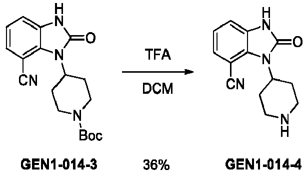

In DCM (10ml) were added tert-butyl 4-(7-cyano-2-oxy-2,3-dihydro-1H-benzo[d]imidazol-1-yl)piperidine-1-carboxylate (680 mg, 2.09 mmol) and TFA (5 mL). The reaction mixture was stirred at room temperature for 12 hours. The mixture was concentrated to obtain a crude product. The crude product was dissolved in water (2ml) and basified to pH = 9 with aqueous Na₂CO₃ (sat. 5 mL). It was then extracted with DCM (10×4 mL). The combined organic layer was dried and concentrated to obtain the desired (170 mg, yield 36%) gray solid. MS (ESI): Rt = 1.10 min, *m*/*z* 243.1, [M + H]⁺. purity: 100% @ 254 nm, 98% @ 214 nm.

### Step 25.5. Synthesis of 2-oxo-3-(1-(2-(4-(trifluoromethyl)phenyl)acetyl)piperidin-4-yl)-2,3-dihydro-1H-benzo[d]imidazole-4-carbonitrile (GEN1-014)

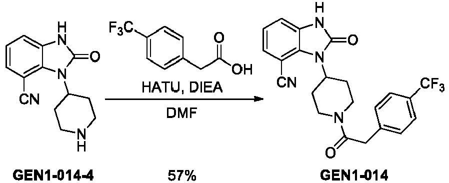

In a soluion of 2-oxo-3-(piperidin-4-yl)-2,3-dihydro-1H-benzo[d]imidazole-4-carbonitrile (80 mg, 0.33 mmol), 2-(4-(trifluoromethyl)phenyl)acetic acid (67 mg, 0.33 mmol) and HATU (190 mg, 0.50 mmol) in DMF (3 mL) was added DIEA (129 mg, 0.998 mmol). The reaction mixture was stirred at room temperature for 12 hours. The mixture was purified with a C18 column (CH3CN: water = 5% ∼ 50%) to obtain the desired compound (80 mg, yield: 57%) as a white solid. ¹H NMR (400 MHz, CDCl₃): δ 9.79 (s, 1H), 7.59 (d, *J* = 8.0 Hz, 2H), 7.40 (d, *J* = 7.6 Hz, 2H), 7.35 (d, *J* = 7.6 Hz, 1H), 7.29-7.26 (m, 1H), 7.15 (t, *J* = 8.0 Hz, 1H), 4.93 (t, *J* = 14.4 Hz, 2H), 4.05 (d, *J* = 13.6 Hz, 1H), 3.86 (s, 2H), 3.23 (t, *J* = 12.4 Hz, 1H), 2.79-2.69 (m, 2H), 2.69-2.58 (m, 1H), 1.97-1.94 (m, 2H). MS (ESI): Rt = 3.71 min, *m*/*z* 403.2, [M + H]⁺, purity: 96.17% @ 254 nm, 93.93% @ 214 nm.

### Example 26. 7-methoxy-1-(1-(2-(4-(trifluoromethyl)phenyl)acetyl)piperidin-4-yl)-1H-benzo[d]imidazole-2(3H)-one (GEN1-015)

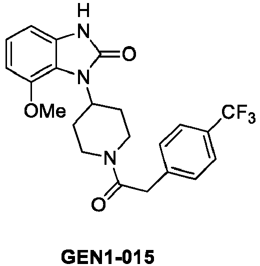

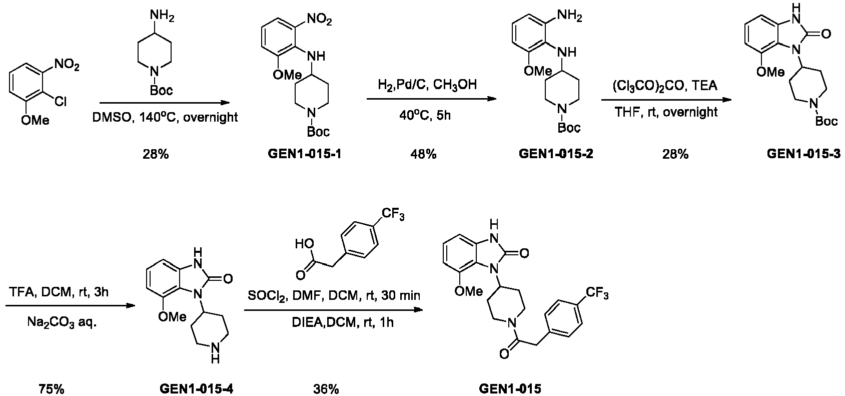

### Step 26.1. Synthesis of tert-butyl 4-((2-methoxy-6-nitrophenyl)amino)piperidine-1-carboxylate (GEN1-015-1)

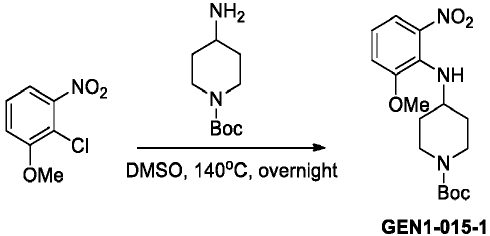

To a solution of 2-chloro-1-methoxy-3-nitrobenzene (1.50 g, 7.98 mmol) and tert-butyl 4-aminopiperidine-1-carboxylate (4.79 g, 23.9 mmol) in DMSO (25 mL) was added K₂CO₃ (3.30 g, 23.9 mmol). The mixture was stirred overnight at 140°C. After cooling, the mixture was poured into 150 mL of water, stirred for 10 minutes, and extracted with ethyl acetate (3×30 mL). The combined organic layer was washed with brine (20 mL), dried over Na₂SO₄ and concentrated. The product was purified by silica gel column chromatography (PE: EA = 20:1 ∼ 10:1) to obtain the desired compound (788 mg, yield 28%). ¹H NMR (300 MHz, CDCl₃): δ 7.74 (d, *J* = 8.7 Hz, 1H), 7.51 (d, *J* = 8.4 Hz, 1H), 6.95 (d, *J* = 8.1 Hz, 1H), 6.71 (t, *J* = 8.4 Hz, 1H), 4.14-4.08 (m, 1H), 3.99-3.95 (m, 2H), 3.88 (s, 3H), 2.93 (t, *J* = 8.7 Hz, 2H), 1.96-1.93 (m, 2H), 1.46 (s, 9H), 1.43-1.34 (m, 2H).

### Step 26.2. Synthesis of tert-butyl 4-((2-amino-6-methoxyphenyl)amino)piperidine-1-carboxylate (GEN1-015-2)

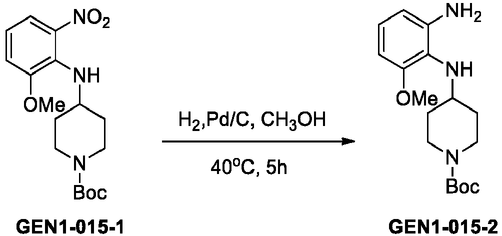

In methanol (15ml) were added tert-butyl 4-((2-methoxy-6-nitrophenyl)amino)piperidine-1-carboxylate (1.15 g, 3.28 mmol), Pd/C (10%, 100 mg). The mixture was stirred under a hydrogen atmosphere at 40°C for 5 hours. The mixture was filtered through diatomaceous earth. After the filtrate was concentrated, the residue was purified by silica gel column chromatography (PE: EA = 10:1 ∼ 5:1) to obtain the desired compound (500 mg, yield 48%) as a yellow oil. MS (ESI) Rt = 1.536 min, *m*/*z* 322.2 [M+H]⁺, purity: 47.9% @ 214 nm.

### Step 26.3. Synthesis of tert-butyl 4-(7-methoxy-2-oxo-2,3-dihydro-1H-benzo[d]imidazol-1-yl)piperidine-1-carboxylate (GEN1-015-3)

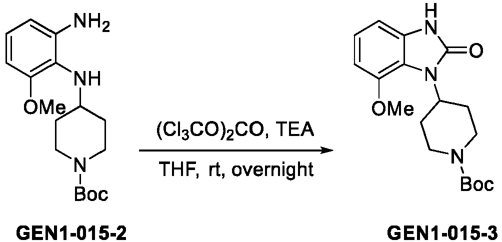

Tert-butyl 4-((2-amino-6-methoxyphenyl)amino)piperidine-1-carboxylate (500 mg, 1.56 mmol) and triethylamine (1.57 g, 15.6 mmol) were repectivey placed in THF (30 mL) and the mixture was cooled to 0°C. Triphosgene (463 mg, 1.56 mmol) was added in batches. The mixture was stirred at room temperature overnight. The mixture was concentrated, and the residue was diluted with ethyl acetate (20 mL) and water (20 mL). Ethyl acetate (3×20 mL) was added to extract the desired compound. The combined organic layer was washed with brine (20 mL), dried over Na₂SO₄ and concentrated. The residue was purified by C18 column chromatography (50-70% CH3CN aqueous solution) to obtain the desired compound (150 mg, yield 28%) as a yellow solid. MS (ESI) Rt = 1.650 min, *m*/*z* 370.2 [M+Na]⁺, purity: 99.1% @ 214 nm.

### Step 26.4. Synthesis of 7-methoxy-1-(piperidin-4-yl)-1H-benzo[d]imidazole-2(3H)-one (GEN1-015-4)

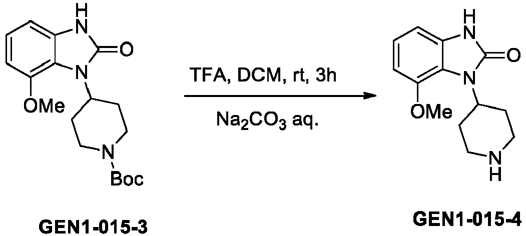

In DCM (4ml) were added tert-butyl 4-(7-methoxy-2-oxy-2,3-dihydro-1H-benzo[d]imidazol-1-yl)piperidine-1-carboxylate (150 mg, 0.430 mmol) and TFA (2mL). The mixture was stirred at room temperature for 3 hours. After the mixture was concentrated, the residue was diluted with DCM (10ml) and aqueous Na₂CO₃ (sat., 10ml). It was then extracted with DCM (2×20 mL). The combined organic layer was washed with brine (20 mL), dried over Na₂SO₄, and concentrated to the desired compound (80 mg, yield 75%) to form a yellow solid. MS (ESI) Rt = 1.101 min, *m*/*z* 248.1 [M+H]⁺, purity: 94.5% @ 214 nm.

### Step 26.5. Synthesis of 7-methoxy-1-(1-(2-(4-(trifluoromethyl)phenyl)acetyl)piperidin-4-yl)-1H-benzo[d]imidazole-2(3H)-one (GEN1-015)

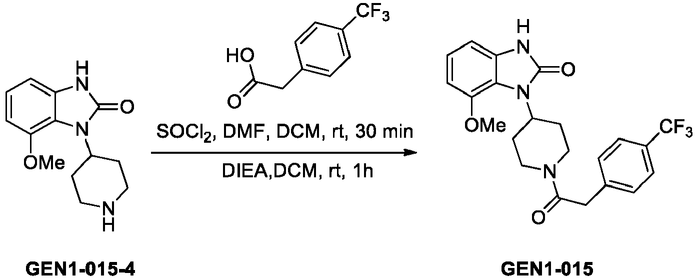

In a solution of 2-(4-(trifluoromethyl)phenyl)acetic acid (73 mg, 0.36 mmol) in DCM (1 mL) was added DMF (1 drop). SOCl₂ (96 mg, 0.81 mmol) was added. After stirring at room temperature for 30 minutes, the mixture was concentrated. The residue was dissolved in DCM (1 mL) and used in the next step. 7-methoxy-1-(piperidin-4-yl)-1H-benzo[d]imidazole-2(3H)-one (80mg, 0.32 mmol) and DIEA (209 mg, 1.62 mmol) in DCM (4ml) was stirred at room temperature for 5 min, and the previous solution was added dropwise. The mixture was stirred at room temperature for 1 hour. The mixture was poured into water (10ml) and extracted with DCM (20ml). The organic layer was dried over Na₂SO₄ and concentrated. The residue was purified using high performance liquid chromatography (Xbridge C18, 5 um, 19 x 150 mm, 20-70% B, A: H₂O (0.1% NH₄HCO₃), B: CH₃CN, uv = 214 nm, F = 15 mL/min) to obtain the desired compound (50 mg, yield 36%) as a white solid. ¹H NMR (400 MHz, CDCl₃): δ 8.63 (br s, 1H), 7.61 (d, *J* = 8.0 Hz, 2H), 7.45 (d, *J* = 7.6 Hz, 2H), 7.01 (t, *J* = 8.0 Hz, 1H), 6.72 (d, *J* = 8.0 Hz, 1H), 6.63 (d, *J* = 8.0 Hz, 1H), 4.87-4.81 (m, 2H), 4.05 (d, *J* = 14.0 Hz, 1H), 3.84 (s, 2H), 3.74 (s, 3H), 3.18 (t, *J* = 12.4 Hz, 1H), 2.70 (td, *J* = 13.2, 2.0 Hz, 1H), 2.47-2.41 (m, 2H), 1.83-1.81 (m, 2H). MS (ESI) Rt = 3.174 min, *m*/*z* 434.2 [M+H]⁺, purity: 98.9% @ 254 nm, 99.7% @ 214 nm.

### Example 27. 1-(1-(4-(trifluoromethyl)benzoyl)piperidin-4-yl)-1H-benzo[d]imidazole-2(3H)-one (GEN1-017)

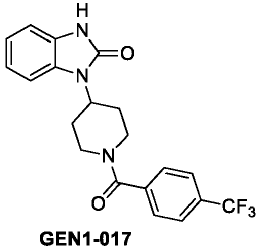

### Step 27.1. Synthesis of 1-(1-(4-(trifluoromethyl)benzoyl)piperidin-4-yl)-1H-benzo[d]imidazole-2(3H)-one (GEN1-017)

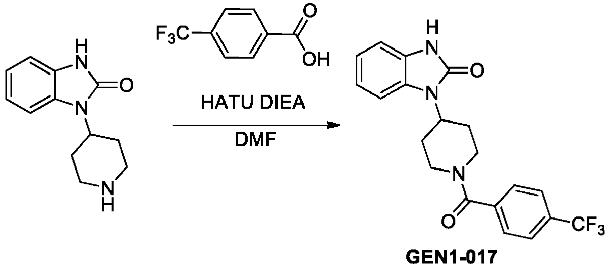

In DMF (3 mL) were added 1-(piperidin-4-yl)-1H-benzo[d]imidazole-2(3H)-one (114 mg, 0.526 mmol), 4-(trifluoro)methyl)benzoic acid (100 mg, 0.562 mmol), HATU (300 mg, 0.843 mmol), DIEA (217 mg, 1.69 mmol). The reaction mixture was stirred at room temperature for 12 hours. The mixture was directly purified with a C18 column (CH₃CN: water = 5% ∼ 50%) to obtain the desired compound (100 mg, yield 50%) as a gray solid. ¹H NMR (400 MHz, DMSO-*d₆*): δ 10.87 (s, 1H), 7.84 (d, *J=* 8.0 Hz, 2H), 7.70 (d, *J=* 8.0 Hz, 2H), 7.38-7.36 (m, 1H), 7.03-6.98 (m, 3H), 4.70-4.67 (m, 1H), 4.51-4.45 (m, 1H), 3.61-3.59 (m, 1H), 3.28-3.21 (m, 1H), 3.02-2.89 (m, 1H), 2.37-2.27 (m, 2H), 1.83-1.65 (m, 2H). MS (ESI): Rt = 3.647 min, *m*/*z* 390.1 [M+H]⁺, purity: 99.21% @ 254 nm, 99.95% @ 214 nm.

### Example 28. 1-(1-(3-(4-(trifluoromethyl)phenyl)propionyl)piperidin-4-yl)-1H-benzo[d]imidazole-2(3H)-one (GEN1-018)

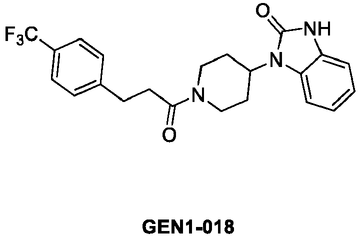

### Step 28.1. Synthesis of 1-(1-(3-(4-(trifluoromethyl)phenyl)propionyl)piperidin-4-yl)-1H-benzo[d]imidazole-2(3H)-one (GEN1-018)

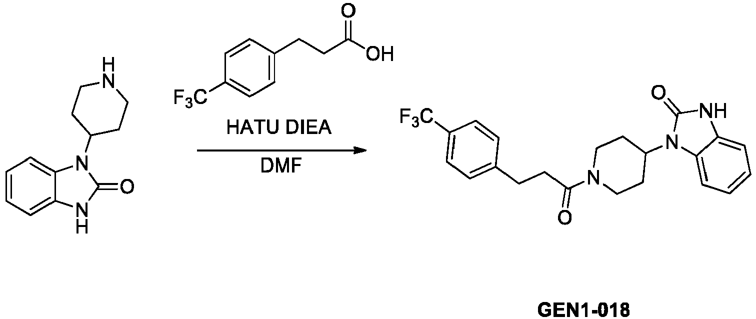

In DMF (3 mL) were added 1-(piperidin-4-yl)-1H-benzo[d]imidazole-2(3H)-one (100 mg, 0.460 mmol), 3-(4- (Trifluoromethyl)phenyl)propionic acid (100 mg, 0.460 mmol) and HATU (262 mg, 0.690 mmol), DIEA (177 mg, 1.37 mmol). The reaction mixture was stirred at room temperature for 12 hours. The mixture was directly purified with a C18 column (CH₃CN: water = 5% ∼ 50%) to obtain the desired compound (100 mg, yield 50%) as a white solid. ¹H NMR (400 MHz, DMSO-*d₆*): δ 10.85 (s, 1H), 7.64 (d, *J* = 8.0 Hz, 2H), 7.52 (d, *J* = 8.0 Hz, 2H), 7.21-7.19 (m, 1H), 6.97-6.96 (m, 3H), 4.58 (d, *J* = 12.8 Hz, 1H), 4.44-4.37 (m, 1H), 4.04 (d, *J* = 12.8 Hz, 1H), 3.13 (t, *J* = 13.2 Hz, 1H), 2.97-2.93 (m, 2 H), 2.73-2.71 (m, 2H), 2.70-2.63 (m, 1H), 2.20-2.06 (m, 2H), 1.71-1.68 (m, 2H). MS (ESI): Rt = 3.748 min, *m*/*z* 418.2 [M+H]⁺, purity: 98.23% @ 254 nm, 99.39% @ 214 nm.

### Example 29. 1-methyl-3-(1-(2-(4-(trifluoromethyl)phenyl)acetyl)piperidin-4-yl)-1H-benzo[d]imidazole-2(3H)-one (GEN1-020)

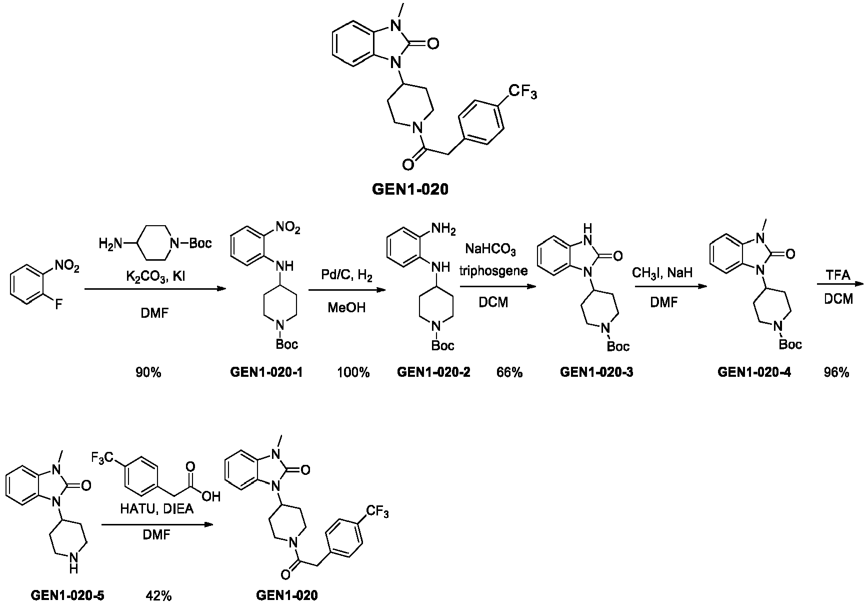

### Step 29.1. Synthesis of tert-butyl 4-((2-nitrophenyl)amino)piperidine-1-carboxylate (GEN1-020-1)

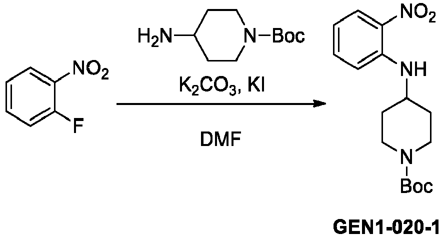

A suspension of 1-fluoro-2-nitrobenzene (3.50 g, 25.0 mmol), tert-butyl 4-aminopiperidine-1-carboxylate (5.0 g, 25 mmol), K₂CO₃ (5.1 g, 37 mmol) and KI (42 mg, 0.25 mmol) in DMF (35 mL) was stirred overnight at 80°C. After cooling to room temperature, the reaction mixture was poured into water (200 mL) and extracted with EtOAc (70 mL×3). The combined organic layer was washed with water (70 mL), brine (70 mL), dried over MgSO₄ and concentrated to obtain the desired compound (7.2 g, yield 90%) as an orange oil. ¹HNMR (400 MHz, CDCl₃): δ 8.18 (dd, *J* = 8.8, 1.2 Hz, 1H), 8.10 (d, *J* = 7.2 Hz, 1H), 7.45-7.41 (m, 1H), 6.87 (d, *J* = 8.8 Hz, 1H), 6.67-6.63 (m, 1H), 4.09-3.94 (m, 2H), 3.74-3.64 (m, 1H), 3.08-3.03 (m, 2H), 2.08-2.05 (m, 2H), 1.61-1.51 (m, 2H), 1.48 (s, 9H).

### Step 29.2. Synthesis of tert-butyl 4-((2-aminophenyl)amino)piperidine-1-carboxylate (GEN1-020-2)

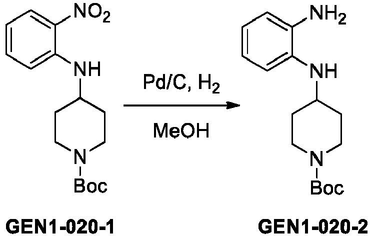

4-tert-butyl((2-nitrophenyl)amino)piperidine-1-carboxylate (7.20 g, 220 mmol) was dissolved in methanol (40 mL), and Pd/C (10%, 720 mg) was added under a nitrogen atmosphere. The resulting mixture was degassed with H₂ and stirred at 35°C overnight under an H₂ atmosphere. The reaction mixture was filtered, and the filtrate was concentrated to obtain the desired compound (6.5 g, yield 100%) as a brown oil. MS (ESI) Rt = 1.81 min, *m*/*z* 292.1, purity: 98% @ 254 nm, 93% @ 214 nm.

### Step 29.3. Synthesis of tert-butyl 4-(2-oxo-2,3-dihydro-1H-benzo[d]imidazol-1-yl)piperidine-1-carboxylate (GEN1-020-3)

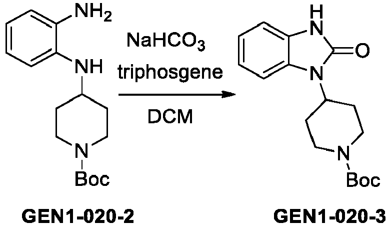

A suspension of tert-butyl 4-((2-aminophenyl)amino)piperidine-1-carboxylate (500 mg, 1.72 mmol) and NaHCO₃ (1.44 g, 17.2 mmol) in DCM (5ml) was added with triphosgene (510 mg, 1.72 mmol) in batches under ice bath conditions. After stirring overnight at room temperature, the reaction mixture was poured into water (50 mL), extracted with DCM (30 mL×3), and the mixed organic layer was washed with brine (30 mL), dried over MgSO₄ and concentrated. The crude product was purified by silica gel column chromatography (DCM: MeOH = 50:1) to obtain the desired compound (360 mg, yield 66%) as a pink solid. ¹HNMR (300 MHz, CDCl₃): δ 9.50 (s, 1H), 7.19-7.08 (m, 4H), 4.60-4.47 (m, 1H), 4.43-4.23 (m, 2H), 2.96-2.87 (m, 2H), 2.45-2.31 (m, 2H), 1.90-1.86 (m, 2H), 1.55 (s, 9H).

### Step 29.4. Synthesis of tert-butyl 4-(3-methyl-2-oxo-2,3-dihydro-1H-benzo[d]imidazol-1-yl)piperidine-1-carboxylate (GEN1-020-4)

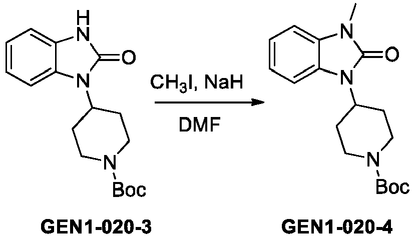

In tert-butyl 4-(2-oxo-2,3-dihydro-1H-benzo[d]imidazol-1-yl)piperidine-1-carboxylate (100 mg, 0.315 mmol) in DMF (2 mL) was added NaH (60%, 25 mg in mineral oil, 0.63 mmol) in an ice bath and under a nitrogen atmosphere. After the mixture was stirred for 15 min, CH₃I (50 mg, 0.35 mmol) was added dropwise under an ice bath. The resulting mixture was then stirred at room temperature for 1.5 hours. The reaction mixture was poured into water (30ml), extracted with EtOAc (20ml×3), and the combined organic layer was washed with water (30ml), brine (30ml), dried over MgSO₄, and concentrated to give the desired compound (130 mg, crude product) as a yellow solid. ¹HNMR (400 MHz, CDCl₃): δ 7.13-7.04 (m, 3H), 7.00-6.98 (m, 1H), 4.53-4.44 (m, 1H), 4.38-4.22 (m, 2H), 3.41 (s, 3H), 2.92-2.79 (m, 2H), 2.37-2.26 (m, 2H), 1.82-1.79 (m, 2H), 1.50 (s, 9H).

### Step 29.5. Synthesis of 1-methyl-3-(piperidin-4-yl)-1H-benzo[d]imidazole-2(3H)-one (GEN1-020-5)

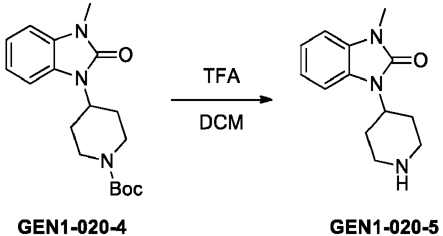

In a solution of tert-butyl 4-(3-methyl-2-oxo-2,3-dihydro-1H-benzo[d]imidazol-1-yl)piperidine-1-carboxylate (130 mg, 0.315 mmol) in DCM (3ml) was added TFA (1ml) at room temperature. The mixture was stirred overnight. The reaction mixture was concentrated and the residue was dissolved in DCM (15ml). Na₂CO₃ (15 mL of saturated solution) was added to the solution and mixed and the mixture was stirred for 20 minutes. The mixture was extracted with DCM (15 mL×2). The combined organic layer was dried with MgSO₄ and concentrated to obtain the desired compound (70 mg, yield 96%, the last two steps) as a white solid. MS (ESI) Rt = 1.12 min, *m*/*z* 232.2 [M+H]⁺, purity: 98% @ 254 nm, 98% @ 214 nm.

### Step 29.6. Synthesis of 1-methyl-3-(1-(2-(4-(trifluoromethyl)phenyl)acetyl)piperidin-4-yl)-1H-benzo[d]imidazole-2(3H)-one (GEN1-020)

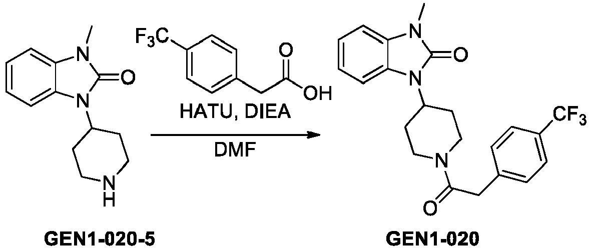

In DMF (2 mL) were added 1-methyl-3-(piperidin-4-yl)-1H-benzo[d]imidazole-2(3H)-one (70 mg, 0.30 mmol), 2-(4-(trifluoromethyl)phenyl)acetic acid (74 mg, 0.36 mmol) and DIEA (117 mg, 0.909 mmol), and HATU (173 mg, 0.455 mmol) was added at room temperature. The resulting mixture was stirred at room temperature for 1.5 hours. The reaction mixture was directly purified by a C18 column (CH₃CN: water = 5% ∼ 45%) to obtain the desired compound (53.3 mg, yield 42%) as a white solid. ¹H NMR (400 MHz, CDCl3) δ 7.63 (d, *J* = 8.0 Hz, 2H), 7.45 (d, *J* = 8.0 Hz, 2H), 7.10 (t, *J* = 7.6 Hz, 1H), 7.03 (t, *J* = 7.6 Hz, 1H), 6.99 (t, *J* = 8.0 Hz, 1H), 6.86 (t, *J* = 8.0 Hz, 1H), 4.89-4.86 (m, 1H), 4.59-4.49 (m, 1H), 4.06-4.02 (m, 1H), 3.86 (s, 2H), 3.40 (s, 3H), 3.23-3.16 (m, 1H), 2.75-2.68 (m, 1H), 2.31-2.20 (m, 1H), 2.09-1.98 (m, 1H), 1.88-1.85 (m, 1H), 1.80-1.76 (m, 1H). MS (ESI) Rt = 3.629 min, *m*/*z* 418.1 [M+H]⁺, purity: 91.32% @ 254 nm, 99.57% @ 214 nm.

### Example 30. Cis-N-(4-(2-oxo-2,3-dihydro-1H-benzo[d]imidazol-1-yl)cyclohexyl)-2-(4- (trifluoromethyl)phenyl)acetamide (GEN1-021)

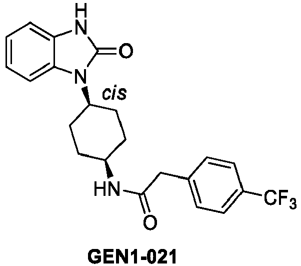

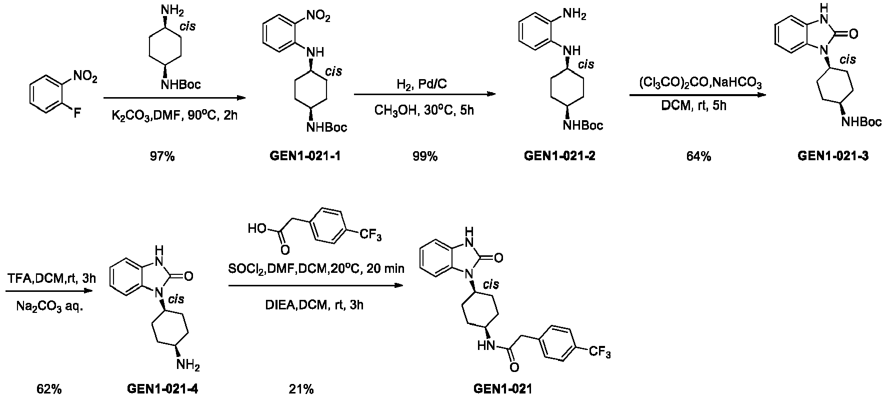

### Step 30.1. Synthesis of tert-butyl cis-(4-((2-nitrophenyl)amino)cyclohexyl)carbamate (GEN1-021-1)

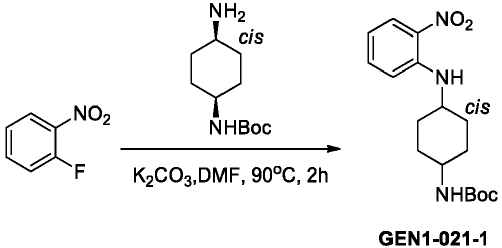

In a solution of 1-fluoro-2-nitrobenzene (500 mg, 3.55 mmol) and tert-butyl cis (4-aminocyclohexyl)carbamate (911 mg, 4.26 mmol) in DMF (15 ml) was added K₂CO₃ (734 mg, 5.32 mmol). The mixture was stirred at 90°C for 2 hours, and then cooled, poured into 60ml of water, stirred for 10 minutes, and filtered. The filter cake was washed with water (2 x 10 mL) and then dissolved in ethyl acetate (50 mL). The organic solution was dried over Na₂SO₄ and concentrated to the desired compound (1.15 g, yield 97%) as an orange solid. ¹H NMR (400 MHz, CDCl₃): δ 8.31-8.29 (m, 1H), 8.19-8.17 (m, 1H), 7.44-7.40 (m, 1H), 6.85 (d, *J=* 8.4 Hz, 1H), 6.65-6.61 (m, 1H), 4.58 (br s, 1H), 3.76 (br s, 1H),3.63 (s, 1H), 1.86-1.82 (m, 6H), 1.60-1.52 (m, 2H), 1.45 (s, 9H).

### Step 30.2. Synthesis of tert-butyl cis-(4-((2-aminophenyl)amino)cyclohexyl)carbamate (GEN1-021-2)

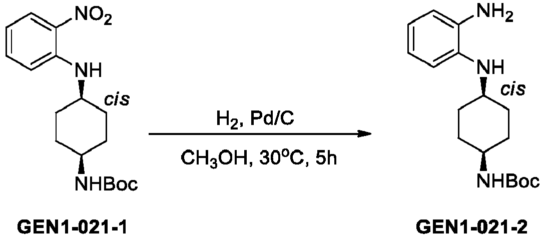

Tert-butyl cis(4-((2-nitrophenyl)amino)cyclohexyl)carbamate (500 mg, 1.49 mmol) was dissolved in methanol (15 mL), and Pd/C (10 %, 50 mg) was added. The mixture was stirred under a hydrogen atmosphere at 30°C for 5 hours. The mixture was filtered through diatomaceous earth. After the filtrate was concentrated, the desired compound (450 mg, yield 99%) was obtained as a brown oil. ¹H NMR (400 MHz, CDCl₃): δ 6.83-6.79 (m, 1H), 6.74-6.72 (m, 1H), 6.69-6.65 (m, 2H), 4.58 (br s, 1H), 3.65 (br s, 1H), 3.44 (s, 1H), 3.31 (br s, 2H), 1.85-1.72 (m, 4H), 1.67-1.61 (m, 4H), 1.45 (s, 9H).

### Step 30.3. Synthesis of tert-butyl cis(4-(2-oxo-2,3-dihydro-1H-benzo[d]imidazol-1-yl)cyclohexyl)carbamate (GEN1-021-3)

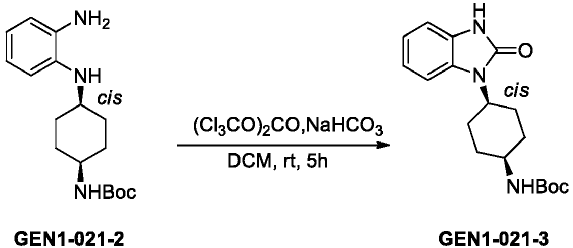

In a solution of tert-butyl cis(4-((2-aminophenyl)amino)cyclohexyl)carbamate (450 mg, 1.48 mmol) in DCM (50 mL) was added NaHCO₃ (1.24 g, 14.8 mmol)). The mixture was cooled to 5°C. Triphosgene (438 mg, 1.48 mmol) was added step by step. The mixture was stirred at room temperature for 5 hours. The mixture was poured into water (50 mL) and extracted with DCM (2×20 mL). The combined organic layer was washed with brine (20 mL), dried over Na₂SO₄ and concentrated. The residue was purified by C18 column chromatography (40∼70% CH₃CN in water) to obtain the desired compound (310 mg, yield 64%). ¹H NMR (400 MHz, CDCl₃): δ 9.19 (br s, 1H), 7.11-7.06 (m, 4 H), 4.99 (d, *J* = 8.8 Hz, 1H), 4.19 (br s, 1H), 3.93 (s, 1H), 2.43-2.33 (m, 2H), 2.05-2.01 (m, 2H), 1.78-1.68 (m, 4H), 1.50 (s, 9H).

### Step 30.4. Synthesis of cis-1-(4-aminocyclohexyl)-1H-benzo[d]imidazole-2(3H)-one (GEN1-021-4)

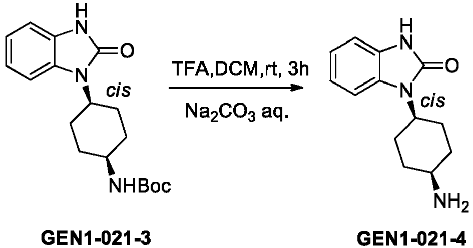

In DCM (4ml) were added tert-butyl cis-(4-(2-oxo-2,3-dihydro-1H-benzo[d]imidazol-1-yl)cyclohexyl)carbamate (300 mg, 0.91 mmol) and TFA (2ml). The mixture was stirred at room temperature for 3 hours. The mixture was concentrated. The residue was diluted with DCM (10ml) and aqueous Na₂CO₃ (sat., 5mL). It was then extracted with DCM (2×20 mL). The combined organic layer was washed with brine (5 mL), dried over Na₂SO₄, and concentrated to the desired compound (130 mg, yield 62%) as a yellow solid. MS (ESI) Rt = 1.14 min, *m*/*z* 232.1 [M+H]⁺, purity: 96.5% @ 214 nm.

### Step 30.5. Synthesis of cis-N-(4-(2-oxo-2,3-dihydro-1H-benzo[d]imidazol-1-yl)cyclohexyl)-2-(4-(trifluoromethyl)phenyl)acetamide (GEN1-021)

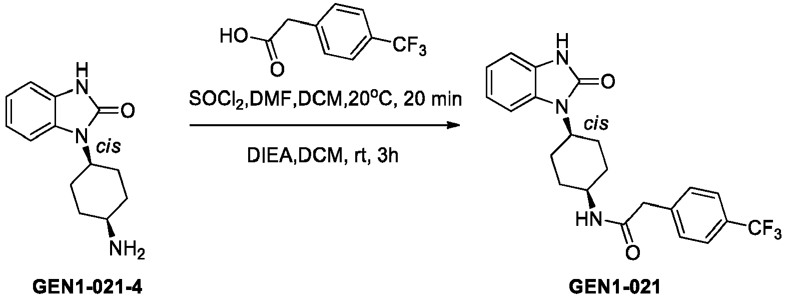

In DCM (1 ml) was added a mixture of 2-(4-(trifluoromethyl)phenyl)acetic acid (74 mg, 0.36 mmol) and DMF (1 drop). SOCl₂ (82 mg, 0.69 mmol) was added. After stirring for 20 minutes at 20°C, the mixture was concentrated. The residue was dissolved in DCM (1 mL) and used in the next step. Cis-1-(4-aminocyclohexyl)-1H-benzo(d)imidazole-2(3H)-one (80 mg, 0.35 mmol) and DIEA (223 mg, 1.73 mmol) in DCM (4 mL) were stirred at room temperature for 10 min, and the previous solution was added dropwise. The mixture was stirred at room temperature for 1 hour. The mixture was poured into water (20 mL) and extracted with DCM (2 x 20 mL). The combined organic layer was washed with brine (20 mL), dried over Na₂SO₄ and concentrated. The residue was purified using high performance liquid chromatography (Xbridge C18, 5 um, 19 x 150 mm, 30-60% B, A: H₂O (0.1 % NH₄HCO₃), B: CH₃CN, uv = 214 nm, F = 15 mL/min) to obtain the desired compound (30 mg, yield: 21%) as a white solid. ¹H NMR (400 MHz, CDCl₃): δ 10.83 (s, 1H), 8.26 (d, *J* = 6.0 Hz, 1H), 7.68 (d, *J* = 8.0 Hz, 2H), 7.56 (d, *J* = 8.0 Hz, 2H), 7.33-7.32 (m, 1H), 7.04-6.94 (m, 3H), 4.22-4.16 (m, 1H), 3.91 (br s, 1H), 3.68 (s, 2H), 2.38-2.27 (m, 2H), 1.92-1.89 (m, 2H), 1.68-1.60 (m, 2H), 1.52-1.49 (m, 2H). MS (ESI) Rt = 3.826 min, *m*/*z* 418.1 [M+H]⁺, purity: 95.6% @ 254 nm, 99.3% @ 214 nm.

### Example 31. Trans-N-(4-(2-oxo-2,3-dihydro-1H-benzo[d]imidazol-1-yl)cyclohexyl)-2-(4-(trifluoromethyl)phenyl)acetamide (GEN1-022)

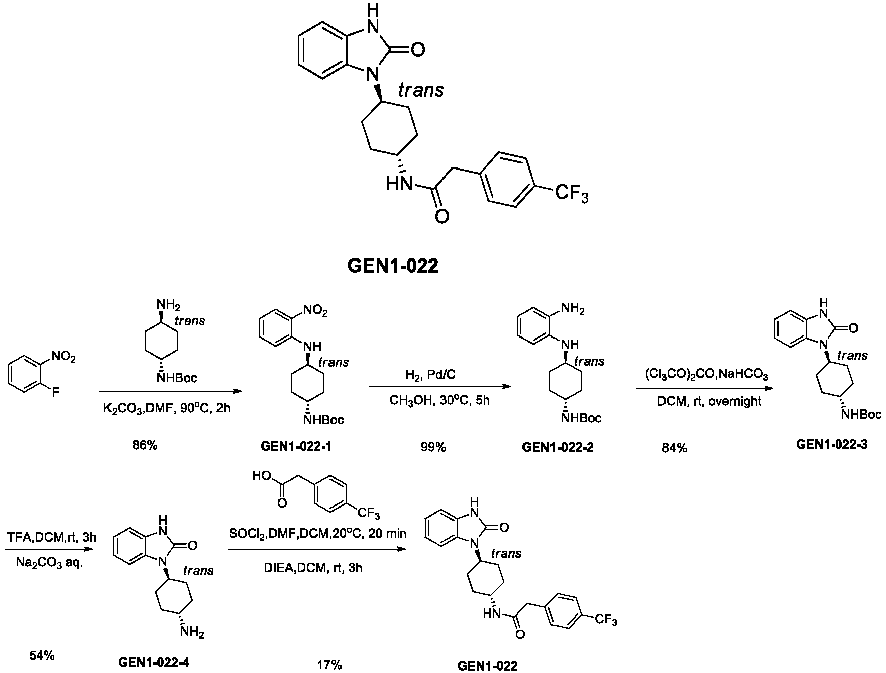

### Step 31.1. Synthesis of tert-butyl trans-4-((2-nitrophenyl)amino)cyclohexyl)carbamate (GEN1-022-1)

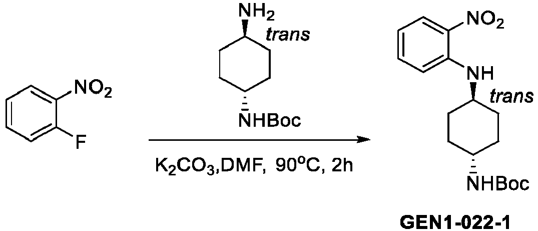

In a solution of 1-fluoro-2-nitrobenzene (500 mg, 3.55 mmol) and tert-butyl trans (4-aminocyclohexyl)carbamate (911 mg, 4.26 mmol) in DMF (15ml) was added K₂CO₃ (734 mg, 5.32 mmol). The mixture was stirred at 90°C for 2 hours and then cooled, and then poured into 60ml of water, stirred for 10 minutes, and filtered. The filter cake was dissolved in ethyl acetate (50 mL). The organic solution was washed with aqueous hydrochloric acid (0.5 M, 2 x 10 ml), brine (20 ml), dried over Na₂SO₄ and concentrated. The product was triturated with petroleum ether (10 mL) to obtain the ideal yellow solid (1.02 g, yield 86%) MS (ESI) Rt = 1.869 min, *m*/*z* 336.2 [M+H]⁺, purity: 98.4% @ 214 nm.

### Step 31.2. Synthesis of tert-butyl trans-4-((2-aminophenyl)amino)cyclohexyl)carbamate (GEN1-022-2)

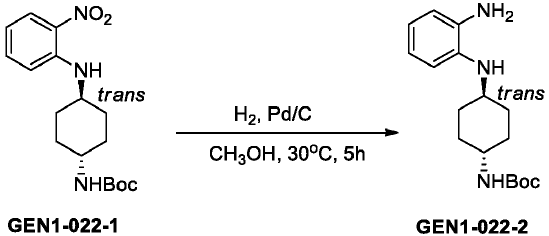

Tert-butyl trans(4-((2-nitrophenyl)amino)cyclohexyl)carbamate (500 mg, 1.49 mmol) was dissolved in methanol (15 mL), and Pd/C (10 %, 50 mg) was added. The mixture was stirred under a hydrogen atmosphere at 30°C for 5 hours. The mixture was filtered through diatomaceous earth. The filtrate was concentrated to obtain the desired compound (450 mg, yield 99%) as a brown solid. MS (ESI) Rt = 1.677 min, *m*/*z* 306.2 [M+H]⁺, purity: 93.5% @ 214 nm.

### Step 31.3. Synthesis of tert-butyl trans(4-(2-oxo-2,3-dihydro-1H-benzo[d]imidazol-1-yl)cyclohexyl)carbamate (GEN1-022-3)

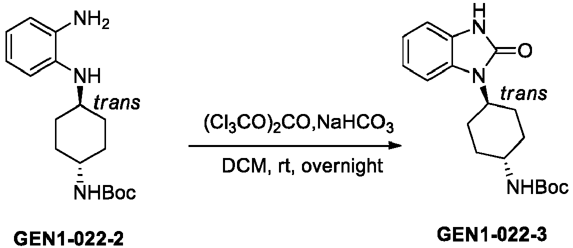

In a solution of tert-butyl trans(4-((2-aminophenyl)amino)cyclohexyl)carbamate (450 mg, 1.48 mmol) in DCM (50ml) was added NaHCO₃ (1.24 g, 14.8 mmol)). The mixture was cooled to 5°C. Triphosgene (438 mg, 1.48 mmol) was added step by step. The mixture was stirred at room temperature overnight. The mixture was poured into water (60 mL) and extracted with DCM: CH₃OH = 10:1 (3 x 30 mL). The combined organic layer was washed with water (20 mL), dried over Na₂SO₄ and concentrated. The residue was triturated with PE: EA = 1:1 (10 mL) to obtain the desired compound (410 mg, yield 84%) as a pink solid. ¹H NMR (400 MHz, DMSO-*d₆*): δ 10.80 (br s, 1H), 7.32-7.30 (m, 1H), 6.99-6.92 (m, 3H), 6.78 (d, *J* = 7.6 Hz, 1H), 4.14-4.07 (m, 1H), 3.47-3.33 (m, 1H), 2.26-2.16 (m, 2H), 1.99-1.89 (m, 2H), 1.68-1.65 (m, 2H), 1.40 (s, 9H), 1.37-1.30 (m, 2H).

### Step 31.4. Synthesis of trans-1-(4-aminocyclohexyl)-1H-benzo[d]imidazole-2(3H)-one (GEN1-022-4)

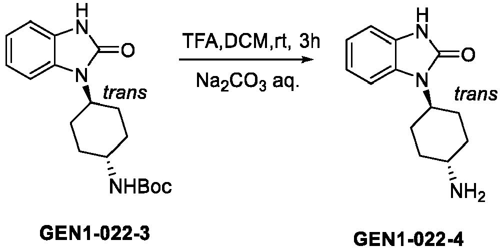

In DCM (4ml) were added tert-butyl trans(4-(2-oxo-2,3-dihydro-1H-benzo[d]imidazol-1-yl)cyclohexyl)carbamate (400 mg, 1.21 mmol) and TFA (2 ml). The mixture was stirred at room temperature for 3 hours. The mixture was concentrated. The residue was diluted with DCM (10ml) and aqueous Na₂CO₃ (sat., 10ml). The mixture was extracted with DCM: CH₃OH = 10:1 (3×30 mL), and the combined organic layers were concentrated. The residue was dissolved in DCM (50 mL) and filtered. After the filtrate was concentrated, the desired compound (150 mg, yield 54%) was obtained as a yellow solid. MS (ESI) Rt = 1.051 min, *m*/*z* 232.1 [M+H]⁺, purity: 88.9 % @ 214 nm.

### Step 31.5. Synthesis of trans-N-(4-(2-oxo-2,3-dihydro-1H-benzo[d]imidazol-1-yl)cyclohexyl)-2-(4-(trifluoromethyl)phenyl)acetamide (GEN1-022)

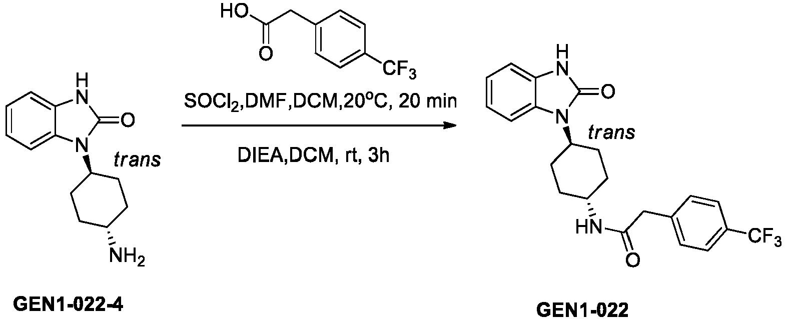

(4-(trifluoromethyl)phenyl)acetic acid (46 mg, 0.23 mmol) and DMF (1 drop) were added to DCM (1 mL). SOCl₂ (52 mg, 0.43 mmol) was added. After stirring for 20 minutes at 20°C, the mixture was concentrated. The residue was dissolved in DCM (1 mL) and used in the next step. Trans 1-(4-aminocyclohexyl)-1H-benzo(d)imidazole-2(3H)-one (50 mg, 0.22 mmol) and DIEA (140 mg, 1.08 mmol) in DCM (4 mL) were stirred at room temperature for 10 min, and the previous solution was added dropwise. The mixture was stirred at room temperature for 3 hours. The mixture was poured into water (20 mL) and extracted with DCM: CH₃OH = 10:1 (2 x 20 mL). The mixed organic layer was washed with brine (20 mL) and concentrated. The residue was purified using high performance liquid chromatography (Xbridge C18, 5 um, 19 x 150 mm, 30-60% B, A: H₂O (0.1% NH₄HCO₃), B: CH₃CN, uv = 214 nm, F = 15 mL/min) to obtain the desired compound (15 mg, yield 17%) as a white solid. H NMR (400 MHz, DMSO-*d₆*): δ 10.80 (s, 1H), 8.12 (d, *J* = 7.6 Hz, 1H), 7.67 (d, *J* = 8.4 Hz, 2H), 7.59 (d, *J* = 8.0 Hz, 2H), 7.36-7.30 (m, 1H), 6.97-6.95 (m, 3H), 4.22-4.13 (m, 1H), 3.76-3.64 (m, 1H), 3.52 (s, 2H), 2.29-2.19 (m, 2H), 1.95-1.92 (m, 2H), 1.70-1.68 (m, 2H), 1.43-1.33 (m, 2H). MS (ESI) Rt = 3.376 min, *m*/*z* 418.1 [M+H]⁺, purity: 95.1% @ 254 nm, 98.6% @ 214 nm.

### Example 32. 1-(1-(2-(4-(trifluoromethyl)phenyl)acetyl)azetidin-3-yl)-1H-benzo[d]imidazole-2(3H)-one (GEN1-023)

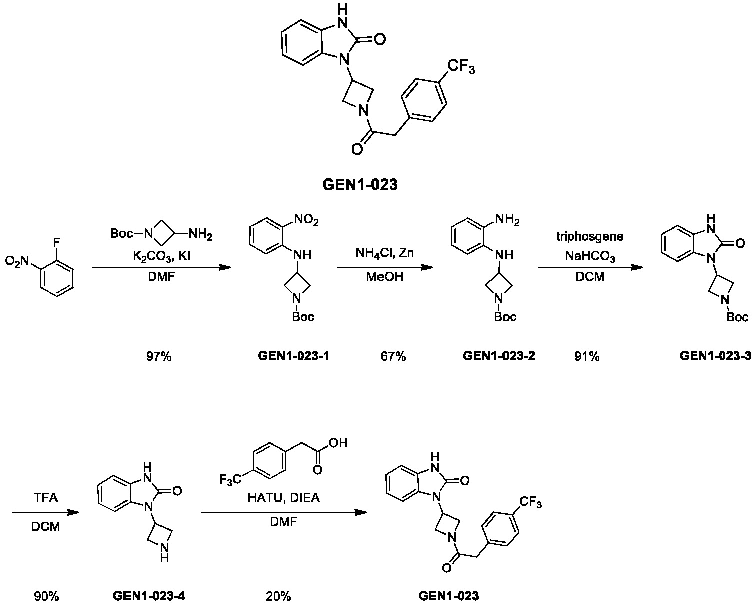

### Step 32.1. Synthesis of tert-butyl 3-((2-nitrophenyl)amino)azetidine-1-carboxylate (GEN1-023-1)

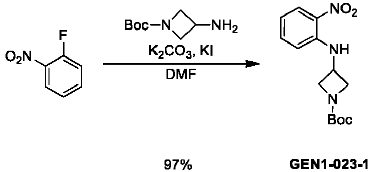

1-fluoro-2-nitrobenzene (1.0 g, 7.1 mmol), tert-butyl 3-aminoazetidine-1-carboxylate (1.3 g, 7.8 mmol) and K₂CO₃ (1.9 g, 14 mmol), KI (40 mg, 0.28 mmol) were added to DMF (20 mL). After stirring for 12 hours at 95°C, it was cooled to room temperature, poured into 50 mL of water, and extracted with EtOAc (30×2 mL). The combined organic layer was washed with brine, dried over Na₂SO₄, and concentrated to the desired (2.1 g, yield 97%) yellow solid. ¹H NMR (400 MHz, DMSO-*d₆*): δ 8.22-8.20 (m, 2H), 7.46 (t, *J* = 7.2 Hz, 1H), 6.76 (t, *J* = 7.2 Hz, 1H), 6.53 (d, *J* = 8.4 Hz, 1H), 4.40-4.33 (m, 3H), 3.90-3.87 (m, 2H), 1.43 (s, 9H).

### Step 32.2. Synthesis of tert-butyl 3-((2-aminophenyl)amino)azetidine-1-carboxylate (GEN1-023-2)

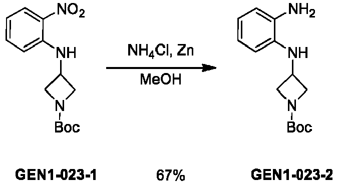

In a solution of tert-butyl 3-((2-nitrophenyl)amino)azetidine-1-carboxylate (1.0 g, 3.4 mmol) and NH₄Cl (890 mg, 17.1 mmol) in MeOH (15 mL) was added Zn powder (890 mg, 13.7 mmol). The reaction mixture was stirred at 35°C for 12 hours. The reaction mixture was filtered and the filtrate was concentrated to the desired (600 mg, 67% yield) red solid. MS (ESI): Rt = 1.54 min, *m*/*z* 164.0, [M + H-Boc]⁺. purity: 95% @ 254 nm, 98% @ 214 nm.

### Step 32.3. Synthesis of tert-butyl 3-(2-oxo-2,3-dihydro-1H-benzo[d]imidazol-1-yl)azetidine-1-carboxylate (GEN1-023-3)

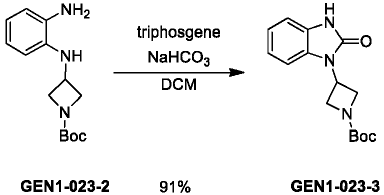

In a mixture of tert-butyl 3-((2-aminophenyl)amino)azetidine-1-carboxylate (600 mg, 2.30 mmol) and NaHCO₃ (1.9 g, 2.3 mmol) in DCM (10ml), triphosgene (541 mg, 1.80 mmol) was added under ice bath. The reaction mixture was stirred at room temperature for 2 hours. The mixture was poured into 10 mL water and extracted with DCM (10×2 mL). The combined organic layer was washed with brine, dried over Na₂SO₄, and concentrated to the desired (598 mg, yield 91%) red solid. MS (ESI): Rt = 1.55 min, *m*/*z* 234.1, [M + 2H-*t*-Bu]⁺. purity: 100% @ 254 nm, 88% @ 214 nm.

### Step 32.4. Synthesis of 1-(azetidin-3-yl)-1H-benzo[d]imidazole-2(3H)-one (GEN1-023-4)

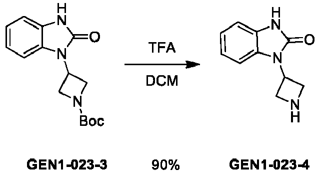

In DCM (4ml) were added tert-butyl 3-(2-oxo-2,3-dihydro-1H-benzo[d]imidazol-1-yl)-azetidine-1-carboxylate (598 mg, 2.07 mmol) and TFA (1ml). The reaction mixture was stirred at room temperature for 12 hours. The mixture was concentrated to obtain a crude product. The crude product was dissolved in water (2ml) and basified to pH=9 with aqueous Na₂CO₃ (sat., 5ml). It was then extracted with DCM (10×4 mL). The combined organic layer was dried over Na₂SO₄ and concentrated to the desired (352 mg, yield 90%) white solid. MS (ESI): Rt = 0.39 min, *m*/*z* 190.0, [M + H]⁺. purity: 95% @ 254 nm, 90% @ 214 nm.

### Step 32.5. Synthesis of 1-(1-(2-(4-(trifluoromethyl)phenyl)acetyl)azetidin-3-yl)-1H-benzo [d] imidazole-2(3H)-one (GEN1-023)

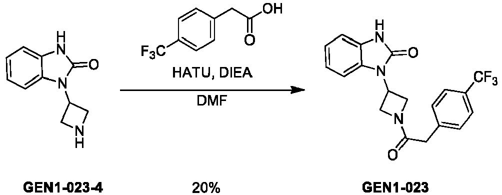

In DMF (1.5 mL) were added 1-(azetidin-3-yl)-1H-benzo[d]imidazole-2(3H)-one (75 mg, 0.40 mmol), 2-(4-(trifluoromethyl)phenyl)acetic acid (81 mg, 0.40 mmol) and HATU (227 mg, 0.597 mmol), DIEA (154 mg, 1.20 mmol). The reaction mixture was stirred at room temperature for 12 hours. The mixture was purified with a C18 column (CH₃CN: water = 5% ∼ 50%) to obtain the desired (30 mg, yield 20%) white solid. ¹H NMR (400 MHz, CDCl₃): δ 8.83 (s, 1H), 7.62 (d, *J* = 7.6 Hz, 2H), 7.48 (d, *J* = 8.4 Hz, 2H), 7.13-7.08 (m, 2H), 7.01-6.94 (m, 2H), 5.37-5.33 (m, 1H), 4.72-4.69 (m, 1H), 4.60-4.54 (m, 2H), 4.54-4.47 (m, 1H), 3.65 (s, 2H). MS (ESI): Rt = 3.45 min, *m*/*z* 376.2 [M+H]⁺, purity: 95.48% @ 254 nm, 99.19% @ 214 nm.

### Example 33. 1-(1-(2-(6-(trifluoromethyl)pyridin-3-yl)acetyl)piperidin-4-yl)-1H-benzo[d]imidazole- 2(3H)-one (GEN1-027)

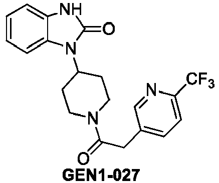

### Step 33.1. Synthesis of 1-(1-(2-(6-(trifluoromethyl)pyridin-3-yl)acetyl)piperidin-4-yl)-1H-benzo[d]imidazole-2(3H)-one (GEN1-027)

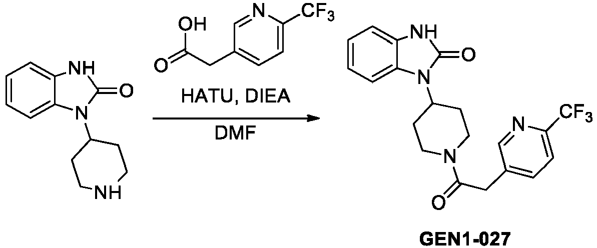

In DMF (2 mL) were added 1-(piperidin-4-yl)-1H-benzo[d]imidazole-2(3H)-one (60 mg, 0.28 mmol), 2-(6- (trifluoromethyl)pyridin-3-yl)acetic acid (64 mg, 0.31 mmol), and DIEA (109 mg, 0.844 mmol), and HATU (160 mg, 0.421 mmol) was added at room temperature. The resulting mixture was stirred at room temperature for 1.5 hours. The reaction mixture was directly purified on a C18 column (CH₃CN: water = 5% ∼ 45%) to obtain the desired compound (65.5 mg, yield 58%) as a white solid. ¹H NMR (400 MHz, DMSO-*d₆*): δ 10.85 (s, 1H), 8.66 (s, 1H), 7.97 (d, *J* = 8.0 Hz, 1H), 7.88 (d, *J* = 7.6 Hz, 1H), 7.20-7.18 (m, 1H), 7.01-6.93 (m, 3H), 4.86-4.55 (m, 1H), 4.49-4.41 (m, 1H), 4.19-4.16 (m, 1H), 4.00 (dd, *J* = 22.0, 16.0 Hz, 2H), 3.28-3.22 (m, 1H), 2.77-2.71 (m, 1H), 2.34-2.23 (m, 1H), 2.20-2.09 (m, 1H), 1.78-1.71 (m, 2H). MS (ESI) Rt = 3.236 min, *m*/*z* 405.2 [M+H]⁺, purity: 98.70% @ 254 nm, 99.59% @ 214 nm.

### Example 34. 1-(1-(2-(5-(trifluoromethyl)pyridin-2-yl)acetyl)piperidin-4-yl)-1H-benzo[d]imidazole-2(3H)-one (GEN1-028)

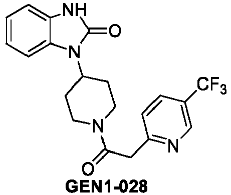

### Step 34.1. Synthesis of 1-(1-(2-(5-(trifluoromethyl)pyridin-2-yl)acetyl)piperidin-4-yl)-1H-benzo[d]imidazole-2(3H)-one (GEN1-028)

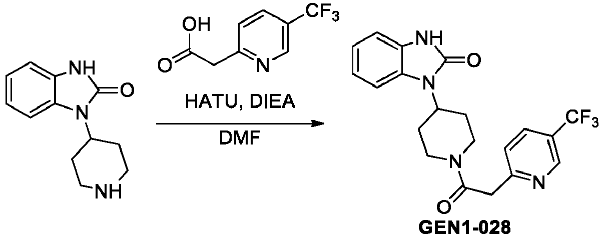

In DMF (1.5 mL) were added 1-(piperidin-4-yl)-1H-benzo[d]imidazole-2(3H)-one (60 mg, 0.28 mmol), 2-(5- (trifluoromethyl)pyridin-2-yl)acetic acid (64 mg, 0.31 mmol), and DIEA (109 mg, 0.844 mmol), and HATU (160 mg, 0.421 mmol) was added at room temperature. The resulting mixture was stirred at room temperature for 1 hour. The reaction mixture was directly purified by a C18 column (CH₃CN: water = 5% ∼ 45%) to obtain the desired compound (20.4 mg, yield 18%) as a white solid. ¹H NMR (400 MHz, CDCl₃): δ 9.46 (s, 1H), 8.83 (s, 1H), 7.95 (dd, *J* = 8.0, 2.0 Hz, 1H), 7.57 (d, *J* = 8.4 Hz, 1H), 7.11-6.99 (m, 4H), 4.89-4.86 (m, 1H), 4.62-4.53 (m, 1H), 4.34-4.30 (m, 1H), 4.14 (d, *J* = 14.4 Hz, 1H), 4.04 (d, *J* = 14.4 Hz, 1H), 3.29-3.22 (m, 1H), 2.78-2.71 (m, 1H), 2.37-2.25 (m, 2H), 1.92-1.89 (m, 2H). MS (ESI) Rt = 3.048 min, *m*/*z* 405.2 [M+H]⁺, purity: 98.20% @ 254 nm, 99.61% @ 214 nm.

### Example 35. 1-(1-(2-(3-(trifluoromethyl)phenyl)acetyl)piperidin-4-yl)-1H-benzo[d]imidazole-2(3H)-one (GEN1-029)

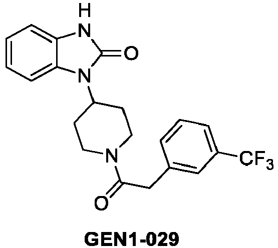

### Step 35.1. Synthesis of 1-(1-(2-(3-(trifluoromethyl)phenyl)acetyl)piperidin-4-yl)-1H-benzo[d]imidazole-2(3H)-one (GEN1-029)

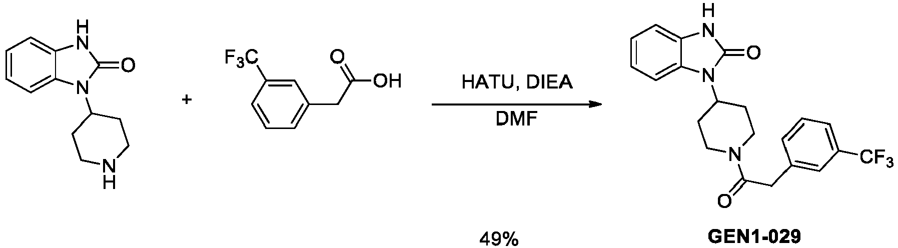

In DMF (3 mL) were added 1-(piperidin-4-yl)-1H-benzo[d]imidazole-2(3H)-one (106 mg, 0.490 mmol), 2-(3-(trifluoromethyl)phenyl)acetic acid (100 mg, 0.490 mmol) and HATU (280 mg, 0.735 mmol), DIEA (190 mg, 1.47 mmol). The reaction mixture was stirred at room temperature for 12 hours. The mixture was directly purified with a C18 column (CH₃CN: water = 5% ∼ 50%) to obtain the desired compound (100 mg, yield 49%) as a white solid. ¹H NMR (400 MHz, CDCl₃): δ 8.58 (s, 1H), 7.59-7.48 (m, 4H), 7.07-7.01 (m, 3H), 6.90-6.88 (m, 1H), 4.90 (d, *J* = 13.2 Hz, 1H), 4.57-4.50 (m, 1H), 4.05 (d, *J* = 14.8 Hz, 1H), 3.87 (s, 2H), 3.24-3.18 (m, 1H), 2.76-2.70 (m, 1H), 2.30-2.26 (m, 1H), 2.05-2.01 (m, 1H), 1.90 (d, *J* = 11.6 Hz, 1H), 1.81 (d, *J* = 11.6 Hz, 1H). MS (ESI): Rt = 3.573 min, *m*/*z* 404.2 [M+H]⁺, purity: 97.12% @ 254 nm, 99.27% @ 214 nm.

### Example 36. 1-(1-(2-(2-(trifluoromethyl)phenyl)acetyl)piperidin-4-yl)-1H-benzo[d]imidazole-2(3H)-one (GEN1-030)

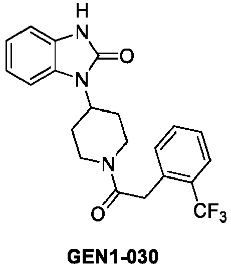

### Step 36.1. Synthesis of 1-(1-(2-(2-(trifluoromethyl)phenyl)acetyl)piperidin-4-yl)-1H-benzo[d]imidazole-2(3H)-one (GEN1-030)

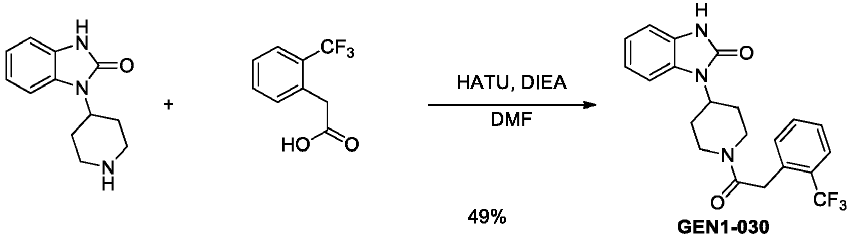

In DMF (3 mL) were added 1-(piperidin-4-yl)-1H-benzo[d]imidazole-2(3H)-one (106 mg, 0.490 mmol), 2-(2-(trifluoromethyl)phenyl)acetic acid (100 mg, 0.490 mmol), HATU (280 mg, 0.735 mmol), and DIEA (190 mg, 1.47 mmol). The reaction mixture was stirred at room temperature for 12 hours. The mixture was purified with a C18 column (CH₃CN: water = 5% ∼ 50%) to obtain the desired compound (100 mg, yield 49%) as a white solid. ¹H NMR (400 MHz, CDCl₃): δ 8.91 (s, 1H), 7.67 (d, *J* = 7.2 Hz, 1H), 7.60-7.56 (m, 1H), 7.46 (d, *J* = 8.0 Hz, 1H), 7.42-7.38 (m, 1H), 7.09-7.05 (m, 4H), 4.91 (d, *J* = 12.8 Hz, 1H), 4.59-4.53 (m, 1H), 4.06-4.02 (m, 2H), 3.92-3.88 (m, 1H), 3.22 (t, *J* = 11.6 Hz, 1H), 2.75 (t, *J* = 11.6 Hz, 1H), 2.37-2.22 (m, 2H), 1.93-1.85 (m, 2 H). MS (ESI): Rt = 3.546 min purity, *m*/*z* 404.2 [M+H]⁺, 93.25% @ 254 nm, 99.04% @ 214 nm.

### Example 37. 1-(1-(2-(4-(tert-butyl)phenyl)acetyl)piperidin-4-yl)-1H-benzo[d]imidazole-2(3H)-one (GEN1-031)

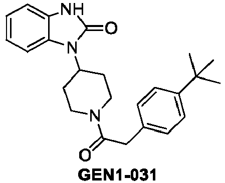

### Step 37.1. Synthesis of 1-(1-(2-(4-(tert-butyl)phenyl)acetyl)piperidin-4-yl)-1H-benzo[d]imidazole-2(3H)-one (GEN1-031)

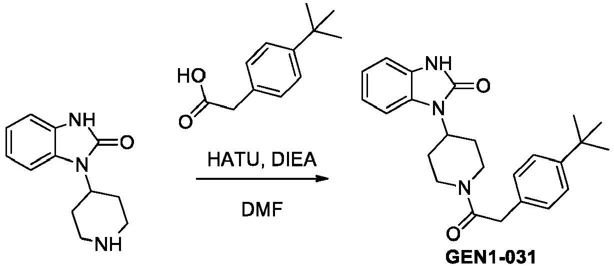

In DMF (2 mL) were added 1-(piperidin-4-yl)-1H-benzo[d]imidazole-2(3H)-one (60 mg, 0.28 mmol), 2-(4-(tert-butyl)phenyl)acetic acid (54 mg, 0.28 mmol), and DIEA (109 mg, 0.844 mmol), and HATU (160 mg, 0.421 mmol) was added at room temperature. The resulting mixture was stirred at room temperature for 1.5 hours. The reaction mixture was directly purified by a C18 column (CH₃CN: water = 5% ∼ 45%) to obtain the desired compound (24 mg, yield 22%) as a white solid. ¹H NMR (400 MHz, CDCl₃): δ 8.95 (s, 1H), 7.40-7.38 (m, 2H), 7.27-7.25 (m, 2H), 7.08-6.99 (m, 3H), 6.90-6.88 (m, 1H), 4.91-4.88 (m, 1H), 4.57-4.48 (m, 1H), 4.09-4.06 (m, 1H), 3.79 (s, 2H), 3.18-3.11 (m, 1H), 2.73-2.65 (m, 1H), 2.30-2.19 (m, 1H), 1.94-1.84 (m, 2H), 1.73-1.69 (m, 1H), 1.29 (s, 9H). MS (ESI) Rt = 2.519 min, *m*/*z* 392.2 [M+H]⁺, purity: 71.40% @ 254 nm, 97.55% @ 214 nm.

### Example 38. 1-(1-(2-(4-cyclopropylphenyl)acetyl)piperidin-4-yl)-1H-benzo[d]imidazole-2(3H)-one (GEN1-032)

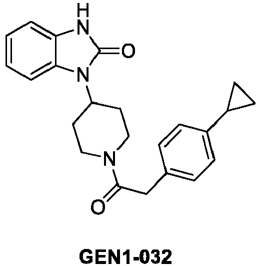

### Step 38.1. Synthesis of 1-(1-(2-(4-cyclopropylphenyl)acetyl)piperidin-4-yl)-1H-benzo[d]imidazole-2(3H)-one (GEN1-032)

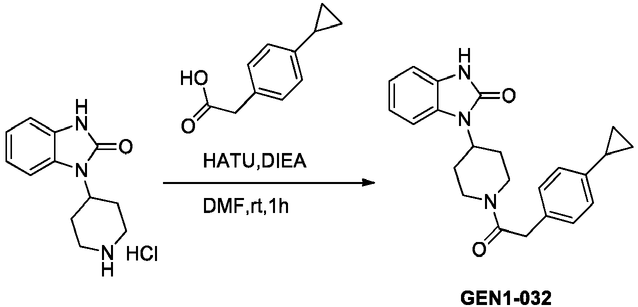

1-(piperidin-4-yl)-1H-benzo[d]imidazole-2(3H)-one hydrochloride (100 mg, 0.390 mmol) and DIEA (152 mg, 1.18 mmol) in DMF (1 mL) were stirred at room temperature for 20 minutes. The solution was used directly in the next step. To a solution of 2-(4-cyclopropylphenyl)acetic acid (76 mg, 0.43 mmol) and DIEA (102 mg, 0.79 mmol) in DMF (2 mL) was added HATU (224 mg, 0.59 mmol). After stirring for 20 minutes at room temperature, the previous solution was added. The mixture was stirred at room temperature for 1 hour. Then the mixture was poured into 15 ml of water and extracted with ethyl acetate (2×20 ml). The combined organic layer was washed with brine (20 mL), dried over Na₂SO₄ and concentrated. The residue was purified using high performance liquid chromatography(Xbridge C18, 5 um, 19 x 150 mm, 30-60% B, A: H₂O (0.1% NH₄HCO₃), B: CH₃CN, uv = 214 nm, F = 15 mL/min) to obtain the desired compound (50 mg, yield 34%) as a white solid. ¹H NMR (400 MHz, CDCl₃): δ 8.58 (s, 1H), 7.23-7.21 (m, 2H), 7.08-7.00 (m, 5H), 6.83-6.81 (m, 1H), 4.88 (d, *J=* 13.6 Hz, 1H), 4.58-4.48 (m, 1H), 4.05 (d, *J=* 13.6 Hz, 1H), 3.77 (s, 2H), 3.13 (t, *J=* 13.2 Hz, 1H), 2.67 (t, *J=* 13.2 Hz, 1H), 2.25-2.14 (m, 1H), 1.90-1.82 (m, 3H), 1.70-1.67 (m, 1H), 0.95-0.92 (m, 2H), 0.66-0.64 (m, 2H). MS (ESI) Rt = 3.548 min, *m*/*z* 376.2 [M+H]⁺, purity: 99.1% @ 254 nm, 99.6% @ 214 nm.

### Example 39. 1-(1-(2-([1,1'-biphenyl]-4-yl)acetyl)piperidin-4-yl)-1H-benzo[d]imidazole-2(3H)-one (GEN1-033)

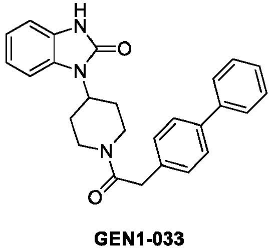

### Step 39.1. Synthesis of 1-(1-(2-([1,1'-biphenyl]-4-yl)acetyl)piperidin-4-yl)-1H-benzo[d]imidazole-2(3H)-one (GEN1-033)

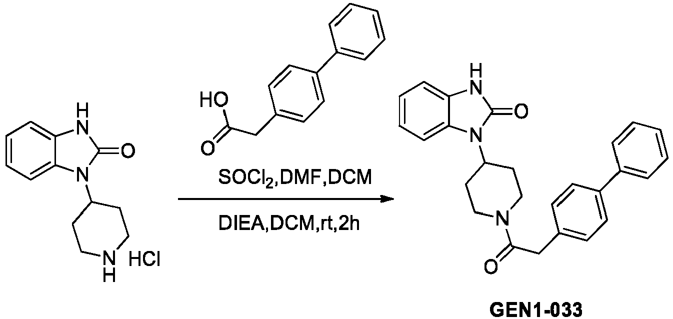

In a solution of 2-([1,1'-biphenyl]-4-yl)acetic acid (73 mg, 0.35 mmol) in DCM (2 mL) was added DMF (1 drop). SOCl₂ (75 mg, 0.63 mmol) was added. After stirring for 20 minutes at 40°C, the mixture was concentrated. The residue was dissolved in DCM (1 mL) and used in the next step. 1-(piperidin-4-yl)-1H-benzo[d]imidazole-2(3H)-one hydrochloride (80 mg, 0.31 mmol) and DIEA (203 mg, 1.57 mmol) in DCM (3ml) were stirred at room temperature for 10 min, and the previous solution was added dropwise. The resulting mixture was stirred for 2 hours. The mixture was poured into water (20 mL) and extracted with DCM (2 x 20 mL). The combined organic layer was washed with brine (20 mL), dried over Na₂SO₄ and concentrated. The residue was purified using high performance liquid chromatography (Xbridge C18, 5 um, 19 x 150 mm, 35-65% B, A: H₂O (0.1% NH₄HCO₃), B: CH₃CN, uv = 214 nm, F = 15 mL/min) to obtain the desired compound (50 mg, yield 39%) as a white solid. ¹H NMR (400 MHz, CDCl₃): δ 9.39 (s, 1H), 7.61-7.59 (m, 2H), 7.57-7.55 (m, 2H), 7.44-7.40 (m, 4H), 7.35-7.32 (m, 1H), 7.07-7.05 (m, 1H), 7.00 (t, *J* = 7.6 Hz, 1H), 6.95-6.91 (m ,1H), 6.88-6.86 (m, 1H), 4.92 (d, *J* = 13.6 Hz, 1H), 4.56-4.50 (m, 1H), 4.11 (d, *J* = 13.2 Hz, 1H), 3.87 (s, 2H), 3.19 (t, *J* = 12.4 Hz, 1H), 2.72 (t, *J* = 12.8 Hz, 1H), 2.31-2.21 (m, 1H), 2.03-1.94 (m, 1H), 1.89-1.86 (m, 1H), 1.77-1.74 (m, 1H). MS (ESI) Rt = 3.475 min, *m*/*z* 412.2 [M+H]⁺, purity: 98.1% @ 254 nm, 98.7% @ 214 nm.

### Example 40. 1-(1-(2-(4-morpholinophenyl)acetyl)piperidin-4-yl)-1H-benzo[d]imidazole-2(3H)-one (GEN1-034)

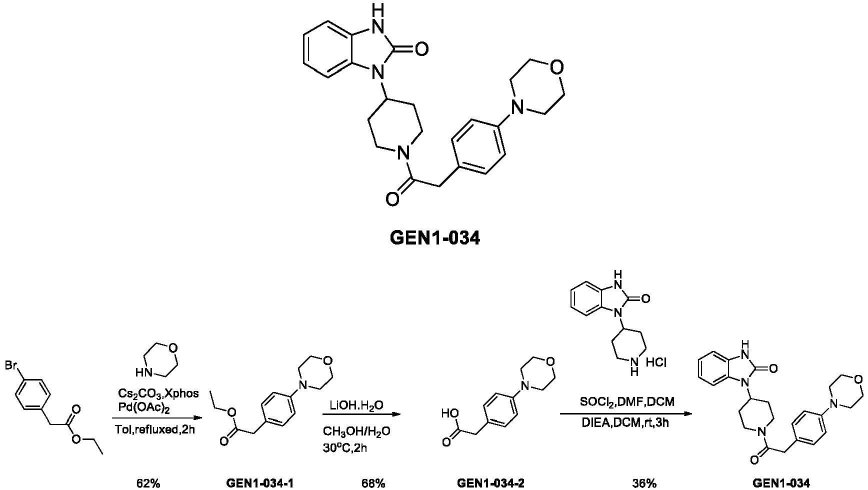

### Step 40.1. Synthesis of ethyl 2-(4-morpholinophenyl)acetate (GEN1-034-1)

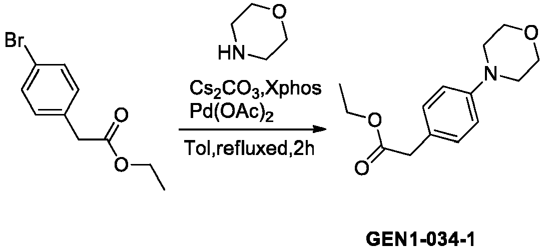

In a mixture of 2-(4-bromophenyl) ethyl acetate (1.00 g, 4.12 mmol), morpholine (430 mg, 4.94 mmol), Cs₂CO₃ (2.68 g, 8.23 mmol) and Xphos (196 mg, 0.41 mmol) in toluene (15ml) was added Pd(OAc)₂ (92 mg, 0.41 mmol). The mixture was refluxed for 2 hours under a nitrogen atmosphere, cooled and filtered. The filtrate was concentrated. The residue was purified by silica gel column chromatography (PE: EA = 10:1 ∼ 5: 1) to obtain the desired compound (630 mg, yield 62%) as a yellow oil. ¹H NMR (400 MHz, CDCl₃): δ 7.19 (d, *J* = 8.4 Hz, 2H), 6.87 (d, *J* = 8.8 Hz, 2H), 4.13 (q, *J* = 7.2 Hz, 2H), 3.85 (t, *J* = 4.8 Hz, 4H), 3.53 (s, 2H), 3.14 (t, *J* = 4.8 Hz, 4H), 1.25 (t, *J* = 7.2 Hz, 3H). MS (ESI) Rt = 1.584 min, *m*/*z* 250.1 [M+H]⁺, purity: 69.9% @ 214 nm.

### Step 40.2. Synthesis of 2-(4-morpholinophenyl)acetic acid (GEN1-034-2)

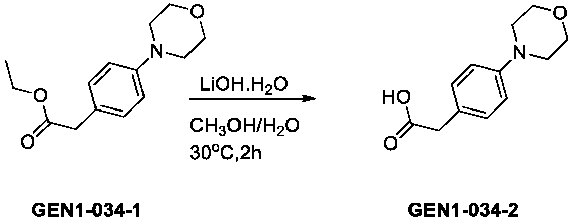

In a solution of ethyl 2-(4-morpholinophenyl)acetate (300 mg, 1.20 mmol) in methanol (4ml), a solution of lithium hydroxide hydrate (152 mg, 3.61 mmol) in water (2ml) was added. After stirring for 2 hours at 30°C, the mixture was concentrated. The residue was diluted with water (10 mL). 10% HCl aqueous solution was added dropwise until pH = 2-3. It was then extracted with DCM (3×20 mL). The combined organic layer was washed with brine (20 mL), dried over Na₂SO₄, and concentrated to the desired compound (180 mg, yield 68%) as a yellow solid. ¹H NMR (400 MHz, CDCl₃): δ 7.19 (d, *J* = 8.8 Hz, 2H), 6.88 (d, *J=* 8.8 Hz, 2H), 3.86 (t, *J =* 4.8 Hz, 4H), 3.57 (s, 2H), 3.14 (t, *J =* 4.8 Hz, 4H).

### Step 40.3. Synthesis of 1-(1-(2-(4-morpholinophenyl)acetyl)piperidin-4-yl)-1H-benzo[d]imidazole-2(3H)-one (GEN1-034)

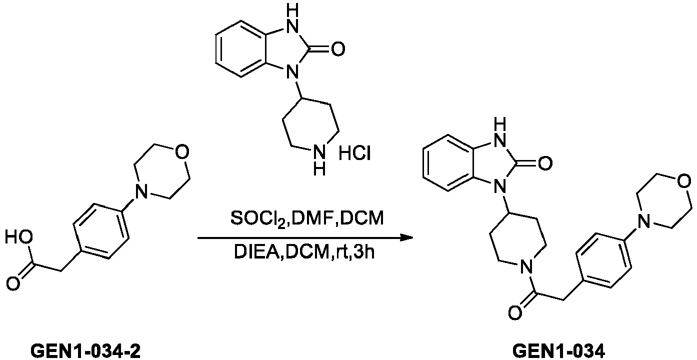

In a solution of 2-(4-morpholinophenyl)acetic acid (96 mg, 0.43 mmol) in DCM (2 mL) was added DMF (1 drop). SOCl₂ (94 mg, 0.79 mmol) was added. After stirring for 30 minutes at 25°C, the mixture was concentrated. The residue was dissolved in DCM (1 mL) and used in the next step. 1-(piperidin-4-yl)-1H-benzo[d]imidazole-2(3H)-one hydrochloride (100 mg, 0.390 mmol) and DIEA (254 mg, 1.97 mmol) in DCM (3ml) were stirred at room temperature for 30 minutes, and the previous solution was added dropwise. The resulting mixture was stirred at room temperature for 3 hours. The mixture was poured into water (20 mL) and extracted with DCM (2 x 20 mL). The combined organic layer was washed with brine (20 mL), dried over Na₂SO₄ and concentrated. The residue was purified using high performance liquid chromatography (Xbridge C18, 5 um, 19 x 150 mm, 25-40% B, A: H₂O (0.1% NH₄HCO₃), B: CH₃CN, uv = 214 nm, F = 15 mL/min) to obtain the desired compound (60 mg, yield 36%) as a white solid. ¹H NMR (400 MHz, CDCl₃): δ 9.27 (s, 1H), 7.24 (d, *J=* 8.4 Hz, 2H), 7.09-6.98 (m, 3H), 6.91 (d, *J* = 8.8 Hz, 2H), 6.86-6.84 (m, 1H), 4.88 (d, *J=* 13.6 Hz, 1H), 4.55-4.49 (m, 1H), 4.07 (d, *J=* 14.0 Hz, 1H), 3.84 (t, *J=* 4.8 Hz, 4 H), 3.75 (s, 2H), 3.17-3.11 (m, 2H), 2.71-2.65 (m, 1H), 2.28-2.18 (m, 1H), 1.94-1.84 (m, 2 H), 1.73-1.69 (m, 1H). MS (ESI) Rt = 2.726 min, *m*/*z* 421.2 [M+H]⁺, purity: 97.5% @ 254 nm, 99.2% @ 214 nm.

### Example 41. 1-(4-(2-amino-1H-benzo[d]imidazol-1-yl)piperidin-1-yl)-2-(4-(trifluoromethyl)phenyl)ethanone (GEN1-055)

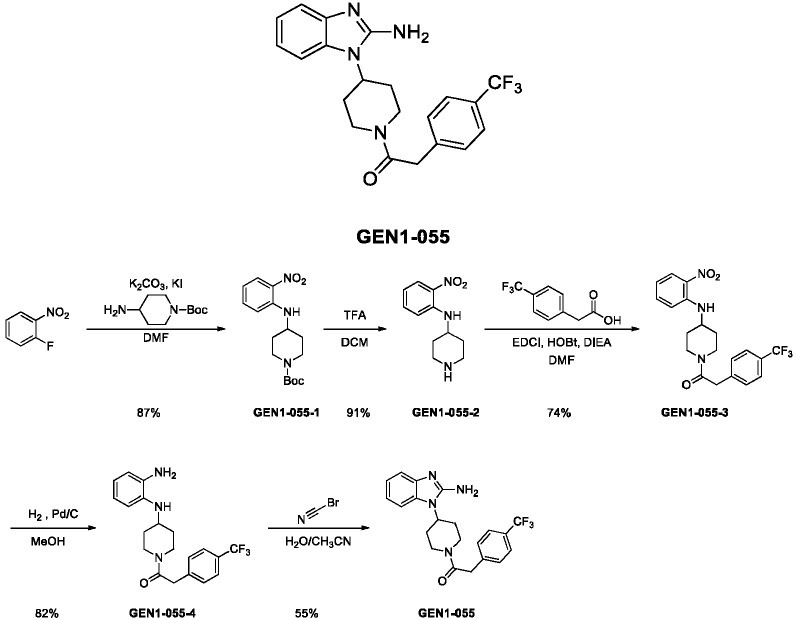

### Step 41.1. Synthesis of tert-butyl 4-((2-nitrophenyl)amino)piperidine-1-carboxylate (GEN1-055-1)

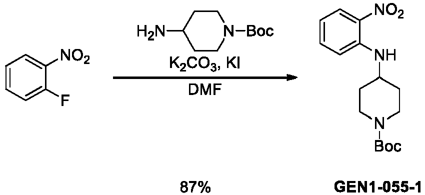

In a solution of 1-fluoro-2-nitrobenzene (2.0 g, 14 mmol), tert-butyl 4-aminopiperidine-1-carboxylate (2.8 g, 14 mmol), K₂CO₃ (3.0 g, 21 mmol) in DMF (30 mL) was added KI (50 mg, 0.30 mmol). The resulting mixture was stirred at 95°C for 12 hours. The mixture was cooled to room temperature, poured into water (50 mL), and extracted with EtOAc (30 mL×2). The combined organic layer was washed with brine, dried over Na₂SO₄, and concentrated to the desired yellow oil (4 g, yield 87%). MS (ESI): Rt = 1.75 min, *m*/*z* 266.1 [M+2H-*t*-Bu]⁺. purity: 93% @ 254 nm, 100% @ 214 nm.

### Step 41.2. Synthesis of N-(2-nitrophenyl)piperidin-4-amine (GEN1-055-2)

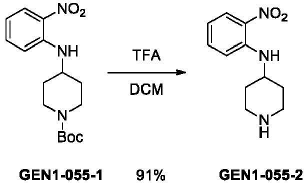

In a solution of tert-butyl 4-((2-nitrophenyl)amino)piperidine-1-carboxylate (4.0 g, 12 mmol) in DCM (30 mL) was added TFA (15 mL). The reaction mixture was stirred at room temperature for 12 hours. The mixture was concentrated and the residue was dissolved in water (50 mL). The aqueous solution was basified with aqueous Na₂CO₃ (saturated solution, 50 ml) to pH = 9, and extracted with DCM (50 mL×3). The combined organic layer was dried over Na₂SO₄ and concentrated to the desired yellow solid (2.5 g, yield 91%). ¹H NMR (400 MHz, CDCl₃): δ 8.18-8.12 (m, 2H), 7.41 (t, *J* = 8.4 Hz, 1H), 6.88 (d, *J* = 8.4 Hz, 1H), 6.62 (t, *J* = 8.4 Hz, 1H), 3.65-3.63 (m, 1H), 3.18-3.15 (m, 2H), 2.81 -2.75 (m, 2H), 2.17 (s, 1H), 2.11-2.08 (m, 2H), 1.58-1.51 (m, 2H).

### Step 41.3. Synthesis of 1-(4-((2-nitrophenyl)amino)piperidin-1-yl)-2-(4-(trifluoromethyl)phenyl)ethanone (GEN1- 055-3)

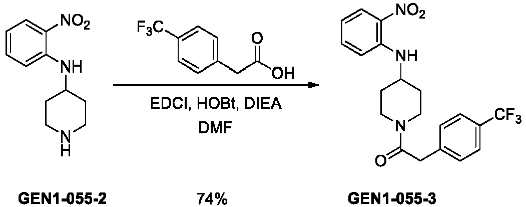

To a solution of N-(2-nitrophenyl)piperidin-4-amine (1.0 g, 4.5 mmol), 2-(4-(trifluoromethyl)phenyl)acetic acid (830 mg, 4.01 mmol), EDCI (1.1 g, 5.8 mmol), HOBT (790 mg, 5.85 mmol) in DMF (30 mL) was added DIEA (1.74 g, 13.5 mmol). The reaction mixture was stirred at room temperature for 12 hours. The mixture was purified with a C18 column (CH₃CN: water = 5% ∼ 50%) to obtain the desired product (1.2 g, yield 74%) as a yellow solid. MS (ESI): Rt = 1.60 min, *m*/*z* 408.3 [M + H]⁺ , purity: 97% @ 254 nm, 100% @ 214 nm.

### Step 41.4. Synthesis of 1-(4-((2-aminophenyl)amino)piperidin-1-yl)-2-(4-(trifluoromethyl)phenyl)ethanone (GEN1-055-4)

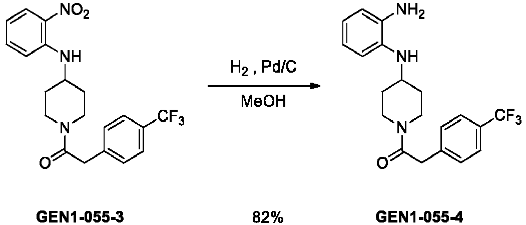

In a solution of 1-(4-((2-nitrophenyl)amino)piperidin-1-yl)-2-(4-(trifluoromethyl)phenyl)ethanone (1.2 g, 2.9 mmol) in MeOH (10 mL), Pd/C (10%, 100 mg) was added under H₂. The reaction mixture was stirred at room temperature for 12 hours. The mixture was filtered and the filtrate was concentrated to the desired product (900 mg, 82% yield) as a gray solid. MS (ESI): Rt = 1.62 min, *m*/*z* 378.3 [M + H]⁺ , purity: 91% @ 254 nm, 76% @ 214 nm.

### Step 41.5. Synthesis of 1-(4-(2-amino-1H-benzo[d]imidazol-1-yl)piperidin-1-yl)-2-(4-(trifluoromethyl)phenyl)ethanone (GEN1-055)

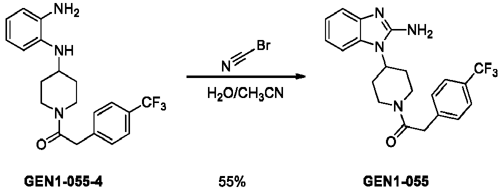

In a solution of 1-(4-((2-aminophenyl)amino)piperidin-1-yl)-2-(4-(trifluoromethyl)phenyl)ethanone (100 mg, 0.265 mmol) in methanol (1 mL) and water (0.1 mL), cyanogen bromide (140 mg, 1.32 mmol) was added. After the mixture was stirred at room temperature for two hours, it was concentrated and purified with a C18 column (CH₃CN: water = 5% ∼ 45%) to obtain the desired compound (60 mg, yield 55%) as a white solid. ¹H NMR (400 MHz,CDCl₃): δ 7.64 (d, J = 8.0 Hz, 2H), 7.46-7.42 (m, 3H), 7.15-7.11 (m, 1H), 7.03-7.02 (m, 2H), 4.95-4.92 (m, 1H), 4.54 (br s, 2H), 4.21-4.07 (m, 2H), 3.88 (s, 2H), 3.18 (t, *J =* 12.8 Hz, 1H), 2.70 (t, *J* = 13.6 Hz, 1H), 2.34-2.22 (m, 1H), 2.12-1.85 (m, 3H). MS (ESI): Rt = 3.46 min, *m*/*z* 403.2 [M+H]⁺, purity: 99.48% @ 254 nm, 98.24% @ 214 nm.

### Example 42: 6-(trifluoromethyl)-1-(1-(2-(4-(trifluoromethyl)phenyl)acetyl)piperidin-4-yl)-1H-benzo[d]imidazole-2(3H)-one (GEN1-009)

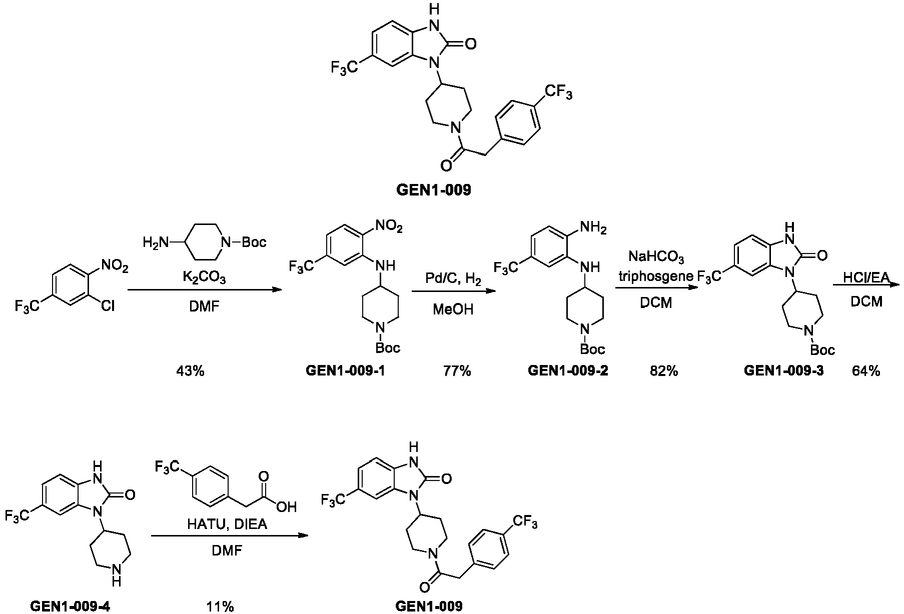

### Step 1. Synthesis of tert-butyl 4-((2-nitro-5-(trifluoromethyl)phenyl)amino)piperidine-1-carboxylate (GEN1-009-1)

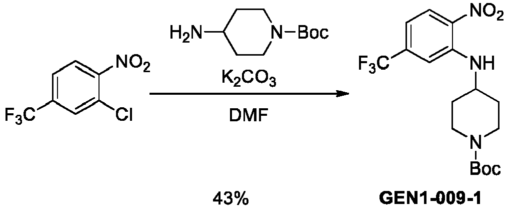

A mixture of 2-chloro-1-nitro-4-(trifluoromethyl)benzene (676 mg, 3.00 mmol), K₂CO₃ (828 mg, 5.99 mmol) and tert-butyl 4-aminopiperidine-1-carboxylate (630 mg, 3.15 mmol) in DMF (5 mL) was stirred at 90°C for 6 hours. After cooling to room temperature, the reaction mixture was poured into water (50 mL) and extracted with EtOAc (50 mL×2). The combined organic layer was washed with water (25 mL), brine (25 mL), dried over MgSO₄ and concentrated. The residue was purified by silica gel flash chromatography (petroleum: ethyl acetate = 10:1) to obtain the desired compound (512 mg, yield 43%) as a yellow oil. ¹HNMR (400 MHz, DMSO-*d₆*): δ 8.25 (d, *J* = 8.8 Hz, 1H), 7.95 (d, *J =* 8.0 Hz, 1H), 7.44 (s, 1H), 6.96 (dd, *J* = 8.8, 1.2 Hz, 1H), 4.06-3.96 (m, 1H), 3.91-3.88 (m, 2H), 3.32-3.09 (m, 2H), 1.93-1.90 (m, 2H), 1.53-1.44 (m, 2H), 1.41 (s, 9H).

### Step 2. Synthesis of tert-butyl 4-((2-amino-5-(trifluoromethyl)phenyl)amino)piperidine-1-carboxylate (GEN1-009-2)

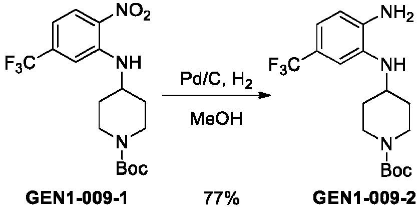

To a suspension of tert-butyl 4-((2-nitro-5-(trifluoromethyl)phenyl)amino)piperidine-1-carboxylate (512 mg, 1.31 mmol) in methanol (3 mL) was added Pd/C (10%, 50 mg) under N2 atmosphere. The resulting mixture was degassed with hydrogen and stirred under a hydrogen atmosphere at 20°C overnight. The reaction mixture was filtered, and the filtrate was concentrated to obtain the desired compound (418 mg, yield 77%) as a gray solid. MS (ESI): Rt = 1.677 min, *m*/*z* 304.1 [M+H]⁺; purity: 86.28% @ 254 nm.

### Step 3. Synthesis of tert-butyl 4-(2-oxo-6-(trifluoromethyl)-2,3-dihydro-1H-benzo[d]imidazol-1-yl)piperidine-1-carboxylate (GEN1-009-3)

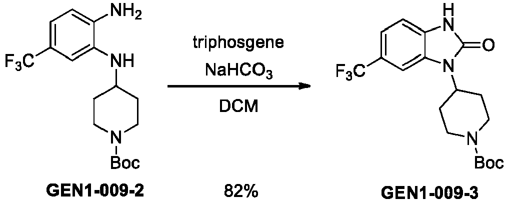

Triphosgene (210 mg, 0.708 mmol) was added to a solution of tert-butyl 4-((2-amino-5-(trifluoromethyl)phenyl)amino)piperidine-1-carboxylate (318 mg, 0.885 mmol) and NaHCO₃ (743 mg, 8.85 mmol) in DCM (10 mL) and stirred at 0°C for 2 hours. The reaction mixture was poured into water (50 mL), extracted with DCM (25 mL×3), and the combined organic layer was washed with brine (25 mL), dried on MgSO₄, and concentrated to obtain the desired compound (342 mg, yield 95%) as a red oil. MS (ESI): Rt = 1.450 min, *m*/*z* 330.2 [M+H]⁺; purity: 81.59% @ 254 nm.

### Step 4. Synthesis of 1-(piperidin-4-yl)-6-(trifluoromethyl)-1H-benzo[d]imidazole-2(3H)-one (GEN1-009-4)

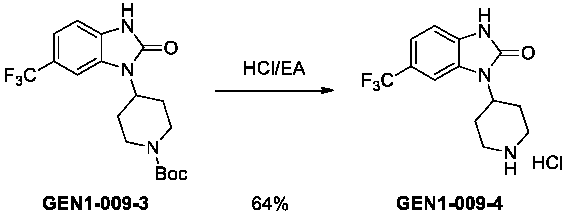

Tert-butyl 4-(2-oxo-6-(trifluoromethyl)-2,3-dihydro-1H-benzo[d]imidazol-1-yl)piperidine-1-carboxylate (342 mg, 0.887 mmol) was added to HCl/EA (6 M, 4ml) and stirred at room temperature for 2 hours. The reaction mixture was concentrated to the desired white solid (256 mg, yield 64%). MS (ESI): Rt = 1.287 min, *m*/*z* 286.1 [M+H]⁺; purity: 71.38% @ 254 nm.

### Step 5. Synthesis of 6-(trifluoromethyl)-1-(1-(2-(4-(trifluoromethyl)phenyl)acetyl)piperidin-4-yl)-1H-benzo[d]imidazole-2(3H)-one (GEN1-009)

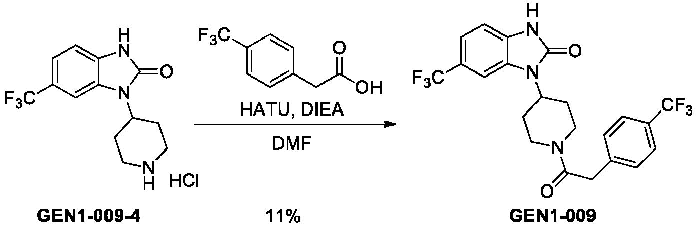

A solution of 2-(4-(trifluoromethyl)phenyl)acetic acid (146 mg, 0.717 mmol) and DIEA (253 mg, 1.96 mmol) was added to DMF (4ml), and HATU (298 mg, 0.782 mmol) was added separately at 0°C. After stirring for 10 min, 1-(piperidin-4-yl)-6-(trifluoromethyl)-1H-benzo[d]imidazole-2(3H)-one (186 mg, 0.652 mmol) was added . The mixture was stirred at room temperature for 4 hours. The reaction mixture was purified by preparative HPLC (Xbridge C185µm19 * 150 mm; B: 40-80%, A: H₂O (0.1% NH₄HCO₃), B: CH₃CN; UV: 214 nm, flow rate: 15 mL/min) to obtain the desired compound (34 mg, yield 11%) as a white solid. ¹H NMR (400 MHz, DMSO-*d₆*): δ 11.32 (s, 1H), 7.68 (d, *J* = 8.0 Hz, 2H), 7.51-7.49 (m, 3H), 7.34 (d, *J* = 8.4 Hz, 1H), 7.13 (d, *J=* 8.0 Hz, 1H), 4.59-4.47 (m, 2H), 4.4-4.11 (m, 1H), 3.92 (s, 2H), 3.21-3.15 (m, 1H), 2.75-2.68 (m, 1H), 2.22-2.13 (m, 2H), 1.75-1.73 (m, 2H). MS (ESI): Rt = 3.047 min, *m*/*z* 472.1 [M+H]⁺; purity: 89.46% @ 254 nm, 98.72% @ 214 nm.

### Example 43: 6-hydroxy-1-(1-(2-(4-(trifluoromethyl)phenyl)acetyl)piperidin-4-yl)-1H-benzo[d]imidazole-2(3H)-one (GEN1-012)

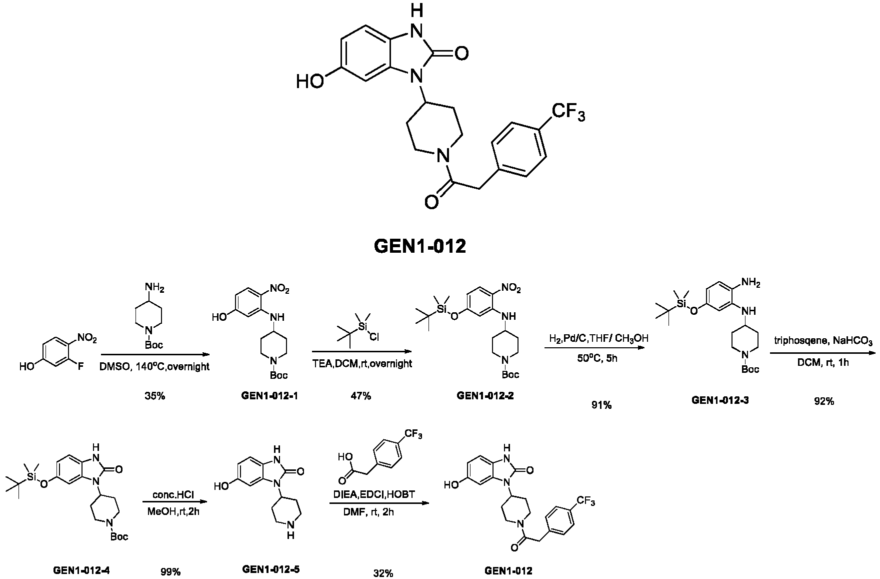

### Step 1. Synthesis of tert-butyl 4-((5-hydroxy-2-nitrophenyl)amino)piperidine-1-carboxylate (GEN1-012-1)

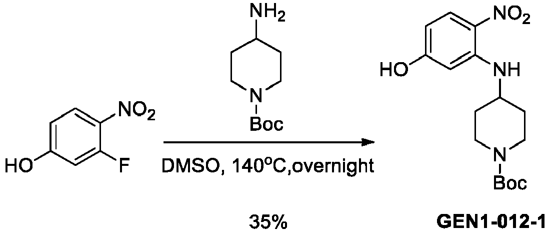

To a solution of the compound 3-fluoro-4-nitrophenol (1.0 g, 6.4 mmol) and tert-butyl 4-aminopiperidine-1-carboxylate (1.53 g, 7.64 mmol) in DMSO (20 mL) was added K₂CO₃ (2.20) g, 15.9 mmol). The mixture was stirred at 100°C overnight. TLC showed 80% of the starting material remaining. The mixture was stirred at 140°C overnight. TLC showed 40% starting material remaining. The reaction mixture was cooled to room temperature and diluted with water (100 mL). EtOAc (20 mL) was added and the mixture was stirred for 5 minutes. The organic layer was discarded, and the aqueous layer was acidified to pH=2 with 1M HCl. The aqueous layer was extracted with EtOAc (50 mL×2). The combined organic phase was washed with brine, dried over anhydrous Na₂SO₄ and concentrated to obtain a residue. The residue was purified by column chromatography on silica gel (petroleum: ethyl acetate = 20:1 to 10:1) to obtain the desired compound (800 mg, yield 35%) as a yellow solid. ¹H NMR (400 MHz, CDCl₃): δ 8.31 (d, *J* = 7.2Hz, 1H), 8.13 (d, *J* = 9.6Hz, 1H), 6.69 (s, 1H), 6.21-6.20 (m,1H), 6.19-6.16 (m, 1H), 4.02-3.99 (m, 2H), 3.61-3.55 (m, 1H), 3.06 (t, *J=* 10.4 Hz, 2H), 2.07-2.04 (m, 2H), 1.60-1.52 (m, 2H), 1.48 (s, 9H).

### Step 2. Synthesis of tert-butyl 4-((5-((tert-butyldimethylsilyl)oxy)-2-nitrophenyl)amino)piperidine-1-carboxylate (GEN1-012-2)

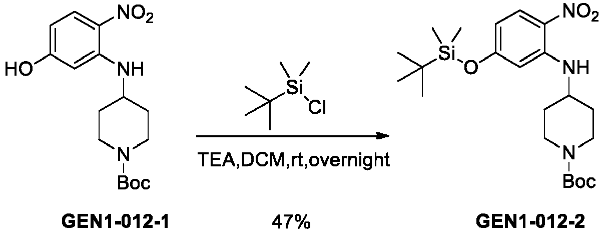

To a solution of tert-butyl 4-((5-hydroxy-2-nitrophenyl)amino)piperidine-1-carboxylate (800mg, 2.37mmol) and TEA (480mg, 4.74mmol) in DCM (10mL) was added a solution of tert-butylchlorochlorodimethylsilane (357 mg, 2.37 mmol) in DCM (2 mL) dropwise at room temperature. After stirring overnight, the reaction mixture was diluted with water (10 mL) and the phases were separated. The organic phase was washed with brine, dried over Na₂SO₄ and concentrated. The residue was purified by silica gel column chromatography (PE:EA = 10:1) to obtain the desired compound (500 mg, yield 47%) as a yellow solid. MS (ESI) Rt = 1.851 min, *m*/*z* 396.0 [M+H-56]⁺; purity: 89.2% @ 214 nm.

### Step 3. Synthesis of 4-((2-amino-5-((tert-butyldimethylsilyl)oxy)phenyl)amino)piperidine-1-carboxylate (GEN1-012-3)

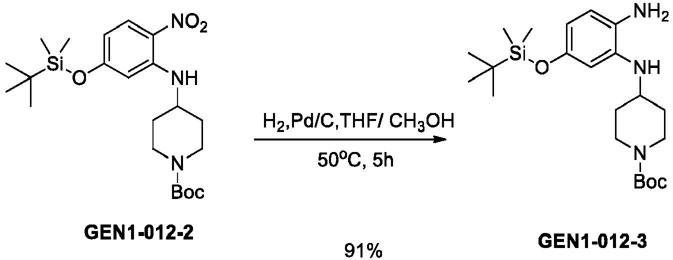

Tert-butyl 4-((5-((tert-butyldimethylsilyl)oxy)-2-nitrophenyl)amino)piperidine-1-carboxylate (500 mg, 1.11 mmol) was added to a solution of THF (3ml) and MeOH (3ml), and then Pd/C (10%, 150 mg) was added. The mixture was hydrogenated under a hydrogen atmosphere at 50°C for 5 hours. The mixture was filtered through celite and concentrated to obtain the desired compound (460 mg, yield 91%), which was used directly in the next step. MS (ESI) Rt = 1.916 min, *m*/*z* 422 [M+H]⁺; purity: 92% @ 214 nm.

### Step 4. Synthesis of tert-butyl 4-(6-(((tert-butyldimethylsilyl)oxy)-2-oxo-2,3-dihydro-1H-benzo[d]imidazole-1-ylpiperidine-1-carboxylate (GEN1-012-4)

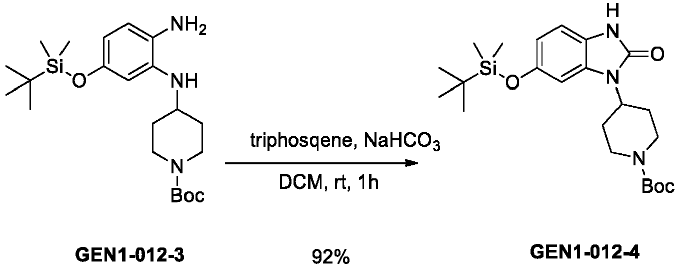

To a mixture of tert-butyl 4-[2-amino-5-[tert-butyl(dimethyl)silyl]oxy-anilino]piperidine-1-carboxylate (460 mg, 1.00 mmol) and NaHCO₃ (843.16 mg, 10.04 mmol) was added in portions of triphosgene (119 mg, 0.41 mmol) in DCM (50 mL) at room temperature. After stirring for 1 hour at room temperature, the reaction mixture was diluted with water (30 mL) and the phases were separated. The organic phase was washed with brine, dried over Na₂SO4 and concentrated. The residue was purified by column chromatography on silica gel (PE:EA = 5:1 to 1:1) to obtain the desired compound (450 mg, yield 92%) as a white solid. ¹H NMR (400 MHz, DMSO-*d₆*): δ 10.66 (s, 1H), 6.82 (d, *J* = 8.0 Hz, 1H), 6.60 (d, *J* = 1.6 Hz, 1H), 6.47 (dd, *J* = 8.0, 2.0 Hz, 1H), 4.38-4.29 (m, 1H), 4.08-4.06 (m, 2H), 2.92-2.84 (m, 2H), 2.16-2.06 (m, 2H), 1.68-1.65 (m, 2H), 1.44 (s, 9H), 0.95 (s, 9H), 0.16 (s, 6H). MS (ESI), Rt = 1.703 min, *m*/*z* 392.3 [M+H-56]⁺; purity: 91.6% @ 214 nm.

### Step 5. Synthesis of 6-hydroxy-1-(piperidin-4-yl)-1H-benzo[d]imidazole-2(3H)-one (GEN1-012-5)

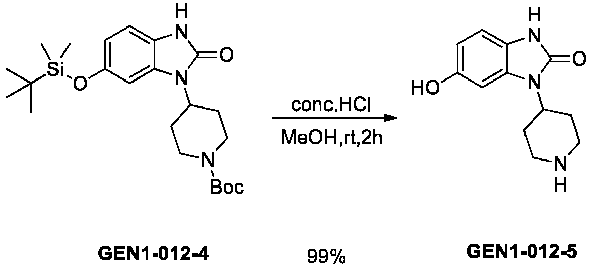

Tert-butyl 4-(6-(((tert-butyldimethylsilyl)oxy)-2-oxo-2,3-dihydro-1H-benzo[d]imidazol-1-ylpiperidine-1-carboxylate (200 mg, 0.41 mmol) was added to a mixed solution of MeOH (3ml) and hydrochloric acid (1ml). The mixture was stirred at room temperature for 2 hours. To the mixture was added a 5% aqueous NaOH solution, pH = 7-8. The mixture was concentrated and dried to obtain the desired compound (140 mg, yield 99%). MS (ESI) Rt = 0.50 min, *m*/*z* 234.1 [M+H]⁺; purity: 90% @ 214 nm, 85% @ 254 nm.

### Step 6. Synthesis of 6-hydroxy-1-(1-(2-(4-(trifluoromethyl)phenyl)acetyl)piperidin-4-yl)-1H-benzo[d]imidazole-2(3H)-one (GEN1-012)

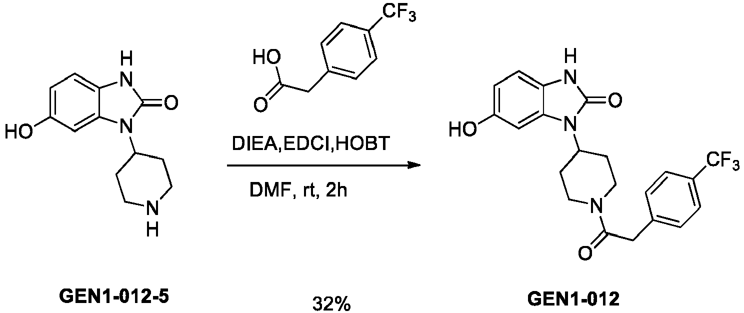

In a solution of 6-hydroxy-1-(piperidin-4-yl)-1H-benzo[d]imidazole-2(3H)-one (98 mg, 0.41 mmol), 2-(4 -(trifluoromethyl)phenyl)acetic acid (92 mg, 0.45 mmol) and DIEA (264 mg, 2.04 mmol) were dissolved in DMF (5 mL), and to this mixture was added HOBT (83 mg, 0.61 mmol) and EDCI (117 mg, 0.61 mmol) and then the mixture was stirred at room temperature for 2 hours. The reaction mixture was diluted with water (10 mL) and extracted with DCM (5 mL×3). The combined organic phase was dried with anhydrous Na₂SO₄ and concentrated. The residue was purified by preparative HPLC [gilson-2, Xbridge C18, 5um, 19 x 150 mm, 30∼60% B, A: H₂O (0.1% TFA), B: ACN, F = 15 mL/min, uv purification = 214nm] to obtain the desired compound (55mg, yield 32%) as a white solid. ¹H NMR (400 MHz, CDCl₃): δ 8.65 (s, 1H), 7.63 (d, *J* = 8.0 Hz, 2H), 7.45 (d, *J* = 7.6 Hz, 2H), 6.84 (d, *J* = 8.4 Hz, 1H), 6.58 (d, *J* = 1.6 Hz, 1H), 6.55 (d, *J* = 2.0 Hz, 1H), 6.31 (br s, 1H), 4.85 (d, *J* = 12.4 Hz, 1H), 4.55-4.47 (m, 1H), 4.02 (d, *J* = 12.4 Hz, 1H), 3.89 (s, 2H), 3.20 (t, *J* = 12.0 Hz, 1H), 2.71 (t, *J* = 12.0 Hz, 1H), 2.28-2.17 (m, 1H), 2.01-1.92 (m, 1H), 1.78 - 1.74 (m, 2H). MS (ESI) Rt = 3.202 min, *m*/*z* 420.2 [M+H]⁺; purity: 99.5% @ 254 nm, 99.9% @ 214 nm.

### Example 44: 7-hydroxy-1-(1-(2-(4-(trifluoromethyl)phenyl)acetyl)piperidin-4-yl)-1H-benzo[d]imidazole-2(3H)-one (GEN1-016)

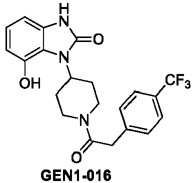

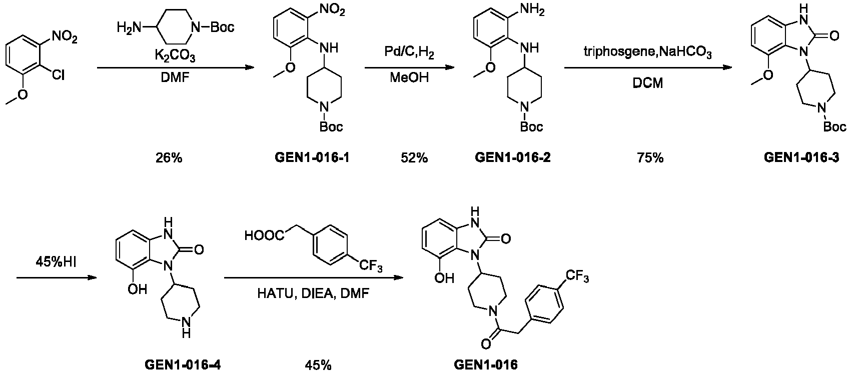

### Step 1. Synthesis of tert-butyl 4-((2-methoxy-6-nitrophenyl)amino)piperidine-1-carboxylate (GEN1-016-1)

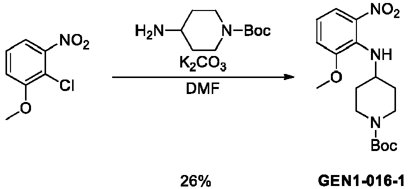

To a solution of 2-chloro-1-methoxy-3-nitrobenzene (3.0 g, 16 mmol) and tert-butyl 4-aminopiperidine-1-carboxylate (6.41 g, 32.0 mmol) in DMF (30 mL) was added K₂CO₃ (4.42 g, 32.0 mmol), and then the mixture was stirred at 120°C for 24 hours. After cooling to room temperature, the resulting mixture was poured into water (300 mL) and extracted with EtOAc (150 mL×3).The combined organic layer was washed with brine (150 mL), dried over anhydrous Na₂SO₄, and purified by silica gel column (PE:EA = 10:1) to obtain the desired compound (1.56 g, yield 26%) as a red oil. ¹HNMR (400 MHz, CDCl₃): δ 7.74 (dd, *J* = 8.8, 1.2 Hz, 1H), 7.50 (t, *J* = 8.0, 1H), 6.95 (dd, *J* = 8.0, 1.2 Hz, 1H), 6.71 (t, *J* = 8.4 Hz, 1H), 4.15-4.06 (m, 1H), 4.02-3.91 (m, 2H), 3.87 (s, 3H), 2.93 (t, *J* = 11.6 Hz, 2H), 1.96-1.92 (m, 2H), 1.46 (s, 9H), 1.41-1.37 (m, 2H). MS (ESI) Rt = 1.77 min, *m*/*z* 296.2 [M+1-56]⁺; purity: 100% @ 254 nm, 94% @ 214 nm.

### Step 2. Synthesis of tert-butyl 4-((2-amino-6-methoxyphenyl)amino)piperidine-1-carboxylate (GEN1-016-2)

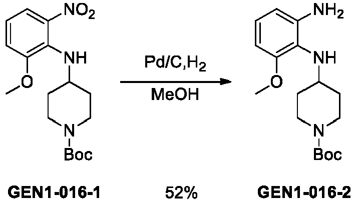

To a solution of tert-butyl 4-(2-methoxy-6-nitro-anilino)piperidine-1-carboxylate (1.06 g, 3.02 mmol) in MeOH (20 mL) was added Pd/ C (321 mg, 0.302 mmol, 10% purity). The mixture was stirred at room temperature for 1 hour under a hydrogen atmosphere. The resulting mixture was filtered, and the filtrate was concentrated to obtain the desired compound (533 mg, yield 52%) as a yellow oil. MS (ESI): Rt = 1.12 min, *m*/*z* 322.3 [M+H]⁺; purity: 91% @ 254 nm, 94% @ 214 nm.

### Step 3. Synthesis of tert-butyl 4-(7-methoxy-2-oxo-2,3-dihydro-1H-benzo[d]imidazol-1-yl)piperidine-1-carboxylate (GEN1-016-3)

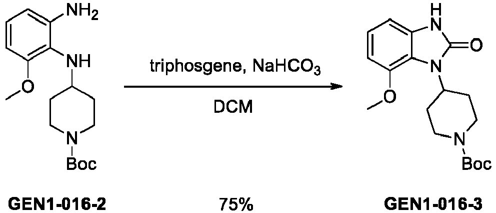

NaHCO₃ (1.39g, 16.6mmol) was added to a solution of tert-butyl 4-(2-amino-6-methoxy-anilino)piperidine-1-carboxylate (533mg, 1.66mmol) in DCM (50mL) at 0°C. Then triphosgene (246 mg, 0.829 mmol) was added in portions. After stirring for 2 hours at 0°C, the resulting mixture was concentrated and diluted with water (20 mL). Then EtOAc (50 mL×3) was added to extract the desired compound. The combined organic layer was washed with brine (50 mL), dried over anhydrous Na₂SO₄ and purified by a C18 column (50-70% B; A: H₂O, B: CH₃CN) to obtain the desired product (530 mg, yield 75%) as a white solid. MS (ESI): Rt = 1.52 min, *m*/*z* 348.3 [M+H]⁺; purity: 82% @ 254 nm, 100% @ 214 nm.

### Step 4. Synthesis of 7-hydroxy-1-(piperidin-4-yl)-1H-benzo[d]imidazole-2(3H)-one (GEN1-016-4)

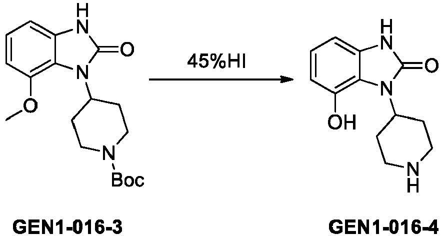

Tert-butyl 4-(7-methoxy-2-oxo-2,3-dihydro-1H-benzo[d]imidazol-1-yl)piperidine-1-carboxylate (200 mg , 0.576 mmol) was dissolved in HI (45% dissolved in water, 5ml), and stirred at 130°C for 3 hours. The mixture was cooled to room temperature and DIEA (5 mL) was added. The mixture was concentrated to obtain the compound (3.06 g crude product) as a white solid. MS (ESI): Rt = 0.48 min, *m*/*z* 234.1 [M+H]⁺; purity: 100% @ 254 nm, 100% @ 214 nm.

### Step 5. Synthesis of 7-hydroxy-1-(1-(2-(4-(trifluoromethyl)phenyl)acetyl)piperidin-4-yl)-1H-benzo[d]imidazole-2(3H)-one (GEN1-016)

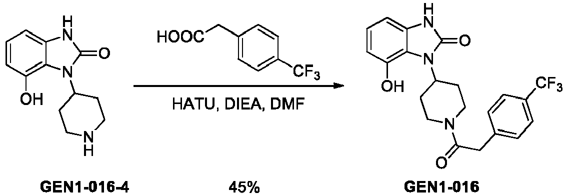

7-hydroxy-1-(piperidin-4-yl)-1H-benzo[d]imidazole-2(3H)-one (135 mg, crude product), 2-[4-(trifluoromethyl) )phenyl]acetic acid (177 mg, 0.867 mmol), HATU (440 mg, 1.16 mmol) and DIEA (224 mg, 1.73 mmol) were dissolved in DMF (6 mL) and stirred at room temperature for 16 hours. The resulting mixture was purified by preparative HPLC (RP-PREP-1 Xbridge C18 5um 19 * 150mm, 20%-65% B; A: H₂O (0.1% NH₄HCO₃), B: CH₃CN; UV: 254nm, flow rate: 15mL / min) to obtain the desired product (110 Mg, yield 45% after two steps), as a white solid. ¹HNMR (400 MHz, DMSO-*d*₆): δ 10.75 (s, 1H), 9.87 (s, 1H), 7.69 (d, *J* = 8.0 Hz, 2H), 7.47 (d, *J* = 8.0 Hz, 2H), 6.79 (t, *J* = 8.0 Hz, 1H), 6.51-6.45 (m, 2H), 4.80-4.66 (m, 1H), 4.55 (d, *J* = 13.6 Hz, 1H), 4.09 (d, *J* = 14.0 Hz, 1H), 3.93 (d, *J* = 15.6 Hz, 1H), 3.83 (d, *J* = 15.6 Hz, 1H), 3.12 (t, *J* = 12.4 Hz, 1H), 2.64 (t, *J* = 12.0 Hz, 1H), 2.47-2.35 (m, 2H), 1.67-1.64 (m, 2H). MS (ESI): Rt = 3.344 min, *m*/*z* 420.1 [M+H]⁺; purity: 94.81% @ 254 nm, 99.63% @ 214 nm.

### Example 45: N-((1s,3s)-3-(2-oxo-2,3-dihydro-1H-benzo[d]imidazol-1-yl)cyclobutyl)-2 -(4-(trifluoromethyl)phenyl)acetamide

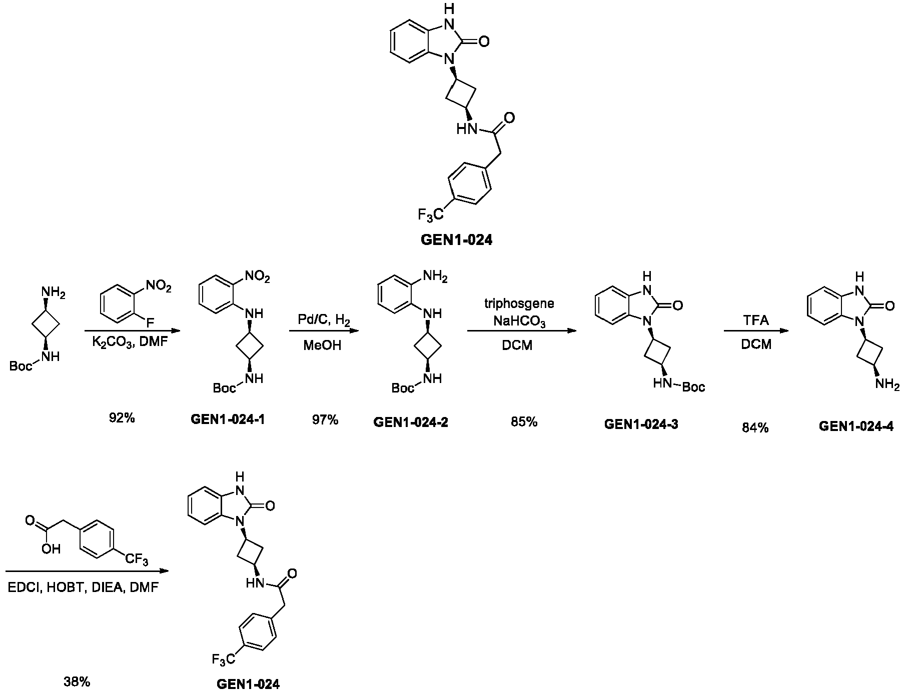

### Step 1. Synthesis of tert-butyl ((1s, 3s)-3-((2-nitrophenyl)amino)cyclobutyl)carbamate (GEN1-024-1)

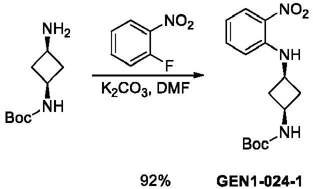

To a solution of tert-butyl ((1s, 3s)-3-aminocyclobutyl)carbamate (500 mg, 2.68 mmol) and 1-fluoro-2-nitrobenzene (455 mg, 3.22 mmol) in DMF (10 mL) was added K₂CO₃ (557mg, 4.03mmol). After stirring overnight at 80°C, the resulting mixture was cooled to room temperature and poured into water (25 mL). EtOAc (80 mL×4) was added to extract the desired compound. The combined organic layer was washed with brine (20 mL), dried over anhydrous Na₂SO₄ and filtered. The filtrate was concentrated, and the residue was purified by a C18 column (CH₃CN: water = 55-75%) to obtain the desired compound (758 mg, yield 92%) as a yellow solid. MS (ESI): Rt = 1.66 min, *m*/*z* 252.2 [M+H-56]⁺; purity: 100% @ 254 nm, 16% @ 214 nm.

### Step 2. Synthesis of tert-butyl ((1s,3s)-3-((2-aminophenyl)amino)cyclobutyl)carbamate (GEN1-024-2)

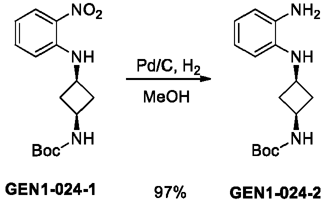

To a solution of tert-butyl ((1s, 3s)-3-(((2-nitrophenyl)amino)cyclobutyl)carbamate (758 mg, 2.47 mmol) in MeOH (30 mL) was added Pd/C (10%, 262 mg, 0.246 mmol). After stirring for 3 hours at room temperature under a hydrogen atmosphere, the resulting mixture was filtered. The filtrate was concentrated to obtain the desired compound (666 mg, yield 97%) as a brown solid. MS (ESI): Rt = 1.54 min, *m*/*z* 278.4 [M+H]⁺; purity: 100% @ 254 nm, 100% @ 214 nm.

### Step 3. Synthesis of tert-butyl ((1s,3s)-3-(2-oxo-2,3-dihydro-1H-benzo[d]imidazol-1-yl)cyclobutyl)carbamate (GEN1-024-3)

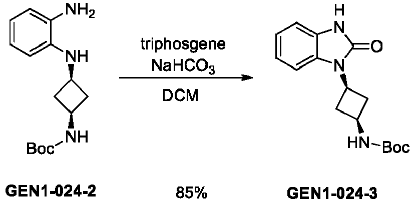

To a suspension of tert-butyl ((1s,3s)-3-(((2-aminophenyl)amino)cyclobutyl)carbamate and NaHCO₃ (2.02 g, 24.0 mmol) in DCM (30 mL) was added triphosgene (356 mg, 1.20 mmol). After stirring at 0°C for 1 hour, the resulting mixture was filtered. The filtrate was concentrated and the residue was purified by a C18 column (CH₃CN: water = 45-65%) to give the desired compound (619 mg, 85% yield) as a white solid. MS (ESI): Rt = 1.44 min, *m*/*z* 304.2 [M+H-100]⁺; purity: 100% @ 254 nm, 100% @ 214 nm.

### Step 4. Synthesis of 1-((1s,3s)-3-aminocyclobutyl)-1H-benzo[d]imidazole-2(3H)-one (GEN1-024-4)

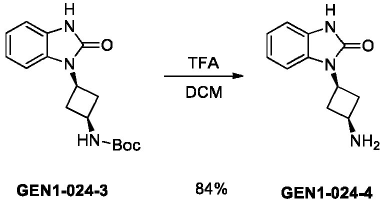

To tert-butyl ((1s, 3s)-3-(2-oxo-2,3-dihydro-1H-benzo[d]imidazol-1-yl)cyclobutyl)carbamate (250 mg, 824 umol) were added DCM (5mL) and TFA (1mL). The resulting mixture was stirred at room temperature for 2 hours. The resulting mixture was basified to pH=8-10 with aqueous Na₂CO₃. Then the mixture was separated, and the organic layer was concentrated. The residue was purified by a C18 column (CH₃CN: water = 20-40%) to obtain the desired compound (140 mg, yield 84%) as a pale yellow oil. MS (ESI): Rt = 0.70 min, *m*/*z* 204.2 [M+H]⁺; purity: 100% @ 254 nm, 100% @ 214 nm.

### Step 5. Synthesis of N-((1s,3s)-3-(2-oxo-2,3-dihydro-1H-benzo[d]imidazol-1-yl)cyclobutyl)-2-(4-(trifluoromethyl)phenyl)acetamide (GEN1-024)

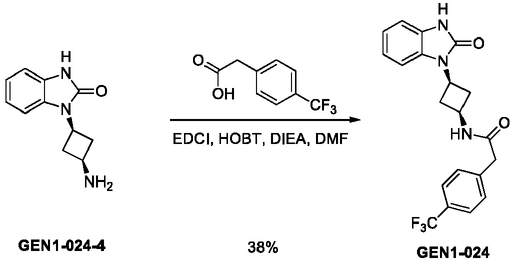

1-((1s,3s)-3-aminocyclobutyl)-1H-benzo[d]imidazole-2(3H)-one (140 mg, 0.689 mmol), EDCI (198 mg, 1.03 mmol)), HOBT (140 mg, 1.04 mmol) and DIEA (267 mg, 2.07 mmol) were dissolved in DMF (2 mL), and 2-[4-(trifluoromethyl)phenyl]acetic acid (155 mg, 0.759 mmol) was added and the mixture was stirred overnight at room temperature. The mixture was then purified by preparative HPLC (preparative 5 Xbridge C18 5um 19 * 150mm, 30-70% B; A: H₂O (0.1% NH₄HCO₃), B: CH₃CN; 214nm; flow rate: 15mL/min) to obtain the desired compound (104 mg, yield 38%)) as a white solid. ¹HNMR (400 MHz, DMSO-*d₆*): δ 10.86 (s, 1H), 8.64 (d, *J* = 7.6 Hz, 1H), 7.68 (d, *J* = 8.0 Hz, 2H), 7.50 (d, *J* = 8.0 Hz, 2H), 7.42-7.39 (m, 1H), 7.05-6.97 (m, 3H), 4.57-4.48 (m, 1H), 4.11-4.01 (m, 1H), 3.55 (s, 2H), 2.81-2.73 (m, 2H), 2.63-2.56 (m, 2H). MS (ESI): Rt = 3.486 min, *m*/*z* 390.1 [M+H]⁺; purity: 97.40% @ 254 nm, 98.82% @ 214 nm.

### Example 46: N-((1r,3r)-3-(2-oxo-2,3-dihydro-1H-benzo[d]imidazol-1-yl)cyclobutyl)-2 -(4-(trifluoromethyl)phenyl)acetamide

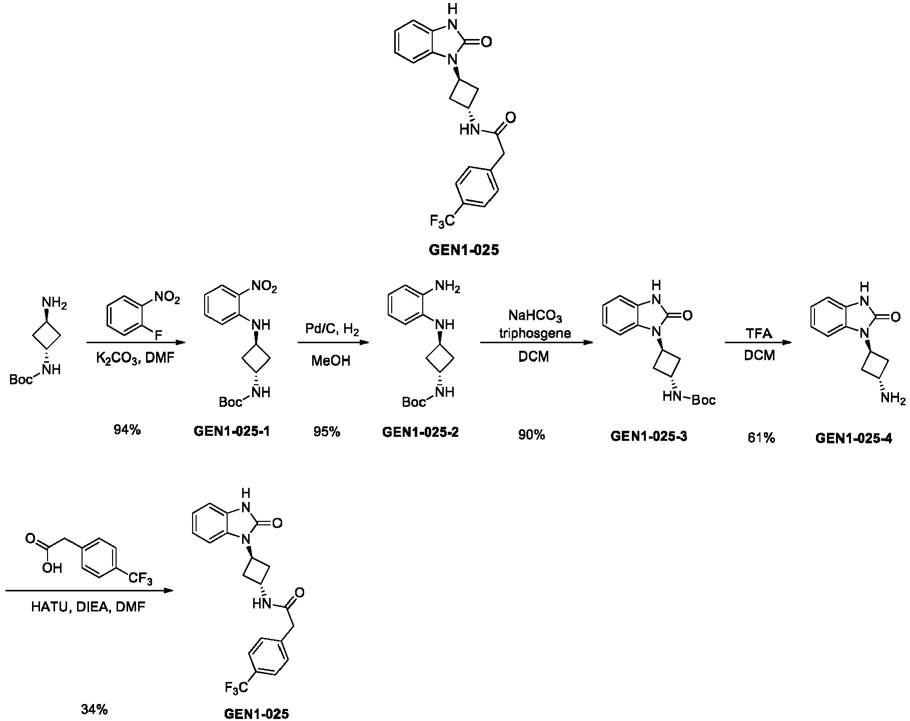

### Step 1. Synthesis of tert-butyl ((1r,3r)-3-(((2-nitrophenyl)amino)cyclobutyl)carbamate (GEN1-025-1)

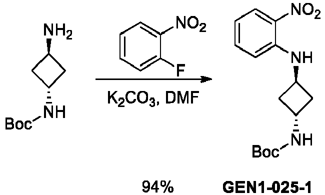

To a solution of 1-fluoro-2-nitrobenzene (273mg, 1.93mmol) and tert-butyl ((1r, 3r)-3-aminocyclobutyl)carbamate (300mg, 1.61mmol) in DMF (4mL) was added K 2 CO 3 (334 mg, 2.42 mmol). The mixture was stirred at 80°C for 16 hours. The mixture was filtered and the filtrate was purified by a C18 column (CH₃CN: water = 55-75%) to obtain the desired compound (466 mg, yield 94%) as a yellow solid. ¹HNMR (400 MHz, CDCl₃): δ 8.18 (dd, *J* = 8.8, 1.6 Hz, 1H), 8.11 (br s, 1H), 7.42 (t, *J* = 6.8 Hz, 1H), 6.70-6.66 (m, 1H), 6.60 (d, *J* = 8.0 Hz, 1H), 4.79 (br s, 1H), 4.33-4.31 (m, 1H), 4.16-4.09 (m, 1H), 2.45-2.40 (m, 4H), 1.46 (s, 9H).

### Step 2. Synthesis of tert-butyl ((1r,3r)-3-((2-aminophenyl)amino)cyclobutyl)carbamate (GEN1-025-2)

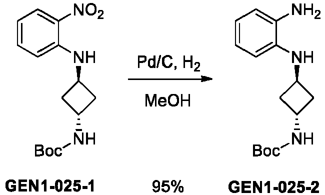

To a solution of tert-butyl ((1r,3r)-3-(((2-nitrophenyl)amino)cyclobutyl)carbamate (416 mg, 1.35 mmol) in MeOH (20 mL) was added Pd/C (10%, 144 mg, 0.135 mmol). The mixture was stirred at room temperature under a hydrogen atmosphere for 2 hours. The resulting mixture was filtered and the filtrate was concentrated. The residue was purified by a C18 column (CH₃CN: water = 50 -70%) to obtain the desired compound (357 mg, yield 95%) as a brown solid. MS (ESI): Rt = 1.51 min, *m*/*z* 222.2 [M+H-56]⁺; purity: 100% @ 254 nm, 100% @ 214 nm.

### Step 3. Synthesis of tert-butyl ((1r,3r)-3-(2-oxo-2,3-dihydro-1H-benzo[d]imidazol-1-yl)cyclobutyl)carbamate (GEN1-025-3)

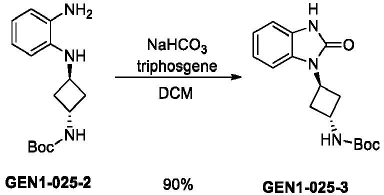

To a suspension of (tert-butyl)((1r,3r)-3-((2-aminophenyl)amino)cyclobutyl)carbamate (350 mg, 1.26 mmol) and NaHCO₃ (1.06 g, 12.6 mmol) in dichloromethane (25 mL) was added triphosgene (187 mg, 0.630 mmol). After stirring at 0°C for 1 hour, the resulting mixture was filtered. The filtrate was concentrated, and the residue was purified by a C18 column (CH₃CN: water = 50-70%) to obtain the desired compound (344 mg, yield 90%) as a gray solid. MS (ESI): Rt = 1.46 min, *m*/*z* 248.2 [M+H-56]⁺; purity: 100% @ 254 nm, 100% @ 214 nm.

### Step 4. Synthesis of 1-((1R,3R)-3-aminocyclobutyl)-1H-benzo[d]imidazole-2(3H)-one (GEN1-025-4)

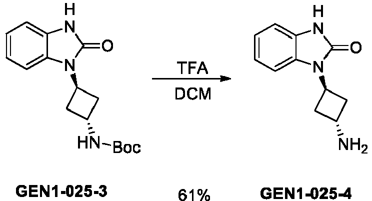

To a solution of tert-butyl ((1r,3r)-3-(2-oxo-2,3-dihydro-1H-benzo[d]imidazol-1-yl)cyclobutyl)carbamate (395 mg, 1.30 mmol) were added DCM (5mL) and TFA (1mL). The mixture was stirred at room temperature for 2 hours. The resulting mixture was basified to pH=8-10 with aqueous Na₂CO₃. Then the mixture was separated, and the organic layer was concentrated. The residue was purified by a C18 column (CH₃CN: water = 15-35%) to obtain the desired compound (179 mg, yield 61%) as a white solid. MS (ESI): Rt = 0.31 min, *m*/*z* 204.1 [M+H]⁺; purity: 99% @ 254 nm, 76% @ 214 nm.

### Step 5. Synthesis of N-((1r,3r)-3-(2-oxo-2,3-dihydro-1H-benzo[d]imidazol-1-yl)cyclobutyl)-2- (4-(trifluoromethyl)phenyl)acetamide (GEN1-025)

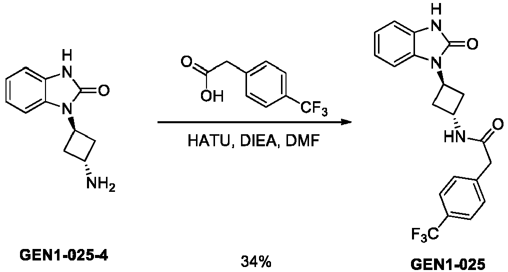

To a solution of 1-((1r,3r)-3-aminocyclobutyl)-1H-benzo[d]imidazole-2(3H)-one (100 mg, 0.492 mmol), HATU (281 mg, 0.739) and DIEA (191 mg, 1.48 mmol) in DMF (2 mL) was added 2-[4-(trifluoromethyl)phenyl]acetic acid (121 mg, 0.593 mmol). After the addition was completed, the mixture was stirred at room temperature for 16 hours. The resulting mixture was purified by preparative HPLC (prep-5 Xbridge C18 5um 19 * 150mm, 30%-70% B; A: H₂O (0.1% NH₄HCO₃), B: CH₃CN; 214nm, flow rate: 15mL/min) to obtain the desired compound (68 mg, yield 34%), as a white solid. ¹HNMR (400 MHz, DMSO-*d₆*): δ 10.86 (s, 1H), 8.73 (d, *J* = 6.8 Hz, 1H), 7.68 (d, *J* = 8.0 Hz, 2H), 7.51 (d, *J* = 8.0 Hz, 2H), 7.32-7.30 (m, 1H), 7.03-6.97 (m, 3H), 5.16-5.07 (m, 1H), 4.37-4.35 (m, 1H), 3.57 (s, 2H), 3.12-3.04 (m, 2H), 2.31-2.24 (m, 2H). MS (ESI): Rt = 3.723 min, *m*/*z* 390.2 [M+H]⁺; purity: 86.87% @ 254 nm, 96.20% @ 214 nm.

### Example 47: 1-(1-(2-(4-(trifluoromethyl)phenyl)acetyl)piperidin-4-yl)-1H-imidazo[4,5-b]pyridine-2(3H)-one

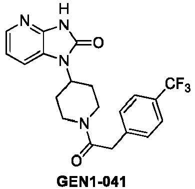

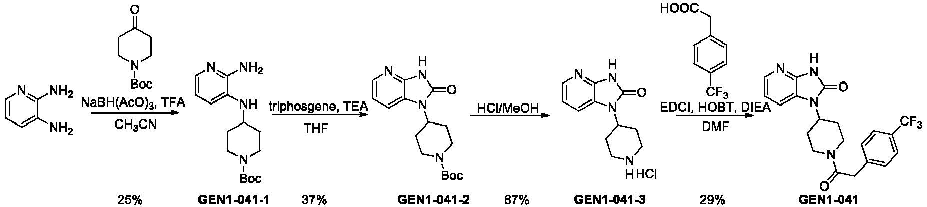

### Step 1. Synthesis of tert-butyl 4-((2-aminopyridin-3-yl)amino)piperidine-1-carboxylate (GEN1-041-1)

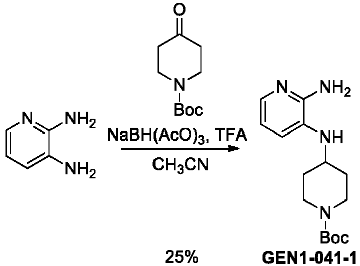

To a suspension of tert-butyl 4-oxopiperidine-1-carboxylate (1.00 g, 5.04 mmol) in acetonitrile (10 mL) was added pyridine-2,3-diamine (500 mg, 4.58 mmol). After stirring for 30 minutes at room temperature, all the solid matter was completely dissolved. The reaction mixture was cooled to 0°C, and TFA (0.7 mL) was added dropwise within 1 minute. The reaction mixture was stirred for another 30 minutes, and sodium triacetoxyborohydride (1.46 g, 6.87 mmol) was added. The mixture was then stirred at room temperature for another 30 minutes. The mixture was cooled to 0°C and basified to pH=13 with aqueous NaOH (4M). The mixture was stirred for another 30 minutes and diluted with ice water (50 mL). The mixture was extracted with DCM (50 mL×3). The combined organic layer was washed with brine (50 mL) and dried over anhydrous Na₂SO₄. The solution was concentrated, and the residue was purified by column chromatography on silica gel (DCM (0.1% TEA): MeOH = 40:1) and further purified by C18 (50-70% B; A: H₂O B: CH₃CN). The desired compound (382 mg, yield 25%) was obtained as a light brown solid. MS (ESI): Rt = 1.45 min, *m*/*z* 293.4 [M+H]⁺; purity: 87% @ 254 nm, 89% @ 214 nm.

### Step 2. Synthesis of tert-butyl 4-(2-oxo-2,3-dihydro-1H-imidazo[4,5-b]pyridin-1-yl)piperidine-1-carboxylate (GEN1-041-2)

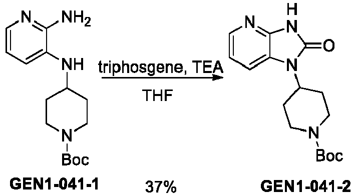

To a solution of tert-butyl 4-((2-aminopyridin-3-yl)amino)piperidine-1-carboxylate (367mg, 1.26mmol) and TEA (254mg, 2.51mmol) in THF (10mL) was slowly added triphosgene (372 mg, 1.25 mmol). After the addition, the mixture was stirred at room temperature for 2 hours. The resulting mixture was concentrated and basified to pH=8-10 with saturated Na₂CO₃. DCM (30 mL×3) was added to extract the desired compound. The combined organic layer was washed with brine (20 mL), dried over anhydrous Na₂SO₄ and concentrated. The residue was purified by a C18 column (45-65% B; A: H₂O B: CH₃CN) to obtain the desired compound (159 mg, yield 37%) as a yellow solid.

MS (ESI): Rt = 1.38 min, *m*/*z* 317.4 [M-H]⁻; purity: 94% @ 254 nm, 100% @ 214 nm.

### Step 3. Synthesis of 1-(piperidin-4-yl)-1H-imidazo[4,5-b]pyridine-2(3H)-monohydrochloride (GEN1-041-3)

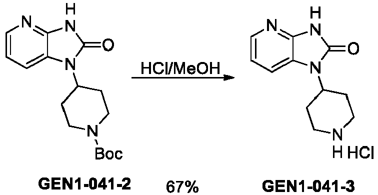

A solution of tert-butyl 4-(2-oxo-2,3-dihydro-1H-imidazo[4,5-b]pyridin-1-yl)piperidine-1-carboxylate (159 mg, 0.499 mmol) in HCl/MeOH (4M, 5mL) was stirred at room temperature for 3 hours. Concentration was performed to obtain the desired compound (173 mg, yield 67%) as a pale yellow solid. MS (ESI): Rt = 0.30 min, *m*/*z* 219.3 [M+H]⁺; purity: 94% @ 254 nm, 100% @ 214 nm.

### Step 4. Synthesis of 1-(1-(2-(4-(trifluoromethyl)phenyl)acetyl)piperidin-4-yl)-1H-imidazo[4,5-b]pyridine-2(3H)-one (GEN1-041)

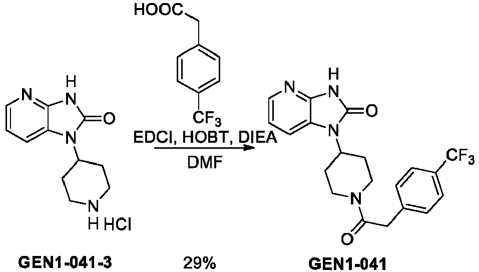

To 1-(piperidin-4-yl)-1H-imidazo[4,5-b]pyridine-2(3H)-monohydrochloride (173 mg, 0.594 mmol) in DIEA (384 mg, 2.97 mmol), EDCI (171 mg, 0.892 mmol), and HOBT (120 mg, 0.888 mmol) was added 2-(4-(trifluoromethyl)phenyl)acetic acid (146 mg, 0.715 mmol) in DMF (2 mL ) and the mixture was stirred overnight at room temperature. The resulting mixture was purified by C18 (45-65% B; A: H₂O B: CH₃CN) to obtain the desired compound (70 mg, yield 29%) as a white solid. ¹HNMR (400 MHz, CDCl₃): δ 10.18 (s, 1H), 8.05 (dd, *J* = 5.6, 1.2 Hz, 1H), 7.64 (d, *J* = 8.0 Hz, 2H), 7.46 (d, *J* = 8.0 Hz, 2H), 7.03 (dd, *J* = 7.6, 1.2 Hz, 1H), 6.97-6.93 (m, 1H), 4.89 (d, *J* = 13.6 Hz, 1H), 4.60-4.51 (m, 1H), 4.06 (d, *J* = 13.6 Hz, 1H), 3.87 (s, 2H), 3.24-3.16 (m, 1H), 2.75-2.67 (m, 1H), 2.19-2.08 (m, 1H), 1.93-1.84 (m, 3H). MS (ESI): Rt = 3.263 min, *m*/*z* 405.1 [M+H]⁺; purity: 96.18% @ 254 nm, 98.61% @ 214 nm.

### Example 48: 1-(1-(2-(4-(trifluoromethyl)phenyl)acetyl)piperidin-4-yl)-1H-imidazo[4,5-c]pyridin-2(3H)-one

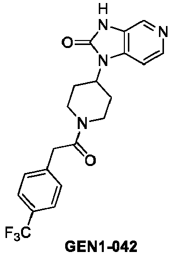

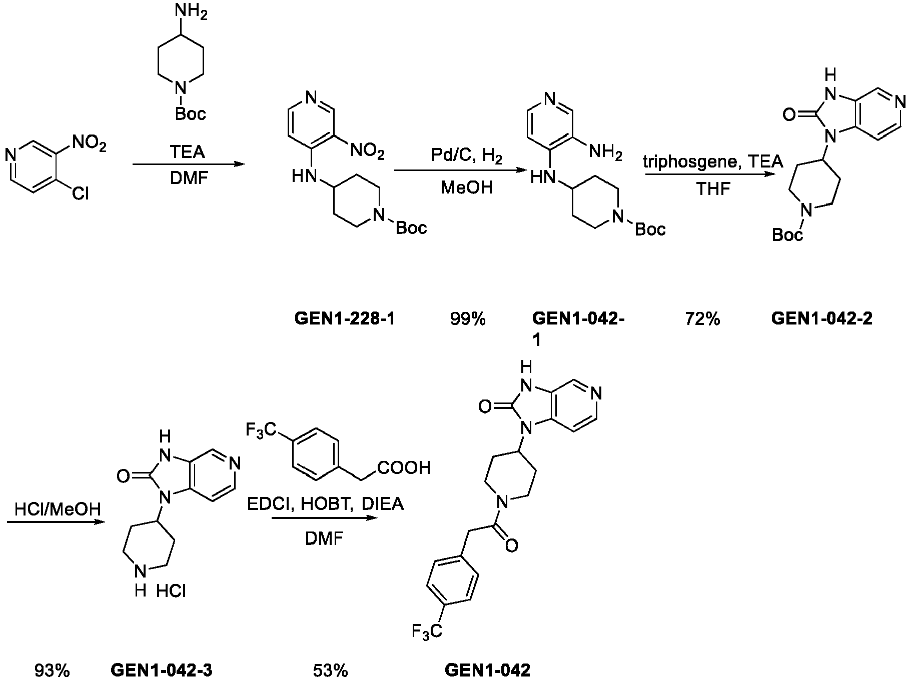

### Step 1. Synthesis of tert-butyl 4-((3-nitropyridine-4-yl)amino)piperidin-1-carboxylate (GEN1-228-1)

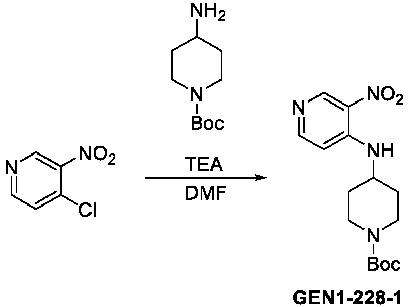

TEA (479 mg, 4.73 mmol) was added to a solution of 4-chloro-3-nitropyridine (500 mg, 3.15 mmol) and tert-butyl 4-aminopiperidine-1-carboxylate (695 mg, 3.47 mmol) in DMF(5 mL). The resulting mixture was stirred at room temperature overnight. The reaction mixture was poured into water (50 mL) and extracted with EtOAc (30 mL X 3).The combined organic layer was washed with brine (30 mL), dried over MgSO₄, filtered and it was concentrated to obtain the desired compound (1.1 g, yield 98%) as a yellow oil. MS (ESI): Rt = 1.57 min, *m*/*z* 323.3 [M+H]⁺; Purity: 91% @ 254 nm, 81% @ 214 nm.

### Step 2. Synthesis of tert-butyl 4-((3-aminopiperidine-4-yl)amino)piperidin-1-carboxylate (GEN1-042-1)

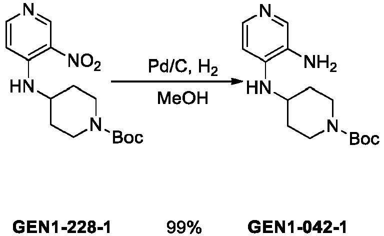

Pd/C (10%, 132 mg) was added to a solution of tert-butyl 4-((3-nitropyridine-4-yl)amino)piperidin-1-carboxylate (400 mg, 1.24 mmol, the synthesis steps could be found in the final report of GEN1-228-1.) in MeOH (10 mL). The mixture was stirred at room temperature under hydrogen for 2 hours. The obtained mixture was filtered, and the filtrate was concentrated to obtain the desired compound (360 mg, yield 99%) as a gray solid. MS (ESI): Rt = 1.36 min, *m*/*z* 293.4 [M+H]⁺; Purity: 100% @ 254 nm, 100% @ 214 nm.

### Step 3. Synthesis of tert-butyl 4-(2-oxo-2,3-dihydro-1H-imidazo[4,5-c]pyridin-1-yl)piperidin-1-carboxylate (GEN1-042-2)

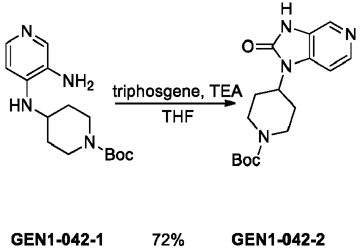

Triphosgene (378 mg, 1.27 mmol) was added to a solution of tert-butyl 4-((3-aminopiperidine-4-yl) amino) piperidin-1-carboxylate (310 mg, 1.06 mmol) and TEA(215 mg, 2.12 mmol) THF (15 mL). After the addition, the mixture was stirred at room temperature for 1 hour. The mixture was then basified to pH=7-8 with saturated NaHCO₃. DCM (40 mL×3) was added to extract the desired compound. The combined organic phase was washed with brine (50 mL), dried over anhydrous Na₂SO₄ and purified by C18 (40-60% B; A: H₂O B: CH₃CN) to obtain the desired compound (243 mg, yield 72%) as a light yellow solid. MS (ESI): Rt = 1.34 min, *m*/*z* 319.4 [M+H]⁺; Purity: 100% @ 254 nm, 100% @ 214 nm.

### Step 4. Synthesis of 1-(piperidin-4-yl)-1H-imidazo[4,5-c]pyridin-2(3H)-monohydrochloride (GEN1-042-3)

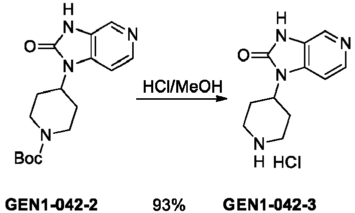

A solution of tert-butyl 4-(2-oxo-2,3-dihydro-1H-imidazo[4,5-c]pyridin-1-yl)piperidin-1-carboxylate (150 mg, 0.471 mmol) in HCl/MeOH (2 M, 10 mL) was stirred at room temperature for 4 hours. The obtained mixture was concentrated to obtain the desired compound (119 mg, yield 100%) as a white solid. MS (ESI): Rt = 0.29 min, *m*/*z* 219.2 [M+H]⁺; Purity: 100% @ 254 nm, 100% @ 214 nm.

### Step 5. Synthesis of 1-(1-(2-(4-(trifluoromethyl)phenyl)acetyl)piperidin-4-yl)-1H-imidazo[4,5-c]pyridin-2(3H)-one (GEN1-042)

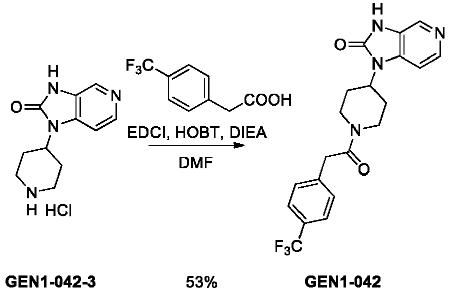

1-(piperidin-4-yl)-1H-imidazo[4,5-c]pyridin-2(3H)-monohydrochloride(119 mg, 0.470 mmol), DIEA(353 mg, 2.73 mmol), added EDCI (157 mg, 0.819 mmol) and HOBT (111 mg, 0.821 mmol) and 2-(4-(trifluoromethyl) phenyl)acetic acid (123 mg, 0.603 mmol) were dissolved in DMF (3 mL) and stirred overnight at room temperature. Then the mixture was purified by preparative HPLC (water 3 sunfire C18 5um 19 * 150mm, 10-40% B; A: H₂O (0.1% TFA), B: CH3CN; 214 nm; flow rate: 15 mL/min) to obtain the desired compound (120 mg, yield 63%) as a white solid. ¹H NMR (400 MHz, CDCl₃): δ 9.50 (s, 1H), 8.37 (s, 1H), 8.26 (d, *J* = 6.0 Hz, 1H), 7.64 (d, *J* = 8.0 Hz, 2H), 7.45 (d, *J* = 8.0 Hz, 2H), 6.83 (d, *J* = 4.8 Hz, 1H), 4.91 (d, *J* = 13.2 Hz, 1H), 4.54-4.46 (m, 1H), 4.07 (d, *J* = 13.2 Hz, 1H), 3.88 (s, 2H), 3.24-3.18 (m, 1H), 2.75-2.69 (m, 1H), 2.27-2.16 (m, 1H), 2.03-1.94 (m, 1H), 1.93-1.90 (m, 1H), 1.85-1.82 (m, 1H). MS (ESI) Rt = 3.486 min, *m*/*z* 405.2 [M+H]⁺; Purity: 98.96% @ 254 nm, 98.42% @ 214 nm.

### Example 49: 3-(1-(2-(4-(trifluoromethyl)phenyl)acetyl)piperidin-4-yl)-1H-imidazo[4,5-c]pyridin-2(3H)-one (GEN1-043)

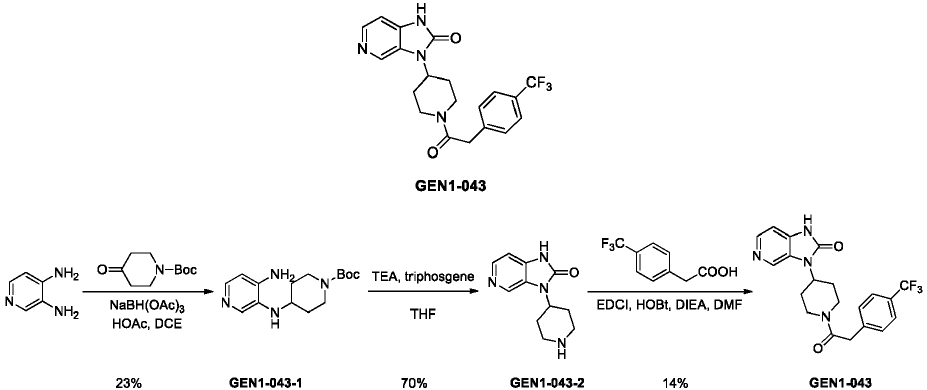

### Step 1. Synthesis of tert-butyl 4-((4-aminopyridin-3-yl)amino)piperidin-1-carboxylate (GEN1-043-1)

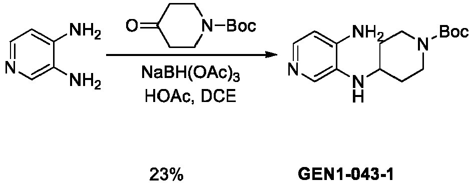

NaBH(OAc)₃ (3.69 g, 17.4 mmol) was added to a solution of pyridine 3,4-diamine (1.0 g, 9.2 mmol), tert-butyl 4-oxopiperidine-1-carboxylate (3.10 g, 15.6 mmol) and acetic acid (10.5 g, 174 mmol) in 1,2-dichloroethane (10 mL). The resulting mixture was stirred at room temperature for 3 days. The resulting mixture was concentrated, and saturated Na₂CO₃ (50 mL) was added. The mixture was extracted with DCM (100 mL × 5), and the combined organic layer was dried over anhydrous Na₂SO₄ and concentrated. The crude product was purified by a silica gel column (DCM: MeOH = 10:1) to obtain the desired compound (620 mg, yield 23%) as a yellow solid. MS (ESI) Rt = 1.32 min, *m*/*z* 293.3 [M+H]⁺; Purity: 100% @ 254 nm, 100% @ 214 nm.

### Step 2. Synthesis of 3-(piperidin-4-yl)-1H-imidazo[4,5-c]pyridin-2(3H)-one (GEN1-043-2)

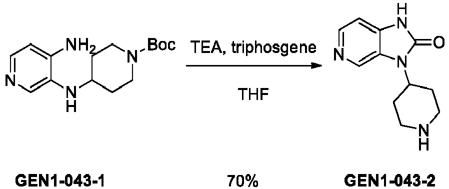

Triphosgene (457 mg, 1.54 mmol) was added to a solution of tert-butyl 4-((4-aminopyridin-3-yl)amino)piperidin-1-carboxylate (450 mg, 1.54 mmol) and TEA(312 mg, 3.08 mmol) in THF (7.50 mL) at 0°C. The mixture was then stirred at room temperature for 1 hour. Then water (5 mL) was added, and the mixture was stirred at 55°C for another 15 minutes. The resulting mixture was concentrated, and the mixture was purified by C18 (5-25% B; A: H₂O, B: CH₃CN) to obtain the desired compound (234 mg, yield 70%) as a white solid. MS (ESI): Rt = 0.29 min, *m*/*z* 219.2 [M+H]⁺; Purity: 100% @ 254 nm, 100% @ 214 nm.

### Step 3. Synthesis of 3-(1-(2-(4-(trifluoromethyl)phenyl)acetyl)piperidin-4-yl)-1H-imidazo[4,5-c]pyridin-2(3H)-one (GEN1-043)

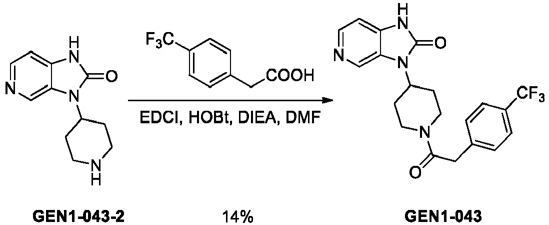

3-(4-piperidinyl)-1H-imidazo[4,5-c]pyridin-2-one (100 mg, 0.458 mumol), 2-[4-(trifluoromethyl)phenyl]acetic acid (113 mg, 0.554 mmol), HOBt (99 mg, 0.73 mmol), DIEA (296 mg, 2.29 mmol) and EDCI (141 mg, 0.736 mmol) were dissolved in DMF (3mL). The mixture was stirred at room temperature for 16 hours. The resulting mixture was purified by preparation. HPLC(prep-5 Xbridge C18 5um 19 * 150 mm, 15-55% B; A: H₂O(0.1%NH₄HCO₃), B: CH₃CN; UV: 214 nm, flow rate: 15 mL/min). The desired compound (27 mg, yield 14%) was obtained as a white solid. ¹HNMR (400 MHz, CDCl₃): δ 8.35-8.31 (m, 2H), 7.63 (d, *J* = 8.0 Hz, 2H), 7.43 (d, *J* = 8.0 Hz, 2H), 7.06 (d, *J* = 5.6 Hz, 1H), 4.95-4.91 (m, 1H), 4.56-4.47 (m, 1H), 4.07 (d, *J* = 12.8 Hz, 1H), 3.88 (s, 2H), 3.24-3.18 (m, 1H), 2.77-2.71 (m, 1H), 2.36-2.25 (m, 1H), 2.17-2.07 (m, 1H), 1.97-1.87 (m, 2H). MS (ESI) Rt = 3.065 min, *m*/*z* 405.2 [M+H]⁺; Purity: 96.88% @ 254 nm, 99.31% @ 214 nm.

### Example 50: 3-(1-(2-(4-(trifluoromethyl)phenyl)acetyl)piperidin-4-yl)-1H-imidazo[4,5-b]pyridin-2(3H)-one

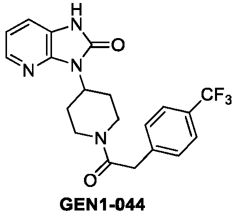

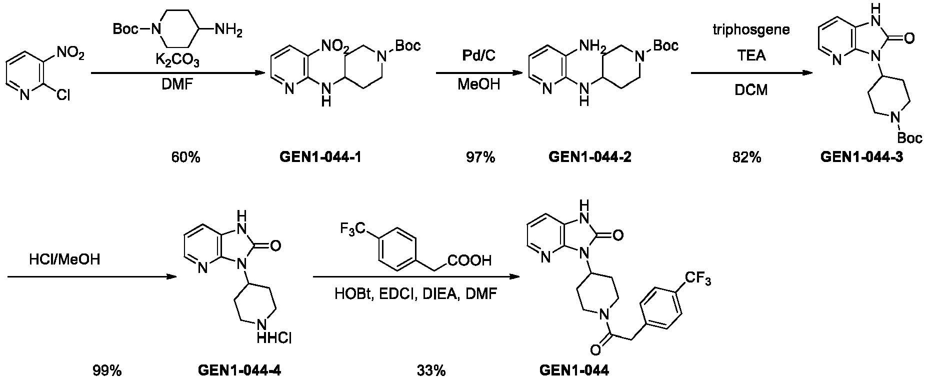

### Step 1. Synthesis of tert-butyl 4-((3-nitropyridine-2-yl)amino)piperidin-1-carboxylate (GEN1-044-1)

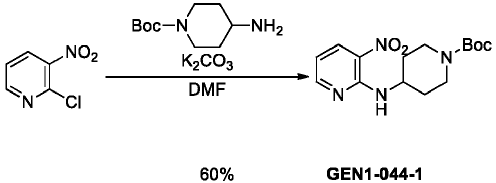

Tert-butyl 4-aminopiperidine-1-carboxylate (834 mg, 4.16 mmol) was added to a suspension of 2-chloro-3-nitropyridine (600 mg, 3.78 mmol) and K₂CO₃ (785 mg, 5.68 mmol) in DMF (6 mL). The mixture was stirred at 80°C for 3 hours. The resulting mixture was poured into water (100 mL), and extracted with EtOAc (100 mL×3).The combined organic layer was washed with brine (100 mL), dried over anhydrous Na₂SO₄, and purified by a silica gel column (PE:EA=4:1) to obtain the desired compound (854 mg, 60% yield) as a yellow solid. MS (ESI): Rt = 1.71 min, *m*/*z* 267.2 [M+H-56]⁺; Purity: 70% @ 254 nm, 65% @ 214 nm.

### Step 2 Synthesis of tert-butyl 4-((3-aminopyridin-2-yl)amino)piperidin-1-carboxylate (GEN1-044-2)

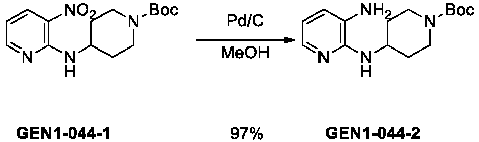

Pd/C (304 mg, 0.286 mmol , purity 10%) was added to a solution of tert-butyl 4-[((3-nitro-2-pyridyl)amino]piperidin-1-carboxylate (920 mg, 2.85 mmol) in MeOH (20 mL). The mixture was stirred under a hydrogen atmosphere at room temperature for 2 hours. The resulting mixture was filtered, and the filtrate was concentrated to obtain the desired compound (807 mg, yield 97%) as a pink solid. MS (ESI): Rt = 1.42 min, *m*/*z* 293.3 [M+H]⁺; Purity: 100% @ 254 nm, 100% @ 214 nm.

### Step 3. Synthesis of tert-butyl 4-(2-oxo-1H-imidazo[4,5-b]pyridin-3(2H)-yl)piperidin-1-carboxylate (GEN1-044-3)

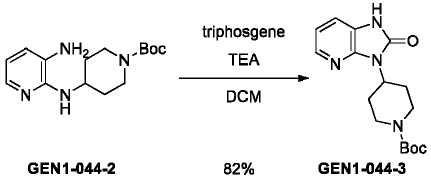

In an ice bath, triphosgene (924 mg, 3.11 mmol) was added to a solution of tert-butyl 4-[((3-amino-2-pyridyl)amino]piperidin-1-carboxylate (607 mg, 2.08 mmol) and TEA (420 mg, 4.15 mmol) in THF (15 mL). The mixture was stirred at 27°C for 4 hours. The obtained mixture was concentrated and purified by a C18 column (60-80% B; A: H₂O, B: CH₃CN) to obtain the desired compound (613 mg, yield 82%) as a white solid.

MS (ESI): Rt = 1.84 min, *m*/*z* 263.2 [M+H]⁺; Purity: 34% @ 254 nm, 100% @ 214 nm.

### Step 4. Synthesis of 3-(piperidin-4-yl)-1H-imidazo[4,5-b]pyridin-2(3H)-monohydrochloride (GEN1-044-4)

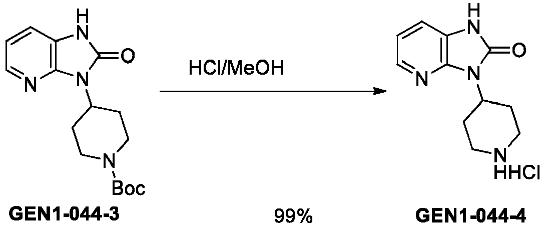

Tert-butyl 4-(2-oxo-1H-imidazo[4,5-b]pyridin-3-yl)piperidin-1-carboxylate (314 mg, 0.986 mmol) was stirred in a concentrated solution of hydrochloric methanol (6 mL) at room temperature for 1 hour. The resulting mixture was concentrated to obtain the desired compound (279 mg, yield 99%) as a white solid. MS (ESI): Rt = 0.33 min, *m*/*z* 219.1 [M+H]⁺; Purity: 100% @ 254 nm, 100% @ 214 nm.

### Step 5. Synthesis of 3-(1-(2-(4-(trifluoromethyl)phenyl)acetyl)piperidin-4-yl)-1H-imidazo[4,5-b]pyridin-2(3H)-one (GEN1-044)

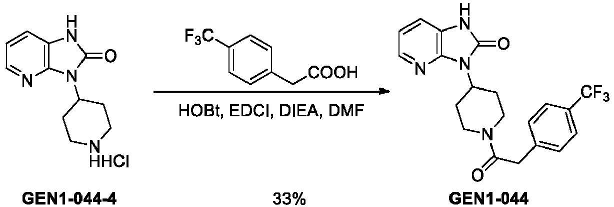

1-ethyl-3-(3-dimethylaminopropyl)carbodiimide hydrochloride (181 mg, 0.944 mmol) was added to a solution of 3-(4-piperidinyl)-1H-imidazo[4,5-b]pyridin-2-one hydrochloride (150 mg, 0.589 mmol), 2-[4-(trifluoromethyl)phenyl]acetic acid (144 mg, 0.705 mmol), HOBt (127 mg, 0.940 mmol) and DIEA (381 mg, 2.95 mmol) in DMF (4 mL). The mixture was stirred at room temperature for 16 hours. The resulting mixture was purified by preparation. HPLC (prep-5 Xbridge C18 5um 19 * 150 mm, 25%-70% B; A: H₂O (0.1% NH₄HCO₃), B:CH₃CN; UV: 214 nm, flow rate: 15 mL/min). The desired compound (82 mg, yield 33%) was obtained as a white solid. ¹H NMR (400 MHz, CDCl₃) δ 9.46 (s, 1H), 8.02 (d, *J* = 5.2 Hz, 1H), 7.61 (d, *J* = 8.0 Hz, 2H), 7.43 (d, *J* = 8.0 Hz, 2H), 7.29 (s, 1H), 6.99 (dd, *J* = 8.0, 5.2 Hz, 1H), 4.89 (d, *J* = 10.4 Hz, 1H), 4.66-4.58 (m, 1H), 4.04 (d, *J* = 14.0 Hz, 1H), 3.87 (s, 2H), 3.21-3.14 (m, 1H), 2.76-2.54 (m, 3H), 1.88-1.80 (m, 2H). MS (ESI): Rt = 3.466 min, *m*/*z* 405.1 [M+H]⁺; Purity: 94.80% @ 254 nm, 97.26% @ 214 nm.

### Example 51: 1-(1-(2-(4-(trifluoromethyl)phenyl)acetyl)piperidin-4-yl)imidazolidine-2-one

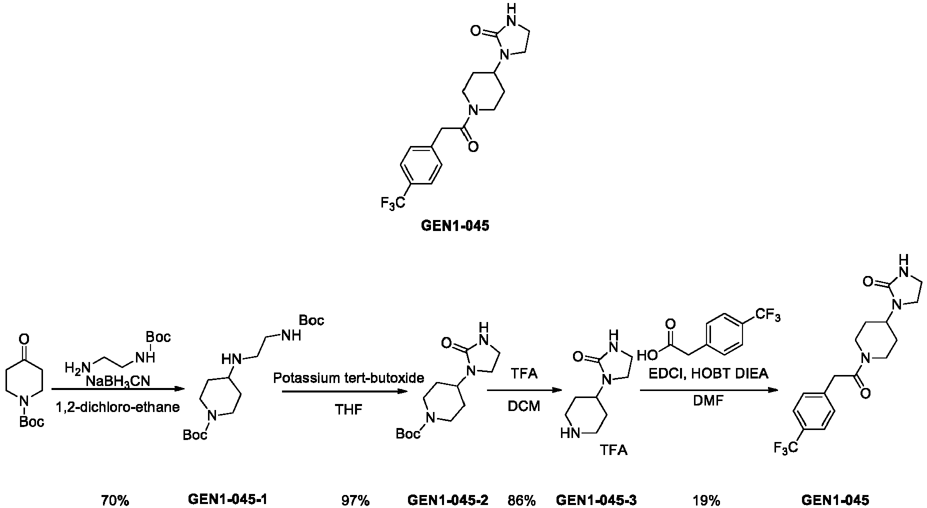

### Step 1. Synthesis of tert-butyl 4-(((2-((tert-butoxycarbonyl)amino)ethyl)amino)piperidin-1-carboxylate

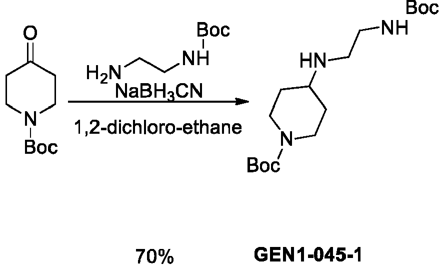

AcOH (1 mL) was added to a solution of tert-butyl 4-oxopiperidine-1-carboxylate (1.00 g, 5.02 mmol) and tert-butyl (2-aminoethyl) carbamate (804 mg, 5.02 mmol) in 1,2-dichloroethane (8 mL) at room temperature. After the mixture was stirred at room temperature for 4 hours, NaBH₃CN (473 mg, 7.53 mmol) was added. After the addition, the mixture was stirred at room temperature for 2 hours. The mixture was diluted with water (20 mL), and extracted with EtOAc (15 mL × 3). The combined organic layer was washed with brine (20 mL), dried over anhydrous Na₂SO₄ and filtered. The filtrate was concentrated to obtain the desired compound (1.5 g, yield 70%) as a colorless oil. MS (ESI): Rt = 1.35 min, *m*/*z* 344.4 [M+H]⁺; Purity: 19% @ 254 nm, 68% @ 214 nm.

### Step 2. Synthesis of tert-butyl 4-(2-oxoimidazolin-1-yl)piperidin-1-carboxylate

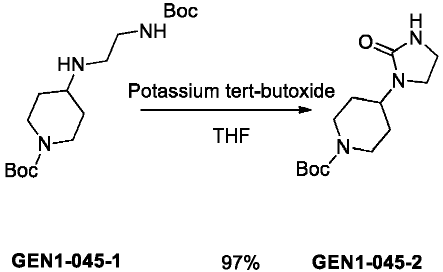

Potassium tert-butoxide (522 mg, 4.65 mmol) was added to a solution of tert-butyl 4-(((2-((tert-butoxycarbonyl)amino)ethyl)amino)piperidin-1-carboxylate (1.00 g, 1.98 mmol, purity 68%) in tetrahydrofuran (10 mL). The mixture was stirred at 55°C overnight. The mixture was then concentrated and the oily residue was diluted with water (20 mL). The mixture was extracted with EtOAc (15 mL×3).The combined organic phase was washed with brine (20 mL), dried over anhydrous Na₂SO₄ and filtered. The filtrate was concentrated, and the residue was purified by a C18 column (ACN/water=20-65%) to obtain the compound (520 mg, yield 97%) as a white solid. MS (ESI): Rt = 1.33 min, *m*/*z* 214.1 [M-56+H]⁺, 270.2 [M+H]⁺; Purity): 100% @ 214 nm.

### Step 3. Synthesis of 1-(piperidinbut-4-yl)imidazolin-2--2-one 2,2,2-trifluoroacetate

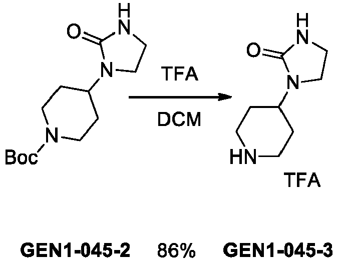

TFA (0.5 mL) was added to a solution of tert-butyl 4-(2-oxoimidazolin-1-yl)piperidin-1-carboxylate (300 mg, 1.11 mmol) in DCM (5 mL). The mixture was stirred at room temperature for 4 hours. Then the mixture was concentrated to obtain the desired compound (300 mg, purity 81%, yield 86%) as a colorless oil. MS (ESI): Rt = 0.28 min, *m*/*z* 170.0 [M+H]⁺; Purity: 39% @ 254 nm, 81% @ 214 nm.

### Step 4. Synthesis of 1-(1-(2-(4-(trifluoromethyl)phenyl)acetyl)piperidin-4-yl)imidazolidine-2-one

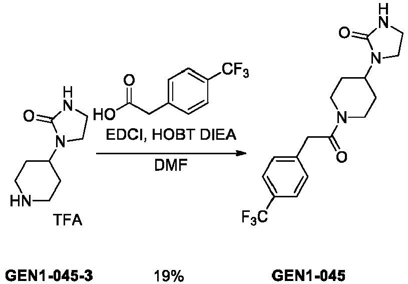

EDCI (275 mg, 1.43 mmol) was added to a solution of 2-(4-(trifluoromethyl)phenyl)acetic acid (233 mg, 1.14 mmol) and HOBT (194 mg, 1.44 mmol) in DMF (5 mL). After the mixture was stirred at room temperature for 5 minutes, 1-(piperidinbut-4-yl)imidazolin-2--2-one 2,2,2-trifluoroacetate (300 mg, 0.953 mmol) and DIEA (370 mg, 2.86 mmol) were added. The mixture was stirred at room temperature for 4 hours. The reaction mixture was diluted with water (20 mL), and extracted with EtOAc (15 mL×3).The combined organic phase was washed with brine (20 mL×2), dried over anhydrous Na₂SO₄ and filtered. The filtrate was concentrated, and the residue was purified by preparation. HPLC [RP-PREP-1 Xbridge C18, 5 um, 19 * 150 mm, 15∼50%B, A:H₂O(0.1%NH₄HCO₃), B: ACN, flow rate: 16 mL / min, UV = 254 nm]. The desired compound (66.2 mg, yield 19%) was obtained as a white solid. ¹HNMR (400 MHz, DMSO-*d*₆): δ 7.67 (d, *J* = 8.0 Hz, 2H), 7.45 (d, *J* = 8.0 Hz, 2H), 6.26 (s, 1H), 4.47 (d, *J* = 13.2 Hz, 1H), 4.02 (d, *J* = 14.0 Hz, 1H), 3.89-3.80 (m, 2H), 3.75-3.67 (m, 1H), 3.25-3.09 (m, 4H), 3.09-3.03 (m, 1H), 2.61-2.55 (m, 1H), 1.56-1.54 (m, 2H), 1.45-1.35 (m, 2H). MS (ESI): Rt = 3.283 min, *m*/*z* 356.2 [M+H]⁺; Purity: 81.46% @ 254 nm, 98.06% @ 214 nm.

### Example 52: 1-acetylamino-N-(1-(2-(4-(tris(trifluoromethyl)phenyl)acetyl)piperidin-4-yl)cyclopropanecarboxamide (GEN1-048)

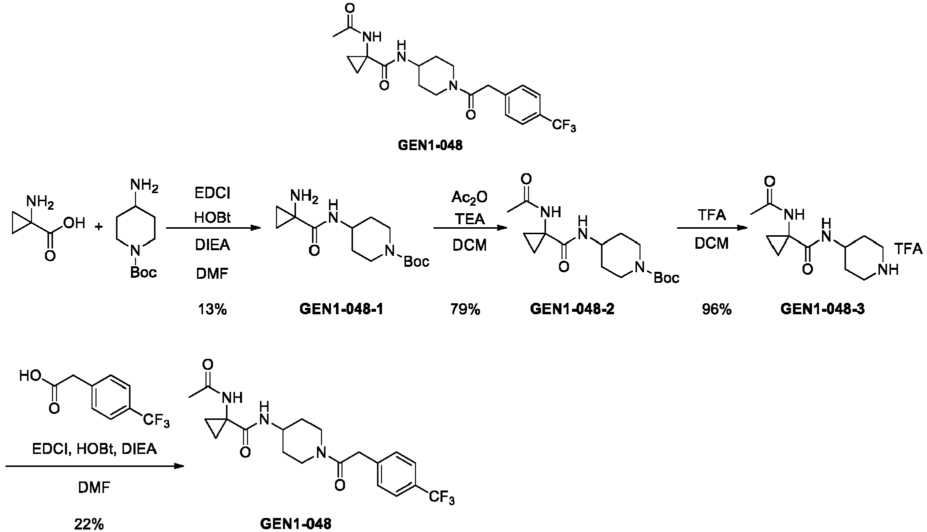

### Step 1. Synthesis of tert-butyl 4-(1-aminocyclopropanecarboxamido)piperidin-1-carboxylate (GEN1-048-1)

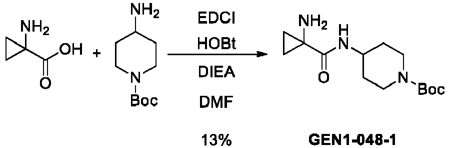

In an ice bath, EDCI (1.42 g, 7.42 mmol) was added to a solution of 1-aminocyclopropanecarboxylic acid (600 mg, 5.93 mmol), tert-butyl 4-aminopiperidine-1-carboxylate (990 mg, 4.94 mmol), HOBt (1.00 g, 7.41 mmol) and DIEA (1.92 g, 14.8 mmol) in DMF (15mL). The resulting mixture was stirred at room temperature overnight. The reaction mixture was poured into water (50 mL), and extracted with EtOAc (20 mL×3).The combined organic layer was washed with brine (20 mL), dried over MgSO₄, filtered and it was concentrated. The crude product was purified by a C18 column (CH₃CN: water = 5% to 50%) to obtain the desired compound (190 mg, yield 13%) as a colorless oil. MS (ESI): Rt = 1.33 min, *m*/*z* 228.1 [M+H-56]⁺; Purity: 31% @ 254 nm, 93% @ 214 nm.

### Step 2. Synthesis of tert-butyl 4-(1-acetylaminocyclopropanecarboxamide)piperidin-1-carboxylate (GEN1-048-2)

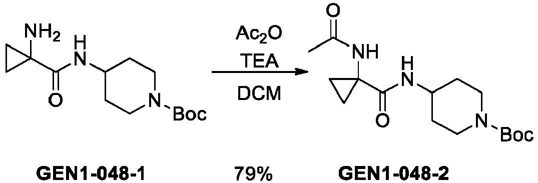

In an ice bath, Ac2O (66 mg, 0.65 mmol) was added to a solution of tert-butyl 4-[((1-aminocyclopropanecarbonyl)amino]piperidin-1-carboxylate (190 mg, 0.536 mmol) and TEA (163 mg, 1.61 mmol) in DCM (5 mL), and then it was stirred at room temperature overnight. The reaction mixture was poured into water (50 mL), and extracted with DCM (20 mL×3).The combined organic layer was washed with brine (20 mL), dried over MgSO₄, filtered and it was concentrated to obtain the desired compound (220 mg crude product, yield 79%) as a brown oil. MS (ESI) Rt = 1.25 min, *m*/*z* 270.2 [M+H-56]⁺; Purity: No absorption @ 254 nm, 63% @ 214 nm.

### Step 3. Synthesis of 1-acetylamino-N-(piperidin-4-yl)cyclopropanecarboxamide trifluoroacetate (GEN1-048-3)

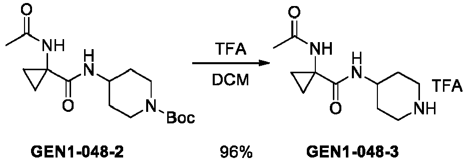

TFA (1 mL) was added to a solution of tert-butyl 4-[((1-acetylaminocyclopropanecarbonylamino)amino]piperidin-1-carboxylate (220 mg, 0.676 mmol) in DCM (4 mL) at room temperature. The resulting mixture was stirred at room temperature for 2 hours. The reaction mixture was concentrated to obtain the desired compound (220 mg crude product) as a brown oil. MS (ESI): Rt = 0.31 min, *m*/*z* 226.2 [M+H]⁺; Purity: No absorption @ 254 nm or 214 nm.

### Step 4. Synthesis of 1-acetylamino-N-(1-(2-(4-(tris(trifluoromethyl)phenyl)acetyl)piperidin-4-yl)cyclopropanecarboxamide (GEN1-048)

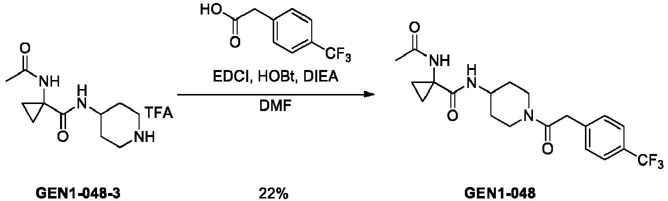

In an ice bath, EDCI (121 mg, 0.631 mmol) was added to 1-acetylamino-N-(4-piperidinyl)cyclopropanecarboxamide TFA salt (220 mg, 0.421 mmol), 2-[4-(trifluoromethyl)phenyl]acetic acid (86 mg, 0.42 mmol), HOBt (85 mg, 0.63 mmol) and DIEA (272 mg, 2.10 mmol) in DMF (2 mL). The resulting mixture was stirred at room temperature overnight. The resulting mixture was purified by a C18 column (CH₃CN: water = 5% to 55%) to obtain the desired compound (38.5 mg, yield 22%) as a white solid. ¹HNMR (400 MHz, DMSO-*d*₆): δ 8.19 (s, 1H), 7.67 (d, *J* = 8.0 Hz, 2H), 7.45 (d, *J* = 8.4 Hz, 2H), 7.41 (d, *J* = 8.0 Hz, 1H), 4.32-4.29 (m, 1H), 3.97-3.94 (m, 1H), 3.87-3.76 (m, 3H), 3.08 (t, *J* = 11.6 Hz, 1H), 2.65 (t, *J* = 11.6 Hz, 1H), 1.80 (s, 3H), 1.65-1.60 (m, 2H), 1.40- 1.29 (m, 2H), 1.24-1.17 (m, 2H), 0.84-0.75 (m, 2H). MS (ESI): Rt = 3.046 min, *m*/*z* 412.2 [M+H]⁺; Purity: 89.16% @ 254 nm, 98.72% @ 214 nm.

### Example 53: 1-phenyl-8-(2-(4-(trifluoromethyl)phenyl)acetyl)-1,3,8triazaspiro[4.5]decane-4-one (GEN1-049)

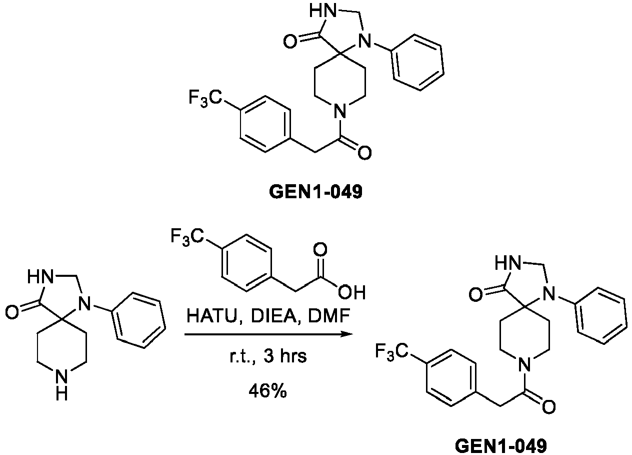

### Step 1. Synthesis of 1-phenyl-8-(2-(4-(trifluoromethyl)phenyl)acetyl)-1,3,8triazaspiro[4.5]decane-4-one (GEN1-049)

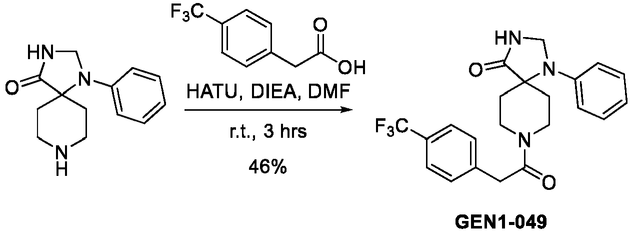

DIEA (56 mg, 0.43 mmol) was added to the mixture of 1-phenyl-1,3,8-triazaspiro[4.5]decane 4-one (50 mg, 0.22 mmol), 2-(4-(trifluoromethyl)phenyl)acetic acid (44 mg, 0.22 mmol) and HATU (123 mg, 0.32 mmol) in DMF (1.0mL). The resulting mixture was stirred at room temperature for 3 hours. The reaction mixture was added dropwise to water (20 mL). The precipitated solid was collected by filtration. The crude product was purified by Prep-TLC (EA / (PE + EA) = 60%) to obtain GEN1-049 (50 mg, 46% yield) as a white solid. ¹H NMR (400 MHz, DMSO-d₆): δ 8.79 (s, 1H), 7.72 (d, *J* = 8.1 Hz, 2H), 7.55 (d, *J* = 8.0 Hz, 2H), 7.17-7.09 (m, 2H), 6.72 (t, *J* = 7.3 Hz, 1H), 6.51 (d, *J* = 8.2 Hz, 2H), 4.56 (s, 2H), 4.41-4.32 (m, 1H), 4.07-3.94 (m, 2H), 3.83-3.68 (m, 2H), 3.31-3.21 (m, 1H), 2.34-2.20 (m, 1H), 2.18-2.04 (m, 1H), 1.60 (t, *J* = 13.4 Hz, 2H). MS (ESI): Rt = 1.52 min, *m*/*z* 417.9 [M + H]⁺, Purity: 100% @ 254 nm, 100% @ 214 nm.

### Example 54: 1-(4-(2-thioxo-2,3-dihydro-1H-benzo[d]imidazol-1-yl)piperidin-1-yl)-2-(4-(trifluoromethyl)phenyl)ethanone

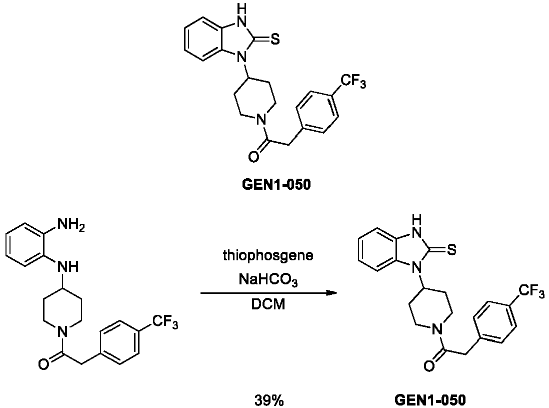

### Step 1. Synthesis of 1-(4-(2-thioxo-2,3-dihydro-1H-benzo[d]imidazol-1-yl)piperidin-1-yl)-2-(4-(trifluoromethyl)phenyl)ethanone (GEN1-050)

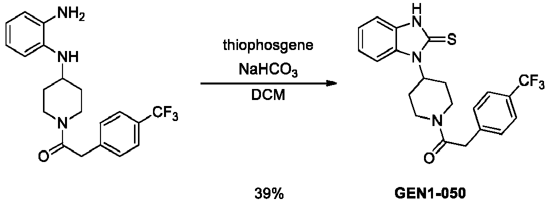

1-[4-(2-aminoanilino)-1-piperidinyl]-2-[4-(trifluoromethyl)phenyl]ethanone (100 mg, 0.265 mmol) and NaHCO₃ (223 mg, 2.65 mmol) were dissolved in DCM (5 mL), and thiophosgene (35 mg, 0.30 mmol) was added dropwise. The mixture was stirred at 0°C for 1 hour. The resulting mixture was concentrated and the residue was purified by preparation. HPLC (prep-2 Xbridge C18 5um 19 * 150mm, 32%-58%B; A: H₂O(0.1%NH₄HCO₃), B: CH₃CN; UV: 214 nm, flow rate: 15mL / min). The desired compound (45 mg, yield 39%) was obtained as a white solid. ¹HNMR (400 MHz, CDCl₃): δ 10.14 (s, 1H), 7.64 (d, *J* = 8.0 Hz, 2H), 7.49 (d, *J* = 8.0 Hz, 2H), 7.22-7.17 (m, 2H), 7.14-7.10 (m, 1H), 7.02 (d, *J* = 8.0 Hz, 1H), 5.49-5.41 (m, 1H), 4.92 (d, *J* = 13.2 Hz, 1H), 4.08 (d, *J* = 14.4 Hz, 1H), 3.90 (s, 2H), 3.33-3.26 (m, 1H), 2.83-2.76 (m, 1H), 2.31-2.21 (m, 1H), 1.97-1.94 (m, 2H), 1.87-1.85 (m, 1H). MS (ESI): Rt = 3.302 min, *m*/*z* 420.1 [M+H]⁺; Purity: 98.73% @ 254 nm, 97.00% @ 214 nm.

### Example 55: 3-(1-(2-(4-(trifluoromethyl)phenyl)acetyl)piperidin-4-yl)indol-2-one (GEN1-051)

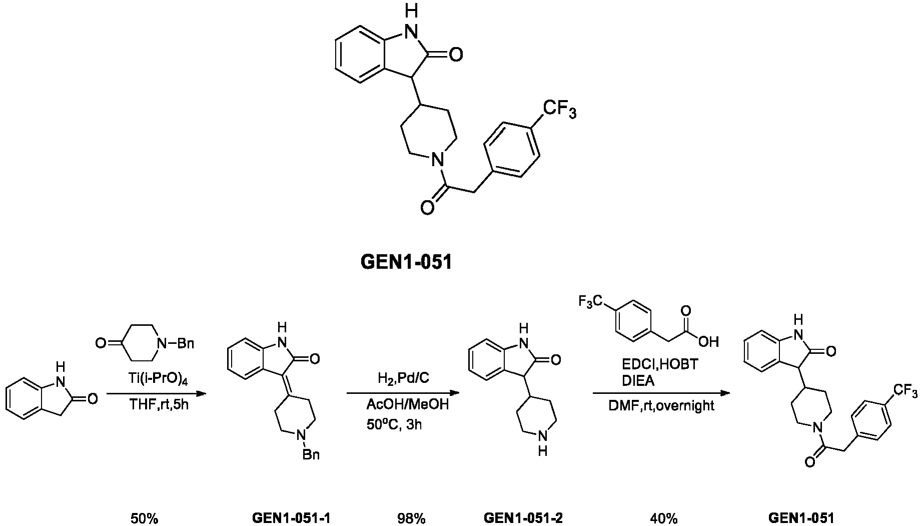

### Step 1. Synthesis of 3-(1-benzylpiperidin-4-alkylene)indol-2-one (GEN1-051-1)

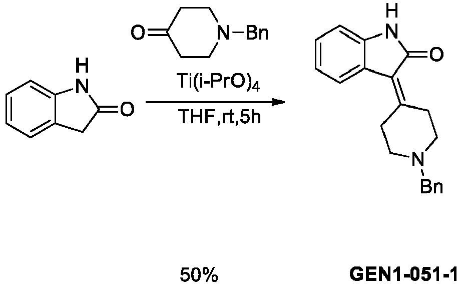

In an ice bath, a mixture of titanium tetraisopropoxide (2.56 g, 9.01 mmol) in THF (10 mL) was added dropwise to a solution of indol-2-one (600 mg, 4.51 mmol) and 1-benzylpiperidin-4-one (938 mg, 4.96 mmol) in THF (30 mL). The mixture was stirred at room temperature for 5 hours, and then was poured into water (50 mL) and EtOAc (20 mL). The mixture was filtered, and the filtrate was extracted with EtOAc (30 mL×2), the organic phases was concentrated and combined. The residue was triturated with CH₃CN (3 mL) and filtered to obtain the desired compound (700 mg, yield 50%) as a yellow solid. ¹H NMR (400 MHz, DMSO-*d*₆): δ 11.24 (s, 1H), 10.61 (s, 1H), 7.64-7.62 (m, 3H), 7.48-7.46 (m, 3H), 7.21 (t, *J* = 7.6 Hz, 1H), 6.95 (t, *J* = 7.6 Hz, 1H), 6.84 (d, *J* = 7.6 Hz, 1H), 4.53 (d, *J* = 15.6 Hz, 1H), 4.35 (s ,2H), 3.51-3.44 (m, 3H), 3.17-2.99 (m, 3H), 2.91-2.85 (m, 1H). MS (ESI) Rt = 1.61 min, *m*/*z* 305 [M+H]⁺; Purity: 100% @ 254 nm, 100% @ 214 nm.

### Step 2. Synthesis of 3-(piperidinbut-4-yl)indol-2-one (GEN1-051-2)

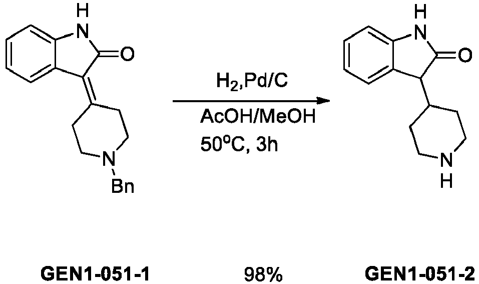

3-(1-benzyl-4-piperidinyl)indol-2-one (200 mg, 0.637 mmol) and Pd/C (10%, 100 mg) were added to a solution of AcOH (0.1 mL) and MeOH (5 mL), and it was stirred at 50°C under H2 (50Psi) for 3 hours. The mixture was filtered and the filtrate was concentrated. The residue was dissolved in MeOH (1 mL), and 10% NaOH aqueous solution was added to adjust pH=8-9. The mixture was concentrated and triturated with DCM/MeOH (10/1, 30 mL) and filtered. The filtrate was concentrated to obtain the desired compound (140 mg, yield 98%) as a yellow oil. MS (ESI): Rt = 0.56 min, *m*/*z* 217.2 [M+H]⁺; Purity: 100% @ 254 nm, 100% @ 214 nm.

### Step 3. Synthesis of 3-(1-(2-(4-(trifluoromethyl)phenyl)acetyl)piperidin-4-yl)indol-2-one (GEN1-051)

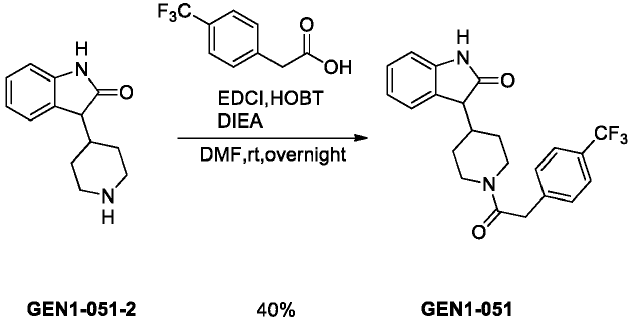

EDCI (186 mg, 0.97 mmol) and HOBT (131 mg, 0.97 mmol) were added to a solution of 3-(4-piperidinyl)indol-2-one (140 mg, 0.65 mmol), 2-(4-(trifluoromethyl)phenyl)acetic acid (132 mg, 0.65 mmol) and DIEA (251 mg, 1.94 mmol) in DMF (3 mL). The mixture was stirred at room temperature overnight, and poured into water (15 mL), and extracted with EtOAc (20 mL×2). The combined organic phase was concentrated. The desired compound (105 mg, yield 40%) was obtained by preparative HPLC (gilson-2, Xbridge C18, 5um, 19 * 150 mm, 50∼80%B, A: H₂O(0.1%NH₄HCO₃), B: ACN, flow rate: 15 mL / min, UV purification residue = 214 nm) as a white solid. ¹H NMR (400 MHz, DMSO-*d*₆): δ 10.36 (d, *J* = 3.2 Hz, 1H), 7.67 (d, *J* = 8.4 Hz, 1H), 7.60 (d, *J* = 8.4 Hz, 1H), 7.44 (d, *J* = 8.0 Hz, 1H), 7.39 (d, *J* = 8.0 Hz, 1H), 7.22 (d, *J* = 7.6 Hz, 0.5H), 7.19-7.15 (m, 1H), 7.02 (d, *J* = 7.2 Hz, 0.5H), 6.96-6.89 (m, 1H), 6.81-6.78 (m, 1H), 4.49-4.37 (m, 1H), 4.02-3.92 (m, 1H), 3.87-3.75 (m, 2H), 3.42-3.39 (m, 1H), 3.00-2.92 (m, 1 H), 2.52-2.44 (m, 1H), 2.26-2.20 (m, 1H), 1.61-1.54 (m, 1H), 1.41-1.11 (m, 3H). MS (ESI): Rt = 3.850 min, *m*/*z* 403.1 [M+H]⁺; Purity: 99.23% @ 254 nm, 99.58% @ 214 nm.

### Example 56: 4-(1-(2-(4-(trifluoromethyl)phenyl)acetyl)piperidin-4-yl)-3,4-dihydroquinoxaline-2(1H)-one

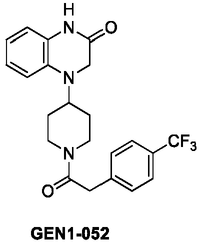

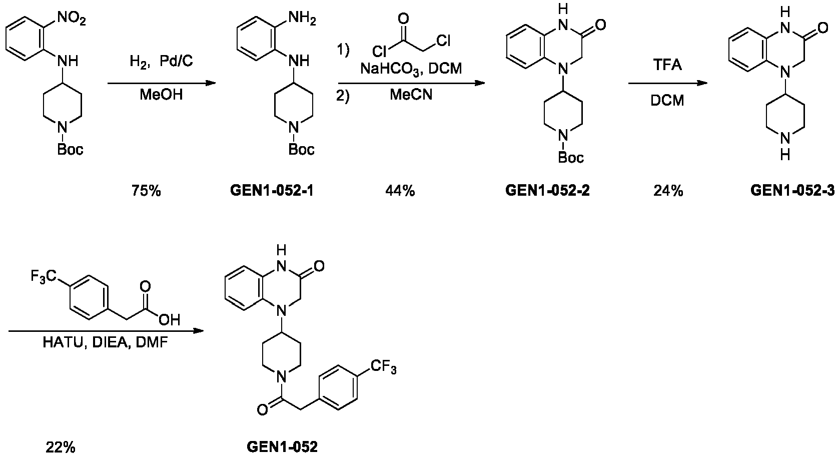

### Step 1. Synthesis of tert-butyl 4-((2-aminophenyl)amino)piperidin-1-carboxylate (GEN1-052-1)

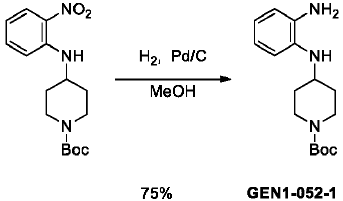

A suspension of tert-butyl 4-(2-nitroanilino)piperidin-1-carboxylate (2.0 g, 6.2 mmol, the synthesis steps could be found in other corresponding examples.) and Pd/C (100 mg, 10% purity) in MeOH (20 mL) was stirred at room temperature under hydrogen overnight. The mixture was filtered, and the filtrate was concentrated to obtain the desired compound (1.7 g, yield 75%) as a brown oil. MS (ESI): Rt = 1.58 min, *m*/*z* 236.1 [M+H-56]⁺; Purity: 100% @ 254 nm, 80% @ 214 nm.

### Step 2. Synthesis of tert-butyl 4-(3-oxo-3,4-dihydroquinoxaline-1(2H)-yl)piperidin-1-carboxylate (GEN1-052-2)

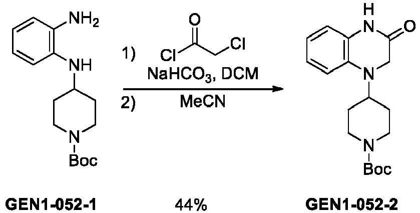

In an ice bath, 2-chloroacetyl chloride (659 mg, 5.83 mmol) was added to a mixture of tert-butyl 4-(2-aminoanilino)piperidin-1-carboxylate (1.7 g, 5.8 mmol) and NaHCO₃ (2.45 g, 29.2 mmol) and DCM (20 mL). The mixture was stirred at room temperature for 0.5 hours, and then concentrated and dissolved in CH₃CN (200 mL). The mixture was stirred at 80°C overnight. The mixture was cooled and filtered. The filtrate was concentrated, and the residue was purified by column chromatography on silica gel (PE:EA = 1:1) to obtain the desired compound (850 mg, yield 44%) as a white solid. ¹H NMR (400 MHz, CDCl₃): δ 8.99 (s, 1H), 7.01-6.97 (m, 1H), 6.83-6.76 (m, 3H), 4.28 (br s, 2H), 3.72 (s, 2H), 3.70-3.62 (m, 1H), 2.85-2.79 (m, 2H), 1.82-1.76 (m, 2H), 1.72-1.66 (m, 2H), 1.48 (s, 9H). MS (ESI): Rt = 0.95 min, m/z 276.1, [M+ H-56]⁺; Purity: 100% @ 254 nm, 100% @ 214 nm.

### Step 3. Synthesis of 4-(piperidin-4-yl)-3,4-dihydroquinoxaline-2(1H)-one (GEN1-052-3)

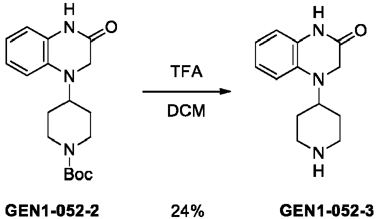

After a solution of tert-butyl 4-(3-oxo-2,4-dihydroquinoxalin-1-yl)piperidin-1-carboxylate (400 mg, 1.21 mmol) in TFA (1.5 mL) was stirred at room temperature overnight, the mixture was concentrated. The residue was dissolved in water (3 mL) and basified to pH=9 with aqueous Na₂CO₃ (saturated 5 mL). The mixture was extracted with DCM (10×4 mL). The combined organic layer was dried over Na₂SO₄ and concentrated to obtain the desired (71 mg, yield 24%) as a white solid. MS (ESI): Rt = 1.11 min, *m*/*z* 232.0 [M+H]⁺; Purity: 100% @ 254 nm, 94% @ 214 nm.

### Step 4. 4-(1-(2-(4-(trifluoromethyl)phenyl)acetyl)piperidin-4-yl)-3,4-dihydroquinoxaline-2(1H)-one (GEN1-052)

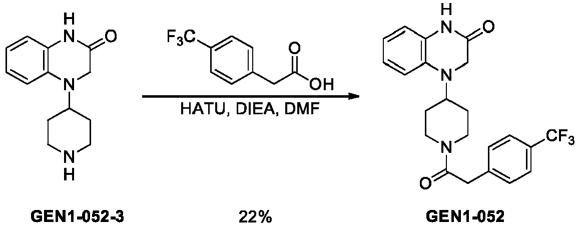

DIEA (159 mg, 1.23 mmol) was added to a solution of 4-(4-piperidinyl)-1,3-dihydroquinoxalin-2-one (71 mg, 0.31 mmol), 2-[4-(trifluoromethyl)phenyl]acetic acid (63 mg, 0.31 mmol), HATU (176 mg, 0.463 mmol) in DMF (1.5 mL). The mixture was stirred for 3 hours at room temperature, and then purified by a C18 column (CH₃CN: water = 5% to 50%) to obtain the desired compound (30mg, yield 22%) as a white solid. ¹H NMR (400 MHz, DMSO-*d*₆): δ 10.39 (s, 1H), 7.68 (d, *J* = 8.0 Hz, 2H), 7.47 (d, *J* = 7.6 Hz, 2H), 6.91-6.86 (m, 2H), 6.80 (d, *J* = 7.2 Hz, 1H), 6.71-6.67 (m, 2H), 4.54-4.51 (m, 1H), 4.09-4.05 (m, 1H), 3.92-3.82 (m, 3H), 3.56-3.47 (m, 2H), 3.22-3.15 (m, 1H), 2.75-2.67 (m, 1H), 1.70-1.50 (m, 4H). MS (ESI): Rt = 2.78 min, *m*/*z* 418.2, [M + H]⁺. Purity: 95.55% @ 254 nm, 97.03% @ 214 nm.

### Example 57: 1-(4-(2,2-dioxybenzo[c] [1,2,5]thiadiazole-1(3H)-yl)piperidin-1-yl)-2-(4-(trifluoromethyl)phenyl)ethenone

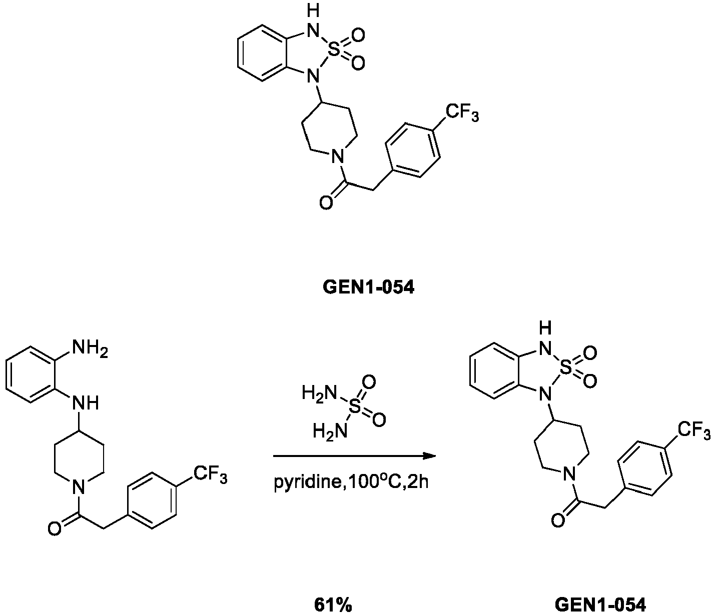

**Step 1. Synthesis of 1-(4-(2,2-dioxybenzo[c] [1,2,5]thiadiazole-1(3H)-yl)piperidin-1-yl)-2-(4-(trifluoromethyl)phenyl)ethenone (GEN1-054)**

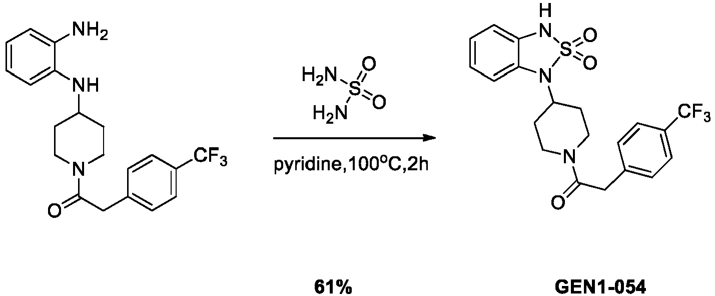

Sulfonamide (48 mg, 0.50 mmol) was added to a solution of 1-(4-(((2-aminophenyl)amino)piperidin-1-yl)-2-(4-(trifluoromethyl)phenyl)ethanon (130 mg, 0.25 mmol, the synthesis steps could be found in GEN1-055) in pyridine (3 mL), and the mixture was stirred at 100°C for 2 hours. The mixture was cooled to 40°C, and then concentrated. The residue was diluted with water (10 mL), and extracted with EtOAc (20 mL×2). The organic phase was washed with brine (20 mL), dried over sodium sulfate and it was concentrated. The residue was purified by column chromatography on silica gel (PE:EA = 1:1) to obtain a crude product (100 mg). The desired compound (68 mg, yield 61%) was obtained by preparative HPLC [gilson-2, Xbridge C18, 5um, 19× 150mm, 40∼80%B, A: H₂O (0.1%NH 4 HCO 3), B: ACN, flow rate: 15mL / min, further purification. UV = 214nm] as a white solid. ¹H NMR (400 MHz, DMSO-*d*₆): δ 11.26 (br s, 1H), 7.68 (d, *J* = 8.0 Hz, 2H), 7.48 (d, *J* = 8.0 Hz, 2H), 6.88-6.82 (m, 3H), 6.79-6.77 (m, 1H), 4.55 (d, *J* = 13.6 Hz, 1H), 4.26-4.22 (m, 1H), 4.11 (d, *J* = 13.2 Hz, 1H), 3.90 (s, 2H), 3.20-3.13 (m, 1H), 2.73-2.66 (m, 1H), 1.98-1.84 (m, 4H). MS (ESI): Rt = 4.647 min, *m*/*z* 440.1 [M+H]⁺; Purity: 86.50% @ 254 nm, 97.84% @ 214 nm.

### Example 58: Synthesis of 1-(4-((2-hydroxyphenyl)amino)piperidin-1-yl)-2-(4-(trifluoromethyl)phenyl)ethenone (GEN1-056)

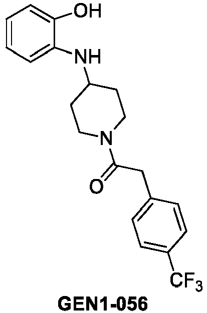

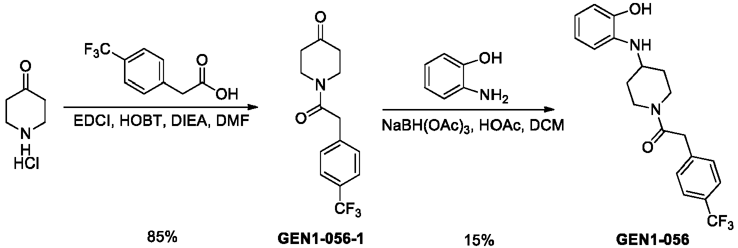

### Step 1. Synthesis of 1-(2-(4-(trifluoromethyl)phenyl)acetyl)piperidin-4-one (GEN1-056-1)

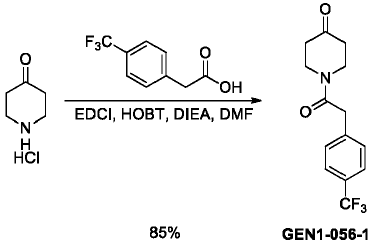

2-(4-(trifluoromethyl)phenyl)acetic acid (580 mg, 2.84 mmol) was added to a solution of 4-piperidone hydrochloride (350 mg, 2.58 mmol), DIEA (1.67 g, 12.9 mmol), EDCI (742 mg, 3.87 mmol) and HOBT (523 mg, 3.87 mmol) in DMF (3 mL), and it was stirred at room temperature overnight. The resulting mixture was poured into water (15 mL), and extracted with DCM (30 mL×3). The combined organic layers were concentrated, and the residue was purified by a C18 column (CH₃CN: water=45-65%) to obtain the desired compound (625 mg, yield 85%) as a yellow oil.

¹HNMR (300 MHz, DMSO-*d₆*): δ 7.71 (d, *J* = 8.1 Hz, 2H), 7.51 (d, *J* = 8.1 Hz, 2H), 3.97 (s, 2H), 3.86-3.76 (m, 4H), 2.44-2.37 (m, 4H).

### Step 2. Synthesis of 1-(4-((2-hydroxyphenyl)amino)piperidin-1-yl)-2-(4-(trifluoromethyl)phenyl)ethenone (GEN1-056)

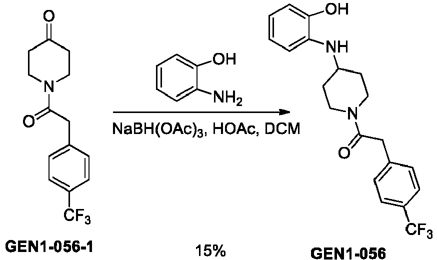

Acetic acid (524 mg, 8.73 mmol) was added to a suspension of 1-(2-(4-(tris(trifluoromethyl)phenyl)acetyl)piperidin-4-one (280 mg, 0.982 mmol), 2-aminophenol (107 mg, 0.982 mmol) and sodium triacetoxyborohydride (312 mg, 1.47 mmol) in DCM (10 mL). The mixture was stirred at room temperature for 24 hours. Saturated NaHCO₃ (20 mL) was added. The mixture was stirred for another 10 minutes,and then extracted with DCM (30 mL×3).The combined organic phase was washed with brine (10 mL), dried over anhydrous Na₂SO₄ and filtered. The filtrate was concentrated and the residue was purified by preparative HPLC (Preparative 5 Xbridge C18 5um 19 * 150mm, 35-85% B; A: H₂O (0.1% NH₄HCO₃), B: CH₃CN; 214 nm; flow rate: 15 mL/min) to obtain the desired compound (58 mg, yield 15%) as a gray solid. ¹H NMR (400M, DMSO-*d₆*): δ 9.18 (s, 1H), 7.67 (d, *J* = 8.4 Hz, 2H), 7.46 (d, *J* = 8.0 Hz, 2H), 6.67-6.56 (m, 3H), 6.41 (td, *J* = 7.6, 2.0 Hz, 1H), 4.30-4.28 (m, 2H), 3.94 (d, *J* = 13.2 Hz, 1H), 3.85 (s, 2H), 3.48-3.46 (m, 1H), 3.17 (t, *J* = 11.6 Hz, 1H), 1.91 (d, *J* = 10.0 Hz, 2H), 1.28-1.19 (m, 2H). MS (ESI): Rt = 2.947 min, *m*/*z* 379.2 [M+H]⁺; Purity: 89.39% @ 254 nm, 96.62% @ 214 nm.

### Example 59: Synthesis of N-(2-((1-(2-(4-(trifluoromethyl)phenyl)acetyl)piperidin-4-yl)amino)phenyl)acetamide

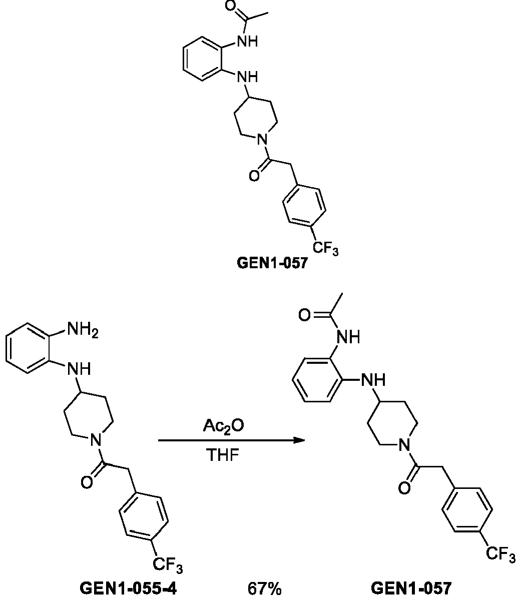

### Step 1. Synthesis of N-(2-((1-(2-(4-(trifluoromethyl)phenyl)acetyl)piperidin-4-yl)amino)phenyl)acetamide (GEN1-057)

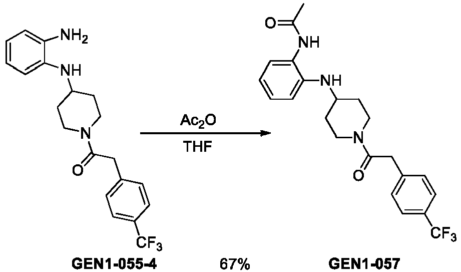

In an ice bath, Ac2O (130mg, 1.27mmol) was added to a solution of 1-(4-(((2-aminophenyl)amino)piperidin-1-yl)-2-(4-(trifluoromethyl)phenyl)ethenone (179 mg, 0.474 mmol, the synthesis steps could be found in the final report of GEN1-055-4.) in THF (7 mL). The mixture was stirred at room temperature for 2 hours, and then concentrated and saturated Na₂CO₃ (10 mL) was added. DCM (25 mL×3) was added to extract the desired compound. The combined organic layer was washed with brine (10 mL), dried over anhydrous Na₂SO₄ and it was concentrated. The residue was purified by preparation. HPLC(prep-2 Xbridge C18 5um 19 * 150mm, 30-55%B; A: H₂O(0.1%NH₄HCO₃), B: CH₃CN; 214nm; flow rate: 15mL/min). The desired compound (135 mg, yield 67%) was obtained as a white solid. ¹H NMR (400 MHz, DMSO-*d*₆): δ 9.06 (s, 1H), 7.67 (d, *J=* 8.0 Hz, 2H), 7.46 (d, *J=* 8.0 Hz, 2H), 7.15-7.13 (m, 1H), 7.02-6.98 (m, 1H), 6.73 (d, *J=* 8.0 Hz, 1H), 6.58-6.54 (m, 1H), 4.66 (d, *J=* 7.6 Hz, 1H), 4.24 (d, *J=* 13.6 Hz, 1H), 3.94 (d, *J* = 13.6 Hz, 1H), 3.86 (s, 2H), 3.55-3.53 (m, 1H), 3.22 (t, *J=* 11.6 Hz, 1H), 2.88 (t, *J=* 10.8 Hz, 1H), 2.03 (s, 3H), 1.93-1.90 (m, 2H), 1.30-1.21 (m, 2H). LCMS (ESI): Rt = 3.654 min, *m*/*z* 420.2 [M+H]⁺; Purity: 99.91% @ 254 nm, 98.94% @ 214 nm.

### Example 60: 1-(1-(2-(4-(trifluoromethyl)phenyl)acetyl)piperidin-4-yl)-1H-indazole-3(2H)-one (GEN1-058)

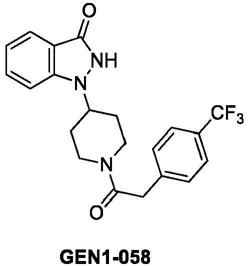

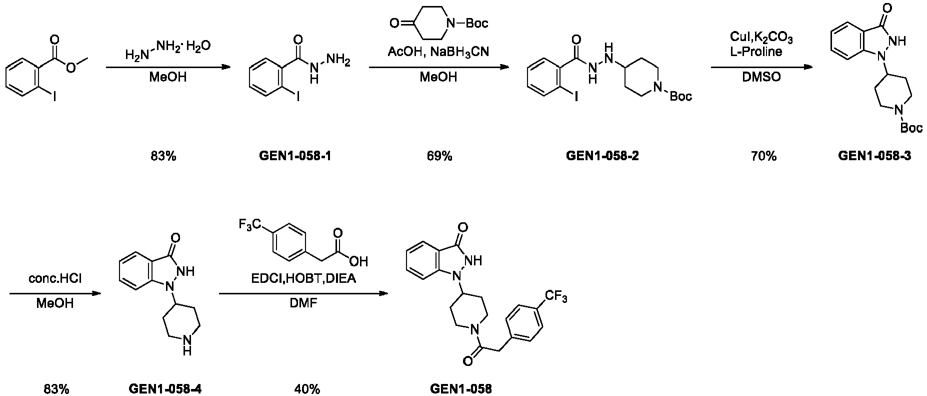

### Step 1. Synthesis of 2-iodobenzohydrazine (GEN1-058-1)

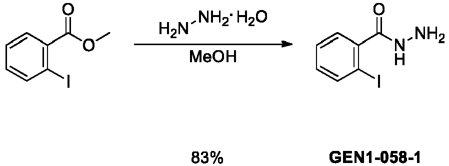

A solution of compound methyl 2-iodobenzoate (3.0 g, 11 mmol) and aqueous solution of hydrazine hydrate (85%, 3.37 g, 57.2 mmol) in methanol (30 mL) was refluxed for 5 hours. The mixture was cooled and concentrated. The residue was triturated with water (20 mL) and filtered to obtain the desired compound (2.5 g, yield 83%) as a white solid. ¹H NMR (400 MHz, DMSO-*d*₆): δ 9.50 (s, 1H), 7.88 (dd, *J* = 7.6, 1.2 Hz, 1H), 7.45-7.41 (m, 1H), 7.29-7.27 (m, 1H), 7.19-7.14 (m, 1H), 4.45 (s, 2H). MS (ESI): Rt = 0.83 min, *m*/*z* 263.0 [M+H]⁺; Purity: 100% @ 254 nm, 100% @ 214 nm.

### Step 2. Synthesis of tert-butyl 4-(2-(2-iodobenzoyl)hydrazino)piperidin-1-carboxylate (GEN1-058-2)

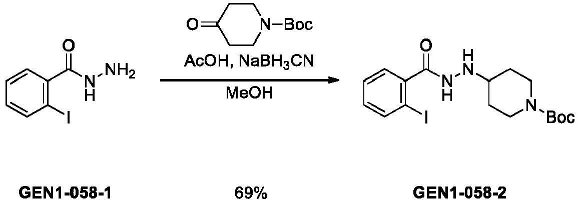

AcOH (275 mg, 4.58 mmol) was added to a solution of compound 2-iodobenzhydrazide (600 mg, 2.29 mmol) and tert-butyl 4-oxopiperidine-1-carboxylate (502 mg, 2.52 mmol) in methanol (10 mL). After the mixture was stirred at room temperature for 1 hour, NaBH₃CN (288 mg, 4.58 mmol) was added in portions in an ice bath. The mixture was stirred at room temperature overnight. The reaction mixture was diluted with water (50 mL), and extracted with EtOAc (30 mL×2). The organic phase was washed with brine (30 mL), dried over sodium sulfate and it was concentrated. The residue was purified by silica gel column chromatography (PE:EA = 5:1) to obtain the desired compound (700 mg, yield 69%) as a white solid. ¹H NMR (400 MHz, CDCl₃): δ 7.88 (d, *J=* 7.6 Hz, 1H), 7.42-7.37 (m, 2H), 7.16-7.12 (m, 1H), 4.05-4.02 (m, 2H), 3.25-3.20 (m, 1H), 2.88 (t*, J=* 11.6 Hz, 2H), 1.94-1.77 (m, 4H), 1.46 (s, 9H).

### Step 3. Synthesis of tert-butyl 4-(3-oxo-2,3-dihydro-1H-indazole-1-yl)piperidin-1-carboxylat (GEN1-058-3)

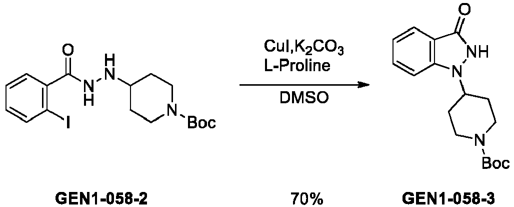

DMSO (10mL), L-proline (36 mg, 0.31 mmol) was added to a mixture of tert-butyl 4-[2-(2-iodobenzoyl)hydrazino]piperidin-1-carboxylate (700 mg, 1.57 mmol), CuI (60 mg, 0.31 mmol) and K₂CO₃ (435 mg, 3.14 mmol). The mixture was stirred at room temperature for 1 hour. Water (30 mL) was added to the mixture, and then extracted with DCM (10 mL×3). The organic phase was concentrated, and the residue was purified by C18 column chromatography (CH₃CN: water = 40% to 80%) to obtain the desired compound (350 mg, yield 70%) as a yellow solid. ¹H NMR (400 MHz, DMSO-*d*₆): δ 10.61 (s, 1H), 7.59 (dd, *J*= 8.0, 1.2 Hz, 1H), 7.50 (d, *J=* 8.4 Hz, 1H), 7.32 (t, *J=* 7.6 Hz, 1H), 6.98 (t, *J* = 7.6 Hz, 1H), 4.61-4.57 (m, 1H), 4.08-4.05 (m, 2H), 2.99-2.87 (m, 2H), 1.84-1.80 (m, 4H), 1.42 (s, 9H). MS (ESI): Rt = 1.50 min, *m*/*z* 318.4 [M+H]⁺; Purity: 100% @ 254 nm, 100% @ 214 nm.

### Step 4. Synthesis of 1-(piperidin-4-yl)-1H-indazole-3(2H)-one (GEN1-058-4)

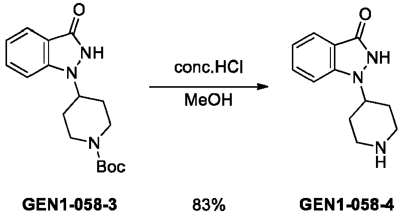

Concentrated HCl (1 mL 36% purity) was a added to a solution of tert-butyl 4-(3-oxo-2H-indazole-1-yl)piperidin-1-carboxylate (350 mg, 1.10 mmol) in methanol (3 mL). The mixture was stirred at room temperature for 1 hour. The mixture was concentrated. The residue was diluted with water (1 mL), and an aqueous Na₂CO₃ solution (1 mL saturated) was added. The mixture was stirred for 1 minute and filtered. The solid was dried to obtain the desired compound (200 mg, 83% yield) as a yellow solid. MS (ESI): Rt = 0.89 min, *m*/*z* 218.2 [M+H]⁺; Purity: 100% @ 214 nm.

### Step 5. Synthesis of 1-(1-(2-(4-(trifluoromethyl)phenyl)acetyl)piperidin-4-yl)-1H-indazole-3(2H)-one (GEN1-058)

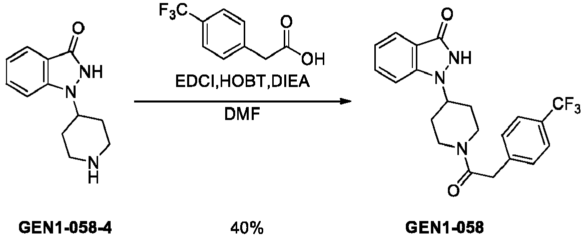

EDCI (265 mg, 1.38 mmol) and HOBT (187 mg, 1.38 mmol) were added to a solution of 1-(4-piperidinyl)-2H-indazole-3-one (200 mg, 0.920 mmol), 2-[4-(trifluoromethyl)phenyl]acetic acid (207 mg, 1.01 mmol) and DIEA (357 mg, 2.76 mmol) in DMF (5 mL). The mixture was stirred at room temperature overnight, the reaction mixture was diluted with water (20 mL), and extracted with DCM (10 mL×3). The combined organic phase was concentrated, and the residue was subjected to preparative HPLC [gilson-2, Xbridge C18, 5 um, 19 * 150 mm, 40∼80% B, A: H₂O(0.1% NH₄HCO₃), B: ACN, flow rate: 15 mL/min, UV = 214 nm] to obtain the desired compound (150 mg, yield 40%) as a white solid. ¹H NMR (400 MHz, DMSO-*d*₆): δ 10.59 (s, 1H), 7.69 (d, *J=* 8.0 Hz, 2H), 7.60 (d, *J=* 8.0 Hz, 1H), 7.50-7.48 (m, 3H), 7.32 (t, *J*= 7.2 Hz, 1H), 6.98 (t, *J*= 7.2 Hz, 1H), 4.69-4.63 (m, 1H), 4.52 (d, *J*= 12.8 Hz, 1H), 4.12 (d, *J=* 13.6 Hz, 1H), 3.90 (s, 2H), 3.24-3.21 (m, 1H), 2.83-2.76 (m, 1H), 1.84-1.78 (m, 4H). MS (ESI): Rt = 3.224 min, *m*/*z* 404.2 [M+H]⁺; Purity: 97.20% @ 254 nm, 99.50% @ 214 nm.

### Example 61: 1-hydroxy-3-(1-(2-(4-(trifluoromethyl)phenyl)acetyl)piperidin-4-yl)-1H-benzo[d]imidazol-2(3H)-one (GEN1-059)

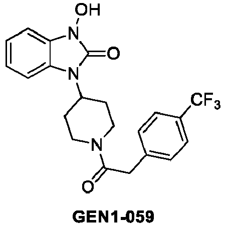

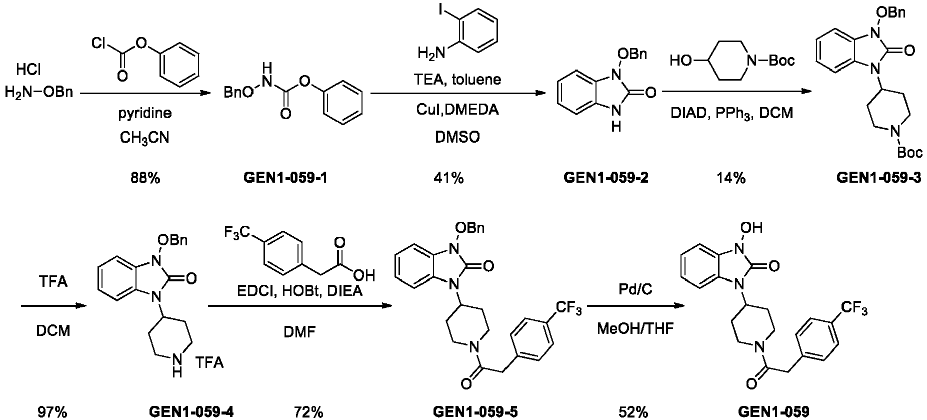

### Step 1. Synthesis of Phenylbenzyloxy carbamate (GEN1-059-1)

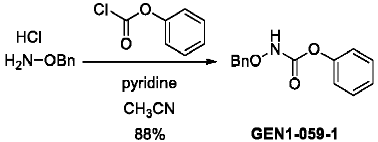

In an ice bath, phosgene phenyl ester (4.90 g, 31.3 mmol) was added dropwise to a solution of O-benzylhydroxylamine hydrochloride (5.0 g, 31 mmol) and pyridine (4.96 g, 62.7 mmol) in CH₃CN (50 mL). The reaction mixture was stirred at room temperature overnight, and then poured into water (150 mL), and extracted with EtOAc (50 mL×3). The combined organic layer was washed with brine (50 mL), dried over MgSO₄ and filtered. The filtrate was concentrated, and the residue was purified by column chromatography on silica gel (PE: EtOAc = 5:1) to obtain the desired compound (7.5 g, yield 88%) as a colorless oil. MS (ESI): Rt = 1.52 min, *m*/*z* 242.2 [M-H]⁻; Purity: 89% @ 254 nm, 49% @ 214 nm.

### Step 2. Synthesis of 1-(benzyloxy)-1H-benzo[d]imidazol-2(3H)-one (GEN1-059-2)

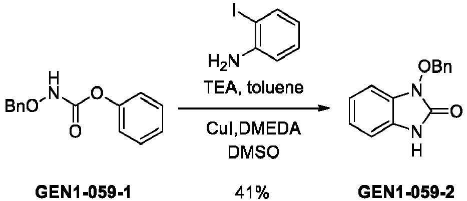

TEA (1.87 g, 18.5 mmol) was added to a suspension of phenyl N-benzyloxycarbamate (1.5 g, 6.2 mmol) and 2-iodoaniline (1.42 g, 6.47 mmol) in toluene (10 mL). The resulting mixture was stirred at 110°C for 2 hours. The reaction mixture was cooled to room temperature, and then concentrated and the residue was dissolved in DMSO (10 mL). TEA (1.25 g, 12.4 mmol), CuI (118 mg, 0.620 mmol) and DMEDA (109 mg, 1.24 mmol) were added to the solution. The resulting mixture was stirred at 110°C for 2 hours. The reaction mixture was cooled to room temperature, and then poured into water (50 mL), and extracted with EtOAc (30 mL×3). The combined organic layer was washed with brine (30 mL), dried over MgSO₄ and filtered. The filtrate was concentrated, and the crude product was purified by column chromatography on silica gel (PE:EtOAc = 5:1 to 1:1) to obtain the desired compound (660 mg, yield 41%) as a white solid. MS (ESI): Rt = 1.45 min, *m*/*z* 241.1 [M+H]⁺; Purity: 78% @ 254 nm, 92% @ 214 nm.

### Step 3. Synthesis of tert-butyl 4-(3-(benzyloxy)-2-oxo-2,3-dihydro-1H-benzo[d]imidazol-1-yl)piperidin-1-carboxylate (GEN1-059-3)

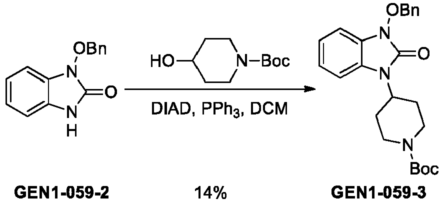

DIAD (569 mg, 2.81 mmol) was added to a solution of PPh3 (735 mg, 2.80 mmol) in DCM (8 mL) under N2 atmosphere in an ice bath. After the mixture was stirred for 10 minutes, a solution of 3-benzyloxy-1H-benzimidazol-2-one (450 mg, 1.87 mmol) and tert-butyl 4-hydroxypiperidine-1-carboxylate (567 mg, 2.82 mmol) in DCM (2 mL) was added dropwise, and it was stirred at room temperature overnight. The mixture was then concentrated, and the crude product was purified by a C18 column (CH₃CN: water = 50% to 70%) to obtain the desired compound (120 mg, yield 14%) as a colorless oil. ¹HNMR (400 MHz, CDCl₃): δ 7.51-7.48 (m, 2H), 7.38-7.37 (m, 3H), 7.09-7.07 (m, 1H), 7.03-6.98 (m, 2H), 6.88-6.86 (m, 1H), 5.24 (s, 2H), 4.49-4.39 (m, 1H), 4.32 (br s, 2H), 2.93-2.76 (m, 2H), 2.36-2.26 (m, 2H), 1.84-1.81 (m, 2H), 1.50 (s, 9H).

### Step 4. Synthesis of 1-(benzyloxy)-3-(piperidin-4-yl)-1H-benzo[d]imidazol-2(3H)-one trifluoroformat (GEN1-059-4)

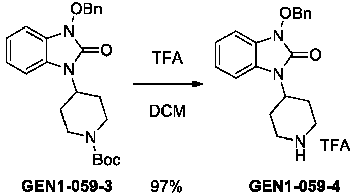

TFA (1 mL) was added to a solution of tert-butyl 4-(3-benzyloxy-2-oxy-benzimidazol-1-yl)piperidin-1-carboxylate (100 mg, 0.236 mmol) in DCM (4 mL). The reaction mixture was stirred at room temperature overnight. The reaction mixture was concentrated to obtain the desired compound (100 mg, yield 97%) as a colorless oil. MS (ESI): Rt = 1.40 min, *m*/*z* 324.4 [M+H]⁺; Purity: 93% @ 254 nm, 100% @ 214 nm.

### Step 5. Synthesis of 1-(benzyloxy)-3-(1-(2-(4-(trifluoromethyl)phenyl)acetyl)piperidin-4-yl)-1H-benzo[d]imidazol-2(3H)-one (GEN1-059-5)

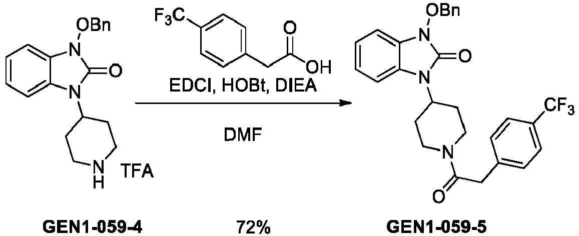

In an ice bath, EDCI (66 mg, 0.34 mmol) was added to a mixture of 1-(benzyloxy)-3-(piperidin-4-yl)-1H-benzo(d)imidazol-2(3H)-one (100 mg, 0.229 mmol), 2-[4-(trifluoromethyl), phenyl]acetic acid (56 mg, 0.27 mmol), HOBT (46 mg, 0.34 mmol) and DIEA (148 mg, 1.15 mmol) in DMF (3mL). The resulting mixture was stirred at room temperature overnight. The reaction mixture was poured into water (50 mL), and extracted with EtOAc (30 mL×3). The combined organic layer was washed with brine (30 mL), dried over MgSO₄ and filtered. The filtrate was concentrated to obtain the desired compound (120 mg, purity 70%, yield 72%) as a brown oil. MS (ESI): Rt = 1.71 min, *m*/*z* 510.5 [M+H]⁺; Purity: 70% @ 254 nm, 31% @ 214 nm.

### Step 6. Synthesis of 1-hydroxy-3-(1-(2-(4-(trifluoromethyl)phenyl)acetyl)piperidin-4-yl)-1H-benzo[d]imidazol-2(3H)-one (GEN1-059)

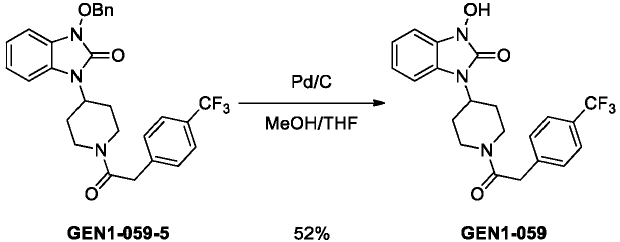

Pd/C (10%, 18 mg, 0.017 mmol) in THF(1mL) was added to a suspension of 1-benzyloxy-3-[1-[2-[4-(trifluoromethyl)phenyl]acetyl]-4-piperidinyl]benzimidazol-2-one (120 mg, 0.165 mmol) in MeOH (5 mL). The resulting mixture was stirred under H₂ atmosphere overnight. The reaction mixture was filtered and the filtrate was concentrated. The crude product was purified by a C18 column (CH₃CN: water = 5% to 60%) to obtain the desired compound (36.7 mg, yield 52%) as a white solid. ¹HNMR (400 MHz, DMSO-*d*₆): δ 10.00 (br s, 1H), 7.71 (d, *J=* 8.0 Hz, 2H), 7.52 (d, *J=* 8.0 Hz, 2H), 7.16 (d, *J=* 8.0 Hz, 1H), 7.10-6.99 (m, 3H), 4.59-4.56 (m, 1H), 4.51-4.42 (m, 1H), 4.15-4.11 (m, 1H), 3.97-3.88 (m, 2H), 3.23-3.16 (m, 1H), 2.75-2.69 (m, 1H), 2.15-2.05 (m, 2H), 1.74-1.71 (m, 2H). MS (ESI): Rt = 3.762 min, *m*/*z* 420.1[M+H]⁺; Purity: 96.11% @ 254 nm, 97.72% @ 214 nm.

### Example 62: 2-(2-oxo-3-(1-(2-(4-(trifluoromethyl)phenyl)acetyl)piperidin-4-yl)-2,3-dihydro-1H-benzo[d]imidazol-1-yl acetic acid (GEN1-060)

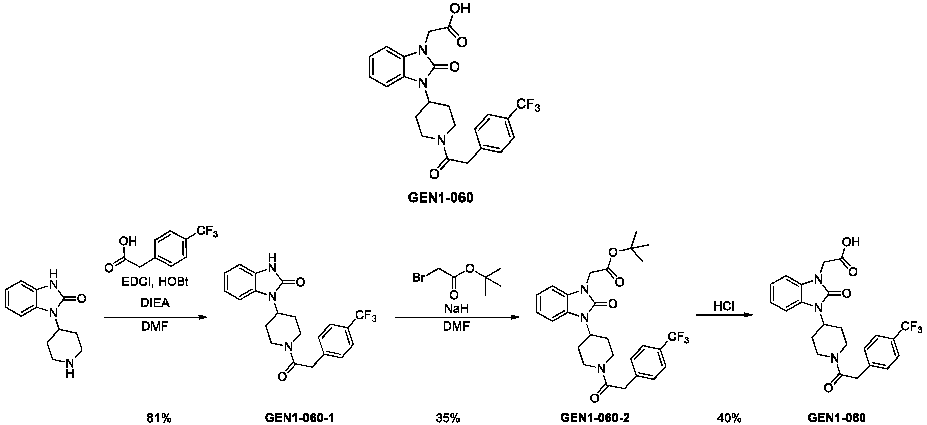

### Step 1. Synthesis of 1-(1-(2-(4-(trifluoromethyl)phenyl)acetyl)piperidin-4-yl)-1H-benzo[d]imidazol-2(3H)-one (GEN1-060-1)

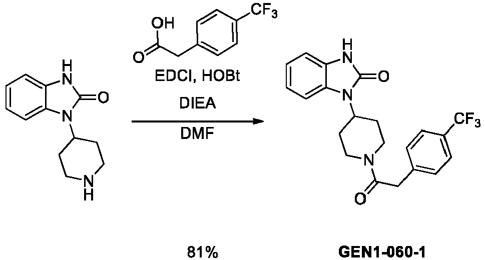

In an ice bath, EDCI (265 mg, 1.38 mmol) was added to a mixture of 3-(4-piperidinyl)-1H-benzimidazol-2-one (200 mg, 0.921 mmol), 2-[4-(trifluoromethyl)phenyl]acetic acid (207 mg, 1.01 mmol), HOBT (187 mg, 1.38mmol) DIEA (357mg, 2.76mmol) and DMF (4mL). The reaction mixture was stirred at room temperature overnight. The resulting mixture was purified by a C18 column (CH₃CN: water = 5% to 55%) to obtain the desired compound (300 mg, yield 81%) as a brown solid. MS (ESI) Rt = 1.48 min, *m*/*z* 404.3 [M+H]⁺; Purity: 100% @ 254 nm, 100% @ 214 nm.

### Step 2. Synthesis of tert-butyl 2-(2-oxo-3-(1-(2-(4-(trifluoromethyl)phenyl)acetyl)piperidin-4-yl)-2,3-dihydro-1H-benzo[d]imidazol acetic acid-1-yl ester (GEN1-060-2)

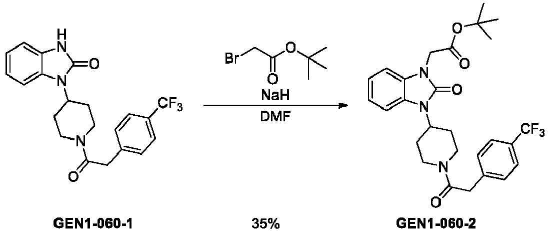

In an ice bath, NaH (21 mg, 0.53 mmol, 60% in mineral oil) was added to a solution of 1-(1-(2-(4-(tris(trifluoromethyl)phenyl)acetyl)piperidin-4-yl)-1H-benzo[d]imidazol-2(3H)-one (140 mg, 0.347 mmol) in DMF (3 mL). The resulting mixture was stirred at room temperature for 15 minutes. Then tert-butyl 2-bromoacetate (81 mg, 0.42 mmol) was added dropwise. The resulting mixture was stirred at room temperature overnight. The reaction mixture was poured into water (30 mL), and extracted with EtOAc (20 mL×3). The combined organic layer was washed with brine (20 mL), dried over MgSO₄, filtered and it was concentrated. The crude product was purified by preparation. TLC(EtOAc). The desired compound (70 mg, yield 35%) was obtained as a brown oil. MS (ESI): Rt = 1.69 min, m/z 462 [M+H-56]⁺; Purity: 19% @ 254 nm, 79% @ 214 nm.

### Step 3. Synthesis of 2-(2-oxo-3-(1-(2-(4-(trifluoromethyl)phenyl)acetyl)piperidin-4-yl)-2,3-dihydro-1H-benzo[d]imidazol-1-yl acetic acid (GEN1-060)

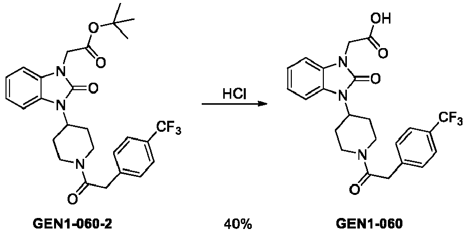

A solution of tert-butyl 2-[2-oxo-3-[1-[2-[4-(trifluoromethyl)phenyl]acetyl]-4-piperidinyl]benzimidazol-1-yl] acetate (70 mg, 0.14 mmol) was stirred in a solution of HCl/EtOAc (4 mL, 4 M) at room temperature overnight. The reaction mixture was concentrated. The residue was purified by a C18 column (CH₃CN: water = 5% to 50%) to obtain the desired compound (25.1 mg, yield 40%) as a white solid. ¹HNMR (400 MHz, DMSO-*d*₆): δ 7.71 (d, *J=* 8.0 Hz, 2H), 7.53 (d, *J=* 8.0 Hz, 2H), 7.11-7.09 (m, 1H), 7.05-6.97 (m, 3H), 4.59-4.56 (m, 1H), 4.51-4.45 (m, 1H), 4.36 (s, 2H), 4.16-4.12 (m, 1H), 3.97-3.87 (m, 2H), 3.24-3.17 (m, 1H), 2.75-2.69 (m, 1H), 2.15-2.05 (m, 2H), 1.73-1.66 (m, 2H). MS (ESI): Rt = 2.952 min, m/z 462.2 [M+H]⁺; Purity: 89.76% @ 254 nm, 99.42% @ 214 nm.

### Example 63: N-(1-(1-(1-(2-(4-(trifluoromethyl)phenyl)acetyl)piperidin-4-yl)-1H-benzo[d]imidazol-2-yl)cyanamide (GEN1-061)

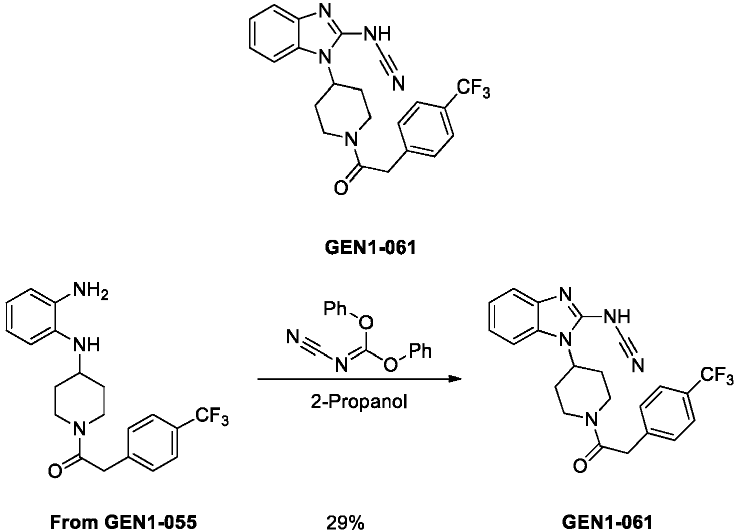

### Step 1. Synthesis of N-(1-(1-(1-(2-(4-(trifluoromethyl)phenyl)acetyl)piperidin-4-yl)-1H-benzo[d]imidazol-2-yl)cyanamide (GEN1-061)

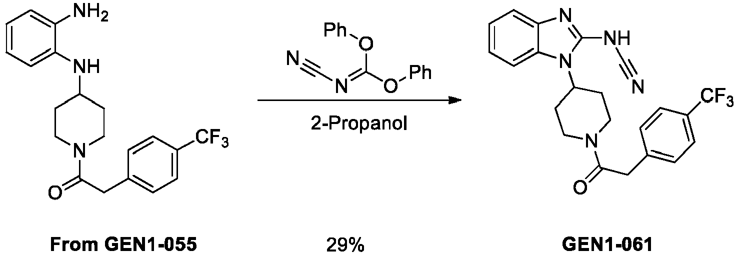

1-[4-(2-aminoanilino)-1-piperidinyl]-2-[4-(trifluoromethyl)phenyl]ethenone (445 mg, 1.18 mmol, the synthesis steps could be found in GEN1-055), triethylamine (359 mg, 3.55 mmol), 2-propanol (10 mL) and diphenoxymethylene cyanamide (282 mg, 1.18 mmol) were mixed and refluxed for 12 hours. The reaction mixture was cooled to room temperature, and then poured into water (15 mL), and extracted with DCM (10 mL) three times. The combined organic layer was washed with brine (10 mL), dried over Na₂SO₄ and it was concentrated to obtain the crude product. The crude product was purified by a C18 column (CH₃CN: water = 5% to 45%) to obtain the desired compound (150 mg, yield 29%) as a white solid. ¹H NMR (400 MHz, CDCl₃): δ 11.96 (br s, 1H), 7.63 (d, *J=* 8.4 Hz, 2H), 7.46 (d, *J=* 8.0 Hz, 2H), 7.30 (d, *J=* 7.6 Hz, 1H), 7.18 (t, *J* = 7.6 Hz, 1H), 7.12 (t, *J* = 7.6 Hz, 1H), 7.01 (d, *J* = 7.6 Hz, 1H), 4.90 (d, *J* = 13.2 Hz, 1H), 4.66-4.55 (m, 1H), 4.06 (d, *J=* 13.6 Hz, 1H), 3.92-3.84 (m, 2H), 3.21 (t, *J*= 12.8 Hz, 1H), 2.72 (t, *J* = 12.8 Hz, 1H), 2.37-2.26 (m, 1H), 2.13-2.03 (m, 1H), 1.88 (d, *J* = 11.6 Hz, 1H), 1.79 (d, *J=* 11.6 Hz, 1H). MS (ESI): Rt = 3.447 min, *m*/*z* 428.2, [M+H]⁺; Purity: 98.64% @ 254 nm, 99.84% @ 214 nm.

### Example 64: N-(2-(2-oxo-2,3-dihydro-1H-benzo[d]imidazol-1-yl)ethyl)-2-(4-(trifluoromethyl)phenyl)acetamide (GEN1-062)

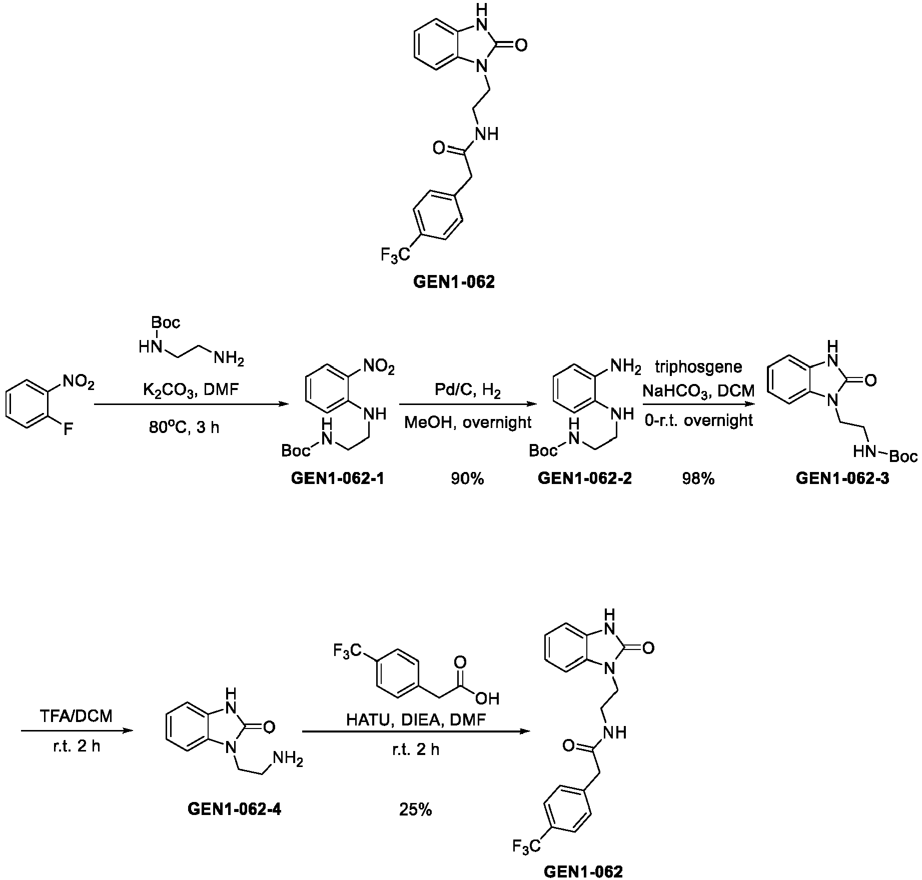

### Step 1. Synthesis of tert-butyl (2-(((2-nitrophenyl)amino)ethyl)carbamate (GEN1-062-1)

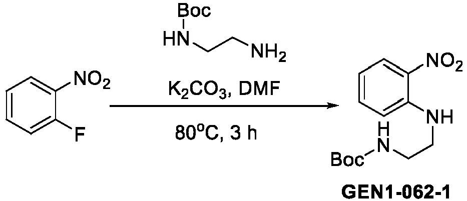

A mixture of 1-fluoro-2-nitrobenzene (500 mg, 2.39 mmol), tert-butyl (2-aminoethyl)carbamate (479 mg, 2.39 mmol), K₂CO₃ (495 mg, 3.58 mmol) and KI (38 mg, 0.24 mmol) in DMF(10 mL) was stirred at 80°C for 3 hours. After it was cooled to room temperature. The resulting mixture was filtered, and the filter cake was washed with EA. The filtrate was concentrated under reduced pressure. The crude product (1.12 g crude product) was used directly in the next step without further purification. TLC: EA/(PE+EA)=5%, Rf=0.2.

### Step 2. Synthesis of tert-butyl (2-(((2-aminophenyl)amino)ethyl) carbamate (GEN1-062-2)

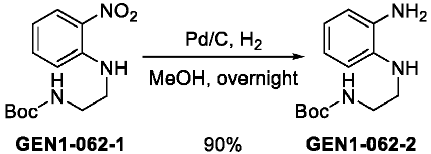

Pd/C (120 mg) was added to a solution of tert-butyl (2-(((2-nitrophenyl)amino)ethyl)carbamate (1.12g crude product) in MeOH (20 mL) under N₂ atmosphere. The resulting mixture was degassed with H₂ and stirred at room temperature under an H₂ atmosphere overnight. The reaction mixture was filtered, and the filter cake was washed with MeOH (50 mL×3). The filtrate was concentrated under reduced pressure. The crude product was purified by silica gel column chromatography and eluted with (EA / (PE + EA) = 20%) to obtain GEN1-062-2 (800 mg, 90% yield) as a purple solid. TLC: EA/(PE+EA)=20%, Rf=0.2.

### Step 3. Synthesis of tert-butyl (2-(2-oxo-2,3-dihydro-1H-benzo[d]imidazol-1-yl)ethyl)carbamate (GEN1-062-3)

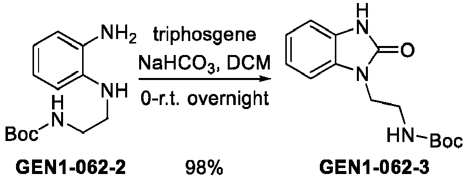

Triphosgene (141 mg, 0.48 mmol) was added in batches to a solution of tert-butyl (2-(((2-aminophenyl)amino)ethyl carbamate (300 mg, 1.20 mmol) and DIEA (309 mg, 2.40 mmol) in EA (12 mL) at 0°C under N₂ atmosphere. The mixture was stirred overnight at room temperature. The reaction mixture was poured into water (20 mL), and extracted with DCM (10 mL×3). The organic layer was washed with water (20 mL), brine (20 mL), dried over anhydrous sodium sulfate and it was concentrated to obtain a pale yellow solid. The crude product was slurried with PE to obtain GEN1-062-3 (324 mg, 98% yield) as a white solid. TLC: EA/(PE+EA)=60%, Rf=0.3

### Step 4. Synthesis of 1-(2-aminoethyl)-1,3-dihydro-2H-benzo[d]imidazol-2-one (GEN1-062-4)

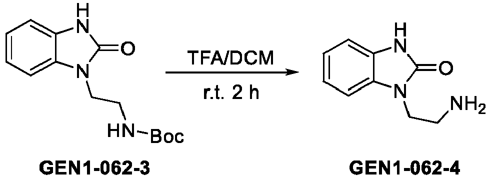

A solution of TFA (1.5 mL) in DCM (2.0 mL) was added dropwise to a mixture of tert-butyl (2-(2-oxo-2,3-dihydro-1H-benzo[d]imidazol-1-yl)ethyl)carbamate (150 mg, 0.54 mmol) and H₂O (0.1 mL) at 0°C under N₂ atmosphere. The resulting mixture was stirred at room temperature under N₂ atmosphere for 2 hours. The resulting mixture was concentrated under vacuum. GEN1-062-4 (165 mg, crude product) was obtained as a white solid. TLC: MeOH/(DCM+MeOH)=5%, Rf=0.2

### Step 5. Synthesis of N-(2-(2-oxo-2,3-dihydro-1H-benzo[d]imidazol-1-yl)ethyl)-2-(4-(trifluoromethyl)phenyl)acetamide (GEN1-062)

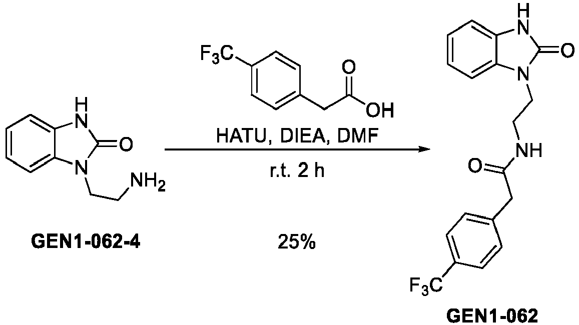

DIEA (348 mg, 2.70 mmol) was added to a solution of 1-(2-aminoethyl)-1,3-dihydro-2H-benzo[d]imidazol-2-one (96 mg, 0.54 mmol), 2-(4-(trifluoromethyl)phenyl)acetic acid (110mg, 0.54mmol) and HATU (380 mg, 0.81 mmol) in DMF (3.0 mL). The resulting mixture was stirred at room temperature for 5 hours. The reaction mixture was added dropwise to water (80 mL). The precipitated solid was collected by filtration. The crude product was recrystallized from THF to obtain GEN1-062 (50 mg, 25% yield) as a white solid. ¹H NMR (400 MHz, DMSO-*d*₆): δ 10.83 (s, 1H), 8.28 (t, *J* = 5.9 Hz, 1H), 7.61 (d, *J* = 8.0 Hz, 2H), 7.36 (d, *J* = 8.0 Hz, 2H), 7.07 - 6.99 (m, 1H), 6.99 - 6.92 (m, 3H), 3.83 (t, *J=* 6.1 Hz, 2H), 3.43 (s, 2H), 3.39 - 3.31 (m, 2H). MS (ESI): Rt = 1.43 min, *m*/*z* 363.9 [M + H]⁺, Purity: 100% @ 254 nm, 100% @ 214 nm.

### Example 65: N-(3-(2-oxo-2,3-dihydro-1H-benzo[d]imidazol-1-yl)propyl)-2-(4-(trifluoromethyl)phenyl)acetamide

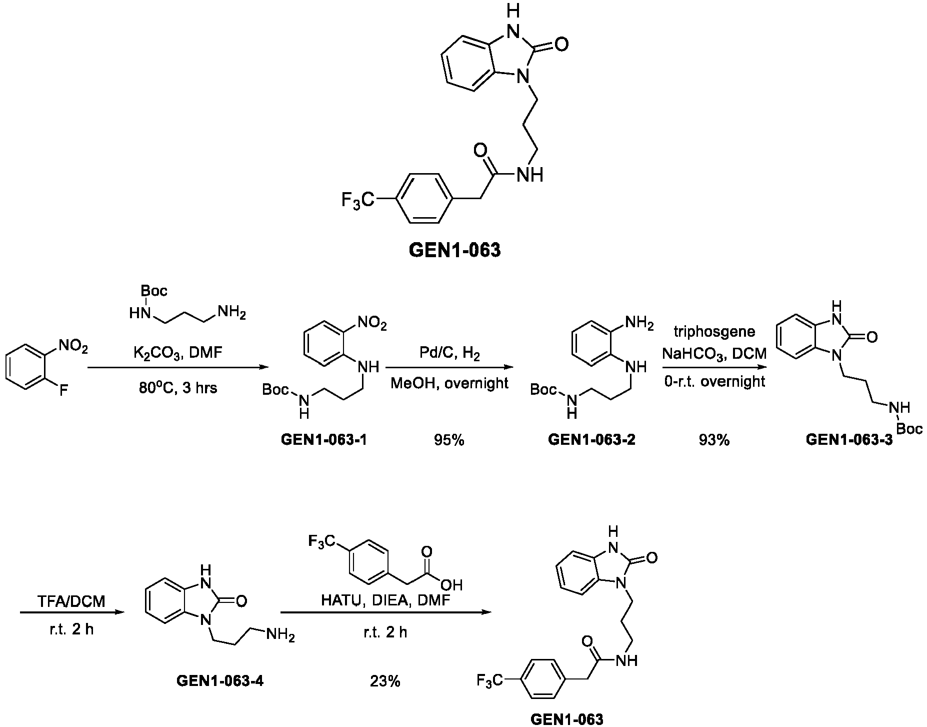

### Step 1. Synthesis of tert-butyl 3-(((2-nitrophenyl)amino)propyl)carbamate (GEN1-063-1)

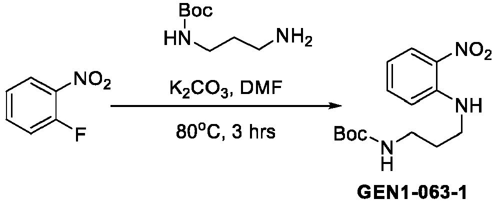

A solution of 1-fluoro-2-nitrobenzene (500 mg, 2.39 mmol), tert-butyl (3-aminopropyl) carbamate (617 mg, 2.39 mmol), K₂CO₃ (734 mg, 3.58 mmol) and KI(58 mg, 0.24 mmol) in DMF (10 mL) was stirred at 80°C for 3 hours. After the reaction mixture was cooled to room temperature, the resulting mixture was filtered, and the filter cake was washed with EA. The filtrate was concentrated under reduced pressure. The crude product (1.24 g crude product) was used directly in the next step without further purification. TLC: EA/(EA+PE)=5%, Rf=0.2

### Step 2. Synthesis of tert-butyl 3-(((2-aminophenyl)amino)propyl)carbamate (GEN1-063-2)

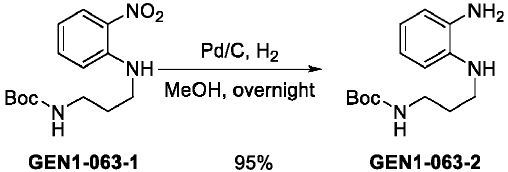

Pd/C (120 mg) was added to a solution of tert-butyl 3-(((2-nitrophenyl)amino)propyl carbamate (1.24 g crude product) in MeOH (30 mL) under N₂ atmosphere. The resulting mixture was degassed with H₂ and stirred at room temperature under an H₂ atmosphere overnight. The reaction mixture was filtered, and the filter cake was washed with MeOH (50 mL×3). The filtrate was concentrated under reduced pressure. The crude product was purified by silica gel column chromatography and eluted with (EA / (PE + EA) = 25%) to obtain GEN1-063-2 (900 mg cude, 96% yield) as a purple solid. TLC: EA/(EA+PE)=30%, Rf=0.4

### Step 3. Synthesis of tert-butyl 2-(2-oxo-2,3-dihydro-1H-benzo[d]imidazol-1-yl)ethyl)carbamate (GEN1-063-3)

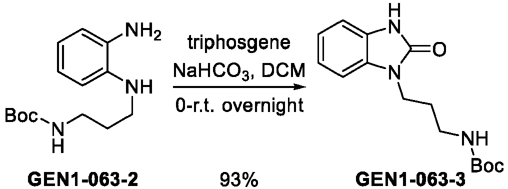

Triphosgene (420 mg, 1.43 mmol) was added in batches to a solution of tert-butyl 3-(((2-aminophenyl)amino)propyl carbamate (900 mg, 3.39 mmol) and DIEA (920 mg, 7.16 mmol) in EA (40 mL) at 0°C under N₂ atmosphere. The resulting mixture was stirred at room temperature under N₂ atmosphere overnight. The reaction mixture was poured into water (40 mL), and extracted with EA(30 mL×3). The organic layer was washed with water (50 mL), brine (50 mL), dried over sodium sulfate and it was concentrated to obtain a pale yellow solid. The crude product was treated with PE to obtain GEN1-063-3 (923 mg, 93% yield) as a white solid. MS (ESI): Rt = 1.40 min, *m*/*z* 192.1 [M - 100 + H]⁺, Purity: 82% @ 254 nm, 78% @ 214 nm.

### Step 4. Synthesis of 1-(3-aminopropyl)-1,3-dihydro-2H-benzo[d]imidazol-2-one (GEN1-063-4)

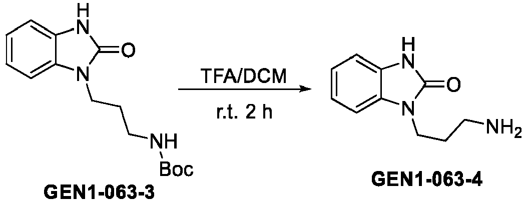

Tert-butyl 2-(2-oxo-2,3-dihydro-1H-benzo[d]imidazol-1-yl)ethyl)carbamate (200 mg, 0.69 mmol) and H₂O (0.2 mL) were mixed in DCM (2.0 mL), and TFA (2.0 mL) was added to the mixture at 0°C under N₂ atmosphere. The resulting mixture was stirred at room temperature under N₂ atmosphere for 2 hours. The resulting mixture was concentrated under vacuum. The crude product (220 mg crude product) was used directly in the next step without further purification. TLC: MeOH/(DCM+MeOH)=5%, Rf=0.2

### Step 5. Synthesis of N-(3-(2-oxo-2,3-dihydro-1H-benzo[d]imidazol-1-yl)propyl)-2-(4-(trifluoromethyl)phenyl)acetamide (GEN1-063)

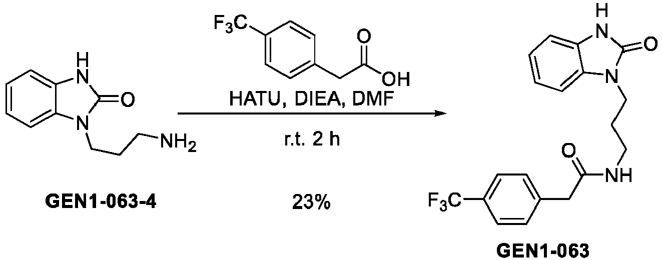

DIEA (352 mg, 2.74 mmol) was added to a solution of 1-(3-aminopropyl)-1,3-dihydro-2H-benzo[d]imidazol-2-one (220 mg crude), 2-(4-(trifluoromethyl)phenyl)acetic acid (140 mg, 0.69 mmol) and HATU (392 mg, 1.03 mmol) in DMF(4 mL). The resulting mixture was stirred at room temperature for 2 hours. The reaction mixture was added dropwise to water (40 mL). The precipitated solid was collected by filtration. The residue was purified by reversed-phase column chromatography under the following conditions: column, C18 silica gel; mobile phase ACN in water, a gradient was 5% to 80% within 40 minutes; UV wavelength was detected at 214 nm. GEN1-063 was obtained as a white solid (60 mg, 23% yield). ¹H NMR (400 MHz, DMSO-*d*₆): δ 10.84 (s, 1H), 8.20 (t, *J=* 5.5 Hz, 1H), 7.66 (d, *J=* 8.0 Hz, 2H), 7.49 (d, *J=* 8.0 Hz, 2H), 7.06-7.00 (m, 1H), 6.99-6.93 (m, 3H), 3.78 (t, *J* = 7.1 Hz, 2H), 3.34 (s, 2H), 3.13-3.03 (m, 2H), 1.82-1.72 (m, 2H). MS (ESI): Rt = 1.43 min, *m*/*z* 378.2 [M + H]⁺, Purity: 100% @ 254 nm, 100% @ 214 nm.

### Example 66: tert-butyl ((2-oxo-2,4,5,6-tetrahydro-1H-imidazo[4,5,1-ij]quinolin-5-yl)methyl)carbamate

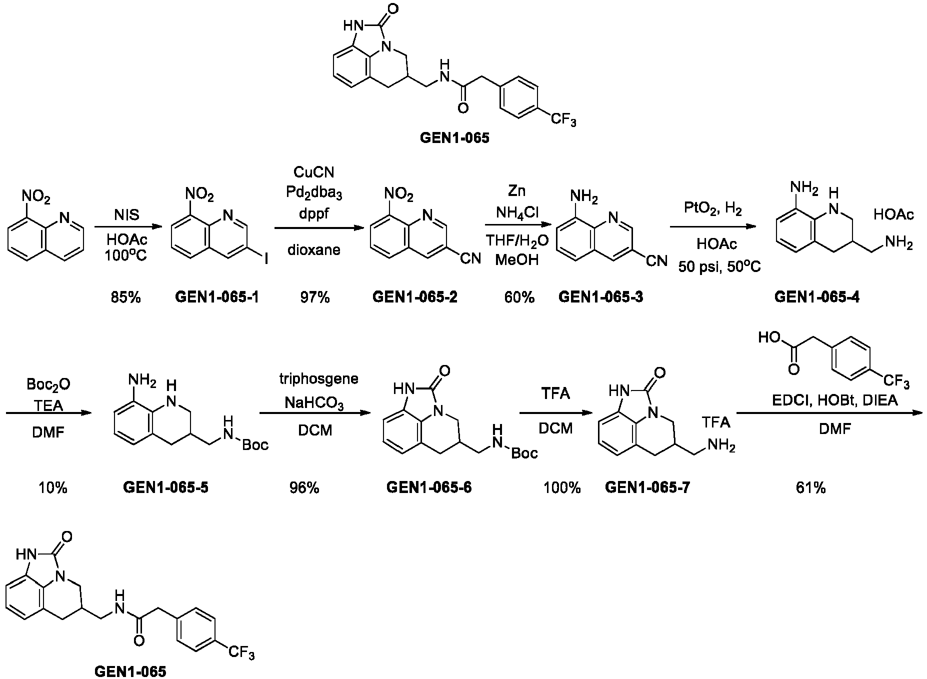

### Step 1. Synthesis of 3-iodo-8-nitroquinoline (GEN1-065-1)

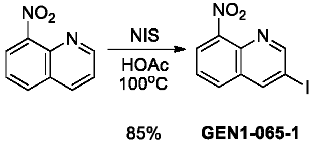

A suspension of 8-nitroquinoline (4.0 g, 23 mmol) and NIS (5.68 g, 25.3 mmol) in acetic acid (40 mL) was stirred at 105°C for 4 hours. The reaction mixture was cooled to room temperature and poured into water (200 mL). The precipitate was collected by filtration to obtain the desired compound (6.0 g, yield 85%) as an ocher solid. ¹HNMR (400 MHz, CDCl₃): δ 9.18 (d, *J* = 2.0 Hz, 1H), 8.66 (d, *J* = 2.0 Hz, 1H), 8.07 (dd, *J* = 7.6, 1.2 Hz, 1H), 7.94 (dd, *J* = 8.4, 1.2 Hz, 1H), 7.65 (t, *J* = 8.0 Hz, 1H).

### Step 2. Synthesis of 8-nitroquinoline-3-nitrile (GEN1-065-2)

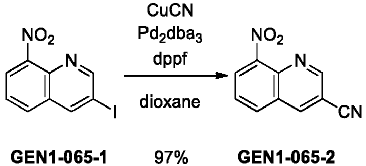

A solution of a suspension formed by 3-iodo-8-nitroquinoline (6.5 g, 22 mmol), CuCN (6.08 g, 65.0 mmol), Pd2(dba)3 (673 mg, 0.650 mmol) and dppf (1.44 g, 2.60 mmol) in dioxane (50 ml) was stirred at 105°C under N₂ atmosphere for 1 hour. The reaction mixture was concentrated and the residue was purified by column chromatography (DCM: MeOH = 10:1) to obtain the desired compound as a crude product. The crude product was triturated with EtOAc (20 mL) and filtered to obtain the desired compound (4.4 g, yield 97%) as an ocher solid. ¹HNMR (400 MHz, DMSO-*d*₆): δ 9.33 (d, *J* = 2.0 Hz, 1H), 9.31 (d, *J* = 2.0 Hz, 1H), 8.51 (dd, *J* = 7.6, 1.2 Hz, 1H), 8.40 (dd, *J =* 8.0, 1.2 Hz, 1H), 7.95 (t, *J* = 8.0 Hz, 1H).

### Step 3. Synthesis of 8-aminoquinoline-3-nitrile (GEN1-065-3)

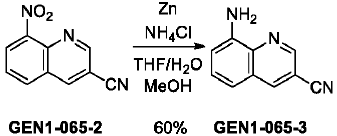

8-nitroquinoline-3-nitrile (4.4 g, 21 mmol), zinc powder (6.86 g, 105 mmol) and NH₄Cl (11.2 g, 209 mmol) were stirred in a suspension of MeOH (30 mL), THF (30 mL) and water (10mL) at 50°C for 3 hours. The reaction mixture was filtered, and the filtrate was poured into water (300 mL), and extracted with EtOAc (100 mL×3). The combined organic layer was washed with brine (100 mL), dried over MgSO₄, filtered and it was concentrated. The crude product was purified by column chromatography on silica gel (PE: EtOAc = 4: 1) to obtain the desired compound (2.3 g, yield 60%) as a brown solid. MS (ESI): Rt = 1.39 min, *m*/*z* 170.0 [M+H]⁺; Purity: 93% @ 254 nm, 100% @ 214 nm.

### Step 4. Synthesis of 3-(aminomethyl)-1,2,3,4-tetrahydroquinoline-8-amine (GEN1-065-4)

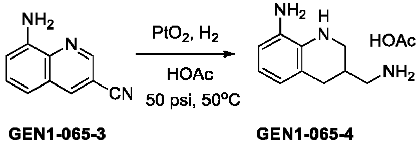

PtO2 (103 mg, 0.38 6mmol, 85% purity) was added to a suspension of 8-aminoquinoline-3-nitrile (1.3g, 7.7mmol) in acetic acid (20 mL). The resulting mixture was stirred at 50°C under H₂ atmosphere (50 psi) overnight. The reaction mixture was filtered and the filtrate was concentrated. The residue was purified by a C18 column (CH₃CN: water = 5 % to 10%) to obtain the desired compound (1.5 g, crude product) as a brown oil. MS (ESI): Rt = 0.29 min, *m*/*z* 178 [M+H]⁺; Purity: 95% @ 254 nm, 90% @ 214 nm.

### Step 5. Synthesis of tert-butyl ((8-amino-1,2,3,4-tetrahydroquinoline-3-yl)methyl)carbamate (GEN1-065-5)

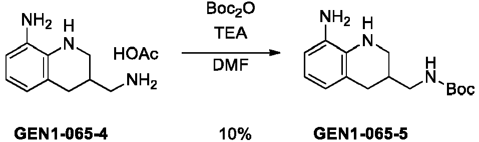

In an ice bath, Boc2O (517 mg, 2.35 mmol) was added dropwise to a solution of 3-(aminomethyl)-1,2,3,4-tetrahydroquinoline-8-amine (840 mg, 4.74 mmol) and TEA (1.44 g, 14.2 mmol) in DMF(10 mL). The resulting mixture was stirred at room temperature for 2 hours. The reaction mixture was poured into water (100 mL), and extracted with EtOAc (50 mL×3). The combined organic layer was washed with brine (50 mL), dried over MgSO₄, filtered and it was concentrated. The crude product was purified by preparation. TLC (DCM: MeOH = 15: 1). The desired compound (140 mg, yield 10%) was obtained as a colorless oil. MS (ESI): Rt = 1.48 min, *m*/*z* 278.2 [M+H]⁺; Purity: 81% @ 254 nm, 91% @ 214 nm.

### Step 6. Synthesis of tert-butyl ((2-oxo-2,4,5,6-tetrahydro-1H-imidazo[4,5,1-ij]quinolin-5-yl)methyl)carbamate (GEN1-065-6)

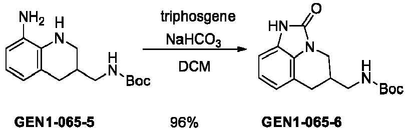

In an ice bath, triphosgene (54 mg, 0.18 mmol) was added to a solution of tert-butyl ((8-amino-1,2,3,4-tetrahydroquinoline-3-yl)methyl)carbamate (100 mg, 0.361 mmol) and NaHCO₃ (303 mg, 3.61 mmol) in DCM (7 mL). The resulting mixture was stirred at room temperature for 1 hour. The reaction mixture was poured into water (50 mL), and extracted with DCM (25 mL×3). The combined organic layer was washed with brine (25 mL), dried over MgSO₄, filtered and it was concentrated to obtain the desired compound (105 mg, yield 96%) as a white solid. MS (ESI): Rt = 1.42 min, *m*/*z* 304.3 [M+H]⁺; Purity: 76% @ 254 nm, 100% @ 214 nm.

### Step 7. Synthesis of 5-(aminomethyl)-5,6-dihydro-1H-imidazo[4,5,1-ij]quinolin-2(4H)-1,2,2,2-trifluoroacetate (GEN1-065-7)

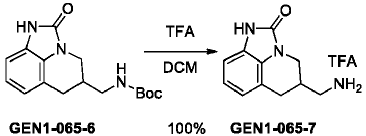

TFA (2 mL) was added to a solution of tert-butyl ((2-oxo-2,4,5,6-tetrahydro-1H-imidazo[4,5,1-ij]quinolin-5-yl)methyl]methyl)carbamate (110 mg, 0.363 mmol) in dichloromethane (6 mL). The resulting mixture was stirred at room temperature overnight. The reaction mixture was concentrated to obtain the desired compound (115 mg, yield 100%) as a brown oil MS (ESI): Rt = 0.28 min, *m*/*z* 204.1 [M+H]⁺; Purity: 80% @ 254 nm, 100% @ 214 nm.

### Step 8. Synthesis of tert-butyl ((2-oxo-2,4,5,6-tetrahydro-1H-imidazo[4,5,1-ij]quinolin-5-yl)methyl)carbamate (GEN1-065-6)

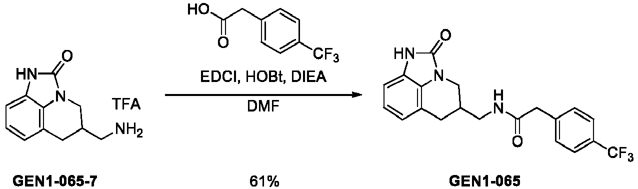

In an ice bath, EDCI (100 mg, 0.522 mmol) was added to a mixture of 5-(aminomethyl)-5,6-dihydro-1H-imidazo[4,5,1-ij]quinolin-2(4H)-1,2,2,2-trifluoroacetate (110 mg, 0.347 mmol), 2-[4-(trifluoromethyl)phenyl]acetic acid (71 mg, 0.35 mmol), HOBt (70 mg, 0.52 mmol) and DIEA (224 mg, 1.73 mmol) in DMF (2 mL). The resulting mixture was stirred overnight. The reaction mixture was poured into water (50 mL), and extracted with EtOAc (25 mL×3). The combined organic layer was washed with brine (20 mL), dried over MgSO₄, filtered and it was concentrated. The crude product was purified by preparation. HPLC(x-Bridge C185 µm 19 * 150 mm; 25-70%B, A: H₂O(0.1%NH₄HCO₃), B: acetonitrile; UV 214 nm; flow rate: 15 mL / min). The desired compound (82.2 mg, yield 61%) was obtained as a white solid. ¹HNMR (400 MHz, DMSO-*d*₆): δ 10.60 (s, 1H), 8.34 (t, *J=* 6.0 Hz, 1H), 7.67 (d, *J=* 8.0 Hz, 2H), 7.50 (d, *J=* 8.0 Hz, 2H), 6.88-6.84 (m, 1H), 6.79-6.75 (m, 2H), 3.89 (dd, *J* = 12.0, 4.0 Hz, 1H), 3.58 (s, 2H), 3.30-3.28 (m, 1H), 3.24-3.19 (m, 1H), 3.17-3.10 (m, 1H), 2.81 (dd, *J* = 12.4, 4.4 Hz, 1H), 2.58-2.54 (m, 1H), 2.23-2.14 (m, 1H). MS (ESI): Rt = 3.425 min, *m*/*z* 390.2 [M+H]⁺; Purity: 92.92% @ 254 nm, 99.65% @ 214 nm.

### Example 67: N-((9H-pyridyl[2,3-b]indol-3-yl)methyl)-2-(4-(trifluoromethyl)phenyl)acetamide

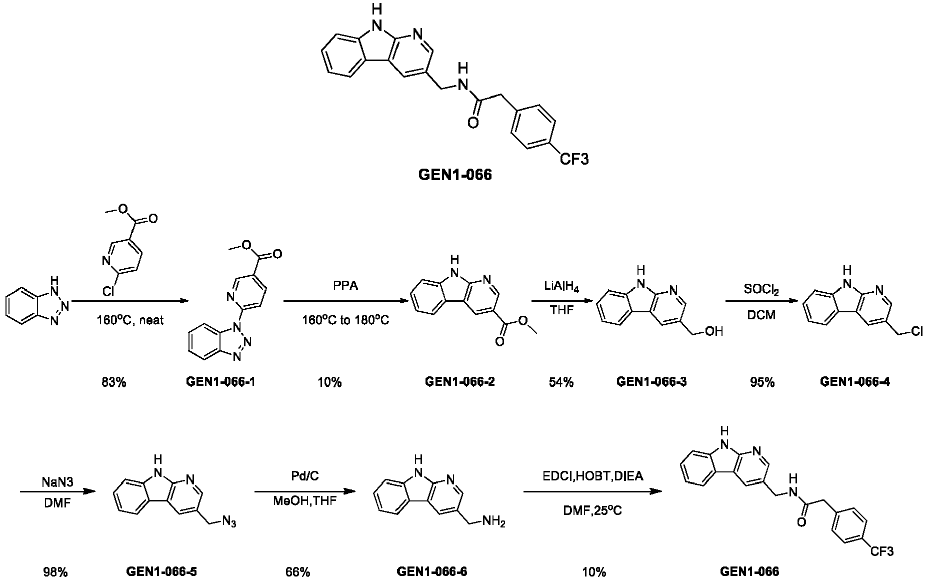

### Step 1. Synthesis of methyl 6-(1H-benzo[d][1,2,3]triazol-1-yl)nicotinate (GEN1-066-1)

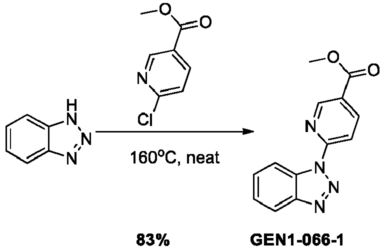

A mixture of methyl 6-chloropyridine-3-carboxylate (10.0 g, 58.3 mmol) and 1H-benzotriazole (7.29 g, 61.2 mmol) was stirred at 160°C for 1.5 hours. The mixture was cooled and dissolved in water (50 mL). Aqueous Na₂CO₃ (20 mL saturated) was added. The organic phase was dried over sodium sulfate and concentrated. The residue was triturated with PE (30 mL) and filtered to obtain the desired compound (12.5 g, yield 83%) as an off-white solid. ¹HNMR (400 MHz, DMSO-*d*₆): δ 9.12 (s, 1H), 8.59 (d, *J=* 8.4 Hz, 1H), 8.54 (dd, *J=* 8.4, 2.0 Hz, 1H), 8.35 (d, *J* = 8.4 Hz, 1H), 8.21 (d, *J* = 8.4 Hz, 1H), 7.75 (t, *J* = 8.0 Hz, 1H), 7.58 (t, *J* = 7.6 Hz, 1H), 3.94 (s, 3H). MS (ESI): Rt = 1.378 min, *m*/*z* 255.1 [M+H]⁺; Purity: 98.07% @ 214 nm.

### Step 2. Synthesis of methyl 9H-pyrido[2,3-b]indol-3-carboxylate (GEN1-066-2)

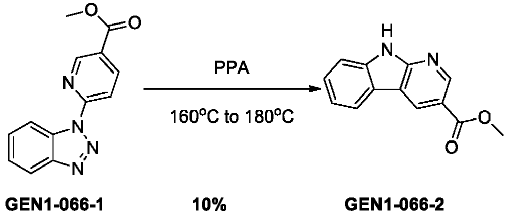

PPA (50 mL) was heated to 160°C. Then methyl 6-(benzotriazol-1-yl)pyridin-3-carboxylate (8.0 g, 31 mmol) was added in portions. The mixture was stirred at 160°C for 30 minutes, and then at 180°C for 30 minutes. The mixture was cooled to 80°C and diluted with water (200 mL). The mixture was then cooled to 5°C and basified to pH=9 with 10% aqueous NaOH solution. The mixture was extracted with EtOAc (50 mL×2). The combined organic phase was washed with brine, dried over anhydrous sodium sulfate and it was concentrated to obtain a yellow solid. The solid was triturated with DCM (5 mL), filtered and dried to obtain the desired product (800 mg, yield 10%) as a yellow solid. ¹HNMR (400 MHz, DMSO-*d*₆): δ 12.29 (s, 1H), 9.07 (s, 1H), 9.00 (d, *J=* 1.6 Hz, 1H), 8.33 (d, *J=* 8.0 Hz, 1H), 7.58-7.51 (m, 2H), 7.32-7.28 (m, 1H), 3.93 (s, 3H). MS (ESI): Rt = 1.270 min, *m*/*z* 227.2 [M+H]⁺; Purity: 76% @ 214 nm.

### Step 3. Synthesis of (9H-pyrido[2,3-b]indol-3-yl)methanol (GEN1-066-3)

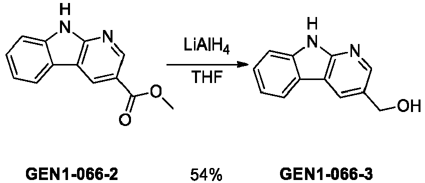

Lithium aluminum hydride (231 mg, 6.08 mmol) was added in batches to a solution of methyl 9H-pyrido[2,3-b]indol-3-carboxylate (800 mg, 3.04 mmol) in THF (20 mL). The mixture was stirred at room temperature for 1 hour. Water (10 drops) was carefully added dropwise to quench the reaction. The mixture was filtered and the filtrate was concentrated. The residue was triturated with PE/EA (1/1, 5 mL) and filtered to obtain the desired compound (380 mg, yield 54%) as a yellow solid. ¹HNMR(400 MHz, DMSO-*d*₆): δ 11.69 (s, 1H), 8.44 (d, *J* = 1.2 Hz, 1H), 8.37 (d, *J* = 2.0 Hz, 1H), 8.15 (d, *J* = 8.0 Hz, 1H), 7.50-7.42 (m, 2H), 7.23-7.19 (m, 1H), 5.23 (t, *J=* 5.6 Hz, 1H), 4.66 (d, *J* = 5.6 Hz, 2H). MS (ESI): Rt = 1.183 min, *m*/*z* 199.1 [M+H]⁺; Purity: 84.78% @ 214 nm.

### Step 4. Synthesis of 3-(chloromethyl)-9H-pyrido[2,3-b]indole (GEN1-066-4)

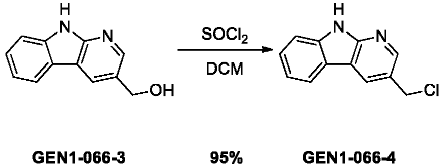

SOCl₂ (90 mg, 0.76 mmol) was added to a solution of 9H-pyrido[2,3-b]indol-3-yl)methanol (50 mg, 0.25 mmol) in toluene (3 mL) and CH₂Cl₂ (1 mL) under nitrogen atmosphere. The resulting mixture was then stirred at 50°C for 18 hours. The reaction mixture was concentrated to obtain the desired compound (61 mg, yield 95%) as a yellow solid. MS (ESI): Rt = 1.342 min, *m*/*z* 217.1 [M+H]⁺; Purity: 84.84% @ 254 nm.

### Step 5. Synthesis of 3-(azidomethyl)-9H-pyrido[2,3-b]indole (GEN1-066-5)

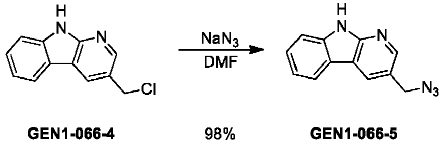

NaN3 (92mg, 1.4mmol) was added to a solution of 3-(chloromethyl)-9H-pyrido[2,3-b]indole (61mg, 0.28mmol) in DMF (2 mL) at 0°C under N₂ atmosphere. The resulting mixture was stirred at room temperature for 2 hours. The reaction mixture was diluted with water (20 mL) and extracted with EtOAc (20 mL). The organic phase was dried over sodium sulfate and concentrated to obtain the desired product (64 mg, yield 98%) as a white solid. MS (ESI): Rt = 1.244 min, *m*/*z* 224.2 [M+H]⁺; Purity: 96.20% @ 254 nm.

### Step 6. Synthesis of (9H-pyrido[2,3-b]indol-3-yl)methanamine (GEN1-066-6)

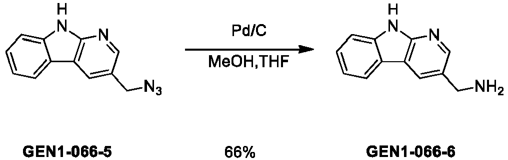

Pd/C (10%, 31 mg,) was added to a solution of 3-(azidomethyl)-9H-pyrido[2,3-b]indole (64 mg, 0.29 mmol) in MeOH(1 mL) and THF(1 mL) under nitrogen atmosphere. The resulting mixture was degassed with H₂ and stirred at 20°C under hydrogen atmosphere overnight. The reaction mixture was filtered, and the filtrate was concentrated to obtain the desired compound (61 mg, yield 66%) as a colorless oil. MS (ESI): Rt = 0.223 min, *m*/*z* 198.2 [M+H]⁺; Purity: 61.12% @ 254 nm.

### Step 7. Synthesis of N-((9H-pyridyl[2,3-b]indol-3-yl)methyl)-2-(4-(trifluoromethyl)phenyl)acetamide (GEN1-066)

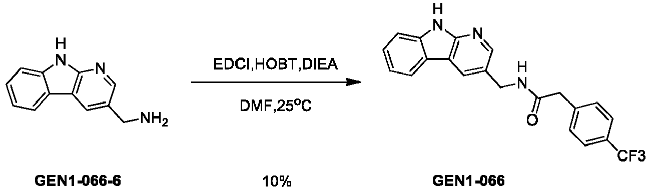

2-[4-(trifluoromethyl)phenyl]acetic acid (180 mg, 0.882 mmol), HOBt (155 mg, 1.15 mmol), EDCI (220 mg, 1.15 mmol) and DIEA (342 mg, 2.65 mmol) were mixed in DMF (15 mL), and the resulting miature was stirred at 20°C for 10 minutes under nitrogen atmosphere. Then 9H-pyrido[2,3-b]indol-3-ylmethylamine (174mg, 0.882mmol) was added. The reaction mixture was stirred at 20°C for 18 hours. The reaction mixture was purified by preparation. HPLC (Xbridge C185µm 19 * 150 mm; B: 40-80%, A: H₂O(0.1%NH₄HCO₃), B: CH₃CN; UV: 214 nm, flow rate: 15 mL / min). The desired compound (33 mg, yield 10%) was obtained as a white solid. ¹HNMR (400 MHz, DMSO-*d*₆): δ 11.73 (s, 1H), 8.70 (t, *J=* 5.6 Hz, 1H), 8.33-8.29 (m, 2H), 8.06 (d, *J=* 8.0 Hz, 1H), 7.68 (d, *J=* 8.0 Hz, 2H), 7.53 (d, *J=* 8.0 Hz, 2H), 7.49-7.42 (m, 2H), 7.20 (t, *J*= 7.2 Hz, 1H), 4.44 (d, *J=* 5.6 Hz, 2H), 3.62 (s, 2H). MS (ESI): Rt = 3.504 min, *m*/*z* 384.1 [M+H]⁺; Purity: 95.94% @ 254 nm, 98.51% @ 214 nm.

### Example 68: 1-(1-((4-(trifluoromethyl)benzyl)sulfonyl)piperidin-4-yl)-1H-benzo[d]imidazol-2(3H)-one

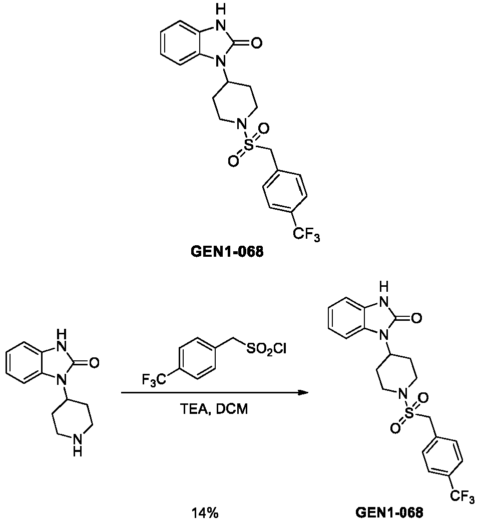

### 1.1 Synthesis of 1-(1-((4-(trifluoromethyl)benzyl)sulfonyl)piperidin-4-yl)-1H-benzo[d]imidazol-2(3H)-one (GEN1-068)

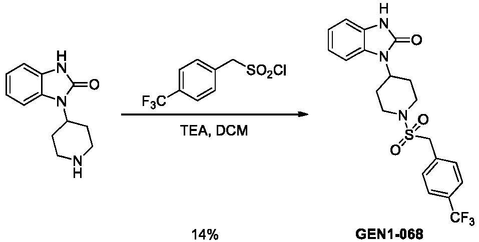

TEA (196 mg, 1.94 mmol) was added to a solution of 3-(4-piperidinyl)-1H-benzimidazol-2-one (140 mg, 0.644 mmol) and [4-(trifluoromethyl)phenyl]methanesulfonyl chloride (167 mg, 0.646 mmol) in DCM (2 mL). After the resulting mixture was stirred at 25°C for 12 hours, the mixture was concentrated and the residue was purified by a C18 column (CH₃CN: water = 5% to 50%) to obtain the desired compound (40 mg, yield 14%) as a white solid. ¹H NMR (400 MHz, DMSO-*d*₆): δ 10.86 (s, 1H), 7.79 (d, *J=* 8.4 Hz, 2H), 7.70 (d, *J=* 8.4 Hz, 2H), 7.19-7.18 (m, 1H), 7.02-6.95 (m, 3H), 4.63 (s, 2H), 4.36-4.27 (m, 1H), 3.73 (d, *J=* 12.4 Hz, 2H), 2.96 (t, *J=* 11.6 Hz, 2H), 2.34-2.24 (m, 2H), 1.74 (d, *J* = 10.8 Hz, 2H). MS (ESI): Rt = 3.690 min, *m*/*z* 438.0, [M-H]⁻; Purity: 97.99% @ 214 nm, 84.49% @ 254 nm.

### Example 69: 1-(1-(2-(4-methoxyphenyl)acetyl)piperidin-4-yl)-1H-benzo[d]imidazol-2(3H)-one

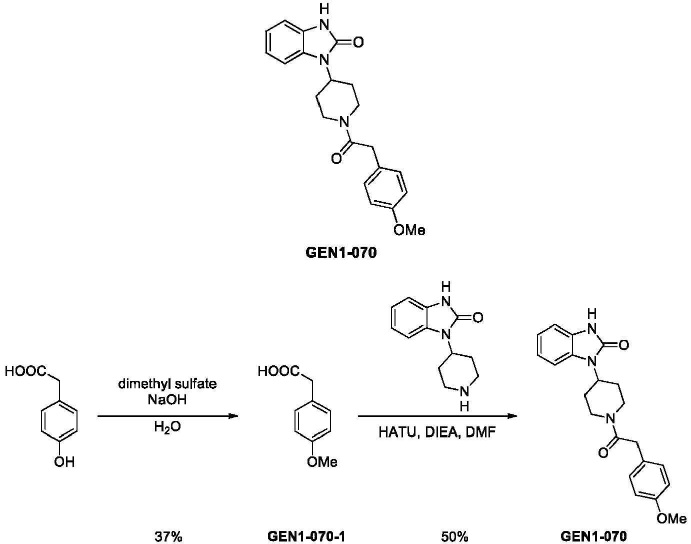

### 1.2 Synthesis of 2-(4-methoxyphenyl)acetic acid (GEN1-070-1)

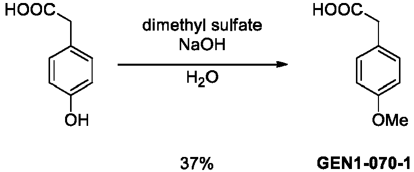

Dimethyl sulfate (2.07 g, 16.4 mmol) was added dropwise to a solution of 2-(4-hydroxyphenyl)acetic acid (1.00 g, 6.57 mmol) and NaOH (1.58 g, 39.4 mmol) in water (15 mL). The resulting mixture was stirred at 45°C for 16 hours. The resulting mixture was acidified with concentrated hydrochloric acid to pH=1-2 and filtered. The filter cake was washed with water (5 mL×2) to obtain the desired compound (401 mg, yield 37%) as a white solid. ¹HNMR (400 MHz, DMSO-*d*₆): δ 12.15 (br s, 1H), 7.16 (d, *J* = 8.8 Hz, 2H), 6.86 (d, *J* = 8.4 Hz, 2H), 3.73 (s, 3H), 3.48 (s, 2H).

### Step 2. Synthesis of 1-(1-(2-(4-methoxyphenyl)acetyl)piperidin-4-yl)-1H-benzo[d]imidazol-2(3H)-one (GEN1-070)

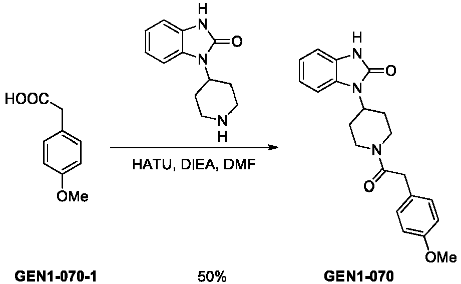

HATU (343 mg, 0.902 mmol) was added to a solution of 3-(4-piperidinyl)-1H-benzimidazol-2-one (157 mg, 0.723 mumol), 2-(4-methoxyphenyl)acetic acid (100 mg, 0.602 mmol) and DIEA (233 mg, 1.80mmol) in DMF (1.5 mL). The resulting mixture was stirred at room temperature for 16 hours. The resulting mixture was purified by preparation. HPLC (waters-3 sunfire C18 5um 19 * 150mm, 20%-50%B; A: H₂O(0.1%TFA), B: CH₃CN; UV: 214 nm, flow rate: 15mL / min). Then saturated Na₂CO₃ was added to basify the mixture to pH=8. The mixture was extracted with EtOAc (20 mL×3), and the combined organic layer was concentrated to obtain the desired compound (111 mg, yield 50%) as a white solid. ¹HNMR (400 MHz, CDCl₃): δ 9.23-9.13 (m, 1H), 7.26-7.24 (m, 2H), 7.08-6.99 (m, 3H), 6.91 (d, *J*= 8.8 Hz, 2H), 6.83 (d, *J=* 7.6 Hz, 1H), 4.88 (d, *J=* 13.2 Hz, 1H), 4.56-4.48 (m, 1H), 4.06 (d, *J=* 14 Hz, 1H), 3.78 (s, 3H), 3.76 (s, 2H), 3.18-3.11 (m, 1H), 2.72-2.65 (m, 1H), 2.27-2.16 (m, 1H), 1.96-1.81 (m, 2H), 1.72-1.67 (m, 1H). MS (ESI): Rt = 3.095 min, *m*/*z* 366.2 [M+H]⁺; Purity: 99.31% @ 254 nm, 99.64% @ 214 nm.

### Example 70: 1-(1-(2-(4-hydroxyphenyl)acetyl)piperidin-4-yl)-1H-benzo[d]imidazol-2(3H) -one

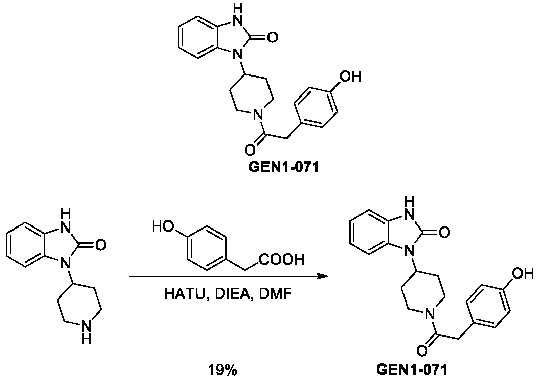

### Step 1. Synthesis of 1-(1-(2-(4-hydroxyphenyl)acetyl)piperidin-4-yl)-1H-benzo[d]imidazol-2(3H) -one (GEN1-071)

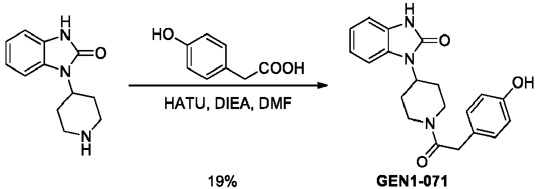

HATU (184mg, 0.484mmol) was added to a solution of 3-(4-piperidinyl)-1H-benzimidazol-2-one (70 mg, 0.32 mumol), 2-(4-hydroxyphenyl)acetic acid (59 mg, 0.39 mmol) and DIEA in DMF (2mL). The mixture was stirred at room temperature for 16 hours. The resulting mixture was purified by preparation. HPLC (prep-5 Xbridge C18 5um 19 * 150mm, 10%-60%B; A: H₂O(0.1%NH₄HCO₃), B: CH₃CN; UV: 214 nm, flow rate: 15 mL / min). The desired compound (22 mg, yield 19%) was obtained as a white solid.

¹HNMR (400 MHz, DMSO-*d*₆): δ 10.83 (s, 1H), 9.27 (s, 1H), 7.10 (d, *J* = 8.0 Hz, 2H), 6.98-6.90 (m, 4H), 6.74 (d, *J=* 8.4 Hz, 2H), 4.56 (d, *J*= 12.8 Hz, 1H), 4.44-4.34 (m, 1H), 4.07 (d, *J=* 13.2 Hz, 1H), 3.70 (d, *J=* 14.8 Hz, 1H), 3.60 (d, *J=* 14.4 Hz, 1H), 3.15-3.09 (m, 1H), 2.68-2.62 (m, 1H), 2.05-1.94 (m, 1H), 1.93-1.82 (m, 1H), 1.65 (d, *J=* 12.4 Hz, 1H), 1.56 (d, *J=* 12.4 Hz, 1H). MS (ESI): Rt = 2.740 min, *m*/*z* 352.2 [M+H]⁺; Purity: 88.18% @ 254 nm, 98.96% @ 214 nm.

### Example 71: 1-(1-(2-(4-(1H-tetrazol-5-yl)phenyl)acetyl)piperidin-4-yl)-1H-benzo [d] imidazol-2(3H)-one (GEN1-072)

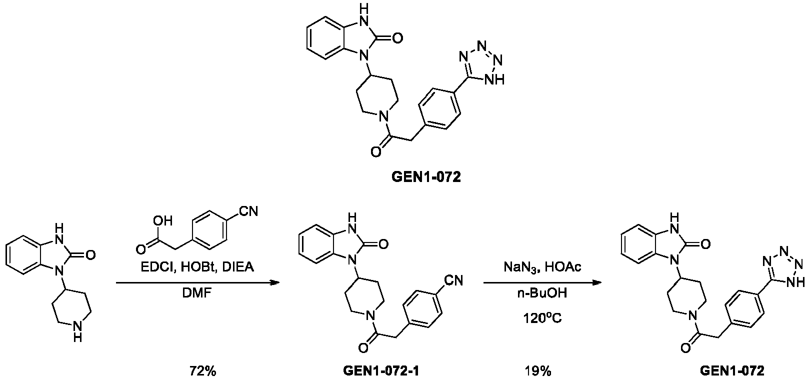

### Step 1. Synthesis of 4-(2-oxo-2-(4-(2-oxo-2,3-dihydro-1H-benzo[d]imidazol-1-yl)piperidin-1-yl)ethyl)benzonitrile (GEN1-072-1)

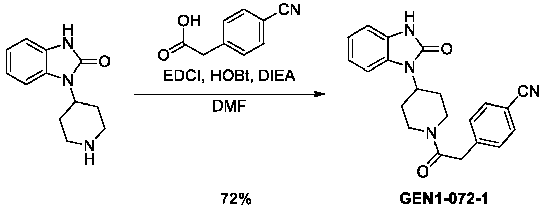

In an ice bath, EDCI (199 mg, 1.04 mmol) was added to a mixture of 3-(4-piperidinyl)-1H-benzimidazol-2-one (150 mg, 0.690 mmol), 2-(4-cyanophenyl)acetic acid (112 mg, 0.695 mmol), HOBT(139 mg, 1.03) and DIEA(269 mg, 2.08 mmol) in DMF (2 mL). The resulting mixture was stirred at room temperature overnight. The reaction mixture was purified by a C18 column (CH₃CN: water = 5% to 50%) to obtain the desired compound (200 mg, yield 72%) as a brown solid. MS (ESI): Rt = 1.32 min, *m*/*z* 361.3 [M+H]⁺; Purity: 100% @ 254 nm, 100% @ 214 nm.

### Step 2. Synthesis of 1-(1-(2-(4-(1H-tetrazol-5-yl)phenyl)acetyl)piperidin-4-yl)-1H-benzo [d] imidazol-2(3H)-one (GEN1-072)

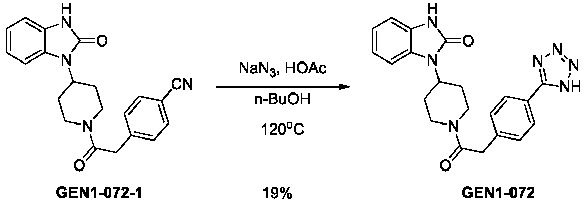

4-[2-oxo-2-[4-(2-oxo-3H-benzimidazol-1-yl)-1-piperidinyl]ethyl]benzonitrile (140 mg, 0.388 mmol) and NaN3 were mixed in acetic acid (60mg, 1.0mmol) and n-butanol (5mL). The resulting mixture was stirred at 125°C under N₂ atmosphere overnight. Then NaN3 (65 mg, 1.0 mmol) and acetic acid (60 mg, 1.0 mmol) were added to the mixture, and the resulting mixture was stirred at 125°C for another 12 hours. The reaction mixture was concentrated and purified by preparative HPLC (Xbridge C18, 5µm 19×150mm; B: 5% to 25% B, A: water (0.1% NH4HCO3), B: acetonitrile; UV: 214nm; MS: 145nm; MS: 185nm; M + H +). Flow rate: 15 mL. The desired compound (30.5 mg, yield 19%) was obtained as a white solid. ¹HNMR (400MHz, DMSO-*d*₆): δ 10.82 (s, 1H), 8.01 (d, *J* = 8.4 Hz, 2H), 7.45 (d, *J* = 8.4 Hz, 2H), 6.99-6.88 (m, 4H), 4.61-4.58 (m, 1H), 4.47-4.36 (m, 1H), 4.15-4.11 (m, 1H), 3.92-3.81 (m, 2H), 3.18 (t, *J =* 12.8 Hz, 1H), 2.70 (t, *J* = 12.4 Hz, 1H), 2.12-1.96 (m, 2H), 1.70-1.62 (m, 2H). MS (ESI): Rt = 3.474 min, m/z 404.2 [M+H]⁺; Purity: 98.73% @ 254 nm, 98.75% @ 214 nm.

### Example 72: 1-(1-(2-(naphth-2-yl)acetyl)piperidin-4-yl)-1H-benzo[d]imidazol-2(3H)-one (GEN1-073)

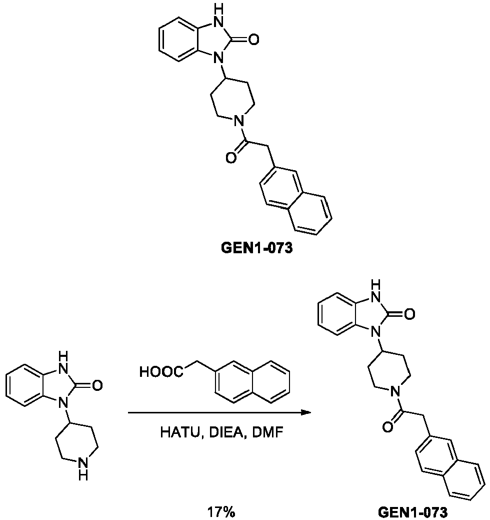

### Step 1. Synthesis of 1-(1-(2-(naphth-2-yl)acetyl)piperidin-4-yl)-1H-benzo[d]imidazol-2(3H)-one (GEN1-073)

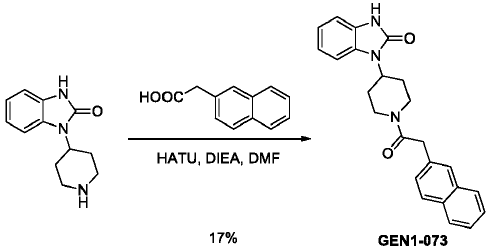

2-(2-naphthyl)acetic acid (103 mg, 0.553 mmol) was added to a solution of 3-(4-piperidinyl)-1H-benzimidazol-2-one (100 mg, 0.460 mmol), HATU (263 mg, 0.692 mmol) and DIEA (178 mg, 1.38 mmol) in DMF(2 mL). The mixture was stirred at room temperature for 16 hours. The resulting mixture was purified by preparation. HPLC (prep-5 Xbridge C18 5um 19 * 150mm, 30%-70%B; A: H₂O(0.1%NH₄HCO₃), B: CH₃CN; 214nm, flow rate: 15 mL/min). The desired compound (30 mg, yield 17%) was obtained as a white solid. ¹HNMR (400 MHz, DMSO-*d*₆): δ 10.80 (s, 1H), 7.92-7.89 (m, 3H), 7.83 (s, 1H), 7.53-7.46 (m, 3H), 6.94-6.91 (m, 2H), 6.89-6.79 (m, 2H), 4.62 (d, *J=* 13.2 Hz, 1H), 4.44-4.36 (m, 1H), 4.16 (d, *J=* 13.2 Hz, 1H), 4.02 (d, *J* = 15.2 Hz, 1H), 3.92 (d, *J* = 15.2 Hz, 1H), 3.18 (t, *J* = 12.4 Hz, 1H), 2.71 (t, *J* = 12.8 Hz, 1H), 2.11-2.00 (m, 1H), 1.95-1.84 (m, 1H), 1.68 (d, *J=* 10.4 Hz, 1H), 1.57 (d, *J=* 10.4 Hz, 1H). MS (ESI): Rt = 3.685 min, *m*/*z* 386.1 [M+H]⁺; Purity: 95.09% @ 254 nm, 97.86% @ 214 nm.

### Example 73: 1-(1-(2-(quinolin-6-yl)acetyl)piperidin-4-yl)-1H-benzo[d]imidazol-2(3H) -one

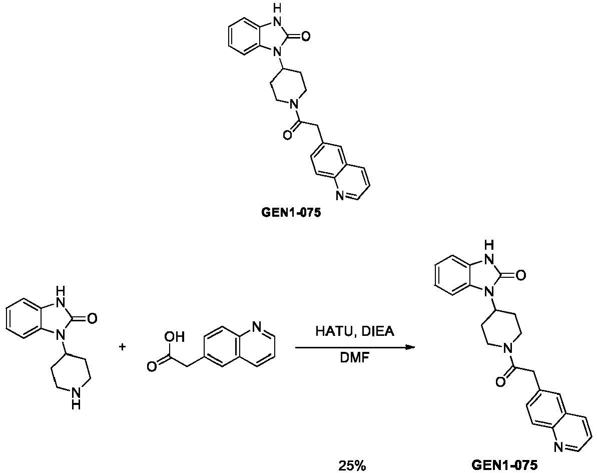

### Step 1. Synthesis of 1-(1-(2-(quinolin-6-yl)acetyl)piperidin-4-yl)-1H-benzo[d]imidazol-2(3H)-one (GEN1-075)

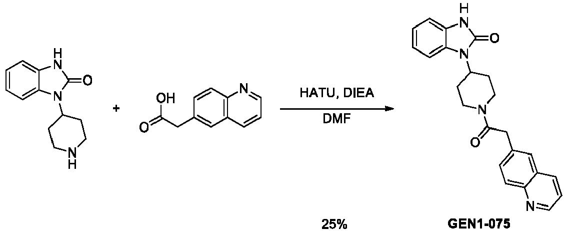

DIEA (387 mg, 2.99 mmol) was added to a solution of 2-(6-quinolinyl)acetic acid (187 mg, 0.999 mmol), 3-(4-piperidinyl)-1H-benzimidazol-2-one (217 mg, 0.999 mmol) and HATU (494 mg, 1.30 mmol) in DMF (4 mL). After the resulting mixture was stirred at 25°C for 6 hours, the mixture was purified by a C18 column (CH₃CN: water = 5% to 50%) to obtain the desired compound (100 mg, yield 25%) as a white solid. ¹H NMR (400 MHz, CDCl₃): δ 9.54 (s, 1H), 8.91 (s, 1H), 8.15 (t, *J=* 8.4 Hz, 2H), 7.78 (s, 1H), 7.71 (d, *J=* 8.4 Hz, 1H), 7.44-7.40 (m, 1H), 7.05-6.98 (m, 2H), 6.89 (t, *J=* 7.6 Hz, 1H), 6.76 (d, *J=* 8.0 Hz, 1H), 4.93 (d, *J=* 13.2 Hz, 1H), 4.57-4.46 (m, 1H), 4.13 (d, *J=* 13.6 Hz, 1H), 4.02 (s, 2H), 3.20 (t, *J=* 12.4 Hz, 1H), 2.73 (t, *J=* 14.8 Hz, 1H), 2.33-2.23 (m, 1H), 2.03-1.93 (m, 1H), 1.89 (d, *J*= 12.8 Hz, 1H), 1.78-1.73 (m, 1H). MS (ESI): Rt = 2.639 min, *m*/*z* 387.2, [M+H]⁺; Purity: 99.84% @ 214 nm, 96.55% @ 254 nm.

### Example 74: 3-[1-[2-(1H-indol-5-yl)acetyl]-4-piperidinyl]-1H-benzimidazol-2-one

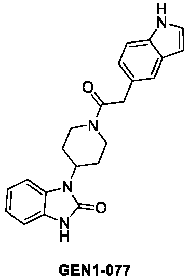

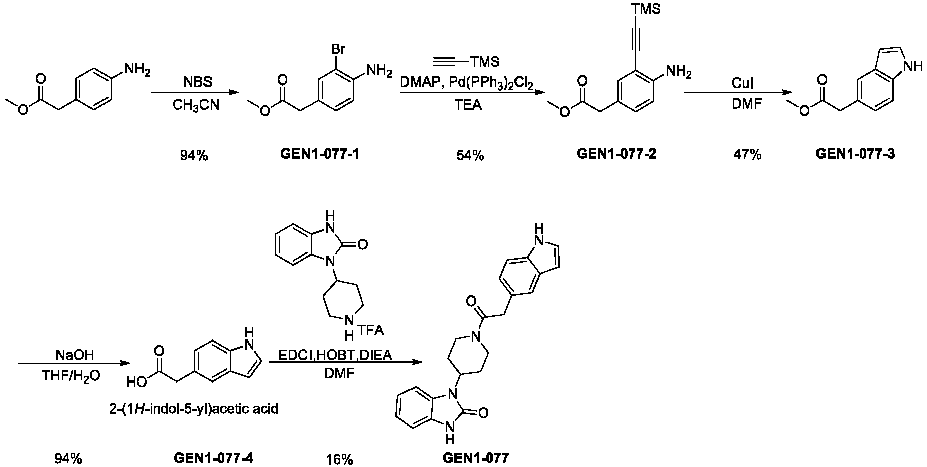

### Step 1. Synthesis of methyl 2-(4-amino-3-bromophenyl)acetate

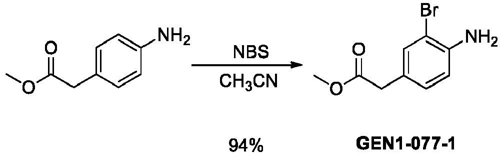

A solution of 1-bromopyrrolidine-2,5-dione (3.23 g, 18.2 mmol) in acetonitrile (10 mL) was added dropwise to a solution of methyl 2-(4-aminophenyl)acetate (3.00 g, 18.2 mmol) in acetonitrile (10 mL). The mixture was stirred at room temperature overnight. The resulting mixture was filtered and the filtrate was concentrated. The residue was purified by a silica gel column (petroleum ether: EtOAc = 5:1) to obtain the desired compound (4.15 g, yield 94%) as a pale yellow oil. MS (ESI) Rt = 1.43 min, *m*/*z* 245.0 [M+H]⁺; Purity: 100% @ 254 nm, 100% @ 214 nm.

### Step 2. Synthesis of methyl 2-(4-amino-3-((trimethylsilyl)ethynyl)phenyl)acetate

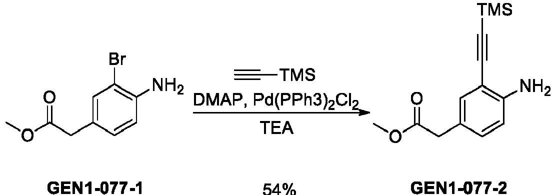

Pd(PPh3)2Cl2 (575 mg, 0.819 mmol) was added to a solution of methyl 2-(4-amino-3-bromophenyl)acetate (2.00 g, 8.19 mmol), ethynyl trimethyl silane (8.05 g, 81.9 mmol) and DMAP (100 mg, 0.819 mmol) in Et3N(10 mL). The mixture was stirred at 70°C under nitrogen overnight. The resulting mixture was concentrated, and water (50 mL) was added. DCM (50 mL×3) was added to extract the desired compound. The combined organic layer was washed with brine (50 mL), dried over anhydrous Na₂SO₄ and filtered. The filtrate was concentrated, and the residue was purified by a silica gel column (petroleum ether: EtOAc = 20:1) to obtain the desired compound (1.16 g, yield 54%) as a yellow oil. MS (ESI) Rt = 1.71 min, m/z 262.2 [M+H]⁺; Purity: 100% @ 254 nm, 100% @ 214 nm.

### Step 3. Synthesis of methyl 2-(1H-indol-5-yl)acetate

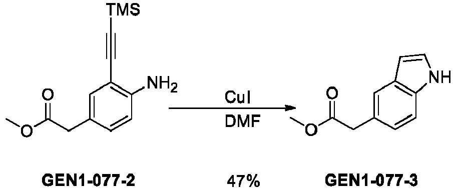

CuI (85 mg, 0.446 mmol) was added to a mixture of methyl 2-(4-amino-3-(((trimethylsilyl)ethynyl)phenyl)acetate (1.16g, 4.44mmol) in DMF(10mL). The resulting mixture was stirred at 90°C for 24 hours under nitrogen. The resulting mixture was cooled to room temperature and poured into water (30 mL). DCM (50 mL×3) was added to extract the desired compound. The combined organic layer was washed with brine (50 mL), dried over anhydrous Na₂SO₄ and it was concentrated. The residue was purified by RP-HPLC (45-65% B; A: H2O B: CH₃CN) to obtain the desired compound (393 mg, yield 47%) as a yellow oil. MS (ESI) Rt = 1.43 min, *m*/*z* 188.2 [M-H]⁻; Purity: 100% @ 254 nm, 100% @ 214 nm.

### Step 4. Synthesis of 2-(1H-indol-5-yl)acetic acid

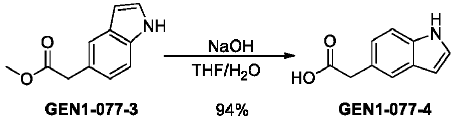

NaOH (166 mg, 4.15 mmol) was added to a solution of methyl 2-(1H-indol-5-yl)acetate (393 mg, 2.08 mmol) in THF (6 mL) and H₂O (3 mL). The mixture was stirred at 50°C overnight. The resulting mixture was concentrated, and water (15 mL) was added. The mixture was acidified to pH=1 with aqueous HCl (12 M), and DCM (50 mL×3) was added to extract the desired compound. The combined organic solution was washed with brine (50 mL), dried over anhydrous Na₂SO₄ and it was concentrated to obtain the desired compound (341 mg, yield 94%) as an ocher solid. MS (ESI) Rt = 1.142 min, *m*/*z* 176.0 [M+H]⁺; Purity: 100% @ 214 nm.

### Step 5. Synthesis of 3-[1-[2-(1H-indol-5-yl)acetyl]-4-piperidinyl]-1H-benzimidazol-2-one (GEN1-077)

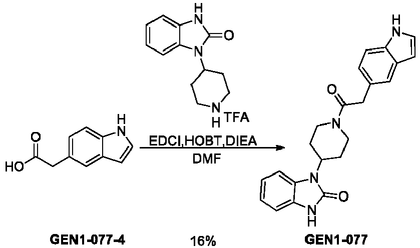

2-(1H-indol-5-yl)acetic acid (100 mg, 0.571 mmol) was added to a solution of 1-(piperidin-4-yl)-1H-benzo[d]imidazol-2(3H)-1 one 2,2,2-trifluoroacetate (254 mg, 0.767 mmol), EDCI (164 mg, 0.856 mmol), HOBT(116 mg, 0.859 mmol) and DIEA(369 mg, 2.86 mmol) in DMF(3 mL). The mixture was stirred at room temperature overnight. The resulting mixture was poured into water (20 mL) and filtered. The precipitate was washed with water (10 mL) and recrystallized from MeOH. Then the precipitate was completely dissolved in DMSO and the resulting mixture was added dropwise to hot water (2 mL). The mixture was filtered and the residue was dried to obtain the desired compound (35 mg, yield 16%) as a white solid. ¹HNMR (400 MHz, DMSO-*d*₆): δ 11.02 (s, 1H), 10.79 (s, 1H), 7.48 (s, 1H), 7.37 (d, *J* = 8.4 Hz, 1H), 7.31 (s, 1H), 7.05 (d, *J=* 8.0 Hz, 1H), 6.92-6.89 (m, 2H), 6.85-6.81 (m, 1H), 6.75-6.73 (m, 1H), 6.41 (s, 1H), 4.60 (d, *J* = 12.8 Hz, 1H), 4.40-4.34 (m, 1H), 4.15-4.09 (m, 1H), 3.89-3.75 (m, 2H), 3.14-3.08 (m, 1H), 2.66 (t, *J=* 12.0 Hz, 1H), 2.02-1.93 (m, 1H), 1.78-1.70 (m, 1H), 1.65-1.63 (m, 1H), 1.50-1.47 (m, 1H). MS (ESI) Rt = 3.835 min, *m*/*z* 375.2 [M+H]⁺; Purity: 97.76% @ 254 nm, 99.07% @ 214 nm.

### Example 75: 1-(1-(2-(5-(trifluoromethyl)furan-2-yl)acetyl)piperidin-4-yl)-1H-benzo[d]imidazol-2(3H)-one

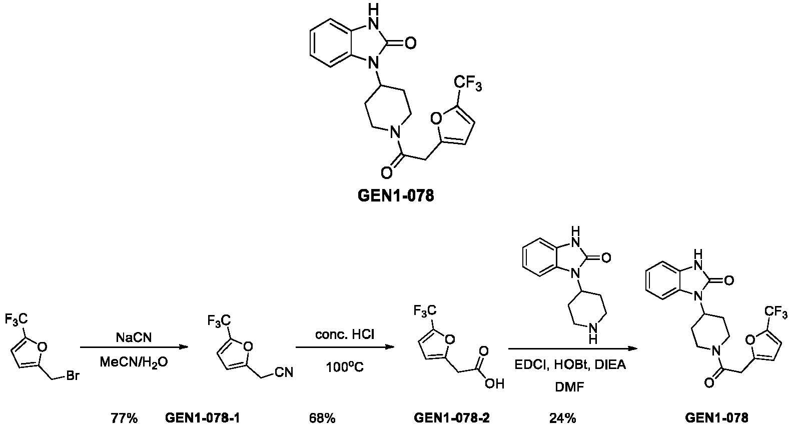

### Step 1. Synthesis of 2-(5-(trifluoromethyl)furan-2-yl)acetonitrile (GEN1-078-1)

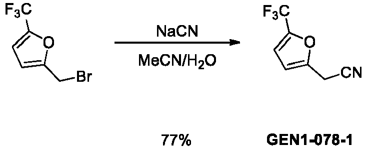

In an ice bath, NaCN (464 mg, 8.75 mmol) was added to a solution of 2-(bromomethyl)-5-(trifluoromethyl)furan (1.0 g, 4.4 mmol) in CH₃CN (10 mL) and water (10 mL). The resulting mixture was stirred at room temperature overnight. The reaction mixture was poured into water (50 mL), and extracted with ether (30 mL×3). The combined organic layer was washed with brine (30mL), dried over MgSO₄, filtered and it was concentrated to obtain the desired compound (650 mg, yield 77%) as a brown liquid. ¹HNMR (400 MHz, CDCl₃): δ 6.78 (dd, *J =* 3.6, 1.2 Hz, 1H), 6.47 (dd, *J* = 3.6, 0.8 Hz, 1H), 3.83 (s, 2H).

### Step 2. Synthesis of 2-(5-(trifluoromethyl)furan-2-yl)acetic acid (GEN1-078-2)

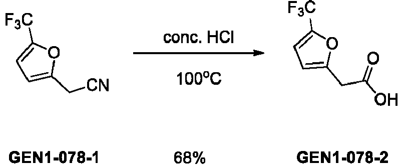

2-(5-(trifluoromethyl)furan-2-yl)acetonitrile (180 mg, 1.03 mmol) and concentrated HCl (1.5 mL) were stirred at 100°C for 1 hour. The reaction mixture was poured into water (20 mL), and extracted with EtOAc (20 mL×3). The combined organic layer was washed with brine (20 mL), dried over MgSO₄, filtered and it was concentrated to obtain the desired compound (150 mg, yield 68%) as a brown liquid. ¹HNMR (400 MHz, DMSO-*d*₆): δ 12.40 (br s, 1H), 7.13 (dd, *J* = 3.2, 1.2 Hz, 1H), 6.50 (d, *J* = 2.8 Hz, 1H), 3.81 (s, 2H).

### Step 3. Synthesis of 1-(1-(2-(5-(trifluoromethyl)furan-2-yl)acetyl)piperidin-4-yl)-1H-benzo[d]imidazol-2(3H)-one (GEN1-078)

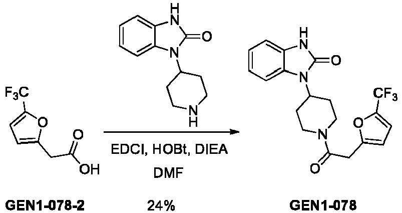

In an ice bath, EDCI (66 mg, 0.34 mmol) was added to a solution of 3-(4-piperidinyl)-1H-benzimidazol-2-one (50 mg, 0.23 mmol), 2-(5-(trifluoromethyl)furan-2-yl)acetic acid (50 mg, 0.26 mmol), HOBt (47 mg, 0.35 mmol) and DIEA (89 mg, 0.69 mmol) in DMF(2mL). The resulting mixture was stirred at room temperature overnight. The reaction mixture was purified by a C18 column (CH₃CN: water = 5% to 55%) to obtain the desired compound (22.3 mg, yield 24%) as a white solid. ¹HNMR (400 MHz, CDCl₃): δ 8.80 (s, 1H), 7.10-7.01 (m, 4H), 6.78 (d, *J* = 4.0 Hz, 1H), 6.39 (d, *J* = 3.2 Hz, 1H), 4.90-4.86 (m, 1H), 4.61-4.52 (m, 1H), 4.18-4.14 (m, 1H), 3.94-3.82 (m, 2H), 3.30 (t, *J* = 14.0 Hz, 1H), 2.75 (t, *J* =12.8 Hz, 1H), 2.39-2.26 (m, 2H), 1.97-1.90 (m, 2H). MS (ESI): Rt = 3.485 min, m/z 394.2 [M+H]⁺; Purity: 87.31% @ 254 nm, 97.50% @ 214 nm.

### Example 76: N-((2-oxo-2,3-dihydro-1H-pyrrolo[2,3-b]pyridin-5-yl)methyl)-2-(4-(trifluoromethyl)phenyl)acetamide

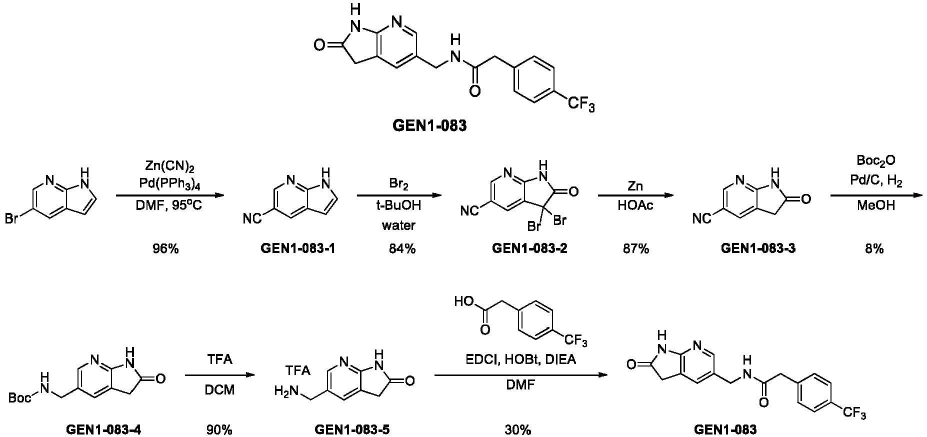

### Step 1. Synthesis of 1H-pyrrolo[2,3-b]pyridin-5-carbonitrile (GEN1-083-1)

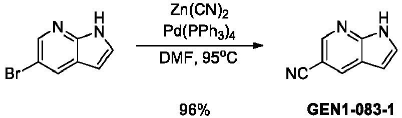

A suspension of 5-bromo-1H-pyrrolo[2,3-b]pyridine (3.0 g, 15 mmol), Zn(CN)2 (2.68 g, 22.8 mmol) and Pd(PPh3)4 (1.76 g, 1.52 mmol) in DMF (30 mL) was stirred at 95°C under N₂ atmosphere overnight. The reaction mixture was poured into water (150 mL), and extracted with EtOAc (50 mL×3). The combined organic layer was washed with brine (50 mL), dried over MgSO₄, filtered and it was concentrated. The crude product was purified by silica gel column chromatography (PE:EtOAc = 4:1 to 1:2) to obtain the desired compound (2.1 g, yield 96%) as a white solid. MS (ESI): Rt = 1.23 min, m/z 143.9 [M+H]⁺; Purity: 100% @ 254 nm, 84% @ 214 nm.

### Step 2. Synthesis of 3,3-dibromo-2-oxo-2,3-dihydro-1H-pyrrolo[2,3-b]pyridin-5-carbonitrile (GEN1-083-2)

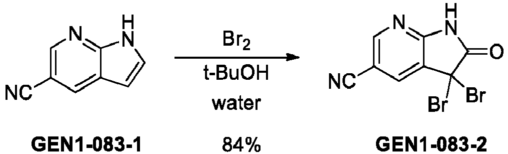

Br₂ (5.86 g, 36.7 mmol) was added dropwise to a suspension of 1H-pyrrolo[2,3-b]pyridin-5-carbonitrile (2.1 g, 15 mmol) in t-BuOH (15 mL) and water (15 mL) at room temperature. The resulting mixture was stirred at room temperature in the dark for 2 hours. The reaction mixture was poured into water (200 mL), and extracted with EtOAc (70 mL×3). The combined organic layer was washed with brine (40 mL), dried over MgSO₄, filtered and it was concentrated to obtain the desired compound (4.35 g, yield 84%) as a brown solid. MS (ESI): Rt = 1.27 min, m/z 316.0 [M+H]⁺; Purity: 59% @ 254 nm, 72% @ 214 nm.

### Step 3. Synthesis of 2-oxo-2,3-dihydro-1H-pyrrolo[2,3-b]pyridin-5-carbonitrile (GEN1-083-3)

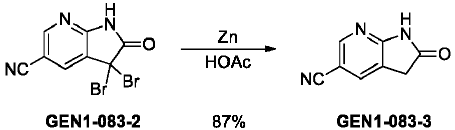

Zinc powder (4.49 g, 68.6 mmol) was added in batches to a solution of 3,3-dibromo-2-oxo-1H-pyrrolo[2,3-b]pyridin-5-carbonitrile (4.35 g, 13.7 mmol) in HOAc (15 mL). The mixture was stirred at room temperature for 1 hour. The reaction mixture was gradually poured into Na₂CO₃ (saturated 150 mL), and the resulting mixture was extracted with EtOAc (100 mL×5). The combined organic layer was washed with brine (30 mL), dried over MgSO₄, filtered and it was concentrated to obtain the desired compound (2.1 g, yield 87%) as a brown solid. ¹HNMR (400 MHz, DMSO-*d*₆): δ 11.56 (s, 1H), 8.55 (s, 1H), 7.96 (s, 1H), 3.63 (s, 2H).

### Step 4. Synthesis of tert-butyl ((2-oxo-2,3-dihydro-1H-pyrrolo[2,3-b]pyridin-5-yl)methyl)carbamate (GEN1-083-4)

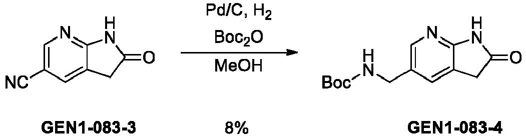

Pd/C (10%, 669 mg) was added to a suspension of 2-oxo-1,3-dihydropyrrolo[2,3-b]pyridin-5-carbonitrile(1.0 g, 6.3 mmol) and Boc2O (1.51 g, 6.91 mmol) in MeOH (60 mL). The reaction mixture was stirred at room temperature under H₂ atmosphere overnight. The reaction mixture was filtered and it was concentrated. The crude product was purified by silica gel column chromatography (DCM:MeOH=40:1) to obtain the desired compound (140 mg, yield 8%) as a brown solid. MS (ESI): Rt = 1.24 min, m/z 264.2 [M+H]⁺; Purity: 90% @ 254 nm, 84% @ 214 nm.

### Step 5. Synthesis of 5-(aminomethyl)-1H-pyrrolo[2,3-b]pyridin-2(3H)-1,2,2,2-trifluoroacetate (GEN1-083-5)

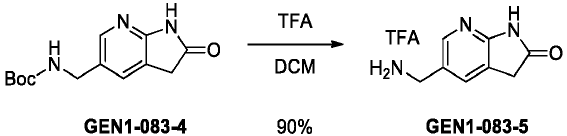

TFA (1.2 mL) was added to a solution of tert-butyl N-[(2-oxo-1,3-dihydropyrrolo[2,3-b]pyridin-5-yl)methyl)carbamate (100 mg, 0.380 mmol) in DCM (6.0 mL). The resulting mixture was stirred at room temperature for 2 hours. The reaction mixture was concentrated to obtain the desired compound (120 mg crude product, yield 90%) as a brown solid. ¹HNMR (400 MHz, DMSO-*d*₆): δ 11.11 (s, 1H), 8.17 (br s, 3H), 8.12 (s, 1H), 7.64 (s, 1H), 4.02-3.98 (m, 2H), 3.60 (s, 2H).

### Step 6. Synthesis of N-((2-oxo-2,3-dihydro-1H-pyrrolo[2,3-b]pyridin-5-yl)methyl)-2-(4-(trifluoromethyl)phenyl)acetamide (GEN1-083)

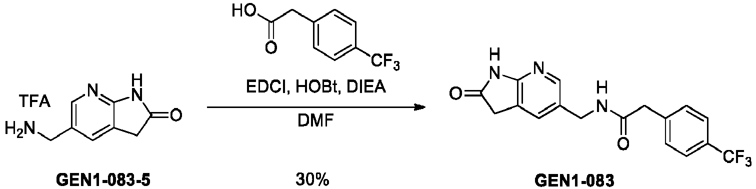

In an ice bath, EDCI (110 mg, 0.574 mmol) was added to a mixture of 5-(aminomethyl)-1H-pyrrolo[2,3-b]pyridin-2-2(3H)-1,2,2,2-trifluoroacetate (105 mg, 0.379 mmol), 2-(4-(trifluoromethyl)phenyl)acetic acid (78 mg, 0.38 mmol), HOBt (77 mg, 0.57 mmol) and DIEA (245 mg, 1.90 mmol) in DMF (3 mL). The resulting mixture was stirred at room temperature overnight. The reaction mixture was purified by a C18 column (CH₃CN: water = 5% to 55%) to obtain the desired compound (40.8 mg, yield 30%) as a white solid. ¹HNMR (400 MHz, DMSO-*d*₆): δ 10.93 (s, 1H), 8.59 (t, *J=* 5.6 Hz, 1H), 7.93 (d, *J=* 1.6 Hz, 1H), 7.67 (d, *J=* 8.0 Hz, 2H), 7.48 (d, *J=* 8.0 Hz, 2H), 7.41 (s, 1H), 4.20 (d, *J=* 6.0 Hz, 2H), 3.57 (s, 2H), 3.52 (s, 2H). MS (ESI): Rt =3.047 min, m/z 350.1 [M+H]⁺; Purity: 98.89% @ 254 nm, 98.77% @ 214 nm.

### Example 77: N-((3-oxoisoindol-5-yl)methyl)-2-(4-(trifluoromethyl)phenyl)acetamide

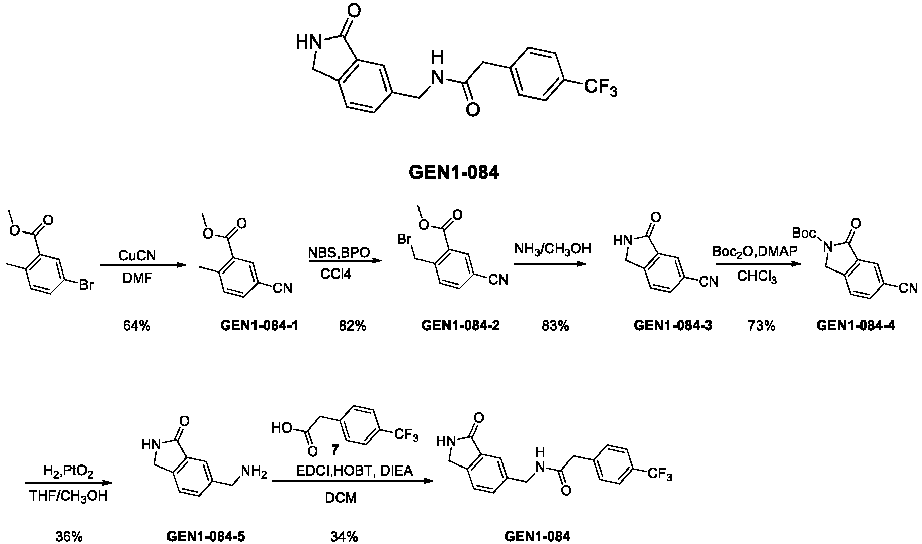

### Step 1. Synthesis of methyl 5-cyano-2-methylbenzoate (GEN1-084-1)

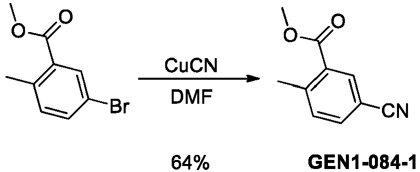

5-bromo-2-methyl-methylbenzoate (2.0 g, 8.73 mmol), KI (290 mg, 1.75 mmol) and CuCN (981 mg, 10.48 mmol) were stirred in DMF (10 mL) at 140°C under nitrogen atmosphere for 5 hours. The mixture was cooled to room temperature and poured into water (30 mL). EtOAc (20 mL×2) was added to extract the desired compound. The combined organic phase was washed with brine (10 mL), dried over Na₂SO₄ and it was concentrated. The residue was purified by silica gel column chromatography (PE:EA = 10:1) to obtain the desired compound (1.0 g, yield 64%) as a yellow solid. ¹H NMR (300 MHz, CDCl₃): δ 8.22 (d, *J =* 1.2 Hz, 1H), 7.67 (dd, *J =* 8.1, 1.5 Hz, 1H), 7.38 (d, *J=* 8.1 Hz, 1H), 3.93 (s, 3H), 2.68 (s, 3H).

### Step 2. Synthesis of methyl 2-(bromomethyl)-5-cyanobenzoate (GEN1-084-2)

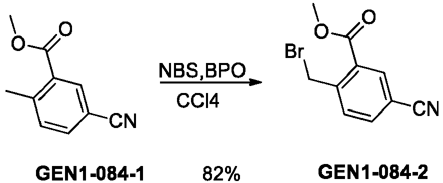

A solution of methyl 5-cyano-2-methylbenzoate (650 mg, 3.71 mmol), NBS (660 mg, 3.71 mmol) and BPO (90 mg, 0.37 mmol) in CCl4 (10 mL) was stirred at 80°C for 2 hours. The mixture was cooled and filtered. The filtrate was poured into water (10 mL) and extracted with PE/EA (5/1, 30 mL×2). The combined organic phase was dried over Na₂SO₄, filtered and it was concentrated to obtain the desired compound (1.1 g, yield 82%) as a yellow oil, which was used directly in the next step. ¹H NMR (400 MHz, CDCl₃): δ 8.27 (d, *J =* 1.6 Hz, 1H), 7.77 (dd, *J =* 8.0, 1.6 Hz, 1H), 7.61 (d, *J=* 8.4 Hz, 1H), 4.96 (s, 2H), 3.99 (s, 3H).

### Step 3. Synthesis of 3-oxoisoindoline-5-carbonitrile (GEN1-084-3)

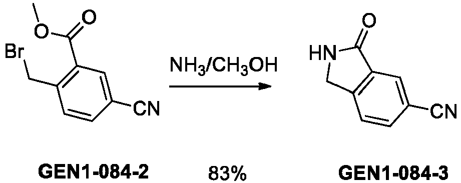

A solution of the compound methyl 2-(bromomethyl)-5-cyanobenzoate (1.1 g, 3.03 mmol) in NH3/MeOH (saturated 20 mL) was stirred at room temperature for 1 hour and then at 70°C for 2 hours. The mixture was cooled and concentrated. The residue was triturated with water (5 mL) and filtered. The filter cake was dried to obtain the desired compound (400 mg, yield 83%) as a yellow solid. ¹H NMR (400 MHz, DMSO-*d*₆): δ 8.86 (s, 1H), 8.11 (s, 1H), 8.05 (dd, *J* = 8.0, 1.6 Hz, 1H), 7.81 (d, *J* = 7.6 Hz, 1H), 4.49 (s, 2H).

### Step 4. Synthesis of tert-butyl 6-cyano-1-oxoisoindoline-2-carboxylate (GEN1-084-4)

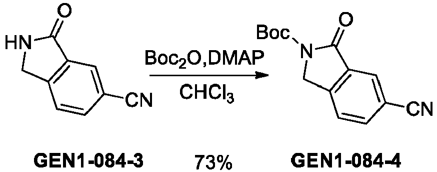

A mixture of 3-oxoisoindoline-5-carbonitrile (400 mg, 2.53 mmol), Boc2O (828 mg, 3.79 mmol), TEA (512 mg, 5.06 mmol) and DMAP (31 mg) and CHCl3 (15 mL) was refluxed for 5 hours. The mixture was concentrated. The residue was purified by column chromatography on silica gel (PE:EA = 1:1) to obtain the desired compound (500 mg, yield 73%) as a yellow solid. ¹H NMR (400 MHz, CDCl₃): δ 8.20 (s, 1H), 7.91 (dd, *J =* 8.0, 1.2 Hz, 1H), 7.63 (d, *J =* 8.0 Hz, 1H), 4.85 (s, 2H), 1.61 (s, 9H). MS (ESI) Rt = 1.400 min, *m*/*z* 203.0 [M+H-56]⁺; Purity: 94.6% @ 214 nm.

### Step 5. Synthesis of 6-(aminomethyl)isoindoline-1-one (GEN1-084-5)

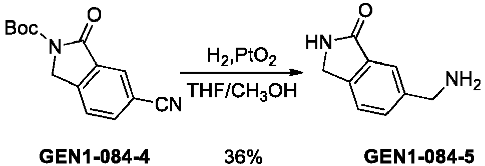

A solution of tert-butyl 6-cyano-1-oxo-isoindoline-2-carboxylate (150 mg, 581 mmol) and PtO2 (30 mg) in THF (2 mL) and methanol (2 mL) was kept at 50 °C under H₂ atmosphere overnight. The mixture was cooled and filtered. The filtrate was concentrated to obtain the crude product of 6-(aminomethyl)isoindoline-1-one (100 mg, 0.21 mmol, yield 36%, purity 60%) as a yellow oil. MS (ESI) Rt = 0.29, 0.34 min, *m*/*z* 163.0 [M+H]⁺; Purity: 60% @ 254 nm, 60% @ 214 nm.

### Step 6. N-((3-oxoisoindol-5-yl)methyl)-2-(4-(trifluoromethyl)phenyl)acetamide (GEN1-084)

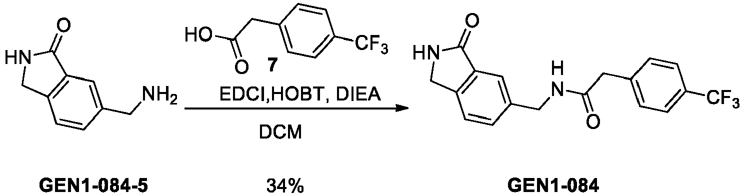

HOBT (42 mg, 0.31 mmol) and EDCI (60 mg, 0.31 mmol) was added to a solution of 6-(aminomethyl)isoindoline-1-one (100 mg, 0.37 mmol, purity 60%), 2-[4-(trifluoromethyl)phenyl]acetic acid (51 mg, 0.25 mmol) and TEA (106 mg, 1.05 mmol) in DCM (5 mL). The mixture was stirred at room temperature for 2 hours. The reaction mixture was diluted with water (10 mL), and the mixture was extracted with DCM (10 mL×2). The combined organic phase was washed with brine, dried over sodium sulfate and it was concentrated. The residue was purified by C18 column chromatography (30 minutes in water, to 60% MeCN, for 15 minutes) to obtain the desired compound (30 mg, yield 34%) as a white solid. ¹H NMR (400 MHz, DMSO-*d*₆): δ 8.70 (t, *J=* 5.6 Hz, 1H), 8.52 (s, 1H), 7.68-7.66 (m, 2H), 7.56 (s, 1H), 7.51-7.49 (m, 3H), 7.46-7.44 (m, 1H), 4.37 (d, *J=* 6.0 Hz, 2H), 4.33 (s, 2H), 3.62 (s, 2H). LCMS (ESI) Rt = 3.413 min, *m*/*z* 349.1 [M+H]⁺; Purity: 85.0% @ 254 nm, 95.2% @ 214 nm.

### Example 78: 1-(2-(4-hydroxy-4-(4-(trifluoromethyl)phenyl)piperidin-1-yl)-2-oxoethyl)-1H-benzo[d]imidazol-2(3H)-one

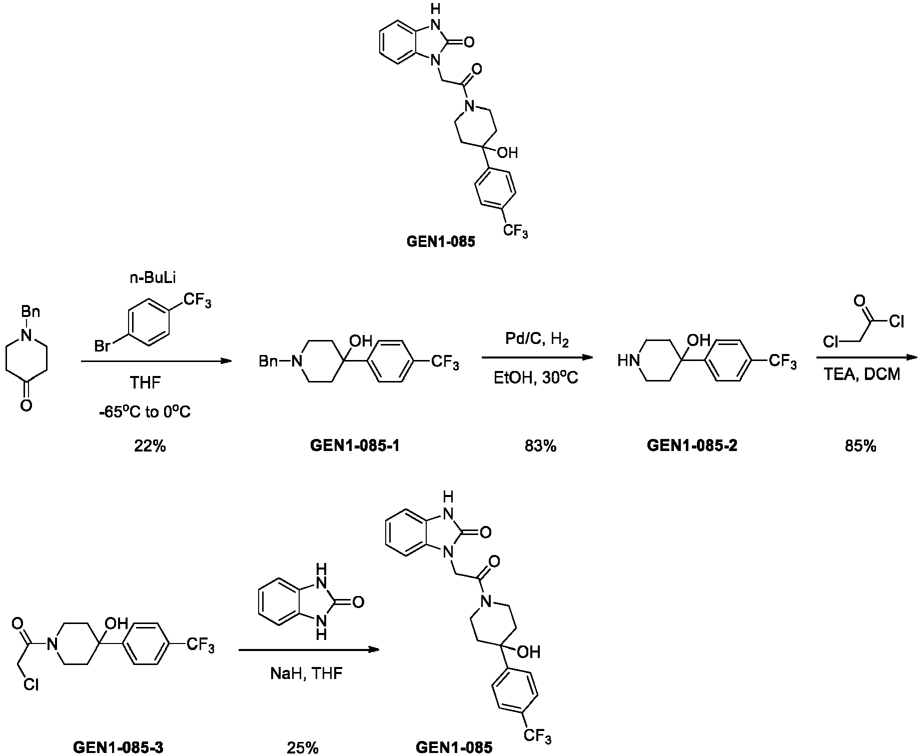

### Step 1. Synthesis of 1-benzyl-4-(4-(trifluoromethyl)phenyl)piperidin-4-ol (GEN1-085-1)

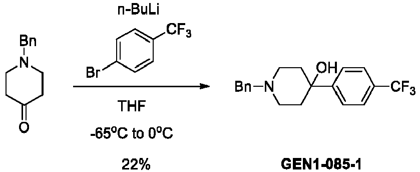

In a dry ice-acetone bath (-65°C) and under N₂ atmosphere, a solution of 1 M n-BuLi in hexane (6.3 mL, 6.3 mmol) was added dropwise to a solution of 1-bromo-4-(trifluoromethyl)benzene (1.43 g, 6.34 mmol) in THF (10 mL). The resulting mixture was stirred at -65°C for 1 hour. Then 1-benzylpiperidin-4-one (1.0 g, 5.3 mmol) in THF (2 mL) was added dropwise to the mixture. The resulting mixture was stirred at -65°C for 30 minutes, and then at 0°C for 2 hours. The reaction mixture was poured into water (50 mL), and extracted with EtOAc (30 mL×3). The combined organic layer was washed with brine (30 mL), dried over MgSO₄, filtered and it was concentrated. The crude product was purified by a C18 column (CH₃CN: water = 5% to 50%) to obtain the desired compound (430 mg, yield 22%) as a colorless oil. MS (ESI): Rt = 1.66 min, *m*/*z* 336.2 [M+H]⁺; Purity: 100% @ 254 nm, 90% @ 214 nm.

### Step 2. Synthesis of 4-(4-(trifluoromethyl)phenyl)piperidin-4-ol (GEN1-085-2)

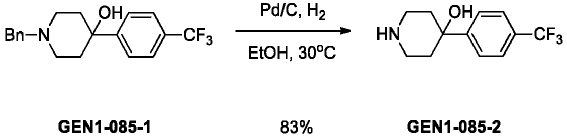

Pd/C (14 mg, 0.013 mmol, purity 10%) was add to a sulotion of 1-benzyl-4-[4-(trifluoromethyl)phenyl]piperidin-4-ol (430 mg, 1.28 mmol) in EtOH (10 mL). The resulting mixture was degassed with H₂ and stirred at 30°C overnight. The reaction mixture was filtered, and the filtrate was concentrated to obtain the desired compound (300 mg, yield 83%) as a gray solid. ¹HNMR (400 MHz, DMSO-*d*₆): δ 7.71-7.66 (m, 4H), 5.03 (s, 1H), 2.95-2.89 (m, 2H), 2.75-2.73 (m, 2H), 1.81 (td, *J =* 12.8, 4.8 Hz, 2H), 1.51-1.48 (m, 2H). MS (ESI): Rt = 1.17 min, *m*/*z* 246.1 [M+H]⁺; Purity: 94% @ 254 nm, 87% @ 214 nm.

### Step 3. Synthesis of 2-chloro-1-(4-hydroxy-4-(4-(trifluoromethyl)phenyl)piperidin-1-yl)ethenone (GEN1-085-3)

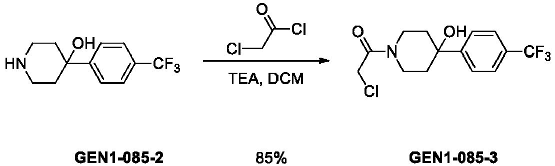

In a salt ice bath, 2-chloroacetyl chloride (46 mg, 0.41 mmol) was added to a solution of 4-[4-(trifluoromethyl)phenyl]piperidin-4-ol (100 mg, 0.408 mmol) and TEA (124 mg, 1.22 mmol) in DCM (4 mL). The resulting mixture was stirred at room temperature for 5 hours. The reaction mixture was poured into water (50 mL), and extracted with DCM (25 mL×3). The combined organic layer was washed with brine (25 mL), dried over MgSO₄, filtered and it was concentrated to obtain the desired compound (130 mg, yield 85%) as a brown oil. ¹HNMR (400 MHz, CDCl₃): δ 7.66-7.59 (m, 4H), 4.59-4.53 (m, 1H), 3.84-3.78 (m, 1H), 3.73-3.64 (m, 1H), 3.23-3.14 (m, 1H), 2.68-2.61 (m, 2H), 2.13-2.01 (m, 3H), 1.89-1.79 (m, 2H).

### Step 4. Synthesis of 1-(2-(4-hydroxy-4-(4-(trifluoromethyl)phenyl)piperidin-1-yl)-2-oxoethyl)-1H-benzo[d]imidazol-2(3H)-one (GEN1-085)

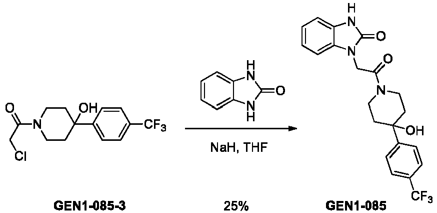

In an ice bath, NaH (24 mg, 0.61 mmol, 60% in mineral oil) was added to a solution of 1,3-dihydrobenzimidazole-2-one (65 mg, 0.48 mmol) in THF (4 mL). The resulting mixture was stirred for 15 minutes in an ice bath. Then, a solution of 2-chloro-1-[4-hydroxy-4-(4-(trifluoromethyl)phenyl]-1piperidinyl)ethanone (130 mg, 0.404 mmol) in THF (1 mL)was added dropwise in an ice bath. The resulting mixture was stirred at room temperature overnight. The reaction mixture was poured into water (50 mL), and extracted with EtOAc (25 mL×3). The combined organic layer was washed with brine (25 mL), dried over MgSO₄, filtered and it was concentrated. The crude product was purified by a C18 column (CH₃CN: water = 5% to 45%) to obtain the desired compound (43.6 mg, yield 25%) as a white solid. ¹HNMR (400 MHz, DMSO-*d*₆): δ 10.84 (s, 1H), 7.73-7.66 (m, 4H), 7.07-6.97 (m, 4H), 5.42 (s, 1H), 4.80 (d, *J =* 16.8 Hz, 1H), 4.69 (d, *J* = 16.8 Hz, 1H), 4.29-4.26 (m, 1H), 3.94-3.90 (m, 1H), 3.55-3.49 (m, 1H), 3.06-2.99 (m, 1H), 2.02 (td, *J* = 12.8, 4.4 Hz, 1H), 1.80 (td, *J* = 13.2, 4.4 Hz, 1H), 1.65 (t, J = 13.6 Hz, 2H). MS (ESI): Rt = 2.822 min, *m*/*z* 420.1 [M+H]⁺; Purity: 94.50% @ 254 nm, 98.67% @ 214 nm.

### Example 79: 6-(2-oxo-2,3-dihydro-1H-benzo[d]imidazol-1-yl)-2-(4-(trifluoromethyl)phenyl)isoquinoline-1 (2H)-one

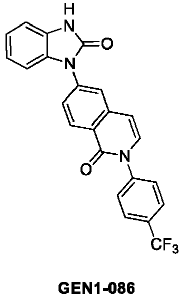

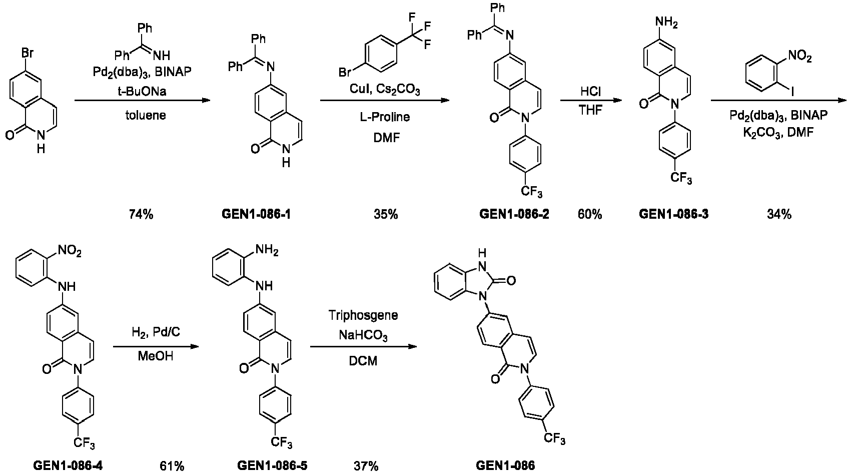

### Step 1. Synthesis of 6-((diphenylmethylene)amino)isoquinoline-1(2H)-one (GEN1-086-1)

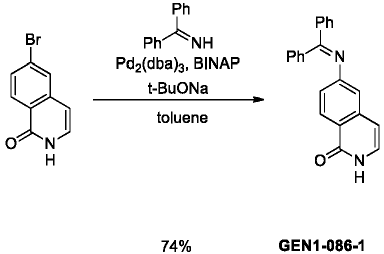

T-BuONa (3.60 g, 37.5 mmol) was added to a solution of 6-bromo-2H-isoquinoline-1-one (2.8 g, 13 mmol), diphenylformimide (2.72 g, 15.0 mmol), BINAP (1.17 g, 1.87 mmol), Pd2(dba)3 (1.29 g, 1.25 mmol) in toluene (45 mL). The mixture was stirred at 105°C under N₂ for 12 hours. The mixture was then cooled to room temperature and poured into water (100 mL). DCM (40 mL×3) was added to extract the desired compound. The combined organic layer was washed with brine (40 mL), dried over Na₂SO₄ and it was concentrated to obtain the crude product. The crude product was purified by column chromatography on silica gel (DCM:MeOH=30:1) to obtain the desired compound (3.0g, yield 71%) as a yellow solid. MS (ESI): Rt = 1.55 min, *m*/*z* 325.3 [M+H]⁺; Purity: 100% @ 254 nm, 100% @ 214 nm.

### Step 2. Synthesis of 6-((diphenylmethylene)amino)-2-(4-(trifluoromethyl)phenyl)isoquinoline-1(2H)-one (GEN1-086-2)

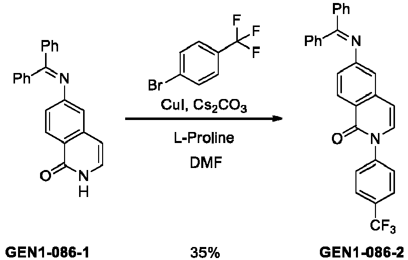

CS₂CO₃ (5.62 g, 17.3 mmol) was added to a mixture of 6-(phenyldimethylimino)-2H-isoquinoline-1-one (2.8 g, 8.6 mmol), 1-bromo-4-(trifluoromethyl)benzene (3.88 g, 17.3 mmol), L-proline (202 mg, 0.864 mmol), CuI (165 mg, 0.866 mmol) in DMF (40 mL). The mixture was stirred at 95°C under N₂ atmosphere for 12 hours. The mixture was cooled to room temperature, and then poured into water (80 mL), and extracted with DCM (50 mL) three times. The combined organic layer was concentrated to obtain a crude product. The crude product was purified by column chromatography on silica gel (PE: EtOAc = 5:1) to obtain the desired compound (1.8 g, yield 35%) as a yellow solid. MS (ESI): Rt = 1.91 min, m/z 469.3 [M+H]⁺; Purity: 84% @ 254 nm, 79% @ 214 nm.

### Step 3. Synthesis of 6-amino-2-(4-(trifluoromethyl)phenyl)isoquinoline-1(2H)-one (GEN1-086-3)

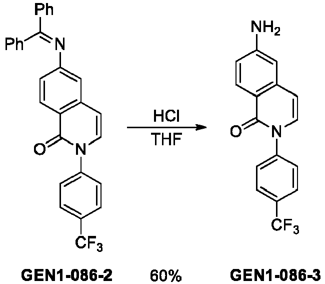

HCl (1.8 mL, 37% aqueous solution) was added to a solution of 6-(phenyldimethylimino)-2-[4-(trifluoromethyl)phenyl]isoquinoline-1-one in THF (15mL) and water (3mL). The mixture was stirred at 25°C for 12 hours, and then basified with aqueous Na₂CO₃ to pH=9 and extracted with EtOAc (30 mL) 3 times. The combined organic layer was washed with brine (30 mL), dried over sodium sulfate and it was concentrated to obtain a crude product. The crude product was purified by column chromatography on silica gel (PE:EtOAc = 1:1) to obtain the desired compound (1.17 g, yield 60%) as a yellow solid. MS (ESI): Rt = 1.52 min, *m*/*z* 305.2 [M+H]⁺; Purity: 100% @ 254 nm, 100% @ 214 nm.

### Step 4. Synthesis of 6-((2-nitrophenyl)amino)-2-(4-(trifluoromethyl)phenyl)isoquinoline-1(2H)-one (GEN1-086-4)

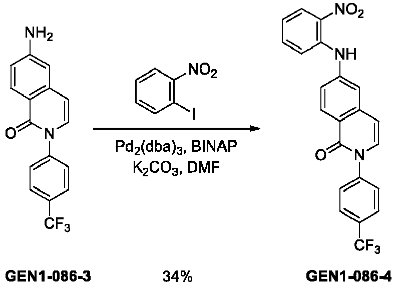

BINAP (215 mg, 0.345 mmol) was added to a mixture of 6-amino-2-[4-(trifluoromethyl)phenyl]isoquinoline-1-one (700 mg, 2.30 mmol), 1-iodo-2-nitrobenzene (630 mg, 2.53 mmol), K₂CO₃ (636 mg, 4.60 mmol) and Pd2(dba)3 (238 mg, 0.230 mmol) in DMF (15 mL). The mixture was stirred at 95°C under N₂ for 8 hours. The mixture was cooled to room temperature and poured into water (25 mL). EtOAc (10 mL×3) was added to extract the desired compound. The combined organic layer was washed with brine (10 mL), dried over Na₂SO₄ and it was concentrated to obtain the crude product. The crude product was purified by silica gel column chromatography (PE:EA=3:1) to obtain the desired compound (350 mg, yield 34%) as a yellow solid. MS (ESI): Rt = 1.77 min, m/z 426.3 [M+H]⁺; Purity: 100% @ 254 nm, 100% @ 214 nm.

### Step 5. Synthesis of 6-((2-aminophenyl)amino)-2-(4-(trifluoromethyl)phenyl)isoquinoline-1(2H)-one (GEN1-086-5)

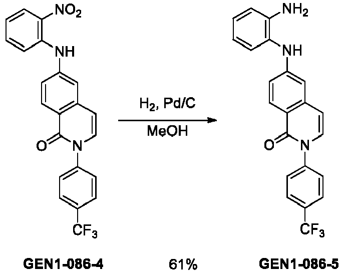

Pd/C (10%, 50 mg) was added to a solution of 6-(2-nitroanilino)-2-[4-(trifluoromethyl)phenyl]isoquinoline-1-one (350 mg, 0.823 mmol) in MeOH (5 mL). The mixture was stirred at 25°C under H₂ for 6 hours. The mixture was filtered, and the filtrate was concentrated to obtain the desired compound (200 mg, yield 61%). MS (ESI): Rt = 1.64 min, m/z 396.3 [M+H]⁺; Purity: 100% @ 254 nm, 100 % @ 214 nm.

### Step 6. Synthesis of 6-(2-oxo-2,3-dihydro-1H-benzo[d]imidazol-1-yl)-2-(4-(trifluoromethyl)phenyl)isoquinoline-1(2H)-one (GEN1-086)

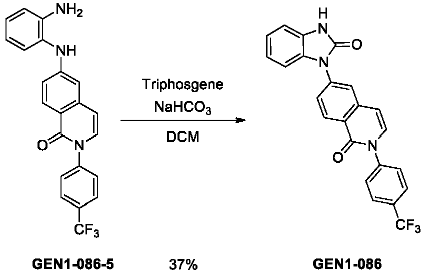

In an ice bath, triphosgene (120 mg, 0.404 mmol) was added to a solution of 6-(2-aminoanilino)-2-[4-(trifluoromethyl)phenyl]isoquinoline-1-one (200 mg, 0.506 mmol) and NaHCO₃ in DCM (6 mL). The mixture was stirred at 25°C for 6 hours, and then poured into water (25 mL), and extracted with DCM (15 mL) 3 times. The combined organic layer was purified by a C18 column (CH₃CN: water = 5% to 50%) to obtain the desired compound (80 mg, yield 37%) as a white solid. ¹H NMR (400 MHz, DMSO-*d*₆): δ 11.32 (s, 1H), 8.41 (d, *J=* 8.8 Hz, 1H), 7.99 (s, 1H), 7.93 (d, *J =* 8.4 Hz, 2H), 7.80-7.78 (m, 3H), 7.60 (d, *J* = 7.6 Hz, 1H), 7.20 (d, *J=* 8.0 Hz, 1H), 7.13-7.07 (m, 3H), 6.87 (d, *J=* 7.2 Hz, 1H). MS (ESI): Rt = 3.013 min, *m*/*z* 422.1 [M+H]⁺; Purity: 98.59% @ 254 nm, 98.91 % @ 214 nm.

### Example 80: 6-(tert-butyl)-1-(1-(2-(4-(trifluoromethyl)phenyl)acetyl)piperidin-4-yl)-1H-benzo[d]imidazol-2(3H)-one

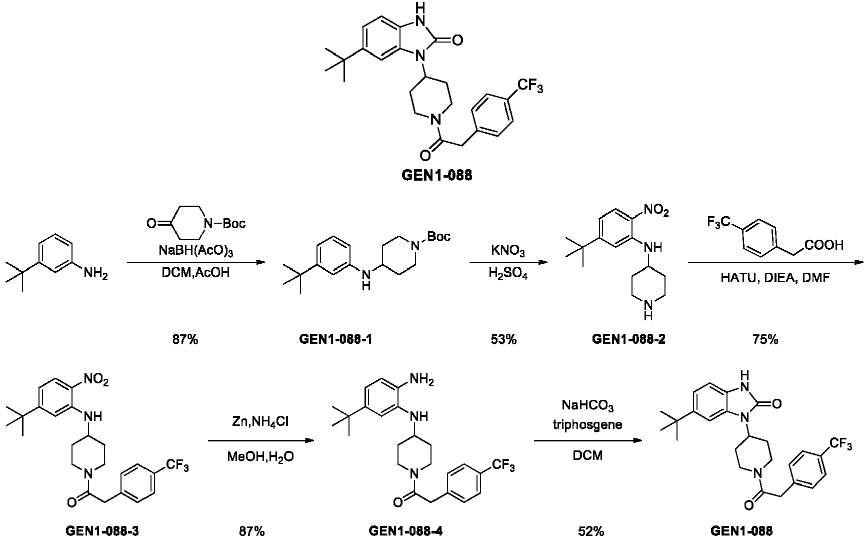

### Step 1. Synthesis of tert-butyl 4-((3-(tert-butyl)phenyl)amino)piperidin-1-carboxylate (GEN1-088-1)

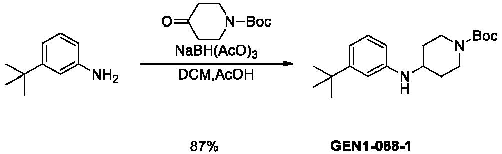

Sodium triacetoxyborohydride (5.68 g, 26.8 mmol) was added to a solution of 3-tert-butylaniline (2.00 g, 13.4 mmol) and tert-butyl 4-oxopiperidine-1-carboxylate (2.94g, 14.7mmol) in DCM (50 mL) and AcOH (3.22 g, 53.6 mmol) at 0°C. The mixture was then stirred at 25°C overnight. The reaction mixture was diluted with water (200 mL), and extracted with EtOAc (100 mL×2). The combined organic layer was dried over anhydrous Na₂SO₄ and concentrated. The residue was purified by silica gel flash chromatography (petroleum: ethyl acetate = 10:1) to obtain the desired compound (4.0 g, yield 87%) as a yellow solid. MS (ESI): Rt = 1.859 min, *m*/*z* 333.2 [M+H]⁺; Purity: 96.87% @ 254 nm.

### Step 2. Synthesis of N-(5-(tert-butyl)-2-nitrophenyl)piperidin-4-amine (GEN1-088-2)

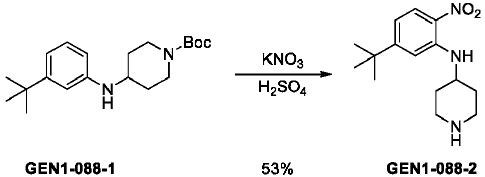

At 0°C, tert-butyl 4-(3-tert-butylanilino)piperidin-1-carboxylate (1.00 g, 3.01 mmol) and potassium nitrate (487 mg, 4.81 mmol) were added into a glass flask, and then H₂SO₄ (10 mL) was slowly added. Then the mixture was stirred at the same temperature for 1.5 hours. The resulting mixture was slowly poured into ice water (100 mL) and stirred for 30 minutes. The mixture was then filtered and the filter cake was washed with DCM (100 mL). The filtrate was extracted with DCM (100 mL×4). The combined organic layer was dried over anhydrous Na₂SO₄ and concentrated. The residue was purified by a silica gel column (DCM:MeOH=20:1) to obtain the desired compound (550 mg, yield 53%) as a yellow solid. MS (ESI): Rt = 1.46 min, *m*/*z* 278.2 [M+H]⁺; Purity: 95% @ 254 nm, 81% @ 214 nm.

### Step 3. Synthesis of 1-(4-((5-(tert-butyl)-2-nitrophenyl)amino)piperidin-1-yl)-2-(4-(trifluoromethyl)phenyl)ethanone (GEN1-088-3)

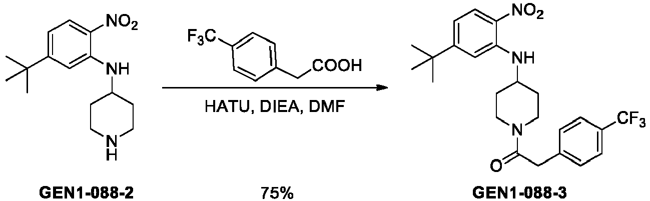

HATU (925 mg, 2.43 mmol) was added to a solution of N-(5-(tert-butyl-2-nitrophenyl)piperidin-4-amine (450 mg , 1.62 mmol), 2-(4-(trifluoromethyl)phenyl)acetic acid (397 mg, 1.95 mmol) DIEA (629 mg, 4.87 mmol) in DMF (5mL). The mixture was stirred at 25°C for 2 hours. Water (20 mL) was added to the resulting mixture, and EtOAc (100 mL×3) was added to extract the desired compound. The combined organic layer was washed with brine (50 mL), and the resulting mixture was dried over anhydrous Na₂SO₄ and it was concentrated. The residue was purified by a silica gel column (PE:EA = 1:1) to obtain the desired compound (565 mg, yield 75%) as a yellow oil. MS (ESI): Rt = 1.88 min, *m*/*z* 464.5 [M+H]⁺; Purity: 100% @ 254 nm, 85% @ 214 nm.

### Step 4. Synthesis of 1-(4-((2-amino-5-(tert-butyl)phenyl)amino)piperidin-1-yl)-2-(4-(trifluoromethyl)phenyl)ethenone (GEN1-088-4)

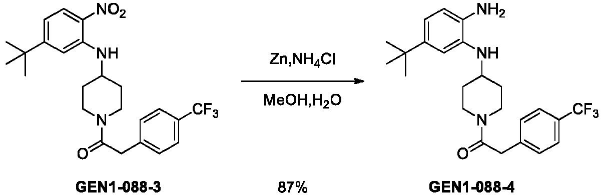

NH₄Cl (978 mg, 18.3 mmol) and zinc powder (797 mg, 12.2 mmol) were added to a suspension of 1-(4-((5-(tert-butyl)-2-nitrophenyl)amino)piperidin-1-yl)-2-(4-(trifluoromethyl)phenyl)ethanone (565 mg, 1.22 mmol) in water (2.5mL) and methanol (10mL). The mixture was stirred at 65°C for 2.5 hours. Then water (25 mL) was added, and the mixture was extracted with EtOAc (50 mL×3). The combined organic layer was washed with brine (50 mL), dried over anhydrous Na₂SO₄ and it was evaporated to obtain the desired compound (506 mg, yield 87%) as a purple oil. MS (ESI) Rt = 1.75 min, *m*/*z* 434.5 [M+H]⁺; Purity: 83% @ 254 nm, 91% @ 214 nm.

### Step 5. Synthesis of 6-(tert-butyl)-1-(1-(2-(4-(trifluoromethyl)phenyl)acetyl)piperidin-4-yl)-1H-benzo[d]imidazol-2(3H)-one (GEN1-088)

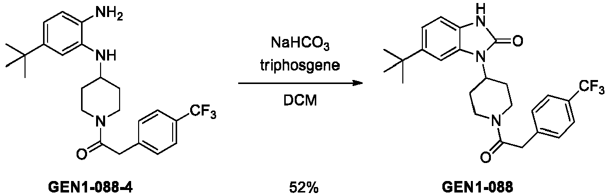

Triphosgene (86 mg, 0.29 mmol) was slowly added to a suspension of 1-(4-((2-amino-5-(tert-butyl)phenyl)amino)piperidin-1-yl)-2-(4-(trifluoromethyl)phenyl)ethenone (250 mg, 0.577 mmol) and NaHCO₃ (484 mg, 5.77 mmol) in DCM (5 mL) at 0°C. The mixture was stirred at 0°C for 1.5 hours. The resulting mixture was concentrated to dryness. The residue was diluted with water (50 mL), and extracted with EtOAc (30 mL×3). The combined organic layer was washed with brine (30 mL), dried over anhydrous Na₂SO₄ and it was concentrated. The residue was purified by preparation. HPLC (preparation 5 Xbridge C18 5um 19 * 150mm, 35%-80% B; A: H₂O (0.1% NH₄HCO₃), B: CH₃CN; UV: 214nm, flow rate: 15mL / min). The desired compound (138 mg, yield 52%) was obtained as a white solid. ¹H NMR (400 MHz, DMSO-*d*₆): δ 10.69 (s, 1H), 7.68 (d, *J=* 8.0 Hz, 2H), 7.51 (d, *J=* 8.0 Hz, 2H), 7.08 (s, 1H), 7.00 (dd, *J=* 8.0, 1.6 Hz, 1H), 6.87 (d, *J=* 8.4 Hz, 1H), 4.57 (d, *J* = 12.8 Hz, 1H), 4.46-4.40 (m, 1H), 4.15 (d, *J=* 12.8 Hz, 1H), 3.97-3.87 (m, 2H), 3.21 (t, *J =* 12.4 Hz, 1H), 2.73 (t, *J =* 12.4 Hz, 1H), 2.21-2.14 (m, 2H), 1.72 (d, *J =* 12.0 Hz, 2H), 1.27 (s, 9H). MS (ESI): Rt = 4.056 min, *m*/*z* 460.3 [M+H]⁺; Purity: 78.90% @ 254 nm, 99.53% @ 214 nm.

### Example 81: 6-cyclopropyl-1-(1-(2-(4-(trifluoromethyl)phenyl)acetyl)piperidin-4-yl)-1H-benzo[d]imidazol-2(3H)-one

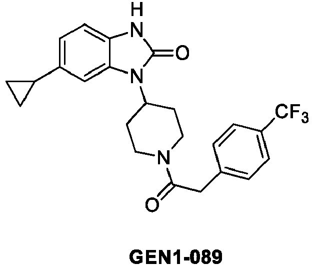

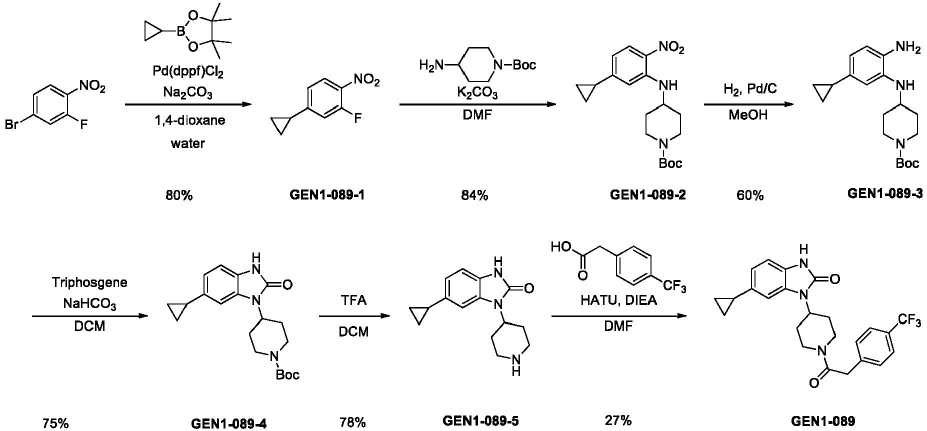

### Step 1. Synthesis of 4-cyclopropyl-2-fluoro-1-nitrobenzene (GEN1-089-1)

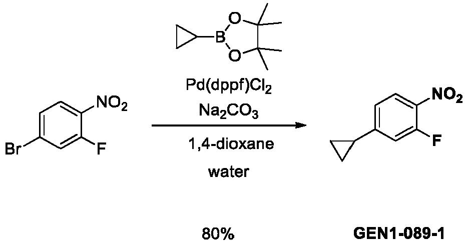

A mixture of 4-bromo-2-fluoro-1-nitrobenzene (3.0 g, 14 mmol) and 2-cyclopropyl-4,4,5,5-tetramethyl-[1,3,2]dioxaborane (2.52 g, 15.0 mmol), Pd(dppf)Cl2 (1.11 g, 1.36 mmol) and Na₂CO₃ (2.89 g, 27.3 mmol) were stirred in a solution of 1,4-dioxane (20 mL) and water (5 mL) at 80°C under N₂ atmosphere for 8 hours. The mixture was cooled to room temperature, and then poured into water (50 mL), and extracted with EtOAc (30 mL) three times. The combined organic layer was washed with brine (30 mL), dried over sodium sulfate and it was concentrated to obtain a crude product. The crude product was purified by silica gel column chromatography (PE:EA = 10:1) to obtain the desired compound (2.2 g, yield 80%) as a yellow solid. ¹H NMR (400 MHz, CDCl₃): δ 8.02 (t, *J=* 11.2 Hz, 1H), 7.00-6.92 (m, 2H), 2.06-1.97 (m, 1H), 1.25-1.18(m, 2H), 0.90-0.84 (m, 2H).

### Step 2. Synthesis of tert-butyl 4-((5-cyclopropyl-2-nitrophenyl)amino)piperidin-1-carboxylate (GEN1-089-2)

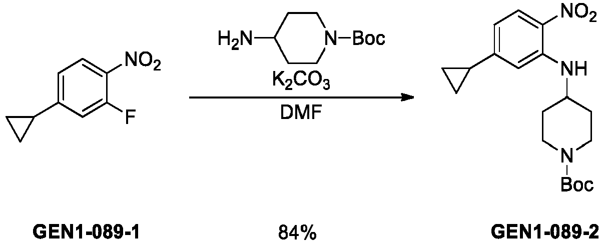

K₂CO₃ (2.29 g, 16.6 mmol) was added to a mixture of 4-cyclopropyl-2-fluoro-1-nitrobenzene (1.5 g, 8.3 mmol) and tert-butyl 4-aminopiperidine-1-carboxylate (1.66 g, 8.28 mmol) in DMF (20 mL). The mixture was stirred at 95°C for 6 hours, and then cooled to room temperature. The mixture was poured into water (50 mL) and extracted with EtOAc (30 mL) three times. The combined organic layer was washed with brine (30 mL), dried over Na₂SO₄ and it was concentrated to obtain the desired compound (2.5 g, yield 84%) as a yellow solid. MS (ESI): Rt = 1.86 min, *m*/*z* 306.3, [M+H-56]⁺; Purity: 100% @ 254 nm, 100% @ 214 nm.

### Step 3. Synthesis of tert-butyl 4-((2-amino-5-cyclopropylphenyl)amino)piperidin-1-carboxylate (GEN1-089-3)

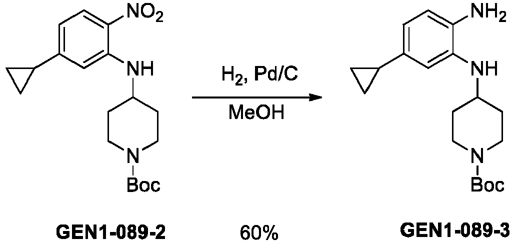

Pd/C (100 mg, 10%) was added to a solution of tert-butyl 4-((5-cyclopropyl-2-nitrophenyl)amino)piperidin-1-carboxylate (1.5 g, 4.2 mmol) in MeOH (20 mL). The mixture was stirred at 25°C under H₂ for 6 hours. The mixture was filtered, and the filtrate was concentrated to obtain the desired compound (900 mg, yield 60%) as a white solid. MS (ESI): Rt = 1.69 min, *m*/*z* 332.1, [M+H]⁺; Purity: 92% @ 254 nm, 74% @ 214 nm.

### Step 4. Synthesis of tert-butyl 4-(6-cyclopropyl-2-oxo-2,3-dihydro-1H-benzo[d]imidazol-1-yl)piperidin-1-carboxylate (GEN1-089-4)

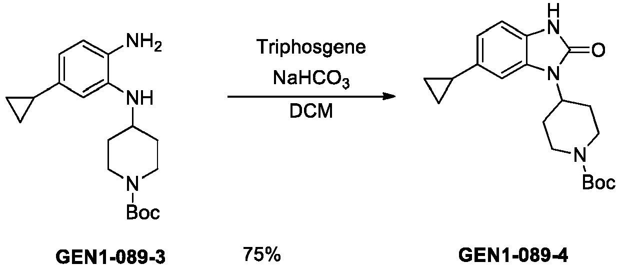

In an ice bath, triphosgene (500 mg, 1.68 mmol) was added to a mixture of tert-butyl 4-((2-amino-5-cyclopropylphenyl)amino) piperidin-1-carboxylate (700 mg, 2.11 mmol) and NaHCO₃ (1.77 g, 21.1 mmol) in DCM (15 mL). The mixture was stirred at room temperature for 2 hours, and then poured into water (30 mL), and extracted with DCM (15 mL) three times. The combined organic layer was washed with brine (15 mL), dried over Na₂SO₄ and it was concentrated to obtain the desired compound (650 mg, yield 75%) as a white solid. MS (ESI): Rt = 1.50 min, *m*/*z* 356.2, [M-H]⁻; Purity: 100% @ 254 nm, 87% @ 214 nm.

### Step 5. Synthesis of 6-cyclopropyl-1-(piperidin-4-yl)-1H-benzo[d]imidazol-2(3H)-one (GEN1-089-5)

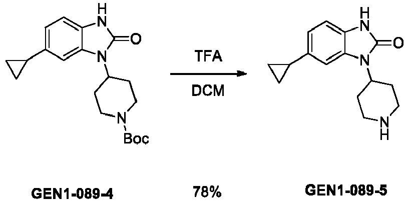

TFA (1.5 mL) was added to a solution of tert-butyl 4-(6-cyclopropyl-2-oxo-2,3-dihydro-1H-benzo[d]imidazol-1-yl)piperidin-1-carboxylate (220 mg, 0.615 mmol) in DCM (3 mL). The mixture was stirred at 25°C for 12 hours, and then concentrated, basified with aqueous Na₂CO₃ (saturated 5 mL) to pH=9, and extracted with EtOAc (10 mL) 3 times. The combined organic layer was washed with brine (15 mL), dried over sodium sulfate and it was concentrated to obtain the desired compound (150 mg, yield 78%) as a white solid. MS (ESI): Rt = 1.16 min, *m*/*z* 258.1 [M+H]⁺; Purity: 82% @ 254 nm, 97 % @ 214 nm.

### Step 6. Synthesis of 6-cyclopropyl-1-(1-(2-(4-(trifluoromethyl)phenyl)acetyl)piperidin-4-yl)-1H-benzo[d]imidazol-2(3H)-one (GEN1-089)

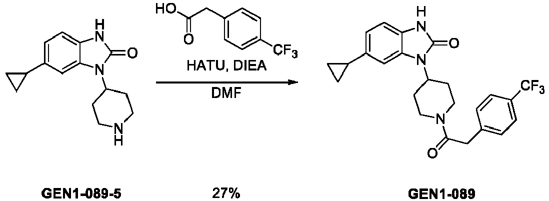

DIEA (226 mg, 1.75 mmol) was added to a solution of 5-cyclopropyl-3-(4-piperidinyl)-1H-benzimidazol-2-one (150 mg, 0.583 mmol), 2-[4-(trifluoromethyl)phenyl] acetic acid (119 mg, 0.583 mmol) and HATU (288 mg, 0.757 mmol) in DMF (2.5 mL). The mixture was stirred at 25°C for 6 hours, and then purified by a C18 column (CH₃CN: water=5% to 50%) to obtain the desired compound (70 mg, yield 27%) as a white solid. ¹H NMR (400 MHz, CDCl₃): δ 9.57 (br s, 1H), 7.63 (d, *J=* 8.0 Hz, 2H), 7.44 (d, *J=* 8.0 Hz, 2H), 6.96 (d, *J=* 8.0 Hz, 1H), 6.77-6.74 (m, 2H), 4.92 (d, *J =* 13.2 Hz, 1H), 4.52-4.44 (m, 1H), 4.06 (d, *J =* 13.6 Hz, 1H), 3.92-3.84 (m, 2H), 3.20 (t, *J=* 12.0 Hz, 1H), 2.73 (t, *J=* 12.0 Hz, 1H), 2.39-2.28 (m, 1H), 2.20-2.09 (m, 1H), 1.95-1.82 (m, 3H), 0.99-0.97 (m, 2H), 0.67-0.60 (m, 2H). MS (ESI): Rt = 3.773 min, *m*/*z* 444.2 [M+H]⁺; Purity: 94.79% @ 254 nm, 99.86 % @ 214 nm.

### Example 82: 6-phenyl-1-(1-(2-(4-(trifluoromethyl)phenyl)acetyl)piperidin-4-yl)-1H-benzo[d]imidazol-2(3H)-one

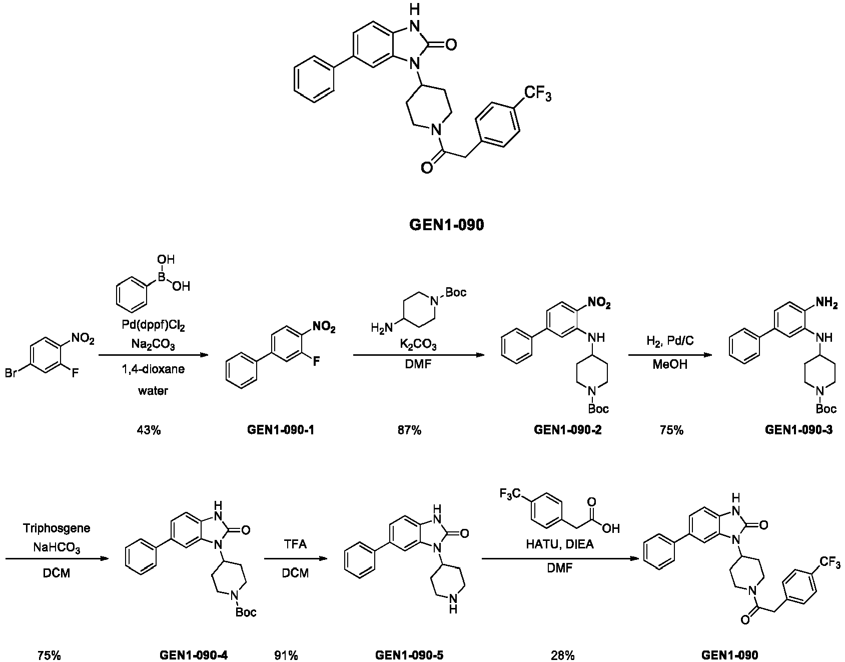

### Step 1. Synthesis of 3-fluoro-4-nitro-1,1'-biphenyl (GEN1-090-1)

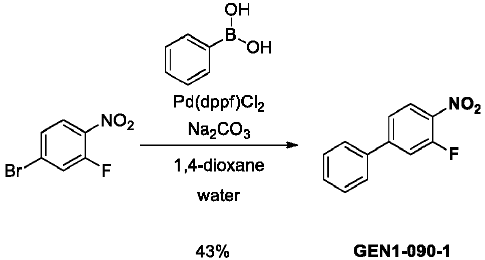

A mixture of 4-bromo-2-fluoro-1-nitrobenzene (1.5 g, 6.8 mmol), phenylboronic acid (832 mg, 6.82 mmol), Pd(dppf)Cl2 (557 mg, 0.682 mmol), Na₂CO₃ (2.17 g, 20.5 mmol) dioxane (15 mL) and water (3 mL) was stirred at 80° C under N₂ for 8 hours. The mixture was cooled to room temperature, and then poured into water (50 mL), and extracted with EtOAc (30 mL) three times. The combined organic layer was washed with brine (30 mL), dried over sodium sulfate and it was concentrated to obtain a crude product. The crude product was purified by silica gel column chromatography (PE:EA = 10:1) to obtain the desired compound (670 mg, yield 43%) as a yellow solid. ¹H NMR (400 MHz, CDCl₃): δ 8.16 (t, *J =* 8.4 Hz, 1H), 7.62-7.60 (m, 2H), 7.53-7.47 (m, 5H).

### Step 2. Synthesis of tert-butyl 4-((4-nitro-[[1,1'-biphenyl]-3-yl)amino)piperidin-1-carboxylate (GEN1-090-2)

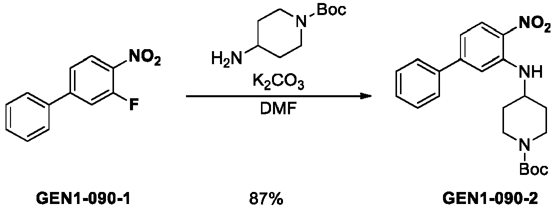

K₂CO₃ (828 mg, 5.99 mmol) was added to a solution of 2-fluoro-1-nitro-4-phenylbenzene (650 mg, 2.99 mmol), tert-butyl 4-aminopiperidine-1-carboxylate (600 mg, 3.00 mmol) in DMF (8 mL). The mixture was stirred at 105°C for 12 hours. The mixture was cooled to room temperature, and then poured into water (20 mL), and extracted with EtOAc (10 mL) three times. The combined organic layer was washed with brine (10 mL), dried over sodium sulfate and it was concentrated to obtain the desired compound (1.1 g, yield 87%) as a yellow solid. MS (ESI): Rt = 1.96 min, *m*/*z* 342.3, [M+H-56]⁺; Purity: 100% @ 254 nm, 94% @ 214 nm.

### Step 3. Synthesis of tert-butyl 4-((4-amino-[[1,1'-biphenyl]-3-yl)amino)piperidin-1-carboxylate (GEN1-090-3)

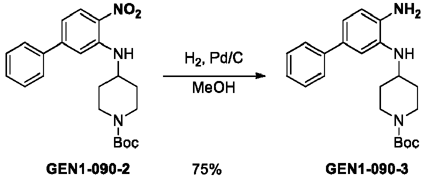

Pd/C (100 mg, 10 %) was added to a solution of 4-(2-nitro-5-phenyl-anilino)piperidin-1-carboxylate (1.1 g, 2.8 mmol) in MeOH (15 mL). The mixture was stirred at 25°C under H₂ for 6 hours. The mixture was filtered, and the filtrate was concentrated to obtain the desired compound (900 mg, yield 75%) as a pink solid. MS (ESI): Rt = 1.43 min, m/z 268.2, [M+H-Boc]⁺; Purity: 89% @ 254 nm, 85% @ 214 nm.

### Step 4. Synthesis of tert-butyl 4-(2-oxo-6-phenyl-2,3-dihydro-1H-benzo[d]imidazol-1-yl)piperidin-1-carboxylate (GEN1-090-4)

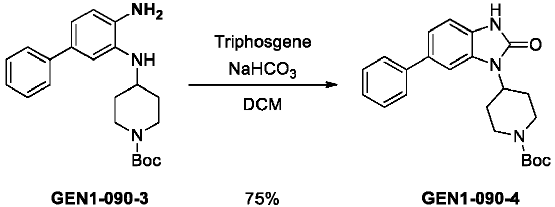

In an ice bath, triphosgene (323mg, 1.09mmol) was added to a solution of tert-butyl 4-(2-amino-5-phenyl-anilino)piperidin-1-carboxylate (500 mg, 1.36 mmol) and NaHCO₃ (1.14g, 13.6mmol) in DCM (10 mL). The mixture was stirred at 25°C for 6 hours, and then poured into water (20 mL), and extracted with DCM (10 mL) three times. The combined organic layer was washed with brine (10 mL), dried over sodium sulfate and it was concentrated to obtain the desired compound (481 mg, yield 75%) as a white solid. MS (ESI): Rt = 1.64 min, m/z 338.1, [M+H-56]⁺; Purity: 100% @ 254 nm, 83% @ 214 nm.

### Step 5. Synthesis of 6-phenyl-1-(piperidin-4-yl)-1H-benzo[d]imidazol-2(3H)-one (GEN1-090-5)

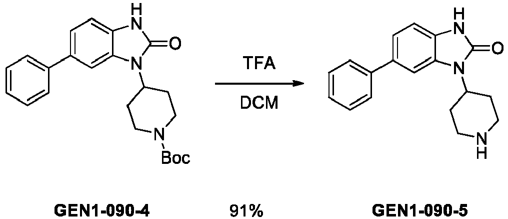

TFA (2 mL) was added to a solution of tert-butyl 4-(2-oxo-6-phenyl-3H-benzimidazol-1-yl)piperidin-1-carboxylate (400 mg, 1.02 mmol) in DCM (4 mL). The mixture was stirred at 25°C for 12 hours, and then concentrated. The residue was dissolved in water (3 mL) and basified with aqueous Na₂CO₃ (saturated 10 mL) to pH=9. The mixture was extracted with DCM (30 mL) three times. The combined organic layer was washed with brine (20 mL), dried over sodium sulfate and it was concentrated to obtain the desired compound (270 mg, yield 91%) as a white solid. MS (ESI): Rt = 1.27 min, m/z 294.2 [M+H]⁺; Purity: 100% @ 254 nm, 100 % @ 214 nm.

### Step 6. Synthesis of 6-phenyl-1-(1-(2-(4-(trifluoromethyl)phenyl)acetyl)piperidin-4-yl)-1H-benzo[d]imidazol-2(3H)-one (GEN1-090)

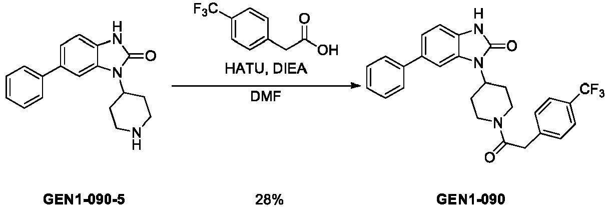

DIEA (200 mg, 1.55 mmol) was added to a solution of 5-phenyl-3-(4-piperidinyl)-1H-benzimidazol-2-one (150 mg, 0.511 mmol), 2-[4-(trifluoromethyl)phenyl]acetic acid (105 mg, 0.514 mmol), HATU (253 mg, 0.665 mmol) in DMF (3 mL). The mixture was stirred at 20°C for 6 hours, and then purified by a C18 column (CH₃CN: water = 5% to 45%) to obtain the desired compound (70 mg, yield 28%) as a white solid. ¹H NMR (400 MHz, DMSO-*d*₆): δ 10.92 (s, 1H), 7.64 (d, *J=* 10.0 Hz, 2H), 7.60 (d, *J=* 8.4 Hz, 2H), 7.49-7.45 (m, 4H), 7.39 (s, 1H), 7.36-7.32 (m, 1H), 7.26 (dd, *J* = 8.0, 1.2 Hz, 1H), 7.05 (d, *J=* 8.0 Hz, 1H), 4.61-4.46 (m, 2H), 4.12-4.09 (m, 1H), 3.96-3.87 (m, 2H), 3.20 (t, *J* = 10.4 Hz, 1H), 2.76-2.69 (m, 1H), 2.26-2.21 (m, 2H), 1.78-1.70 (m, 2H). MS (ESI): Rt = 7.627 min, *m*/*z* 480.1 [M+H]⁺; Purity: 99.56% @ 254 nm, 99.29 % @ 214 nm.

### Example 83: 7-nitro-1-(1-(2-(4-(trifluoromethyl)phenyl)acetyl)piperidin-4-yl)-1H-benzo[d]imidazol-2(3H)-one

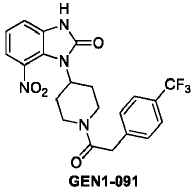

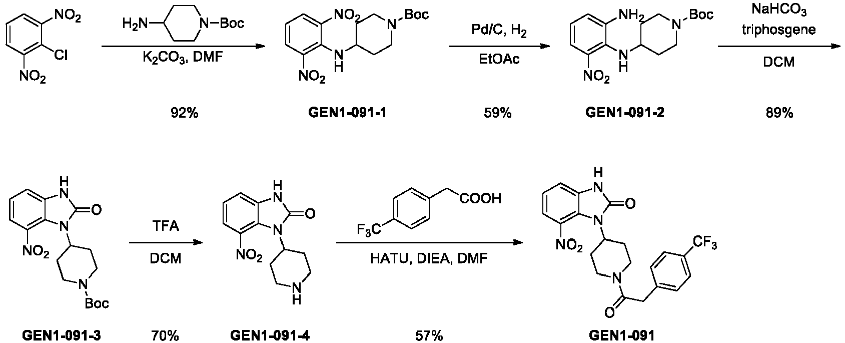

### Step 1. Synthesis of tert-butyl 4-(2,6-dinitroanilino)piperidin-1-carboxylate (GEN1-091-1)

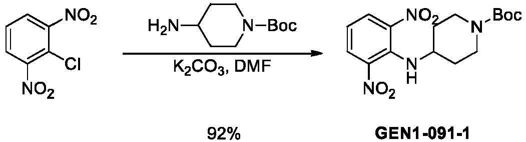

Tert-butyl 4-aminopiperidine-1-carboxylate (1.09 g, 5.43 mmol) was added to a suspension of 2-chloro-1,3-dinitrobenzene (1.00 g, 4.94 mmol) and K₂CO₃ (1.02 g, 7.41 mmol) in DMF (10 mL). The mixture was stirred at 70°C for 3.5 hours. The reaction mixture was diluted with water (100 mL), and extracted with EtOAc (40 mL×3). The combined organic phase was washed with brine (50 mL), dried over anhydrous Na₂SO₄ and filtered. The filtrate was concentrated to obtain the desired compound (2.38 g, yield 92%) as a yellow oil. ¹HNMR (400 MHz, CDCl₃): δ 8.13 (d, *J=* 8.4 Hz, 2H), 8.09 (d, *J=* 8.0 Hz, 1H), 6.83 (t, *J=* 8.4 Hz, 1H), 4.03-3.86 (m, 2H), 3.29-3.20 (m, 1H), 2.90-2.82 (m, 2H),1.94-1.90 (m, 2H), 1.43 (s, 9H),1.41-1.34 (m, 2H).

### Step 2. Synthesis of tert-butyl 4-((2-amino-6-nitrophenyl)amino)piperidin-1-carboxylate (GEN1-091-2)

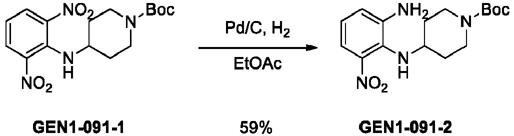

Pd/C (10%, 145 mg) was added to a solution of tert-butyl 4-(2,6-dinitroanilino)piperidin-1-carboxylate (1.0 g, 2.7 mmol) in EtOAc (100 mL). The resulting mixture was stirred at room temperature under H₂ atmosphere for 2 hours. The obtained mixture was filtered and the filtrate was purified by a silica gel column (PE:EA = 3:1) to obtain the desired compound (601 mg, yield 59%) as a red oil. ¹HNMR (400 MHz, DMSO-*d*₆): δ 7.15 (dd, *J* = 8.0, 1.2 Hz, 1H), 6.96 (d, *J =* 8.0 Hz, 1H), 6.87 (t, *J=* 8.0 Hz, 1H), 5.29 (d, *J=* 10.4 Hz, 1H), 5.24 (s, 2H), 3.85 (d, *J=* 11.6 Hz, 2H), 3.29-3.26 (m, 1H), 2.70-2.67 (m, 2H), 1.73 (d, *J=* 11.2 Hz, 1H), 1.38 (s, 9H), 1.19-1.16 (m, 2H).

### Step 3. Synthesis of tert-butyl 4-(7-nitro-2-oxo-2,3-dihydro-1H-benzo[d]imidazol-1-yl)piperidin-1-carboxylate (GEN1-091-3)

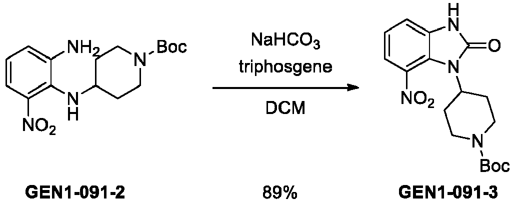

A solution of triphosgene (216 mg, 0.728 mmol) in DCM (5 mL) was added dropwise to a solution of tert-butyl 4-(2-amino-6-nitro-anilino)piperidin-1-carboxylate (489 mg, 1.45 mmol) and NaHCO₃ (1.22g, 14.5mmol) in DCM (20mL) at 0°C. After the addition, the mixture was stirred at 0°C for 1.5 hours. The resulting mixture was poured into water (100 mL), and extracted with DCM (50 mL×3). The combined organics were washed with brine (30 mL), dried over sodium sulfate and it was concentrated to obtain the desired compound (537 mg, yield 89%) as a yellow solid. MS (ESI): Rt = 1.60 min, *m*/*z* 307.2 [M+H-56]⁺; Purity: 87% @ 254 nm, 89% @ 214 nm.

### Step 4. Synthesis of 7-nitro-1-(piperidin-4-yl)-1H-benzo[d]imidazol-2(3H)-one (GEN1-091-4)

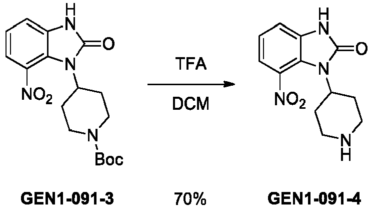

TFA (4 mL) was added to a solution of tert-butyl 4-(7-nitro-2-oxo-3H-benzimidazol-1-yl)piperidin-1-carboxylate (537 mg, 1.48 mmol) in DCM (20 mL). The mixture was stirred at room temperature for 1 hour. The resulting mixture was concentrated, and NaHCO₃ (saturated aqueous solution, 20 mL) was added to adjust the pH to 8. EtOAc (100 mL x 3) was added to extract the desired compound. The combined organic layer was dried with sodium sulfate and concentrated to obtain the desired compound (272 mg, yield 70%) as a brown solid. MS (ESI): Rt = 1.14 min, *m*/*z* 263.1 [M+H]⁺; Purity: 100% @ 254 nm, 100% @ 214 nm.

### Step 5. Synthesis of 7-nitro-1-(1-(2-(4-(trifluoromethyl)phenyl)acetyl)piperidin-4-yl)-1H-benzo[d]imidazol-2(3H)-one (GEN1-091)

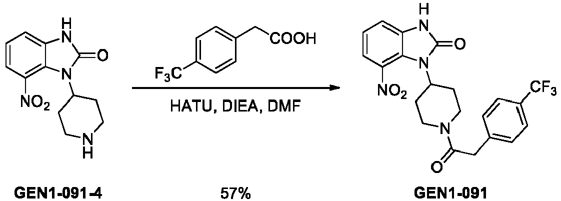

HATU (217 mg, 0.571 mmol) was added to a solution of 4-nitro-3-(4-piperidinyl)-1H-benzimidazol-2-one (100 mg, 0.381 mmol), 2-(4-(trifluoromethyl)phenyl)acetic acid (93 mg, 0.46 mmol) and DIEA (148 mg, 1.15 mmol) in DMF (2 mL). The mixture was stirred at room temperature for 2 hours. The resulting mixture was filtered and the filtrate was purified by preparation. HPLC (waters-3 Xbridge C18 5um 19 * 150mm, 30%-80% B; A: H₂O (0.1% TFA), B: CH₃CN; UV: 254 nm, flow rate: 15mL / min). The desired compound (100 mg, yield 57%) was obtained as a pale yellow solid. ¹H NMR (400 MHz, CDCl₃) δ 9.79 (s, 1H), 7.61-7.58 (m, 3H), 7.40 (d, *J=* 8.0 Hz, 2H), 7.30 (d, *J=* 7.6 Hz, 1H), 7.15 (t, *J=* 8.0 Hz, 1H), 4.87 (d, *J* = 12.8 Hz, 1H), 4.08-4.00 (m, 2H), 3.85 (s, 2H), 3.11-3.05 (m, 1H), 2.77-2.67 (m, 1H), 2.64-2.55 (m, 2H), 1.93 (t, *J=* 8.0 Hz, 1H). MS (ESI): Rt = 3.816 min, *m*/*z* 449.2 [M+H]⁺; Purity: 98.41% @ 254 nm, 99.80% @ 214 nm.

### Example 84: 2-oxo-3-(1-(2-(4-(trifluoromethyl)phenyl)acetyl)piperidin-4-yl)-2,3-dihydro-1H-benzo[d]imidazol-4-sulfonamide

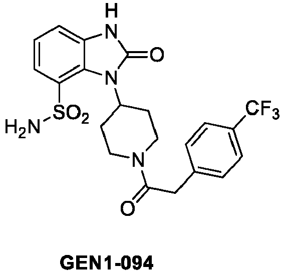

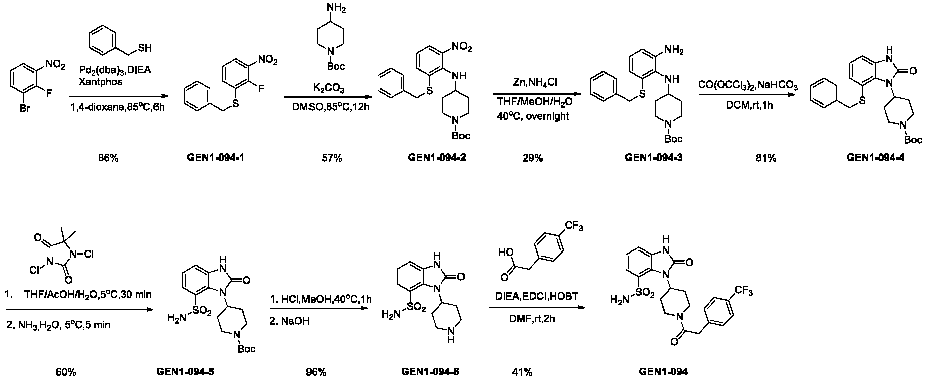

### Step 1. Synthesis of benzyl (2-fluoro-3-nitrophenyl) sulfoxide (GEN1-094-1)

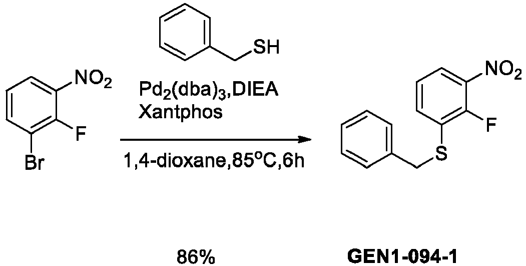

Xantphos(789 mg, 1.36 mmol) was added to a solution of 1-bromo-2-fluoro-3-nitrobenzene (3.0 g, 14 mmol), thiobenzyl alcohol (2.03 g, 16.4 mmol), Pd2(dba)3 (1.41 g, 1.36 mmol) and DIEA (3.52 g, 27.3 mmol) in dioxane (50 mL). The mixture was stirred at 85°C under N₂ for 6 hours. The mixture was cooled to room temperature, and then poured into water (50 mL), and extracted with EtOAc (30 mL×3). The combined organic layer was washed with brine (100 mL), dried over sodium sulfate and it was concentrated to obtain a crude product. The crude product was purified by silica gel column chromatography (petroleum: ethyl acetate = 10:1) and C18 column chromatography (60-90% CH₃CN in water) to obtain the desired product (3.1 g, yield 86%) as a yellow solid. MS (ESI): Rt = 1.75 min, *m*/*z* 262.1 [M-H]⁻; Purity: 100% @ 254 nm, 100% @ 214 nm.

### Step 2. Synthesis of tert-butyl 4-((2-(benzylthio)-6-nitrophenyl)amino)piperidin-1-carboxylate (GEN1-094-2)

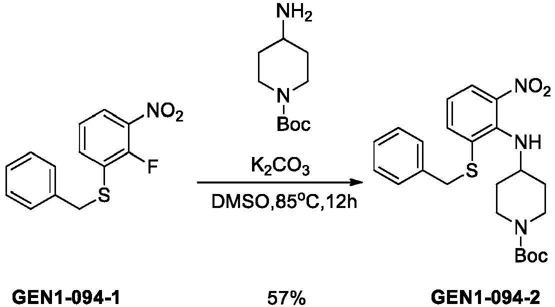

K₂CO₃ (3.25 g, 23.6 mmol) was added to a solution of 1-benzylsulfanyl-2-fluoro-3-nitrobenzene (3.1 g, 12 mmol), tert-butyl 4-aminopiperidine-1-carboxylate (2.83 g, 14.1 mmol) in DMSO (40 mL). The mixture was stirred at 85°C under N₂ for 12 hours. The mixture was cooled to room temperature, and then poured into water (100 mL), and extracted with EtOAc (50 mL) three times. The combined organic layer was washed with brine, dried over sodium sulfate and it was concentrated. The crude product was purified by silica gel column chromatography (petroleum ether: ethyl acetate = 10:1) and C18 column chromatography (60-90% CH₃CN in water) to obtain the desired compound (3.0 g, yield 57%) as a red oil. MS (ESI): Rt = 1.97 min, *m*/*z* 388.3 [M+H-56]⁺; Purity: 100% @ 254 nm, Purity: 100% @ 214 nm.

### Step 3. Synthesis of tert-butyl 4-((2-amino-6-(benzylthio)phenyl)amino)piperidin-1-carboxylate (GEN1-094-3)

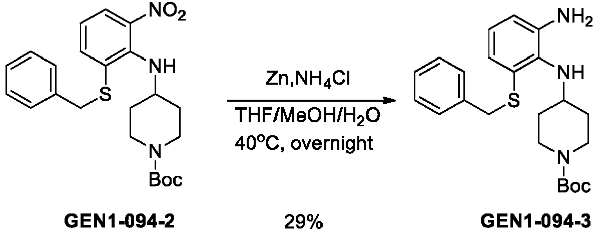

NH₄Cl (2.17 g, 40.6 mmol) was added to a mixture of tert-butyl 4-(2-benzylsulfanyl-6-nitro-anilino)piperidine-l-carboxylate (3.0 g, 6.8 mmol) in THF (20 mL), MeOH (20 mL) and water (20 mL), and zinc powder (2.21 g, 33.8 mmol) was added in batches. The mixture was stirred at 40°C overnight. The mixture was filtered and the filtrate was concentrated. The residue was diluted with water and extracted with EA (50 mL×3). The combined organic phase was dried over anhydrous sodium sulfate and concentrated. The residue was purified by column chromatography on silica gel (petroleum ether: ethyl acetate = 5:1) to obtain the desired compound (800 mg, yield 29%) as a yellow oil. MS (ESI): Rt = 1.878 min, *m*/*z* 414.2 [M+H]⁺; Purity: 98% @ 214 nm.

### Step 4. Synthesis of tert-butyl 4-(7-(benzylthio)-2-oxo-2,3-dihydro-1H-benzo[d]imidazol-1-yl)piperidin-1-carboxylate (GEN1-094-4)

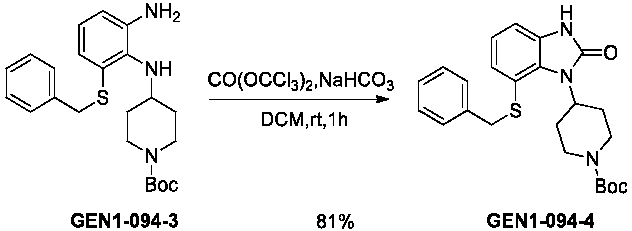

Triphosgene (124 mg, 0.420 mmol) was added to a mixture of tert-butyl 4-(2-amino-6-benzylsulfanyl-anilino)piperidin-1-carboxylate (480 mg, 1.04 mmol) and NaHCO₃ (877 mg, 10.5 mmol) in DCM (30 mL). The mixture was stirred at room temperature for 1 hour, and then poured into water (20 mL), and extracted with DCM (20 mL×3). The combined organic layer was washed with brine (50 mL), dried over sodium sulfate and it was concentrated. The residue was purified by silica gel column chromatography (petroleum ether: ethyl acetate = 1:1) to obtain the desired compound (390 mg, yield 81%) as a yellow solid. ¹H NMR (400 MHz, CDCl₃): δ 9.21 (s, 1H), 7.22-7.21 (m, 3H), 7.19-7.17 (m, 1H), 7.04-6.98 (m, 4H), 5.51-5.46 (m, 1H), 4.20-4.09 (m, 2H), 4.04 (s, 2H), 2.71-2.65 (m, 2H), 2.58-2.48 (m, 2H), 1.48 (s, 9H), 1.45-1.41 (m, 2H).

### Step 5. Synthesis of tert-butyl 4-(2-oxo-7-sulfamoyl-2,3-dihydro-1H-benzo[d]imidazol-1-yl)piperidin-1-carboxylate (GEN1-094-5)

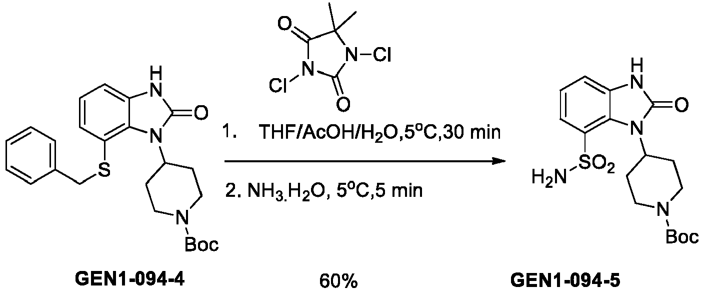

1,3-dichloro-5,5-dimethylimidazolidine-2,4-dione (187 mg, 0.95 mmol) was added in batches to a solution of compound tert-butyl 4-(7-benzylsulfanyl-2-oxo-3H-benzimidazol-1-yl)piperidin-1-carboxylate (200 mg, 0.43 mmol) in THF (4 mL) and water (0.4 mL) at 5°C. After the mixture was stirred at 5°C for 30 minutes, NH₃·H₂O (25% aqueous solution, 3 mL) was added. The mixture was stirred for 5 minutes. The reaction mixture was diluted with water (20 mL), and extracted with EtOAc (20 mL×2). The combined organic phase was washed with brine, dried over sodium sulfate and it was concentrated. The residue was triturated with petroleum ether/ethyl acetate (5/1, 2 mL) and filtered. The solid was collected to obtain the desired compound (120 mg, yield 60%) as a yellow solid. MS (ESI): Rt = 1.35 min, *m*/*z* 397.4 [M+H]⁺; Purity: 86% @ 214 nm, 100% @ 254 nm.

### Step 6. Synthesis of 2-oxo-3-(1-(2-(4-(trifluoromethyl)phenyl)acetyl)piperidin-4-yl)-2,3-dihydro-1H-benzo[d]imidazol-4-sulfonamide (GEN1-094-6)

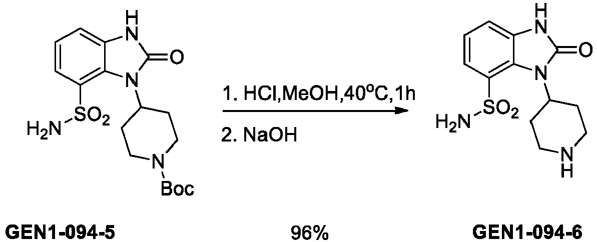

HCl (1 ml) was added to a solution of tert-butyl 4-(2-oxo-7-sulfamoyl-3H-benzimidazol-1-yl)piperidin-1-carboxylate (120 mg, 0.260 mmol) in MeOH (2mL). The mixture was stirred at 40°C for 1 hour, and then concentrated. The residue was dissolved in water (1 mL). Aqueous NaOH (10%) was added dropwise to adjust pH=8. Then EtOAc (2 mL×2) was added to extract the desired compound. The combined organic solution was concentrated to obtain the desired compound (180 mg, yield 96%) as a yellow solid. MS (ESI): Rt = 0.49 min, *m*/*z* 297.2 [M+H]⁺; Purity: 70% @ 254 nm, 80% @ 214 nm.

### Step 7. Synthesis of 2-oxo-3-(1-(2-(4-(trifluoromethyl)phenyl)acetyl)piperidin-4-yl)-2,3-dihydro-1H-benzo[d]imidazol-4-sulfonamide (GEN1-094)

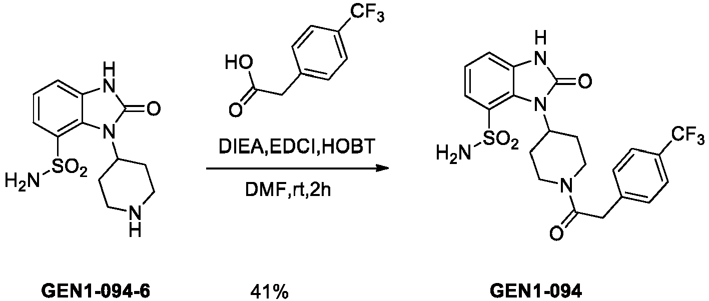

HOBT (50 mg, 0.37 mmol) and EDCI (72 mg, 0.37 mmol) were added to a solution of 2-oxo-3-(4-piperidinyl)-1H-benzimidazol-4-sulfonamide (180 mg, 0.245 mmol), 2-(4-(trifluoromethyl)phenyl)acetic acid (51 mg, 0.25 mmol), DIEA (97 mg, 0.75 mmol) in DMF (3mL). The mixture was stirred at room temperature for 2 hours, and then poured into water (10 mL), and extracted with DCM (10 mL×3). The combined organic phase was concentrated. The residue was purified by C18 column chromatography [40∼80% CH₃CN in water (0.1% TFA)]. The desired compound (50 mg, yield 41%) was obtained as a white solid. ¹H NMR (400 MHz, DMSO-*d*₆): δ 11.34 (s, 1H), 7.84 (s, 2H), 7.68 (d, *J=* 8.0 Hz, 2H), 7.52-7.47 (m, 3H), 7.19 (d, *J* = 7.2 Hz, 1H), 7.11 (t, *J =* 8.0 Hz, 1H), 5.31-5.25 (m, 1H), 4.58-4.55 (m, 1H), 4.15-4.12 (m, 1H), 3.96-3.84 (m, 2H), 3.56-3.37 (m, 1H), 3.01 (t, *J* = 12.8 Hz, 1H), 2.58-2.55 (m, 1H), 2.43-2.40 (m, 1H), 1.74-1.72 (m, 2H). ¹⁹F NMR (376 MHz, DMSO-*d*₆): δ -60.79 (s, 3F). MS (ESI): Rt = 3.316 min, *m*/*z* 483.1 [M+H]⁺; Purity: 81.97% @ 254 nm, 98.80% @ 214 nm.

### Example 85: Synthesis of 2-oxo-3-(1-(2-(4-(trifluoromethyl)phenyl)acetyl)piperidin-4-yl)-2,3-dihydro-1H-benzo[d]imidazol-4-carboxamide (GEN1-095)

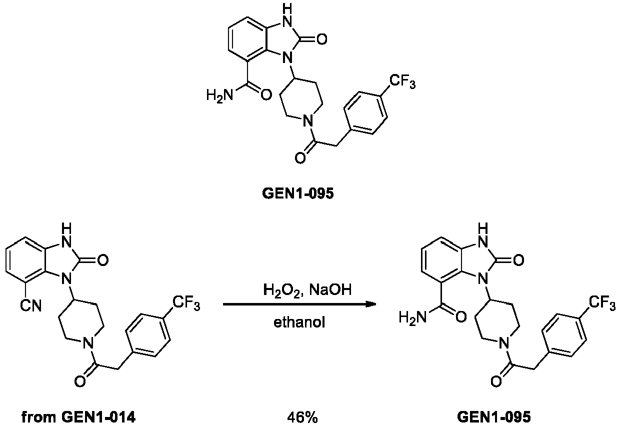

### Step 1. Synthesis of 2-oxo-3-(1-(2-(4-(trifluoromethyl)phenyl)acetyl)piperidin-4-yl)-2,3-dihydro-1H-benzo[d]imidazol-4-carboxamide (GEN1-095)

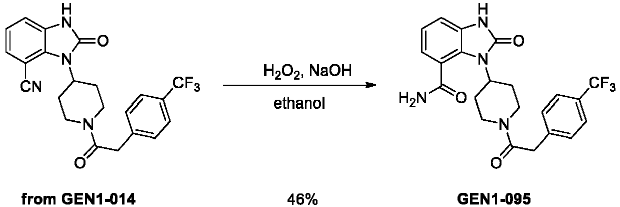

A mixture of 2-oxo-3-[1-[2-[4-(trifluoromethyl)phenyl]acetyl]-4-piperidinyl]-1H-benzimidazol-4-carbonitrile (150 mg, 0.350 mmol, the synthesis steps could be found in GEN1-014) was stirred in ethanol (2 mL), NaOH (21 mg, 0.53 mmol) and hydrogen peroxide (30%, 2 mL) at 55°C for 12 hours. The mixture was cooled to room temperature, and then poured into water (20 mL), and extracted with DCM (10 mL) three times. The combined organic layer was washed with brine, dried over sodium sulfate and it was concentrated to obtain a crude product. The crude product was purified by a C18 column (CH₃CN: water = 5% to 50%) to obtain the desired compound (75 mg, yield 46%) as a white solid. ¹H NMR (400 MHz, DMSO-*d*₆): δ 11.03 (s, 1H), 8.10 (s, 1H), 7.70-7.67 (m, 3H), 7.47 (d, *J=* 8.0 Hz, 2H), 7.01-6.97 (m, 3H), 4.58 (d, *J* = 10.4 Hz, 1H), 4.38-4.32 (m, 1H), 4.17 (d, *J =* 13.2 Hz, 1H), 3.95-3.84 (m, 2H), 2.90 (t, *J =* 13.2 Hz, 1H), 2.43-2.33 (m, 3H), 1.79-1.74 (m, 2H). MS (ESI): Rt = 2.663 min, *m*/*z* 447.1, [M+H]⁺; Purity: 96.31% @ 254 nm, 98.86% @ 214 nm.

### Example 86: 1-(1-(2-(benzo[d][1,3]dioxol-5-yl)acetyl)piperidin-4-yl)-1H-benzo[d]imidazol-2(3H)-one

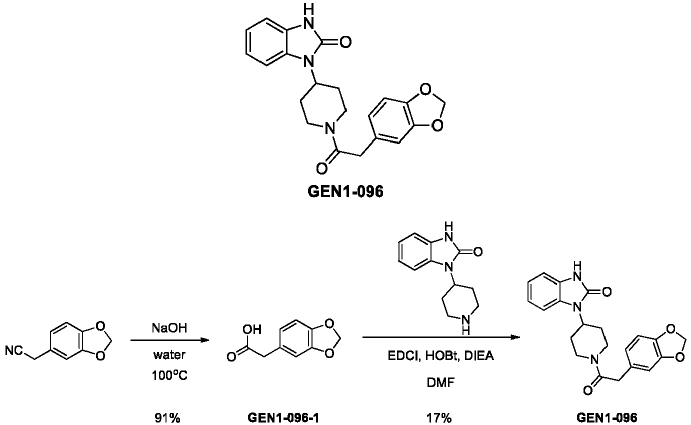

### Step 1. Synthesis of 2-(benzo[d] [1,3]dioxa-5-yl)acetic acid (GEN1-096-1)

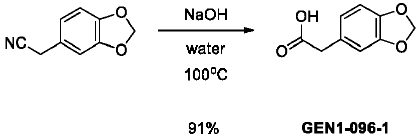

A suspension of 2-(1,3-benzodiaxazol-5-yl)acetonitrile (300 mg, 1.86 mmol) and NaOH (224 mg, 5.60 mmol) in water (15 mL) was stirred at 100°C for 2 hours. The reaction mixture was acidified to pH=2 with 1M HCl, and then extracted with EtOAc (15 mL×3). The combined organic layer was washed with brine (15 mL), dried with MgSO₄, filtered and it was concentrated to obtain the desired compound (320 mg, yield 91%) as a white solid. ¹HNMR (300 MHz, CDCl₃): δ 6.78-6.70 (m, 3H), 5.95 (s, 2H), 3.56 (s, 2H).

### Step 2. Synthesis of 1-(1-(2-(benzo[d][1,3]dioxol-5-yl)acetyl)piperidin-4-yl)-1H-benzo[d]imidazol-2(3H)-one (GEN1-096)

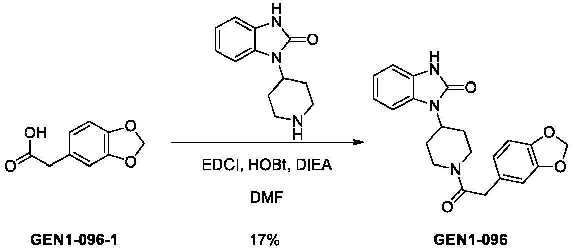

In an ice bath, EDCI (80 mg, 0.42 mmol) was added to a suspension of 2-(1,3-benzodioxazol-5-yl)acetic acid (50 mg, 0.28 mmol), 3-(4-piperidinyl)-1H-benzimidazol-2-one (57 mg, 0.26 mmol), HOBT (56 mg, 0.41 mmol) and DIEA (108 mg, 0.836 mmol) in DMF (2 mL). The resulting mixture was stirred at room temperature overnight. The reaction mixture was purified by a C18 column (CH₃CN: water = 5% to 50%) to obtain the desired compound (17.9 mg, yield 17%) as a white solid. ¹HNMR (400 MHz, CDCl₃): δ 8.83 (s, 1H), 7.09-7.00 (m, 3H), 6.90-6.86 (m, 2H), 6.80-6.75 (m, 2H), 5.95 (s, 2H), 4.90-4.86 (m, 1H), 4.59-4.48 (m, 1H), 4.07-4.03 (m, 1H), 3.74 (s, 2H), 3.15 (t, *J=* 12.8 Hz, 1H), 2.69 (t, *J=* 14.0 Hz, 1H), 2.31-2.19 (m, 1H), 1.98-1.84 (m, 2H), 1.75-1.71 (m, 1H). MS (ESI): Rt = 3.140 min, *m*/*z* 380.1 [M+H]⁺; Purity: 92.84% @ 254 nm, 99.07% @ 214 nm.

### Example 87: 1-(1-(2-(4-(dimethylamino)phenyl)acetyl)piperidin-4-yl)-1H-benzo[d]imidazol-2(3H)-one

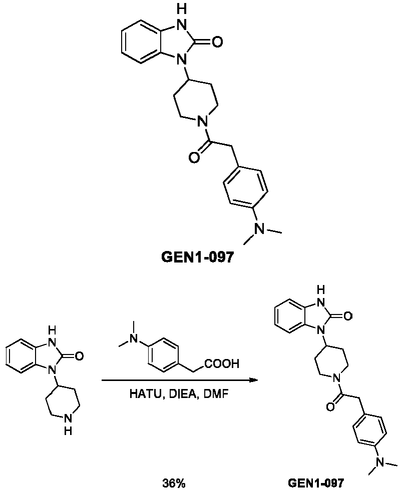

### Step 1. Synthesis of 1-(1-(2-(4-(dimethylamino)phenyl)acetyl)piperidin-4-yl)-1H-benzo[d]imidazol-2(3H)-one (GEN1-097)

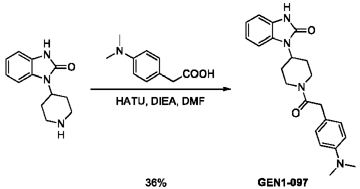

HATU (263 mg, 0.692 mmol) was added to a solution of 3-(4-piperidinyl)-1H-benzimidazol-2-one (100 mg, 0.460 mmol), 2-(4-(dimethylamino)phenyl)acetic acid (99 mg, 0.55 mmol) and DIEA (179mg, 1.38mmol) in DMF (1.5mL). The mixture was stirred at room temperature for 16 hours. The resulting mixture was purified by preparation. HPLC (Waters-Large-prep-2 Xbridge C18 5um 19 * 150mm, 2%-40%B; A: H₂O(0.1%TFA), B: CH₃CN; UV: 214 nm, flow rate: 15mL / min). Then saturated Na₂CO₃ was added to basify the solution to pH=8. The mixture was extracted with EtOAc (20 mL×3), and the combined organic layer was concentrated to obtain the desired compound (64 mg, yield 36%) as a white solid. ¹HNMR (400 MHz, DMSO-*d*₆): δ 10.82 (s, 1H), 7.12 (d, *J=* 8.8 Hz, 2H), 6.95-6.93 (m, 4H), 6.72 (d, *J=* 8.4 Hz, 2H), 4.57 (d, *J=* 12.4 Hz, 1H), 4.42-4.36 (m, 1H), 4.07 (d, *J=* 13.2 Hz, 1H), 3.70-3.58 (m, 2H), 3.14-3.08 (m, 1H), 2.85 (s, 6H), 2.68-2.62 (m, 1H), 2.05-1.96 (m, 1H), 1.91-1.83 (m, 1H), 1.66 (d, *J =* 11.6 Hz, 1H), 1.55 (d, *J=* 10.8 Hz, 1H). MS (ESI): Rt = 3.286 min, *m*/*z* 379.2 [M+H]⁺; Purity: 99.37% @ 254 nm, 99.34% @ 214 nm.

### Example 88: 1-(1-(2-(adamantan-1-yl)acetyl)piperidin-4-yl)-1H-benzo[d]imidazol-2(3H)-one

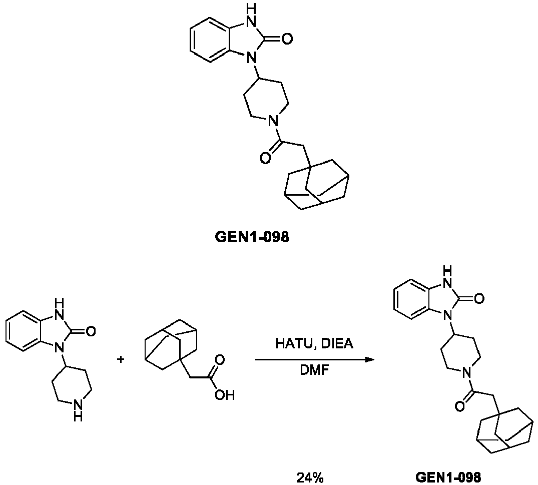

### Step 1. Synthesis of 1-(1-(2-(adamantan-1-yl)acetyl)piperidin-4-yl)-1H-benzo[d]imidazol-2(3H)-one (GEN1-098)

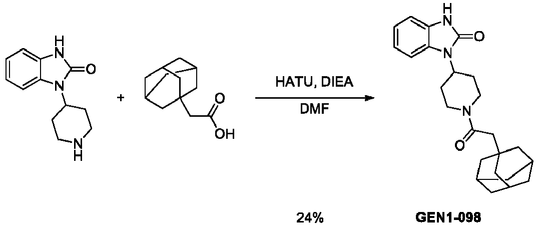

DIEA (200mg, 1.55mmol) was added to a solution of 2-(1-adamantyl)acetic acid (100 mg, 0.515 mmol), 3-(4-piperidinyl)-1H-benzimidazol-2-one (112 mg, 0.516 mmol) and HATU (255 mg, 0.671 mmol) in DMF (3mL). The mixture was stirred at 25°C for 6 hours, and then purified by a C18 column (CH₃CN: water=5% to 50%) to obtain the desired compound (50 mg, yield 24%) as a white solid. ¹HNMR (400 MHz, CDCl₃): δ 8.84 (s, 1H), 7.12-7.03 (m, 4H), 4.96 (d, *J =* 16.0 Hz, 1H), 4.60-4.52 (m, 1H), 4.17 (d, *J =* 15.2 Hz, 1H), 3.20 (t, *J =* 12.8 Hz, 1H), 2.66 (t, *J* = 12.8 Hz, 1H), 2.40-2.18 (m, 4H), 2.00 (s, 3H), 1.92-1.89 (m, 2H), 1.75-1.66 (m, 12H). MS (ESI): Rt = 4.059 min, *m*/*z* 394.2, [M+H]⁺; Purity: 81.04% @ 254 nm, 98.96% @ 214 nm.

### Example 89: 5-nitro-4-(1-(2-(4-(trifluoromethyl)phenyl)acetyl)piperidin-4-yl)-3,4-dihydroquinoxaline-2(1H)-one

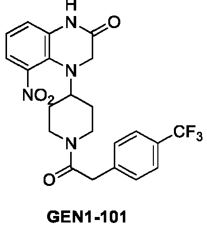

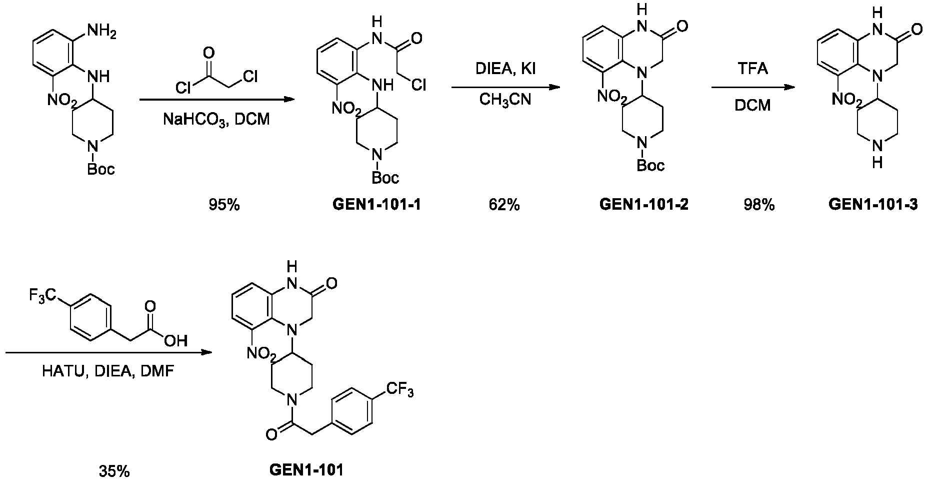

### Step 1. Synthesis of tert-butyl 4-((2-(2-chloroacetamido)-6-nitrophenyl)amino)piperidin-1-carboxylate (GEN1-101-1)

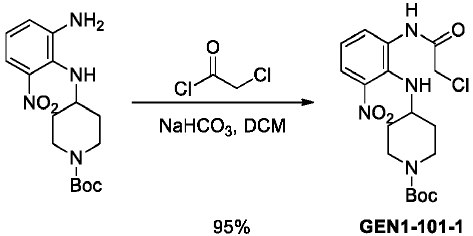

2-chloroacetyl chloride (200 mg, 1.77 mmol) was added dropwise to a suspension of tert-butyl 4-(2-amino-6-nitro-anilino)piperidin-1-carboxylate (596 mg, 1.77 mmol, the synthesis steps could be found in GEN1-091) and NaHCO₃ (744 mg, 8.86 mmol) in DCM (20 mL). The resulting mixture was stirred at 0°C for 1 hour, and then concentrated and poured into water (20 mL). The mixture was extracted with DCM (50 mL×3). The combined organic solution was washed with brine (50 mL), dried over anhydrous Na₂SO₄ and it was concentrated. The residue was purified by a silica gel column (PE:EA = 5:1) to obtain the desired compound (694 mg, yield 95%) as a red oil. ¹HNMR (300 MHz, CDCl₃): δ 9.24 (s, 1H), 8.62 (d, *J=* 8.1 Hz, 1H), 7.88 (dd, *J* = 8.4, 1.2 Hz, 1H), 7.25 (t, *J=* 8.1 Hz, 1H), 5.78-5.54 (m, 1H), 4.34 (s, 2H), 4.17-4.11 (m, 2H), 3.10-3.07 (m, 1H), 2.77-2.69 (m, 2H), 1.88-1.72 (m, 2H), 1.51 (s, 9H), 1.44-1.32 (m, 2H). MS (ESI): Rt = 1.64 min, *m*/*z* 411.4 [M-H]⁻; Purity: 100% @ 254 nm, 100% @ 214 nm.

### Step 2. Synthesis of tert-butyl 4-(8-nitro-3-oxo-3,4-dihydroquinoxaline-1(2H)-yl)piperidin-1-carboxylate (GEN1-101-2)

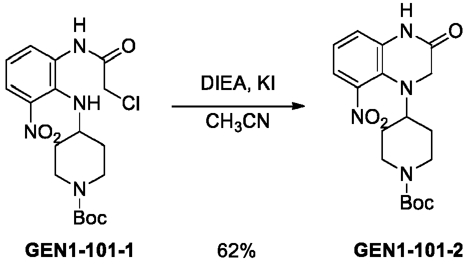

A solution of tert-butyl 4-[2-[((2-chloroacetyl)amino]-6-nitro-anilino]piperidin-1-carboxylate (594 mg, 1.44 mmol), DIEA (558 mg, 4.32 mmol) and KI (under N₂ atmosphere at 80°C, 72 mg, 0.43 mmol) in acetonitrile (20 mL) was stirred for 22 hours. The obtained mixture was concentrated and purified by C18 column (50-70% B; A: H₂O, B: CH₃CN) to obtain the desired compound (336 mg, yield 62%) as a yellow solid. MS (ESI): Rt = 1.53 min, *m*/*z* 375.4 [M-H]⁻; Purity: 100% @ 254 nm, 100% @ 214 nm.

### Step 3. Synthesis of 5-nitro-4-(piperidin-4-yl)-3,4-dihydroquinoxaline-2(1H)-one (GEN1-101-3)

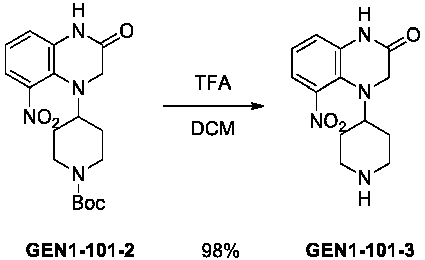

A solution of tert-butyl 4-(8-nitro-3-oxo-3,4-dihydroquinoxaline-1-yl)piperidin-1-carboxylate (356 mg, 0.946 mmol) and TFA (1.54 g, 13.5 mmol) in DCM (15 mL) was stirred at room temperature for 3 hours. The resulting mixture was concentrated and diluted with water (15 mL). Then, saturated Na₂CO₃ aqueous solution was added to basify the solution to pH=8. The mixture was extracted with DCM (50 mL×4). The combined organic layer was dried over anhydrous sodium sulfate and concentrated to obtain the desired compound (256 mg, yield 98%) as a yellow solid. MS (ESI): Rt = 1.11 min, *m*/*z* 277.2 [M+H]⁺; Purity: 100% @ 254 nm, 100% @ 214 nm.

### Step 4. 5-nitro-4-(1-(2-(4-(trifluoromethyl)phenyl)acetyl)piperidin-4-yl)-3,4-dihydroquinoxaline-2(1H)-one (GEN1-101)

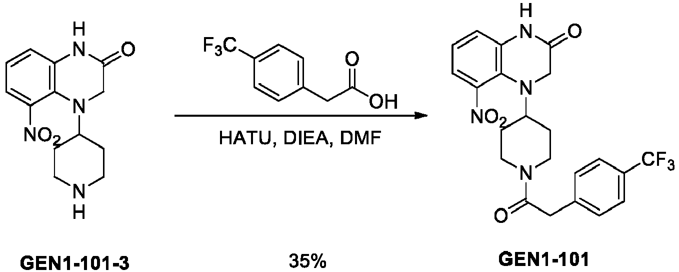

HATU (248 mg, 0.652 mmol) was added to a solution of 5-nitro-4-(4-piperidinyl)-1,3-dihydroquinoxaline-2-one (120 mg, 0.434 mmol), 2-(4-(trifluoromethyl)phenyl)acetic acid (106 mg, 0.519 mmol) and DIEA (168 mg, 1.30 mmol) in DMF (3mL). The mixture was stirred at room temperature for 2 hours. The resulting mixture was purified by preparation. HPLC (prep-2 Xbridge C18 5um 19 * 150 mm, 33%-50%B; A: H₂O(0.1% NH₄HCO₃), B: CH₃CN; UV: 214 nm, flow rate: 15mL / min). The desired compound (71 mg, yield 35%) was obtained as a pale yellow solid. ¹HNMR (400 MHz, DMSO-*d*₆): δ 10.77 (s, 1H), 7.66 (d, *J=* 8.0 Hz, 2H), 7.59 (dd, *J* = 6.8, 2.4 Hz, 1H), 7.44 (d, *J=* 8.0 Hz, 2H), 7.21-7.15 (m, 2H), 4.43 (d, *J* = 12.8 Hz, 1H), 4.00 (d, *J=* 14.0 Hz, 1H), 3.88-3.78 (m, 2H), 3.62 (s, 2H), 3.21-3.14 (m, 1H), 2.92 (t, *J=* 12.4 Hz, 1H), 2.47-2.41 (m, 1H), 1.65-1.62 (m, 2H), 1.56-1.40 (m, 2H). MS (ESI): Rt = 3.948 min, *m*/*z* 463.1 [M+H]⁺; Purity: 99.39% @ 254 nm, 98.84% @ 214 nm.

### Example 90: 5-(1-(2-(4-(trifluoromethyl)phenyl)acetyl)piperidin-4-yl)-4,5-dihydro-1H-benzo[b][1,4]diazepine-2(3H)-one

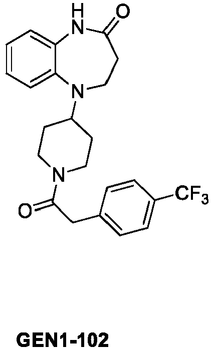

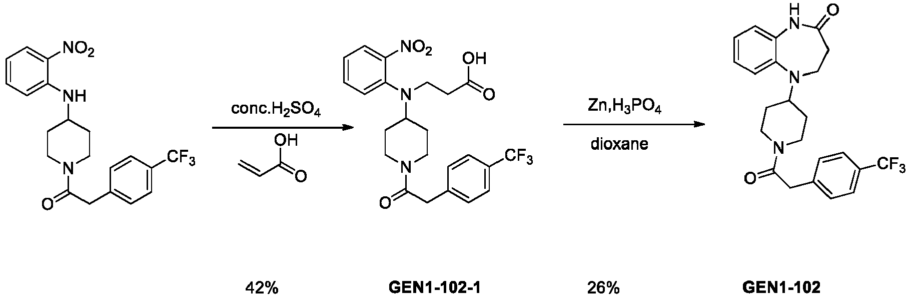

### Step 1. Synthesis of 3-((2-nitrophenyl)(1-(2-(4-(trifluoromethyl)phenyl)acetyl)piperidin-4-yl)amino)propionic acid (GEN1-102-1)

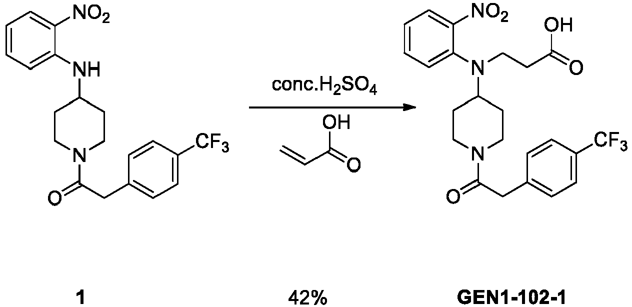

Concentrated acrylic acid (4 mL) and sulfuric acid (2.0 g) were added to a mixture of 1-(4-((2-nitrophenyl)amino)piperidin-1-yl)-2-(4-(trifluoromethyl)phenyl)ethanone (2.00 g, 3.98 mmol, the synthesis steps could be found in GEN1-055). The mixture was stirred at 100°C for 3 hours, and then cooled to room temperature and poured into ice water (50 mL). The mixture was extracted with EtOAc (20 mL×3). The combined organic phase was washed with brine, dried over sodium sulfate and it was concentrated to obtain a residue. The residue was purified by C18 column chromatography (40-80% ACN in water) to obtain the desired compound (800 mg, yield 42%) as a yellow oil. LCMS (ESI) Rt = 1.37 min, *m*/*z* 480.5 [M+H]⁺; Purity: 100% @ 254 nm, 100% @ 214 nm.

### Step 2. Synthesis of 5-(1-(2-(4-(trifluoromethyl)phenyl)acetyl)piperidin-4-yl)-4,5-dihydro-1H-benzo[b][1,4]diazepine-2(3H)-one (GEN1-102)

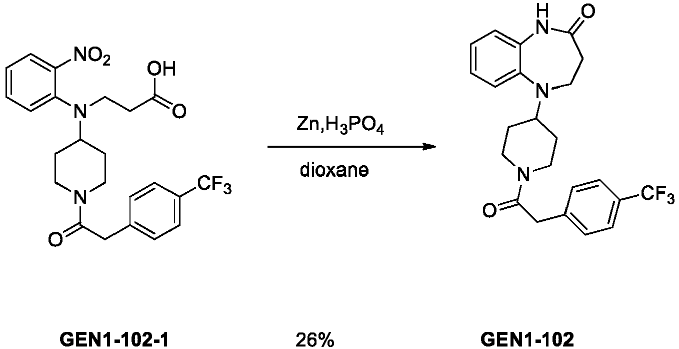

Zinc powder (1.09 g, 16.7 mmol) and H₃PO4 (1 g, 85% purity) were added to a compound 3-((2-nitrophenyl)(1-(2-(4-(trifluoromethyl)phenyl)acetyl)piperidin-4-yl)amino)propionic acid (800 mg, 1.67 mmol) in dioxane (5mL). The mixture was stirred at 70°C for 30 minutes and then at 100°C for 3 hours. The mixture was cooled to room temperature and filtered. The filter cake was washed with EtOAc (20 mL). The combined filtrates were concentrated to obtain a residue. The residue was purified by C18 (50-80% ACN in water) and purified by preparative HPLC [gilson-2, Xbridge C18, 5um, 19 x 150 mm, 40-80% B, A: H₂O (0.1% TFA) .), B: ACN, F = 15 mL/min, uv = 214 nm], the desired compound (188 mg, yield 26%) was obtained as a white solid. ¹H NMR (400 MHz, DMSO-*d*₆): δ 9.41 (s, 1H), 7.67 (d, *J =* 8.0 Hz, 2H), 7.45 (d, *J =* 8.0 Hz, 2H), 7.15-7.13 (m, 1H), 7.07-7.03 (m, 1H), 6.96-6.89 (m, 2H), 4.27 (d, *J=* 12.0 Hz, 1H), 3.91 (d, *J=* 14.8 Hz, 1H), 3.84 (s, 2H), 3.52 (t*, J=* 10.4 Hz, 1 H), 3.34 (t, *J=* 10.4 Hz, 2H), 3.14 (t, *J* = 13.2 Hz, 1H), 2.78 (t, *J =* 12.8 Hz, 1H), 2.32-2.28 (m, 2H), 1.81-1.73 (m, 2H), 1.56-1.46 (m ,2H). ¹⁹F NMR (376 MHz, DMSO-*d*₆): δ 60.80. LCMS (ESI) Rt = 3.396 min, *m*/*z* 432.1 [M+H]⁺; Purity: 98.7% @ 254 nm, 97.3% @ 214 nm.

### Example 91: (Cis)-1-(1-(2-(4-(trifluoromethyl)cyclohexyl)acetyl)piperidin-4-yl)-1H-benzo[d]imidazol-2(3H)-one (GEN1-107) and (trans)-1-(1-(2-(4-(trifluoromethyl)cyclohexyl)acetyl)piperidin-4-yl)-1H-benzo[d]imidazol-2(3H)-one (GEN1-108)

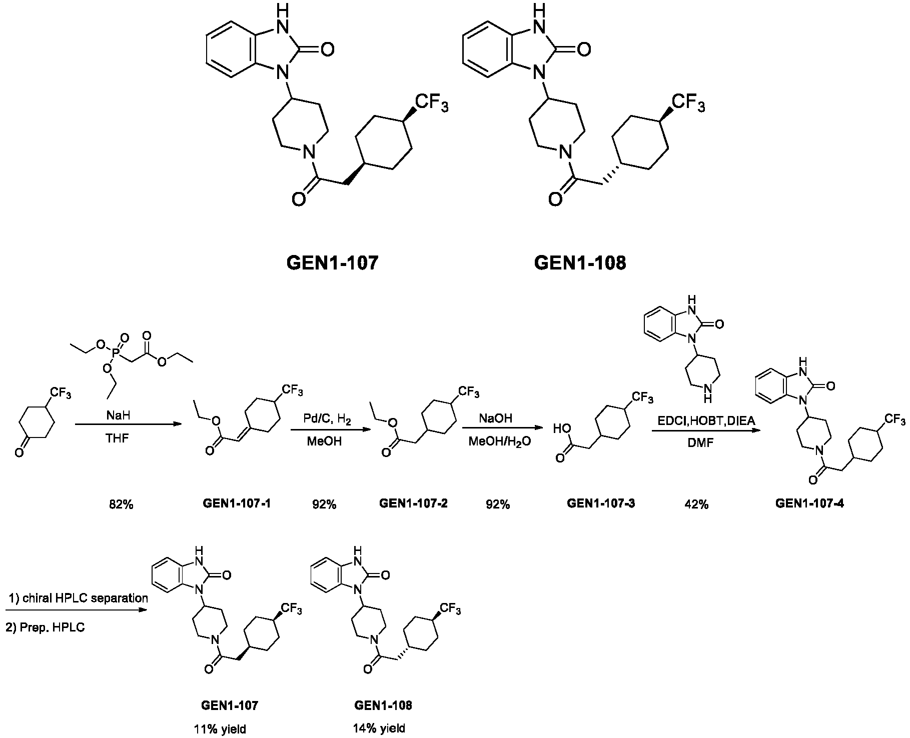

### Step 1. Synthesis of ethyl 2-(4-(trifluoromethyl)cyclohexylidene) acetate (GEN1-107-1)

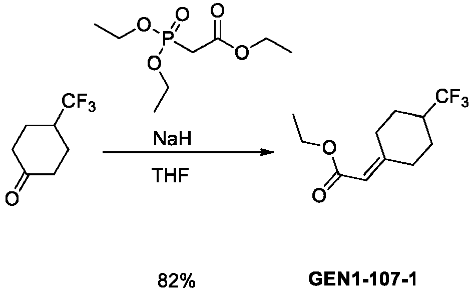

In an ice bath, NaH (60% mineral oil, 433 mg, 10.8 mmol) was added in portions to a solution of ethyl 2-diethoxyphosphoryl acetate (1.94 g, 8.67 mmol) in THF (40 mL). After the mixture was stirred at room temperature for 1 hour, 4-(trifluoromethyl)cyclohexanone (1.2 g, 7.2 mmol) was added dropwise. The resulting mixture was stirred at room temperature overnight. The reaction mixture was diluted with water (50 mL), and extracted with EtOAc (30 mL×2). The combined organic phase was dried over anhydrous Na₂SO₄ and filtered. The filtrate was concentrated, and the residue was purified by column chromatography on silica gel (petroleum: ethyl acetate = 10:1) to obtain the desired compound (1.4 g, yield 82%) as a yellow oil. ¹H NMR (400 MHz, CDCl₃): δ 5.68-5.56 (m, 1H), 4.14 (q, *J* = 7.2 Hz, 2H), 2.97 (s, 2H), 2.29-2.21 (m, 2H), 2.16-2.08 (m, 3H), 2.04-2.00 (m, 1H), 1.60-1.47 (m, 1H), 1.26 (t, *J* = 7.2 Hz, 3H).

### Step 2. Synthesis of ethyl 2-(4-(trifluoromethyl)cyclohexyl) acetate (GEN1-107-2)

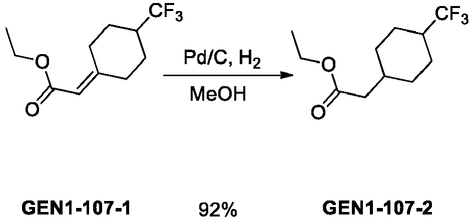

A mixture of ethyl 2-[4-(trifluoromethyl)cyclohexylidene] acetate (1.4 g, 5.9 mmol) and Pd/C (10%, 300 mg) in MeOH (50 mL) was hydrogenated at 40°C under a H₂ atmosphere overnight. The mixture was filtered through diatomaceous earth, and the filtrate was concentrated to obtain the desired compound (1.3 g, yield 92%) as a colorless liquid. ¹H NMR (400 MHz, CDCl3): δ 4.13 (q, J = 7.2 Hz, 2H), 2.35-2.19 (m, 2H), 1.96-1.56 (m, 8H), 1.40-1.30 (m, 1H), 1.26 (t, J = 7.2 Hz, 3H), 1.06-0.96 (m, 1H).

### Step 3. Synthesis of 2-(4-(trifluoromethyl)cyclohexyl)acetic acid (GEN1-107-3)

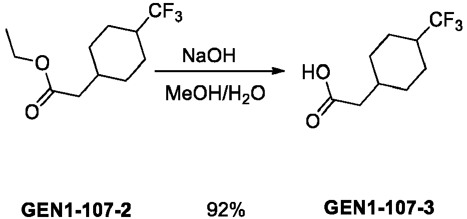

A solution of NaOH (436 mg, 10.9 mmol) in water (10mL) was added to a solution of compound ethyl 2-[4-(trifluoromethyl)cyclohexyl] acetate (1.30g, 5.46mmol) in MeOH (20mL) and stirred at 40°Cfor 2 hours. The mixture was concentrated. The residue was diluted with water (30 mL) and extracted with EtOAc (20 mL). The aqueous layer was acidified with IN HCl to pH=2. The acidified aqueous layer was extracted with EtOAc (30 mL×2). The combined organic phase was washed with brine, dried over anhydrous Na₂SO₄ and filtered. The filtrate was concentrated to obtain the desired compound (1.05 g, yield 92%) as a yellow oil. ¹H NMR (400 MHz, CDCl3): δ 2.41-2.39 (m, 0.5H), 2.27-2.26 (m, 1.5H), 2.23-2.19 (m, 0.5 H), 2.13-2.05 (m, 0.5H), 1.98-1.91 (m, 3.5H), 1.85-1.75 (m, 1H), 1.72-1.69 (m, 0.5H), 1.63-1.58 (m, 1H), 1.41-1.30 (m, 1.5H), 1.09-0.98 (m, 1.5H).

### Step 4. Synthesis of 1-(1-(2-(4-(trifluoromethyl)cyclohexyl)acetyl)piperidin-4-yl)-1H-benzo[d]imidazol-2(3H)-one (GEN1-107-4)

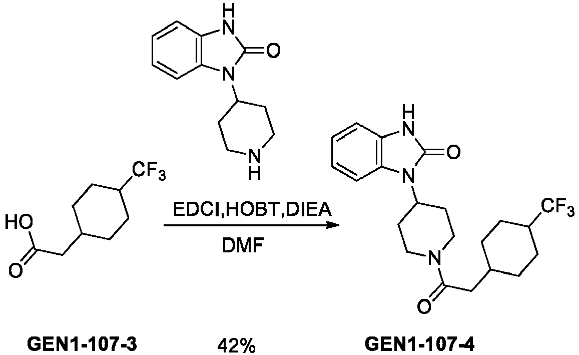

The compound 3-(4-piperidinyl)-1H-benzimidazol-2-one (300 mg, 1.38 mmol), 2-(4-(trifluoromethyl)cyclohexyl)acetic acid (305 mg, 1.45 mmol)) and DIEA (535 mg, 4.14 mmol) were dissolved in DMF (3 mL), and EDCI (397 mg, 2.07 mmol) and HOBT (280 mg, 2.07 mmol) were added. The mixture was stirred at room temperature overnight. The reaction mixture was diluted with water (50 mL), and extracted with EtOAc (20 mL×2). The combined organic phase was concentrated, and the residue was purified by C₁₈ column chromatography (acetonitrile: water = 40∼70%) to obtain the desired compound (240 mg, yield 42%) as a yellow solid. MS (ESI): Rt = 1.52 min, m/z 410.4 [M+H]⁺; Purity: 100% @ 254 nm, 100% @ 214 nm.

### Step 5. Synthesis of (cis)-1-(1-(2-(4-(trifluoromethyl)cyclohexyl)acetyl)piperidin-4-yl)-1H-benzo[d]imidazol-2(3H)-one (GEN1-107) and (trans)-1-(1-(2-(4-(trifluoromethyl)cyclohexyl)acetyl)piperidin-4-yl)-1H-benzo[d]imidazol-2(3H)-one (GEN1-108)

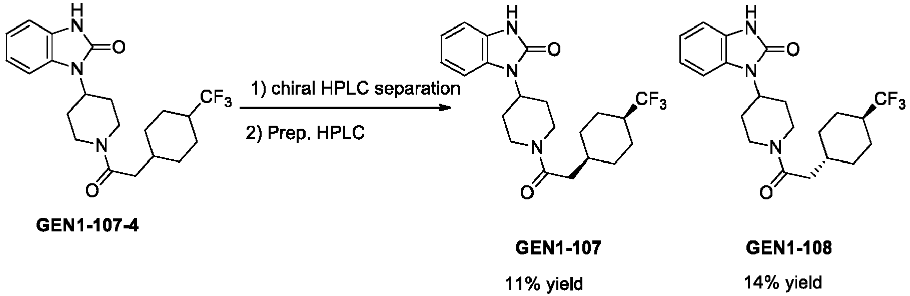

A cis/trans mixture of 1-(1-(2-(4-(trifluoromethyl)cyclohexyl)acetyl)piperidin-4-yl)-1H-benzo[d]imidazol-2(3H)-one (240 mg, 0.59) was separated by preparative chiral HPLC [Method: IB column, CO₂: IPA(0.2%DEA)=80:20, flow rate=0.6 mL / min, UV=230 nm] to obtain 1-(1-(2-((1S,4S)-4-(trifluoromethyl)cyclohexyl)acetyl)piperidin-4-yl)-1H-benzo[d]imidazole-2(3H)-one (45 mg, purity 91%) and 1-(1-(2-((1R,4R)-4-(trifluoromethyl)cyclohexyl)acetyl)piperidin-4-yl)-1H-benzo[d]imidazol-2(3H)-one (80 mg, purity 91%).

The two products were passed through preparative HPLC [gilson-2, Xbridge C₁₈, 5um, 19 * 150 mm, 40∼80%B, A: H₂O(0.1%NH₄HCO₃), B: ACN, flow rate: 15 mL/min, UV = 214nm] to obtain 1-(1-(2-((1S,4S)-4-(trifluoromethyl)cyclohexyl)acetyl)piperidin-4-yl)-1H-benzo[d]imidazol-2(3H)-one (28 mg, yield 11%) as a white solid and 1-(1-(2-((1R,4R)-4-(trifluoromethyl)cyclohexyl)acetyl)piperidin-4-yl)-1H-benzo[d]imidazol-2(3H)-one(35 mg, yield 14%) as a white solid.

Analyze data, (1-(2-((1S,4S)-4-(trifluoromethyl)cyclohexyl)acetyl)piperidin-4-yl)-1H-benzo(d)imidazol-2(3H)-one: ¹H NMR (400 MHz, DMSO-*d*₆): δ 10.84 (s, 1H), 7.20-7.18 (m, 1H), 6.99-6.97 (m, 3H), 4.59 (d, *J* = 13.2 Hz, 1H), 4.44-4.37 (m, 1H), 4.08 (d, *J* = 13.2 Hz, 1H), 3.15 (t, *J* = 12.4 Hz, 1H), 2.64 (t, *J* = 12.0 Hz, 1H), 2.42-2.40 (m, 2H), 2.29-2.23 (m, 2H), 2.15-2.09 (m, 2H), 1.76-1.68 (m, 2H), 1.64-1.61 (m, 2H), 1.56-1.52 (m, 6H). MS (ESI): Rt = 3.855 min, *m*/*z* 410.2 [M+H]⁺; Purity: 97.1% @ 254 nm, 97.7% @ 214 nm.

Analyze data, 1-(1-(2-((1R,4R)-4-(trifluoromethyl)cyclohexyl)acetyl)piperidin-4-yl)-1H-benzo[d]imidazol-2(3H)-one: ¹H NMR (400 MHz, DMSO-*d*₆): δ 10.84 (s, 1H), 7.20-7.17 (m, 1H), 6.97-6.96 (m, 3H), 4.59 (d, *J* = 12.4 Hz, 1H), 4.45-4.37 (m, 1H), 4.03 (d, *J* = 13.2 Hz, 1H), 3.15 (t, *J* = 12.4 Hz, 1H), 2.64 (t, *J* = 12.0 Hz, 1H), 2.29-2.27 (m, 2H), 2.24-2.06 (m, 3H), 1.88-1.83 (m, 4H), 1.74-1.69 (m, 3H), 1.32-1.22 (m, 2H), 1.10-0.98 (m, 2H). MS (ESI): Rt = 3.862 min, *m*/*z* 410.1 [M+H]⁺; Purity: 98.5% @ 254 nm, 98.2% @ 214 nm.

### Example 92: N-(4-(2-oxo-2-(4-(2-oxo-2,3-dihydro-1H-benzo[d]imidazol-1-yl)piperidin-1-yl)ethyl)phenyl)acetamide (GEN1-109)

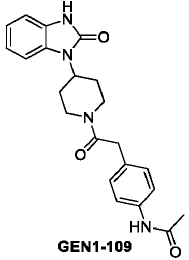

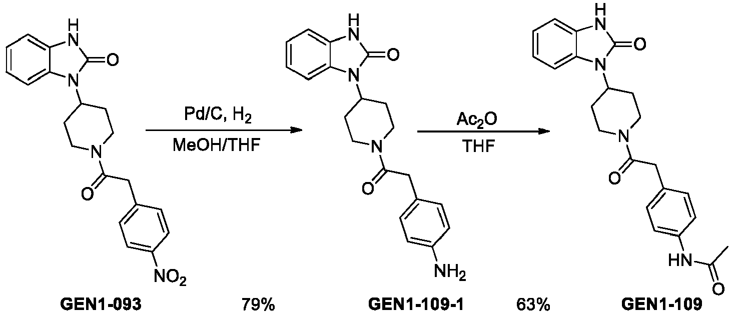

### Step 1. Synthesis of 1-(1-(2-(4-aminophenyl)acetyl)piperidin-4-yl)-1H-benzo[d]imidazol-2(3H)-one (GEN1-109-1)

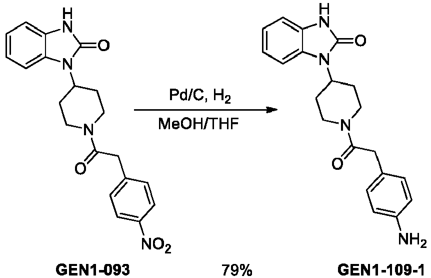

1-(1-(2-(4-nitrophenyl)acetyl)piperidin-4-yl)-1H-benzo[d]imidazol-2(3H)-one (274 mg, 720 mmol, the synthesis steps could be found in the final report of GEN 1-093) was dissolved in a solution of MeOH (20 mL) and THF (20 mL), and Pd/C (10% purity, 77 mg, 0.72 mmol) was added. The resulting mixture was stirred in hydrogen for 3 hours at room temperature. The mixture was filtered, concentrated and purified by C₁₈ (40-60% B; A: H₂O B: CH₃CN) to obtain the desired compound (200 mg, yield 79%) as a yellow solid. MS (ESI): Rt = 1.24 min, *m*/*z* 351.4 [M+H]⁺; Purity: 100% @ 254 nm, 100% @ 214 nm.

### Step 2. Synthesis of N-(4-(2-oxo-2-(4-(2-oxo-2,3-dihydro-1H-benzo[d]imidazol-1-yl)piperidin-1-yl)ethyl)phenyl)acetamide (GEN1-109)

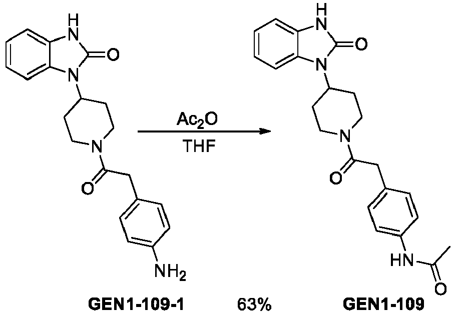

1-(1-(2-(4-aminophenyl)acetyl)piperidin-4-yl)-1H-benzo[d]imidazol-2(3H)-one (80 mg, 0.23 mmol) was dissolved in THF (6 mL), and Ac₂O (35 mg, 0.34 mmol) was added dropwise in an ice bath, and after the addition, the mixture was stirred at room temperature for 4 hours. The mixture was concentrated and the residue was purified by preparative HPLC (preparative 5 Xbridge C18 5um 19 * 150mm, 10-60% B; A: H₂O (0.1% NH₄HCO₃), B: CH₃CN; 214 nm, flow rate: 15mL/min), the desired compound (59 mg, yield 63%) was obtained as a white solid. ¹H NMR (400 MHz, CDCl₃): δ 8.76 (s, 1H), 7.52 (d, *J* = 8.8 Hz, 2H), 7.33 (s, 1H), 7.27 (d, *J=* 8.0 Hz, 2H), 7.08-7.02 (m, 3H), 6.91-6.85 (m, 1H), 4.88 (d, *J* = 15.2 Hz, 1H), 4.54-4.45 (m, 1H), 4.03 (d, *J* = 13.2 Hz, 1H), 3.78 (s, 2H), 3.16-3.10 (m, 1H), 2.71-2.64 (m, 1H), 2.30-2.19 (s, 1H), 2.16 (s, 3H), 2.00-1.90 (m, 1H), 1.87-1.83 (m, 1H), 1.73-1.69 (m, 1H). MS (ESI): Rt = 3.134 min, *m*/*z* 393.2 [M+H]⁺; Purity: 99.00% @ 254 nm, 96.16% @ 214 nm.

### Example 93: (N-(4-(2-oxo-2-(4-(2-oxo-2,3-dihydro-1H-benzo[d]imidazol-1-yl)piperidin-1-yl)ethyl)phenyl)methanesulfonamide (GEN1-110)

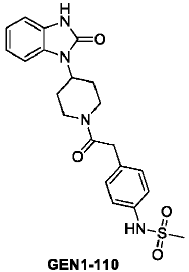

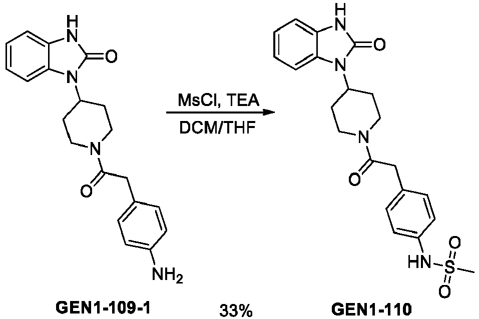

### Step 1. Synthesis of (N-(4-(2-oxo-2-(4-(2-oxo-2,3-dihydro-1H-benzo[d]imidazol-1-yl)piperidin-1-yl)ethyl)phenyl)methanesulfonamide (GEN1-110)

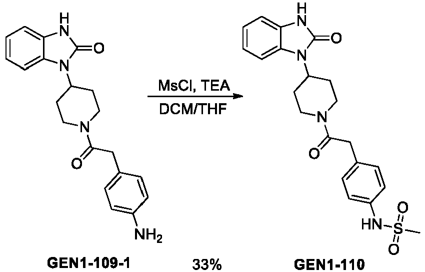

1-(1-(2-(4-aminophenyl)acetyl)piperidin-4-yl)-1H-benzo[d]imidazol-2(3H)-one (100 mg, 0.285 mmol, the synthesis steps could be found in the final report of GEN1-109-1) and TEA (144 mg, 1.42 mmol) were dissolved in DCM (5 mL) and THF (5 mL), and MsCl (163 mg, 1.42 mmol) was added in four batches at 0°C. The mixture was then stirred at room temperature for 28 hours. The resulting mixture was concentrated and the residue was purified by preparation (Preparation 5 Xbridge C₁₈ 5 um 19*150 mm, 20-60% B; A: H₂O (0.1% NH₄HCO₃), B: CH₃CN; 214 nm, flow rate: 15 mL/min). The desired compound (42 mg, yield 33%) was obtained as a white solid. ¹HNMR (400 MHz, CDCl₃): δ 8.73 (s, 1H), 7.37 (s, 1H), 7.31-7.29 (m, 2H), 7.25-7.23 (m, 2H), 7.09-7.05 (m, 3H), 6.94-6.92 (m, 1H), 4.88 (d, *J* = 12.0 Hz, 1H), 4.50-4.42 (m, 1H), 4.06 (d, *J* = 13.6 Hz, 1H), 3.79 (s, 2H), 3.21-3.15 (m, 1H), 2.93 (s, 3H), 2.73-2.67 (m, 1H), 2.35-2.25 (m, 1H), 2.08-1.98 (m, 1H), 1.87 (d, *J* = 12.8 Hz, 1H), 1.76 (d, *J* = 10.8 Hz, 1H). MS (ESI): Rt = 2.669 min, *m*/*z* 429.2 [M+H]⁺; 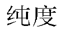 (Purity): 86.53% @ 254 nm, 95.14% @ 214 nm.

### Example 94: 1-(1-(2-(4-(tert-butyl)phenyl)acetyl)piperidin-4-yl)-7-(trifluoromethyl)-1H-benzo[d]imidazol-2(3H)-one (GEN1-115)

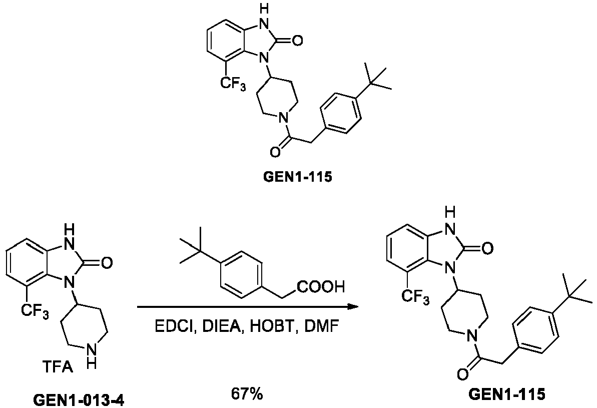

### Step 1. Synthesis of 1-(1-(2-(4-(tert-butyl)phenyl)acetyl)piperidin-4-yl)-7-(trifluoromethyl)-1H-benzo[d]imidazol-2(3H)-one (GEN1-115)

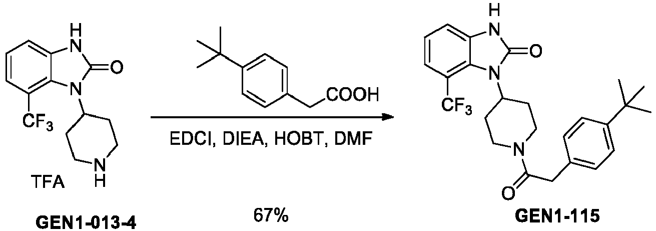

Compound 1-(piperidin-4-yl)-7-(trifluoromethyl)-1H-benzo[d]imidazol-2(3H)-1,2,2,2-trifluoroacetate (100 mg, 250 umol, the synthesis steps could be found in the final report of GEN1-013-4), EDCI (72 mg, 0.36 mmol), HOBT (51 mg, 0.38 mmol) and DIEA (162 mg, 1.25 mmol) were dissolved in DMF (2 mL) and 2-(4-tert-butylphenyl)acetic acid (58 mg, 0.30 mmol) was added. After the addition, the resulting mixture was stirred at room temperature for 16 hours. Then the mixture was purified by preparation (preparation 5 Xbridge C₁₈ 5 um 19 * 150 mm, 50-95% B; A: H₂O (0.1% NH₄HCO₃), B: CH₃CN; 214 nm, flow rate: 15 mL/min). The desired compound (79 mg, yield 67%) was obtained as a white solid. ¹H NMR (400 MHz, CDCl₃): δ 10.27 (s, 1H), 7.39 (d, *J* = 8.4 Hz, 1H), 7.35-7.31 (m, 3H), 7.22-7.20 (m, 2H), 7.14 (t, *J* = 8.0 Hz, 1H), 4.90 (d, *J* = 11.6 Hz, 1H), 4.45-4.39 (m, 1H), 4.08 (d, *J* = 14.0 Hz, 1H), 3.82-3.74 (m, 2H), 3.07-3.00 (m, 1H), 2.83-2.58 (m, 3H), 1.80 (t, *J* = 14.4 Hz, 2H), 1.28 (s, 9H). LCMS (ESI): Rt = 4.026 min, *m*/*z* 460.2 [M+H]⁺; Purity: 98.02% @ 254 nm, 99.33% @ 214 nm.

### Example 95: 1-(1-(2-(benzo[d][1,3]dioxol-5-yl)acetyl)piperidin-4-yl)-7-(trifluoromethyl)-1,3-dihydro-2H-benzo[d]imidazol-2-one (GEN1-116)

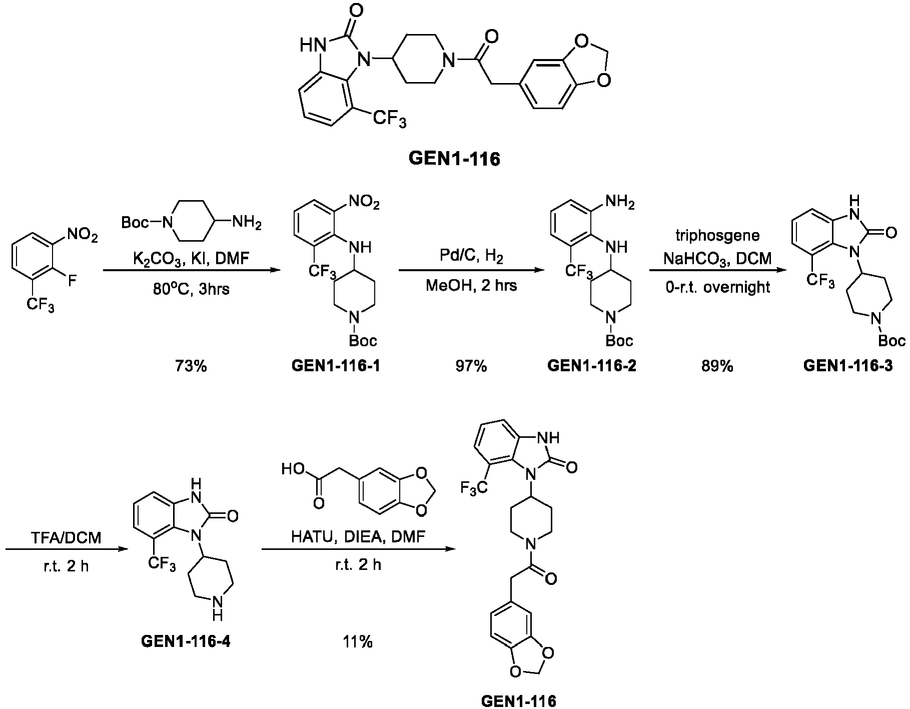

### Step 1. tert-butyl 4-(((2-nitro-6-(trifluoromethyl)phenyl)amino)piperidin-1-carboxylate (GEN1-116-1)

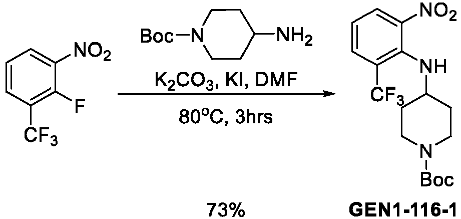

2-fluoro-1-nitro-3-(trifluoromethyl)benzene (500 mg, 2.39 mmol), tert-butyl 4-aminopiperidine-1-carboxylate (479 mg, 2.39 mmol), K₂CO₃ (495 mg, 3.58 mmol) and KI (38 mg, 0.24 mmol) were suspended in DMF (12 mL) and it was stirred at 80°C for 3 hours. The reaction mixture was cooled, poured into 25 ml water, extracted with EtOAc (20ml×3), the combined organic layer was washed with 50 ml water and 50 mL brine, dried over Na₂SO₄ and concentrated to obtain a black oily. The crude product was purified by silica gel column chromatography (PE: EtOAc = 1: 45) to obtain the desired compound (681 mg, yield 73%) as a yellow oil. TLC: EA/(EA+PE)=5%, Rf=0.3

### Step 2. tert-butyl 4-((2-amino-6-(trifluoromethyl)phenyl)amino)piperidin-1-carboxylate (GEN1-116-2)

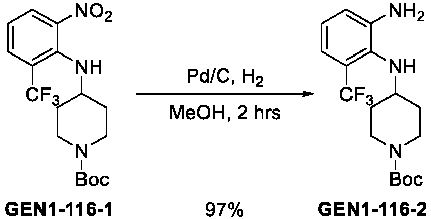

A mixture of tert-butyl 4-((2-nitro-6-(trifluoromethyl)phenyl)amino)piperidin-1-carboxylate (681 mg, 1.75 mmol) and Pd/C (100 mg) in MeOH (13 mL) was hydrogenated under a H2 atmosphere at room temperature for 2 hours. The mixture was filtered through kieselguhr, and the filtrate was concentrated to obtain the desired compound (612 mg, yield 97%) as a black-yellow oil. TLC: EA/(EA+PE)=20%, Rf=0.2

### Step 3. Tert-butyl 4-(2-oxo-7-(trifluoromethyl)-2,3-dihydro-1H-benzo[d]imidazol-1-yl)piperidin-1-carboxylate (GEN1-116-3)

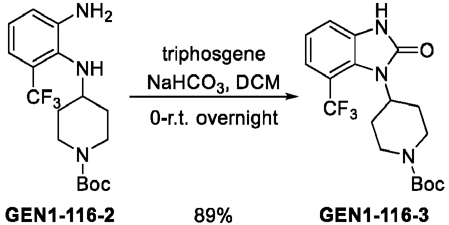

In an ice bath, triphosgene (125 mg, 0.43 mmol) was added to a suspension of tert-butyl 4-((2-amino-6-(trifluoromethyl)phenyl)amino)piperidin-1-carboxylate (306 mg, 0.85 mmol) and NaHCO₃ (358 mg, 4.26 mmol) in DCM (15ml). The resulting mixture was stirred overnight. The reaction mixture was poured into 20 mL of water, extracted with DCM (10 mL × 3), the mixed organic layer was washed with brine (20 mL), dried over Na₂SO₄, filtered and the filtrate was concentrated to obtain the desired compound (292 mg, yield 89%) as a white solid. TLC: EA/(EA+PE)=80%, Rf=0.4

### Step 4. 1-(piperidin-4-yl)-7-(trifluoromethyl)-1,3-dihydro-2H-benzo[d]imidazol-2-one (GEN1-116-4)

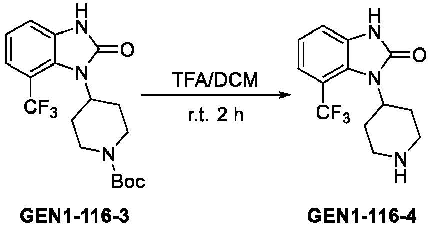

DCM (5.0 mL) was added to tert-butyl 4-(2-oxo-7-(trifluoromethyl)-2,3-dihydro-1H-benzo[d]imidazol-1-yl)piperidin-1-carboxylate (290 mg, 0.75 mmol), and TFA (1.5 mL) was added dropwise in an ice bath, and the mixture was stirred at room temperature for 6 hours. The reaction mixture was concentrated, and the residue was purified by reverse-phase flash chromatography (C₁₈ reversed silica gel column; mobile phase CH₃CN, water (0.1% NH₃H₂O), 5% to 70% gradient, 40 minutes; detection, UV 214nm). The desired compound (193 mg, yield 90%) was obtained as a white solid. MS (ESI): Rt =1.19 min, *m*/*z* 286.1 [M+H]⁺ , Purity: 92% @ 254 nm, 97% @ 214 nm.

### Step 5. 1-(1-(2-(benzo[d] [1,3]dioxol-5-yl)acetyl)piperidin-4-yl)-7-(trifluoromethyl)-1,3-dihydro-2H-benzo[d]imidazol-2-one (GEN1-116)

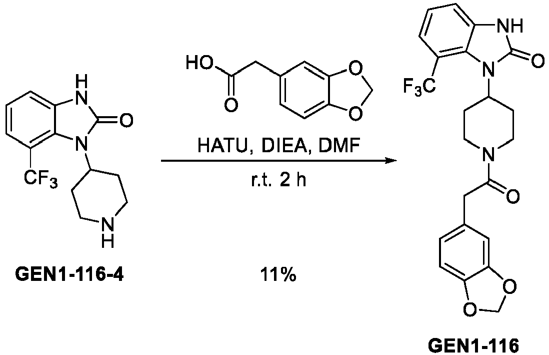

Compound 1-(piperidin-4-yl)-7-(trifluoromethyl)-1,3-dihydro-2H-benzo[d]imidazol-2-one (148 mg, 0.52 mmol), HATU (296 mg, 0.78 mmol), DIEA (536 mg, 4.16 mmol) were dissolved in DMF (3 mL) and 2-(benzo[d] [1,3]dioxa-5-yl)acetic acid (94 mg, 0.52 mmol) was added. After the addition, the resulting mixture was stirred at room temperature for 15 hours. The reaction mixture was added dropwise to water (50 mL) and the mixture was filtered to obtain a white solid. The crude product was purified by reversed-phase flash chromatography (C₁₈ reversed silica gel column; mobile phase CH₃CN, water, 10% to 70% gradient, 40 minutes; detection, UV 214nm). The desired compound (25.4 mg, yield 11%)) was obtained as a white solid. ¹H NMR (400 MHz, DMSO-d₆) δ 11.45 (s, 1H), 7.36 (d, *J* = 8.0 Hz, 1H), 7.27 (d, *J* = 7.6 Hz, 1H), 7.16 (t, *J* = 7.9 Hz, 1H), 6.87 - 6.78 (m, 2H),6.74-6.67 (m, 1H), 5.98 (d, *J* = 1.4 Hz, 2H), 4.58 (d, *J* = 11.1 Hz, 1H), 4.23 (s, 1H), 4.14 (d, *J* = 14.0 Hz, 1H), 3.76-3.59 (m, 2H), 2.97 (t, *J* = 13.2 Hz, 1H), 2.61-2.51 (m, 1H), 2.49 - 2.38 (m, 1H), 1.73 - 1.65 (m, 2H). MS (ESI): Rt = 3.49 min, *m*/*z* 448.2 [M+H]⁺, Purity: 92% @ 254 nm, 98% @ 214 nm.

### Example 96: 1-(1-(2-(quinolin-6-yl)acetyl)piperidin-4-yl)-7-(trifluoromethyl)-1H-benzo[d]imidazol-2(3H)-one (GEN1-117)

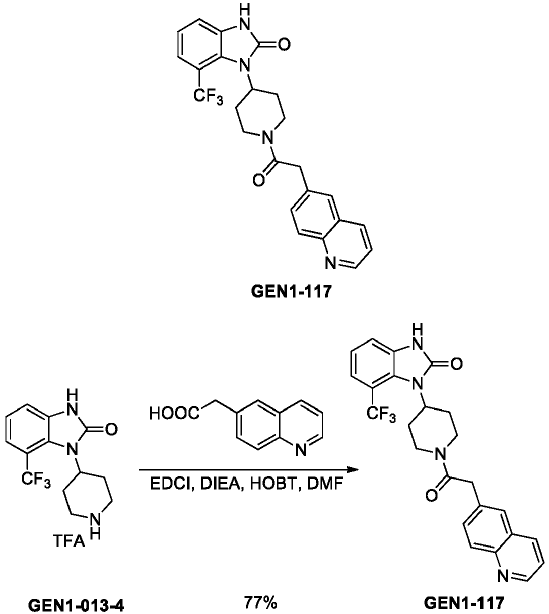

### Step 1. 1-(1-(2-(quinolin-6-yl)acetyl)piperidin-4-yl)-7-(trifluoromethyl)-1H-benzo[d]imidazol-2(3H)-one (GEN1-117)

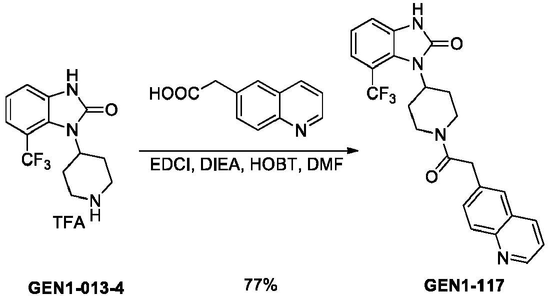

Compound 1-(piperidm-4-yl)-7-(trifluoromethyl)-1H-benzo[d]imidazol-2(3H)-one 2,2,2-trifluoroacetate (100 mg, 250 umol, the synthesis steps could be found in the final report of GEN1-013-4), EDCI (72 mg, 0.36 mmol), HOBT (51 mg, 0.38 mmol), DIEA (162 mg, 1.25 mmol) were dissolved in DMF (2 mL) and 2-(6-quinolinyl)acetic acid (56 mg , 0.30 mmol) was added. After the addition, the resulting mixture was stirred at room temperature for 16 hours. Then the mixture was purified by preparation (Preparation 5 Xbridge C₁₈ 5um 19 * 150mm, 30-70% B; A: H₂O (0.1% NH₄HCO₃), B: CH₃CN; 214 nm, flow rate: 15 mL/min). The desired compound (88 mg, yield 77%) was obtained as a white solid. ¹HNMR (400 MHz, DMSO-*d*₆): δ 11.45 (s, 1H), 8.87 (dd, *J* = 4.4, 1.6 Hz, 1H), 8.33 (d, *J* = 7.2 Hz, 1H), 7.97 (d, *J* = 8.4 Hz, 1H), 7.82 (s, 1H), 7.66 (dd, *J* = 8.8, 2.0 Hz, 1H), 7.52 (dd, *J* = 8.4, 4.4 Hz, 1H), 7.36 (d, *J* = 8.4 Hz, 1H), 7.28 (d, *J* = 7.2 Hz, 1H), 7.16 (t, *J* = 8.0 Hz, 1H), 4.64 (d, *J* =10.0 Hz, 1H), 4.29-4.20 (m, 2H), 4.01-3.96 (m, 2H), 3.09-3.02 (m, 1H), 2.61-2.54 (m, 3H), 1.72 (s, 2H). LCMS (ESI): Rt = 3.694 min, *m*/*z* 455.1 [M+H]⁺; Purity: 98.00% @ 254 nm, 99.57% @ 214 nm.

### Example 97: 1-(1-(2-(4-(dimethylamino)phenyl)acetyl)piperidin-4-yl)-7-(trifluoromethyl)-1,3-dihydro-2H-benzo[d]imidazol-2-one (GEN1-118)

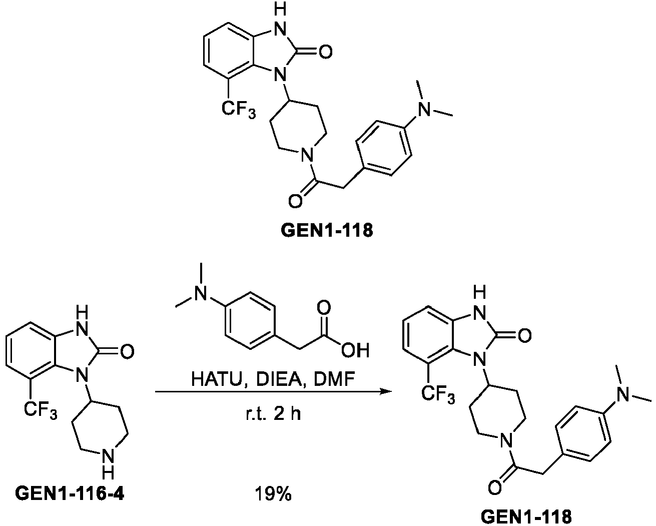

### Step 1. 1-(1-(2-(4-(dimethylamino)phenyl)acetyl)piperidin-4-yl)-7-(trifluoromethyl)-1,3-dihydro-2H-benzo[d]imidazol-2-one (GEN1-118)

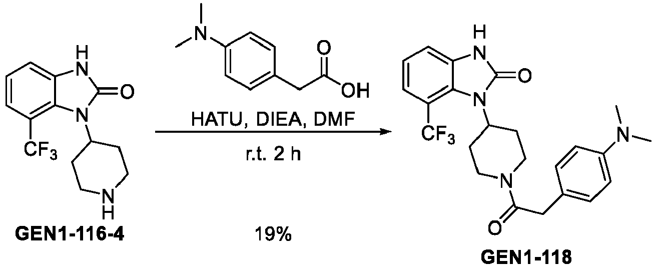

Compound 1-(piperidin-4-yl)-7-(trifluoromethyl)-1,3-dihydro-2H-benzo[d]imidazol-2-one (71 mg, 0.25 mmol), HATU (140 mg, 0.37 mmol), DIEA (129 mg, 1.00 mmol) were dissolved in DMF (1.5 mL) and 2-(4-(dimethylamino)phenyl)acetic acid (46 mg, 0.25 mmol) was added. After the addition, the resulting mixture was stirred at room temperature for 5 hours. The reaction mixture was added dropwise to water (50 mL) and the mixture was filtered to obtain a white solid. The crude product was purified by Prep-TLC (MeOH/(MeOH + DCM)=60%). The desired compound (21.1 mg, yield 19%) was obtained as a white solid. ¹H NMR (400 MHz, DMSO-*d*₆): δ 11.45 (s, 1H), 7.36 (d, *J* = 8.2, 1H), 7.27 (d, *J* = 7.8 Hz, 1H), 7.16 (t, *J* = 7.9 Hz, 1H), 7.06 (d, *J* = 8.6 Hz, 2H), 6.67 (d, *J=* 8.4 Hz, 2H), 4.67 - 4.54 (m, 1H), 4.29-4.07 (m, 2H), 3.74-3.49 (m, 2H), 2.93 (t, *J =* 13.1 Hz, 1H), 2.86 (s, 6H), 2.56-2.51 (m, 1H), 2.49-2.33 (m, 2H), 1.75-1.59 (m, 2H). MS (ESI): Rt = 1.62 min, *m*/*z* 446.9 [M+H]⁺, Purity: 100% @ 254 nm, 100% @ 214 nm.

### Example 98: 4-(1-(2-(quinolin-6-yl)acetyl)piperidin-4-yl)-3,4-dihydroquinoxaline-2(1H)-one (GEN1-120)

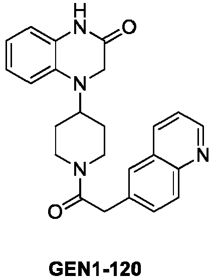

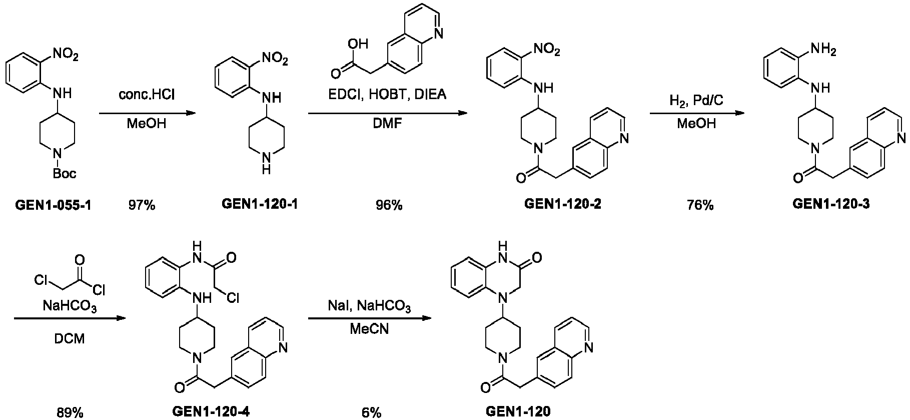

### Step 1. Ethyl N-(2-nitrophenyl)piperidin-4-amine (GEN1-120-1)

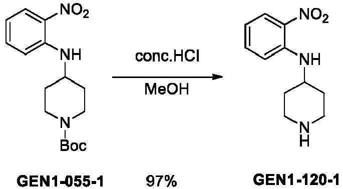

The compound tert-butyl 4-(2-nitroanilino)piperidin-1-carboxylate (300 mg, 0.930 mmol, the synthesis steps could be found in the final report of GEN1-055-1.) was dissolved in a solution of MeOH (5 mL), concentrated hydrochloric acid (2 mL) was added at room temperature, and the mixture was stirred at room temperature for 1 hour, the mixture was concentrated. The residue was diluted with water (10 mL), and a saturated aqueous solution of Na₂CO₃ (20 mL) was added dropwise. The mixture was extracted with DCM: MeOH = 10: 1 (30 mL×2). The combined organic phase was washed with brine (10 mL), dried over Na₂SO₄ and it was concentrated to obtain the desired compound (200 mg, yield 97%) as an orange solid. MS (ESI): Rt = 1.20 min, *m*/*z* 222.2 [M+H]⁺; Purity: 100% @ 254 nm, 100% @ 214 nm.

### Step 2. 1-(4-((2-nitrophenyl)amino)piperidin-1-yl)-2-(quinolin-6-yl)ethanone (GEN1-120-2)

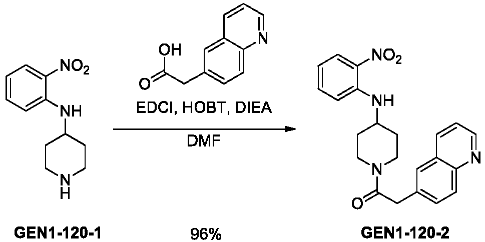

N-(2-nitrophenyl)piperidin-4-amine (200 mg, 0.900 mmol), 2-(6-quinolinyl)acetic acid (169 mg, 0.900 mmol) and DIEA (350 mg, 2.71 mmol) were dissolved in DMF (5 mL), and EDCI (260 mg, 1.36 mmol) and HOBT (183 mg, 1.36 mmol) were added to the mixed solution at 5°C. The mixture was stirred at room temperature overnight. The reaction mixture was diluted with water (20 mL), and extracted with EtOAc (20 mL×3). The combined organic phase was dried over anhydrous Na₂SO₄ and filtered. The filtrate was concentrated, and the residue was purified by C₁₈ flash chromatography (CH₃CN: water=50% to 80%) to obtain the desired compound (340 mg, yield 96%) as a yellow solid. MS (ESI): Rt = 1.50 min, *m*/*z* 391.4 [M+H]⁺; Purity: 100% @ 254 nm, 100% @ 214 nm.

### Step 3. 1-(4-((2-aminophenyl)amino)piperidin-1-yl)-2-(quinolin-6-yl)ethanone (GEN1-120-3)

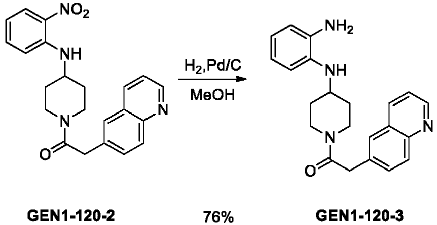

A mixture of 1-(4-((2-nitrophenyl)amino)piperidin-1-yl)-2-(quinolin-6-yl)ethenone (340 mg, 0.870 mmol) and Pd/C (10%, 100 mg) in methanol (10 mL) was hydrogenated under an H₂ atmosphere at 70°C for 3 hours. The mixture was cooled and filtered through kieselguhr. The filtrate was concentrated to obtain the desired compound (300 mg, yield 76%) as a brown oil. MS (ESI): Rt = 1.37 min, *m*/*z* 361.5 [M+H]⁺; Purity: 86% @ 254 nm, 79% @ 214 nm.

### Step 4. 2-chloro-N-(2-(((1-(2-(quinolin-6-yl)acetyl)piperidin-4-yl)amino)phenyl)acetamide (GEN1-120-4)

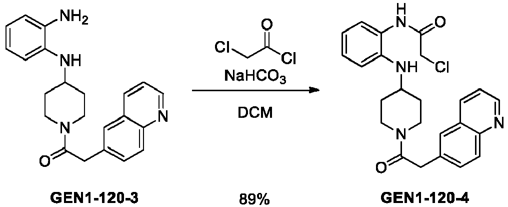

1-(4-(((2-aminophenyl)amino)piperidin-1-yl)-2-(quinolin-6-yl)ethenone (300 mg, 0.660 mmol) and NaHCO₃ (276 mg, 3.29 mmol) were dissolved in DCM (50 mL), and a solution of chloroacetyl chloride (74 mg, 0.66 mmol) in DCM (1 mL) was added dropwise at 0°C. The mixture was stirred at 0°C for 1 hour. The reaction mixture was diluted with water (20 mL), and extracted with DCM (10 mL×2). The combined organic phase was washed with brine (20 mL), dried over anhydrous Na₂SO₄ and it was filtered. The filtrate was concentrated to obtain the desired compound (300 mg, yield 89%) as a brown solid. MS (ESI): Rt = 1.38 min, *m*/*z* 437.4 [M+H]⁺; Purity: 82% @ 254 nm, 85% @ 214 nm.

### Step 5. 4-(1-(2-(quinolin-6-yl)acetyl)piperidin-4-yl)-3,4-dihydroquinoxaline-2(1H)-one (GEN1-120)

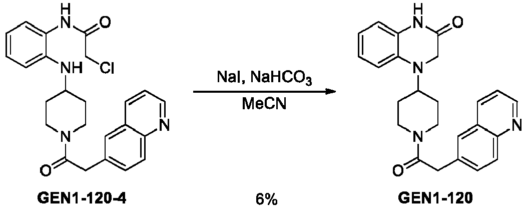

2-chloro-N-(2-((1-(2-(quinolin-6-yl)acetyl)piperidin-4-yl)amino)phenyl)acetamide (300 mg, 0.580 mmol), NaHCO₃ (245 mg, 2.92 mmol) and NaI (18 mg, 0.12 mmol) were dissolved in acetonitrile (50 mL), and the mixture was stirred at 90°C for 2 hours. The mixture was cooled and concentrated. The residue was triturated with water (5 mL) and it was filterred. The filter cake was washed with water (5 mL×2) and dried to obtain a crude product (200 mg). Then the mixture was purified three times by preparation [gilson-2, Xbridge C18, 5um, 19 * 150 mm, 20-60% B, A: H₂O (0.1% NH₄HCO₃), B: ACN, flow rate: 15 mL / min, UV purification = 214nm] to obtain the desired compound (15 mg, yield 6%) as a white solid. ¹H NMR (400 MHz, DMSO-*d*₆): δ 10.36 (s, 1H), 8.86 (dd, *J* = 4.0, 2.0 Hz, 1H), 8.33 (d, *J* = 8.4 Hz, 1H), 7.97 (d, *J* = 8.4 Hz, 1H), 7.81 (s, 1H), 7.65 (dd, *J* = 8.8, 2.0 Hz, 1H), 7.51 (dd, *J* = 8.4, 4.0 Hz, 1H), 6.90-6.85 (m, 2H), 6.81-6.79 (m, 1H), 6.71-6.67 (m, 1H), 4.57 (d, *J* = 12.8 Hz, 1H), 4.13 (d, *J* = 12.4 Hz, 1H), 4.02-3.92 (m, 2H), 3.87-3.81 (m, 1H), 3.49 (dd, *J* = 32.4, 6.8 Hz, 2H), 3.19 (t, *J* = 12.0 Hz, 1H), 2.73 (t, *J* = 12.4 Hz, 1H), 1.71-1.53 (m, 4H). MS (ESI): Rt = 2.789 min, *m*/*z* 401.2 [M+H]⁺; Purity: 91.05% @ 254 nm, 93.87% @ 214 nm.

### Example 99: 4-(1-(2-(4-(dimethylamino)phenyl)acetyl)piperidin-4-yl)-3,4-dihydroquinoxaline-2(1H)-one (GEN1-121)

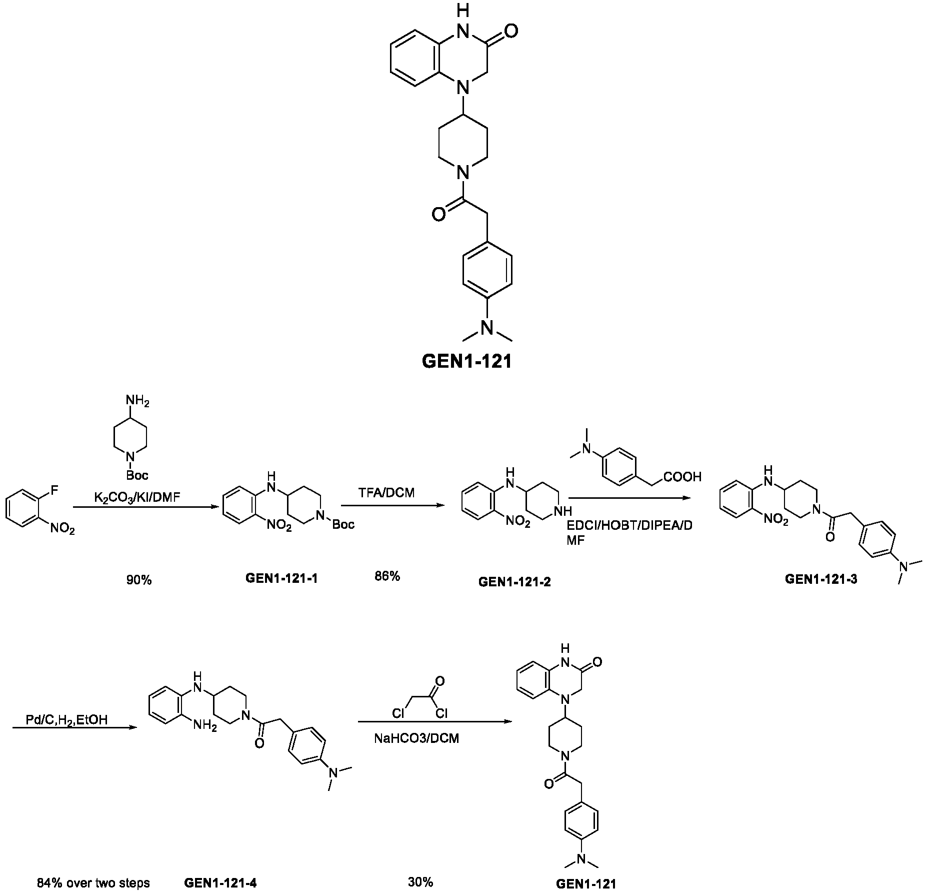

### Stpe 1. Synthesis of tert-butyl 4-((2-nitrophenyl)amino)piperidin-1-carboxylate (GEN1-121-1)

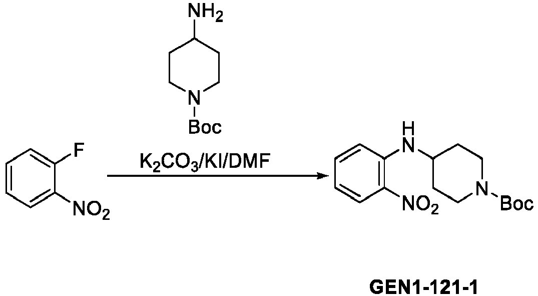

Tert-butyl 4-aminopiperidine-1-carboxylate (9.94 g, 49.61 mmol) was added to a suspension of 1-fluoro-2-nitrobenzene (7.00 g, 49.61 mmol), K₂CO₃ (13.71 g, 99.22 mmol) and KI (823.36 mg, 4.96 mmol) in DMF (50 mL). The resulting mixture was stirred at 80°C for 4 hours. The reaction mixture was diluted with water (300 mL), and extracted with EtOAc (100 mL×3). The combined organic phase was washed with brine (150 mL), dried over anhydrous Na₂SO₄ and it was filtered. The filtrate was concentrated to obtain the desired compound (14.35 g, yield 90%) as a yellow oil.

### Step 2. Synthesis of N-(2-nitrophenyl)piperidin-4-amine (GEN1-121-2)

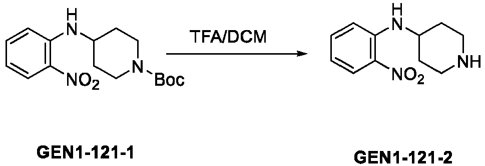

TFA (4 mL) was added to a solution of tert-butyl 4-(((2-nitrophenyl)amino)piperidin-1-carboxylate (1.4 g, 4.36 mmol) in DCM (20 mL). The mixture was stirred at room temperature for 1 hour. The resulting mixture was concentrated and redissolved in DCM (20ml). Then the mixture was concentrated and purified by silica gel column chromatography (PE:EA = 1:1) to obtain the desired compound (830mg, yield 86) %) as a brown solid. MS (ESI): Rt = 1.22 min, *m*/*z* 222.1 [M+H]⁺; Purity: 100% @ 254 nm, 100% @ 214 nm.

### Step 3. Synthesis of 2-(4-(dimethylamino)phenyl)-1-(4-((2-nitrophenyl)amino)piperidin-1-yl)ethan-1-one (GEN1-121-3)

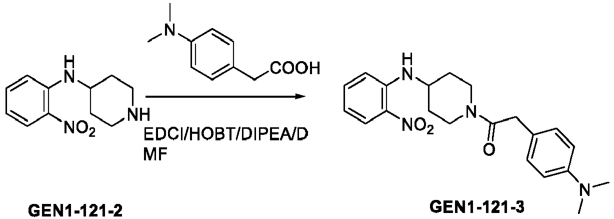

EDCI (260 mg, 1.356 mmol) was added to a solution of N-(2-nitrophenyl)piperidin-4-amine (200 mg, 0.904 mmol), 2-(4-(dimethylamino)phenyl)acetic acid (194.4 mg, 1.085 mmol), HOBT (183.2 mg, 1.356), DIEA (233.2 mg, 1.808 mmol) in DMF (5 mL). The resulting mixture was stirred at room temperature for 1 h. The reaction mixture was diluted with water (300 mL), and it was extracted with EtOAc (100 mL×3). The combined organic phase was washed with brine (150 mL), dried over anhydrous Na₂SO₄ and it was filtered. The filtrate was concentrated to obtain the crude product (310 mg), which was used directly in the next step without purification.

### Step 4. Synthesis of 1-(4-((2-aminophenyl)amino)piperidin-1-yl)-2-(4-(dimethylamino)phenyl)ethan-1-one (GEN1-121-4)

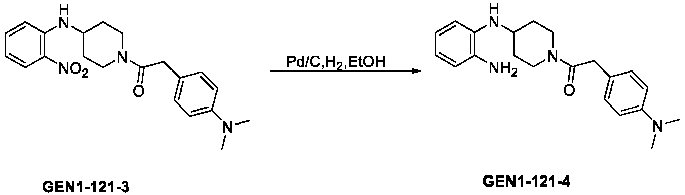

Pd/C (30 mg, 5% purity) was added to a solution of 2-(4-(dimethylamino)phenyl)-1-(4-(((2-nitrophenyl)amino)piperidin-1-yl)ethan-1-one (310 mg, 0.81 mmol) in EtOH (20 mL) at room temperature, and the resulting mixture was replaced with hydrogen and stirred overnight under a hydrogen atmosphere. The mixture was filtered, and the filtrate was concentrated to obtain the desired compound (240 mg, two steps yield: 84%) as a brown oil

### Step 5. 4-(1-(2-(4-(dimethylamino)phenyl)acetyl)piperidin-4-yl)-3,4-dihydroquinoxaline-2(1H)-one (GEN1-121)

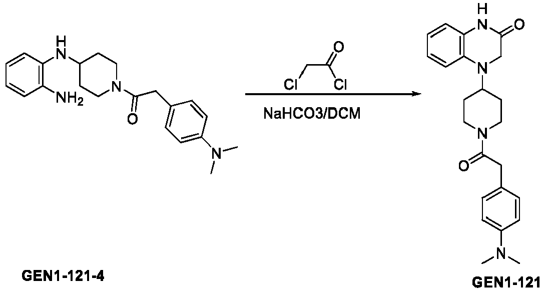

In an ice bath, 2-chloroacetyl chloride (77 mg, 0.68 mmol) was added to a solution of 1-(4-(((2-aminophenyl)amino)piperidin-1-yl)-2-(4-(dimethylamino)phenyl)ethan-1-one (240 mg, 0.68 mmol), NaHCO₃ (286 mg, 3.4 mmol) in DCM (5 mL). The resulting mixture was stirred at room temperature for 0.5 hour. The mixture was concentrated and dissolved in CH₃CN (10 mL). The mixture was stirred at 80°C overnight. The mixture was cooled and filtered. The filtrate was concentrated to obtain a crude product. The crude product was purified by reverse phase chromatography. Purification conditions: C18 silica gel column; mobile phase ACN: H₂O, gradient: 5% to 50%; 45 minutes, monitoring wavelength: 214 nm. The desired compound (80 mg, yield 30%) was obtained as a white solid. ¹HNMR (400 MHz, DMSO-d6) δ 10.37 (s, 1H), 7.06 (d, J = 8.6 Hz, 2H), 6.87 (d, J = 4.2 Hz, 2H), 6.79 (d, J = 7.6 Hz, 1H), 6.74 - 6.62 (m, 3H), 4.52 (d, J = 12.9 Hz, 1H), 4.01 (d, J = 13.6 Hz, 1H), 3.83-3.77 (m, 1H), 3.63-3.53 (m, 2H), 3.53 - 3.36 (m, 2H), 3.14-3.04 (m, 1H), 2.86 (s, 6H), 2.71-2.60 (m, 1H), 1.65 (d, J = 12.5 Hz, 1H), 1.61 - 1.30 (m, 3H). MS (ESI): Rt = 3.484 min, m/z 393.2, [M+H]⁺; Purity: 97.9% @ 254 nm, 98.66% @ 214 nm.

### Example 100: 4-(1-(2-(4-(tert-butyl)phenyl)acetyl)piperidin-4-yl)-3,4-dihydroquinoxaline-2(1H)-one (GEN1-122)

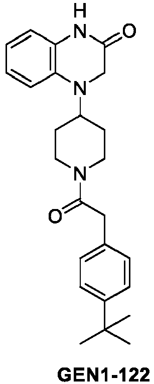

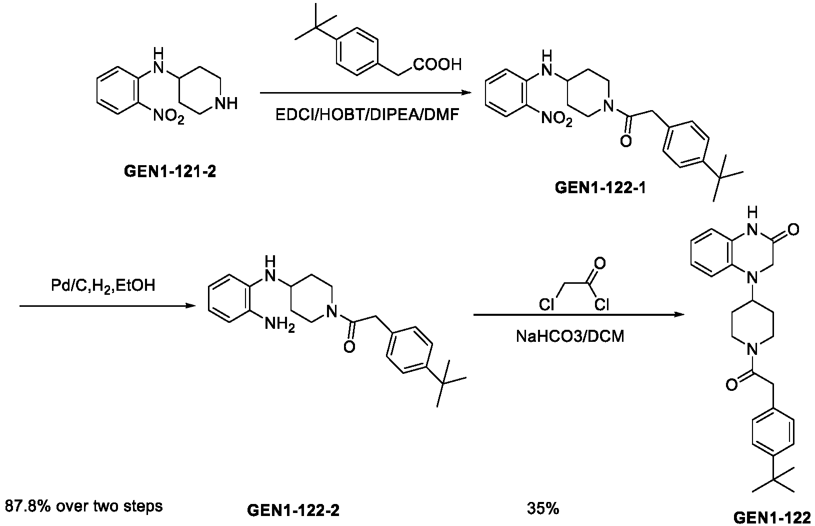

### Stpe 1. Synthesis of tert-butyl 4-((2-nitrophenyl)amino)piperidin-1-carboxylate (GEN1-122-1)

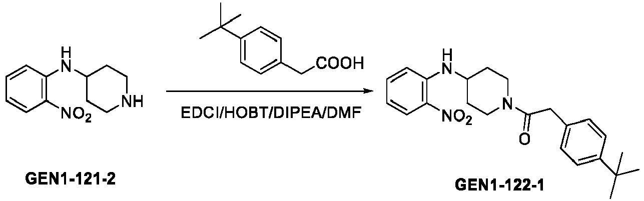

EDCI (260 mg, 1.356 mmol) was added a solution ofN-(2-nitrophenyl)piperidin-4-amine (200 mg, 0.904 mmol, the synthesis steps could be found in the final report of GEN1-121-2), 2-(4-(tert-butyl)phenyl)acetic acid (208.6 mg, 1.085 mmol), HOBT (183.2 mg, 1.356 mmol), DIEA (233.2 mg, 1.808 mmol) in DMF (5 mL). The resulting mixture was stirred at room temperature for 1 h. The reaction mixture was diluted with water (100 mL), and extracted with EtOAc (30 mL×3). The combined organic phase was washed with brine (50 mL), dried over anhydrous Na₂SO₄ and it was filtered. The filtrate was concentrated to obtain a crude product (350 mg). The crude product is used directly in the next step without purification.

### Step 2. Synthesis of 1-(4-((2-aminophenyl)amino)piperidin-1-yl)-2-(4-(tert-butyl)phenyl)ethan-1-one (GEN1-122-2)

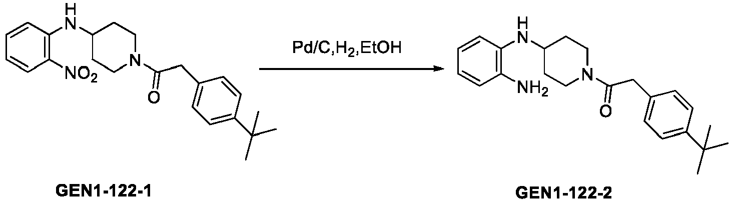

Pd/C (35 mg, 5% purity) was added to a solution of 2-(4-(tert-butyl)phenyl)-1-(4-(((2-nitrophenyl)amino)piperidin-1-yl)ethan-1-one (350 mg , 0.885 mmol) in EtOH (20mL). The resulting mixture was replaced with hydrogen and it was stirred at room temperature overnight under a hydrogen atmosphere. The mixture was filtered and the filtrate was concentrated to obtain the desired compound (284 mg, two steps yield: 87.8%) is brown oil. MS (ESI): Rt = 1.64 min, m/z 366.2, [M+H]⁺; Purity: 86.19% @ 254 nm, 90.14% @ 214 nm.

### Step 3. Synthesis of 4-(1-(2-(4-(tert-butyl)phenyl)acetyl)piperidin-4-yl)-3,4-dihydroquinoxaline-2(1H)-one (GEN1-122)

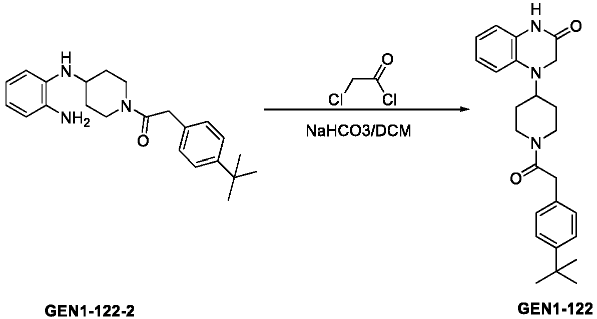

In an ice bath, 2-chloroacetyl chloride (88 mg, 0.777 mmol) was added to a solution of 1-(4-(((2-aminophenyl)amino)piperidin-1-yl)-2-(4-(tert-butyl)phenyl)ethan-1 -one (284 mg, 0.777 mmol), NaHCO₃ (334mg, 3.885mmol) in DCM (5mL). The resulting mixture was stirred at room temperature for 0.5 hour. The mixture was concentrated and it was dissolved in CH₃CN (10 mL). The mixture was stirred at 80°C overnight. The mixture was cooled and filtered. The filtrate was concentrated to obtain a crude product. The crude product was purified by reverse phase chromatography. Purification conditions: C18 silica gel column; mobile phase ACN: H₂O, gradient: 5% to 50%; 45 minutes, monitoring wavelength: 214 nm. The desired compound (110 mg, yield 35%) was obtained as a white solid. ¹H NMR (400 MHz, DMSO-d6) δ 10.37 (s, 1H), 7.33 (d, J = 4.4 Hz, 2H), 7.17 (d, J = 8.2 Hz, 2H), 6.73-6.64 (m, 2H), 6.79 (d, J = 7.6 Hz, 1H), 6.69 (d, J = 3.8 Hz, 1H), 4.52 (d, J = 12.9 Hz, 1H), 4.03 (d, J = 13.5 Hz, 1H), 3.888-.377 (m, 1H), 3.76 - 3.63 (m, 2H), 3.53 - 3.37 (m, 2H), 3.12 (t, J = 12.8 Hz, 1H), 2.67 (t, J = 12.3 Hz, 1H), 1.73 - 1.32 (m, 4H), 1.27 (s, 9H). MS (ESI): Rt = 1.59 min, m/z 406.3, [M+H]⁺; Purity: 100% @ 254 nm, 100% @ 214 nm.

### Example 101: 3-methyl-4-(1-(2-(4-(trifluoromethyl)phenyl)acetyl)piperidin-4-yl)-3,4-dihydroquinoxaline-2(1H)-one (GEN1-125)

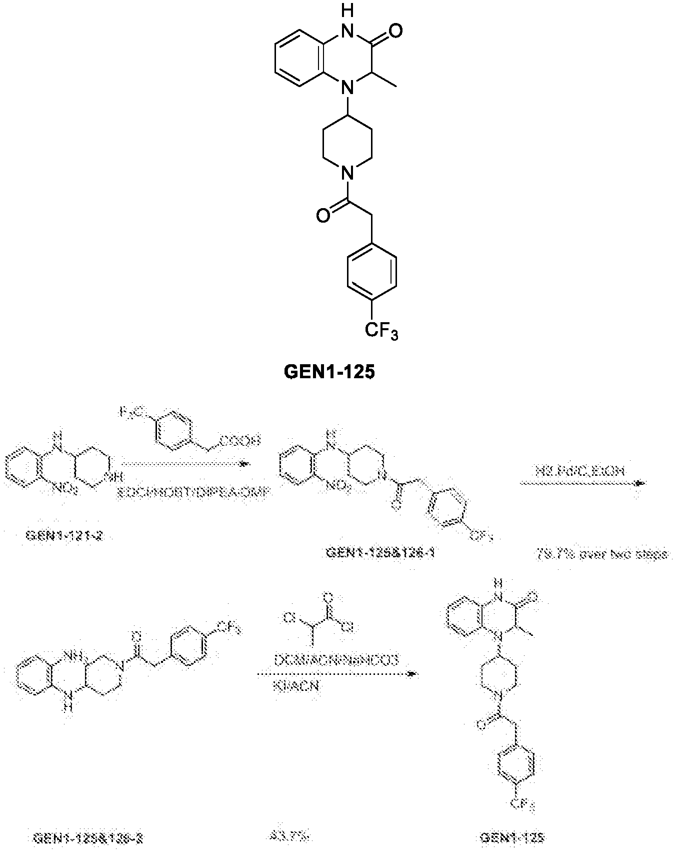

### Stpe 1. Synthesis of tert-butyl 4-((2-nitrophenyl)amino)piperidin-1-carboxylate (GEN1-122-1)

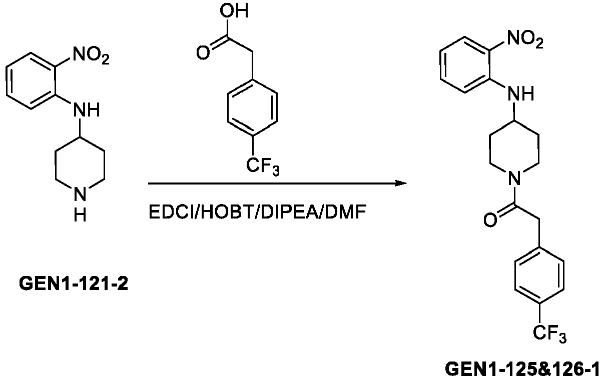

EDCI (535 mg, 2.79 mmol) was added to a solution of N-methyl-2-(piperidin-4-ylamino)benzamide (450 mg, 1.86 mmol), 2-(4-(trifluoromethyl)phenyl)acetic acid (456 mg, 2.23 mmol, the synthesis steps could be found in GEN1-121-2), HOBT (377 mg, 2.79 mmol) and DIEA (480 mg, 3.72 mmol) in DMF (10 mL). The resulting mixture was stirred at room temperature for 1 h. The reaction mixture was diluted with water (100 mL), and extracted with EtOAc (50 mL×3). The combined organic phase was washed with brine (150 mL), dried over anhydrous Na₂SO₄ and it was filtered. The filtrate was concentrated to obtain the desired crude compound (1 g) as a yellow oil.

### Step 2. Synthesis of 1-(4-((2-aminophenyl)amino)piperidin-1-yl)-2-(4-(trifluoromethyl)phenyl)ethan-1-one (GEN1-125&126-2)

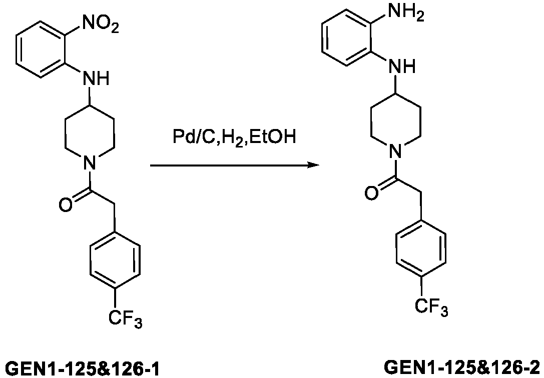

Pd/C (80 mg, 5% purity) was added to a solution of 1-(4-((2-nitrophenyl)amino)piperidin-1-yl)-2-(4-(trifluoromethyl)phenyl)ethan-1-one (1 g, crude product, 1.86 mmol) in EtOH (50 mL). The resulting mixture was replaced with hydrogen and it was stirred overnight at room temperature under a hydrogen atmosphere. The mixture was filtered and the filtrate was concentrated to obtain a crude product. The crude product was purified by silica gel column chromatography (PE:EA = 1:1) to obtain the desired compound (560 mg, yield 79.7%) as a pale yellow solid. MS (ESI): Rt = 1.54 min, *m*/*z* 378.1, [M+H]⁺; Purity: 95.64% @ 254 nm, 100% @ 214 nm.

### Step 3. Synthesis of 3-methyl-4-(1-(2-(4-(trifluoromethyl)phenyl)acetyl)piperidin-4-yl)-3,4-dihydroquinoxaline-2(1H)-one (GEN1-125)

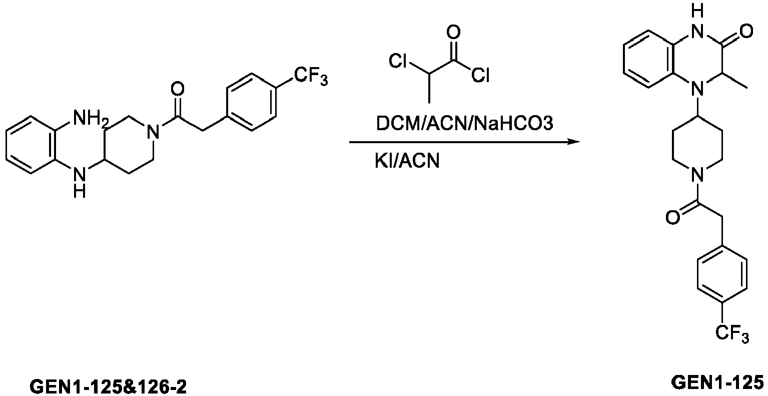

In an ice bath, 2-chloropropionyl chloride (33.64 mg, 0.265 mmol) was added to a solution of 1-(4-((2-aminophenyl)amino)piperidin-1-yl)-2-(4-(trifluoromethyl)phenyl)ethan-1-one (100 mg , 0.265 mmol), NaHCO₃ (222.6 mg, 2.65 mmol) in DCM (5 mL). The resulting mixture was stirred at room temperature for 0.5 hour. The mixture was concentrated and dissolved in CH₃CN (10 mL). The mixture was stirred at 80°C overnight. The mixture was cooled and filtered. The filtrate was concentrated to obtain a crude product. The crude product was purified by reverse phase chromatography. Purification conditions: C18 silica gel column; mobile phase ACN: H₂O, gradient: 5% to 50%; 40 minutes, monitoring wavelength: 214 nm. (50mg, yield 43.7%), white solid. ¹H NMR (400 MHz, DMSO-d6) δ 10.32 (s, 1H), 7.74 - 7.64 (m, 2H), 7.47 (t, J = 7.5 Hz, 2H), 7.00 - 6.87 (m, 2H), 6.84 - 6.71 (m, 2H), 4.55-4.40 (m, 1H), 4.10-3.97 (m, 1H), 3.96 - 3.77 (m, 3H), 3.75-3.62 (m, 1H), 3.22-3.04 (m, 1H), 2.77 - 2.58 (m, 1H), 1.97-1.83 (m, 1H), 1.70 - 1.41 (m, 3H), 0.94 (dd, J = 16.7, 6.7 Hz, 3H). MS (ESI): Rt = 3.825 min, m/z 132.2, [M+H]⁺; Purity: 96.6% @ 254 nm, 96.37% @ 214 nm.

### Example 102: 1-(1-(2-(4-(trifluoromethyl)phenyl)acetyl)piperidin-4-yl)-1,4-dihydroquinoxaline-2,3-dione (GEN1-127)

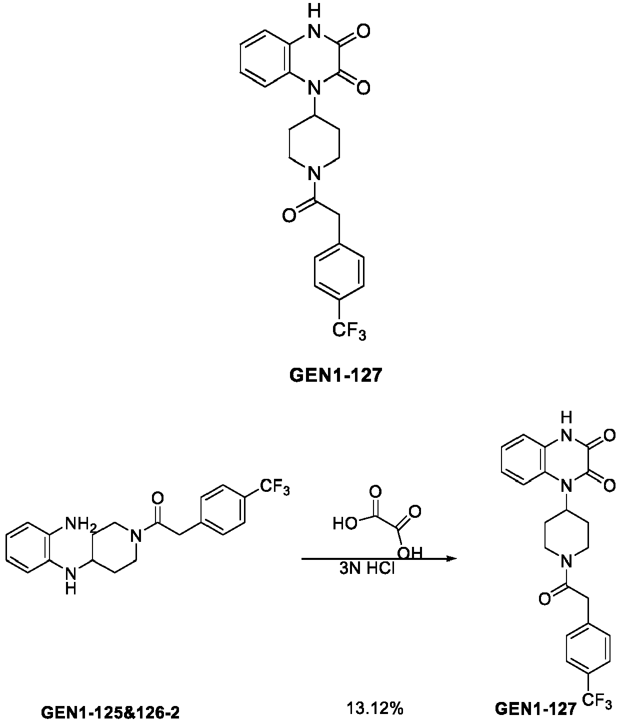

### Step 1. Synthesis of 1-(1-(2-(4-(trifluoromethyl)phenyl)acetyl)piperidin-4-yl)-1,4-dihydroquinoxaline-2,3-dione (GEN1-127)

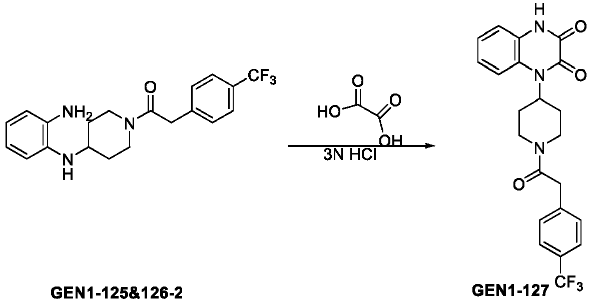

In an ice bath, oxalic acid (133.6 mg, 0.53 mmol) was added to a solution of 1-(4-(((2-aminophenyl)amino)piperidin-1-yl)-2-(4-(trifluoromethyl)phenyl)ethan-1-one (100 mg, 0.265 mmol, the synthesis steps could be found in GEN1-**125&126-2**) in 3N HCl (6 mL, 18 mmol). The resulting mixture was stirred at room temperature for 0.5 hours. The mixture was concentrated and dissolved in CH₃CN (10mL). The resulting mixture was stirred at 80°C for 3h. The mixture was cooled and filtered. The filtrate was concentrated, and the crude product was purified by reverse phase chromatography. Purification conditions: C18 silica gel column; mobile phase ACN: H₂O, gradient: 5% to 50 %; 45 minutes, monitoring wavelength: 214 nm. The desired compound ((15mg, yield 13.12%) was obtained as a white solid. ¹H NMR (400 MHz, DMSO-d6) δ 12.03 (s, 1H), 7.75 (d, J = 8.0 Hz, 2H), 7.65 (s, 1H), 7.56 (d, J = 8.0 Hz, 2H), 7.27-7.18 (m, 3H), 4.81 (s, 1H), 4.60 (d, J = 13.0 Hz, 1H), 4.17 (d, J = 13.6 Hz, 1H), 3.97 (s, 2H), 3.34-3.24 (m, 1H), 2.83 (t, J = 12.7 Hz, 1H), 2.54-2.36 (m, 2H), 1.76 (d, J = 12.4 Hz, 2H). MS (ESI): Rt = 1.42 min, m/z 432.1, [M+H]⁺; Purity: 100% @ 254 nm, 100% @ 214 nm.

### Example 103: 1-(1-(2-(4-(trifluoromethyl)phenyl)ethylthio)piperidin-4-yl)-1,3-dihydro-2H-benzo [d] imidazol-2-one (GEN1-128)

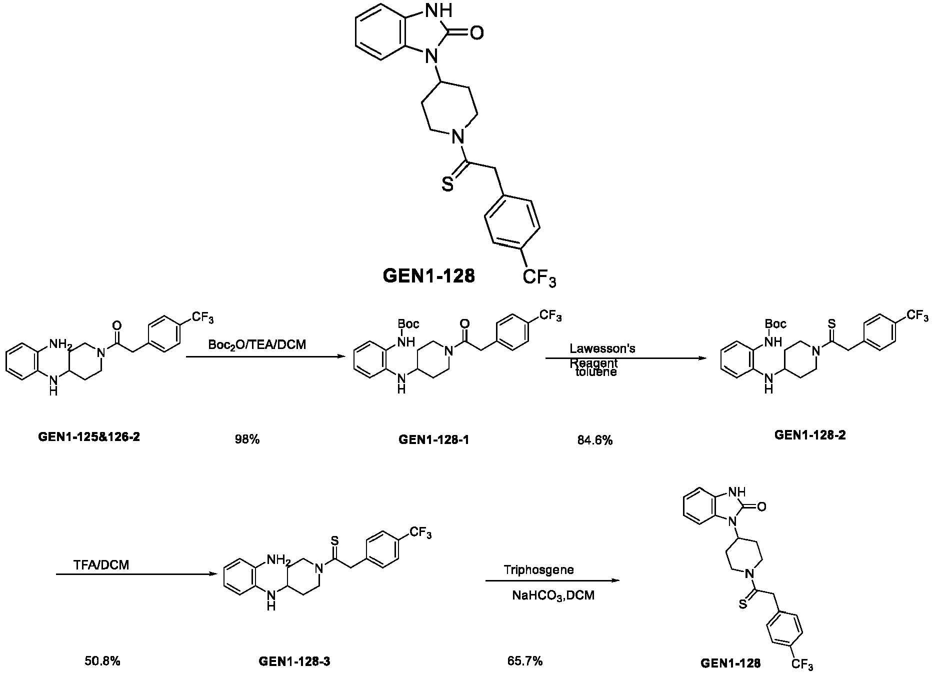

### Step 1. Synthesis of tert-butyl (2-((1-(2-(4-(4-(trifluoromethyl)phenyl)acetyl)piperidin-4-yl)amino)phenyl)carbamate (GEN1-128-1)

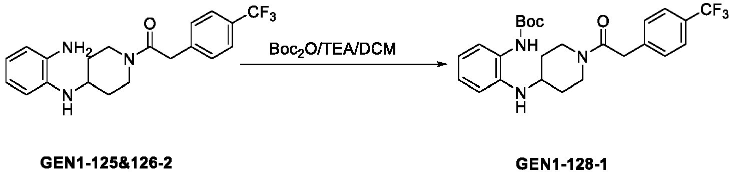

Boc2O (336.1 mg, 1.54 mmol) was added to a solution of 1-(4-((2-aminophenyl)amino)piperidin-1-yl)-2-(4-(trifluoromethyl)phenyl)ethan-1-one (484 mg, 1.28 mmol, the synthesis steps could be found in GEN1-125 & 126-2), TEA (389 mg, 3.84 mmol) in DCM (10 mL). The resulting mixture was stirred at room temperature for 16 hours. The reaction mixture was diluted with water (50 mL), and it was extracted with DCM (100 mL×3). The combined organic phase was washed with aqueous NaHCO₃ (50ml×3) and brine (50mL), then dried over anhydrous Na2SO4 and it was filtered. The filtrate was concentrated to obtain the desired compound (600 mg, yield 98%) as a pale yellow oil.

### Step 2. Synthesis of tert-butyl (2-((1-(2-(4-(4-(trifluoromethyl)phenyl)acetyl)piperidin-4-yl)amino)phenyl)carbamate (GEN1-128-2)

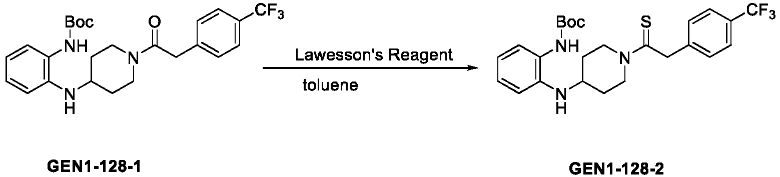

Under the protection of nitrogen, Lawson's reagent (169.5 mg, 0.419 mmol) was added to a solution of tert-butyl (2-((1-(2-(4-(tris(trifluoromethyl)phenyl)acetyl)piperidin-4-yl)amino)phenyl) carbamate (400 mg, 0.838 mmol) in toluene (10 mL). The resulting mixture was stirred at 80°C for 4 hours. The mixture was concentrated and purified by silica gel column chromatography (PE:EA=3:1) to obtain the desired compound (350 mg, yield 84.6%) as a pale yellow solid. MS (ESI): Rt = 1.78 min, m/z 494.1, [M+H]⁺; Purity: 100% @ 254 nm, 100% @ 214 nm.

### Step 3. Synthesis of 1-(4-((2-aminophenyl)amino)piperidin-1-yl)-2-(4-(trifluoromethyl)phenyl)ethane-1-thione (GEN1-128-3)

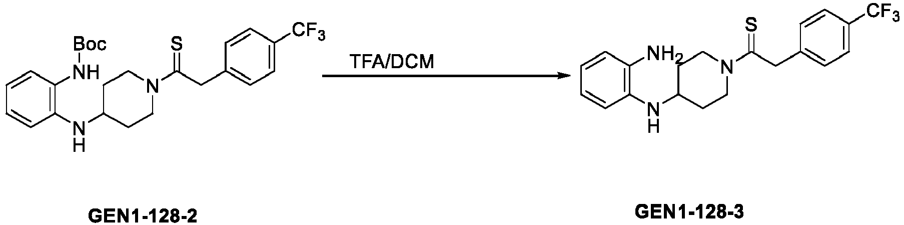

TFA (2 mL) was added to a solution of N-(2-nitrophenyl)piperidin-4-amine (350 mg, 0.89 mmol) in DCM(10mL). The mixture was stirred at room temperature for 0.5 hour. The obtained mixture was concentrated to obtain a crude product. The crude product was purified by reverse phase chromatography. Purification conditions: C18 silica gel column; mobile phase ACN: H₂O (0.1% NH₃H₂O), gradient: 5% to 50%; 45 minutes, monitoring wavelength: 214 nm. The desired compound (200 mg, yield 50.8%) was obtained as a pale yellow solid. MS (ESI): Rt = 1.66 min, m/z 394.3, [M+H]⁺; Purity: 88.81% @ 254 nm, 96.35% @ 214 nm.

### Step 4. Synthesis of 1-(1-(2-(4-(trifluoromethyl)phenyl)ethylthio)piperidin-4-yl)-1,3-dihydro-2H-benzo[d]imidazol-2-one (GEN1-128)

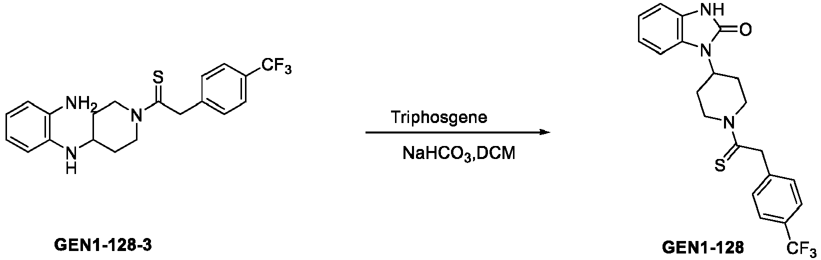

Triphosgene (124 mg, 0.420 mmol) was added to a suspension of 1-(4-(((2-aminophenyl)amino)piperidin-1-yl)-2-(4-(trifluoromethyl)phenyl)ethane-1-thione (100 mg, 0.254 mmol), NaHCO₃ (213.4 mg, 2.54 mmol) in DCM (10 mL). The resulting mixture was stirred at room temperature for 1 hour. The mixture was poured into water (20 mL), and it was extracted with DCM (20 mL×3). The combined organic layer was washed with brine (50 mL), then dried over Na₂SO₄ and it was concentrated. The crude product was purified by reverse phase chromatography. Purification conditions: C18 silica gel column; mobile phase ACN: H₂O, gradient: 5% to 50%; 45 minutes, monitoring wavelength: 214 nm. The desired compound (70 mg, yield 65.7%) was obtained as a yellow solid. ¹H NMR (400 MHz, Chloroform-d) δ 8.40 (s, 1H), 7.65 (d, J = 8.1 Hz, 2H), 7.59 (d, J = 8.1 Hz, 2H), 7.10-3.99 (m, 3H), 6.76 (d, J = 7.3 Hz, 1H), 5.94-5.86 (m, 1H), 4.67 - 4.56 (m, 1H), 4.56 - 4.39 (m, 2H), 4.37-4.28 (m, 1H), 3.38 - 3.20 (m, 1H), 3.16 - 3.01 (m, 1H), 2.49-2.35 (m, 1H), 2.05 - 1.70 (m, 3H). MS (ESI): Rt = 1.57 min, m/z 420.2, [M+H]⁺; Purity: 100% @ 254 nm.

### Example 104: 1-(4-(2-thiooxy-7-(trifluoromethyl)-2,3-dihydro-1H-benzo[d]imidazol-1-yl)piperidin-1-yl)-2-(4-(trifluoromethyl)phenyl ethanone (GEN1-132)

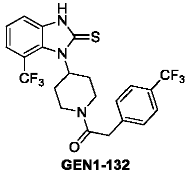

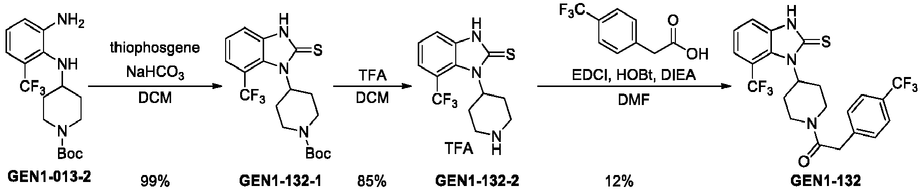

### Step 1. Tert-butyl 4-(2-thioxa-7-(trifluoromethyl)-2,3-dihydro-1H-benzo[d]imidazol-1-yl)piperidine-1-carboxylate (GEN1-132-1)

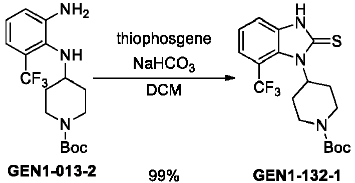

Tert-butyl 4-(((2-amino-6-(trifluoromethyl)phenyl)amino)piperidine-1-carboxylate (360 mg, 1.00 mmol, the synthesis steps could be found in the final report of GEN1-013-2) and NaHCO₃ (421 mg, 5.01 mmol) were dissolved in DCM (10 mL), and thiophosgene (116 mg, 1.01 mmol) was added dropwise in an ice bath. After the mixture was stirred in an ice bath for 1 hour, the reaction mixture was poured into water (50 mL), and extracted with DCM (30 mL×3). The combined organic layer was washed with brine (30 mL), dried over MgSO₄, filtered and it was concentrated to obtain the desired compound (400 mg, yield 99%) as a brown solid. MS (ESI): Rt = 1.75 min, *m*/*z* 346.3 [M+H-56]⁺; Purity: 100% @ 254 nm, 100% @ 214 nm.

### Step 2. 1-(piperidin-4-yl)-7-(trifluoromethyl)-1H-benzo[d]imidazol-2(3H)-thione 2,2,2-trifluoroacetate (GEN1-132-2)

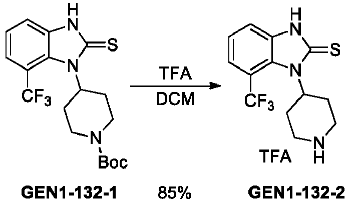

Tert-butyl 4-(2-thioxa-7-(trifluoromethyl)-2,3-dihydro-1H-benzo[d]imidazol-1-yl)piperidine-1-carboxylate (400 mg, 0.996 mmol) was dissolved in DCM (5 mL), and TFA (1 mL) was added at room temperature. The resulting mixture was stirred at room temperature overnight. The reaction mixture was concentrated to obtain the desired compound (400 mg, yield 85%) as a brown solid. MS (ESI) *m*/*z* 302.3 [M+H]⁺, Rt: 1.34 min, Purity: 88% @ 254 nm, 58% @ 214 nm.

### Step 3. Synthesis of 1-(4-(2-thiooxy-7-(trifluoromethyl)-2,3-dihydro-1H-benzo[d]imidazol-1-yl)piperidin-1-yl)-2-(4-(trifluoromethyl)phenyl ethanone (GEN1-132)

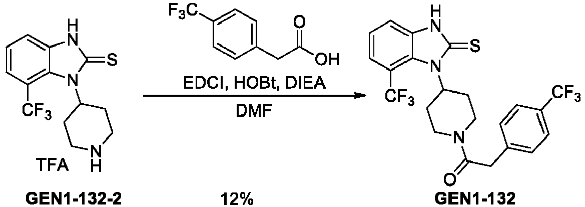

1-(piperidin-4-yl)-7-(trifluoromethyl)-1H-benzo[d]imidazol-2(3H)-thione 2,2,2-trifluoroacetate (200 mg, 0.482 mmol), 2-(4-(trifluoromethyl)phenyl)acetic acid (108 mg, 0.529 mmol), HOBT (98 mg, 0.73 mmol) and DIEA (311 mg, 2.41 mmol) were dissolved in DMF (3 mL), EDCI (139 mg, 0.725 mmol) was added in an ice bath. The resulting mixture was stirred at room temperature overnight. The reaction mixture was poured into water (50 mL), and it was extracted with EtOAc (30 mL×3). The combined organic layer was washed with brine (30 mL), dried over MgSO₄ and filtered. The filtrate was concentrated, and the crude product was purified by a C₁₈ column (CH₃CN: water = 5% to 60%) to obtain the desired compound (29.9 mg, yield 12%) as a pale yellow solid. ¹HNMR (400 MHz, CDCl₃): δ 7.61 (d, *J* = 7.6 Hz, 2H), 7.56 (d, *J* = 7.6 Hz, 1H), 7.44-7.42 (m, 3H), 7.29 (t, *J* = 7.6 Hz, 1H), 4.94-4.91 (m, 1H), 4.81-4.70 (m, 1H), 4.07-4.03 (m, 1H), 3.94-3.85 (m, 2H), 3.62-3.48 (m, 2H), 3.15-3.08 (m, 1H), 2.69-2.62 (m, 1H), 1.81-1.74 (m, 2H). MS (ESI): Rt = 3.675 min, *m*/*z* 488.1 [M+H]⁺; Purity: 97.92% @ 254 nm, 95.01% @ 214 nm.

### Example 105: 1-(4-(3-thiooxa3,4-dihydroquinoxaline-1(2H)-yl)piperidin-1-yl)-2-(4-(trifluoromethyl)phenyl ethanone (GEN1-133)

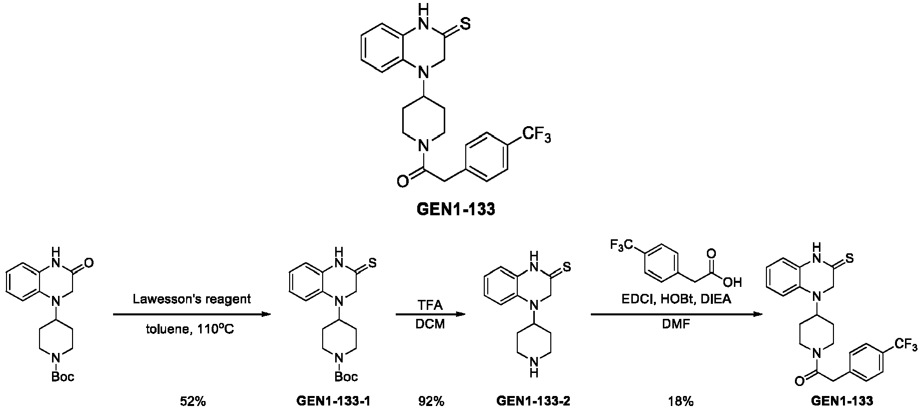

### Step 1. Tert-butyl 4-(3-thio-3,4-dihydroquinoxaline-1(2H)-yl)piperidin-1-carboxylate (GEN1-133-1)

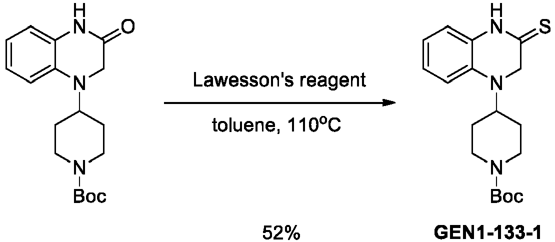

Tert-butyl 4-(3-oxo-3,4-dihydroquinoxaline-1(2H)-yl)piperidin-1-carboxylate (300 mg, 0.905 mmol, the synthesis steps could be found in the final report of GEN1-052-2) and Lawesson's reagent (220 mg, 0.544 mmol) were dissolved in toluene (5 mL) and it was stired at 110°C for 2 hours. The reaction mixture was concentrated, and the residue was purified by column chromatography on silica gel (petroleum ether: EtOAc = 10:1 to 5:1) to obtain the desired compound (155 mg, yield 52%) as a yellow solid. ¹HNMR (400 MHz, CDCl₃): δ 9.57 (s, 1H), 7.09-7.04 (m, 1H), 6.83-6.77 (m, 3H), 4.39-4.18 (m, 2H), 4.06 (s, 2H), 3.66-3.57 (m, 1H), 2.84-2.75 (m, 2H), 1.79-1.77 (m, 2H), 1.73-1.66 (m, 2H), 1.48 (s, 9H). MS (ESI): Rt = 1.66 min, *m*/*z* 292.3 [M+H-56]⁺; Purity: 100% @ 254 nm, 100% @ 214 nm.

### Step 2. 4-(piperidin-4-yl)-3,4-dihydroquinoxaline-2(1H)-thione (GEN1-133-2)

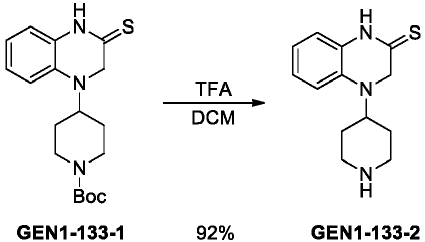

TFA (0.3 mL) was added to a solution of tert-butyl 4-(3-thio-3,4-dihydroquinoxaline-1(2H)-yl)piperidin-1-carboxylate (70 mg, 0.20 mmol) in DCM (1.5 mL). The reaction mixture was stirred at room temperature for 1 hour. Then the mixture was poured into Na₂CO₃ (aqueous solution, 15%, 30 mL), and it was extracted with DCM (20 mL×3). The combined organic layer was dried over MgSO₄, filtered and concentrated to obtain the desired compound (46 mg, yield 92%) as a yellow solid. MS (ESI): Rt = 1.11 min, *m*/*z* 248.2 [M+H]⁺, Purity: 100% @ 254 nm, 100% @ 214 nm.

### Step 3. 1-(4-(3-thiooxa3,4-dihydroquinoxaline-1(2H)-yl)piperidin-1-yl)-2-(4-(trifluoromethyl)phenyl ethanone (GEN1-133)

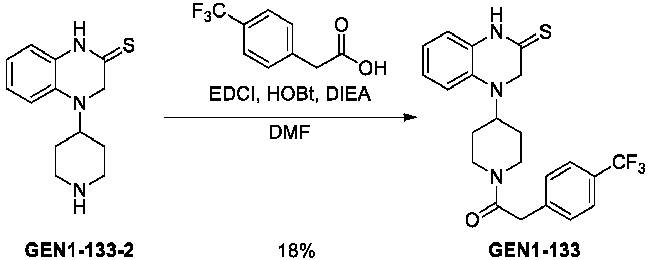

In an ice bath, EDCI (57 mg, 0.30 mmol) was added to a solution of 4-(piperidin-4-yl)-3,4-dihydroquinoxaline-2(1H)-thione (46 mg, 0.185 mmol), HOBT (40 mg, 0.30 mmol) and DIEA (128 mg, 0.990 mmol) in DMF (2 mL). The resulting mixture was stirred at room temperature overnight. The reaction mixture was poured into water (50 mL), and it was extracted with EtOAc (30 mL×3). The combined organic layer was washed with brine (30 mL), dried over MgSO₄, filtered and it was concentrated. The crude product was passed through a C₁₈ column (CH₃CN: water = 5% to 60%) to obtain the desired compound (14.0 mg, yield 18%) as a yellow solid. ¹HNMR (400 MHz, DMSO-*d*₆): δ 12.43 (s, 1H), 7.68 (d, *J* = 8.0 Hz, 2H), 7.47 (d, *J* = 8.0 Hz, 2H), 7.03-6.96 (m, 3H), 6.74 (t, *J* = 7.2 Hz, 1H), 4.53-4.50 (m, 1H), 4.08-4.05 (m, 1H), 3.92-3.83 (m, 5H), 3.20-3.13 (m, 1H), 2.72-2.66 (m, 1H), 1.69-1.58 (m, 3H), 1.54-1.44 (m, 1H). MS (ESI): Rt = 4.120 min, *m*/*z* 434.1 [M+H]⁺, Purity: 97.96% @ 254 nm, 97.36% @ 214 nm.

### Example 106: 8-(trifluoromethyl)-4-(1-(2-(4-(trifluoromethyl)phenyl)acetyl)piperidin-4-yl)-3,4-dihydroquinoxaline-2(1H)-one (GEN1-134)

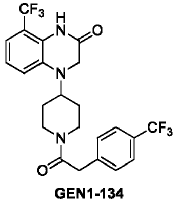

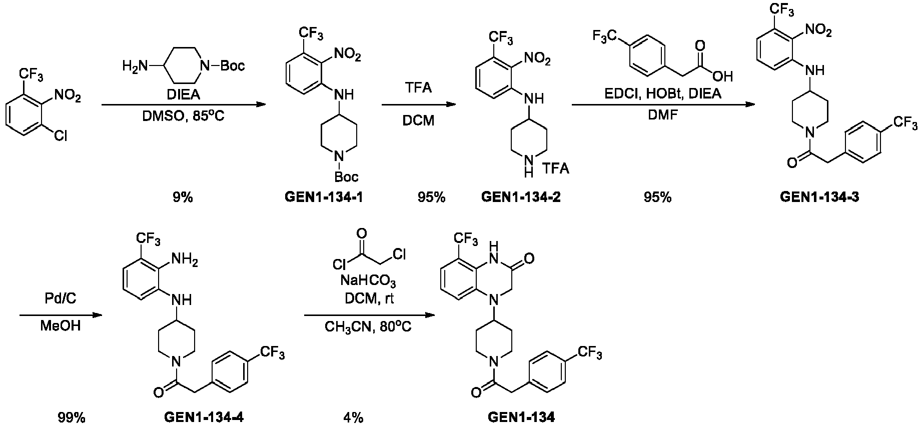

### Step 1. Tert-butyl 4-((2-nitro-3-(trifluoromethyl)phenyl)amino)piperidin-1-carboxylate (GEN1-134-1)

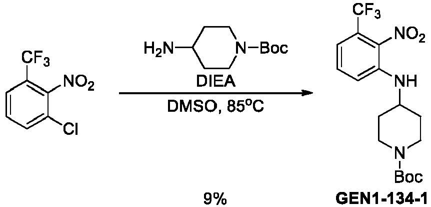

1-chloro-2-nitro-3-(trifluoromethyl)benzene (800 mg, 3.55 mmol), tert-butyl 4-aminopiperidine-1-carboxylate (1.07 g, 5.32 mmol) and DIEA (688 mg, 5.32 mmol) were dissolved in DMSO (7 mL) and the mixture was stirred at 90°C overnight. After the mixture was cooled to room temperature, the reaction mixture was poured into water (50 mL), and extracted with EtOAc (30 mL×3). The combined organic layer was washed with water (30 mL), brine (30 mL), dried over MgSO₄ and filtered. The filtrate was concentrated and the crude product was purified by column chromatography on silica gel (petroleum ether: EtOAc = 5:1 to 3:1) to obtain the desired compound (138 mg, yield 9%) as a yellow solid. ¹H NMR (400 MHz, CDCl₃): δ 7.42 (t, *J* = 8.0 Hz, 1H), 7.05-7.03 (m, 2H), 5.79 (d, *J* = 6.8 Hz, 1H), 4.03-4.01 (m, 2H), 3.61-3.52 (m, 1H), 2.99 (t, *J* = 11.2 Hz, 2H), 2.02-2.00 (m, 2H), 1.50-1.41 (m, 11H).

### Step 2. N-(2-nitro-3-(trifluoromethyl)phenyl)piperidin-4-amine 2,2,2-trifluoroacetate (GEN1-134-2)

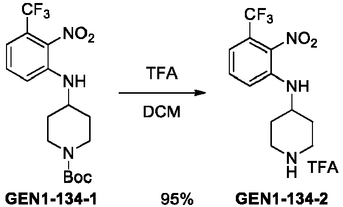

TFA (1 mL) was added to a solution of tert-butyl 4-((2-nitro-3-(trifluoromethyl)phenyl)amino)piperidin-1-carboxylate (130 mg, 0.334 mmol) in DCM (5mL). The reaction mixture was stirred at room temperature for 2 hours. Then the mixture was concentrated to obtain the desired compound (134 mg, yield 95%) as a brown oil. MS (ESI): Rt = 1.39 min, *m*/*z* 290.3 [M+H]⁺, Purity: 100% @ 254 nm, 95% @ 214 nm.

### Step 3. 1-(4-((2-nitro-3-(trifluoromethyl)phenyl)amino)piperidin-1-yl)-2-(4-(trifluoromethyl)phenyl)ethenone (GEN1-134-3)

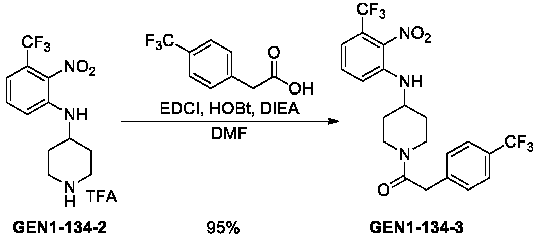

In an ice bath, EDCI (96mg, 0.50 mmol) was added to a solution of N-(2-nitro-3-(trifluoromethyl)phenyl)piperidin-4-amine 2,2,2-trifluoroacetate (134 mg, 0.332 mmol), 2-(4-(trifluoromethyl)phenyl)acetic acid (82 mg, 0.40 mmol), HOBT (69 mg, 0.51 mmol) and DIEA (216 mg, 1.67 mmol) in DMF (3mL). The resulting mixture was stirred at room temperature overnight. The reaction mixture was poured into water (50 mL), and extracted with EtOAc (20 mL×3). The combined organic layer was washed with brine (20 mL), dried over MgSO₄, filtered and it was concentrated to obtain the desired compound (150 mg, yield 95%) as a yellow solid. MS (ESI): Rt = 1.74 min, *m*/*z* 476.5 [M+H]⁺, Purity: 100% @ 254 nm, 100% @ 214 nm.

### Step 4. 1-(4-((2-amino-3-(trifluoromethyl)phenyl)amino)piperidin-1-yl)-2-(4-(trifluoromethyl)phenyl)ethenone (GEN1-134-4)

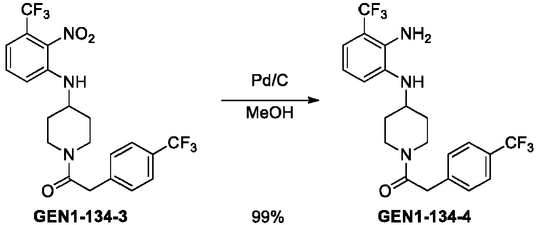

1-(4-((2-nitro-3-(trifluoromethyl)phenyl)amino)piperidin-1-yl)-2-(4-(trifluoromethyl)phenyl)ethenone (150 mg, 0.316 mmol) was added to a solution of Pd/C (10% purity, 34 mg) in MeOH (5 mL). The resulting mixture was stirred under H₂ atmosphere for 2 hours. The reaction mixture was filtered, and the filtrate was concentrated to obtain the desired compound (140 mg, yield 99%) as a colorless oil. MS (ESI): Rt = 1.70 min, *m*/*z* 446.5 [M+H]⁺, Purity: 100% @ 254 nm, 100% @ 214 nm.

### Step 5. 8-(trifluoromethyl)-4-(1-(2-(4-(trifluoromethyl)phenyl)acetyl)piperidin-4-yl)-3,4-dihydroquinoxaline-2(1H)-one (GEN1-134)

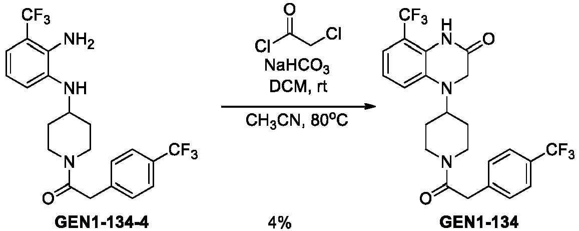

In an ice bath, 2-chloroacetyl chloride (30mg, 0.27mmol) was added to a solution of 1-(4-((2-amino-3-(trifluoromethyl)phenyl)amino)piperidin-1-yl)-2-(4-(trifluoromethyl)phenyl)ethenone (100 mg, 0.225 mmol), NaHCO₃ (38 mg, 0.45 mmol) in DCM (5mL). The resulting mixture was stirred in an ice bath for 1 hour. The mixture was concentrated and then dissolved in CH₃CN (5 mL). Then KI (4 mg, 0.02 mmol) was added to the mixture, and the mixture was stirred at 85°C for 2 hours. The reaction mixture was filtered and the filtrate was concentrated. The crude product was purified by a C₁₈ column (CH₃CN: water = 5% to 65%) to obtain the desired compound (4.3 mg, yield 4%) as a white solid. ∘ ¹H NMR (400 MHz, DMSO-*d*₆): δ 9.62 (s, 1H), 7.68 (d, *J* = 8.0 Hz, 2H), 7.47 (d, *J* = 7.6 Hz, 2H), 7.29 (d, *J* = 7.6 Hz, 1H), 7.13-7.05 (m, 2H), 4.54-4.51 (m, 1H), 4.10-4.06 (m, 1H), 3.98-3.82 (m, 3H), 3.62-3.54 (m, 2H), 3.22-3.16 (m, 1H), 2.76-2.70 (m, 1H), 1.72-1.49 (m, 4H). MS (ESI): Rt = 4.321 min, *m*/*z* 486.2 [M+H]⁺, Purity: 91.41% @ 254 nm, 98.35% @ 214 nm.

### Example 107: 7-(trifluoromethyl)-4-(1-(2-(4-(trifluoromethyl)phenyl)acetyl)piperidin-4-yl)-3,4-dihydroquinoxaline-2(1H)-one (GEN1-135)

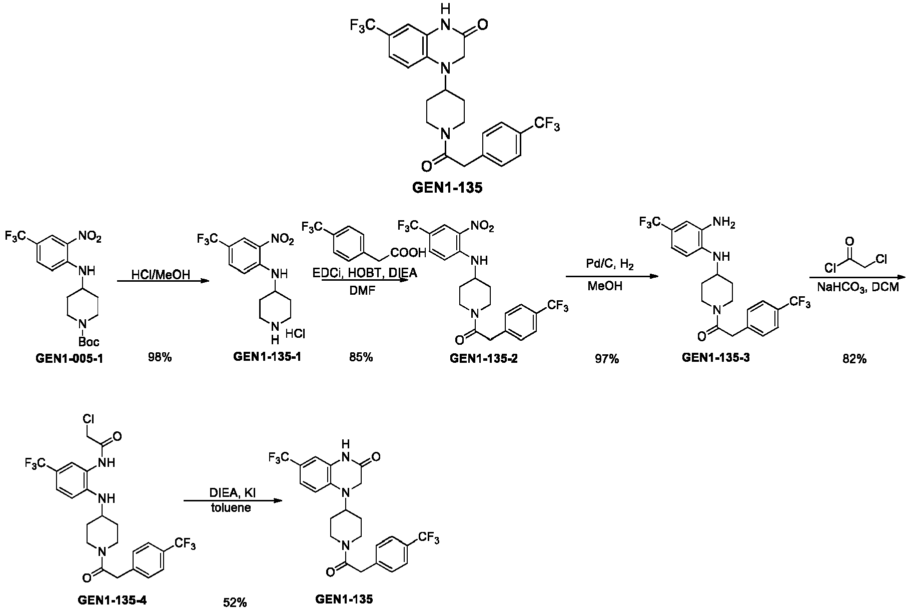

### Step 1. N-(2-nitro-4-(trifluoromethyl)phenyl)piperidin-4-amine hydrochloride (GEN1-135-1)

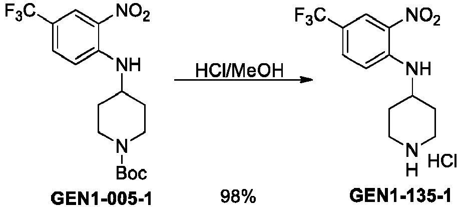

Tert-butyl 4-(((2-nitro-4-(trifluoromethyl)phenyl)amino)piperidin-1-carboxylate (500 mg, 1.28 mmol) was dissolved in HCl/MeOH (10 mL, 4 M) and the mixture was stirred at room temperature for 2.5 hours. The resulting mixture was concentrated to obtain the desired compound (408 mg, yield 98%) as a yellow solid. LCMS (ESI): Rt = 1.46 min, *m*/*z* 290.3 [M+H]⁺; Purity: 100% @ 254 nm, 100% @ 214 nm.

### Step 2. 1-(4-((2-nitro-4-(trifluoromethyl)phenyl)amino)piperidin-1-yl)-2-(4-(trifluoromethyl)phenyl)ethenone (GEN1-135-2)

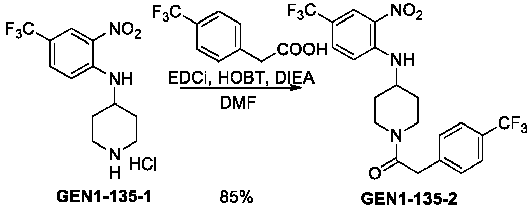

2-(4-(trifluoromethyl)phenyl)acetic acid (307 mg, 1.50 mmol) was added to a solution of N-(2-nitro-4-(trifluoromethyl)phenyl)piperidin-4-amine hydrochloride (408 mg, 1.25 mmol), DIEA (809 mg, 6.26 mmol), EDCI (360 mg, 1.88 mmol), HOBT (254 mg , 1.88 mmol) in DMF (5 mL). The resulting mixture was stirred at room temperature overnight. Then the mixture was purified by C₁₈ (60-80% B; A: H₂O B: CH₃CN) to obtain the desired compound (506 mg, yield 85%) as a yellow oil. LCMS (ESI): Rt = 1.76 min, *m*/*z* 476.5 [M+H]⁺; Purity: 100% @ 254 nm, 100% @ 214 nm.

### Step 3. 1-(4-((2-amino-4-(trifluoromethyl)phenyl)amino)piperidin-1-yl)-2-(4-(trifluoromethyl)phenyl)ethanone (GEN1-135-3)

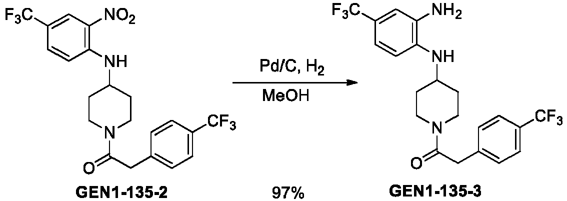

1-(4-((2-nitro-4-(trifluoromethyl)phenyl)amino)piperidin-1-yl)-2-(4-(trifluoromethyl)phenyl)ethenone (506 mg, 1.06 mmol) was added to a solution of Pd/C (10% purity, 113 mg) in MeOH (25 mL). The mixture was stirred at room temperature under a hydrogen atmosphere for 2 hours. The resulting mixture was filtered, and the filtrate was concentrated to obtain the desired compound (461 mg, yield 97%) as a gray solid. LCMS (ESI): Rt =1.65 min, *m*/*z* 446.5 [M+H]⁺; Purity: 100% @ 254 nm, 100% @ 214 nm.

### Step 4. 2-chloro-N-(5-(trifluoromethyl)-2-((1-(2-(4-(trifluoromethyl)phenyl)acetyl)piperidin-4-yl)amino)phenyl)acetamide (GEN1-135-4)

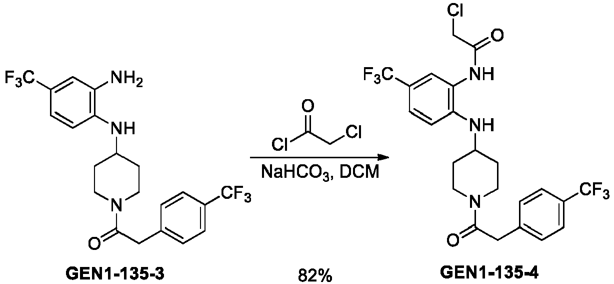

In an ice bath, 2-chloroacetyl chloride (187 mg, 1.66 mmol) was added dropwise to a solution of 1-(4-((2-amino-4-(trifluoromethyl)phenyl)amino)piperidin-1-yl)-2-(4-(trifluoromethyl)phenyl)ethenone (461 mg, 1.04 mmol), NaHCO₃ (435 mg, 5.18 mmol) in DCM (10 mL). After the addition, the mixture was stirred at 0°C for half an hour. The resulting mixture was filtered. The filtrate was then concentrated, and the residue was purified by C18 (55-75% B; A: H₂O B: CH₃CN) to obtain the desired compound (445 mg, yield 82%) as a white solid. LCMS (ESI): Rt = 1.64 min, *m*/*z* 522.5 [M+H]⁺;Purity: 100% @ 254 nm, 100% @ 214 nm.

### Step 5. 7-(trifluoromethyl)-4-(1-(2-(4-(trifluoromethyl)phenyl)acetyl)piperidin-4-yl)-3,4-dihydroquinoxaline-2(1H)-one (GEN1-135)

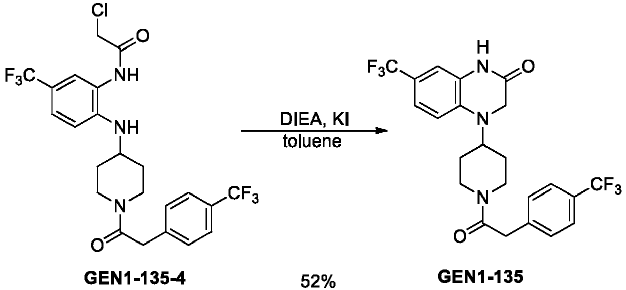

KI (10 mg, 60.2 umol) was added to a solution of 2-chloro-N-(5-(trifluoromethyl)-2-((1-(2-(4-(trifluoromethyl)phenyl)acetyl)piperidin-4-yl)amino)phenyl)acetamide (200 mg, 383 umol), DIEA (248 mg, 1.92 mmol) in toluene (12 mL). The mixture was stirred at 105°C under a nitrogen atmosphere for 2 days. The obtained mixture was concentrated and the residue was purified. HPLC (preparation 5 Xbridge C₁₈ 5 um 19 * 150 mm, 35-80% B; A: H₂O (0.1% NH₄HCO₃), B: CH₃CN; 214 nm; flow rate: 15 mL/min). The desired compound (99 mg, yield 52%) was obtained as a white solid. ¹H NMR (400 MHz, DMSO-*d*₆): δ 10.63 (s, 1H), 7.68 (d, *J* = 8.0 Hz, 2H), 7.47 (d, *J* = 8.0 Hz, 2H), 7.20-7.18 (m, 1H), 7.06 (d, *J* = 2.0 Hz, 1H), 7.03 (d, *J* = 8.8 Hz, 1H), 4.53 (d, *J* = 12.4 Hz, 1H), 4.08 (d, *J* = 12.4 Hz, 1H), 3.99-3.94 (m, 1H), 3.93-3.82 (m, 2H), 3.73-3.63 (m, 2H), 3.22-3.16 (m, 1H), 2.75-2.70 (m, 1H), 1.71-1.52 (m, 4H). LCMS (ESI): Rt = 9.607 min, *m*/*z* 486.2 [M+H]⁺; Purity: 95.39% @ 254 nm, 97.55% @ 214 nm.

### Example 108: 2-(quinolin-6-yl)-1-(4-(2-thio-2,3-dihydro-1H-benzo(d)imidazol-1-yl)piperidin-1-yl)ethan-1-one (GEN1-137)

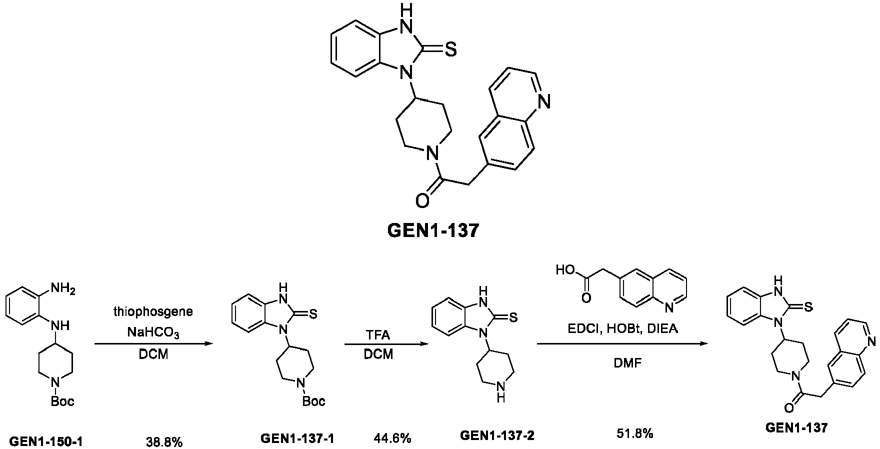

### Step 1. Synthesis of tert-butyl 4-(2-thio-2,3-dihydro-1H-benzo[d]imidazol-1-yl)piperidin-1-carboxylate (GEN1-137-1)

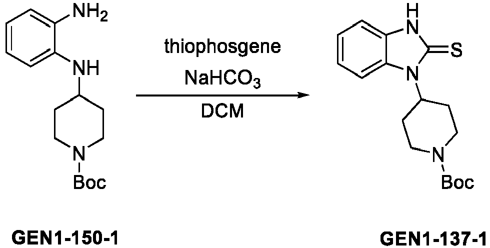

Thiophosgene (170.4 mg, 1.482 mmol) was added to a suspension of tert-butyl 4-((2-aminophenyl)amino)piperidin-1-carboxylate (360 mg, 1.235 mmol, the synthesis steps could be found in GEN1-150-1), NaHCO₃ (1.037 g, 12.35 mmol) in DCM (15 mL). The resulting mixture was stirred at room temperature for 1 hour. The mixture was poured into water (20 mL), and extracted with DCM (20 mL×3). The combined organic layer was washed with brine (50 mL), dried over Na₂SO₄ and it was concentrated to obtain a crude product. The crude product was purified by silica gel column chromatography (PE:EA=3:1) to obtain the desired compound (160 mg, yield 38.8%) as a yellow solid. MS (ESI): Rt = 1.55 min, m/z 356.0, [M+H]⁺; Purity: 100% @ 254 nm, 100% @ 214 nm.

### Step 2. Synthesis of 1-(piperidin-4-yl)-1,3-dihydro-2H-benzo[d]imidazol-2-thione (GEN1-137-2)

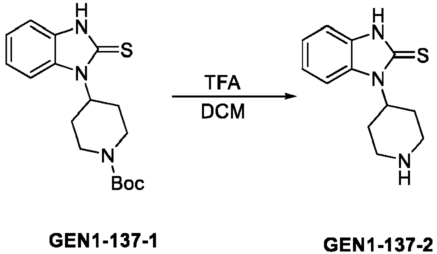

TFA (2 mL) was added to a solution of tert-butyl 4-(2-thio-2, 3-dihydro-1H-benzo[d]imidazol-1-yl)piperidin-1-carboxylate (160 mg, 0.48 mmol) in DCM (10 mL). The resulting mixture was stirred at room temperature for 0.5 hour. The resulting mixture was stirred at room temperature for 0.5 hour. The resulting mixture was concentrated to obtain a crude product. The crude product was purified by reverse phase chromatography. Purification conditions: C18 silica gel column; mobile phase ACN: H₂O (0.1% NH₃H₂O), gradient: 5% to 50%; 45 minutes, monitoring wavelength: 214 nm. The desired compound (50 mg, yield 44.6%) was obtained as a white solid. MS (ESI): Rt = 0.31 min, m/z 234.1, [M+H]⁺;

### Step 3. Synthesis of 2-(quinolin-6-yl)-1-(4-(2-thio-2,3-dihydro-1H-benzo(d)imidazol-1-yl)piperidin-1-yl)ethan-1-one (GEN1-137)

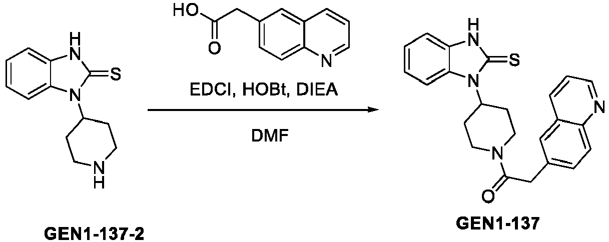

EDCI (52.33 mg, 0.273 mmol) was added to a solution of 1-(piperidin-4-yl)-1,3-dihydro-2H-benzo[d]imidazol-2-thione (42.5 mg, 0.182 mmol), 2-(quinolin-6-yl)acetic acid (51.1 mg, 0.273 mmol), HOBT (36.89 mg, 0.273 mmol) and DIEA (47 mg, 0.364 mmol) in DMF (1 mL). The resulting mixture was stirred at room temperature for 1 h. The reaction mixture was diluted with water (30 mL), and extracted with EtOAc (20 mL×3). The combined organic phase was washed with brine (50 mL), dried over anhydrous Na₂SO₄ and filtered. The filtrate was concentrated to obtain a crude product. The crude product was purified by reverse phase chromatography. Purification conditions: C18 silica gel column; mobile phase ACN: H₂O, gradient: 5% to 50%; 45 minutes, monitoring wavelength: 214 nm. The desired compound (38 mg, yield 51.8%) was obtained as a white solid. ¹H NMR (400 MHz, Chloroform-d) δ 10.08 (s, 1H), 8.92 (dd, J = 4.3, 1.6 Hz, 1H), 8.16 (dd, J = 8.8, 3.1 Hz, 2H), 7.81 (s, 1H), 7.75 (dd, J = 8.7, 2.0 Hz, 1H), 7.43 (dd, J = 8.3, 4.2 Hz, 1H), 7.17 - 7.08 (m, 2H), 6.97 - 6.83 (m, 2H), 5.50-5.36 (m, 1H), 4.95 (d, J = 13.5 Hz, 1H), 4.16 (d, J = 13.9 Hz, 1H), 4.04 (s, 2H), 3.36-3.24 (m, 1H), 2.87-2.75 (m, 1H), 2.32-2.16 (m, 1H), 1.79 (s, 3H). MS (ESI): Rt = 1.30 min, m/z 403.0, [M + H]⁺; Purity: 100% @ 254 nm, 100% @ 214 nm.

### Example 109: 1-(4-(2-bromo-1H-indol-3-yl)piperidin-1-yl)-2-(4-(trifluoromethyl)phenyl)ethenone (GEN1-139)

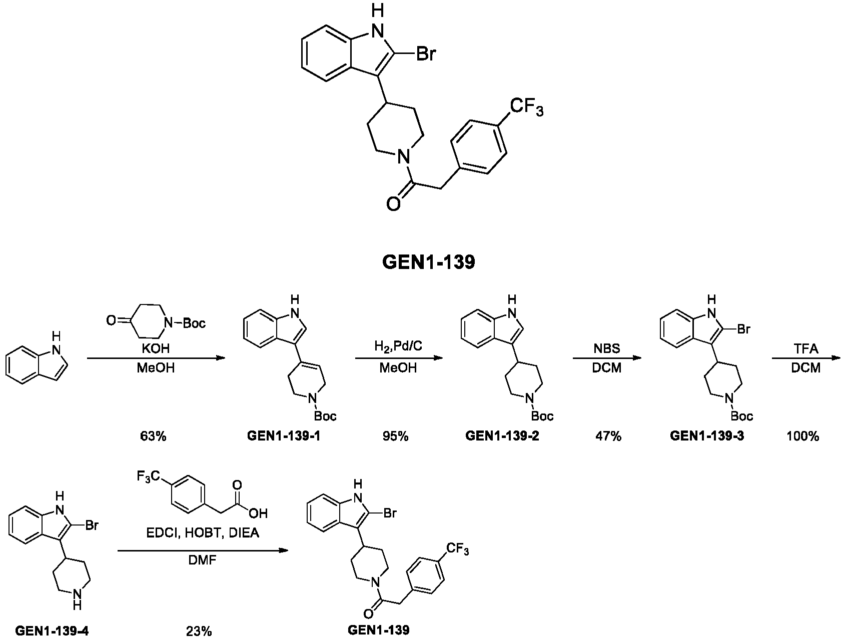

### Step 1. Tert-butyl 4-(1H-indol-3-yl)-5,6-dihydropyridine-1(2H)-carboxylate (GEN1-139-1)

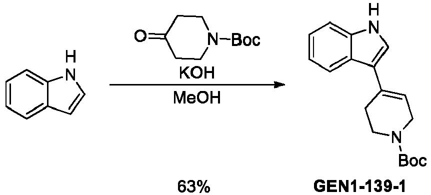

KOH (563 mg, 10.0 mmol) was added to a solution of tert-butyl 4-oxocyclopiperidine-1-carboxylate (1.0 g, 5.0 mmol) and indole (588 mg, 5.02 mmol) in methanol (20 mL). The mixture was stirred at 70°C for 5 hours. The reaction mixture was concentrated, and the residue was diluted with water (20 mL). After concentration, HCl was added to acidify the aqueous solution to pH=6-7. The aqueous solution was extracted with EtOAc (20 mL×3). The combined organic phase was washed with brine (20 mL), dried over anhydrous Na₂SO₄ and it was filtered. The filtrate was concentrated, and the residue was purified by silica gel chromatography (PE: EtOAc = 10:1) to obtain the desired compound (950 mg, yield 63%) as a yellow solid. ¹H NMR (400 MHz, CDCl₃): δ 8.17 (br s, 1H), 7.88 (d, *J=* 8.0 Hz, 1H), 7.38 (d, *J=* 8.0 Hz, 1H), 7.24-7.14 (m, 3H), 6.17 (s, 1H), 4.14-4.13 (m, 2H), 3.70-3.67 (m, 2H), 2.62-2.53 (m, 2H), 1.50 (s, 9H). MS (ESI): Rt = 1.69 min, *m*/*z* 297.4 [M-H]⁻; Purity: 100% @ 254 nm, 100% @ 214 nm.

### Step 2. Tert-butyl 4-(1H-indol-3-yl)piperidin-1-carboxylate (GEN1-139-2)

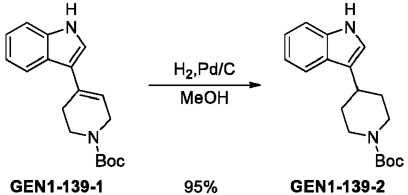

Tert-butyl 4-(1H-indol-3-yl)-5,6-dihydropyridine-1(2H)-carboxylate (950 mg, 3.18 mmol) was added to a solution of Pd/C (10%, 200 mg) in tetrahydrofuran (5 mL) and MeOH (5 mL) and the mixture was hydrogenated under H₂ atmosphere at 50°C for 2 hours. The mixture was cooled to room temperature and filtered through kieselguhr. The filtrate was concentrated to obtain the desired compound (1.0 g, yield 95%) as a white solid. ¹HNMR (400 MHz, CDCl₃): δ 8.09 (br s, 1H), 7.63 (d, *J=* 7.6 Hz, 1H), 7.36 (d, *J=* 8.0 Hz, 1H), 7.21-7.17 (m, 1H), 7.12-7.08 (m, 1H), 6.95 (d, *J* = 2.0 Hz, 1H), 4.22 (br s, 2H), 3.01 -2.87 (m, 3H), 2.05-2.02 (m, 2H), 1.71-1.62 (m, 2H), 1.49 (s, 9H). MS (ESI): Rt = 1.75 min, *m*/*z* 299.4 [M-H]⁻; Purity: 91% @ 254 nm, 70% @ 214 nm.

### Step 3. Tert-butyl 4-(2-bromo-1H-indol-3-yl)piperidin-1-carboxylate (GEN1-139-3)

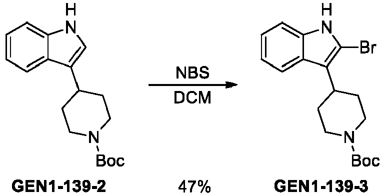

NBS (86 mg, 0.48 mmol) was added in portions to a solution of tert-butyl 4-(1H-indol-3-yl)piperidin-1-carboxylate (160 mg, 0.480 mmol) in DCM (10 mL) at 0 °C. After the addition, the mixture was stirred at 0°C for 1 hour. The reaction mixture was diluted with water (10 mL), and extracted with DCM (10 mL×2). The combined organic phase was dried over anhydrous Na₂SO₄ and filtered. The filtrate was concentrated, and the residue was purified by silica gel chromatography (PE : EtOAc = 6:1) to obtain the desired compound (110 mg, yield 47%) as a yellow solid. ¹H NMR (400 MHz, CDCl₃): δ 8.05 (s, 1H), 7.64 (d, *J =* 8.0 Hz, 1H), 7.29 (d, *J* = 7.6 Hz, 1H), 7.17-7.14 (m, 1H), 7.10-7.06 (m, 1H), 4.34-4.25 (m, 2H), 3.00-2.93 (m, 1H), 2.87-2.80 (m, 2H), 2.19-2.08 (m, 2H), 1.76-1.73 (m, 2H), 1.52 (s, 9H).

### Step 4. 2-bromo-3-(piperidin-4-yl)-1H-indole (GEN1-139-4)

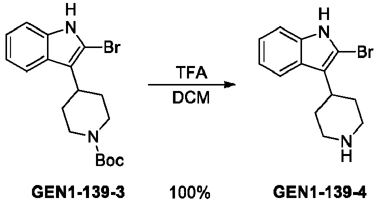

TFA (800 mg) was added dropwise to a solution of tert-butyl 4-(2-bromo-1H-indol-3-yl)piperidin-1-carboxylate (110 mg, 0.230 mmol) in DCM (3 mL) at room temperature. After it was stirred at room temperature for 30 minutes, the reaction mixture was concentrated and the residue was diluted with water (5 mL). The aqueous solution was basified with saturated Na₂CO₃ solution (5 mL), and extracted with DCM (10 mL×3). The combined organic phase was washed with brine (15 mL), dried over anhydrous sodium sulfate and it was concentrated to obtain the desired compound (80 mg crude product, yield 100%) as a yellow solid. MS (ESI): Rt = 1.28 min, *m*/*z* 279.3 [M-H]⁺; Purity: 65% @ 254 nm, 81% @ 214 nm.

### Step 5. 1-(4-(2-bromo-1H-indol-3-yl)piperidin-1-yl)-2-(4-(trifluoromethyl)phenyl)ethenone (GEN1-139)

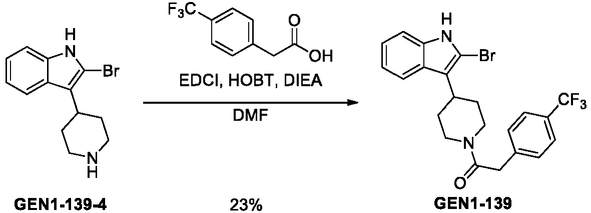

EDCI (89 mg, 0.47 mmol) and HOBT (63 mg, 0.47 mmol) were added to a solution of 2-bromo-3-(4-piperidinyl)-1H-indole (80 mg, 0.23 mmol), 2-[4-(trifluoromethyl)phenyl]acetic acid (52 mg, 0.26 mmol), DIEA (150 mg, 1.16 mmol) in DMF (3 mL). The mixture was stirred at room temperature overnight. The reaction mixture was diluted with water (15 mL), and extracted with EtOAc (10 mL×2). The combined organic phase was washed with brine (5 mL), dried over anhydrous Na₂SO₄ and filtered. The filtrate was concentrated and the residue was purified by preparative HPLC [gilson-2, Xbridge C₁₈, 5 um, 19×150 mm, 30~70%B, A: H₂O(0.1%NH₄HCO₃), B: ACN, flow rate: 15ml/min, UV = 214 nm] to obtain the desired compound (25 mg, yield 23%) as a yellow solid. ¹H NMR (400 MHz, DMSO-*d*₆): δ 11.60 (s, 1H), 7.72 (d, *J=* 8.0 Hz, 2H), 7.55 (d, *J=* 8.0 Hz, 2H), 7.37 (d, *J=* 8.0 Hz, 1H), 7.26 (d, *J=* 8.0 Hz, 1H), 7.06 (t, *J=* 8.0 Hz, 1H), 6.94 (t, *J=* 8.0 Hz, 1H), 4.57 (d, *J=* 12.0 Hz, 1H), 4.10 (d, *J* = 14.0 Hz, 1H), 3.99 (d, *J=* 14.8 Hz, 1H), 3.85 (d, *J=* 15.2 Hz, 1H), 3.16 (t, *J=* 11.6 Hz, 1H), 3.05-2.98 (m, 1H), 2.67 (t, *J=* 12.4 Hz, 1H), 1.88-1.71 (m, 2H), 1.65-1.56 (m, 2H). MS (ESI): Rt = 7.005 min, *m*/*z* 465.1 [M+H]⁺; Purity: 96.54% @ 254 nm, 98.80% @ 214 nm.

### Example 110: 1-(1-(2-(quinoxalin-6-yl)acetyl)piperidin-4-yl)-7-(trifluoromethyl)-1,3-dihydro-2H-benzo[d]imidazol-2-one (GEN1-140)

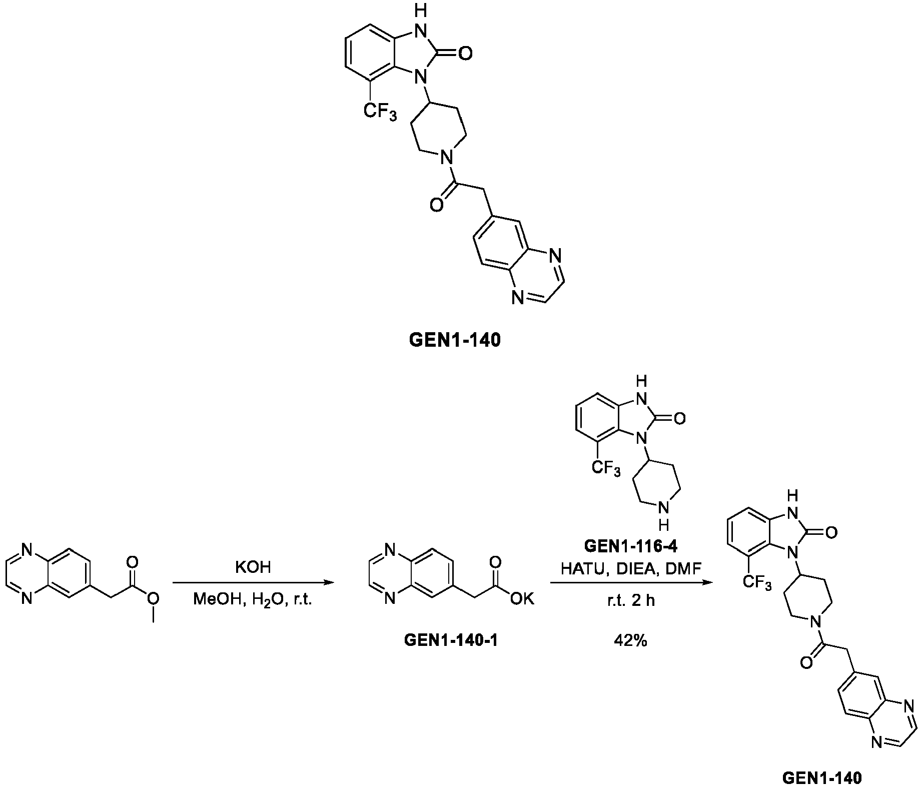

### Step 1. Potassium 2-(quinoxaline-6-yl)acetate (GEN1-140-1)

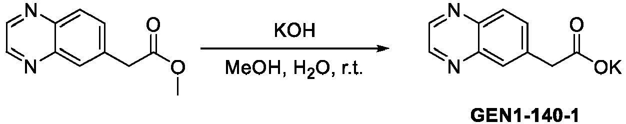

A solution of KOH (28 mg, 0.50 mmol) in water (0.2 mL) was add a solution of methyl 2-(quinoxalin-6-yl)acetate (100 mg, 0.50 mmol) in MeOH (2 mL). The resulting mixture was stirred at room temperature for 5 hours. The resulting mixture was concentrated. The crude product was used directly in the next step without further purification. TLC: MeOH/(DCM+MeOH)=5%, Rf=0.6

### Step 2. Synthesis of 1-(1-(2-(quinoxalin-6-yl)acetyl)piperidin-4-yl)-7-(trifluoromethyl)-1,3-dihydro-2H-benzo[d]imidazol-2-one (GEN1-140)

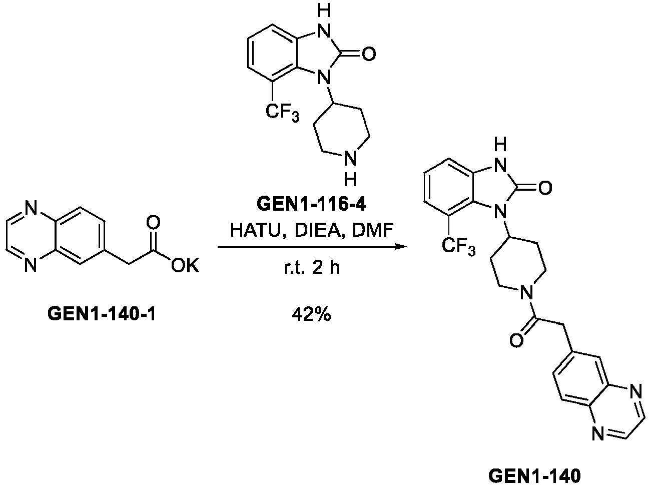

DIEA (125 mg, 0.98 mmol) was added to a solution of potassium 2-(quinoxaline-6-yl)acetate (102 mg crude), 1-(piperidin-4-yl)-7-(trifluoromethyl)-1,3-dihydro-2H-benzo[d]imidazol-2-one (140 mg, 0.49 mmol), HATU (280 mg, 0.74 mmol) in DMF (2.5 mL). The resulting mixture was stirred at room temperature for 2 hours. The reaction mixture was added dropwise to water (20 mL). The precipitated solid was collected by filtration. The crude product was purified by reverse-phase flash chromatography (C₁₈ reverse silica column; mobile phase CH₃CN, water, 10% to 70% gradient, 40 minutes; detection, UV 214 nm) to obtain the desired compound (94.3 mg, yield 42%) as a white solid. ¹H NMR (400 MHz, DMSO-*d*₆): δ 11.45 (s, 1H), 8.96 - 8.89 (m, 2H), 8.05 (d, *J* = 8.6 Hz, 1H), 8.01 - 7.96 (m, 1H), 7.81 - 7.72 (m, 1H), 7.36 (d, *J* = 8.2 , 1H), 7.30 - 7.24 (m, 1H), 7.16 (t, *J* = 7.9 Hz, 1H), 4.62 (d, *J=* 12.5 Hz, 1H), 4.35 - 4.20 (m, 2H), 4.18 - 3.97 (m, 2H), 3.06 (t, *J=* 13.2 Hz, 1H), 2.67-2.51 (m, 2H), 2.48-2.43 (m, 1H), 1.82 - 1.61 (m, 2H). MS (ESI): Rt = 1.42 min, *m*/*z* 456.1 [M + H]⁺, Purity: 100% @ 254 nm, 100% @ 214 nm.

### Example 111: 7-(trifluoromethyl)-1-(1-(1-(4-(trifluoromethyl)phenyl)cyclopropane-1-carbonyl)piperidin-4-yl)-1,3-dihydro-2H-benzo[d]imidazol-2-one (GEN1-146)

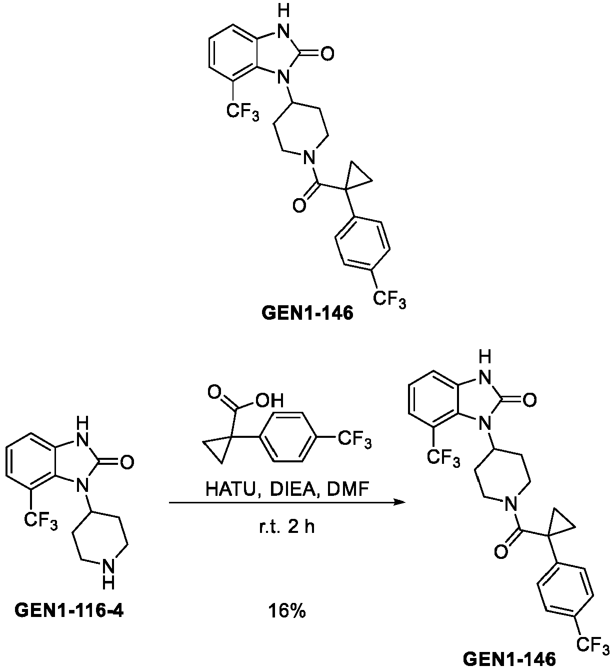

### Step 1. 7-(trifluoromethyl)-1-(1-(1-(4-(trifluoromethyl)phenyl)cyclopropane-1-carbonyl)piperidin-4-yl)-1,3-dihydro-2H-benzo[d]imidazol-2-one (GEN1-146)

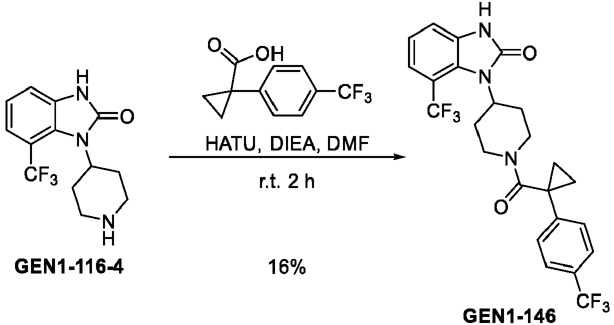

DIEA (67 mg, 0.52 mmol) was added a solution of 1-(piperidin-4-yl)-7-(trifluoromethyl)-1,3-dihydro-2H-benzo[d]imidazol-2-one (37 mg, 0.13 mmol), 1-(4-(trifluoromethyl)phenyl)cyclopropane-1-carboxylic acid (46 mg, 0.13 mmol), HATU (74 mg, 0.19 mmol) in DMF (1.0 mL). The resulting mixture was stirred at room temperature for 15 hours. The reaction mixture was added dropwise to water (30 mL). The precipitated solid was collected by filtration. The crude product was purified by reverse-phase flash chromatography (C₁₈ reverse silica column; mobile phase CH₃CN, water, 5% to 70% gradient, 40 minutes; detection, UV 214 nm) to obtain the desired compound (10.4 mg, yield 16%) as a white solid. ¹H NMR (400 MHz, DMSO-d₆): δ 11.46 (s, 1H), 7.67 (d, *J*= 8.2 Hz, 2H), 7.41 - 7.30 (m, 3H), 7.25 (d, *J*= 7.2 Hz, 1H), 7.14 (t, *J*= 7.9 Hz, 1H), 4.75-4.45 (m, 1H), 4.29-3.92 (m, 2H), 2.96-2.58 (m, 2H), 2.4 -2.15 (m, 2H), 1.82-1.50 (m, 2H), 1.48-1.24 (m, 4H). MS (ESI): Rt = 1.72 min, *m*/*z* 497.9 [M + H]⁺ , Purity: 91% @ 254 nm, 100% @ 214 nm.

### Example 112: 4-(2-oxo-7-(trifluoromethyl)-2,3-dihydro-1H-benzo[d]imidazol-1-yl)-N-(4-(trifluoromethyl)phenyl)piperidin-1-carboxamide (GEN1-149)

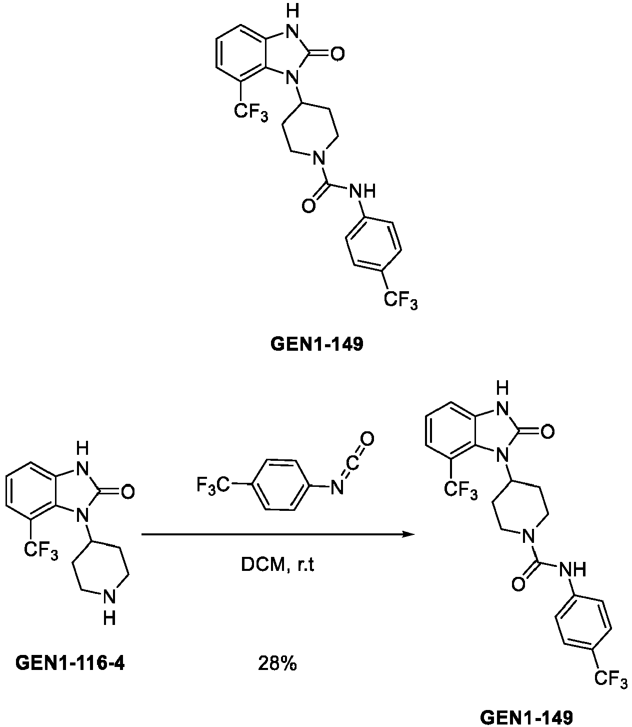

### Step 1. 4-(2-oxo-7-(trifluoromethyl)-2,3-dihydro-1H-benzo[d]imidazol-1-yl)-N-(4-(trifluoromethyl)phenyl)piperidin-1-carboxamide (GEN1-149)

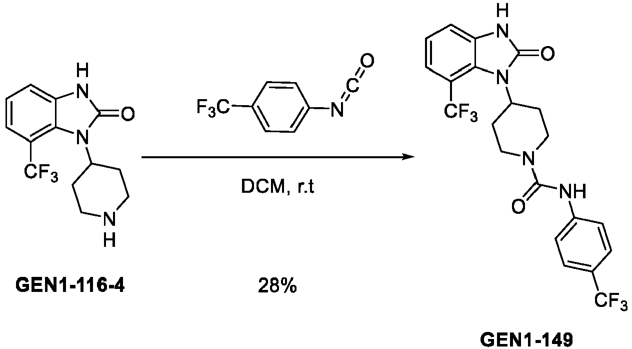

A solution of 1-isocyanato-4-(trifluoromethyl)benzene (33 mg, 0.17 mmol) in DCM (2 mL) was added to a solution of 1-(piperidin-4-yl)-7-(trifluoromethyl)-1,3-dihydro-2H-benzo[d]imidazol-2-one (50 mg, 0.17 mmol) in DCM (8 mL). The resulting mixture was stirred at room temperature for 15 hours. The precipitated solid was collected by filtration, and washed with DCM and water. The crude product was purified by reverse-phase flash chromatography (C₁₈ reverse silica column; mobile phase CH₃CN, water, 20% to 80% gradient, 50 minutes; detection, UV 214 nm) to obtain the desired compound (23.2 mg, yield 28%) as a white solid. ¹H NMR (400 MHz, DMSO-d₆): δ 11.47 (s, 1H), 9.00 (s, 1H), 7.71 (d, *J*= 8.6 Hz, 2H), 7.60 (d, *J=* 8.6 Hz, 2H), 7.38 (d, *J=* 8.0 Hz, 1H), 7.29 (d, *J=* 7.7 Hz, 1H), 7.17 (t, *J=* 7.9 Hz, 1H), 4.41-4.19 (m, 3H), 2.82 (t, *J=* 13.1 Hz, 2H), 2.70-2.53 (m, 2H), 1.73 (d, *J=* 12.4 Hz, 2H). MS (ESI): Rt = 1.63 min, *m*/*z* 473.0 [M+H]⁺, 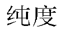 (Purity): 100% @ 254 nm, 93% @ 214 nm.

### Example 113: (E)-1-(4-(3-(hydroxyimino)-3,4-dihydroquinoxaline-1(2H)-yl)piperidin-1-yl)-2-(4-(trifluoromethyl)phenyl)ethane-1-one (GEN1-150)

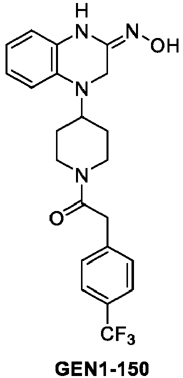

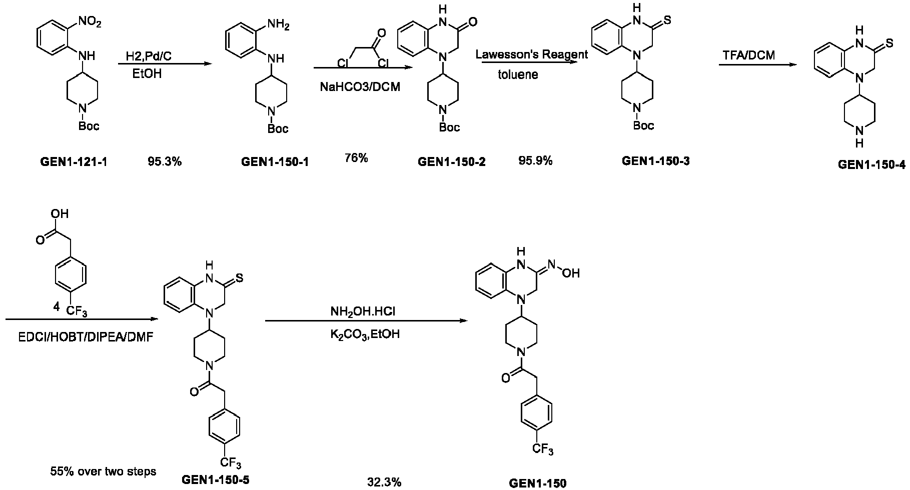

### Step 1. Synthesis of tert-butyl 4-((2-aminophenyl)amino)piperidin-1-carboxylate (GEN1-150-1)

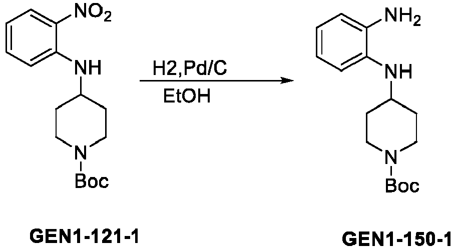

Pd/C (100 mg, 5% purity) was added to a solution of tert-butyl 4-(2-nitroanilino)piperidin-1-carboxylate (2.9 g, 9.0 mmol, the synthesis steps could be found in GENl-121-1) in EtOH(30 mL). The resulting mixture was replaced with hydrogen and was stirred at room temperature under a hydrogen atmosphere overnight. The mixture was filtered, and the filtrate was concentrated to obtain the desired compound (2.5 g, yield 95.3%) as a brown oil.

### Step 2. Synthesis of tert-butyl 4-(3-oxo-3,4-dihydroquinoxaline-1(2H)-yl)piperidin-1-carboxylate (GEN1-052-2)

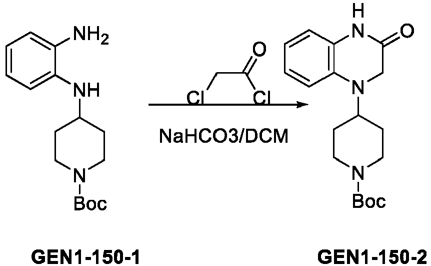

In an ice bath, 2-chloroacetyl chloride (565 mg , 5.0 mmol) was added to a suspension of tert-butyl 4-(2-aminoanilino)piperidin-1-carboxylate (1.456 g, 5.0 mmol) and NaHCO₃ (2.10 g, 25 mmol) in DCM (25 mL). The resulting mixture was stirred at room temperature for 0.5 hours. The mixture was concentrated and dissolved in CH₃CN (100 mL). The resulting mixture was stirred at 80°C overnight. The mixture was cooled and filtered. The filtrate was concentrated, and the residue was purified by silica gel column chromatography (PE:EA = 1:1) to obtain the desired compound (1.26 g, yield 76%) as a white solid.

### Step 3. Synthesis of tert-butyl 4-(3-thio-3,4-dihydroquinoxaline-1(2H)-yl)piperidin-1-carboxylate (GEN1-150-3)

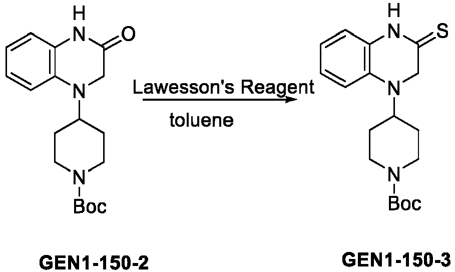

Under the protection of nitrogen, Lawson's reagent (121.3 mg, 0.30 mmol) was added to a suspension of 4-(3-oxo-3,4-dihydroquinoxaline-1(2H)-yl)piperidin-1-carboxylic acid (200 mg, 0.60 mmol), NaHCO₃ (252 mg, 3.0 mmol) in toluene (10 mL). The resulting mixture was stirred at 80°C for 4 hours. The obtained mixture was concentrated and purified by silica gel column chromatography (PE:EA=3:1) to obtain the desired compound (200 mg, yield 95.9%) as a pale yellow solid. MS (ESI): Rt = 1.62 min, m/z 371.1, [M+H]⁺; Purity: 96.9% @ 254 nm, 98.04% @ 214 nm.

### Step 4. Synthesis of tert-butyl 4-(3-thio-3,4-dihydroquinoxaline-1(2H)-yl)piperidin-1-carboxylate (GEN1-150-4)

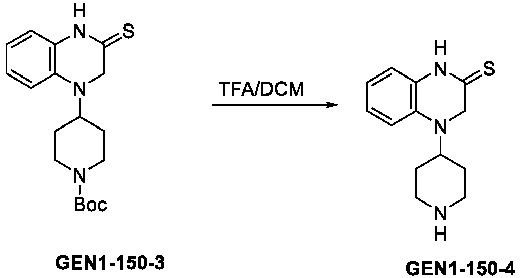

TFA (2 mL) was added to a solution of tert-butyl 4-(3-thio-3,4-dihydroquinoxaline-1(2H)-yl)piperidin-1-carboxylate (200 mg, 0.57 mmol) in DCM (10 mL). The resulting mixture was stirred at room temperature for 1 hour. The resulting mixture was concentrated and redissolved in DCM (10ml). Then the mixture was concentrated again to obtain the crude product (200 mg) as a brown solid. The crude product was used directly in the next step without purification. MS (ESI): Rt = 1.17 min, m/z 248.2, [M+H]⁺; Purity: 85.44% @ 254 nm, 63.67% @ 214 nm.

### Step 5. Synthesis of 1-(4-(3-thio-3,4-dihydroquinoxaline-1(2H)-yl)piperidin-1-yl)-2-(4-(trifluoromethyl)phenyl)ethan-1-one (GEN1-150-5)

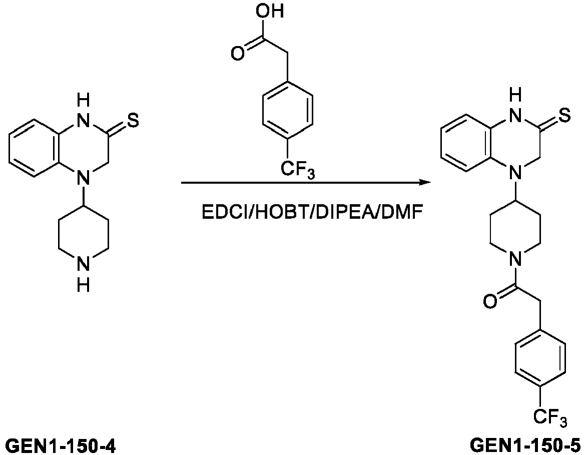

EDCI (164 mg, 0.855 mmol) was added to a solution of tert-butyl 2-(4-(trifluoromethyl)4-(3-thiooxa3,4-dihydroquinoxaline-1(2H)-yl)piperidin-1-carboxylate (200 mg, crude product, 0.57 mmol), phenyl)acetic acid (139.6 mg, 0.684 mmol), HOBT (115.5 mg, 0.855 mmol) and DIEA (294.5 mg, 2.28 mmol) in DMF (5 mL). The resulting mixture was stirred at room temperature for 1 h. The reaction mixture was diluted with water (80 mL), and extracted with EtOAc (25 mL×3). The combined organic phase was washed with brine (50 mL), dried over anhydrous Na₂SO₄ and filtered. The filtrate was concentrated to obtain a crude product. The crude product was purified by silica gel column chromatography (PE:EA = 2:1) to obtain the desired compound (136 mg, yield 55%) as a white solid. MS (ESI): Rt = 1.59 min, m/z 434.4, [M+H]+; Purity: 96.58% @ 254 nm, 96.55% @ 214 nm.

### Step 6. Synthesis of (E)-1-(4-(3-(hydroxyimino)-3,4-dihydroquinoxaline-1(2H)-yl)piperidin-1-yl)-2-(4-(trifluoromethyl)phenyl)ethane-1-one (GEN1-150)

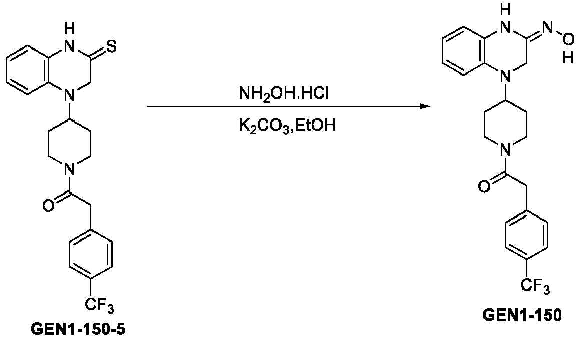

Hydroxylamine hydrochloride (12 mg, 0.173 mmol) was added to a solution of 1-(4-(3-thioxo-3,4-dihydroquinoxalalin-1(2H)-yl)piperidin-1-yl)-2-(4-(trifluoromethyl)phenyl)ethan-1-one (50 mg, 0.115 mmol), K₂CO₃ (31.8 mg, 0.23 mmol) in EtOH (10 mL). The resulting mixture was stirred at 80°C for 3 hours. The mixture was poured into water (20 mL), and extracted with EA (30 mL×3). The combined organic layer was washed with brine (50 mL), dried over Na₂SO₄ and concentrated. The crude product was purified by reverse phase chromatography. Purification conditions: C18 silica gel column; mobile phase ACN: H₂O, gradient: 5% to 50%; 45 minutes, monitoring wavelength: 214 nm. The desired compound (20 mg, yield 32.3%) was obtained as a white solid. ¹H NMR (400 MHz, DMSO-d6) δ 9.43 (s, 1H), 9.01 (s, 1H), 7.67 (d, J = 8.0 Hz, 2H), 7.47 (d, J = 8.0 Hz, 2H), 7.02 (dd, J = 7.7, 1.5 Hz, 1H), 6.84 (d, J = 8.0 Hz, 1H), 6.73 (td, J = 7.7, 1.6 Hz, 1H), 6.65 (td, J = 7.5, 1.2 Hz, 1H), 4.51 (d, J = 12.9 Hz, 1H), 4.07 (d, J = 13.6 Hz, 1H), 3.95-3.75 (m, 3H), 3.40 (d, J = 2.4 Hz, 2H), 3.17 (t, J = 12.2 Hz, 1H), 2.70 (t, J = 12.4 Hz, 1H), 1.87 - 1.34 (m, 4H). MS (ESI): Rt = 1.46 min, m/z 432.8, [M+H]⁺; Purity: 100% @ 254 nm, 97.37% @ 214 nm.

### Example 114: 2-oxo-3-(1-(2-(quinolin-6-yl)acetyl)piperidin-4-yl)-2,3-dihydro-1H-benzo[d]imidazol-4-carbonitrile (GEN1-151)

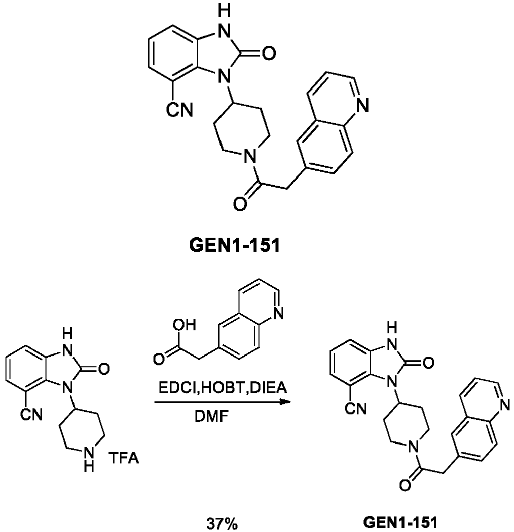

### Step 1. 2-oxo-3-(1-(2-(quinolin-6-yl)acetyl)piperidin-4-yl)-2,3-dihydro-1H-benzo[d]imidazol-4-carbonitrile (GEN1-151)

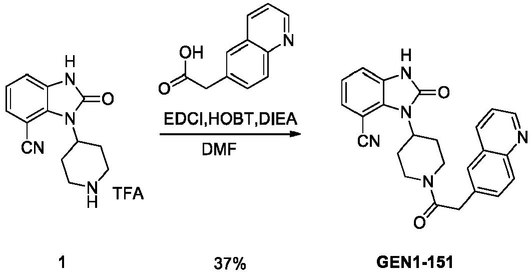

DIEA (435 mg, 3.37 mmol) was added to a solution of 2-oxo-3-(piperidin-4-yl)-2,3-dihydro-1H-benzo[d]imidazol-4-carbonitrile 2,2,2-trifluoroacetate (150 mg, 0.421 mmol, the synthesis steps could be found in the final report of GEN1-014-4) in DMF (3 mL). After the mixture was stirred at room temperature for 10 minutes, 2-(6-quinolinyl)acetic acid (79 mg, 0.42 mmol), EDCI (121 mg, 0.630 mmol) and HOBT (85 mg, 0.63 mmol) were added. The resulting mixture was stirred at room temperature overnight. The reaction mixture was diluted with water (15 mL), and extracted with EtOAc (10 mL×2). The combined organic phase was washed with brine (5 mL), dried over anhydrous Na₂SO₄ and filtered. The filtrate was concentrated and the residue was purified by preparative HPLC [gilson-2, Xbridge C₁₈, 5 µm, 19×150 mm, 30∼70%B, A: H₂O(0.1%NH₄HCO₃), B: ACN, flow rate: 15 ml/min, UV = 214nm] to obtain the desired compound (65 mg, yield 37%) as a white solid. ¹H NMR (400 MHz, DMSO-*d*₆): δ 11.43 (br s, 1H), 8.87 (dd, *J=* 4.0, 1.6 Hz, 1H), 8.32 (d, *J=* 7.6 Hz, 1H), 7.97 (d, *J=* 8.4 Hz, 1H), 7.81 (s , 1H), 7.66 (dd, *J* = 8.4, 2.0 Hz, 1H), 7.52 (dd, *J* = 8.4, 4.0 Hz, 1H), 7.41-7.39 (m, 1H), 7.27 (d, *J* = 6.8 Hz, 1H), 7.13 (t, *J=* 8.0 Hz, 1H), 4.84-4.76 (m, 1H), 4.66 (d, *J* = 12.8 Hz, 1H), 4.24 (d, *J* = 14.0 Hz, 1H), 4.05-3.95 (m, 2H), 3.15-3.09 (m, 1H), 2.67-2.60 (m, 1H), 2.46-2.33 (m, 2H), 1.90-1.87 (m, 2H). MS (ESI): Rt = 2.915 min, *m*/*z* 411.9 [M+H]⁺; Purity: 99.62% @ 254 nm, 99.52% @ 214 nm.

### Example 115: Synthesis of 2-oxo-1-(1-(1-(2-(4-(trifluoromethyl)phenyl)acetyl)piperidin-4-yl)-2,3-dihydro-1H-benzo[d]imidazol-4-carboxylic acid (GEN1-153)

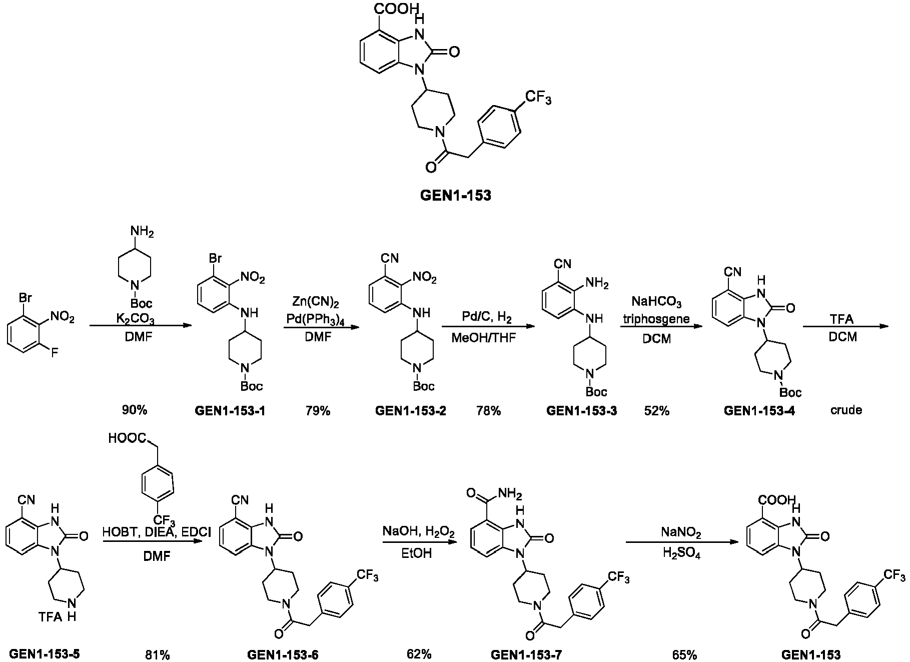

### Step 1. Synthesis of tert-butyl 4-((3-bromo-2-nitrophenyl)amino)piperidin-1-carboxylate

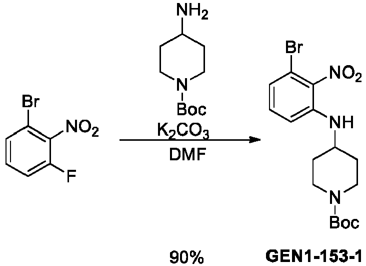

K₂CO₃ (4.71 g, 34.1 mmol) was added to a suspension of 1-bromo-3-fluoro-2-nitrobenzene (5.00 g, 22.7 mmol) and tert-butyl 4-aminopiperidine-1-carboxylate (4.78 g, 23.9 mmol) in DMF (25 mL). The mixture was stirred at 70°C overnight. After the mixture was cooled to room temperature, water (100 mL) was added. DCM (150 mL×3) was added to extract the desired compound. The combined organic layer was washed with brine (150 mL), dried over anhydrous Na₂SO₄ and filtered. The filtrate was concentrated, and the residue was purified by a silica gel column (petroleum ether: EtOAc = 10:1) to obtain the desired compound (8.20 g, yield 90%) as a yellow solid. MS (ESI) Rt = 1.83 min, *m*/*z* 398.4 [M-H]⁻; Purity: 100% @ 254 nm, 100% @ 214 nm.

### Step 2. Synthesis of tert-butyl 4-((3-cyano-2-nitrophenyl)amino)piperidin-1-carboxylate

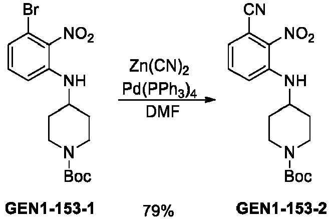

Pd(PPh3)4 (577 mg, 499 umol) was added to a mixture of tert-butyl 4-((3-bromo-2-nitrophenyl)amino)piperidin-1-carboxylate (2.00 g, 5.00 mmol), and zinc cyanide (1.17 g, 9.99 mmol) and DMF (25 mL). The mixture was stirred at 80°C for 28 hours under a nitrogen atmosphere. Then the mixture was poured into a solution of NaClO (10%, 100 mL). After the mixture was stirred at room temperature for 30 minutes, EtOAc (100 mL×3) was added to extract the desired compound. The combined organic layer was washed with a solution of NaClO (10%, 100 mL) and brine (100 mL), dried over anhydrous Na₂SO₄ and filtered. The filtrate was concentrated, and the residue was purified by a silica gel column (petroleum ether: EtOAc = 4: 1) to obtain the desired compound (1.45 g, yield 79%) as an orange solid. MS (ESI): Rt = 1.66 min, *m*/*z* 345.4 [M-H]⁻; Purity: 100% @ 254 nm, 94% @ 214 nm.

### Step 3. Synthesis of tert-butyl 4-((2-amino-3-cyanophenyl)amino)piperidin-1-carboxylate

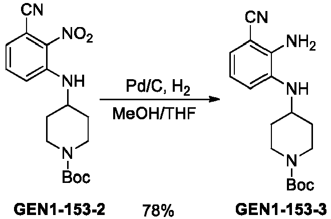

Pd/C (10%, 320 mg) was added to a solution of tert-butyl 4-((3-cyano-2-nitrophenyl)amino)piperidin-1-carboxylate (700 mg, 2.02 mmol) in MeOH (24 mL) and THF (8 mL). The mixture was stirred at room temperature under hydrogen for 6 hours. The mixture was filtered, and the filtrate was concentrated to obtain the desired compound (620 mg, yield 78%) as a brown solid. MS (ESI): Rt = 1.65 min, *m*/*z* 315.4 [M-H]⁻; Purity: 82% @ 254 nm, 80% @ 214 nm.

### Step 4. Synthesis of tert-butyl 4-(4-cyano-2-oxo-2,3-dihydro-1H-benzo[d]imidazol-1-yl)piperidin-1-carboxylate

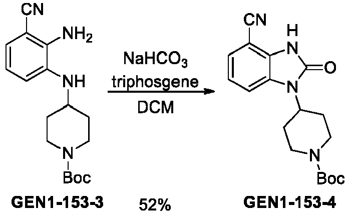

In an ice bath, triphosgene (291 mg, 0.981 mmol) was added to a suspension of tert-butyl 4-(((2-amino-3-cyanophenyl)amino)piperidin-1-carboxylate (620 mg, 1.96 mmol) and NaHCO₃ (1.65 g, 19.6 mmol) in DCM (15 mL). The mixture was stirred at 0°C for 1 hour. The mixture was filtered and the filtrate was concentrated. The residue was purified by C18 (50-70% B, A: H₂O B: CH₃CN) to obtain the desired compound (352 mg, yield 52%) as a white solid. MS (ESI): Rt = 1.52 min, *m*/*z* 287.2 [M-56+H]⁺; Purity: 100 % @ 254 nm, 100% @ 214 nm.

### Step 5. Synthesis of 2-oxo-1-(piperidin-4-yl)-2,3-dihydro-1H-benzo[d]imidazol-4-carbonitrile2,2,2-trifluoroacetate

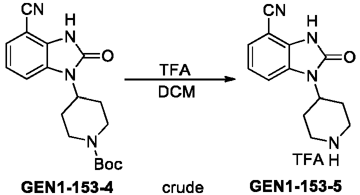

TFA (1 mL) was added to a solution of tert-butyl 4-(4-cyano-2-oxo-2,3-dihydro-1H-benzo[d]imidazol-1-yl)piperidin-1-carboxylate (312 mg, 0.911 mmol) in DCM (6 mL). The mixture was stirred at room temperature for 1.5 hours. The resulting mixture was concentrated to obtain the desired compound (441 mg, crude product) as a pale yellow solid. MS (ESI): Rt = 0.71 min, *m*/*z* 243.2 [M+H]⁺; Purity: 100% @ 254 nm, 100% @ 214 nm.

### Step 6. Synthesis of 2-oxo-1-(1-(2-(4-(trifluoromethyl)phenyl)acetyl)piperidin-4-yl)-2,3-dihydro-1H-benzo[d]imidazol-4-carbonitrile

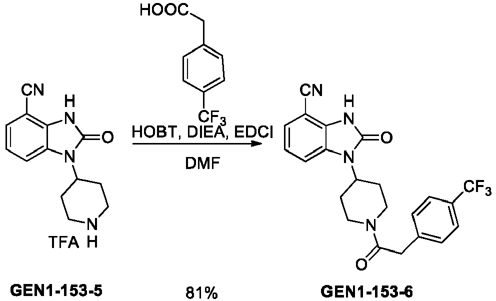

2-(4-(trifluoromethyl)phenyl)acetic acid (303 mg, 1.48 mmol) was added to a mixture of 2-oxo-1-(piperidin-4-yl)-2,3-dihydro-1H-benzo[d]imidazol-4-carbonitrile2,2,2-trifluoroacetate (441 mg, 1.24 mmol), DIEA (800 mg, 6.19 mmol), EDCI (356 mg, 1.86 mmol) and HOBT (251 mg, 1.86 mmol) in DMF (8 mL). The mixture was stirred at room temperature overnight. The resulting mixture was poured into water (30 mL), and extracted with DCM (30 mL×3). The combined organic layer was washed with brine (50 mL), dried over anhydrous Na₂SO₄ and filtered. The filtrate was concentrated and the residue was purified by C18 (50-70% B; A: H₂O B: CH₃CN) to obtain the desired compound (429 mg, yield 81%) as a light brown solid. MS (ESI): Rt = 1.51 min, *m*/*z* 429.4 [M+H]⁺; Purity: 100% @ 254 nm, 100% @ 214 nm.

### Step 7. Synthesis of 2-oxo-1-(1-(2-(4-(trifluoromethyl)phenyl)acetyl)piperidin-4-yl)-2,3-dihydro-1H-benzo[d]imidazol-4-carboxamide

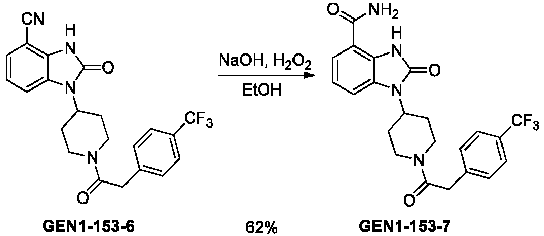

NaOH (28 mg, 0.700 mmol) was added to a mixture of 2-oxo-1-(1-(2-(4-(tris(trifluoromethyl)phenyl)acetyl)piperidin-4-yl)-2,3-dihydro-1H-benzo[d]imidazol-4-carbonitrile (200 mg, 0.467 mmol), aqueous hydrogen peroxide (30%, 2.5 mL) and ethanol (2.5 mL). The mixture was stirred at 55°C overnight. The resulting mixture was filtered, and the residue was washed with water (3mL) and dried to obtain the desired compound (143mg, yield 62%) as a white solid. MS (ESI): Rt = 1.43 min, *m*/*z* 447.4 [M+H]⁺; Purity: 100% @ 254 nm, 91% @ 214 nm.

### Step 8. Synthesis of 2-oxo-1-(1-(1-(2-(4-(trifluoromethyl)phenyl)acetyl)piperidin-4-yl)-2,3-dihydro-1H-benzo[d]imidazol-4-carboxylic acid (GEN1-153)

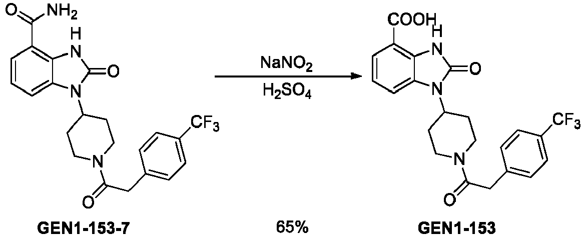

NaNO2 (26 mg, 0.38 mmol) was added to a mixture of 2-oxo-1-(1-(2-(4-(trifluoromethyl)phenyl)acetyl)piperidin-4-yl)-2,3-dihydro-1H-benzo[d]imidazol-4-carboxamide (113 mg, 0.253 mmol) and aqueous H2SO4 (75%, 2 mL). The mixture was stirred at 80°C for 1 hour. After it was cooled to room temperature, the mixture was poured into water (20 mL). The mixture was extracted with DCM (20 mL×3). The combined organic layer was washed with brine (20 mL), dried over anhydrous Na₂SO₄ and filtered. The filtrate was concentrated, and the residue was purified by preparation. HPLC (prep-9 Xbridge C18 5 um 19 * 150 mm, 30%-55% B; A: H₂O (0.1% TFA), B: CH₃CN; 214 nm, flow rate: 15mL / min). The desired compound (74 mg, yield 65%) was obtained as a gray solid. ¹HNMR (400 MHz, DMSO-*d*₆): δ 13.05 (br s, 1H), 10.63 (s, 1H), 7.71 (d, *J=* 8.0 Hz, 2H), 7.53-7.48 (m, 3H), 7.33 (d, *J* = 8.0 Hz, 1H), 7.06 (t, *J* = 8.0 Hz, 1H), 4.60-4.57 (m, 1H), 4.50-4.44 (m, 1H), 4.15-4.11 (m, 1H), 3.97-3.88 (m, 2H), 3.22-3.16 (m, 1H), 2.74-2.69 (m, 1H), 2.16-2.07 (m, 2H), 1.74-1.69 (m, 2H). MS (ESI): Rt = 3.308 min, *m*/*z* 448.1 [M+H]⁺; Purity: 99.87% @ 254 nm, 99.76% @ 214 nm.

### Example 116: 4-morpholino-1-(1-(2-(4-(trifluoromethyl)phenyl)acetyl)piperidin-4-yl)-1H-benzo[d]imidazol-2(3H)-one (GEN1-154)

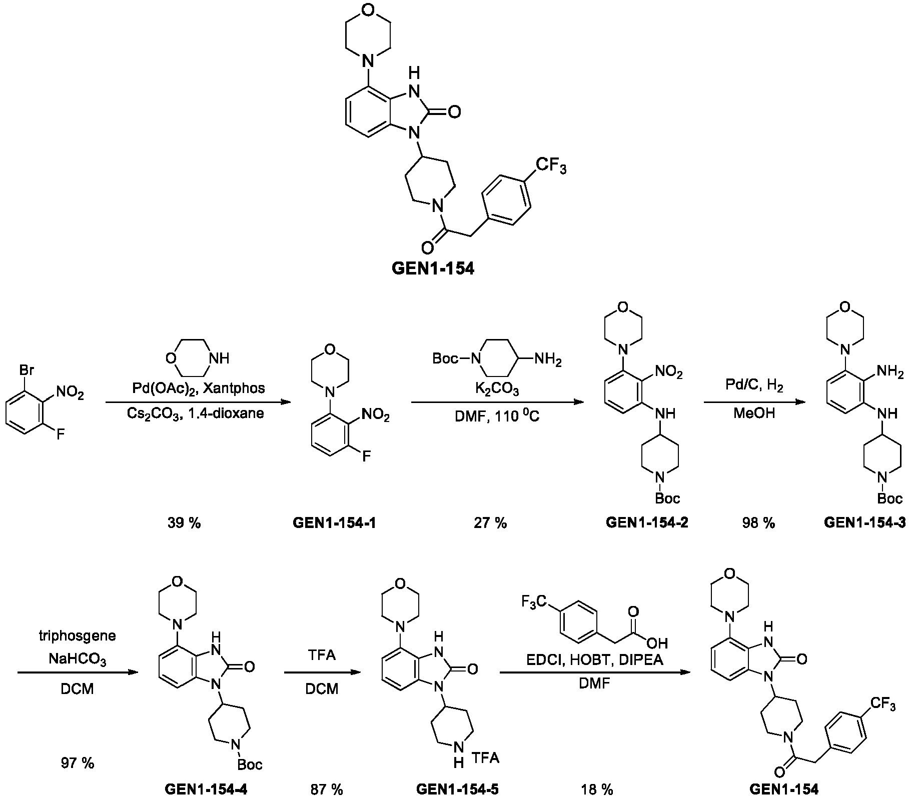

### Step 1. 4-(3-fluoro-2-nitrophenyl)morpholine (GEN1-154-1)

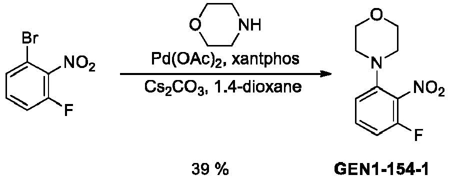

Cs₂CO₃ (533 mg, 1.64 mmol) was added to a solution of 1-bromo-3-fluoro-2-nitrobenzene (3.00 g, 13.6 mmol), Pd(OAc)₂ (367 mg, 1.63 mmol) and Xantphos (947 mg, 1.64 mmol) in 1,4 dioxane (10 mL). Then morpholine (1.66 g, 19.1 mmol) was added, and the mixture was heated at 110°C under N₂ atmosphere for 4 hours. After it was cooled to room temperature, the mixture was poured into water (15 mL), and extracted with EtOAc (15 mL×3). The combined organic layer was washed with brine (15 mL×2), dried over anhydrous Na₂SO₄ and it was concentrated. The residue was purified by column chromatography on silica gel (petroleum ether:EA=10:1) to obtain the desired compound (1.20 g, yield 39%) as a yellow oil. ¹H NMR (400MHz, CDCl₃): δ 7.43-7.37 (m, 1H), 6.97-6.90 (m, 2H), 3.80-3.78 (m, 4H), 3.04-3.02 (m, 4H).

### Step 2. Tert-butyl 4-((3-morpholino-2-nitrophenyl)amino)piperidin-1-carboxylate (GEN1-154-2)

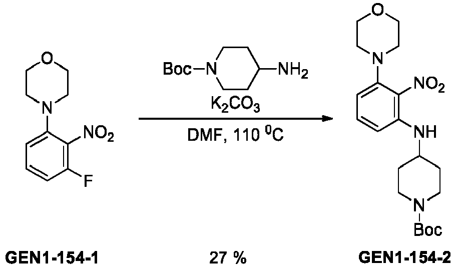

Tert-butyl 4-aminopiperidine-1-carboxylate (531 mg, 2.65 mmol) was added to a solution of 4-(3-fluoro-2-nitro-phenyl)morpholine (500 mg, 2.21 mmol) and K₂CO₃ (458 mg, 3.31 mmol) in DMF (8 mL). After the mixture was stirred at 110°C for 16 hours, and then cooled to room temperature. The mixture was poured into water (20 mL), and extracted with EtOAc (15 mL×3). The combined organic layer was washed with brine (20 mL×2), dried over anhydrous sodium sulfate and concentrated to obtain the desired compound (240 mg, yield 27%) as a yellow oil. MS (ESI): Rt = 1.71 min, m/z 407.5 [M+H]⁺, Purity: 56% @254nm, 54% @214nm.

### Step 3. Tert-butyl 4-((2-amino-3-morpholinophenyl)amino)piperidin-1-carboxylate (GEN1-154-3)

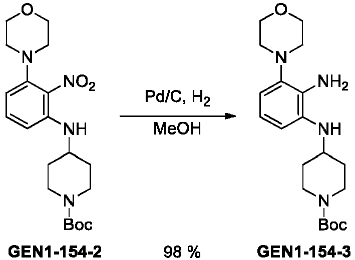

Tert-butyl 4-(3-morpholino-2-nitro-anilino)piperidin-1-carboxylate (240 mg, 590 umol) was added to a solution of Pd/C (10% purity, 70 mg) in MeOH (8 mL), and it was hydrogenated at room temperature for 1.5 hours. Then the reaction mixture was filtered, concentrated and purified by a C₁₈ column (CAN/water=30-70%) to obtain the desired compound (242 mg, yield 98%) as a brown oil. MS (ESI): Rt = 1.63 min, m/z 377.5 [M+H]⁺; Purity: 100% @254nm, 100% @214nm.

### Step 4. Tert-butyl 4-(4-morpholino-2-oxo-2,3-dihydro-1H-benzo[d]imidazol-1-yl)piperidin-1-carboxylate (GEN1-154-4)

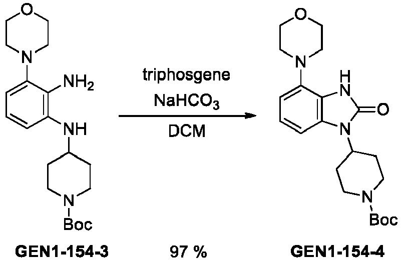

Triphosgene (191 mg, 0.643 mmol) was added to a solution of tert-butyl 4-((2-amino-3-morpholinophenyl)amino)piperidin-1-carboxylate (242 mg, 0.643 mmol) and NaHCO₃ (216 mg, 2.57 mmol) in DCM (5 mL) at 0 °C. The mixture was stirred at room temperature for 2 hours. Then the mixture was poured into water (15 mL), and extracted with DCM (10 ml×3). The combined organic layer was washed with brine (15 mL×2), dried over anhydrous Na₂SO₄ and it was concentrated to obtain a white solid. Then the crude product was purified through a C₁₈ column (CH₃CN: water = 10%-60%) to obtain the desired compound (280 mg, yield 97%) as a white solid. MS (ESI): Rt = 1.52 min, m/z 403.5 [M+H]⁺, Purity: 89% @254nm, 89% @214nm.

### Step 5. 7-morpholino-3-(4-piperidinyl)-1H-benzimidazol-2-one (GEN1-154-5)

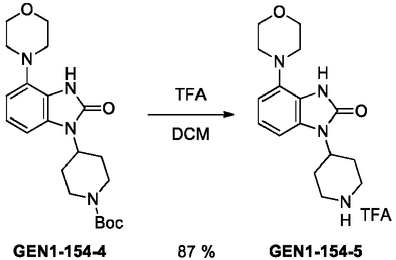

TFA (0.5 mL) was added to a solution of tert-butyl 4-(4-morpholino-2-oxo-2,3-dihydro-1H-benzo[d]imidazol-1-yl)piperidin-1-carboxylate (150 mg, 0.373 mmol) in dichloromethane (10 mL). After the mixture was stirred for 2 hours, the mixture was concentrated to obtain the desired compound (150 mg, yield 87%) as a white solid. MS (ESI): Rt = 1.12 min, m/z 303.4 [M+H]⁺; Purity: 91% @254 nm, 92% @214 nm.

### Step 6. 7-morpholino-3-(4-piperidinyl)-1H-benzimidazol-2-one (GEN1-154)

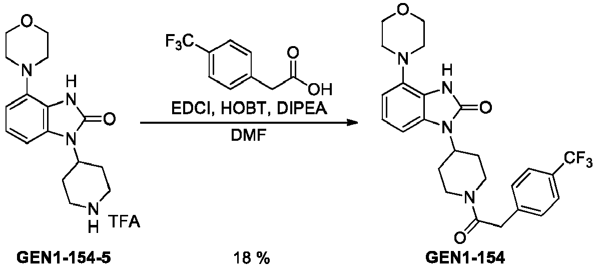

HOBT (60 mg, 0.488 mmol) was added to a solution of 2-(4-(trifluoromethyl)phenyl)acetic acid (65 mg, 0.323 mmol) and EDCI (91 mg, 0.486 mmol) in DMF (10 mL). After the mixture was stirred at room temperature for 5 minutes, 4-morpholino-1-(piperidin-4-yl)-1H-benzo[d]imidazol-2(3H)-1,2,2,2-trifluoroacetate (150 mg, 0.324 mmol) and DIPEA (125 mg, 0.97 mmol) were added to the reaction solution. The mixture was stirred at room temperature for 4 hours. The mixture was diluted with water (15 mL), and extracted with EtOAc (10 mL×3). The combined organic phase was washed with brine (15 mL×2), dried over anhydrous Na₂SO₄ and concentrated. The residue was purified by preparative HPLC (RP-PREP-1 Xbridge C₁₈ 5 um 19 * 150 mm, 20%-75%, B; A: H₂O (0.1% NH₄HCO₃), B: CH₃CN; UV: 214 nm, flow rate: 15 mL/min) to obtain the desired compound (29.5 mg, yield 18%) as a white solid. ¹H NMR (400 MHz, DMSO-*d*₆): δ 10.87 (s, 1H), 7.70 (d, *J=* 8.4 Hz, 2H), 7.52 (d, *J=* 8.0 Hz), 6.92-6.88 (m, 1H), 6.76 (d, *J=* 8.0 Hz, 1H), 6.63 (d, *J=* 8.0 Hz, 1H), 4.57 (d, *J* = 12.8 Hz, 1H), 4.44-4.37 (m, 1H), 4.12 (d, *J=* 13.6 Hz, 1H), 3.97-3.87 (m, 2H), 3.76-3.74 (m, 4H), 3.21-3.15 (m, 1H), 2.90-2.88 (m, 4H), 2.73-2.67 (m, 1H), 2.13-2.04 (m, 2H), 1.70-1.64 (m, 2H). MS (ESI): Rt = 3.85 min, m/z 489.2 [M+H]⁺; Purity: 98.27% @254 nm, 98.69% @214 nm.

### Example 117: 1-(1-(2-(4-chlorophenyl)acetyl)piperidin-4-yl)-7-(trifluoromethyl)-1H-benzo[d]imidazol-2(3H)-one (GEN1-156)

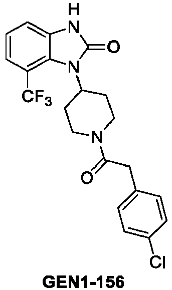

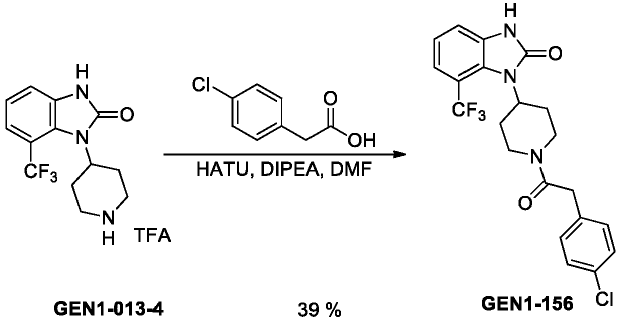

### Step 1. 1-(1-(2-(4-chlorophenyl)acetyl)piperidin-4-yl)-7-(trifluoromethyl)-1H-benzo[d]imidazol-2(3H)-one (GEN1-156)

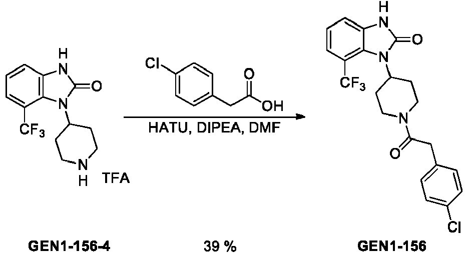

A mixture of 2-(4-chlorophenyl)acetic acid (51 mg, 0.30 mmol) and HATU (142 mg, 0.38 mmol) in DMF (3 mL) was stirred at 0°C for 20 minutes. Then 1-(piperidin-4-yl)-7-(trifluoromethyl)-1H-benzo[d]imidazol-2(3H)-1,2,2,2-trifluoroacetate (100 mg, 0.250 mmol, the synthesis steps could be found in the final report of GEN1-013-4.) and DIPEA (97 mg, 0.75 mmol) were added to the reaction solution, and the resulting mixture was stirred at 20°C for 2 hours. The mixture was poured into water (15 mL), and extracted with EtOAc (10 ml×3). The combined organic layer was washed with brine (10 mL×2), dried over anhydrous Na₂SO₄ and it was concentrated. The residue was purified by preparative HPLC [RP-PREP-1, Xbridge C18, 5um, 19 * 150 mm, 40∼75%B, GT = 10, A: H₂O (0.1% NH₄HCO₃), B: ACN, flow rate): 15 ml/min, UV = 214nm] to obtain the desired compound (43 mg, yield 39%) as a white solid. ¹H NMR (400 MHz, CDCl₃): δ 9.78 (s, 1H), 7.40 (d, *J* = 8.0 Hz, 1H), 7.32-7.27 (m, 3H), 7.23-7.21 (m, 2H), 7.15 (t, *J=* 8.0 Hz, 1H), 4.89 (d, *J*= 12.8 Hz, 1H), 4.46-4.38 (m, 1H), 4.02 (d, *J=* 14.0 Hz, 1H), 3.77 (s, 2H), 3.10-3.03 (m, 1H), 2.77-2.59 (m, 3H), 1.84-1.77 (m, 2H). MS (ESI): Rt = 4.094 min, *m*/*z* 438.1 [M+H]⁺; Purity: 71.60% @ 254 nm, 98.86% @ 214 nm.

### Example 118: 1-(1-(2-(4-bromophenyl)acetyl)piperidin-4-yl)-7-(trifluoromethyl)-1H-benzo[d]imidazol-2(3H)-one (GEN1-157)

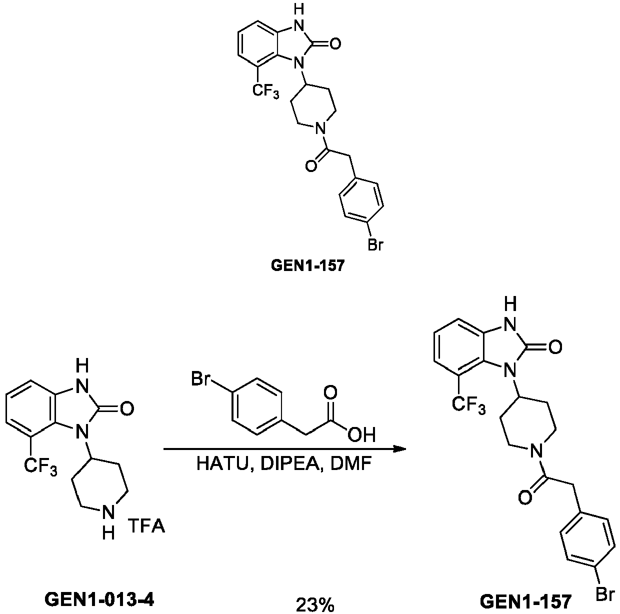

### Step 1. 1-(1-(2-(4-bromophenyl)acetyl)piperidin-4-yl)-7-(trifluoromethyl)-1H-benzo[d]imidazol-2(3H)-one (GEN1-157)

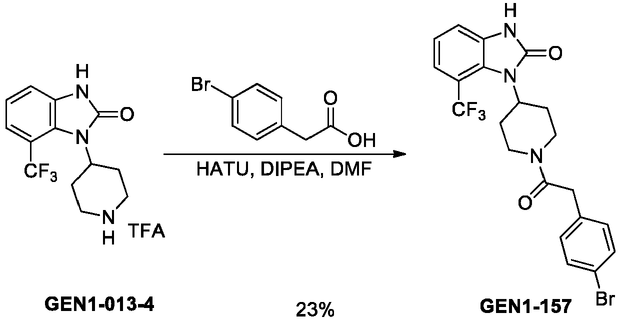

A mixture of 2-(4-bromophenyl)acetic acid (64 mg, 0.30 mmol) and HATU (143 mg, 376 umol) in DMF (2 mL) was stirred at 0°C for 40 minutes. Then 1-(piperidin-4-yl)-7-(trifluoromethyl)-1H-benzo[d]imidazol-2(3H)-1,2,2,2-trifluoroacetate (100 mg , 0.250 mmol, the synthesis steps could be found in the final report of GEN1-013-4.) and DIPEA (97 mg, 0.75 mmol) were added to the reaction solution. The resulting mixture was stirred at 20°C for 2 hours. The mixture was poured into water (15 mL), and extracted with EtOAc (15 mL×3). The combined organic layer was washed with brine (10 mL×3), dried over anhydrous Na₂SO₄ and it was concentrated. The residue was purified by preparative HPLC [water-3, sunfire C₁₈, 5 um, 19 * 150 mm, 30∼80% B, A: H₂O (0.1% NH₄HCO₃), B: ACN, flow rate: 15 mL / min, UV purification = 214nm] to obtain the desired compound (27 mg, yield 23%) as a white solid. ¹H NMR (400 MHz, CDCl₃): δ 9.88 (s, 1H), 7.46 (d, *J=* 8.4 Hz, 2H), 7.40 (d, *J=* 8 Hz, 1H), 7.29-7.26 (m, 1H), 7.17-7.13 (m, 3H), 4.88 (d, *J=* 12.4 Hz, 1H), 4.51-4.39 (m, 1H), 4.02 (d*, J=* 14.8 Hz, 1H), 3.75 (s, 2H), 3.06 (t, *J=* 13.2 Hz, 1H), 2.79-2.58 (m, 3H), 1.82 (t, *J=* 5.6 Hz, 2H). MS (ESI): Rt = 2.146 min, *m*/*z* 482.1 [M+H]⁺; Purity: 92.78% @ 254 nm, 99.00% @ 214 nm.

### Example 119: 1-(1-(2-methyl-2-(4-(trifluoromethyl)phenyl)propionyl)piperidin-4-yl)-7-(trifluoromethyl)-1H-benzo[d]imidazol-2(3H)-one (GEN1-158)

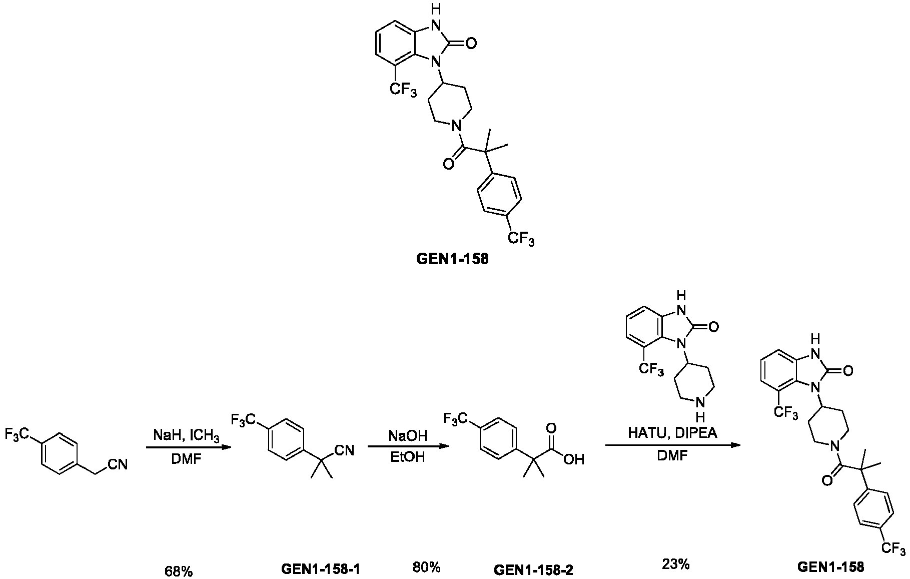

### Step 1. Synthesis of 2-methyl-2-(4-(trifluoromethyl)phenyl)propionitrile

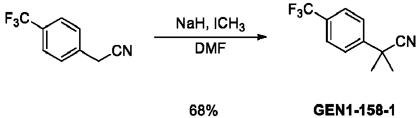

60% NaH (519 mg, 13.0 mmol) was added to a solution of 2-(4-(trifluoromethyl)phenyl)acetonitrile (1.00 g, 5.40 mmol) in DMF (10 mL) under nitrogen and at 0°C, and then CH3I (1.99 g, 14.0 mmol) was added. The mixture was warmed to room temperature and stirred for 4 hours. The mixture was quenched with water (15 mL), and extracted with EtOAc (15 mL×3). The combined organic layer was washed with brine (10 mL), and dried over anhydrous Na₂SO₄ to obtain the desired compound (1.12 g, yield 68%) as a colorless oil. ¹HNMR (400 MHz, DMSO-*d*₆): δ 7.67-7.62 (m, 4H), 1.76 (s, 6H).

### Step 2. Synthesis of 2-methyl-2-(4-(trifluoromethyl)phenyl)propionic acid

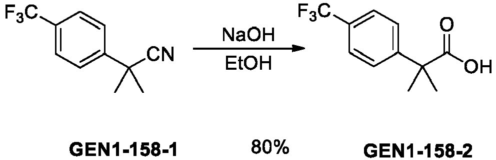

A solution of 2-methyl-2-(4-(trifluoromethyl)phenyl)propionitrile (400 mg, 1.88 mmol) and NaOH (10 M, 1 mL) in EtOH (5 mL) was refluxed at 100°C overnight. After the mixture was cooled to room temperature, EtOAc (15 mL) was added. The two layers were separated, and the organic solution was extracted with water (10 mL×3). The combined aqueous layer was acidified with concentrated hydrochloric acid to pH=2-3. HCl solution. Then the aqueous layer was extracted with EtOAc (20 mL×3). The combined organic layer was washed with brine (20 mL×2), dried over anhydrous Na₂SO₄ and concentrated in vacuo to obtain the desired compound (390 mg, yield 81%) as a white solid. ¹HNMR (400 MHz, DMSO-*d*₆): δ 12.56 (s, 1H), 7.70 (d, *J=* 8.0 Hz, 2H), 7.58 (d, *J=* 8.0 Hz, 2H), 1.51 (s, 6H).

### Step 3. Synthesis of 1-(1-(2-methyl-2-(4-(trifluoromethyl)phenyl)propionyl)piperidin-4-yl)-7-(trifluoromethyl)-1H-benzo[d]imidazol-2(3H)-one (GEN1-158)

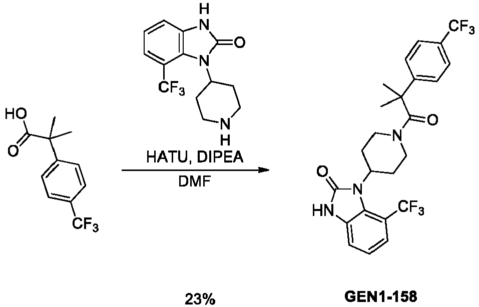

HOBT (51 mg, 0.38 mmol) was added to a solution of 2-methyl-2-(4-(trifluoromethyl)phenyl)propionic acid (105 mg, 452 umol) and EDCI (108 mg, 0.565 mmol) in DMF (5 mL). After the mixture was stirred at room temperature for 5 minutes, 1-(piperidin-4-yl)-7-(trifluoromethyl)-1H-benzo[d]imidazol-2(3H)-1-one, 2,2,2-trifluoroacetate (150 mg, 0.376 mmol, the synthesis steps could be found in GEN1-013-4) was added to DIPEA (49 mg, 0.38 mmol). The mixture was stirred at room temperature for 4 hours. The reaction mixture was diluted with water (15 mL), and extracted with EtOAc (10 mL×3). The combined organic phase was washed with brine (15 mL×2), dried over anhydrous Na₂SO₄ and concentrated. The residue was purified by preparation. HPLC (prep-3 Xbridge C18 5um 19 * 150 mm, 30%-80%, GT = 10min, B; A: H₂O (0.1% NH₄HCO₃), B: CH₃CN; UV: 214 nm; flow rate: 15 mL/min). The desired compound (44.2 mg, yield 23%) was obtained as a white solid. ¹HNMR (400 MHz, CDCl₃): δ 9.85 (s, 1H), 7.72 (d, *J=* 8.0 Hz, 2H), 7.46 (d, *J=* 8.0 Hz, 2H), 7.32 (d, *J=* 8.0 Hz, 1H), 7.24 (d, *J* = 8.0 Hz, 1H), 7.15-7.11 (m, 1H), 4.96 (br s, 1H), 4.34-4.28 (m, 1H), 3.48 (br s, 1H), 2.71-2.39 (m, 4H), 1.64-1.60 (m, 8H). MS (ESI): Rt = 3.480 min, *m*/*z* 500.2 [M+H]⁺; Purity: 94.57% @ 254 nm, 99.20% @ 214 nm.

### Example 120: 1-(1-(2-(2,3-dihydrobenzo[b] [1,4]dioxin-6-yl)acetyl)piperidin-4-yl)-7-(trifluoromethyl)-1,3-dihydro-2H-benzo[d]imidazol-2-one (GEN1-159)

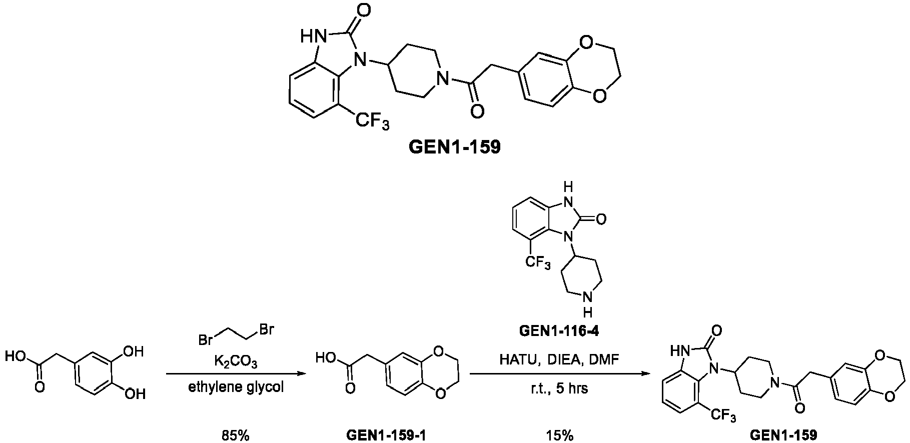

### Step 1. 2-(2,3-dihydrobenzo[b][1,4]dioxacyclohexene-6-yl)acetic acid (GEN1-159-1)

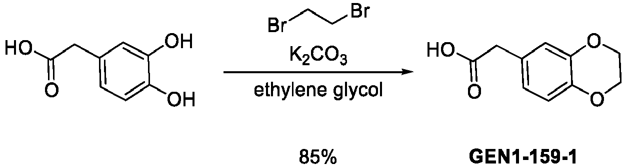

1,2-dibromoethane (1.13 g, 6.0 mmol) was added to a solution of 2-(3,4-dihydroxyphenyl)acetic acid (500 mg, 3.0 mmol) and K₂CO₃ (1.24 g, 9.0 mmol) in ethylene glycol (5.0 mL). The resulting mixture was stirred at 120°C for 4.5 hours under a N₂ atmosphere. The reaction mixture was cooled to room temperature and diluted with water (40 mL). EtOAc (40 mL) was added and the mixture was stirred for 5 minutes. The organic layer was discarded, and the aqueous layer was acidified with 2M HCl to pH <1. The aqueous layer was extracted with EA (60 mL×3). The combined organic layer was washed with water (150 mL), brine (150 mL), dried over Na₂SO₄ and it was concentrated to obtain a black oil. The crude product was purified by silica gel column chromatography and eluted with (EA/(PE + EA)=60%) to obtain the desired compound (495 mg, yield 85%) as a black oil. MS (ESI): Rt = 1.01 min, *m*/*z* 217 [M+Na]⁺, Purity: 100% @ 254 nm, 100% @ 214 nm.

### Step 2. 1-(1-(2-(2,3-dihydrobenzo[b][1,4]dioxin-6-yl)acetyl)piperidin-4-yl)-7-(trifluoromethyl)-1,3-dihydro-2H-benzo[d]imidazol-2-one (GEN1-159)

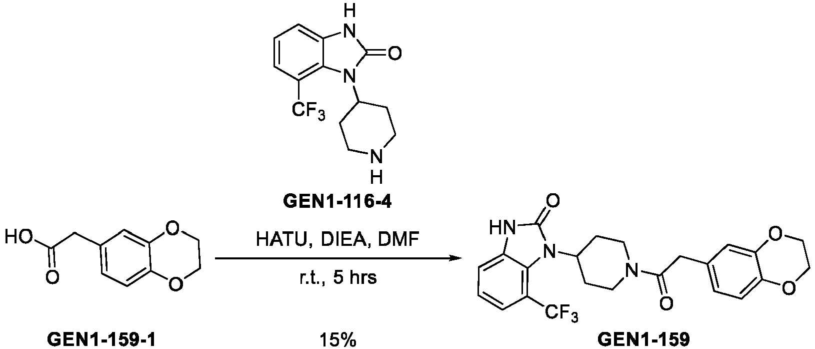

DIEA (45 mg, 0.35 mmol) was added to a solution of 2-(2,3-dihydrobenzo[b][1,4]dioxacyclohexene-6-yl)acetic acid (50 mg, 0.17 mmol), 1-(piperidin-4-yl)-7-(trifluoromethyl)-1,3-dihydro-2H-benzo(d)imidazol-2-one (37 mg, 0.19 mmol) and HATU (100 mg, 0.26 mmol) in DMF (1.0 mL). The resulting mixture was stirred at room temperature for 16 hours. The reaction mixture was added dropwise to water (10 mL). The precipitated solid was collected by filtration. The crude product was purified by reverse-phase flash chromatography (C₁₈ reverse silica gel column; mobile phase CH₃CN, water, 5% to 75% gradient, 40 minutes; detection, UV 214 nm) to obtain the desired compound (12 mg, yield 15%) as a white solid. ¹HNMR (400 MHz, DMSO-*d*₆): δ 11.46 (s, 1H), 7.35 (d, *J=* 7.6 Hz, 1H), 7.27 (d, *J* = 7.7 Hz, 1H), 7.16 (t, *J* = 7.9 Hz, 1H), 6.80-6.72 (m, 2H), 6.71-6.66 (m, 1H), 4.62-4.54 (m, 1H), 4.25-4.19 (m, 5H), 4.17-4.09 (m, 1H), 3.72-3.53 (m, 2H), 2.96 (t, *J=* 13.1 Hz, 1H), 2.57-2.51 (m, 1H), 2.49 (s, 2H), 1.73-1.64 (m, 2H). MS (ESI): Rt = 1.52 min, *m*/*z* 462.4 [M+H]⁺, Purity: 91% @ 254 nm, 100% @ 214 nm.

### Example 121: 1-(4-(2-oxo-7-(trifluoromethyl)-2,3-dihydro-1H-benzo[d]imidazol-1-yl)piperidin-1-yl)-2-(4-(trifluoromethyl)phenyl)1,2-dione (GEN1-160)

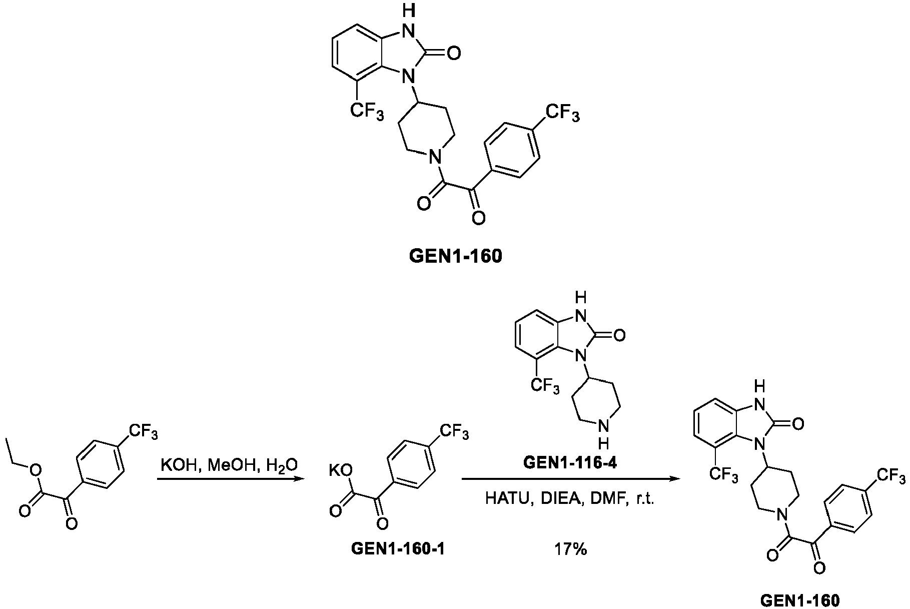

### Step 1. Potassium 2-oxo-2-(4-(trifluoromethyl)phenyl)acetate (GEN1-160-1)

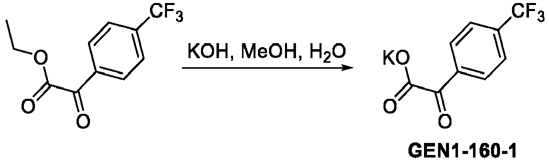

A solution of KOH (24 mg, 0.4 mmol) in water (0.2 mL) was added to a solution of 2-oxo-2-(4-(trifluoromethyl)phenyl) ethyl acetate (100 mg, 0.4 mmol) in MeOH (2.0 mL). The resulting mixture was stirred at room temperature for 4 hours. The resulting mixture was concentrated to obtain the desired compound (58 mg crude product) as a white solid. TLC: MeOH/(MeOH+DCM)=10%, Rf=0.2

### Step 2. 1-(4-(2-oxo-7-(trifluoromethyl)-2,3-dihydro-1H-benzo[d]imidazol-1-yl)piperidin-1-yl)-2-(4-(trifluoromethyl)phenyl)1,2-dione (GEN1-160)

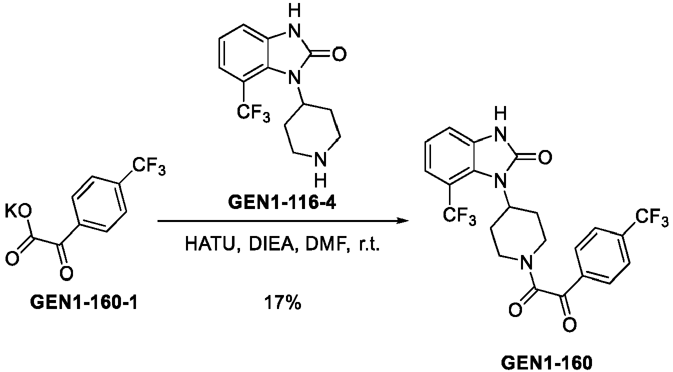

DIEA (45 mg, 0.35 mmol) was added to a solution of 1-(piperidin-4-yl)-7-(trifluoromethyl)-1,3-dihydro-2H-benzo[d]imidazol-2-one(50 mg, 0.17 mmol), potassium 2-oxo-2-(4-(trifluoromethyl)phenyl)acetate (58 mg crude product), EDCI (50 mg, 0.26 mmol) and HOBT (36 mg, 0.26 mmol) in DMF (1.0 mL). The resulting mixture was stirred at room temperature for 16 hours. The reaction mixture was added dropwise to water (20 mL). The precipitated solid was collected by filtration. The crude product was purified by reverse-phase flash chromatography (C₁₈ reverse silica column; mobile phase CH₃CN, water, 5% to 70% gradient, 40 minutes; detection, UV 214 nm) to obtain the desired compound (14.5 mg, yield 17%) as a white solid. ¹H NMR (400 MHz, DMSO-*d*₆): δ 11.52 (s, 1H), 8.13 (d, *J* = 8.1 Hz, 2H), 8.02 (d, *J=* 8.3 Hz, 2H), 7.37 (d, *J=* 8.0 Hz, 1H), 7.29 (d, *J=* 7.7 Hz, 1H), 7.17 (t, *J=* 7.9 Hz, 1H), 4.60 (d, *J=* 13.3 Hz, 1H), 4.41-4.27 (m, 1H), 3.62 (d, *J=* 13.8 Hz, 1H), 3.23 (t, *J=* 12.7 Hz, 1H), 2.94 (t, *J=* 13.0 Hz, 1H), 2.72-2.55 (m, 2H), 1.88 (d, *J* = 12.8 Hz, 1H), 1.71 (d, *J=* 12.7 Hz, 1H). MS (ESI): Rt = 1.64 min, *m*/*z* 486.0 [M+H]⁺, Purity: 100% @ 254 nm, 100% @ 214 nm.

### Example 122: 3-(1-(2-(4-(trifluoromethyl)phenyl)acetyl)piperidin-4-yl)-3,4-dihydroquinazolin-2(1H)-one (GEN1-161)

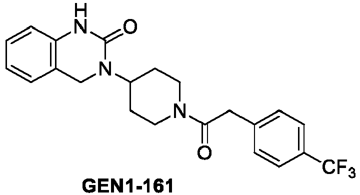

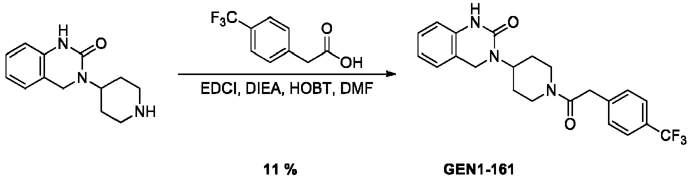

### Step 1. 3-(1-(2-(4-(trifluoromethyl)phenyl)acetyl)piperidin-4-yl)-3,4-dihydroquinazolin-2(1H)-one

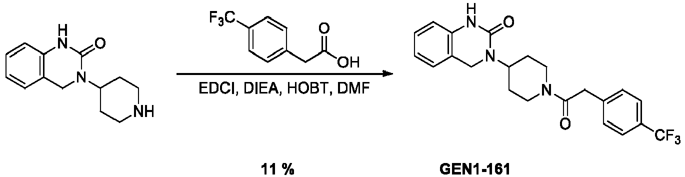

DIEA (1.1 mL) was added to a solution of 3-(piperidin-4-yl)-3,4-dihydroquinazoline-2(1H)-one (50 mg, 0.22 mmol) in DMF (3 mL). After the mixture was stirred at room temperature for 10 minutes, 2-(4-(trifluoromethyl)phenyl)acetic acid (53 mg, 0.26 mmol), EDCI (63 mg, 0.33 mmol) and HOBT (44 mg, 0.33 mmol) were added. The resulting mixture was stirred at room temperature for 4 hours. The reaction mixture was diluted with water (20 mL), and extracted with EtOAc (15 mL×3). The combined organic phase was washed with brine (15 mL×2), dried over anhydrous Na₂SO₄ and concentrated. The residue was purified by a C18 column (CH₃CN: water = 50-70%) to obtain the desired compound (10.3 mg, yield 11%) as a white solid. ¹HNMR (400M, CDCl₃): δ 7.61 (d, *J=* 8.0 Hz, 2H), 7.41 (d, *J=* 8.0 Hz, 2H), 7.18 (t, *J=* 7.6 Hz, 1H), 7.03 (d, *J=* 7.6 Hz, 1H), 6.98-6.94 (m, 1H), 6.66-6.61 (m, 2H), 4.82-4.79 (m, 1H), 4.62-4.53 (m, 1H), 4.28-4.16 (m, 2H), 3.98-3.95 (m, 1H), 3.81 (s, 2H), 3.19-3.12 (m, 1H), 2.70-2.64 (m, 1H), 1.79-1.66 (m, 3H), 1.46-1.42 (m, 1H). MS (ESI): Rt = 3.352 min, m/z 418.2 [M+H]⁺; Purity: 99.11% @254nm, 98.90% @214nm.

### Example 123: 1-(1-(2-(4-(trifluoromethyl)phenyl)acetyl)piperidin-4-yl)-3,4-dihydroquinazolin-2(1H)-one (GEN1-162)

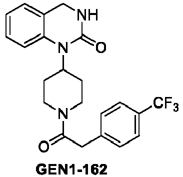

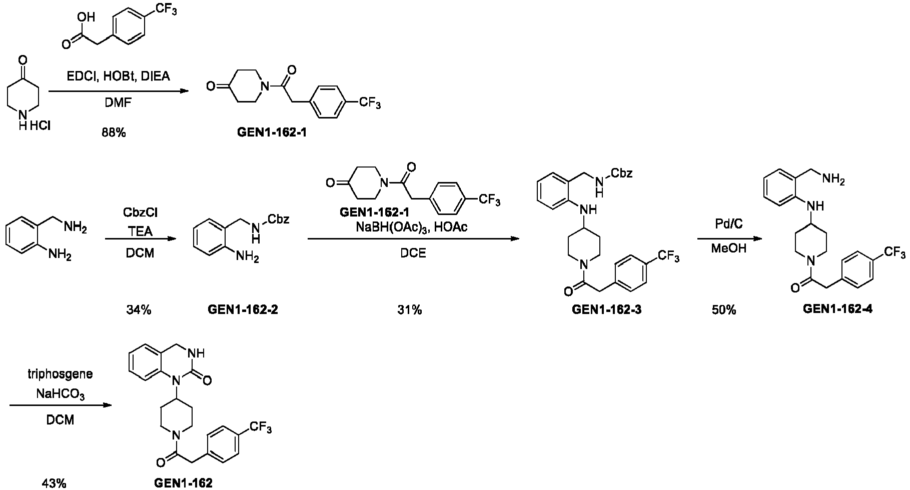

### Step 1. 1-(2-(4-(trifluoromethyl)phenyl)acetyl)piperidin-4-one (GEN1-162-1)

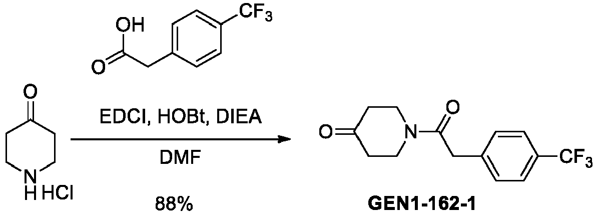

In an ice bath, EDCI (1.06 g, 5.53 mmol) was added to a solution of piperidin-4-one hydrochloride (500 mg, 3.69 mmol), 2-[4-(trifluoromethyl)phenyl]acetic acid (753 mg, 3.69 mmol), HOBT (747 mg, 5.53 mmol), DIEA (2.38 g, 18.4 mmol) in DMF (10 mL). The resulting mixture was stirred overnight. The mixture was poured into water (70 mL), and extracted with EtOAc (30 mL×3). The combined organic layer was washed with water (30 mL), brine (30 mL), dried over MgSO₄ and it was concentrated to obtain the desired compound (1.05 g, yield 88%) as a yellow oil. ¹H NMR (400MHz, CDCl₃): δ 7.61 (d, *J* = 8.0 Hz, 2H), 7.41 (d, *J* = 8.0 Hz, 2H), 3.92 (t, *J=* 6.0 Hz, 2H), 3.87 (s, 2H), 3.76 (t, *J=* 6.0 Hz, 2H), 2.47 (t, *J=* 6.0 Hz, 2H), 2.32 (t, *J* = 6.0 Hz, 2H).

### Step 2. Benzyl 2-aminobenzylamino carbamate (GEN1-162-2)

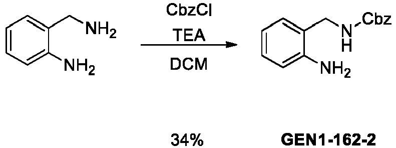

In an ice bath, benzyl phosgene (1.40 g, 8.19 mmol) was added to a solution of 2-(aminomethyl)aniline (1.0 g, 8.2 mmol) and TEA (1.24 g, 12.3 mmol) in DCM (20 mL). The reaction mixture was stirred at room temperature for 2 hours. The reaction mixture was poured into water (50 mL), and extracted with DCM (30 mL×3). The combined organic layer was washed with brine (30 mL), dried over MgSO₄ and it was concentrated to obtain the desired compound (1.35 g, yield 34%) as a pale yellow solid. MS (ESI): Rt = 1.52 min, *m*/*z* 257.3[M+H]⁺, Purity: 51% @ 254 nm, 53% @ 214 nm.

### Step 3. Carbamate 2-(((1-(2-(4-(trifluoromethyl)phenyl)acetyl)piperidin-4-yl)amino)benzylcarbamate (GEN1-162-3)

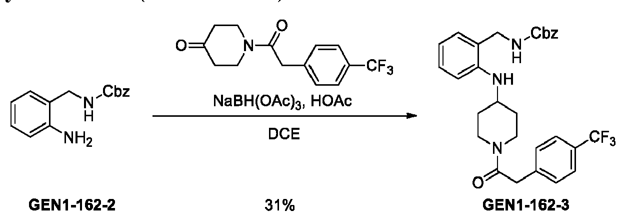

NaBH(OAc)₃ (248 mg, 1.17 mmol) was added to a solution of benzyl 2-aminobenzylamino carbamate (200 mg, 0.780 mmol), 1-(2-(4-(trifluoromethyl)phenyl)acetyl)piperidin-4-one (223 mg, 0.782 mmol) and two drops of HOAc in DCE (5 mL). The resulting mixture was stirred at 65°C overnight. The reaction mixture was poured into water (30 mL), and extracted with DCM (20 mL×3). The combined organic layer was dried over MgSO₄ and concentrated. The crude product was purified by column chromatography on silica gel (petroleum ether: EtOAc = 3:1 to 1:1) to obtain the desired compound (140 mg, yield 31%) as a colorless oil. MS (ESI): Rt = 1.72 min, *m*/*z* 526.6[M+H]⁺, Purity: 90% @ 254 nm, 67% @ 214 nm.

### Step 4. 1-(4-((2-(aminomethyl)phenyl)amino)piperidin-1-yl)-2-(4-(trifluoromethyl)phenyl)ethenone (GEN1-162-4)

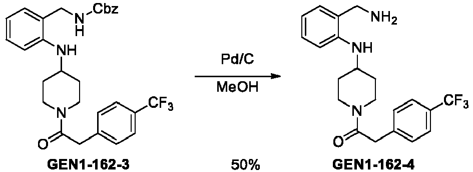

Pd/C (10% purity, 28 mg) was added to a solution of 2-((1-(2-(4-(trifluoromethyl)phenyl)acetyl)piperidin-4-yl)amino)benzylcarbamate (140 mg, 0.266 mmol) in MeOH (4 mL). The resulting mixture was hydrogenated at room temperature for 2 hours. The reaction mixture was filtered, and the filtrate was concentrated to obtain the desired compound (57 mg, yield 50%) as a colorless oil. MS (ESI): Rt = 1.55 min, *m*/*z* 390.5 [M-H]⁻, Purity: 92% @ 254 nm, 73% @214nm.

### Step 5. 1-(1-(2-(4-(trifluoromethyl)phenyl)acetyl)piperidin-4-yl)-3,4-dihydroquinazolin-2(1H)-one (GEN1-162)

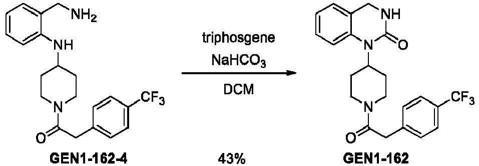

Triphosgene (21 mg, 0.071 mmol) was added to a solution of 1-(4-((2-(aminomethyl)phenyl)amino)piperidin-1-yl)-2-(4-(trifluoromethyl)phenyl)ethenone (57 mg, 0.15 mmol) and NaHCO₃ (61 mg, 0.73 mmol) in DCM (4 mL) in an ice bath. The resulting mixture was stirred at room temperature overnight. The reaction mixture was filtered and the filtrate was concentrated. The residue was purified by a C₁₈ column (CH₃CN: water=5% to 55%) to obtain the desired compound (27 mg, yield 43%) as a white solid. ¹HNMR (400 MHz, CDCl₃): δ 7.61 (d, *J* = 7.6 Hz, 2H), 7.41 (d, *J* = 8.0 Hz, 2H), 7.21 (t, *J* = 7.6 Hz, 1H), 7.08 (d, *J* = 7.2 Hz, 1H), 7.00 (t, J= 7.2 Hz, 1H), 6.90 (d, *J=* 8.4 Hz, 1H), 4.93 (s, 1H), 4.85-4.82 (m, 1H), 4.28 (s, 2H), 4.24-4.15 (m, 1H), 4.02-3.98 (m, 1H), 3.83 (s, 2H), 3.15-3.08 (m, 1H), 2.70-2.44 (m, 3H), 1.88-1.80 (m, 2H). MS (ESI): Rt = 3.996 min, *m*/*z* 418.2 [M+H]⁺, Purity: 98.59% @ 254 nm, 96.85% @ 214 nm.

### Example 124: 1-(pyridin-2-yl)-3-(1-(2-(4-(trifluoromethyl)phenyl)acetyl)piperidin-4-yl)urea (GEN1-163)

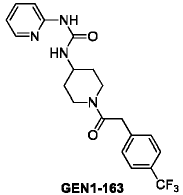

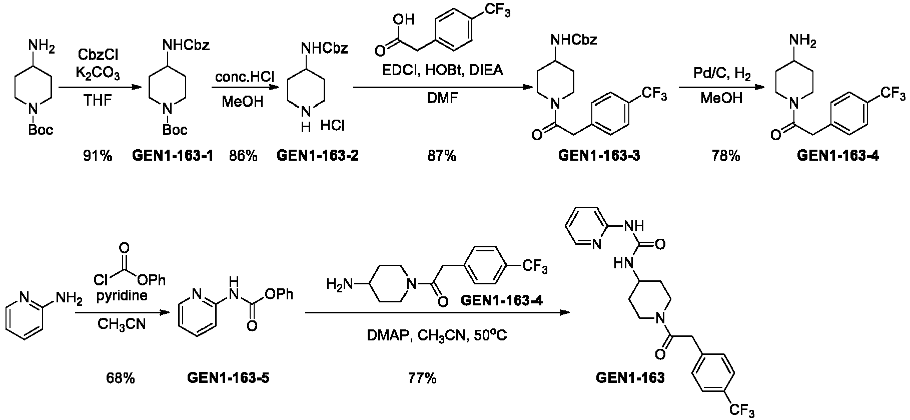

### Step 1. Tert-butyl 4-((((benzyloxy)carbonyl)amino)piperidin-1-carboxylate (GEN1-163-1)

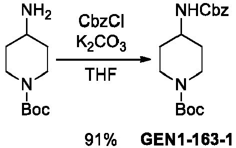

Carbo-benzyl chloride (2.80 g, 16.5 mmol) was added dropwise to a solution of tert-butyl 4-aminopiperidine-1-carboxylate (3.00 g, 15.0 mmol) and Na₂CO₃ (2.40 g, 22.6 mmol) in THF (30 mL) at room temperature. After it was stirred at room temperature for 1 hour, the reaction mixture was concentrated, and the brown oily residue was diluted with water (50 mL). The aqueous solution was extracted with EtOAc (30 mL×2). The combined organic phase was washed with saturated aqueous NaHCO₃ solution, dried over anhydrous sodium sulfate and concentrated to obtain the desired compound (4.9 g, yield 91%) as a white solid. LCMS (ESI): Rt = 1.63 min, *m*/*z* 235.2 [M+H-Boc]⁺, Purity: 28% @ 254 nm, 93% @ 214 nm.

### Step 2. Benzylpiperidin-4-yl carbamate hydrochloride (GEN1-163-2)

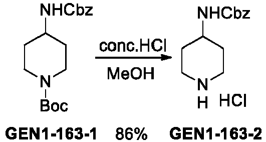

Concentrated HCl (5 mL) was added dropwise to a solution of tert-butyl 4-((((benzyloxy)carbonyl)amino)piperidin-1-carboxylate (4.90 g, 14.8 mmol) in MeOH (40 mL) at room temperature. After it was stirred overnight at room temperature, the mixture was concentrated to obtain the desired compound (3.6 g, yield 86%) as a white solid. ¹H NMR (400 MHz, DMSO-*d*₆): δ 8.82 (br s, 1H), 8.60 (br s, 1H), 7.52 (d, *J* = 7.2 Hz, 1H), 7.39-7.28 (m, 5H), 5.02 (s, 2H), 3.67-3.54 (m, 1H), 3.23-3.20 (m, 2H), 2.98-2.90 (m, 2H), 1.93-1.88 (m, 2H), 1.64-1.54 (m, 2H).

### Step 3. benzyl(1-(2-(4-(tris(trifluoromethyl)phenyl)acetyl)piperidin-4-yl)carbamate (GEN1-163-3)

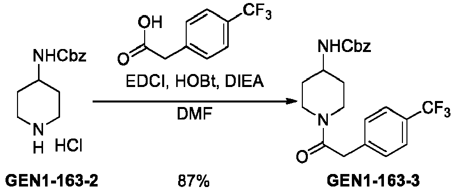

At room temperature, DIEA (1.19 g, 9.23 mmol) was added dropwise to a solution of benzylpiperidin-4-yl carbamate hydrochloride (500 mg, 1.85 mmol) and HOBT (374 mg, 2.77 mmol) in DMF (30 mL). After the mixture was stirred for 20 minutes, 2-(4-(trifluoromethyl)phenyl)acetic acid (415 mg, 2.03 mmol) was added in portions. The resulting mixture was stirred at room temperature overnight. The reaction mixture was diluted with water (50 mL), and extracted with EtOAc (30 mL×2). The combined organic phase was dried with anhydrous sodium sulfate and concentrated to obtain the desired compound (800 mg, yield 87%) as a yellow oil. LCMS (ESI): Rt = 1.66 min, *m*/*z* 421.4 [M+H]⁺; Purity: 97% @ 254 nm, 84% @ 214 nm.

### Step 4. 1-(4-aminopiperidin-1-yl)-2-(4-(trifluoromethyl)phenyl)ethenone (GEN1-163-4)

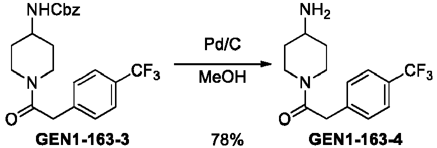

Benzyl(1-(2-(4-(tris(trifluoromethyl)phenyl)acetyl)piperidin-4-yl)carbamate (500 mg, 0.999 mmol) and Pd/C (10% purity, 53 mg) were dissolved in a solution of MeOH (5 mL). The mixture was hydrogenated at room temperature overnight. The reaction mixture was filtered, and the filtrate was concentrated to obtain the desired compound (380 mg, yield 78%) as a white solid. ¹H NMR (400 MHz, CDCl₃): δ 7.58 (d, *J* = 8.0 Hz, 2H), 7.37 (d, *J* = 8.0 Hz, 2H), 4.52-4.49 (m, 1H), 3.83-3.75 (m, 3H), 3.09-3.02 (m, 1H), 2.93-2.86 (m, 1H), 2.79-2.72 (m, 1H), 1.92-1.75 (m, 2H), 1.30-1.20 (m, 2H).

### Step 5. Phenylpyridin-2-yl carbamate (GEN1-163-5)

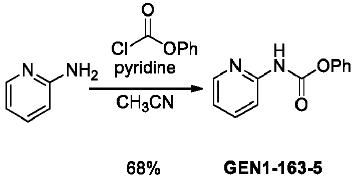

In an ice bath, phosgene phenyl ester (832 mg, 5.31 mmol) was added dropwise to a solution of pyridin-2-amine (500 mg, 5.31 mmol) and pyridine (630 mg, 7.96 mmol) in CH₃CN (10 mL). The resulting white suspension was stirred at 0°C for 1 hour. The reaction mixture was poured into water (70 mL), and extracted with EtOAc (40 mL×3). The combined organic layer was washed with water (40 mL), brine (40 mL), dried over MgSO₄ and filtered. The filtrate was concentrated to obtain the desired compound (910 mg, yield 68%) as a white solid. ¹HNMR (400 MHz, CDCl₃): δ 9.94 (s, 1H), 8.42 (d, *J* = 7.2 Hz, 1H), 8.07 (d, *J* = 8.8 Hz, 1H), 7.75-7.71 (m, 1H), 7.45-7.41 (m, 2H), 7.29-7.22 (m, 4H), 7.04-7.01 (m, 1H).

### Step 6. 1-(pyridin-2-yl)-3-(1-(2-(4-(trifluoromethyl)phenyl)acetyl)piperidin-4-yl)urea (GEN1-163)

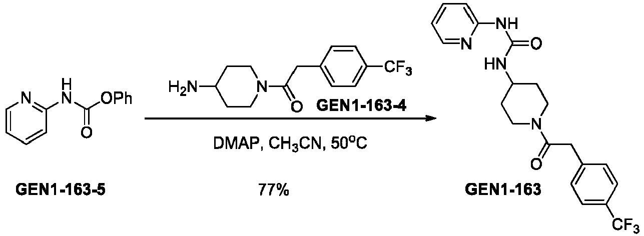

A solution of phenyl N-(2-pyridyl)carbamate (100 mg, 0.467 mmol), 1-(4-aminopiperidin-1-yl)-2-(4-(trifluoromethyl)phenyl)ethenone (134 mg, 0.468 mmol), DMAP (58 mg, 0.47 mmol) were dissolved in a solution of CH₃CN (4 mL). The mixture was stirred at 50°C for 5 hours. The reaction mixture was concentrated and the residue was purified by a C₁₈ column (CH₃CN: water=5% to 55%) to obtain the desired compound (148.1 mg, yield 77%) as a white solid. ¹H NMR (400 MHz, CDCl₃): δ 9.55 (d, *J* = 5.6 Hz, 1H), 8.14 (d, *J* = 5.2 Hz, 1H), 8.05-7.78 (m, 1H), 7.60-7.57 (m, 3H), 7.39 (d, *J=* 8.0 Hz, 2H), 6.90-6.87 (m, 1H), 6.73-6.71 (m, 1H), 4.39-4.35 (m, 1H), 4.09-3.97 (m, 1H), 3.86-3.75 (m, 3H), 3.29-3.22 (m, 1H), 3.11-3.04 (m, 1H), 2.05-1.99 (m, 2H), 1.59-1.49 (m, 1H), 1.43-1.34 (m, 1H). LCMS (ESI): Rt = 3.681 min, *m*/*z* 407.1 [M+H]⁺; Purity: 99.52% @ 254 nm, 99.03% @ 214 nm.

### Example 125: 1-(pyridin-2-yl)-1-(1-(2-(4-(trifluoromethyl)phenyl)acetyl)piperidin-4-yl)urea

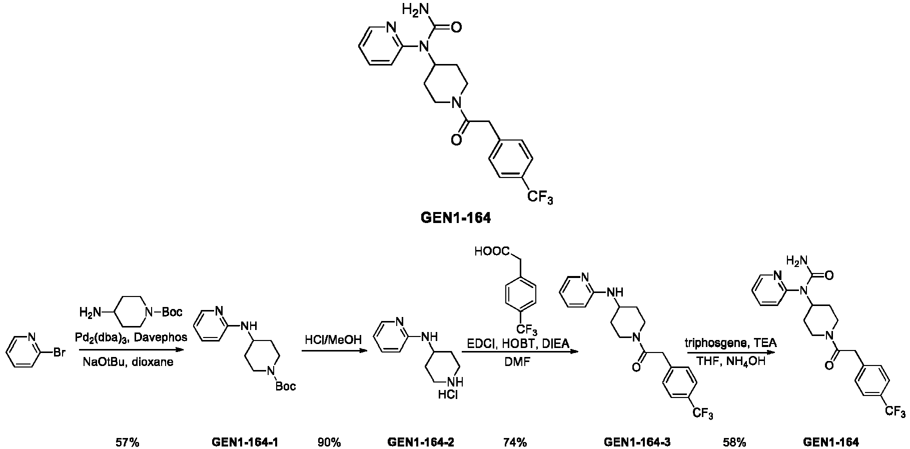

### Step 1. Synthesis of tert-butyl 4-(pyridin-2-ylamino)piperidin-1-carboxylate

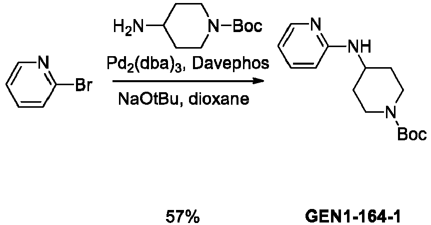

Pd2(dba)3 (517 mg, 0.500 mmol) was added to a mixture of 2-bromopyridine (1.18 g, 7.49 mmol), tert-butyl 4-aminopiperidine-1-carboxylate (1.00 g, 4.99 mmol), Davephos (393 mg, 1.00 mmol) and NaOtBu (1.44 g, 15.0 mmol) in dioxane (20mL). The mixture was stirred at 90°C under nitrogen overnight. The resulting mixture was concentrated, and water (50 mL) was added. Then DCM (100 mL×3) was added to extract the desired compound. The combined organic layer was washed with brine, dried over anhydrous Na₂SO₄ and it was concentrated. The residue was purified by a C18 column (50-70% B; A: H₂O B: CH₃CN) to obtain the desired compound (794 mg, yield 57%) as an ocher solid. MS (ESI): Rt = 1.54 min, *m*/*z* 278.3 [M+H]⁺; Purity: 100% @ 254 nm, 100% @ 214 nm.

### Step 2. Synthesis of N-(piperidin-4-yl)pyridin-2-amine hydrochloride

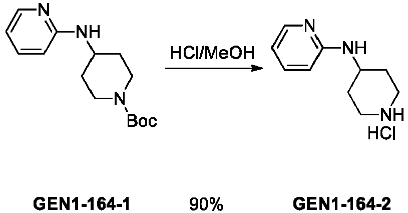

A suspension of tert-butyl 4-(pyridin-2-ylamino)piperidin-1-carboxylate (665 mg, 2.40 mmol) in HCl/MeOH (15 mL, 4M) was stirred for 2 hours at room temperature. The resulting mixture was concentrated to obtain the desired compound (638 mg, yield 90%) as a yellow solid. MS (ESI): Rt = 0.28 min, *m*/*z* 178.1 [M+H]⁺; Purity: 85% @ 254 nm, 42% @ 214 nm.

### Step 3. Synthesis of 1-(4-(pyridin-2-ylamino)piperidin-1-yl)-2-(4-(trifluoromethyl)phenyl)ethenone

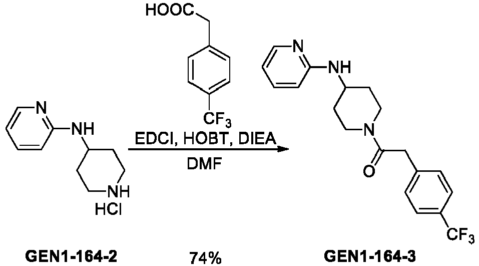

2-(4-(trifluoromethyl)phenyl)acetic acid (122 mg, 0.598 mmol) was added to a solution of N-(piperidin-4-yl)pyridin-2-amine hydrochloride (100 mg, 0.400 mmol), DIEA (310 mg, 2.40 mmol) ), EDCI (138 mg, 0.720 mmol) and HOBT (97 mg, 0.72 mmol) in DMF (2 mL). The resulting mixture was stirred at room temperature overnight. Then the mixture was purified by a C18 column (50-70% B; A: H₂O B: CH₃CN) to obtain the desired compound (107 mg, yield 74%) as a light brown solid. MS (ESI): Rt = 1.54 min, *m*/*z* 364.4 [M+H]⁺; Purity: 100% @ 254 nm, 100% @ 214 nm.

### Step 4. Synthesis of 1-(pyridin-2-yl)-1-(1-(2-(4-(trifluoromethyl)phenyl)acetyl)piperidin-4-yl)urea

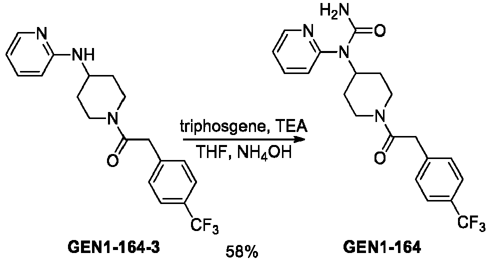

Triphosgene (262 mg, 883 umol) was added to a solution of 1-(4-(pyridin-2-ylamino) piperidin-1-yl)-2-(4-(trifluoromethyl)phenyl)ethenone (107 mg, 0.294 mmol) and TEA (179 mg, 1.77 mmol) in THF (2 mL) in a dry ice acetone bath. The mixture was stirred for 15 minutes at -65°C, and then warmed to room temperature and stirred for 3 hours. The mixture was concentrated and aqueous NH₄OH solution (2 mL, 28% purity) was added. The mixture was stirred overnight at room temperature, and then concentrated and the residue was purified by preparation. HPLC (prep-1 Xbridge C18 5um 19 * 150mm, 10-60%B; A: H₂O(0.1%NH₄HCO₃), B: CH₃CN; 254 nm; flow rate: 15 mL / min). The desired compound (70 mg, yield 58%) was obtained as a gray solid. ¹HNMR (400 MHz, DMSO-*d*₆): δ 8.44 (dd, *J* = 4.8, 1.6 Hz, 1H), 7.80 (td, *J* = 7.6,2.0 Hz, 1H), 7.61 (d, *J* = 8.0 Hz, 2H), 7.38 (d, *J* = 8.0 Hz, 2H), 7.30-7.27 (m, 1H), 7.16 (d, *J* = 7.6 Hz, 1H), 5.80 (s, 2H), 4.41 (d, *J* = 12.8 Hz, 1H), 4.36-4.28 (m, 1H), 3.94 (d, *J **=*** 14.0 Hz, 1H), 3.82-3.73 (m, 2H), 3.05-2.99 (m, 1H), 2.58-2.50 (m, 1H), 1.75 (d, *J =* 11.2 Hz, 1H), 1.66 (d*, J =* 12.4 Hz, 1H), 1.41-1.31 (m, 1H), 1.29-1.19 (m, 1H). MS (ESI): Rt = 2.723 min, *m*/*z* 407.2 [M+H]⁺; Purity: 99.52% @ 254 nm, 99.11% @ 214 nm.

### Example 126: 4-methyl-5-phenyl-1-(1-(2-(4-(trifluoromethyl)phenyl)acetyl)piperidin-4-yl)-1H-imidazole-2(3H)-one (GEN1-166)

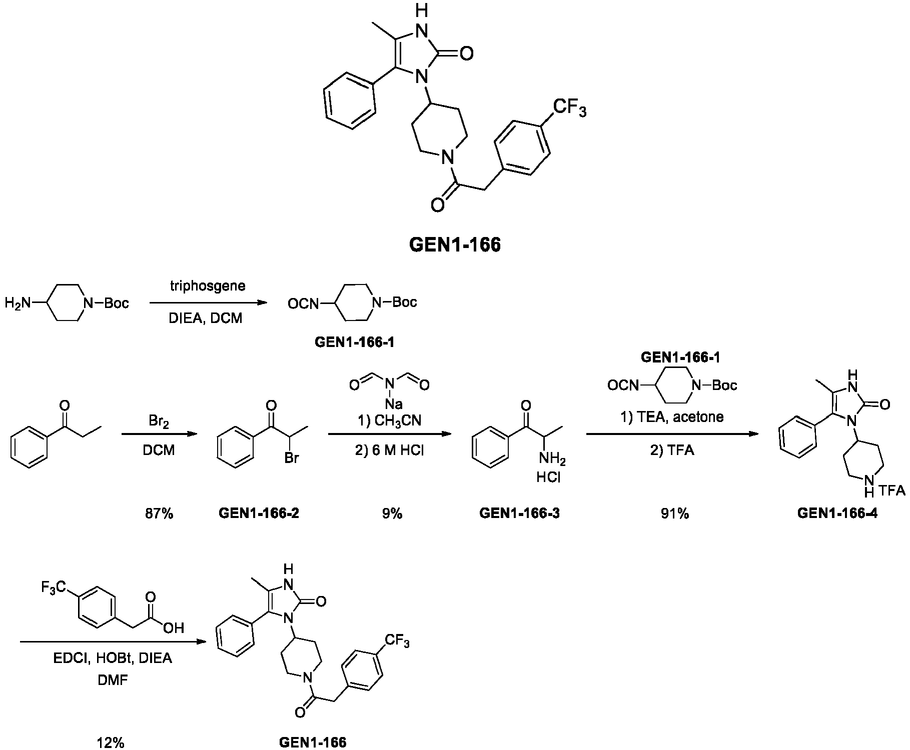

### Step 1. Tert-butyl 4-isocyanatopiperidine-1-carboxylate (GEN1-166-1)

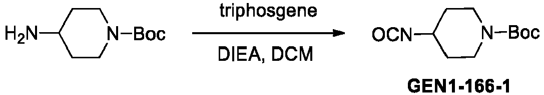

In an ice bath, a solution of triphosgene (296 mg, 0.997 mmol) in DCM (8 mL) was added dropwise to a solution of tert-butyl 4-aminopiperidine-1-carboxylate (500 mg, 2.50 mmol) and DIEA (807 mg, 6.24 mmol) in DCM (7 mL). The reaction mixture was stirred at room temperature for 3 hours. The reaction mixture was concentrated to obtain the desired compound (1.2 g crude product), which was used in the next step without further purification.

### Step 2. 2-bromo-1-phenylpropan-1-one (GEN1-166-2)

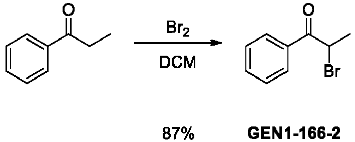

Br₂ (5.96 g, 37.3 mmol) was added dropwise to a solution of isopropiophenone (5.00 g, 37.3 mmol) in DCM (80 mL) in an ice bath. The resulting mixture was stirred at room temperature for 3 hours. The reaction mixture was poured into water (100 mL), and extracted with DCM (50 mL×3). The combined organic layer was washed with aqueous NaHCO₃ (10%, 50 mL), brine (50 mL), dried over MgSO₄ and filtered. The filtrate was concentrated to obtain the desired compound (7.7 g, yield 87%) as a light brown oil. ¹H NMR (400 MHz, CDCl₃): δ 8.04-8.02 (m, 2H), 7.61-7.58 (m, 1H), 7.51-7.47 (m, 2H), 5.30 (q, *J =* 6.4 Hz, 1H), 1.91 (d, *J =* 6.4 Hz, 3H).

### Step 3. 2-amino-1-phenylpropan-1-one hydrochloride (GEN1-166-3)

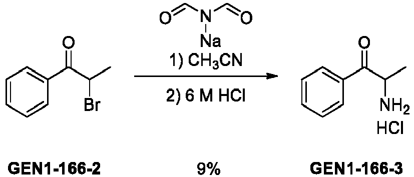

Sodium dimethylformamide (535 mg, 5.63 mmol) was added to a solution of 2-bromo-1-phenylpropan-1-one (1.00 g, 4.70 mmol) in CH₃CN (10 mL). The reaction mixture was stirred at 70°C overnight. The reaction mixture was concentrated, and the residue was dissolved in 6M HCl (10 mL). The reaction mixture was stirred at 100°C for 1 hour, and then concentrated. The crude product was purified by recrystallization in i-PrOH to obtain the desired compound (80 mg, yield 9%) as a white solid. ¹HNMR (400 MHz, DMSO-*d*₆): δ 8.58 (br s, 3H), 8.08-8.05 (m, 2H), 7.76-7.72 (m, 1H), 7.62-7.58 (m, 2H), 5.12 (q, *J* = 7.2 Hz, 1H), 1.44 (d, *J* = 7.2 Hz, 3H).

### Step 4 4-methyl-5-phenyl-1-(piperidin-4-yl)-1H-imidazol-2(3H)-one 2,2,2-trifluoroacetate (GEN1-166-4)

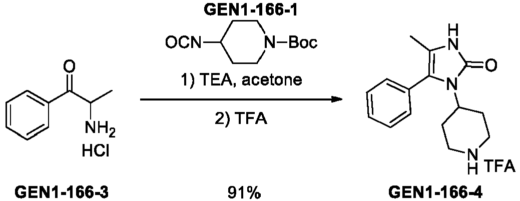

Tert-butyl 4-isocyanatopiperidine-1-carboxylate (117 mg, 0.518 mmol) and TEA (52 mg, 0.51 mmol) were added to a suspension of 2-amino-1-phenylpropan-1-one hydrochloride (80 mg, 0.43 mmol) in acetone (5 mL). After the mixture was stirred at room temperature for 2 hours, TFA (1 mL) was added. After the reaction mixture was stirred overnight at room temperature, and then poured into water (50 mL), and extracted with EtOAc (30 mL×3). The combined organic layer was washed with brine (30 mL), dried over MgSO₄, filtered and it was concentrated to obtain the desired compound (160 mg, yield 91%) as a brown oil. LCMS (ESI): Rt = 1.25 min, *m*/*z* 258.2 [M+H]⁺; Purity: 100% @ 254 nm, 91% @ 214 nm.

### Step 5. 4-methyl-5-phenyl-1-(1-(2-(4-(trifluoromethyl)phenyl)acetyl)piperidin-4-yl)-1H-imidazole-2(3H)-one (GEN1-166)

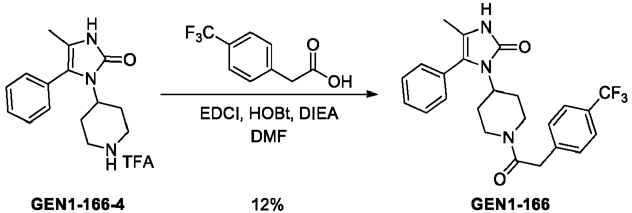

In an ice bath, EDCI (124 mg, 0.65 mmol) was added to a solution of 4-methyl-5-phenyl-1-(piperidin-4-yl)-1H-imidazol-2(3H)-one 2,2,2-trifluoroacetate (160 mg, 0.431 mmol), 2-4-(trifluoromethyl)phenyl)acetic acid (97 mg, 0.48 mmol), HOBt (87 mg, 0.64 mmol) and DIEA (167 mg, 1.29 mmol) in DMF (5 mL). The reaction mixture was stirred overnight at room temperature, and then poured into water (50 mL), and extracted with EtOAc (30 mL×3). The combined organic layer was washed with brine (30 mL), dried over MgSO₄, filtered and it was concentrated. The crude product was purified by C₁₈ (CH₃CN: water = 5% to 45%). Then, the desired compound (22.1 mg, yield 12%) was obtained as a white solid by preparative TLC (EtOAc:MeOH=15:1). ¹HNMR (400 MHz, DMSO-*d*₆): δ 10.12 (s, 1H), 7.65 (d, *J* = 8.4Hz, 2H), 7.47-7.35 (m, 5H), 7.26 (d, *J =* 8.4 Hz, 2H), 4.44-4.41 (m, 1H), 4.01-3.97 (m, 1H), 3.87-3.77 (m, 2H), 3.67-3.58 (m, 1H), 2.90-2.84 (m, 1H), 2.42-2.18 (m, 3H), 1.85 (s, 3H), 1.67-1.55 (m, 2H). LCMS (ESI): Rt = 3.939 min, *m*/*z* 444.2 [M+H]⁺; Purity: 98.54% @ 254 nm, 98.86% @ 214 nm.

### Example 127: 1'-(2-(4-(trifluoromethyl)phenyl)acetyl)spiro[indoline-3,4'-piperidine]-2-one (GEN1-167)

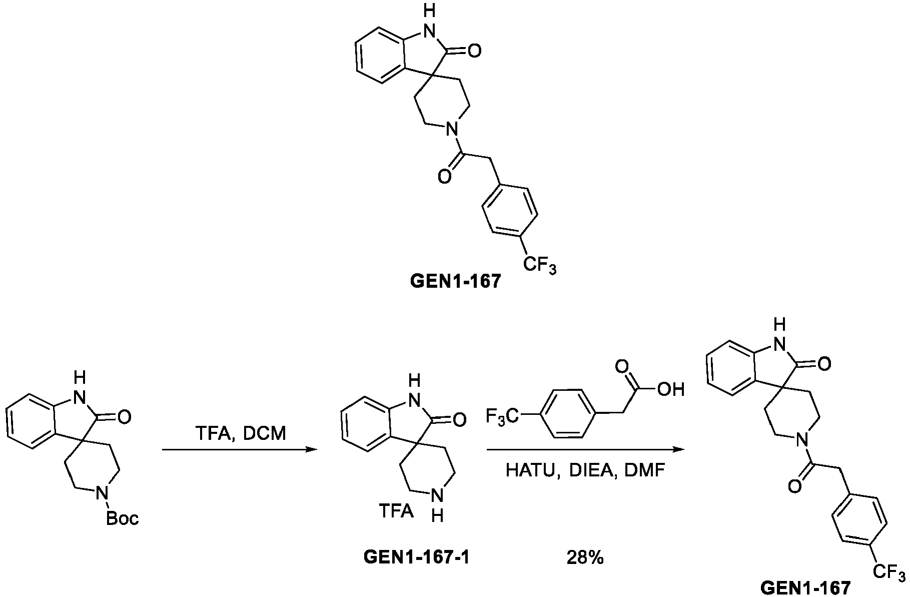

### Step 1. Spiro[indoline-3,4'-piperidin]-2-one 2,2,2-trifluoroacetate (GEN1-167-1)

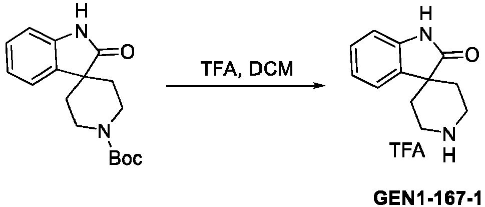

TFA (0.25 mL) was added dropwise to a solution of tert-butyl 2-oxospiro[indoline-3,4'-piperidine]-1'-carboxylate (100 mg, 0.33 mmol) in DCM (5.0 mL) at 0°C. The resulting mixture was stirred at room temperature for 4 hours. The resulting mixture was concentrated. The desired compound (110 mg crude product) was obtained and used directly in the next step without further purification. MS (ESI): Rt = 1.67 min, *m*/*z* 203.0 [M + H]⁺ , Purity: 100% @ 254 nm, 100% @ 214 nm.

### Step 2. 1'-(2-(4-(trifluoromethyl)phenyl)acetyl)spiro[indoline-3,4'-piperidine]-2-one (GEN1-167)

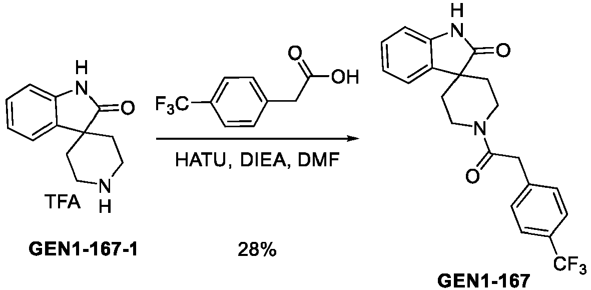

DIEA (170 mg, 1.32 mmol) was added to a solution of spiro[indoline-3,4'-piperidin]-2-one 2,2,2-trifluoroacetate (110 mg crude product), 2-(4-(trifluoromethyl)phenyl)acetic acid (81 mg, 0.40 mmol), EDCI (95 mg, 0.49 mmol), HOBT (67 mg, 0.49 mmol) in DMF (2.0 mL). The resulting mixture was stirred at room temperature for 16 hours. The reaction mixture was poured into water (15 mL), and extracted with EA (16 mL×3). The combined organic layer was washed with water (30 mL), brine (40 mL), dried over Na₂SO₄ and it was concentrated to obtain a yellow oil. The residue was purified by reverse-phase flash chromatography (C₁₈ reverse silica column; mobile phase CH₃CN, water, 20% to 70% gradient, 50 minutes; detection, UV 214nm) to obtain the desired compound (26.8 mg, yield 28%) as a white solid. ¹H NMR (400 MHz, DMSO-d₆): δ 10.44 (s, 1H), 7.70 (d, *J =* 8.0 Hz, 2H), 7.50 (d, *J =* 8.0 Hz, 2H), 7.37 (d, *J =* 7.4 Hz, 1H), 7.19 (t, *J* = 7.7, 1H), 6.95 (t, *J =* 7.6, 1H), 6.85 (d, *J =* 7.7 Hz, 1H), 3.99-3.84 (m, 4H), 3.83-3.70 (m, 2H), 1.75-1.57 (m, 4H). MS (ESI): Rt = 1.51 min, *m*/*z* 388.9 [M + H]⁺ , Purity: 100% @ 254 nm, 100% @ 214 nm.

### Example 128: 1-(1-(2-(3,4-dimethoxyphenyl)acetyl)piperidin-4-yl)-7-(trifluoromethyl)-1,3-dihydro-2H-benzo[d]imidazol-2-one (GEN1-168)

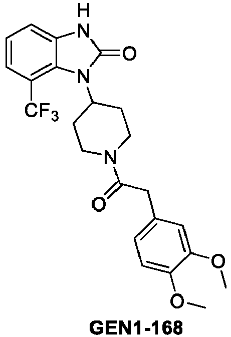

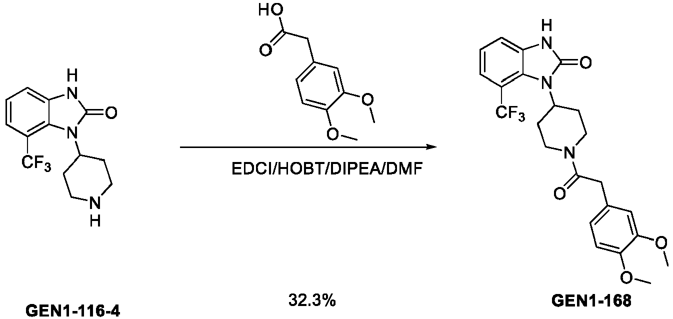

### Step 1. Synthesis of 1-(1-(2-(3,4-dimethoxyphenyl)acetyl)piperidin-4-yl)-7-(trifluoromethyl)-1,3-dihydro-2H-benzo[d]imidazol-2-one (GEN1-168)

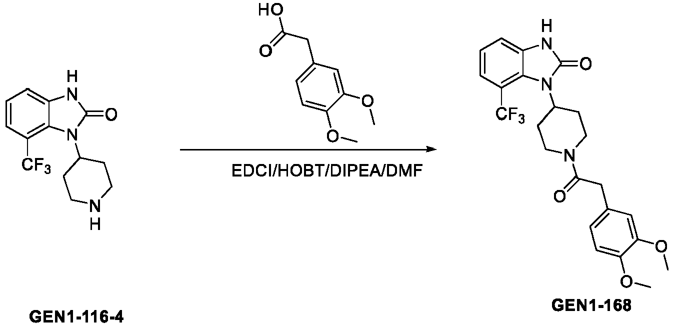

EDCI (57.5 mg, 0.3 mmol) was added to a solution of 1-(piperidin-4-yl)-7-(trifluoromethyl)-1,3-dihydro-2H-benzo[d]imidazol-2-one (57 mg, 0.2 mmol, the synthesis steps could be found in GEN1-116-4), 2-(3,4-dimethoxyphenyl)acetic acid (47 mg, 0.24 mmol), HOBT (40.5 mg, 0.3 mmol) and DIEA (51.6 mg, 0.4 mmol) in DMF (1 mL). The resulting mixture was stirred at room temperature for 1 h. The reaction mixture was diluted with water (30 mL), and extracted with EtOAc (20 mL×3). The combined organic phase was washed with brine (30 mL), dried over anhydrous Na₂SO₄ and filtered. The filtrate was concentrated to obtain a crude product. The crude product was purified by reverse phase chromatography. Purification conditions: C18 silica gel column; mobile phase ACN: H₂O, gradient: 5% to 50%; 45 minutes, monitoring wavelength: 214 nm. The desired compound (30 mg, yield 32.3%) was obtained as a white solid. ¹H NMR (400 MHz, Chloroform-d) δ 9.60 (s, 1H), 7.39 (d, J = 8.1 Hz, 1H), 7.29 (d, J = 7.8 Hz, 1H), 7.14 (t, J = 8.0 Hz, 1H), 6.86-6.76 (m, 3H), 4.90 (d, J = 13.1 Hz, 1H), 4.46 - 4.35 (m, 1H), 4.07 (d, J = 13.7 Hz, 1H), 3.90 (s, 3H), 3.85 (s, 3H), 3.75 (s, 2H), 3.03 (t, J = 13.1 Hz, 1H), 2.84 - 2.55 (m, 3H), 1.87-1.72 (m, 2H). MS (ESI): Rt = 1.47 min, m/z 464.2, [M + H]⁺; Purity: 100% @ 254 nm.

### Example 129: 1-(1-(1-(2-(1H-indol-3-yl)acetyl)piperidin-4-yl)-7-(trifluoromethyl)-1,3-dihydro-2H-benzo[d]imidazol-2-one (GEN1-169)

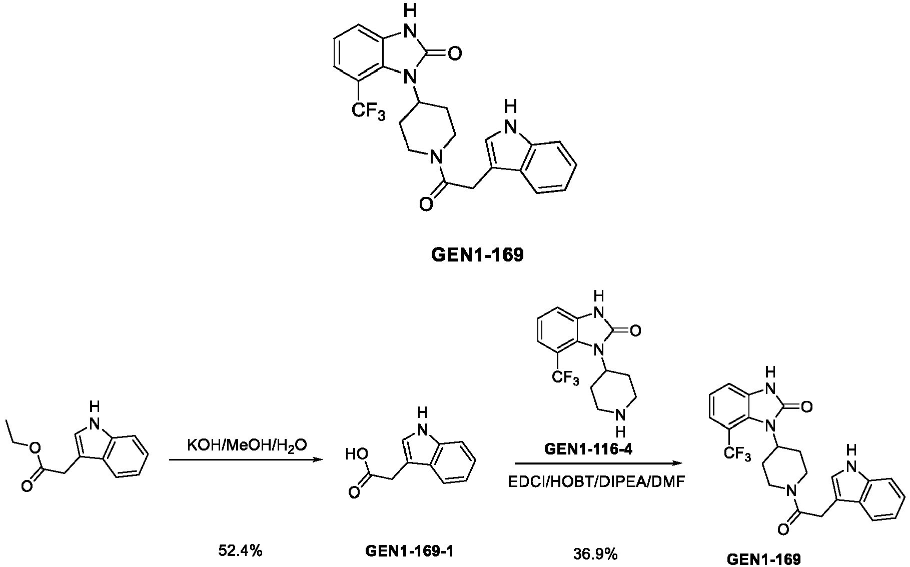

### Step 1. Synthesis of 2-(1H-indol-3-yl)acetic acid (GEN1-218-1)

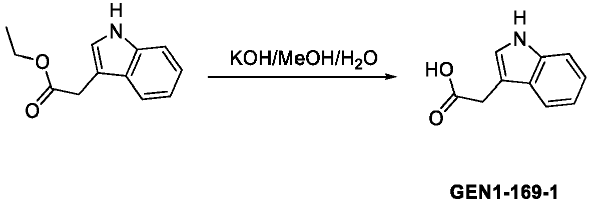

KOH (41.2 mg, 0.735 mmol) was added to a suspension of 2-(1H-indol-3-yl)ethyl acetate (100mg, 0.49mmol) in MeOH (5ml), H₂O (5ml) at room temperature. The resulting mixture was stirred at room temperature for 3h. The reaction mixture was diluted with water (30 mL), and the pH was adjusted to 4 with IN HCl. Then the mixture was extracted with EtOAc (30 mL×3). The combined organic phase was washed with brine (30 mL), dried over anhydrous Na₂SO₄ and filtered. The filtrate was concentrated to obtain the desired compound (45 mg, yield 52.4%) as a white solid. MS (ESI): Rt = 0.26 min, m/z 176.2, [M + H]⁺;

### Step 2. Synthesis of 1-(1-(1-(2-(1H-indol-3-yl)acetyl)piperidin-4-yl)-7-(trifluoromethyl)-1,3-dihydro-2H-benzo[d]imidazol-2-one (GEN1-169)

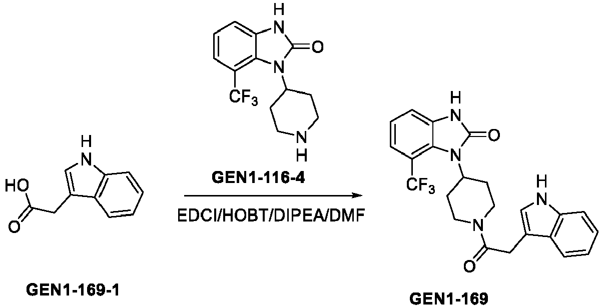

EDCI (50.4 mg, 0.263 mmol) was added to a solution of 1-(piperidin-4-yl)-7-(trifluoromethyl)-1,3-dihydro-2H-benzo[d]imidazol-2-one (50 mg, 0.175 mmol, the synthesis steps could be found in GEN1-116 -4), 2-(1H-indol-3-yl)acetic acid (44.8 mg, 0.21 mmol), HOBT (35.5 mg, 0.263 mmol) and DIEA (45.2 mg, 0.35 mmol) in DMF (1 mL). The resulting mixture was stirred at room temperature for 1 h. The reaction mixture was diluted with water (30 mL), and extracted with EtOAc (20 mL×3). The combined organic phase was washed with brine (30 mL), dried over anhydrous Na₂SO₄ and filtered. The filtrate was concentrated to obtain a crude product. The crude product was purified by reverse phase chromatography. Purification conditions: C18 silica gel column; mobile phase ACN: H₂O, gradient: 5% to 50%; 45 minutes, monitoring wavelength: 214 nm. The desired compound (28.6 mg, yield 36.9%) was obtained as a white solid. ¹H NMR (400 MHz, DMSO-d6) δ 11.5-11.34(m,1H), 10.91 (s, 1H), 7.57 (d, J = 7.9 Hz, 1H), 7.41-7.31 (m, 2H), 7.28 (d, J = 7.6 Hz, 1H), 7.24-7.20 (m, 1H), 7.19-7.12 (m, 1H), 7.07 (t, J = 7.5 Hz, 1H), 6.97 (t, J = 7.4 Hz, 1H), 4.70-4.54 (m, 1H), 4.30-4.16 (m, 2H), 3.92 - 3.70 (m, 2H), 2.98 (t, J = 13.0 Hz, 2H), 2.71-2.56 (m, 1H), 2.47-2.31 (m, 2H), 1.74-1.64 (m, 2H). MS (ESI): Rt = 1.50 min, m/z 443.2, [M + H]⁺; Purity: 100% @ 254 nm.

### Example 130: (E)-N-(1-(1-(1-(2-(quinolin-6-yl)acetyl)piperidin-4-yl)-7-(trifluoromethyl)-1,3-dihydro-2H-benzo[d]imidazol-2-propylene)acetamide (GEN1-171)

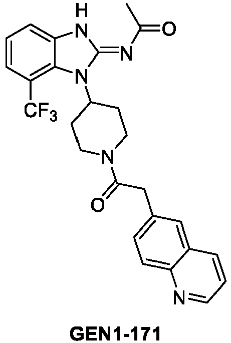

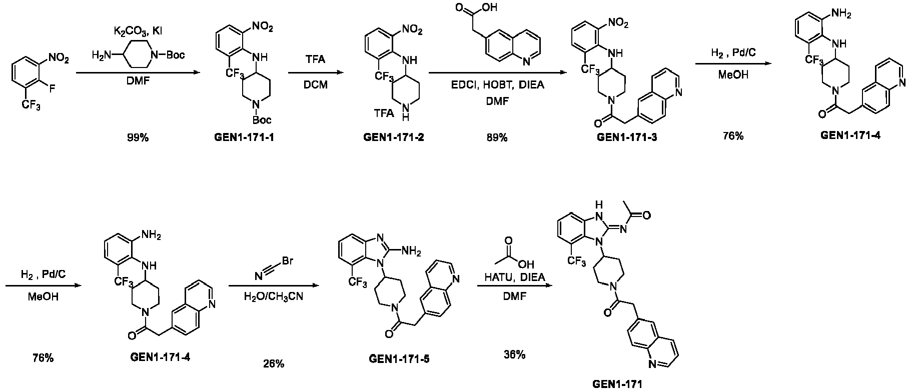

### Step 1. tert-butyl 4-(((2-nitro-6-(trifluoromethyl)phenyl)amino)piperidin-1-carboxylate (GEN1-171-1)

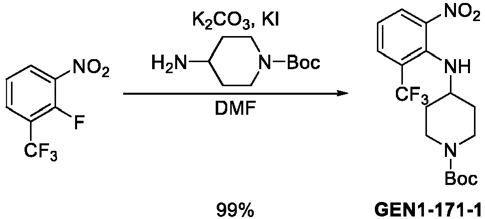

2-fluoro-1-nitro-3-(trifluoromethyl)benzene (3.0 g, 14 mmol), tert-butyl 4-aminopiperidine-1-carboxylate (2.87 g, 14 mmol), K₂CO₃ (2.97 g, 21 mmol), and KI (238 mg, 1.4 mmol) were dissolved in a solution of DMF (60 mL). The reaction mixture was stirred at 80°C for 16 hours. The reaction mixture was cooled to room temperature, and then poured into water (125 mL), and extracted with EA (120 mL×3). The combined organic layer was washed with water (150 mL), brine (150 mL), dried over Na₂SO₄ and it was concentrated to obtain a black oil. The crude product was purified by silica gel column chromatography, eluted with (EA/(PE + EA) = 5%), to obtain the desired compound (5.57 g, 99% yield) as a yellow oil. TLC: EA/(PE+EA)=5%, Rf=0.3

### Step 2. N-(2-nitro-6-(trifluoromethyl)phenyl)piperidin-4-amine 2,2,2-trifluoroacetate (GEN1-171-2)

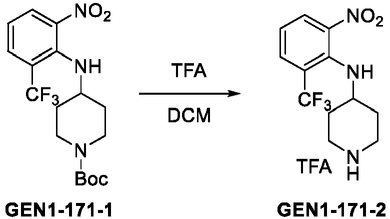

TFA (1.0 mL) was added dropwise to a solution of tert-butyl 4-(((2-nitro-6-(trifluoromethyl)phenyl)amino)piperidin-1-carboxylate (500 mg, 0.51 mmol) in DCM (10.0 mL) at 0°C. The resulting mixture was stirred at room temperature for 2 hours. The resulting mixture was concentrated. The desired compound (660 mg crude product) could be used directly in the next step without further purification. TLC: EA/(PE+EA)=80%, Rf=0.4

### Step 3. 1-(4-((2-nitro-6-(trifluoromethyl)phenyl)amino)piperidin-1-yl)-2-(quinolin-6-yl)ethan-1-one (GEN1-171-3)

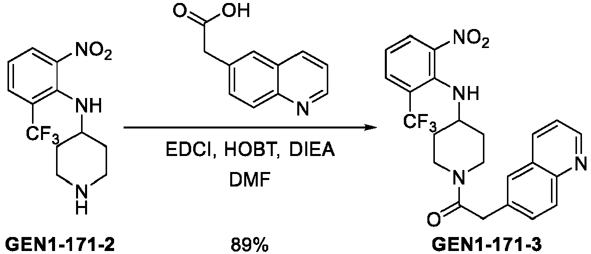

DIEA (664 mg, 5.15 mmol) was added to a solution of N-(2-nitro-6-(trifluoromethyl)phenyl)piperidin-4-amine 2,2,2-trifluoroacetate (660 mg crude), 2-(quinolin-6-yl)acetic acid (240 mg, 1.28 mmol), EDCI (370 mg, 1.93 mmol), HOBT (260 mg, 1.93 mmol) in DMF (10 mL). The resulting mixture was stirred at room temperature for 16 hours. The reaction mixture was poured into water (20 mL), and extracted with EA (50 mL×3). The combined organic layer was washed with water (90 mL), brine (90 mL), dried over Na₂SO₄ and it was concentrated to obtain a yellow oil. The crude product was purified by silica gel column chromatography and eluted with (EA/(PE + EA)=80%) to obtain the desired compound (528 mg, yield 89%) as a yellow solid. MS (ESI): Rt = 1.53 min, *m*/*z* 459.0 [M + H]⁺ , Purity: 100% @ 254 nm, 92% @ 214 nm.

### Step 4. 1-(4-((2-amino-6-(trifluoromethyl)phenyl)amino)piperidin-1-yl)-2-(quinolin-6-yl)ethan-1-one (GEN1-171-4)

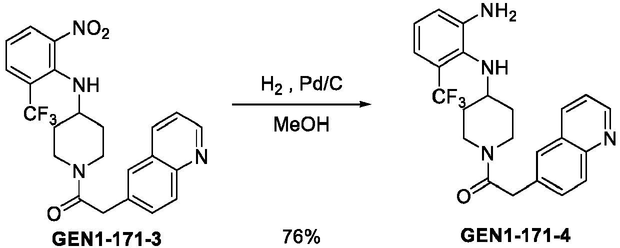

Pd/C (60 mg) was added to a solution of 1-(4-((2-nitro-6-(trifluoromethyl)phenyl)amino)piperidin-1-yl)-2-(quinolin-6-yl)ethan-1-one (514 mg, 1.12 mmol) in methanol (25 mL). The resulting mixture was hydrogenated at room temperature for 4 hours. The reaction mixture was filtered, and the filter cake was washed with MeOH (50 mL×3). The filtrate was concentrated under reduced pressure. The crude product was purified by silica gel column chromatography, eluted with (EA/(PE + EA=60%), to obtain the desired compound (366 mg, yield 76%) as a pale yellow solid. MS (ESI): Rt = 1.47 min, *m*/*z* 428.8 [M + H]⁺ , Purity: 100% @ 254 nm, 86% @ 214 nm.

### Step 5. 1-(4-(2-amino-7-(trifluoromethyl)-1H-benzo[d]imidazol-1-yl)piperidin-1-yl)-2-(quinolin-6-yl)ethan-1-one (GEN1-171-5)

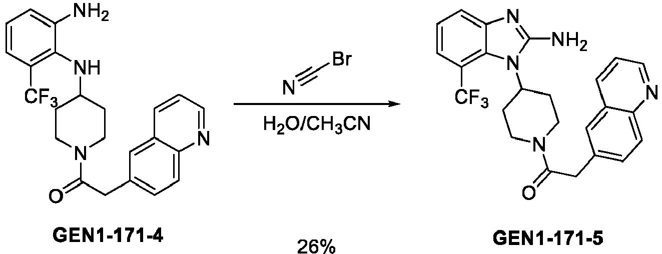

Cyanogen bromide (453 mg, 4.27 mmol) was added to a solution of 1-(4-((2-amino-6-(trifluoromethyl)phenyl)amino)piperidin-1-yl)-2-(quinolin-6-yl)ethan-1-one (366 mg, 0.85 mmol) in MeCN (10 mL). The resulting mixture was stirred at room temperature for 36 hours. The reaction mixture was poured into water (20 mL) and extracted with EA (30 mL X 3). The organic layer was washed with water (50 mL), brine (50 mL), dried over Na₂SO₄ and it was concentrated to obtain a light yellow solid. The crude product was purified by reverse-phase flash chromatography (C₁₈ reverse silica column; mobile phase CH₃CN, water, 15% to 70% gradient, 50 minutes; detection, UV 214nm) to obtain the desired compound (100 mg, yield 26%) as a white solid. ¹H NMR (400 MHz, DMSO-d₆): δ 8.87 (d, *J =* 4.2 Hz, 1H), 8.38-8.28 (m, 1H), 7.97 (d, *J =* 8.7 Hz, 1H), 7.83 (d, *J =* 1.9 Hz, 1H), 7.67 (d, *J =* 8.7 Hz, 1H), 7.52 (d, *J =* 8.3 Hz, 1H), 7.44 (d, *J =* 7.7 Hz, 1H), 7.25 (d, *J* = 7.8 Hz, 1H), 7.12 (t, *J* = 7.8 Hz, 1H), 6.57 (s, 2H), 4.76-4.65 (m, 1H), 4.59-4.46 (m, 1H), 4.32-4.21 (m, 1H), 4.15-3.82 (m, 2H), 3.05 (t, *J* = 13.6 Hz, 1H), 2.67-2.52 (m, 2H), 2.46-2.27 (m, 1H), 1.82-1.65 (m, 2H). MS (ESI): Rt = 1.43 min, *m*/*z* 453.8 [M + H]⁺ , Purity: 100% @ 254 nm, 100% @ 214 nm.

### Step 6. (E)-N-(1-(1-(1-(2-(quinolin-6-yl)acetyl)piperidin-4-yl)-7-(trifluoromethyl)-1,3-dihydro-2H-benzo[d]imidazol-2-propylene)acetamide (GEN1-171)

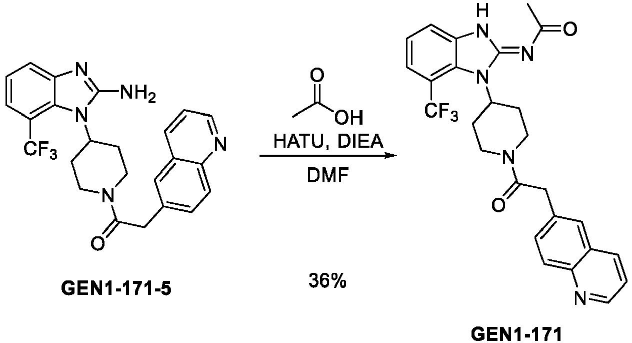

DIEA (43 mg, 0.33 mmol) was added to a solution of 1-(4-(2-amino-7-(trifluoromethyl)-1H-benzo[d]imidazol-1-yl)piperidin-1-yl)-2-(quinolin-6-yl)ethan-1-one (40 mg, 0.09 mmol), acetic acid (6 mg, 0.10 mmol) and HATU (64 mg, 0.17 mmol) in DMF (0.5 mL). The resulting mixture was stirred at room temperature for 120 hours. The reaction mixture was poured into water (5 mL) and extracted with EA (8 mL×3). The combined organic layer was washed with water (20 mL), brine (20 mL), dried over Na₂SO₄ and it was concentrated to obtain a black oil. The crude product was purified by reverse-phase flash chromatography (C₁₈ reverse silica column; mobile phase CH₃CN, water, 15% to 65% gradient, 50 minutes; detection, UV 214 nm) to obtain the desired compound (16 mg, yield 36%) as a white solid. ¹H NMR (400 MHz, DMSO-d₆): δ 10.36 (s, 1H), 8.91-8.84 (m, 1H), 8.33 (d, *J =* 9.2 Hz, 1H), 7.98 (d, *J* = 8.6 Hz, 1H), 7.90 (d, *J =* 8.0 Hz, 1H), 7.85 (s, 1H), 7.71-7.64 (m, 2H), 7.55-7.50 (m, 1H), 7.38 (t, *J* = 7.9 Hz, 1H), 4.72-4.50 (m, 2H), 4.37-4.25 (m, 1H), 4.10-3.91 (m, 2H), 3.10 (t, *J* = 13.1 Hz, 1H), 2.61 (t, *J* = 12.8 Hz, 1H), 1.88 (s, 3H), 1.86 - 1.71 (m, 2H). MS (ESI): Rt = 1.28 min, *m*/*z* 496.2 [M + H]⁺, Purity: 100% @ 254 nm, 10 0% @ 214 nm.

### Example 131: (E)-N-(1-(1-(1-(2-(quinolin-6-yl)acetyl)piperidin-4-yl)-7-(trifluoromethyl)-1,3-dihydro-2H-benzo[d]imidazol-2-alkylene)benzamide (GEN1-172)

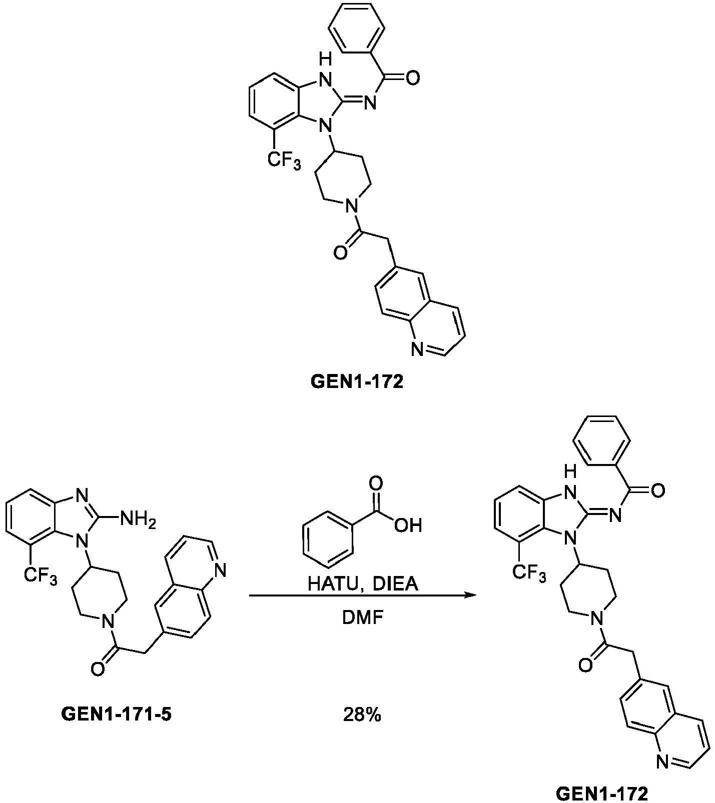

### Step 1. (E)-N-(1-(1-(1-(2-(quinolin-6-yl)acetyl)piperidin-4-yl)-7-(trifluoromethyl)-1,3-dihydro-2H-benzo[d]imidazol-2-alkylene)benzamide (GEN1-172)

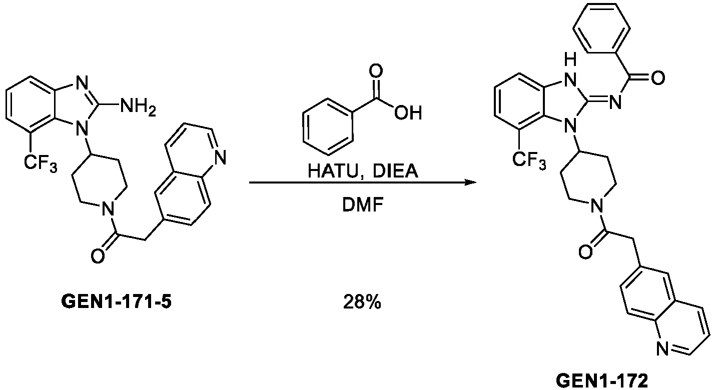

DIEA (68 mg, 0.53 mmol) was added to a solution of 1-(4-(2-amino-7-(trifluoromethyl)-1H-benzo[d]imidazol-1-yl)piperidin-1-yl)-2-(quinolin-6-yl)ethan-1-one (60 mg, 0.13 mmol), benzoic acid (20 mg, 0.16 mmol) and HATU (100 mg, 0.26 mmol) in DMF (1.0 mL). The resulting mixture was stirred at room temperature for 180 hours. The reaction mixture was poured into water (5 mL) and extracted with EA (8 mL×3). The organic layer was washed with water (20 mL), brine (20 mL), dried over Na₂SO₄ and it was concentrated to obtain a black oil. The crude product was purified by reverse-phase flash chromatography (C₁₈ reverse silica column; mobile phase CH₃CN, water, 15% to 65% gradient, 50 minutes; detection, UV 214nm) to obtain the desired compound (20 mg, yield 28%) as a white solid. ¹H NMR (400 MHz, DMSO-d₆): δ 13.28 (s, 1H), 8.89-8.83 (m, 1H), 8.24 (d, *J =* 8.0 Hz, 1H), 8.16-8.10 (m, 2H), 7.95-7.86 (m, 2H), 7.83 (s, 1H), 7.72-7.67 (m, 1H), 7.65 (d, *J =* 7.9 Hz, 1H), 7.54-7.45 (m, 2H), 7.45-7.34 (m, 3H), 4.75-4.56 (m, 2H), 4.37-4.30 (m, 1H), 4.23-3.95 (m, 2H), 3.28-3.06 (m, 3H), 2.74-2.62 (m, 1H), 1.89-1.81 (m, 2H). MS (ESI): Rt = 1.50 min, *m*/*z* 558.3 [M + H]⁺ , Purity: 100% @ 254 nm, 100% @ 214 nm.

### Example 132: 1-(1-(2-(quinolin-6-yl)acetyl)azacycloheptane-4-yl)-7-(trifluoromethyl)-1,3-dihydro-2H-benzo[d]imidazol-2-one (GEN1-173)

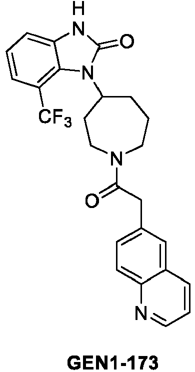

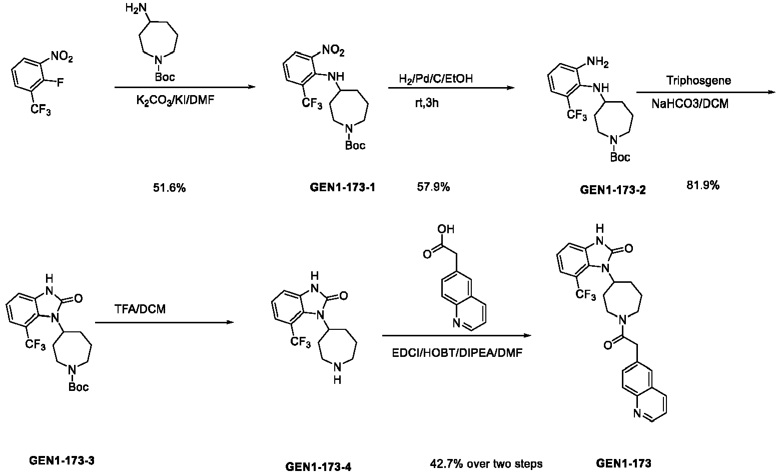

### Step 1. Synthesis of tert-butyl 4-(((2-nitro-6-(trifluoromethyl)phenyl)amino)azacycloheptane-1-carboxylate (GEN1-173-1)

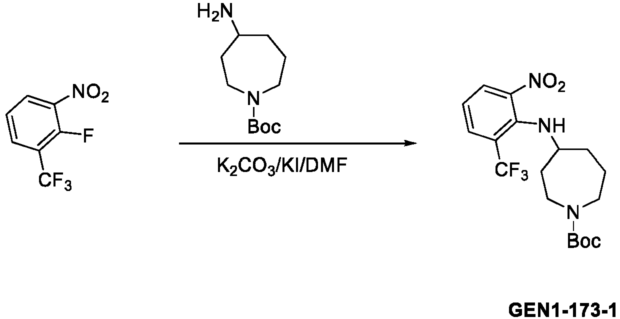

Tert-butyl 4-aminoazacycloheptane-1-carboxylate (185.2 mg, 0.864 mmol) was added to a suspension of 2-fluoro-1-nitro-3-(trifluoromethyl)benzene (150mg, 0.72mmol), K₂CO₃ (199 mg, 1.44 mmol), KI(12 mg, 0.072 mmol) in DMF (5 mL). The resulting mixture was stirred at 80°C for 3 hours. The reaction mixture was diluted with water (80 mL), and extracted with EtOAc (30 mL×3). The combined organic phase was washed with brine (50 mL), dried over anhydrous Na₂SO₄ and filtered. The filtrate was concentrated to obtain the desired compound (150 mg, yield 51.6%) as a yellow oil. MS (ESI): Rt = 1.85 min, m/z 426.4, [M + H]⁺; Purity: 60.94% @ 254 nm, 38.26% @ 214 nm.

### Step 2. Synthesis of tert-butyl 4-((2-amino-6-(trifluoromethyl)phenyl)amino)azacycloheptane-1-carboxylate (GEN1-173-2)

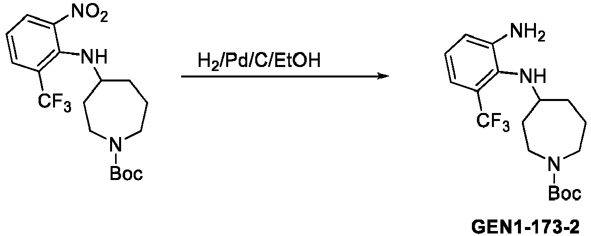

Pd/C (15 mg, 5% purity) was added to a solution of tert-butyl 4-(((2-nitro-6-(trifluoromethyl)phenyl)amino)azacycloheptane-1-carboxylate (150 mg, 0.37 mmol) in EtOH (20 mL) at room temperature. The resulting mixture was replaced with hydrogen and stirred under a hydrogen atmosphere for 3 hours. The mixture was filtered and the filtrate was concentrated. The residue was purified by silica gel column chromatography (PE:EA = 1:1) to obtain the desired compound (80 mg, yield 57.9%) as a pale yellow solid.

### Step 3. Tert-butyl 4-(2-oxo-7-(trifluoromethyl)-2,3-dihydro-1H-benzo[d]imidazol-1-yl)azacycloheptane-1-carboxylate (GEN1-173-3)

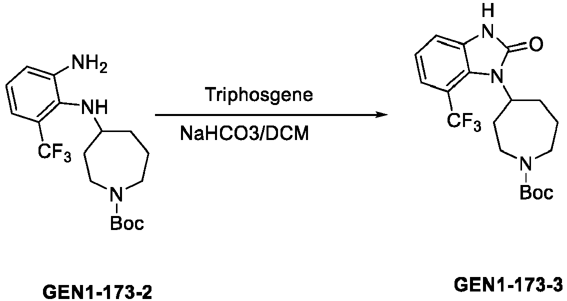

Triphosgene (18.68 mg, 0.086 mmol) was added to a suspension of tert-butyl 4-(((2-amino-6-(trifluoromethyl)phenyl)amino)azacycloheptane-1-carboxylate (80 mg, 0.214 mmol), NaHCO₃ (90 mg, 1.07 mmol) in DCM (10 mL) at room temperature. The resulting mixture was stirred at room temperature for 1 hour. The mixture was poured into water (20 mL), and extracted with DCM (20 mL×3). The combined organic layer was washed with brine (50 mL), dried over Na₂SO₄ and it was concentrated. The crude product was purified by reverse phase chromatography. Purification conditions: C18 silica gel column; mobile phase ACN: H₂O, gradient: 5% to 50%; 45 minutes, monitoring wavelength: 214 nm. The desired compound (70 mg, yield 81.9%) was obtained as a pale yellow solid. MS (ESI): Rt = 1.66 min, m/z 422.1, [M + H]⁺; Purity: 56.18% @ 254 nm, 100% @ 214 nm.

### Step 4. Synthesis of 1-(aza-4-yl)-7-(trifluoromethyl)-1,3-dihydro-2H-benzo[d]imidazol-2-one (GEN1-173-4)

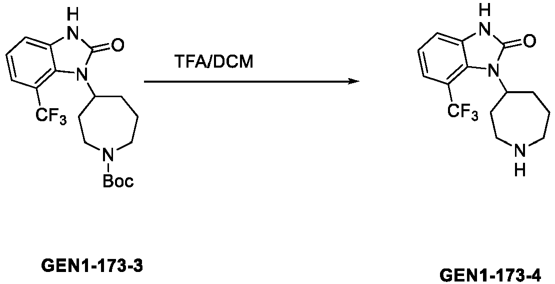

TFA (2 mL) was added to a solution of tert-butyl 4-(2-oxo-7-(trifluoromethyl)-2,3-dihydro-1H-benzo[d]imidazol-1-yl)azacycloheptane-1-carboxylate (70 mg, 0.175 mmol) in DCM (10 mL). The resulting mixture was stirred at room temperature for 1 hour. The resulting mixture was concentrated and redissolved in DCM (20ml). Then the mixture was concentrated again to obtain the desired crude compound (100 mg) as a brown solid. It was used directly in the next step without purification. MS (ESI): Rt = 1.27 min, m/z 300.1, [M + H]⁺; Purity: 94% @ 214 nm.

### Step 5. Synthesis of 1-(1-(2-(quinolin-6-yl)acetyl)azacycloheptane-4-yl)-7-(trifluoromethyl)-1,3-dihydro-2H-benzo[d]imidazol-2-one (GEN1-173)

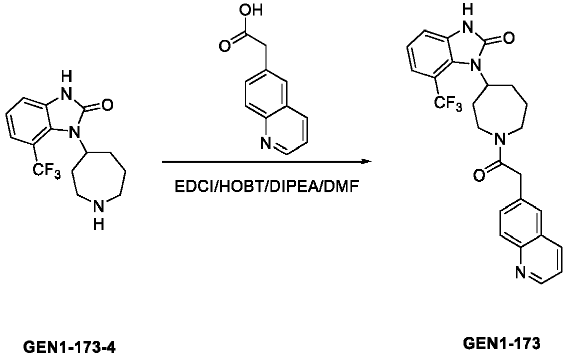

EDCI (50.3 mg, 0.2625 mmol) was added to a solution of 1-(aza-4-yl)-7-(trifluoromethyl)-1,3-dihydro-2H-benzo[d]imidazol-2-one (100 mg, crude product, 0.175 mmol), 2-(quinolin-6 yl)acetic acid (39.3 mg, 0.21 mmol), HOBT (35.47 mg, 0.2625 mmol) and DIEA (90.4 mg, 0.7 mmol) in DMF (2 mL). The resulting mixture was stirred at room temperature for 1 h. The reaction mixture was diluted with water (50 mL), and extracted with EtOAc (20 mL×3). The combined organic phase was washed with brine (50 mL), dried over anhydrous Na₂SO₄ and filtered. The filtrate was concentrated to obtain a crude product. The crude product counld be used directly in the next step without purification. The crude product was purified by reverse phase chromatography. Purification conditions: C18 silica gel column; mobile phase ACN: H₂O, gradient: 5% to 50%; 45 minutes, monitoring wavelength: 214 nm. The desired compound (35 mg, yield 42.7%) was obtained as a white solid. ¹HNMR (400 MHz, Chloroform-d) δ 9.61 (d, J = 33.3 Hz, 1H), 8.90 (s, 1H), 8.30 - 8.09 (m, 2H), 7.86 - 7.65 (m, 2H), 7.47 - 7.32 (m, 2H), 7.23 (t, J = 7.9 Hz, 1H), 7.14-7.03 (m, 1H), 4.48 - 4.30 (m, 1H), 4.06 - 3.84 (m, 3H), 3.81 - 3.67 (m, 1H), 3.62 - 3.52 (m, 1H), 3.50-3.39 (m, 1H), 2.94 - 2.62 (m, 2H), 2.27-2.13 (m, 1H), 2.08 - 1.98 (m, 1H), 1.90 - 1.54 (m, 2H). MS (ESI): Rt = 1.42 min, m/z 468.9, [M + H]⁺; Purity: 100% @ 254 nm, 100% @ 214 nm.

### Example 133: 7-(trifluoromethyl)-1-((1-(2-(4-(trifluoromethyl)phenyl)acetyl)piperidin-4-yl)methyl)-1H-benzo[d]imidazol-2(3H)-one (GEN1-174)

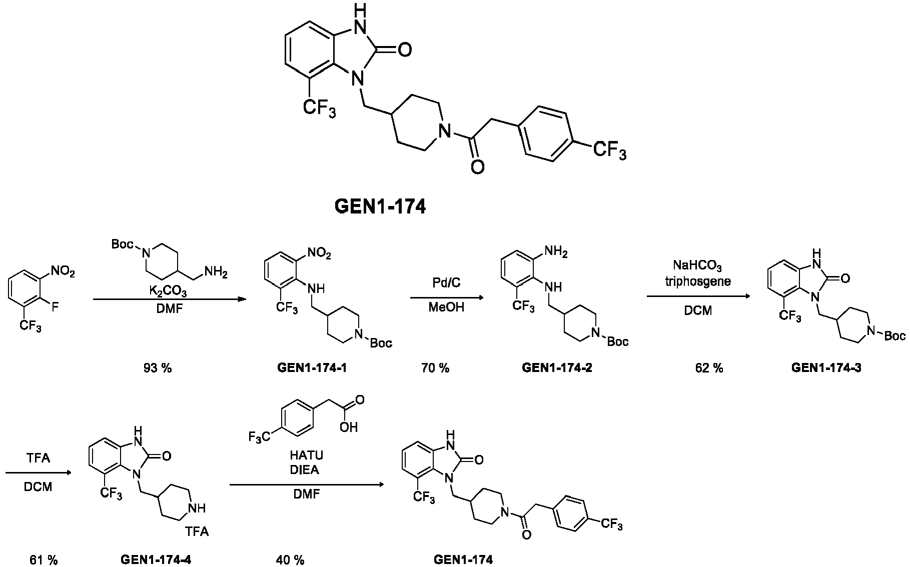

### Step 1. Synthesis of tert-butyl 4-((((2-nitro-6-(trifluoromethyl)phenyl)amino)methyl)piperidin-1-carboxylate

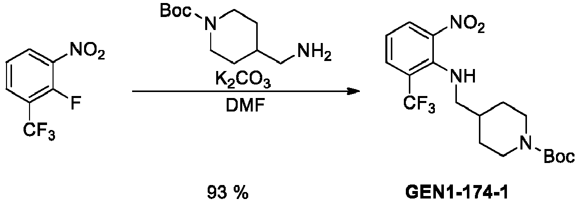

A mixture of 2-fluoro-1-nitro-3-(trifluoromethyl)benzene (600 mg, 2.87 mmol), tert-butyl 4-(aminomethyl)piperidin-1-carboxylate (613 mg, 2.86 mmol) and K₂CO₃ (595 mg, 4.31 mmol) in DMF (10 mL) was stirred at 85°C for 4 hours. The mixture was diluted with water (15 mL), and extracted with EtOAc (20 mL×3). The combined organic layer was washed with brine (20 mL), dried over anhydrous Na₂SO₄ and it was concentrated to obtain the desired compound (3 g, 93% yield, 36% purity at 214 nm) as a yellow oil. MS (ESI): Rt = 1.88 min, *m*/*z* 348.3 [M-56+H]⁺; Purity: 23% @ 254 nm, 36% @ 214 nm.

### Step 2. Synthesis of tert-butyl 4-((((2-amino-6-(trifluoromethyl)phenyl)amino)methyl)piperidin-1-carboxylate

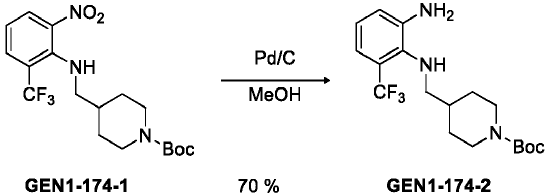

A solution of tert-butyl 4-((((2-2-nitro-6-(trifluoromethyl)phenyl)amino)methyl)piperidin-1-carboxylate (3 g, 2.68 mmol, 36% purity) and Pd/C (10%, 21 mg) in MeOH (5 mL) was hydrogenated for 4 hours. The reaction mixture was then filtered and the filtrate was concentrated. The crude product was purified by a C18 column (ACN/water=40-80%) to obtain the desired compound (700 mg, yield 70%) as a brown oil. MS (ESI): Rt = 1.85 min, *m*/*z* 318.3 [M-56+H]⁺; Purity: 100% @ 254 nm, 94% @ 214 nm.

### Step 3. Synthesis of tert-butyl 4-((2-oxo-7-(trifluoromethyl)-2,3-dihydro-1H-benzo[d]imidazol-1-yl)methyl)piperidin-1-carboxylate

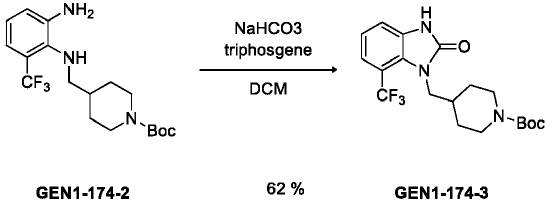

NaHCO₃ (270 mg, 3.21 mmol) was added to a solution of tert-butyl 4-((((2-amino-6-(trifluoromethyl)phenyl)amino)methyl)piperidin-1-carboxylate (300 mg, 0.803 mmol) in DCM (5 mL) at 0°C. Then triphosgene (238mg, 0.802mmol) was added. The resulting mixture was warmed to room temperature and stirred for 2 hours. The mixture was poured into water (15 mL), and extracted with EtOAc (10 mL×3). The combined organic layer was washed with brine (15 mL×2), dried over anhydrous Na₂SO₄ and it was concentrated. The residue was purified by a C18 column (50-70% B; A: H₂O, B: CH₃CN) to obtain the desired compound (220 mg, yield 62%) as a white solid. MS (ESI): Rt = 1.71 min, *m*/*z* 398.5 [M-H]⁻; Purity: 48% @ 254 nm, 100% @ 214 nm.

### Step 4. Synthesis of 1-(piperidin-4-ylmethyl)-7-(trifluoromethyl)-1H-benzo[d]imidazol-2(3H)-1-one 2,2,2-trifluoroacetate

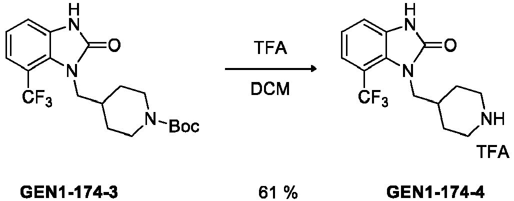

Tert-butyl 4-((2-oxo-7-(trifluoromethyl)-2,3-dihydro-1H-benzo[d]imidazol-1-yl)methyl)piperidin-1-carboxylate (100 mg, 250 umol) was dissolved in dichloromethane (5mL) and TFA (0.5mL) and it was stirred overnight. The mixture was concentrated to obtain the desired compound (70 mg, yield 61%) as a colorless oil. MS (ESI): Rt = 1.28 min, *m*/*z* 300.3 [M+H]⁺; Purity: 71% @ 254 nm, 100% @ 214 nm.

### Step 5. Synthesis of 7-(trifluoromethyl)-1-((1-(2-(4-(trifluoromethyl)phenyl)acetyl)piperidin-4-yl)methyl)-1H-benzo[d]imidazol-2(3H)-one (GEN1-174)

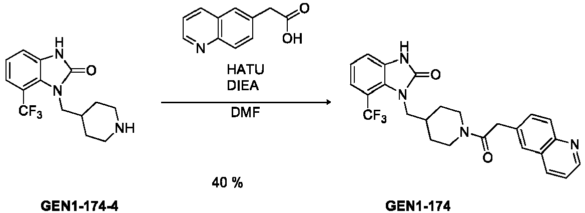

A mixture of compound 2-(6-quinolinyl)acetic acid (38 mg, 0.203 mmol) and HATU (97 mg, 0.255 mmol) in DMF (5 mL) was stirred at 0°C for 40 minutes. Then 1-(piperidinbut-4-ylmethyl)-7-(trifluoromethyl)-1H-benzo[d]imidazol-2(3H)-1-one 2,2,2-trifluoroacetate (70 mg, 0.17 mmol) and DIEA (66 mg 0.51 mmol) were added. The mixture was stirred at room temperature for 4 hours, and then poured into water (15 mL), and extracted with EtOAc (10 mL×3). The combined organic layer was washed with brine (20 mL), dried over anhydrous Na₂SO₄ and concentrated. The residue was purified by preparation. HPLC [water 3, sunfire C18, 5um, 19 * 150 mm, 30∼80% B, A: H₂O (0.1% NH₄HCO₃), B: ACN, flow rate: 15 mL / min, UV = 214 nm]. The desired compound (32.4 mg, yield 40%) was obtained as a white solid. ¹H NMR (400 MHz, DMSO-*d*₆): δ 11.48 (s, 1H), 8.86 (dd, *J =* 4.0, 1.2 Hz, 1H), 8.30 (d, *J =* 7.6 Hz, 1H), 7.95 (d, *J =* 8.4 Hz, 1H), 7.78 (s, 1H), 7.62 (dd, *J =* 8.4, 2.0 Hz, 1H), 7.51 (dd, *J =* 8.4, 4.0 Hz, 1H), 7.34 (d, *J =* 8.0 Hz, 1H), 7.30 (d, *J =* 7.6 Hz, 1H), 7.17 (t, *J =* 8.0 Hz, 1H), 4.38 (d, *J =* 12.4 Hz, 1H), 4.00 (d, *J =* 13.6 Hz, 1H), 3.91 (s, 2H), 3.78 (d, *J =* 7.2 Hz, 2H), 2.93 (t, *J =* 12.4 Hz, 1H), 2.45- 2.48 (m, 1H), 2.03-1.96 (m, 1H), 1.49 (t, *J =* 11.8 Hz, 2H), 1.13-1.04 (m, 2H). MS (ESI): Rt = 3.137 min, *m*/*z* 469.2 [M+H]⁺; Purity: 93.19% @ 254 nm, 99.10% @ 214 nm.

### Example 134: 5-[1-[2-[4-(trifluoromethyl)phenyl]acetyl]-4-piperidinyl]-1,3-dihydro-1,4-benzodiazepine-2-one (GEN1-176)

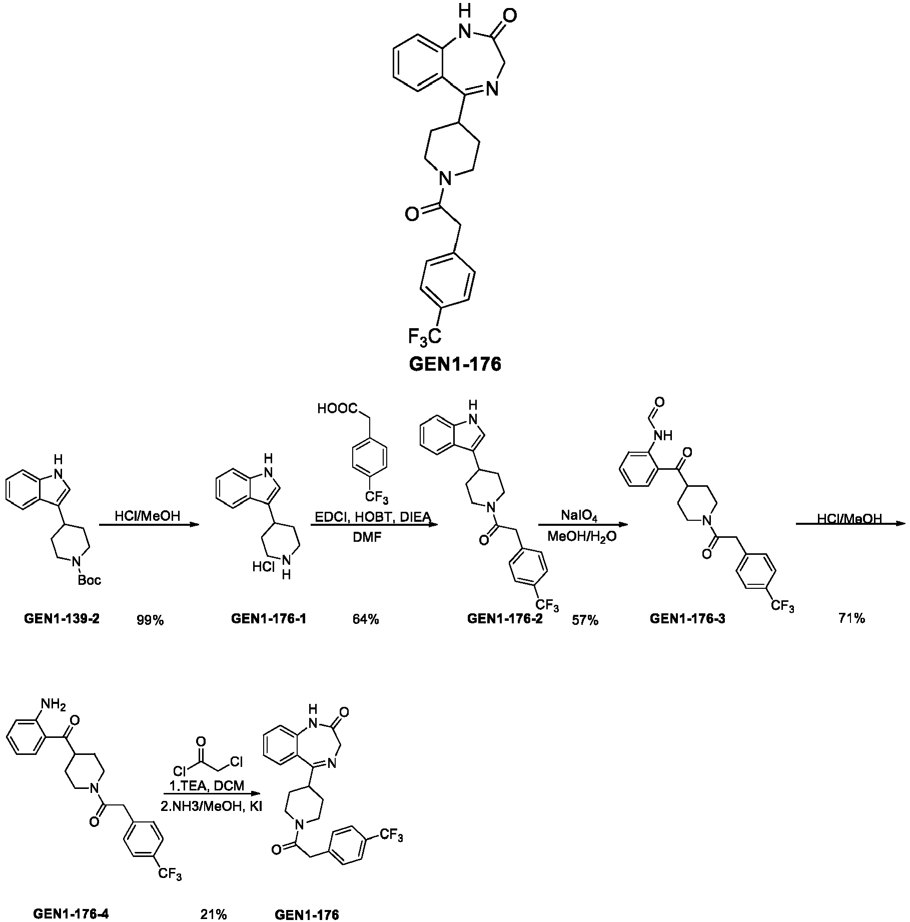

### Step 1. Synthesis of 3-(piperidin-4-yl)-1H-indole hydrochloride

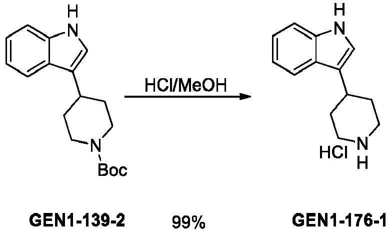

Tert-butyl 4-(1H-indol-3-yl)piperidin-1-carboxylate (450 mg, 1.50 mmol, the synthesis steps could be found in GEN1-139-2) in HCl/MeOH (6 M, 10) was stirred at room temperature for 2 hours. The resulting mixture was concentrated to obtain the desired compound (350 mg, yield 99%) as a brown solid. MS (ESI): Rt = 1.15 min, *m*/*z* 201.1 [M+H]⁺; Purity: 100% @ 254 nm, 100% @ 214 nm.

### Step 2. Synthesis of 1-(4-(1H-indol-3-yl)piperidin-1-yl)-2-(4-(trifluoromethyl)phenyl)ethenone

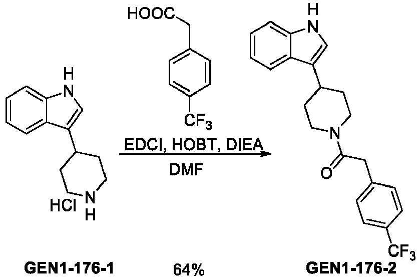

2-(4-(trifluoromethyl)phenyl)acetic acid (478 mg, 2.34 mmol) was added to a solution of 3-(piperidin-4-yl)-1H-indole hydrochloride (462 mg, 1.95 mmol), DIEA (1.26 g, 9.76 mmol), EDCI (561 mg, 2.93 mmol) and HOBT (396 mg, 2.93 mmol) in DMF (5 mL). The mixture was stirred at room temperature overnight. The resulting mixture was purified by C18 (50-60% B, A: H₂O B: CH₃CN) to obtain the desired compound (480 mg, yield 64%) as a brown solid. MS (ESI): Rt = 1.51 min, *m*/*z* 387.4 [M+H]⁺; Purity: 100% @ 254 nm, 100% @ 214 nm.

### Step 3. Synthesis of N-(2-(1-(2-(4-(trifluoromethyl)phenyl)acetyl)piperidin-4-carbonyl)phenyl)carboxamide

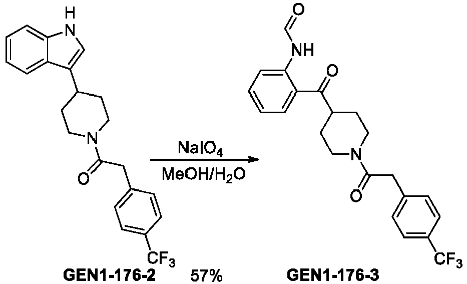

Sodium periodate (631 mg, 2.95 mmol) was added to a suspension of 1-(4-(1H-indol-3-yl)piperidin-1-yl)-2-(4-(trifluoromethyl)phenyl)ethanone (380 mg, 0.983 mmol) in MeOH (10 mL) and water (5 mL). The mixture was stirred at 70°C overnight. The resulting mixture was concentrated, and water (20 mL) was added. DCM (30 mL×3) was added to extract the desired compound. The combined organic layer was washed with brine (50 mL), dried over anhydrous Na₂SO₄ and filtered. The filtrate was concentrated, and the residue was purified by C18 (50-70% B; A: H₂O B: CH₃CN) to obtain the desired compound (235 mg, yield 57%) as a yellow oil. MS (ESI): Rt = 1.56 min, *m*/*z* 419.4 [M+H]⁺; Purity: 100% @ 254 nm, 100% @ 214 nm.

### Step 4. Synthesis of 1-(4-(2-aminobenzoyl)piperidin-1-yl)-2-(4-(trifluoromethyl)phenyl)ethenone

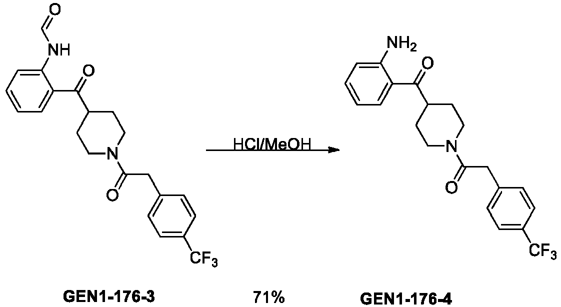

A solution of N-(2-(1-(2-(4-(4-(trifluoromethyl)phenyl)acetyl)piperidin-4-carbonyl)phenyl)carboxamide (215 mg, 514 umol) in HCl/MeOH (7 M, 5 mL) was stirred at room temperature for 1 hour. The resulting mixture was concentrated, and then saturated Na₂CO₃ solution (10 mL) was added. The mixture was extracted with DCM (20 mL×3). The combined organic layer was washed with brine (30 mL), dried over anhydrous Na₂SO₄ and filtered. The filtrate was concentrated to obtain the desired compound (143 mg, yield 71%) as a yellow oil. MS (ESI): Rt = 1.59 min, *m*/*z* 391.4 [M+H]⁺; Purity: 100% @ 254 nm, 100% @ 214 nm.

### Step 5. Synthesis of 5-[1-[2-[4-(trifluoromethyl)phenyl]acetyl]-4-piperidinyl]-1,3-dihydro-1,4-benzodiazepine-2-one (GEN1-176)

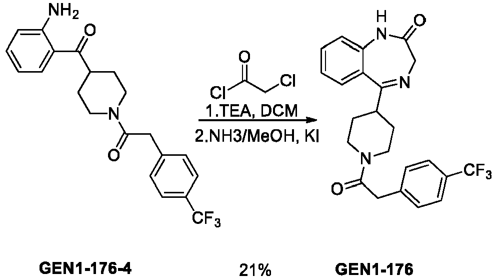

In an ice water bath, 2-chloroacetyl chloride (46 mg, 0.41 mmol) was added dropwise to a solution of 1-(4-(2-aminobenzoyl)piperidin-1-yl)-2-(4-(trifluoromethyl)phenyl)ethenone (143 mg, 0.366 mmol) and TEA (56 mg, 0.55 mmol) in DCM (3 mL) The mixture was stirred at room temperature for 2 hours, and then the solvent was evaporated. KI (6 mg, 36 umol) and NH3/MeOH (9M, 3mL) were added. The mixture was stirred for another 2 days at room temperature. The resulting mixture was concentrated and the residue was purified by preparation. HPLC (prep-9 Xbridge C18 5um 19 * 150mm, 30%-50% B; A: H₂O (0.1% NH3.H₂O), B: CH₃CN; 214 nm, flow rate: 15 mL / min). The desired compound (34 mg, yield 21%) was obtained as a white solid. ¹HNMR (400 MHz, DMSO-*d*₆): δ 10.35 (s, 1H), 7.73 (dd, *J =* 8.0, 0.8 Hz, 1H), 7.65 (d, *J =* 8.0 Hz, 2H), 7.51-7.47 (m, 1H), 7.43 (d, *J =* 8.4 Hz, 2H), 7.24-7.20 (m, 1H), 7.15 (dd, *J =* 8.0, 0.8 Hz, 1H), 4.33 (d, *J* = 12.8 Hz, 1H), 3.97-3.86 (m, 3H), 3.82-3.78 (m, 2H), 3.24-3.18 (m, 1H), 3.13-3.07 (m, 1H), 2.72-2.66 (m, 1H), 1.75-1.64 (m, 2H), 1.39-1.24 (m, 2H). MS (ESI): Rt = 3.657 min, *m*/*z* 430.2 [M+H]⁺; Purity: 98.11% @ 254 nm, 99.38% @ 214 nm.

### Example 135: 8-(2-(4-(trifluoromethyl)phenyl)acetyl)-1,3,8-triazaspiro[4.5]decane-2,4-dione (GEN1-177)

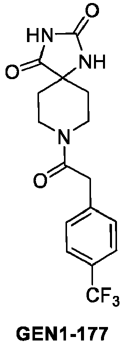

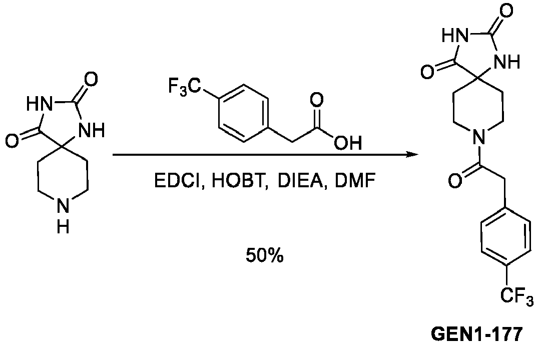

### Step 1. 8-(2-(4-(trifluoromethyl)phenyl)acetyl)-1,3,8-triazaspiro[4.5]decane-2,4-dione (GEN1-177)

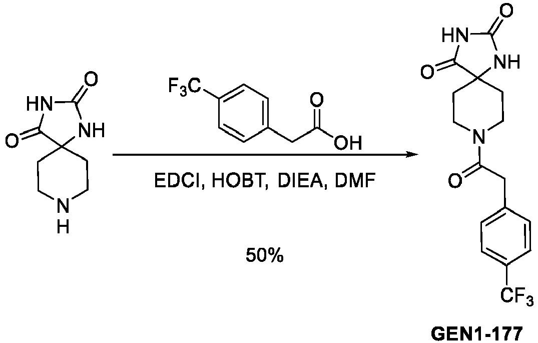

DIEA (153 mg, 1.19 mmol) was added to a solution of 1,3,8-triazaspiro[4.5]decane-2,4-dione (100 mg, 0.59 mmol), 2-(4-(trifluoromethyl)phenyl)acetic acid (133 mg, 0.65 mmol), EDCI (170 mg, 0.88 mmol) and HOBT (120 mg, 0.89 mmol) in DMF (2.0 mL). The resulting mixture was stirred at room temperature for 8 hours. The reaction mixture was poured into water (15 mL) and extracted with EA (25 mL×3). The combined organic layer was washed with water (60 mL), brine (60 mL), dried over Na₂SO₄ and it was concentrated to obtain a yellow oil. The crude product was purified by reverse-phase flash chromatography (C₁₈ reverse silica column; mobile phase CH₃CN, water, 10% to 65% gradient, 50 minutes; detection, UV 214 nm) to obtain the desired compound (105 mg, yield 50%) as a white solid. ¹HNMR (400 MHz, DMSO-d₆): δ 10.75 (s, 1H), 8.58 (s, 1H), 7.67 (d, *J =* 8.1 Hz, 2H), 7.45 (d, *J =* 8.0 Hz, 2H), 4.24 - 4.13 (m, 1H), 3.96 - 3.87 (m, 1H), 3.87 (s, 2H), 3.40 - 3.28 (m, 1H), 3.09-2.97 (m, 1H), 1.78 - 1.59 (m, 2H), 1.59 - 1.49 (m, 2H). MS (ESI): Rt = 1.37 min, *m*/*z* 356.0 [M + H]⁺ , Purity: 100% @ 254 nm, 100% @ 214 nm.

### Example 136: 1'-(2-(4-(trifluoromethyl)phenyl)acetyl)spiro[benzo[d][1,3]oxazine-4,4'-piperidine]-2(1H)-one (GEN1-178)

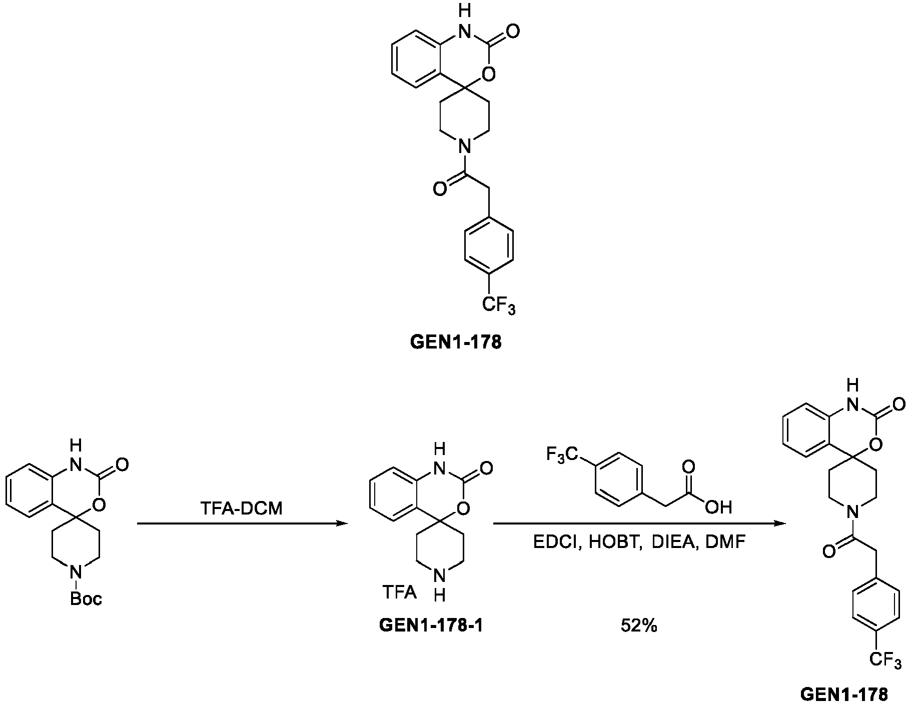

### Step 1. Spiro[benzo[d][1,3]oxazine-4,4'-piperidine]-2(1H)-one 2,2,2-trifluoroacetate (GEN1-178-1)

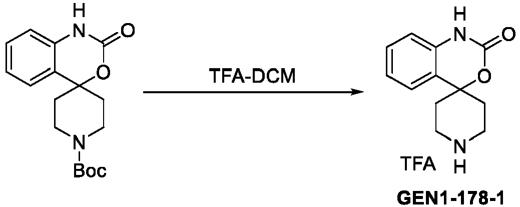

TFA (0.3 mL) was added dropwise to a solution of tert-butyl 2-oxo-1,2-dihydrospiro[benzo[d][1,3]oxazine-4,4'-piperidine]-1'-carboxylate (100 mg, 0.32 mmol) in DCM (1.0 mL) at 0°C. The resulting mixture was stirred at room temperature for 2 hours. The obtained mixture was concentrated to obtain the desired compound (96 mg crude product), which was used directly in the next step without further purification.

### Step 2. 1'-(2-(4-(trifluoromethyl)phenyl)acetyl)spiro[benzo[d][1,3]oxazine-4,4'-piperidine]-2(1H)-one (GEN1-178)

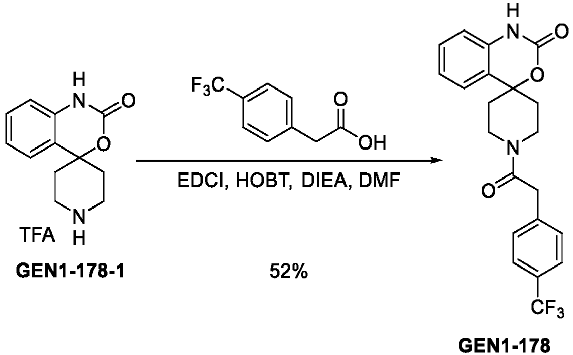

DIEA (160 mg, 1.24 mmol) was added to a solution of spiro[benzo[d][1,3]oxazine-4,4'-piperidine]-2(1H)-one 2,2,2-trifluoroacetate (96 mg crude), 2-(4-(trifluoromethyl)phenyl)acetic acid (70 mg, 0.34 mmol), EDCI (90 mg, 0.46 mmol), HOBT (64 mg, 0.46 mmol) in DMF (1.5 mL). The resulting mixture was stirred at room temperature for 8 hours. The reaction mixture was poured into water (15 mL) and extracted with EA (25 mL×3). The combined organic layer was washed with water (60 mL), brine (60 mL), dried over Na₂SO₄ and it was concentrated to obtain a yellow oil. The crude product was purified by reverse-phase flash chromatography (C₁₈ reverse silica column; mobile phase CH₃CN, water, 15% to 75% gradient, 50 minutes; detection, UV 214 nm) to obtain the desired compound (66 mg, yield 52%) as a white solid. ¹H NMR (400 MHz, DMSO-d₆): δ 10.30 (s, 1H), 7.68 (d, *J =* 8.0 Hz, 2H), 7.48 (d, *J =* 7.9 Hz, 2H), 7.29-7.18 (m, 2H), 7.02 (t, *J =* 7.5 Hz, 1H), 6.89 (d, *J =* 7.9 Hz, 1H), 4.51-4.36 (m, 1H), 4.05-3.82 (m, 3H), 3.45-3.35 (m, 1H), 2.99-2.85 (m, 1H), 2.03-1.81 (m, 4H). MS (ESI): Rt = 1.42 min, *m*/*z* 405.1 [M + H]⁺ , Purity: 100% @ 254 nm, 100% @ 214 nm.

### Example 137: 1-(2-(4-(trifluoromethyl)phenyl)acetyl)-1'H-spiro[piperidin-4,4'-quinazoline]-2'(3'H)-one (GEN1-179)

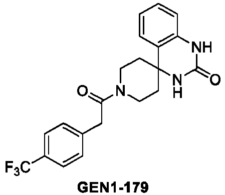

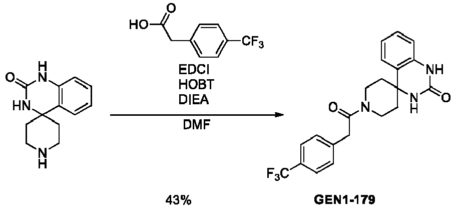

### Step 1. 1-(2-(4-(trifluoromethyl)phenyl)acetyl)-1'H-spiro[piperidin-4,4'-quinazoline]-2'(3'H)-one (GEN1-179)

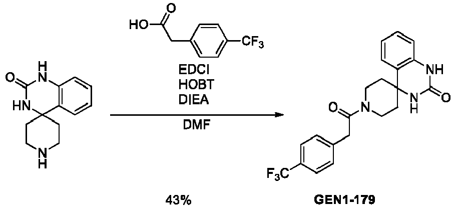

EDCI (106 mg, 0.553 mmol) was added to a solution of 2-(4-(trifluoromethyl)phenyl)acetic acid (90 mg, 0.441 mmol) and HOBT (74 mg, 0.548 mmol) in DMF (4 mL). After the mixture was stirred at room temperature for 5 minutes, 1'H-spiro[piperidin-4,4'-quinazoline]-2'(3'H)-one (80 mg, 0.368 mmol) and DIEA (142 mg , 1.10 mmol) were added. The resulting mixture was stirred at room temperature for 4 hours. The reaction mixture was diluted with water (20 mL), and extracted with EtOAc (15 mL×3). The combined organic phase was washed with brine (20 mL×2), dried over anhydrous Na₂SO₄ and it was concentrated. The residue was purified by HPLC [RP-PREP-1 Xbridge C18 5um, 19 * 150 mm, 20∼75%B, A: H₂O (0.1% NH₄HCO₃), B: ACN, flow rate: 16 mL / min, UV = 214nm] to obtain the desired compound (65 mg, yield 43%) as a white solid. ¹HNMR (400 MHz, DMSO-*d*₆): δ 9.24 (s, 1H), 7.69 (d, *J =* 8.0 Hz, 2H), 7.49 (d, *J =* 8.0 Hz, 2H), 7.29 (s, 1H), 7.15-7.11 (m, 2H), 6.89-6.85 (m, 1H), 6.80 (d, *J =* 8.4 Hz, 1H), 4.31-4.27 (m, 1H), 3.88-3.84 (m, 3H), 3.60-3.54 (m, 1H), 3.16-3.08 (m, 1H), 1.80-1.65 (m, 4H). MS (ESI): Rt = 3.707 min, m/z 404.2 [M+H]⁺; Purity: 98.05% @254nm, 97.34% @214nm.

### Example 138: Synthesis of 1-(4-(4-acetyl-3,4-dihydroquinoxaline-1(2H)-yl)piperidin-1-yl)-2-(4-(trifluoromethyl)phenyl)ethenone (GEN1-243)

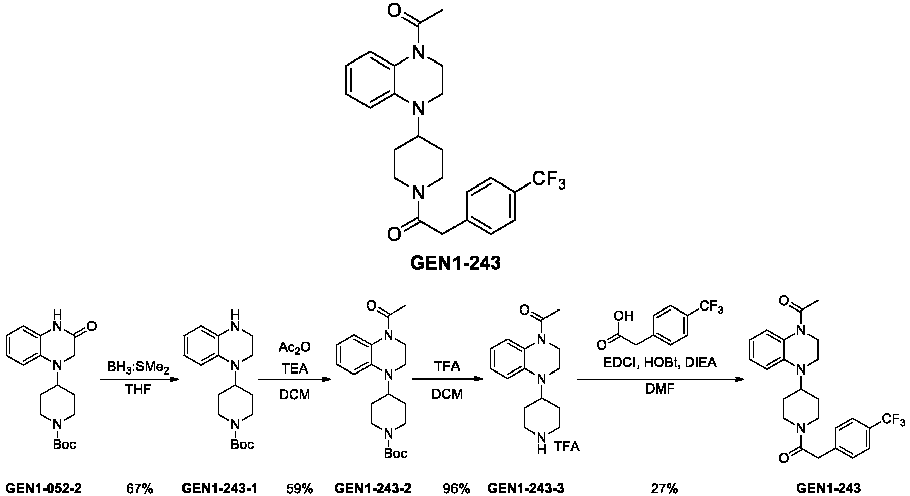

### Step 1. Synthesis of tert-butyl 4-(3,4-dihydroquinoxaline-1(2H)-yl)piperidin-1-carboxylate

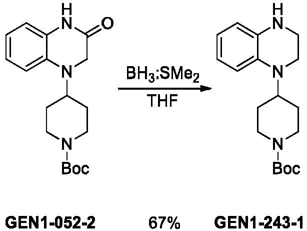

In an ice bath, a solution of borane dimethyl sulfide complex (10M) in dimethyl sulfide (3.0 mmol, 0.3 mL) was added dropwise to a solution of tert-butyl 4-(3-oxo-3,4-dihydroquinoxaline-1(2H)-yl)piperidin-1-carboxylate(200 mg, 0.603 mmol, the synthesis steps could be found in GEN1-052-2) in anhydrous THF (5 mL). The resulting mixture was stirred at room temperature overnight. MeOH (0.5 mL) was added dropwise in an ice bath, and the mixture was stirred for 20 minutes. The reaction mixture was concentrated and the residue was purified by column chromatography on silica gel (petroleum ether: EtOAc = 1:1) to obtain the desired compound (150 mg, yield 67%) as a brown oil. ¹HNMR (400 MHz, CDCl₃): δ 7.20-7.18 (d, *J =* 8.0 Hz, 1H), 7.11 (t, *J* = 8.4 Hz,, 1H), 6.78 (d, *J =* 8.0 Hz, 1H), 6.66-6.62 (m, 1H), 5.51 (s, 1H), 4.33-4.20 (m, 2H), 3.91-3.84 (m, 1H), 3.83-3.76 (m, 1H), 3.44-3.35 (m, 2H), 3.28-3.23 (m, 1H), 2.84-2.76 (m, 2H), 1.85-1.69 (m, 4H), 1.48 (s, 9H).

### Step 2. Synthesis of tert-butyl 4-(4-acetyl-3,4-dihydroquinoxaline-1(2H)-yl)piperidin-1-carboxylate

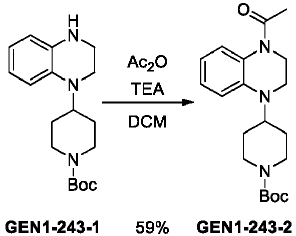

Ac2O (72 mg, 0.71 mmol) was added to a solution of tert-butyl 4-(3,4-dihydroquinoxaline-1(2H)-yl)piperidin-1-carboxylate (150 mg, 0.473 mmol) and TEA (143 mg, 1.41 mmol) in DCM (5 mL) in an ice bath. The resulting mixture was stirred at room temperature for 2 hours, and then diluted with DCM (30 mL). The organic layer was washed with water (20 mL), dried over MgSO₄ and filtered. The filtrate was concentrated to obtain the desired compound (110 mg, yield 59%) as a brown oil. MS (ESI): Rt = 1.61 min, *m*/*z* 260.0 [M+H-100]⁺; Purity: 91% @ 254 nm, 75% @ 214 nm.

### Step 3. Synthesis of 1-(4-(piperidin-4-yl)-3,4-dihydroquinoxaline-1(2H)-yl)ethanone 2,2,2-trifluoroacetate

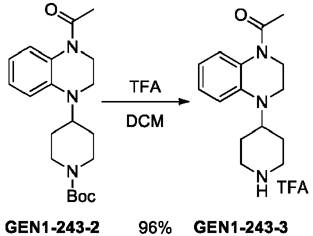

TFA (1 mL) was added to a solution of tert-butyl 4-(4-acetyl-3,4-dihydroquinoxaline-1(2H)-yl)piperidin-1-carboxylate (110 mg, 0.306 mmol) in DCM (5 mL). The reaction mixture was stirred for 1.5 hours at room temperature, and then concentrated to obtain the desired compound (110 mg, yield 96%) as a brown oil. MS (ESI): Rt = 1.16 min, *m*/*z* 260.2 [M+H]⁺; Purity: 100% @ 254 nm, 100% @ 214 nm.

### Step 4. Synthesis of 1-(4-(4-acetyl-3,4-dihydroquinoxaline-1(2H)-yl)piperidin-1-yl)-2-(4-(trifluoromethyl)phenyl)ethenone (GEN1-243)

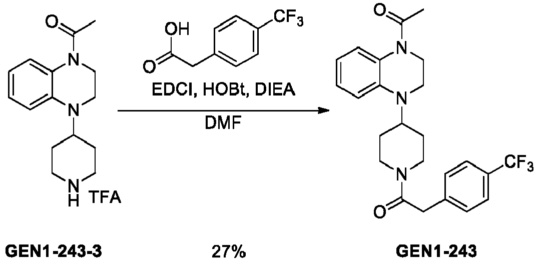

EDCI (85 mg, 0.44 mmol) was added to a solution of 1-(4-(piperidin-4-yl)-3,4-dihydroquinoxaline-1(2H)-yl)ethanone 2,2,2-trifluoroacetate (110 mg, 0.295 mmol), 4-(trifluoromethyl)phenyl)acetic acid (66 mg, 0.32 mmol), HOBt (60 mg, 0.44 mmol) and DIEA (114 mg, 0.882 mmol) in DMF (3 mL). The resulting mixture was stirred at room temperature overnight. The reaction mixture was poured into water (50 mL), and extracted with EtOAc (30 mL×3). The combined organic layer was washed with brine (30 mL), dried over MgSO₄, filtered and it was concentrated. The crude product was purified by a C18 column (CH₃CN: water = 5% to 50%) to obtain the desired compound (36.2 mg, yield 27%) as a white solid. ¹HNMR (400 MHz, CDCl₃): δ 7.60 (d, *J =* 8.4 Hz, 2H), 7.39 (d, *J =* 8.0 Hz, 2H), 7.08 (t, *J* = 8.0 Hz, 1H), 7.04-6.97 (m, 1H), 6.72 (d, *J =* 8.0 Hz, 1H), 6.66 (t, *J =* 8.0 Hz, 1H), 4.86-4.82 (m, 1H), 4.02-3.97 (m, 1H), 3.89-3.77 (m, 5H), 3.28-3.18 (m, 2H), 3.15-3.08 (m, 1H), 2.69-2.62 (m, 1H), 2.22 (s, 3H), 1.88-1.78 (m, 2H), 1.70-1.60 (m, 1H), 1.51-1.37 (m, 1H). MS (ESI): Rt = 3.783 min, *m*/*z* 446.2 [M+H]⁺; Purity: 99.37% @ 254 nm, 99.57% @ 214 nm.

### Example 139: 8-(trifluoromethyl)-1-(1-(2-(4-(trifluoromethyl)phenyl)acetyl)piperidin-4-yl)-3,4-dihydroquinazolin-2(1H)-one (GEN1-265)

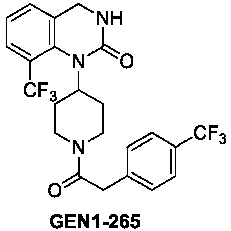

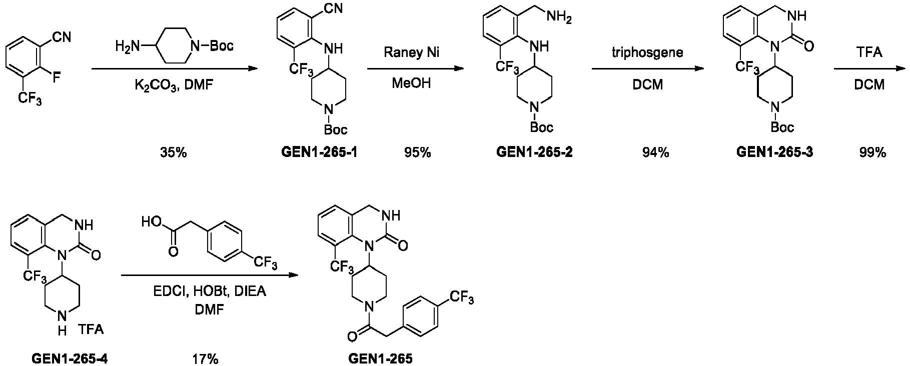

### Step 1. Synthesis of tert-butyl 4-((2-cyano-6-(trifluoromethyl)phenyl)amino)piperidin-1-carboxylate

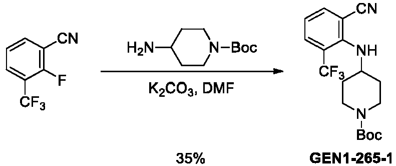

2-fluoro-3-(trifluoromethyl)benzonitrile (500 mg, 2.64 mmol), tert-butyl 4-aminopiperidine-1-carboxylate (556 mg, 2.78 mmol) and K₂CO₃ (548 mg, 3.96 mmol) were stirred in DMF (5 mL) at 85°C for 3 hours. The mixture was poured into water (50 mL), and extracted with EtOAc (30 mL×3). The combined organic layer was washed with brine (30 mL), dried over MgSO₄, filtered and it was concentrated. The crude product was purified by column chromatography on silica gel (petroleum ether: EtOAc = 5:1) to obtain the desired compound (450 mg, yield 35%) as a colorless oil. ¹HNMR (400 MHz, CDCl₃): δ 7.65 (dd, *J=* 8.0 Hz, 3.6 Hz, 2H), 6.87 (t, *J* = 8.0 Hz, 1H), 4.33-4.31 (m, 1H), 4.26-4.16 (m, 1H), 4.13-4.02 (m, 2H), 2.96-2.90 (m, 2H), 2.11-2.08 (m, 2H), 1.47 (s, 9H), 1.45-1.35 (m, 2H).

### Step 2. Synthesis of tert-butyl 4-((2-(aminomethyl)-6-(trifluoromethyl)phenyl)amino)piperidin-1-carboxylate

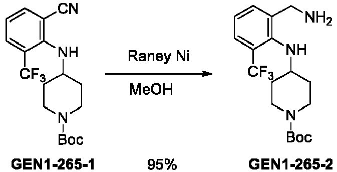

Raney nickel (20 mg) was added to a solution of tert-butyl 4-(((2-cyano-6-(trifluoromethyl)phenyl)amino)piperidin-1-carboxylate (100 mg, 0.271 mmol) and ammonium hydroxide (0.5 mL, 25%) in MeOH (5 mL). The reaction mixture was stirred under H₂ atmosphere for 2 hours. The reaction mixture was filtered, and the filtrate was concentrated to obtain the desired compound (100 mg, yield 95%) as a colorless oil. MS (ESI): Rt = 1.66 min, m/z 318.3 [M+H-56]⁺; Purity: 100% @ 254 nm, 95% @ 214 nm.

### Step 3. Synthesis of tert-butyl 4-(2-oxo-8-(trifluoromethyl)-3,4-dihydroquinazolin-1(2H)-yl)piperidin-1-carboxylate

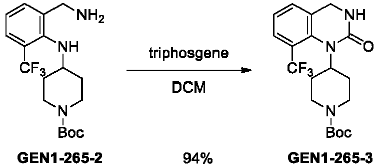

In an ice bath, triphosgene (32 mg, 0.11 mmol) was added to a solution of tert-butyl 4-((2-(aminomethyl)-6-(trifluoromethyl)phenyl)amino)piperidin-1-carboxylate (100 mg, 0.268 mmol) and NaHCO₃ (112 mg, 1.33 mmol) in DCM (10 mL). The reaction mixture was stirred overnight. The reaction mixture was poured into water (50 mL), and extracted with DCM (30 mL×3). The combined organic layer was dried over MgSO₄, filtered and it was concentrated to obtain the desired compound (130 mg, yield 94%) as a colorless oil. MS (ESI): Rt = 1.64 min, *m*/*z* 344.3 [M+H-56]⁺; Purity: 77% @ 254 nm, 53% @ 214 nm.

### Step 4. Synthesis of 1-(piperidin-4-yl)-8-(trifluoromethyl)-3,4-dihydroquinazolin-2(1H)-1-one 2,2,2-trifluoroacetate

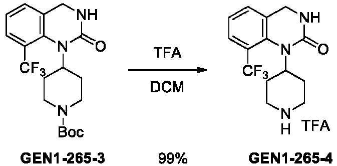

TFA (0.6 mL) was added to a solution of tert-butyl 4-(2-oxo-8-(trifluoromethyl)-3,4-dihydroquinazolin-1(2H)-yl)piperidin-1-carboxylate (130 mg, 0.251 mmol) in DCM (3 mL). The resulting mixture was stirred at room temperature for 2 hours. The reaction mixture was concentrated to obtain the desired compound (110 mg, yield 99%) as a brown oil.

MS (ESI): Rt = 1.34 min, m/z 299.9 [M+H]⁺; Purity: 94% @ 254 nm, 85% @ 214 nm.

### Step 5. Synthesis of 8-(trifluoromethyl)-1-(1-(2-(4-(trifluoromethyl)phenyl)acetyl)piperidin-4-yl)-3,4-dihydroquinazolin-2(1H)-one (GEN1-265)

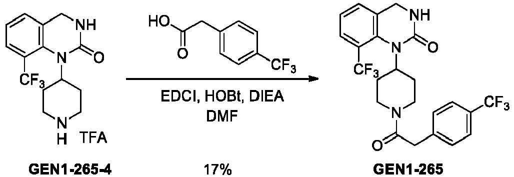

In an ice bath, EDCI (101 mg, 0.527 mmol) was added to a solution of 1-(piperidin-4-yl)-8-(trifluoromethyl)-3,4-dihydroquinazolin-2(1H)-1-one 2,2,2-trifluoroacetate (86 mg, 0.42 mmol), HOBt (71 mg, 0.53 mmol) and DIEA(136 mg, 1.05 mmol) in DMF (3 mL). The resulting mixture was stirred overnight. The reaction mixture was poured into water (50 mL), and extracted with EtOAc (20 mL×3). The combined organic layer was washed with brine (20 mL), dried with MgSO₄, filtered and it was concentrated. The crude product was purified by a C18 column (CH₃CN: water = 5% to 60%) to obtain the desired compound (30.2 mg, yield 17%) as a white solid. ¹HNMR (400 MHz, DMSO-*d*₆): δ 7.67-7.65 (m, 3H), 7.52 (d, *J =* 7.2 Hz, 1H), 7.44 (d, *J =* 8.0 Hz, 2H), 7.36 (s, 1H), 7.28 (t, *J =* 8.0 Hz, 1H), 4.47-4.44 (m, 1H), 4.20-4.12 (m, 2H), 4.06-4.02 (m, 1H), 3.89-3.80 (m, 2H), 3.51-3.41 (m, 1H), 2.95-2.89 (m, 1H), 2.46-2.42 (m, 1H), 2.33-2.22 (m, 1H), 2.19-2.10 (m, 1H), 1.95-1.89 (m, 2H). MS (ESI): Rt = 10.440 min, *m*/*z* 486.2 [M+H]⁺, Purity: 98.30% @ 254 nm, 95.91% @ 214 nm.

### Example 140: 7-(trifluoromethyl)-1-(1-(2-(4-(trifluoromethyl)phenyl)acetyl)piperidin-4-yl)-3,4-dihydroquinazolin-2(1H)-one (GEN1-266)

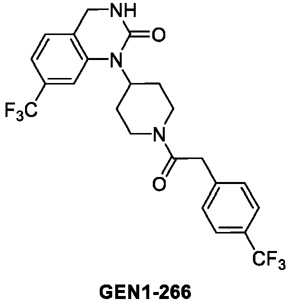

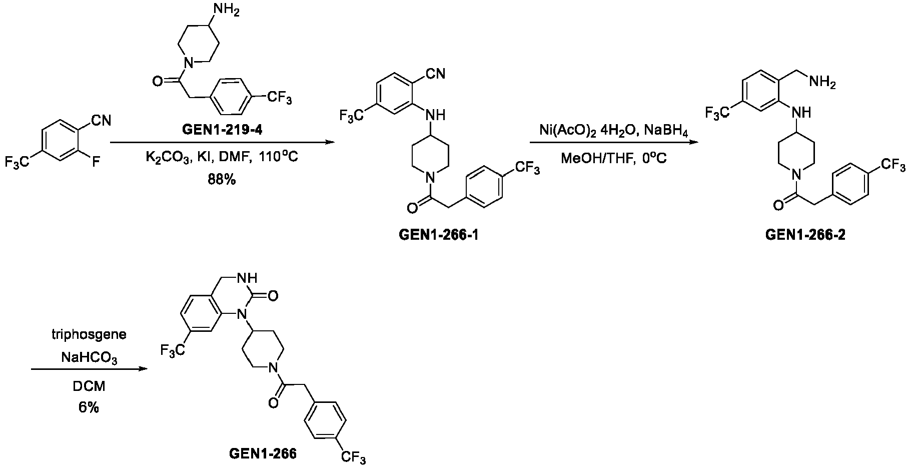

### Step 1. 4-(trifluoromethyl)-2-((1-(2-(4-(trifluoromethyl)phenyl)acetyl)piperidin-4-yl)amino)benzonitrile (GEN1-266-1)

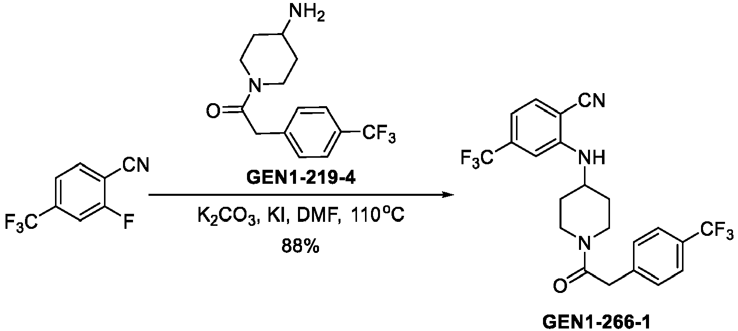

2-fluoro-4-(trifluoromethyl)benzonitrile (150 mg, 0.79 mmol), 1-(4-aminopiperidin-1-yl)-2-(4-(trifluoromethyl)phenyl)ethan-1-one, K₂CO₃ (219 mg, 1.58 mmol) and KI (132 mg, 0.79 mmol) were suspended in DMF (3.0 mL) and the suspension was stirred at 110°C for 5 hours. The reaction mixture was cooled, poured into 20 ml of water, and extracted with EA (25 mL×3). The organic layer was washed with brine (70 mL), dried over Na₂SO₄ and it was concentrated to obtain a pale yellow oil. The crude product was purified by silica gel column chromatography and eluted with (EA/(EA + PE)=30%) to obtain the desired compound (325 mg, 88% yield) as a pale yellow solid. MS (ESI): Rt=1.73 min, m/z 456.2 [M+H]⁺ , Purity: 93% @ 254 nm, 92% @ 214 nm.

### Step 2. 1-(4-((2-(aminomethyl)-5-(trifluoromethyl)phenyl)amino)piperidin-1-yl)-2-(4-(trifluoromethyl)phenyl)ethan-1-one (GEN1-266-2)

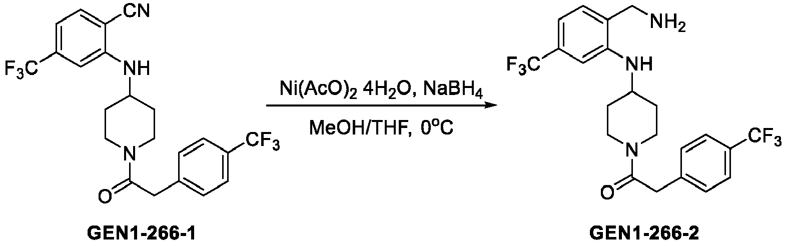

NaBH4 (108 mg, 2.85 mmol) was added in batches to a mixed solution of 4-(trifluoromethyl)-2-((1-(2-(4-(trifluoromethyl)phenyl)acetyl)piperidin-4-yl)amino)benzonitrile (325 mg, 0.71 mmol) and Ni(AcO)₂ 4H₂O (355 mg, 1.42 mmol) in MeOH (24 mL) and THF (12 mL) at 0°C. The resulting mixture was stirred at 0°C under N₂ atmosphere for 0.5 hour. The reaction mixture was poured into aqueous NH₄Cl solution (50 mL), and extracted with EA (55 mL×3). The organic layer was washed with water (150 mL), brine (150 mL), dried over Na₂SO₄ and it was concentrated to obtain a yellow solid (386 mg) which was used directly in the next step without further purification. TLC: EA/(PE+EA)=50%, Rf=0.3

### Step 3. 7-(trifluoromethyl)-1-(1-(2-(4-(trifluoromethyl)phenyl)acetyl)piperidin-4-yl)-3,4-dihydroquinazolin-2(1H)-one (GEN1-266)

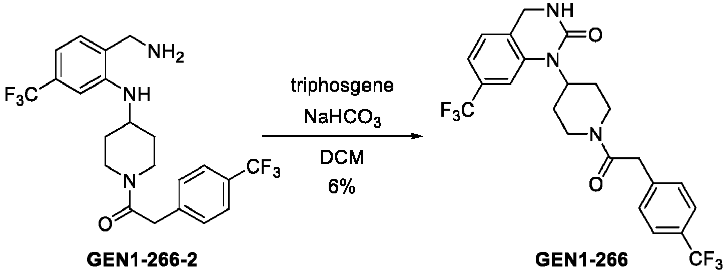

Triphosgene (147 mg, 0.50 mmol) was added in batches to a solution of 1-(4-((2-(aminomethyl)-5-(trifluoromethyl)phenyl)amino)piperidin-1-yl)-2-(4-(trifluoromethyl)phenyl)ethan-1-one (386 mg crude product) and NaHCO₃ (420 mg, 5.0 mmol) in DCM (50 mL) at 0°C. The resulting mixture was stirred at room temperature under N₂ atmosphere for 5 hours. The reaction mixture was poured into water (100 mL), and extracted with DCM (100 mL×3). The organic layer was washed with water (300 mL), brine (300 mL), dried over Na₂SO₄ and it was concentrated to obtain a pale yellow solid. The crude product was purified by reverse-phase flash chromatography (C₁₈ reverse silica column; mobile phase CH₃CN, water, 15% to 70% gradient, 50 minutes; detection, UV 214 nm) to obtain the desired compound (20 mg, yield 6%) as a white solid. ¹H NMR (400 MHz, DMSO-d₆) δ: 7.67 (d, *J*=8.0 Hz, 2H), 7.47 (d, *J*=7.9 Hz, 2H), 7.40 (d, *J*=7.8 Hz, 1H), 7.33 (d, *J*=7.8 Hz, 1H), 7.30-7.21 (m, 2H), 4.50 (d, *J*=12.9 Hz, 1H), 4.19 (s, 2H), 4.17-3.99 (m, 1H), 3.95 - 3.81 (m, 2H), 3.20 (t, *J*=12.9 Hz, 1H), 2.73 (t, *J*=12.7 Hz, 1H), 2.41 (t, *J*=12.3 Hz, 2H), 1.74 (d, *J*=12.4 Hz, 2H). MS (ESI): Rt=1.61 min, m/z 486.1 [M+H]⁺, Purity: 100% @ 254 nm, 100% @ 214 nm.

### Example 141: Synthesis of 1-(1-(2-(quinolin-6-yl)acetyl)azetidin-3-yl)-7-(trifluoromethyl)-1H-benzo[d]imidazol-2(3H)- (GEN1-267)

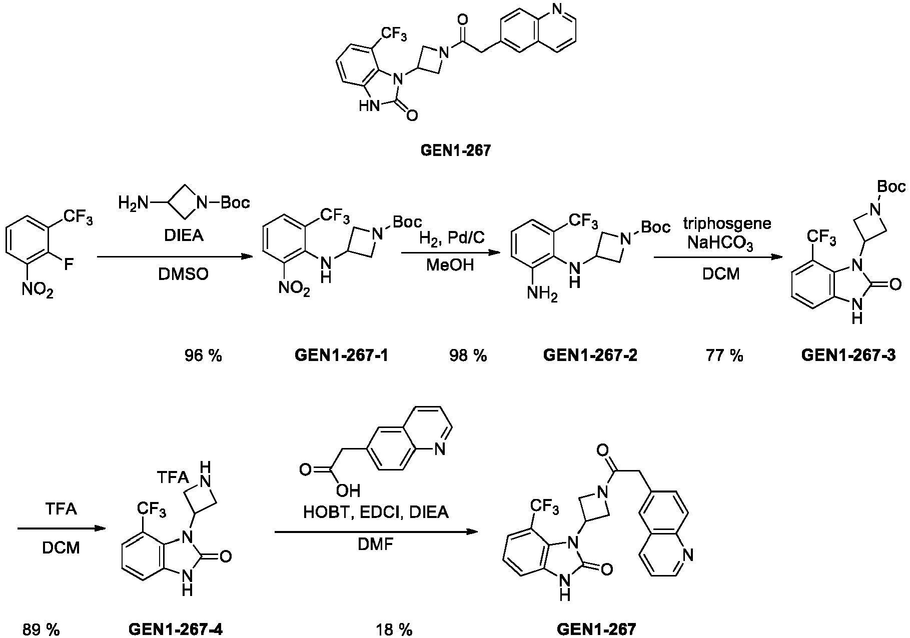

### Step 1. Synthesis of tert-butyl 3-((2-nitro-6-(trifluoromethyl)phenyl)amino)azetidine-1-carboxylate

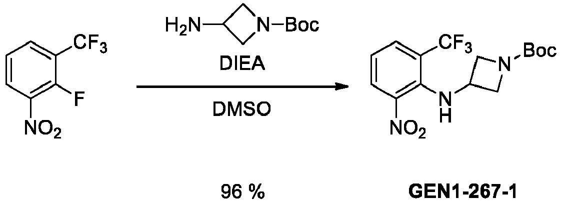

3-aminoazetidine-1-tert-butyl ester (494 mg, 2.87 mmol) was added to a solution of 2-fluoro-1-nitro-3-(trifluoromethyl)benzene (300 mg, 1.43 mmol) and DIEA (371 mg, 2.87 mmol) in DMSO (4 mL). The mixture was stirred at 75°C for 4 hours, and then poured into water (20 mL), and extracted with EtOAc (15 mL×3). The combined organic layer was washed with brine (20 mL), dried over anhydrous Na₂SO₄ and filtered. The filtrate was concentrated to obtain the desired compound (500mg, yield 96%) as a yellow oil. MS (ESI): Rt = 1.73 min, m/z 306.1 [M-56+H]⁺; Purity: 100% @254nm, 100% @214nm.

### Step 2. Synthesis of tert-butyl 3-((2-amino-6-(trifluoromethyl)phenyl)amino)azetidine-1-carboxylate

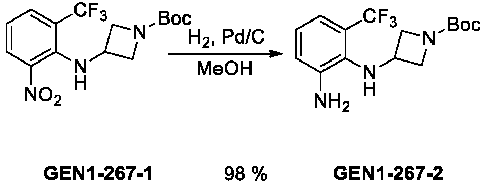

Pd/C (10%, 147 mg) was added to a solution of tert-butyl 3-((2-nitro-6-(trifluoromethyl)phenyl)amino)azetidine-1-carboxylate (500 mg, 1.38 mmol) in MeOH (5 mL). The mixture was stirred at room temperature under an H₂ atmosphere (balloon) for 1.5 hours. Then the mixture was filtered, and the filtrate was concentrated to obtain (450 mg, yield 98%) a pink solid. MS (ESI): Rt = 1.68 min, m/z 276.1 [M-56+H]⁺; Purity: 100%, @254nm, 100%, @214nm.

### Step 3. Synthesis of tert-butyl 3-(2-oxo-7-(trifluoromethyl)-2,3-dihydro-1H-benzo[d]imidazol-1-yl)azetidine-1-carboxylate

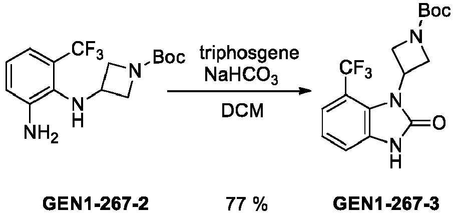

NaHCO₃ (456 mg, 5.43 mmol) was added to a solution of tert-butyl 3-((2-amino-6-(trifluoromethyl)phenyl)amino)azetidine-1-carboxylate (450 mg, 1.36 mmol) and triphosgene (524 mg, 1.77 mmol) in DCM (5 mL) at room temperature. The mixture was stirred at room temperature for 4 hours. Then the mixture was poured into water (15 mL), and extracted with EtOAc (10 mL×2). The combined organic layer was washed with brine (15 mL), dried over anhydrous Na₂SO₄ and filtered. The filtrate was concentrated to obtain the desired compound (490 mg, yield 77%) as a white solid. MS (ESI): Rt = 1.61 min, m/z 302.1 [M-56+H]⁺; Purity: 44%, @254nm, 76%, @214nm.

### Step 4. Synthesis of 1-(azetidin-3-yl)-7-(trifluoromethyl)-1H-benzo[d]imidazol-2(3H)-one 2,2,2-trifluoroacetate

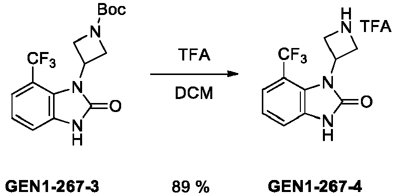

TFA (0.5 mL) was added to a solution of tert-butyl 3-(2-oxo-7-(trifluoromethyl)-2,3-dihydro-1H-benzo[d]imidazol-1-yl)azetidine-1-carboxylate (200 mg, 0.425 mmol) in DCM (5 mL). The mixture was stirred at room temperature overnight. The mixture was concentrated in vacuo to obtain the desired compound (150 mg, yield 89%) as a brown oil. MS (ESI): Rt = 1.16 min, m/z 258.1 [M+H]⁺; Purity: 24%, @254nm, 94%, @214nm.

### Step 5: Synthesis of 1-(1-(2-(quinolin-6-yl)acetyl)azetidin-3-yl)-7-(trifluoromethyl)-1H-benzo[d]imidazol-2(3H)-(GEN1-267)

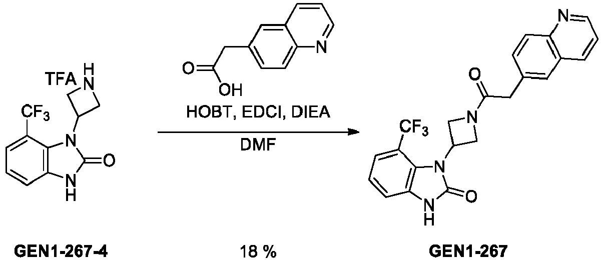

EDCI (109 mg, 0.569 mmol) was added to a solution of 2-(quinolin-6-yl)acetic acid (85 mg, 0.454 mmol) and HOBT (77 mg, 0.570 mmol) in DMF (5 mL). After the mixture was stirred at room temperature for 5 minutes, 1-(azetidin-3-yl)-7-(trifluoromethyl)-1H-benzo[d]imidazol-2(3H)-one 2,2,2-trifluoroacetate (150 mg, 0.380 mmol) and DIEA (147 mg, 1.14 mmol) were added. The mixture was stirred at room temperature for 4 hours. The reaction mixture was diluted with water (20 mL), and extracted with EtOAc (15 mL×3). The combined organic phase was washed with brine (20 mL), dried over anhydrous Na₂SO₄ and it was concentrated. The residue was purified by preparation. HPLC [RP-PREP-9 Xbridge C18, 5um, 19 * 150 mm, 15∼40% B, A: water (0.1% TFA), B: CAN; flow rate: 16mL / min, UV = 2× 14 nm]. The desired compound (29.8 mg, yield 18%) was obtained as a white solid. ¹HNMR (400 MHz, DMSO-*d*₆): δ 11.63 (s, 1H), 8.87 (dd, *J* = 4.0, 1.6 Hz, 1H), 8.83 (d, *J* = 9.2 Hz, 1H), 7.97 (d, *J* = 8.8 Hz, 1H), 7.86 (d, *J* = 1.6 Hz, 1H), 7.69 (dd, *J* = 8.8, 2.0 Hz, 1H), 7.52 (dd, *J* = 8.4, 4.0 Hz, 1H), 7.36 (d, *J=* 8.0 Hz, 1H), 7.31 (d, *J* = 7.2 Hz, 1H), 7.19 (t, *J* = 8.0 Hz, 1H), 5.19-5.11 (m, 1H), 5.02-4.99 (m, 1H), 4.65 (dd, *J* = 9.6, 6.0 Hz, 1H), 4.59 (t, *J* = 8.8 Hz, 1H), 4.20 (t, *J* = 8.8 Hz, 1H), 3.71 (s, 2H).MS (ESI): Rt = 3.048 min, m/z 427.1 [M+H]⁺; Purity: 97.29% @254nm, 97.56% @214nm.

### Example 142: 7-(trifluoromethyl)-1-(1-(2-((1R,4R)-4-(trifluoromethyl)cyclohexyl)acetyl)piperidin-4-yl)-1H-benzo[d]imidazol-2(3H)-one and 7-(trifluoromethyl)-1-(1-(2-(((1s,4s)-4-(trifluoromethyl)cyclohexyl)acetyl)piperidin-4-yl)-1H-benzo[d]imidazol-2(3H)-one (GEN1-268 and GEN1-269)

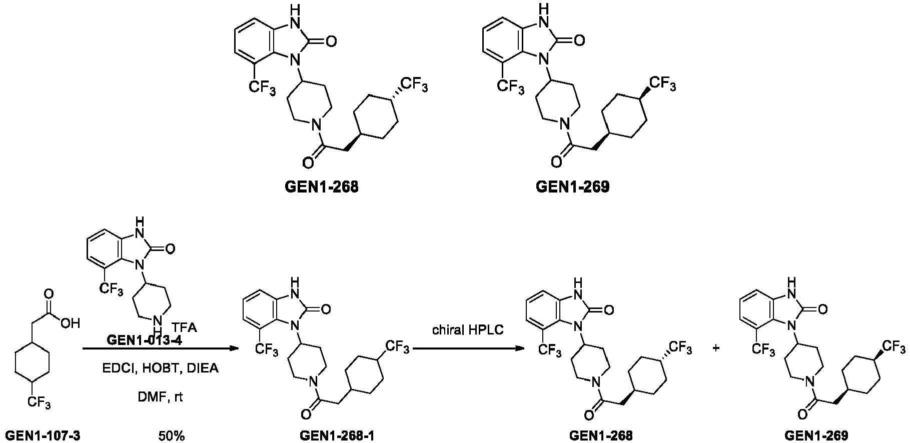

### Step 1. Synthesis of 7-(trifluoromethyl)-1-(1-(2-(4-(trifluoromethyl)cyclohexyl)acetyl)piperidin-4-yl)-1H-benzo[d]imidazol-2(3H)-one

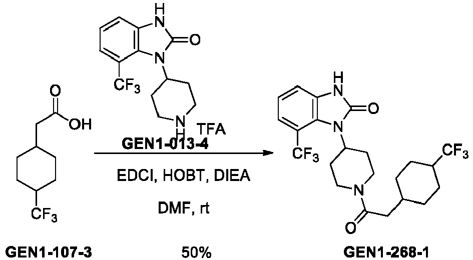

EDCI (180 mg, 0.940 mmol) was added a solution of 1-(piperidin-4-yl)-7-(trifluoromethyl)-1H-benzo[d]imidazol-2(3H)-one,2,2,2-trifluoroacetate (250 mg, 0.626 mmol, the synthesis steps could be found in GEN1-013-4), 2-(4-(trifluoromethyl)cyclohexyl)acetic acid (145 mg, 0.690 mmol, the synthesis steps could be found in GEN1-107-3), HOBt (127 mg, 0.940 mmol) and DIEA (243 mg, 1.88 mmol) in DMF (5 mL). The resulting mixture was stirred at room temperature overnight. The mixture was poured into water (50 mL), and extracted with EtOAc (30 mL×3). The combined organic layer was washed with brine (30 mL), dried over MgSO₄, filtered and it was concentrated. The crude product was purified by a C18 column (CH₃CN: water = 5% to 65%) to obtain the desired compound (150 mg, yield 50%) as a white solid. MS (ESI): Rt = 1.67 min, *m*/*z* 477.9 [M+H] ⁺, Purity: 100% @ 214 nm.

### Step 2. Synthesis of 7-(trifluoromethyl)-1-(1-(2-((1R,4R)-4-(trifluoromethyl)cyclohexyl)acetyl)piperidin-4-yl)-1H-benzo[d]imidazol-2(3H)-one and 7-(trifluoromethyl)-1-(1-(2-(((1s,4s)-4-(trifluoromethyl)cyclohexyl)acetyl)piperidin -4-yl)-1H-benzo[d]imidazol-2(3H)-one (GEN1-268 and GEN1-269)

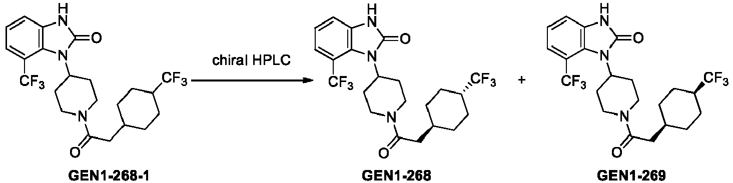

7-(trifluoromethyl)-1-(1-(2-(4-(trifluoromethyl)cyclohexyl)acetyl)piperidin-4-yl)-1H-benzo[d]imidazol-2(3H)-one (150 mg, 0.314 mmol) was separated by chiral HPLC (OD-H, UV 214 nm, Hex / EtOH: 95/5) to obtain the desired peak 1 (GEN1-269, 37.3 mg, yield 25%) and the desired peak 2(GEN1- 268, 50.8 mg, yield 34%) , both of which were white solids. The absolute configuration of the compound was determined by comparing the reported values of similar structures based on nuclear magnetic data and literature (J. Am. Chem. Soc. 2017, 139, 7, 2577-2580). **Peak 1 (GEN1-268):** ¹HNMR (400 MHz, DMSO-*d*₆): δ 11.44 (s, 1H), 7.37 (d, *J* = 8.4 Hz, 1H), 7.28 (d, *J* = 6.8 Hz, 1H), 7.16 (t, *J* = 8.0 Hz, 1H), 4.61-4.59 (m, 1H), 4.30-4.22 (m, 1H), 4.07-4.04 (m, 1H), 3.04-2.98 (m, 1H), 2.62-2.52 (m, 1H), 2.47-2.37 (m, 2H), 2.27-2.25 (m, 2H), 2.23-2.13 (m, 1H), 1.87-1.82 (m, 4H), 1.74-1.67 (m, 3H), 1.31-1.21 (m, 2H), 1.10-0.96 (m, 2H). MS (ESI): Rt = 4.353 min, *m*/*z* 478.2 [M+H]⁺; Purity: 97.40% @ 254 nm, 99.61% @ 214 nm. Chiral analysis (Method: Column: Chiralpak OD-H, 5 um 4.6 * 250 mm; Mobile Phase: hexane: ethanol = 95: 5 at 1 mL/ min; Temp: 30°C; Wavelength: 214 nm), Rt = 13.799 min, 100% stereopure.

**Peak 2 (GEN1-269):** ¹H NMR (400 MHz, DMSO-*d*₆): δ 11.45 (s, 1H), 7.37 (d, *J* = 8.4 Hz, 1H), 7.28 (d, *J* = 6.8 Hz, 1H), 7.16 (t, *J* = 8.0 Hz, 1H), 4.61-4.59 (m, 1H), 4.30-4.20 (m, 1H), 4.13-4.09 (m, 1H), 3.04-2.98 (m, 1H), 2.62-2.52 (m, 1H), 2.47-2.42 (m, 2H), 2.39-2.38 (m, 2H), 2.34-2.21 (m, 1H), 2.16-2.10 (m, 1H), 1.75-1.48 (m, 10H). MS (ESI): Rt = 4.336 min, *m*/*z* 478.2 [M+H]⁺; Purity: 97.23% @ 254 nm, 99.47% @ 214 nm. Chiral analysis (Method: Column: Chiralpak OD-H, 5 um 4.6 * 250 mm; Mobile Phase: hexane: ethanol = 95: 5 at 1 mL/ min; Temp: 30 °C; Wavelength: 214 nm), Rt = 16.731 min, 99.57% stereopure.

### Example 143: 1-(1-(2-(3-(methoxyphenyl)acetyl)piperidin-4-yl)-1,3-dihydro-2H-benzo[d]imidazol-2-one (GEN1-270)

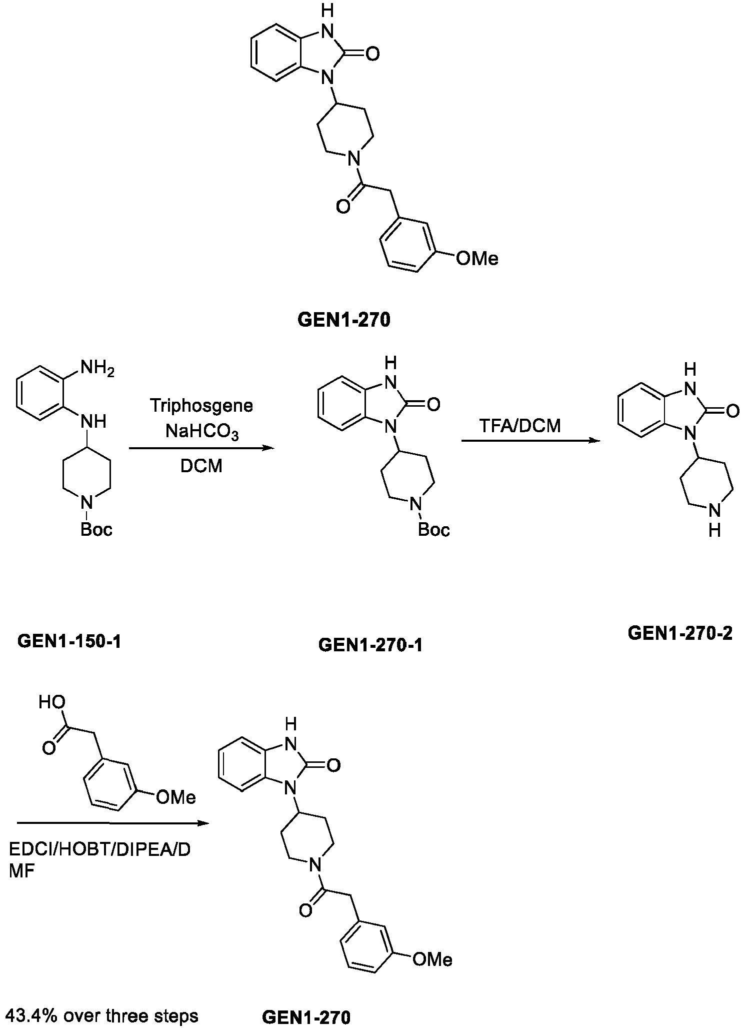

### Step 1. Synthesis of tert-butyl 4-(2-oxo-2,3-dihydro-1H-benzo[d]imidazol-1-yl)piperidin-1-carboxylate (GEN1-270-1)

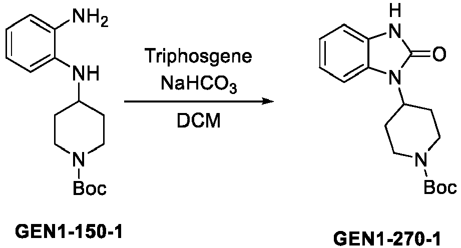

Triphosgene (303 mg, 1.03 mmol) was added in batches to a solution of tert-butyl 4-((2-aminophenyl)amino)piperidin-1-carboxylate (300 mg, 1.03 mmol) and NaHCO₃ (866 mg, 10.3 mmol) in DCM (12 mL) under nitrogen protection at 0°C. The resulting mixture was stirred overnight at room temperature under nitrogen protection. The reaction mixture was poured into water (20 mL) and extracted with DCM (25 mL×3). The organic layer was washed with water (50 mL), brine (50 mL), dried over Na₂SO₄ and it was concentrated to obtain the desired crude product (256 mg) as a brown solid. It was used directly in the next step without purification.

### Step 2. Synthesis of 1-(piperidin-4-yl)-1,3-dihydro-2H-benzo[d]imidazol-2-one (GEN1-270-2)

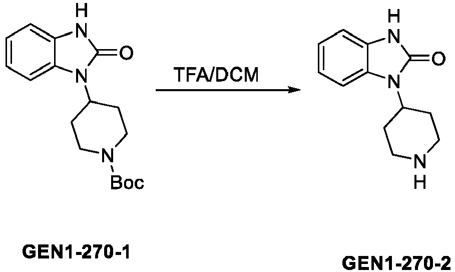

TFA (2 mL) was added to a solution of tert-butyl 4-(2-oxo-2,3-dihydro-1H-benzo[d]imidazol-1-yl)piperidin-1-carboxylate (200 mg, 0.63 mmol) in DCM (10 mL).The resulting mixture was stirred at room temperature for 1 hour. The resulting mixture was concentrated and redissolved in DCM (20ml). Then the mixture was concentrated again to obtain the desired crude compound (200 mg) as a brown solid. MS (ESI): Rt = 0.31 min, *m*/*z* 218.1 [M+H]⁺.

### Step 3. Synthesis of 1-(1-(2-(3-(methoxyphenyl)acetyl)piperidin-4-yl)-1,3-dihydro-2H-benzo[d]imidazol-2-one (GEN1-270)

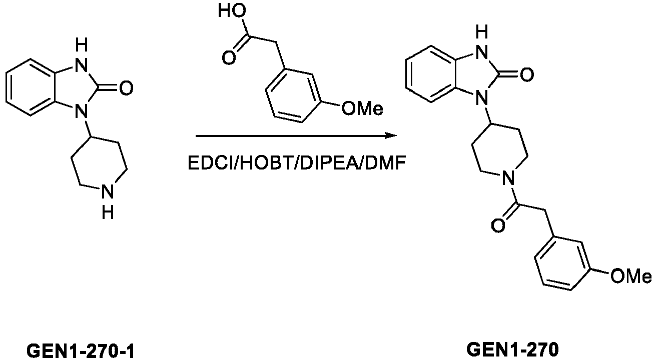

EDCI (90.7 mg, 0.473 mmol) was added to a solution of 1-(piperidin-4-yl)-1,3-dihydro-2H-benzo[d]imidazol-2-one (100 mg, crude product, 0.315 mmol), 2-(3-methoxyphenyl)acetic acid (62.8 mg, 0.378 mmol), HOBT (63.9 mg, 0.473 mmol) and DIEA (162.7 mg, 1.26 mmol) in DMF (5 mL). The resulting mixture was stirred at room temperature for 1 h. The reaction mixture was diluted with water (100 mL), and extracted with EtOAc (30 mL×3). The combined organic phase was washed with brine (50 mL), dried over anhydrous Na₂SO₄ and filtered. The crude product was obtained by concentrating. The crude product was purified by reverse phase chromatography. Purification conditions: C18 silica gel column; mobile phase ACN: H₂O, gradient: 5% to 50%; 45 minutes, monitoring wavelength: 214 nm. The desired compound (50 mg, two steps yield: 43.4%) was obtained as a white solid. ¹H NMR (400 MHz, DMSO-d6) δ 10.86 (s, 1H), 7.27 (t, J = 8.0 Hz, 1H), 7.00 - 6.91 (m, 4H), 6.91 - 6.86 (m, 2H), 6.84 - 6.79 (m, 1H), 4.57 (d, J = 13.1 Hz, 1H), 4.48-4.33 (m, 1H), 4.08 (d, J = 13.8 Hz, 1H), 3.85 - 3.67 (m, 5H), 3.14 (t, J = 12.4 Hz, 1H), 2.68 (t, J = 9.0 Hz, 1H), 2.11-1.82 (m, 2H), 1.73 - 1.52 (m, 2H). MS (ESI): Rt = 1.32 min, *m*/*z* 366.1 [M+H]⁺; Purity: Purity% @ 254 nm, 100% @ 214 nm.

### Example 144: 1-(1-(2-(2-(2-methoxyphenyl)acetyl)piperidin-4-yl)-1,3-dihydro-2H-benzo[d]imidazol-2-one (GEN1-271)

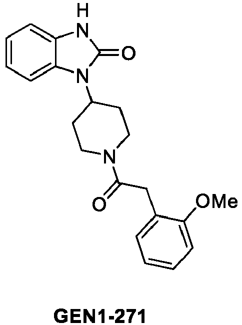

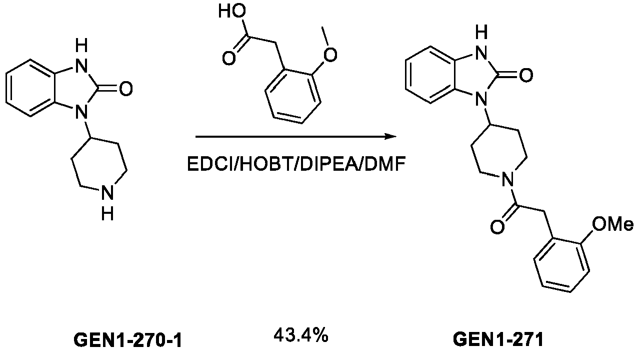

### Step 1. Synthesis of 1-(1-(2-(2-(2-methoxyphenyl)acetyl)piperidin-4-yl)-1,3-dihydro-2H-benzo[d]imidazol-2-one (GEN1-271)

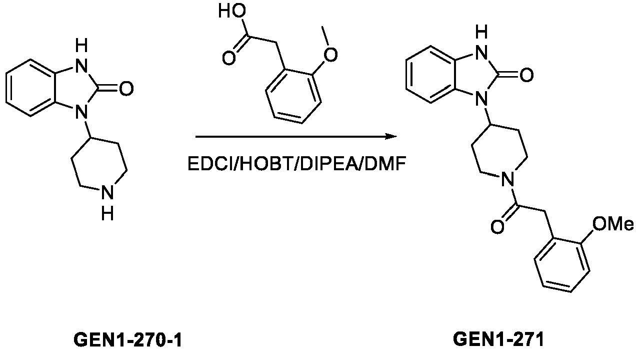

EDCI (90.7 mg, 0.473 mmol) was added to a solution of 1-(piperidin-4-yl)-1,3-dihydro-2H-benzo[d]imidazole-2-one (100 mg, crude product, 0.315 mmol, the synthesis steps could be found in GEN1-270-1), 2-(2-methoxyphenyl)acetic acid (62.8 mg, 0.378 mmol), HOBT (63.9 mg, 0.473 mmol) and DIEA (162.7 mg, 1.26 mmol) in DMF (5 mL). The resulting mixture was stirred at room temperature for 1 h. The reaction mixture was diluted with water (100 mL), and extracted with EtOAc (30 mL×3). The combined organic phase was washed with brine (50 mL), dried over anhydrous Na₂SO₄ and filtered. The filtrate was concentrated to obtain the crude product. The crude product was purified by reverse phase chromatography. Purification conditions: C18 silica gel column; mobile phase ACN: H₂O, gradient: 5% to 50%; 45 minutes, monitoring wavelength: 214 nm. The desired compound (50 mg, yield 43.4%) was obtained as a white solid. ¹H NMR (400 MHz, DMSO-d6) δ 10.85 (s, 1H), 7.28 -7.15 (m, 2H), 7.08 - 7.02 (m, 1H), 7.00 - 6.89 (m, 5H), 4.55 (d, J = 13.1 Hz, 1H), 4.46 - 4.34 (m, 1H), 4.03 (d, J = 13.7 Hz, 1H), 3.80 - 3.54 (m, 5H), 3.16 (t, J = 13.0 Hz, 1H), 2.68 (t, J = 12.6 Hz, 1H), 2.15-1.96 (m, 2H), 1.73-1.56 (m, 2H). MS (ESI): Rt = 1.39 min, *m*/*z* 366.1 [M+H]⁺; Purity: 100% @ 254 nm, 100% @ 214 nm.

### Example 145: 1-(1-(2-(1,2,3,4-tetrahydroquinolin-6-yl)acetyl)piperidin-4-yl)-7-(trifluoromethyl)-1,3-dihydro-2H-benzo[d]imidazol-2-one (GEN1-276)

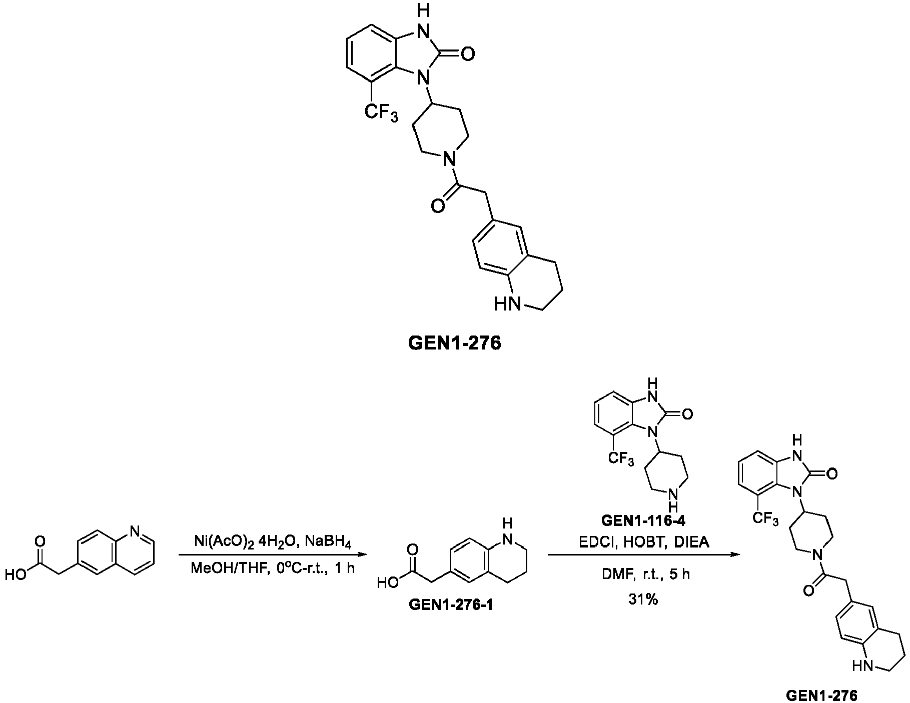

### Step 1. 2-(1,2,3,4-Tetrahydroquinolin-6-yl)acetic acid (GEN1-276-1)

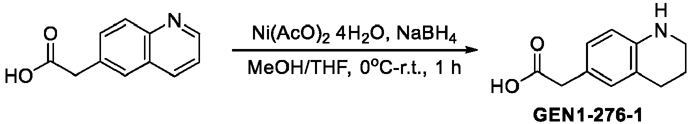

NaBH₄ (183 mg, 4.81 mmol) was added in batches to a mixed solution of 2-(quinolin-6-yl)acetic acid (150 mg, 0.80 mmol) and Ni(AcO)₂ 4H₂O (600 mg, 2.41 mmol) in MeOH (4.0 mL) and THF (2.0 mL) at 0°C. The resulting mixture was stirred at room temperature under N₂ atmosphere for 1 hour. The reaction mixture was poured into aqueous NH₄Cl solution (20 mL), and extracted with EA (25 mL×3). The organic layer was washed with water (80 mL), brine (80 mL), dried over Na₂SO₄ and it was concentrated to obtain a yellow solid (110 mg crude product) which was used directly in the next step without further purification. MS (ESI) Rt=0.27 min, m/z 192.2 [M+H]⁺.

### Step 2. 1-(1-(2-(1,2,3,4-tetrahydroquinolin-6-yl)acetyl)piperidin-4-yl)-7-(trifluoromethyl)-1,3-dihydro-2H-benzo[d]imidazol-2-one (GEN1-276)

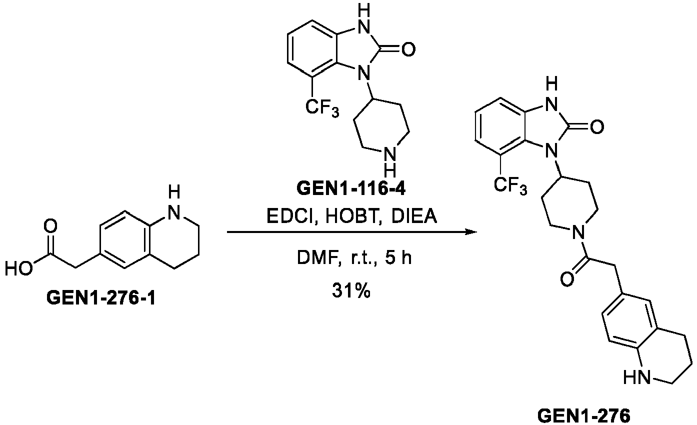

DIEA (414 mg, 3.21 mmol) was added to a solution of 1-(piperidin-4-yl)-7-(trifluoromethyl)-1,3-dihydro-2H-benzo[d]imidazol-2-one (228 mg, 0.80 mmol), 2-(1,2,3,4-tetrahydroquinolin-6-yl)acetic acid (110 mg crude product), EDCI (231 mg, 1.20 mmol) and HOBT (162 mg, 1.20 mmol) in DMF (5.0 mL). The resulting mixture was stirred at room temperature for 5 hours. The reaction mixture was poured into water (30 mL), and extracted with EA (25 mL×3). The combined organic layer was washed with water (80 mL), brine (80 mL), dried over Na₂SO₄ and it was concentrated to obtain a yellow oil. The crude product was purified by reverse-phase flash chromatography (C₁₈ reverse silica column; mobile phase CH₃CN, water, 10% to 75% gradient, 55 minutes; detection, UV 214 nm) to obtain the desired compound (115 mg, yield 31%) as a white solid. ¹H NMR (400 MHz, DMSO-*d*₆)δ: 11.42 (s, 1H), 7.34 (d, *J*=8.1, 1.2 Hz, 1H), 7.29-7.24 (m, 1H), 7.14 (t, *J*=7.9 Hz, 1H), 6.74-6.66 (m, 2H), 6.35 (d, *J*=7.9 Hz, 1H), 5.51 (s, 1H), 4.64-4.53 (m, 1H), 4.27-4.16 (m, 1H), 4.15-4.04 (m, 2H), 3.18-3.09 (m, 2H), 2.99-2.87 (m, 1H), 2.72-2.56 (m, 2H), 2.55-2.51 (m, 1H), 2.48-2.40 (m, 2H), 1.83-1.72 (m, 2H), 1.71-1.59 (m, 2H). MS (ESI) Rt=1.54 min, m/z 459.2 [M+H]⁺, Purity: 100% @ 254 nm, 100% @ 214 nm.

### Example 146: 6-phenyl-7-(trifluoromethyl)-1-(1-(2-(4-(trifluoromethyl)phenyl)acetyl)piperidin-4-yl)-1H-benzo[d]imidazol-2(3H)-one (GEN1-277)

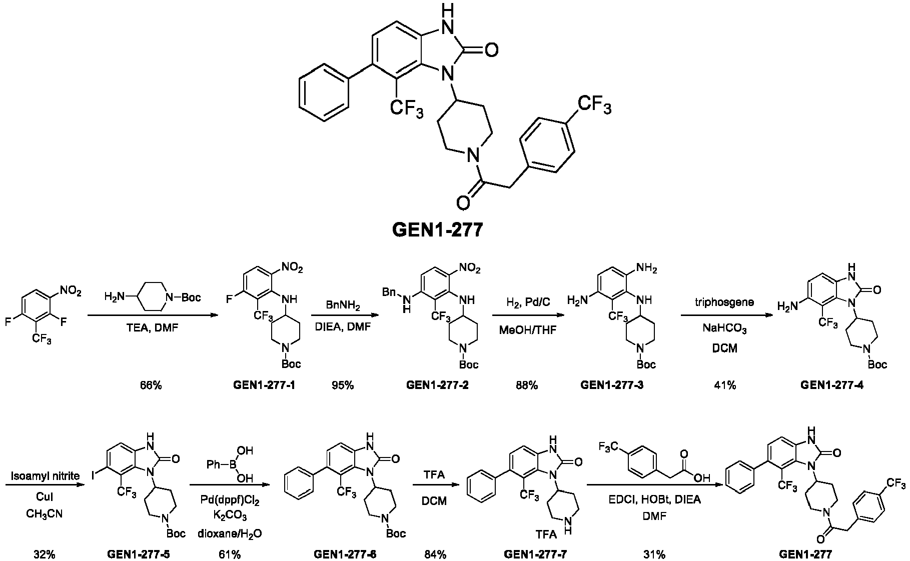

### Step 1. Synthesis of tert-butyl 4-((3-fluoro-6-nitro-2-(trifluoromethyl)phenyl)amino)piperidin-1-carboxylate

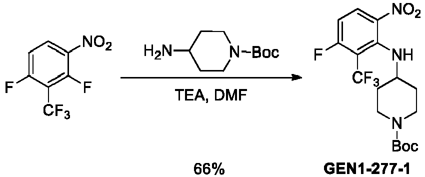

1,3-difluoro-4-nitro-2-(trifluoromethyl)benzene (2.0 g, 8.8 mmol), tert-butyl 4-aminopiperidine-1-carboxylate (1.76 g, 8.81 mmol) and TEA (1.34 g, 13.2 mmol) were stirred in DMF (40 mL) at room temperature overnight. The reaction mixture was poured into water (300 mL), and extracted with EtOAc (60 mL×3). The combined organic layer was washed with brine (60 mL), dried over MgSO₄, filtered and it was concentrated. The crude product was purified by column chromatography on silica gel (petroleum ether: EtOAc = 10:1 to 2:1) to obtain the desired compound (2.49 g, yield 66%) as a yellow solid.

¹HNMR (400 MHz, CDCl₃): δ 8.27 (dd, *J* = 12.4, 7.6 Hz, 1H), 7.05 (d, *J* = 13.6 Hz, 1H), 6.70 (t, *J* = 13.2 Hz, 1H), 4.14-3.93 (m, 2H), 3.49-3.34 (m, 1H), 2.86-2.78 (m, 2H), 1.95-1.91 (m, 2H), 1.45 (s, 9H), 1.42-1.30 (m, 2H).

### Step 2. Synthesis of tert-butyl 4-((3-(benzylamino))-6-nitro-2-(trifluoromethyl)phenyl)amino)piperidin-1-carboxylate

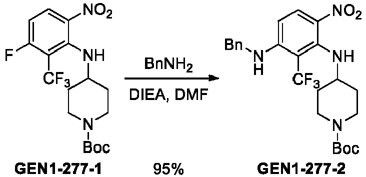

Tert-butyl 4-((3-fluoro-6-nitro-2-(trifluoromethyl)phenyl)amino)piperidin-1-carboxylate (2.49 g, 6.11 mmol), benzylamine (786 mg, 7.34 mmol) and DIEA (1.18 g, 9.15 mmol) were stirred in DMF (30 mL) at 85°C for 3 hours. The reaction mixture was poured into water (200 mL), and extracted with EtOAc (50 mL×3). The combined organic layer was washed with water (30 mL×2), brine (30 mL), dried over MgSO₄, filtered and it was concentrated to obtain the desired compound (3.03 g, yield 95%) as a yellow solid.

¹HNMR (400 MHz, CDCl₃): δ 8.13 (d, *J* = 9.6 Hz, 1H), 7.82 (d, *J* = 10.0 Hz, 1H), 7.41-7.30 (m, 5H), 6.23 (d, *J* = 10.0 Hz, 1H), 5.51 (s, 1H), 4.50 (d, *J* = 5.6 Hz, 2H), 4.08-3.91 (m, 2H), 3.41-3.32 (m, 1H), 2.86-2.80 (m, 2H), 1.93-1.90 (m, 2H), 1.45 (s, 9H), 1.42-1.33 (m, 2H).

### Step 3. Synthesis of tert-butyl 4-((3,6-diamino-2-(trifluoromethyl)phenyl)amino)piperidin-1-carboxylate

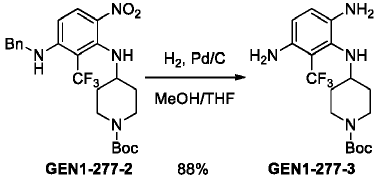

Pd/C (137 mg, purity 10%) was added to a solution of tert-butyl 4-(((3-(benzylamino))-6-nitro-2-(trifluoromethyl)phenyl)amino)piperidin-1-carboxylate (1.5 g, 2.6 mmol) in MeOH (15 mL). The reaction mixture was stirred overnight under H₂ atmosphere, and then filtered and the filtrate was concentrated. The crude product was purified by column chromatography on silica gel (petroleum ether: EtOAc = 2:1 to 1:1) to obtain the desired compound (971 mg, yield 88%) as a light brown oil. MS (ESI): Rt = 1.58 min, *m*/*z* 319.3 [M+H-56]⁺; Purity: 100% @ 254 nm, 87% @ 214 nm.

### Step 4. Synthesis of tert-butyl 4-(6-amino-2-oxo-7-(trifluoromethyl)-2,3-dihydro-1H-benzo[d]imidazol-1-yl)piperidin-1-carboxylate

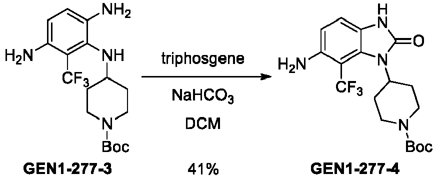

In an ice bath, a solution of triphosgene (234 mg, 0.789 mmol) in DCM (5 mL) was added dropwise to a suspension of tert-butyl 4-((3,6-diamino-2-(trifluoromethyl)phenyl)amino)piperidine-1-carboxylate (971 mg, 2.26 mmol) and NaHCO₃ (948 mg, 11.3 mmol). The resulting mixture was stirred at 15°C for 1 hour. The reaction mixture was poured into water (50 mL), and extracted with DCM (20 mL×3). The combined organic layer was washed with brine (20 mL), dried with MgSO₄, filtered and it was concentrated. The crude product was purified by column chromatography on silica gel (petroleum ether: EtOAc = 1:1 to 1:3) to obtain the desired compound (410 mg, yield 41%) as a white solid. ¹HNMR (400 MHz, CDCl₃): δ 10.14 (s, 1H), 7.01 (d, *J* = 8.4 Hz, 1H), 6.46 (d, *J* = 8.4 Hz, 1H), 4.36-4.07 (m, 5H), 2.83-2.68 (m, 4H), 1.77-1.65 (m, 2H), 1.49 (s, 9H).

### Step 5. Synthesis of tert-butyl 4-(6-iodo-2-oxo-7-(trifluoromethyl)-2,3-dihydro-1H-benzo[d]imidazol-1-yl)piperidin-1-carboxylate

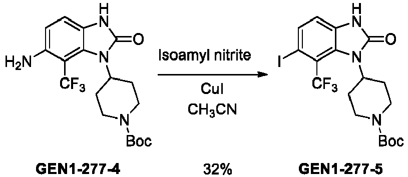

A suspension of isoamyl nitrite (241 mg, 2.06 mmol) and CuI (234 mg, 1.23 mmol) in CH₃CN (10 mL) was stirred at 65°C under N₂ atmosphere for 45 minutes. A solution of tert-butyl 4-(6-amino-2-oxo-7-(trifluoromethyl)-2,3-dihydro-1H-benzo[d]imidazol-1-yl)piperidin-1-carboxylate (410 mg, 1.02mmol) in CH₃CN (3mL) and DCM (2mL) was added. The resulting mixture was stirred at 65°C for 1 hour. The reaction mixture was concentrated, and the residue was purified by column chromatography on silica gel (petroleum ether: EtOAc = 3:1 to 1:1) to obtain the desired compound (180 mg, yield 32%) as a brown solid. MS (ESI): Rt = 1.72 min, *m*/*z* 456.0 [M+H-56]⁺; Purity: 92% @ 254 nm, 71% @ 214 nm.

### Step 6. Synthesis of tert-butyl 4-(2-oxo-6-phenyl-7-(trifluoromethyl)-2,3-dihydro-1H-benzo[d]imidazol-1-yl)piperidin-1-carboxylate

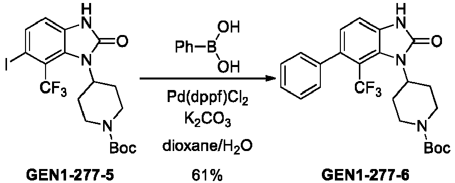

A mixture of tert-butyl 4-(6-iodo-2-oxo-7-(trifluoromethyl)-2,3-dihydro-1H-benzo[d]imidazol-1-yl)piperidin-1-carboxylate (110 mg, 0.215 mmol), phenylboronic acid (39 mg, 0.320 mmol), Pd(dppf)C12 (36 mg, 0.044 mmol) and Na₂CO₃ (69 mg, 0.65 mmol) in dioxane (4 mL) and water (1 mL) was stirred at 75°C under N₂ atmosphere for 3 hours .The reaction mixture was poured into water (40 mL), and extracted with EtOAc (20 mL×3). The combined organic layer was washed with brine (20 mL), dried over MgSO₄, filtered and it was concentrated. The crude product was purified by column chromatography on silica gel (petroleum ether: EtOAc = 3:1 to 1:1) to obtain the desired compound (78 mg, yield 61%) as a white solid. MS (ESI): Rt = 1.83 min, *m*/*z* 460.5 [M+H-56]⁺; Purity: 100% @ 254 nm, 78% @ 214 nm.

### Step 7. Synthesis of 6-phenyl-1-(piperidin-4-yl)-7-(trifluoromethyl)-1H-benzo[d]imidazol-2(3H)-one 2,2,2-trifluoroacetate

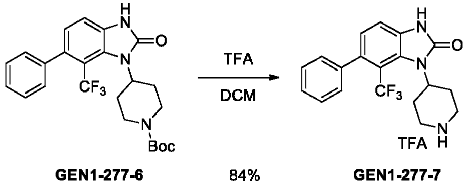

TFA (0.6 mL) was added to a solution of tert-butyl 4-(2-oxo-6-phenyl-7-(trifluoromethyl)-2,3-dihydro-1H-benzo[d]imidazol-1-yl)piperidin-1-carboxylate (78 mg 0.13 mmol) in DCM (3 mL). The reaction mixture was stirred at room temperature for 2 hours, and then concentrated to obtain the desired compound (62 mg, yield 84%) as a gray solid. MS (ESI): Rt = 1.43 min, *m*/*z* 362.3 [M+H]⁺; Purity: 100% @ 254 nm, 82% @ 214 nm.

### Step 8. Synthesis of 6-phenyl-7-(trifluoromethyl)-1-(1-(2-(4-(trifluoromethyl)phenyl)acetyl)piperidin-4-yl)-1H-benzo[d]imidazol-2(3H)-one (GEN1-277)

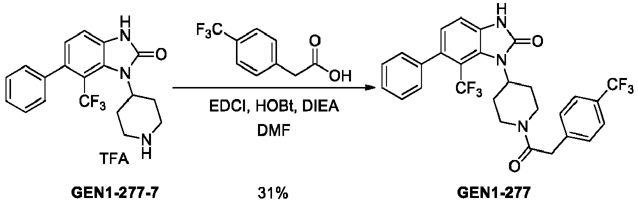

In an ice bath, EDCI (31 mg, 0.16 mmol) was added to a mixture of 6-phenyl-1-(piperidin-4-yl)-7-(trifluoromethyl)-1H-benzo[d]imidazol-2(3H)-one,2,2,2-trifluoroacetate (62 mg, 0.11 mmol), 2-(4-(trifluoromethyl)phenyl)acetic acid (26 mg, 0.13 mmol), HOBt (22 mg, 0.16 mmol) and DIEA (41 mg, 0.32 mmol) in DMF (3 mL). The reaction mixture was stirred at room temperature overnight. The reaction mixture was filtered and the filtrate was purified through a C18 column (CH₃CN: water = 5% to 60%) to obtain the desired compound (18.3 mg, yield 31%) as a white solid. ¹HNMR (400 MHz, DMSO-*d*₆): δ 11.47 (s, 1H), 7.68 (d, *J* = 8.0 Hz, 2H), 7.48 (d, *J* = 8.0 Hz, 2H), 7.45-7.36 (m, 3H), 7.34-7.32 (m, 2H), 7.25 (d, *J* = 8.0 Hz, 1H), 7.00 (d, *J* = 8.0 Hz, 1H), 4.61-4.58 (m, 1H), 4.25-4.12 (m, 2H), 3.95-3.86 (m, 2H), 3.07-3.01 (m, 1H), 2.60-2.52 (m, 3H), 1.75-1.73 (m, 2H). MS (ESI): Rt = 3.616 min, *m*/*z* 548.2 [M+H]⁺; Purity: 98.94% @ 254 nm, 98.69% @ 214 nm.

### Example 147: 1-(1-(2-(4-chloro-3-methoxyphenyl)acetyl)piperidin-4-yl)-7-(trifluoromethyl)-1,3-dihydro-2H-benzo[d]imidazol-2-one (GEN1-278)

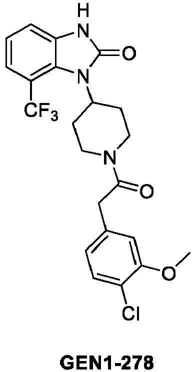

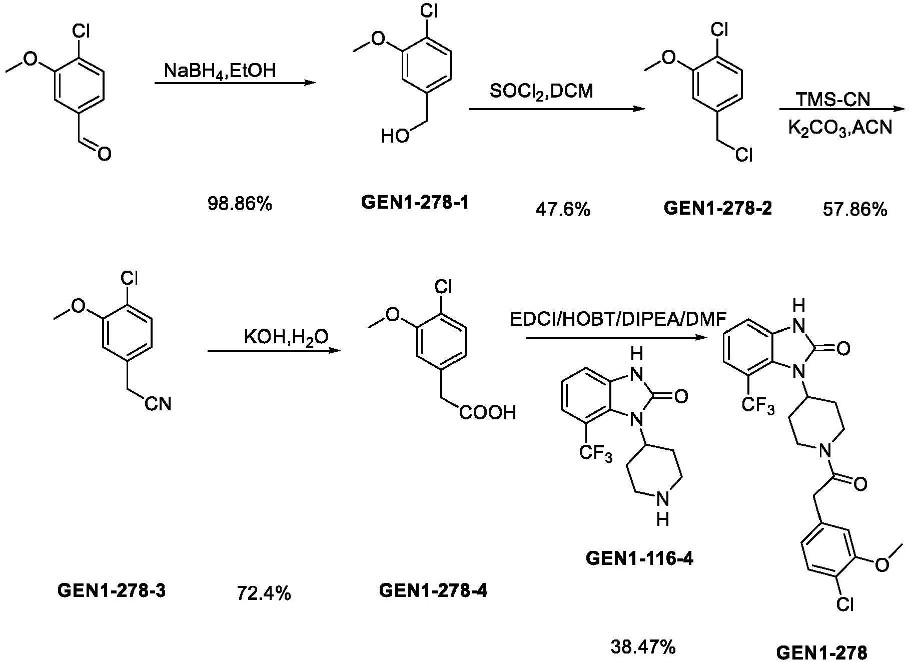

### Step 1. Synthesis of (4-chloro-3-methoxyphenyl)methanol (GEN1-278-1)

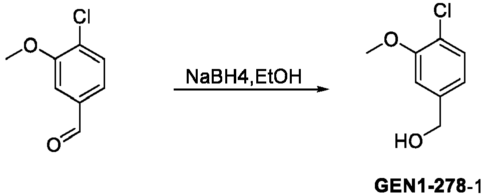

At 0°C, NaBH₄ (532 mg, 14.06 mmol) was added in portions to a solution of 4-chloro-3-methoxybenzaldehyde (2 g, 11.72 mmol) in EtOH (50 ml). The resulting mixture was then stirred at room temperature for 3 hours. The reaction mixture was diluted with water (500 mL), and extracted with EtOAc (100 mL×3). The combined organic phase was washed with brine (150 mL), dried over anhydrous Na₂SO₄ and filtered. The filtrate was concentrated, and the resulting crude product was purified by silica gel column chromatography (PE:EA = 1:1) to obtain the desired compound (2g, yield 98.86%) as a white solid. MS (ESI): Rt = 1.30 min; Purity: 100% @ 254 nm, 100% @ 214 nm.

### Step 2. Synthesis of 1-chloro-4-(chloromethyl)-2-methoxybenzene (GEN1-278-2)

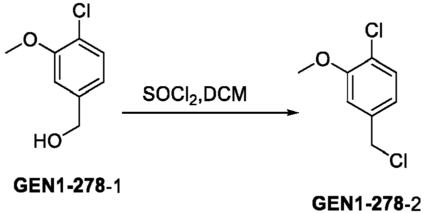

SOCl₂ (6.2 g, 52.15 mmol) was added to a solution of (4-chloro-3-methoxyphenyl)methanol (1.8 g, 10.43 mmol) in DCM (50 ml) at room temperature. The resulting mixture was then stirred at 40°C for 3 hours. After no TLC raw material remained, the reaction solution was concentrated, and the obtained crude product was purified by silica gel column chromatography (PE:EA = 3:1) to obtain the desired compound (950 mg, yield 47.6%) as a yellow oil. 1H NMR (400 MHz, DMSO-d6) δ 7.42 (d, J = 8.1 Hz, 1H), 7.24 (d, J = 1.9 Hz, 1H), 7.03 (dd, J = 8.1, 1.9 Hz, 1H), 4.75 (s, 2H), 3.86 (s, 3H).

### Step 3. Synthesis of 2-(4-chloro-3-methoxyphenyl)acetonitrile (GEN1-278-3)

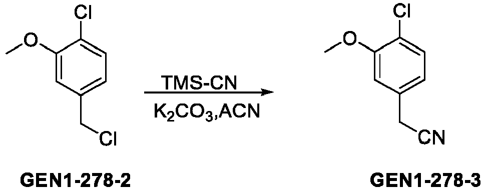

TMS-CN (753 mg, 7.59 mmol) was added to a suspension of 1-chloro-4-(chloromethyl)-2-methoxybenzene(1 g, 5.234 mmol), K₂CO₃ (832 mg, 6.02 mmol) in ACN (30 mL) at room temperature. The resulting mixture was stirred at 60°C for 6 h. Then it was stirred at room temperature for 16 h. TLC showed that about 50% of the starting material remained. Then TMS-CN (374mg, 3.77mmol) was added to the reaction solution. The resulting mixture was stirred for another 6 hours at 60°C. The mixture was poured into 150 mL of water, and extracted with EtOAc (50 mL×3). The combined organic layer was washed with brine (60 mL), dried over anhydrous Na₂SO₄ and it was concentrated. The residue was purified by silica gel column chromatography (PE:EA from 10:1 to 4:1) to obtain the desired compound (550 mg, yield 57.86%). ¹HNMR (400 MHz, DMSO-*d*₆) δ 7.45 (d, *J* = 8.1 Hz, 1H), 7.14 (d, *J* = 2.0 Hz, 1H), 6.95 (dd, *J* = 8.1, 2.0 Hz, 1H), 4.04 (s, 2H), 3.86 (s, 3H).

### Step 4. Synthesis of 2-(4-chloro-3-methoxyphenyl)acetic acid (GEN1-278-4)

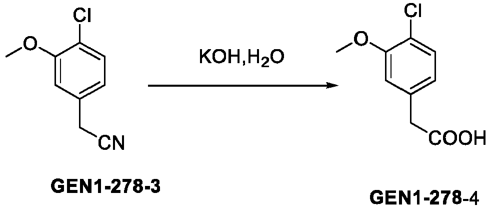

NaOH (82.6 mg, 2.065 mmol) was added to a mixed solution of 2-(4-chloro-3-methoxyphenyl)acetonitrile (250 mg, 1.377 mmol) in THF (3.5 mL) and H₂O (3.5 ml). The resulting mixture was stirred at 80°C for 16 hours. The mixture was then cooled to room temperature. The mixture was poured into water (150 mL), and extracted with EtOAc (50 mL×3). The pH of the aqueous layer was adjusted to 4 with IN HCl, and it was extracted with EtOAc (50 mL×3). The combined organic layer was washed with brine (60 mL), dried over anhydrous Na₂SO₄ and it was concentrated to obtain the desired compound (200 mg, yield 72.4%) as a white solid. MS (ESI): Rt = 1.30 min, m/z 199.0 [M-H]⁻, Purity: 100% @ 254 nm, 100% @ 214 nm.

### Step 5. Synthesis of 1-(1-(2-(4-chloro-3-methoxyphenyl)acetyl)piperidin-4-yl)-7-(trifluoromethyl)-1,3-dihydro-2H-benzo[d]imidazol-2-one (GEN1-278)

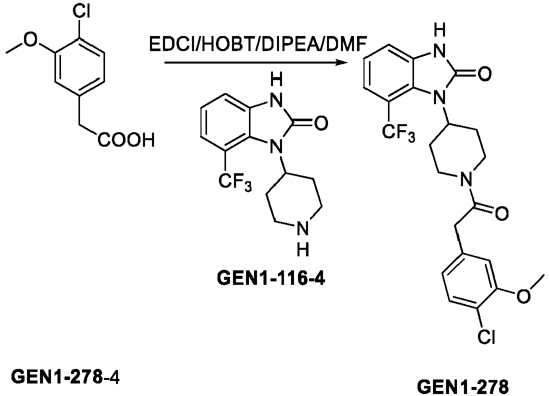

DIPEA (96.93 mg, 0.75 mmol) was added to a solution of 1-(piperidin-4-yl)-7-(trifluoromethyl)-1,3-dihydro-2H-benzo[d]imidazol-2-one (71.25 mg, 0.25 mmol, the synthesis steps could be found in GEN1-116-4), 2-(4-chloro-3-methoxyphenyl)acetic acid (50.16 mg, 0.25 mmol), HOBT (50.67 mg, 0.375 mmol) and EDCI (71.89 mg, 0.375 mmol) in DMF (1 mL) at room temperature. The resulting mixture was stirred at room temperature for 1 hour. The reaction mixture was diluted with water (30 mL), and extracted with EtOAc (30 mL×3). The combined organic phase was washed with brine (30 mL), dried over anhydrous Na₂SO₄ and filtered. The filtrate was concentrated to obtain the crude product. The crude product was purified by reverse phase chromatography. Purification conditions: C18 silica gel column; mobile phase ACN: H₂O, gradient: 5% to 50%; 45 minutes, monitoring wavelength: 214 nm. The desired compound (45 mg, yield 38.47%) was obtained as a white solid. 1H NMR (400 MHz, DMSO-d6) δ 11.49 (s, 1H), 7.41 -7.30 (m, 2H), 7.29-7.24 (m, 1H), 7.20-7.11(m, 1H), 7.02 (s, 1H), 6.83 (d, J = 8.1Hz, 1H), 4.59 (d, J = 11.9 Hz, 1H), 4.31-4.18 (m, 1H), 4.13 (d, J = 13.8 Hz, 1H), 3.94-3.82 (m, 3H), 3.77 (s, 2H), 3.00 (t, J = 13.3 Hz, 1H), 2.65 - 2.36 (m, 7H), 1.69 (t, J = 10.9 Hz, 2H).MS (ESI): Rt = 1.58 min, *m*/*z* 468.1 [M+H]⁺; Purity: 100% @ 254 nm, 100% @ 214 nm.100%.

### Example 148: N-(2-((1-(2-(pyridin-4-yl)acetyl)piperidin-4-yl)amino)phenyl)acetamide (GEN1-213)

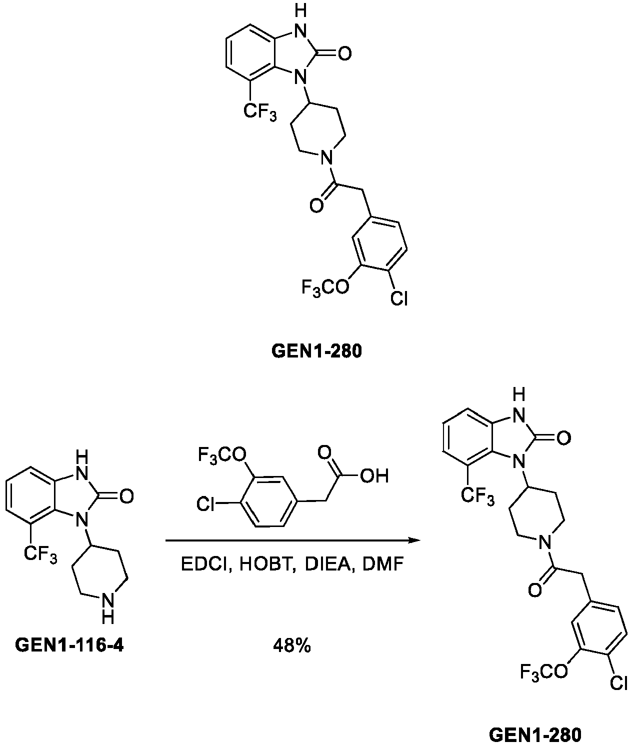

### Step 1. Synthesis of 1-(1-(2-(4-chloro-3-(trifluoromethoxy)phenyl)acetyl)piperidin-4-yl)-7-(trifluoromethyl)-1,3-dihydro-2H-benzo[d]imidazol-2-one (GEN1-280)

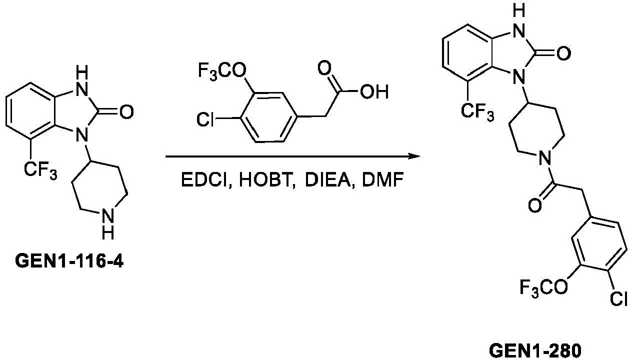

DIEA (90 mg, 0.70 mmol) was added to a solution of 1-(piperidin-4-yl)-7-(trifluoromethyl)-1,3-dihydro-2H-benzo[d]imidazol-2-one (100 mg, 0.34 mmol), 2-(4-chloro-3-(trifluoromethoxy)phenyl)acetic acid (88 mg, 0.34 mmol), EDCI (100 mg, 0.52 mmol), HOBT (72 mg, 0.52 mmol) in DMF (1.0 mL). The resulting mixture was stirred at room temperature for 8 hours. The reaction mixture was poured into water (20 mL), and extracted with EtOAc (25 mL×3). The combined organic phase was washed with water (70 mL), brine (70 mL), and then dried over Na₂SO₄ and it was concentrated to obtain a yellow oil. The crude product was purified by reverse phase chromatography. Purification conditions: C18 silica gel column; mobile phase ACN: H₂O, gradient: 10% to 75%; 50 minutes, monitoring wavelength: 214 nm. The desired compound (84 mg, 48% yield) was obtained as a white solid. ¹H NMR (400 MHz, DMSO-d₆): δ 11.49 (s, 1H), 7.62 (d, *J* = 8.3 Hz, 1H), 7.49-7.45 (m, 1H), 7.36 (d, *J* = 8.2 Hz, 1H), 7.34-7.30 (m, 1H), 7.28 (d, *J* = 7.8 Hz, 1H), 7.16 (t, *J* = 7.9 Hz, 1H), 4.66-4.50 (m, 1H), 4.32-4.09 (m, 2H), 3.88 (s, 2H), 3.03 (t, *J* = 13.2 Hz, 1H), 2.64-2.52 (m, 2H), 2.48-2.37 (m, 1H), 1.78-1.63 (m, 2H). MS (ESI): Rt = 1.72 min, *m*/*z* 522.0 [M + H]⁺, Purity: 100% @ 254 nm, 100% @ 214 nm.

### Example 149: 7-chloro-1-(1-(1-(2-(4-(trifluoromethyl)phenyl)acetyl)piperidin-4-yl)-1,3-dihydro-2H-benzo[d]imidazol-2-one (GEN1-281)

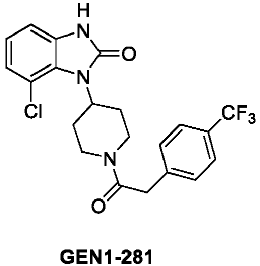

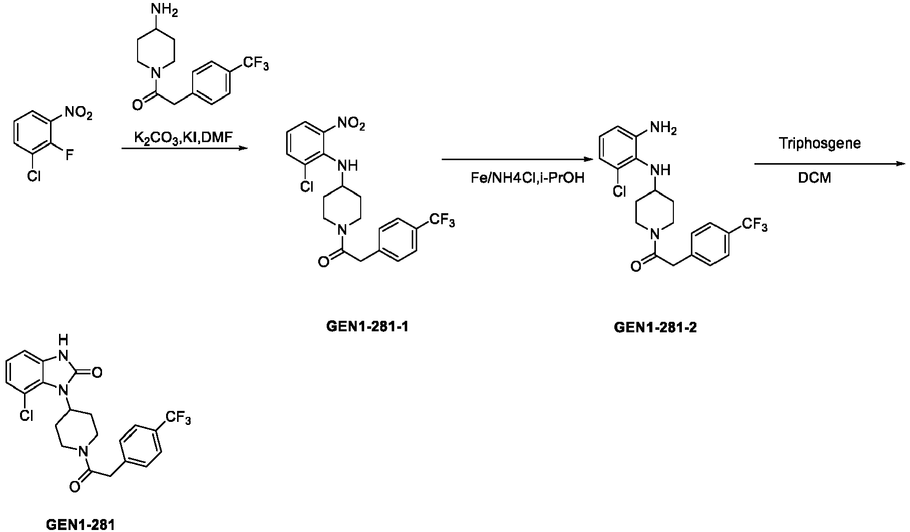

### Step 1. Synthesis of 1-(4-((2-chloro-6-nitrophenyl)amino)piperidin-1-yl)-2-(4-(trifluoromethyl)phenyl)ethan-1-one (GEN1-281-1)

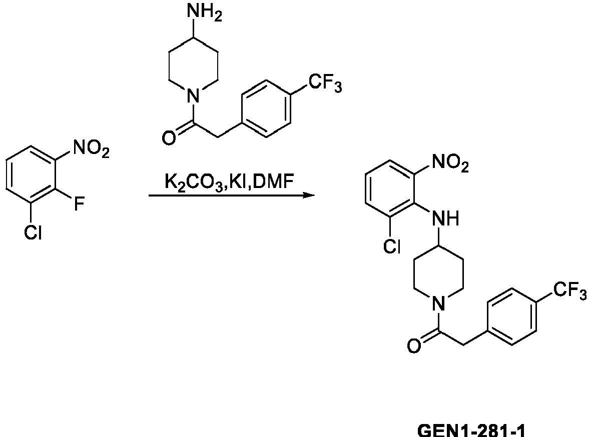

1-(4-aminopiperidin-1-yl)-2-(4-(trifluoromethyl)phenyl)ethan-1-one (171.8 mg, 0.6 mmol, the synthesis steps could be found in GEN1-219-4) was added to a suspension of 1-chloro-2-fluoro-3-nitrobenzene (106 mg, 0.6 mmol), K₂CO₃ (165.9 mg, 1.2 mmol) and KI (10 mg, 0.06 mmol) in DMF (5 mL). The resulting mixture was stirred at 80°C for 4 hours. The reaction mixture was diluted with water (80 mL), and extracted with EtOAc (30 mL×3). The combined organic phase was washed with brine (50 mL), dried over anhydrous Na₂SO₄ and filtered. The filtrate was concentrated to obtain the crude product. The crude product was purified by silica gel column chromatography (PE:EA=3:1) to obtain the desired compound (210mg, yield 79.2%) as a yellow oil. MS (ESI): Rt = 1.72 min, *m*/*z* 442.3 [M+H]⁺; Purity: 79.95% @ 254 nm, 75.68% @ 214 nm.

### Step 2. Synthesis of 1-(4-((2-amino-6-chlorophenyl)amino)piperidin-1-yl)-2-(4-(trifluoromethyl)phenyl)ethan-1-one (GEN1-281-2)

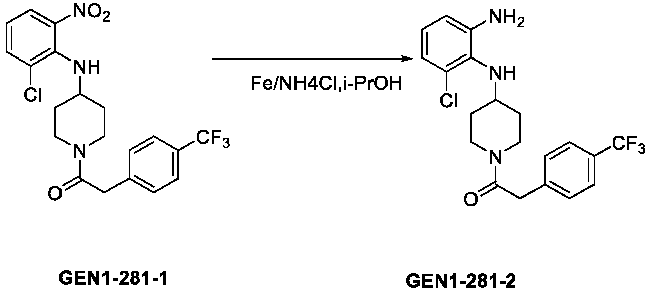

Fe (266 mg, 4.75 mmol) was added to a solution of 1-(4-((2-chloro-6-nitrophenyl)amino)piperidin-1-yl)-2-(4-(trifluoromethyl)phenyl)ethan-1-one (210 mg, 0.475 mmol), NH₄Cl (508 mg, 9.5 mmol) in i-PrOH (20 mL). The resulting mixture was stirred at room temperature for 3h. The mixture was filtered and the filtrate was concentrated and the residue was purified by column chromatography on silica gel (PE:EA = 1:1 to 100% EA) to obtain the desired compound (150 mg, yield 76.7%) as a pale yellow solid. MS (ESI): Rt = 1.64 min, *m*/*z* 412.1 [M+H]⁺; Purity: 73.71% @ 254 nm, 68.73% @ 214 nm.

### Step 3. Synthesis of 7-chloro-1-(1-(1-(2-(4-(trifluoromethyl)phenyl)acetyl)piperidin-4-yl)-1,3-dihydro-2H-benzo[d]imidazol-2-one (GEN1-281)

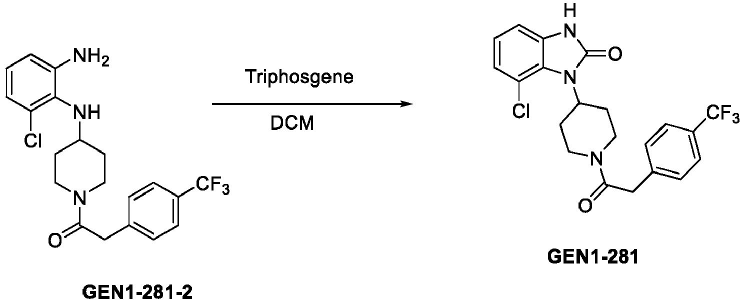

Triphosgene (43.2 mg, 0.146 mmol) was added to a suspension of 1-(4-((2-amino-6-chlorophenyl)amino)piperidin-1-yl)-2-(4-(trifluoromethyl)phenyl)ethan-1-one (150 mg, 0.364 mmol), NaHCO₃ (153 mg, 1.82 mmol) in DCM (10 mL) at room temperature. The resulting mixture was stirred at room temperature for 1 hour. The mixture was poured into water (20 mL), and extracted with DCM (20 mL×3). The combined organic layer was washed with brine (30 mL), dried over Na₂SO₄ and it was concentrated to obtain the crude product. The crude product was purified by reverse phase chromatography. Purification conditions: C18 silica gel column; mobile phase ACN: H₂O, gradient: 5%-50%; 45 minutes, monitoring wavelength: 214 nm. The desired compound (90 mg, yield 56.5%) was obtained as a white solid. ¹H NMR (400 MHz, DMSO-d6) δ 11.7-10.6 (m, 1H), 7.67 (d, J = 8.1 Hz, 2H), 7.48 (d, J = 7.9 Hz, 2H), 7.04 - 6.90 (m, 3H), 5.10 (s, 1H), 4.63 - 4.48 (m, 1H), 4.20-4.07 (m, 1H), 4.00-3.78 (m, 2H), 3.11 (t, J = 13.3 Hz, 1H), 2.64 (t, J = 12.9 Hz, 1H), 2.49-2.26 (m, 2H), 1.89-1.65 (m, 2H). MS (ESI): Rt = 1.61 min, *m*/*z* 235.1 [M+H]⁺; Purity: 100% @ 254 nm, 100% @ 214 nm.

### Example 150: 7-methyl-1-(1-(1-(2-(4-(trifluoromethyl)phenyl)acetyl)piperidin-4-yl)-1,3-dihydro-2H-benzo[d]imidazol-2-one (GEN1-282)

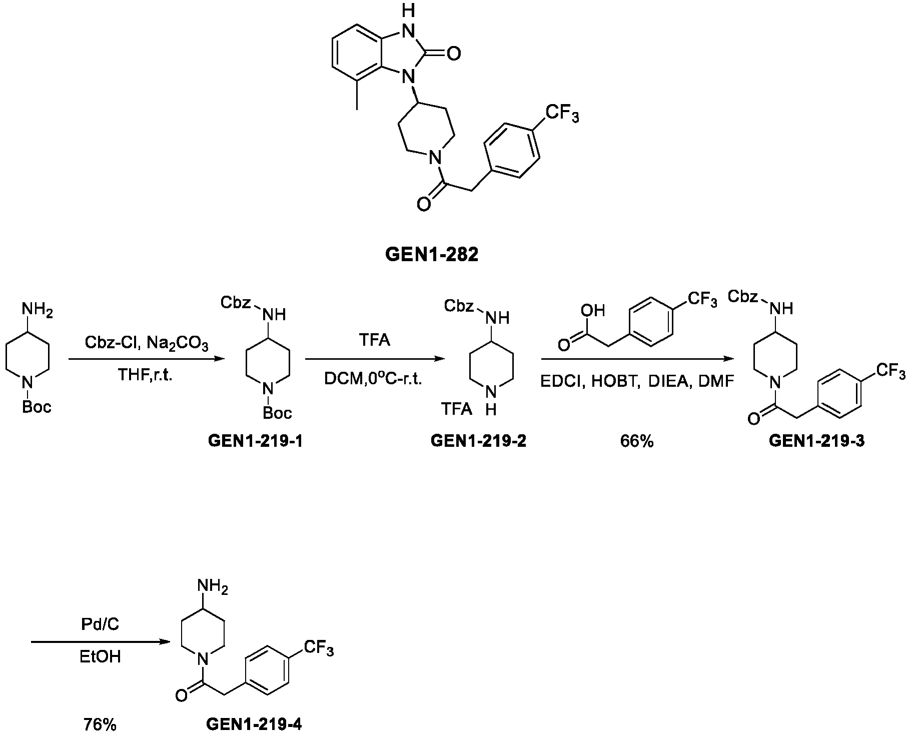

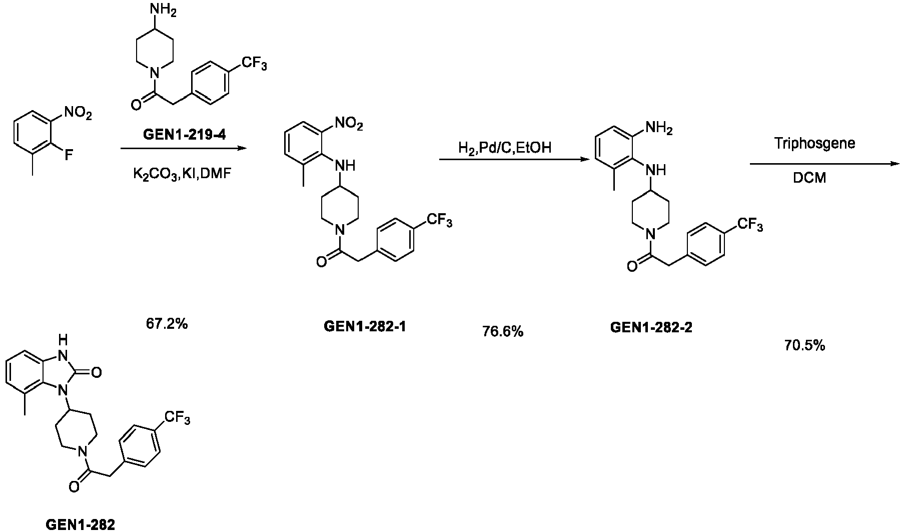

### Step 1. Synthesis of tert-butyl 4-((((benzyloxy)carbonyl)amino)piperidin-1-carboxylate (GEN1-219-1)

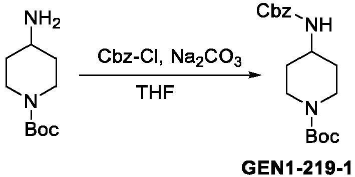

Under the protection of nitrogen, the temperature was controlled at 0°C, CbzCl (3.2 g, 18.7 mmol) was added to a suspension of tert-butyl 4-aminopiperidine-1-carboxylate (3.0 g, 15.0 mmol) and Na₂CO₃ (3.18 g, 30.0 mmol) in THF (90 mL). The resulting mixture was stirred at room temperature for 16 hours under nitrogen protection. The reaction mixture was poured into ice water (150 mL), and extracted with EtOAc (200 mL×3). The combined organic layer was washed with brine (500 mL), then dried over Na₂SO₄ and it was concentrated to obtain the crude product (6.71 g) as a pale yellow oil. It was used directly in the next step without purification. TLC: EA/(PE+EA)=20%, Rf=0.5.

### Step 2. Synthesis of benzylpiperidin-4-yl carbamate trifluoroacetate (GEN1-219-2)

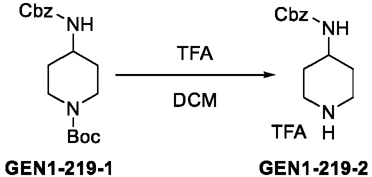

Under the protection of nitrogen, the temperature was controlled at 0°C, TFA (11 mL) was added dropwise to a solution of tert-butyl 4-((((benzyloxy)carbonyl)amino)piperidin-1-carboxylate (6.71g crude product) in DCM (100mL). The resulting mixture was stirred at room temperature for 4 hours under the protection of nitrogen. The resulting mixture was concentrated to obtain a crude product (8.5 g). The crude product was used directly in the next step without purification. MS (ESI): Rt = 0.31 min, m/z 235.1 [M + H]+ .

### Step 3. Synthesis of benzyl (1-(2-(4-(tris(trifluoromethyl)phenyl)acetyl)piperidin-4-yl)carbamate (GEN1-219-3)

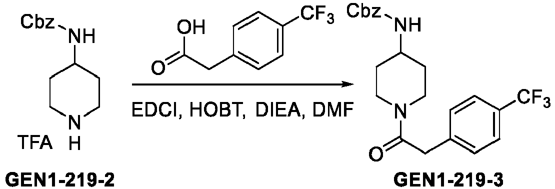

DIEA (10.45 g, 81 mmol) was added to a solution of benzylpiperidin-4-ylcarbamate benzyl trifluoroacetate (7.7 g crude product), 2-(4-(trifluoromethyl)phenyl)acetic acid (2.75 g, 13.48 mmol), EDCI (3.9 mg, 20.31 mmol) and HOBT (2.74 g, 20.30 mmol) in DMF (50 mL). The resulting mixture was stirred at room temperature for 8 hours. The reaction mixture was poured into water (250 mL), and extracted with EtOAc (250 mL×3). The organic phase was washed with water (600 mL), brine (600 mL), dried over Na₂SO₄ and it was concentrated to obtain a yellow oil. The crude product was purified by silica gel column chromatography (PE: EtOAc = 3:7) to obtain the desired compound (4.16 g, yield 66%) as a white solid. MS (ESI): Rt = 1.50 min, m/z 421.2 [M + H]⁺, Purity: 100% @ 254 nm, 75% @ 214 nm.

### Step 4. Synthesis of 1-(4-aminopiperidin-1-yl)-2-(4-(trifluoromethyl)phenyl)ethan-1-one (GEN1-219-4)

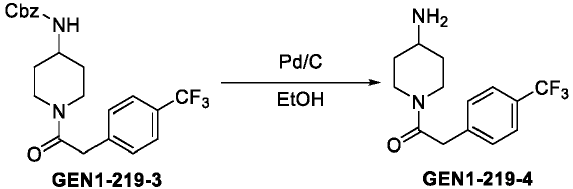

Pd/C (400 mg) was added to a solution of benzyl(1-(2-(4-(tris(trifluoromethyl)phenyl)acetyl)piperidin-4-yl)carbamate (2.0 g, 4.76 mmol) in MeOH (140 mL) under nitrogen protection. The resulting mixture was replaced with H₂ and stirred at room temperature under H₂ atmosphere overnight. The reaction mixture was filtered, and the filter cake was washed with MeOH (100 mL×3). The filtrate was concentrated to dryness, and the resulting crude product was purified by silica gel column chromatography (MeOH:DCM = 2:23) to obtain the desired compound (1.04 g, yield 76%) as a pale yellow solid. MS (ESI): Rt = 1.14 min, m/z 287.1 [M + H]+, Purity: 100% @ 254 nm, 100% @ 214 nm.

### Step 5. Synthesis of 1-(4-((2-methyl-6-nitrophenyl)amino)piperidin-1-yl)-2-(4-(trifluoromethyl)phenyl)ethan-1-one (GEN1-282-1)

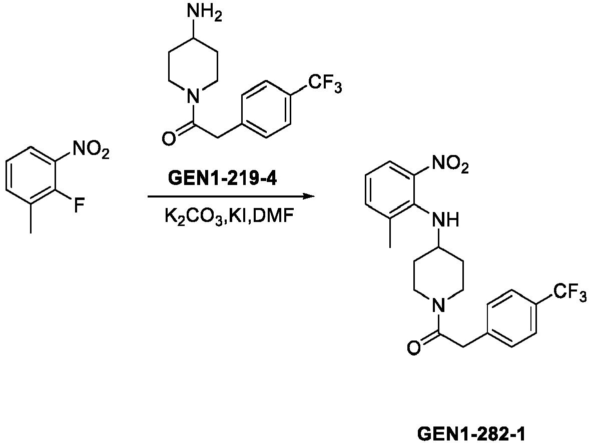

1-(4-aminopiperidin-1-yl)-2-(4-(trifluoromethyl)phenyl)ethan-1-one (171.8 mg, 0.6 mmol, the synthesis steps could be found in GEN 1-219-4) was added to a suspension of 2-fluoro-1-methyl-3-nitrobenzene (93 mg, 0.6 mmol), K₂CO₃ (165.9 mg, 1.2 mmol) and KI (10 mg, 0.06 mmol) in DMF (5 mL). The resulting mixture was stirred at 80°C for 4 hours. The reaction mixture was diluted with water (80 mL), and extracted with EtOAc (30 mL×3). The combined organic phase was washed with brine (50 mL), dried over anhydrous Na₂SO₄ and filtered. The filtrate was concentrated and purified by silica gel column chromatography (PE:EA=3:1) to obtain the desired compound (170 mg, yield 67.2%) as a yellow oil. MS (ESI): Rt = 1.71 min, *m*/*z* 422.3 [M+H]⁺; Purity: 71.06% @ 254 nm, 72.49% @ 214 nm.

### Step 6. Synthesis of 1-(4-((2-amino-6-methylphenyl)amino)piperidin-1-yl)-2-(4-(trifluoromethyl)phenyl)ethan-1-one (GEN1-282-2)

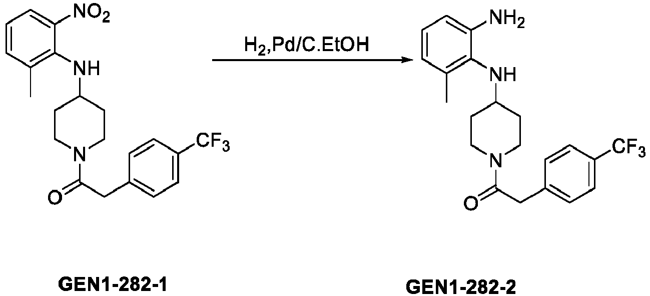

Pd/C (20 mg, 5% purity) was added to a solution of 1-(4-((2-methyl-6-nitrophenyl)amino)piperidin-1-yl)-2-(4-(trifluoromethyl)phenyl)ethan-1-one (170 mg, 0.40 mmol) in EtOH (20 mL). The resulting mixture was replaced with hydrogen and was stirred under a hydrogen atmosphere overnight. The mixture was filtered, and the filtrate was concentrated. The residue was purified by silica gel column chromatography (PE:EA = 1:1 to 100% EA) to obtain the desired compound (120 mg, yield 76.6%) as a pale yellow solid. MS (ESI): Rt = 1.59 min, *m*/*z* 392.2 [M+H]⁺; Purity: 86% @ 254 nm, 85.57% @ 214 nm.

### Step 7. Synthesis of 7-methyl-1-(1-(1-(2-(4-(trifluoromethyl)phenyl)acetyl)piperidin-4-yl)-1,3-dihydro-2H-benzo[d]imidazol-2-one (GEN1-282)

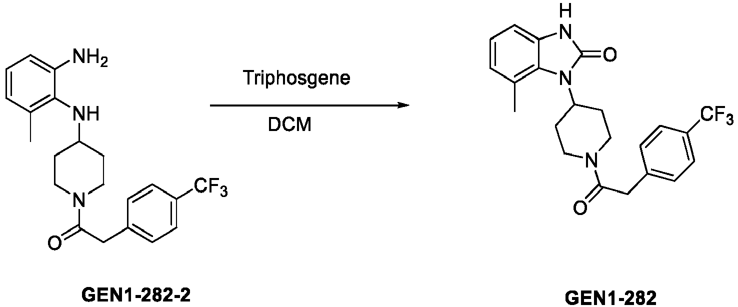

Triphosgene (36.3 mg, 0.123 mmol) was added to a suspension of 1-(4-((2-amino-6-methylphenyl)amino)piperidin-1-yl)-2-(4-(trifluoromethyl)phenyl)ethan-1-one (120 mg, 0.306 mmol), NaHCO₃ (129 mg, 1.53 mmol) in DCM (10 mL) at room temperature. The resulting mixture was stirred at room temperature for 1 hour. The mixture was poured into water (20 mL), and extracted with DCM (20 mL×3). The combined organic layer was washed with brine (30 mL), dried over Na₂SO₄ and it was concentrated. The crude residue was purified by reverse phase chromatography. Purification conditions: C18 silica gel column; mobile phase ACN: H₂O, gradient: 5% to 50%; 45 minutes, monitoring wavelength: 214 nm. The desired compound (90 mg, yield 70.5%) was obtained as a white solid. ¹HNMR (400 MHz, DMSO-d6) δ 10.80 (s, 1H), 7.68 (d, J = 8.1 Hz, 2H), 7.48 (d, J = 8.0 Hz, 2H), 6.92 - 6.71 (m, 3H), 4.61-4.47 (m, 2H), 4.16-4.04 (m, 1H), 3.99-3.81 (m, 2H), 3.19 - 3.08 (m, 1H), 2.72-2.60 (m, 1H), 2.55 (s, 3H), 2.49-2.28 (m, 2H), 1.84-1.72 (m, 2H). MS (ESI): Rt = 1.56 min, *m*/*z* 418.2 [M+H]⁺; Purity: 100% @ 254 nm, 100% @ 214 nm.

### Example 151: 7-(trifluoromethyl)-1-(1-(2-(4-(4-(trifluoromethyl)piperidin-1-yl)acetyl)piperidin-4-yl)-1,3-dihydro-2H-benzo[d]imidazol-2-one (GEN1-285)

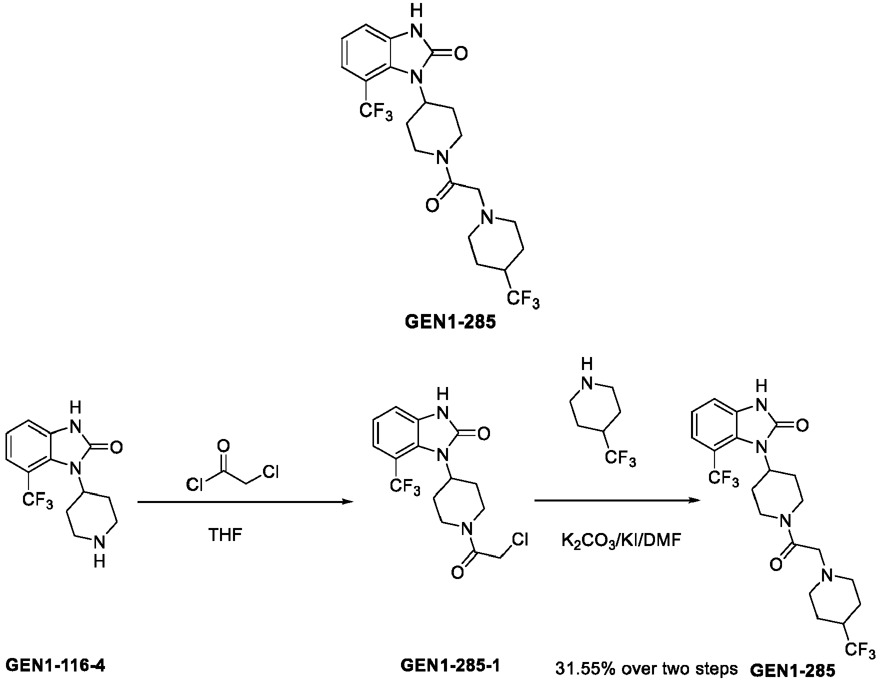

### Step 1. Synthesis of 1-(1-(2-chloroacetyl)piperidin-4-yl)-7-(trifluoromethyl)-1,3-dihydro-2H-benzo[d]imidazol-2-one (GEN1-285-1)

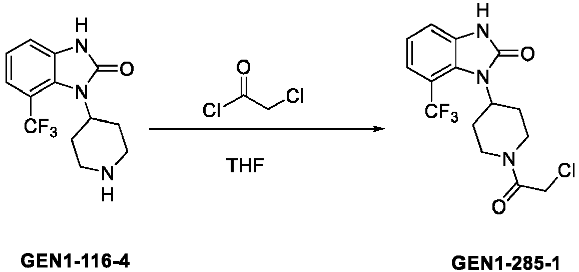

2-chloroacetyl chloride (135.53 mg, 1.2 mmol) was added to a solution of 1-(piperidin-4-yl)-7-(trifluoromethyl)-1,3-dihydro-2H-benzo[d]imidazol-2-one (285 mg, 1 mmol, the synthesis steps could be found in GEN1-116-4) in THF (20 mL) at room temperature. The resulting mixture was then stirred at room temperature for 16 hours. The mixture was poured into water (80 mL), and extracted with EtOAc (30 mL×3). The combined organic layer was washed with brine (50 mL), dried over anhydrous Na₂SO₄ and it was concentrated to obtain (350 mg) crude product. The crude product was used directly in the next step. MS (ESI): RT = 1.45 min, m/z 362.0 [M+H]+, Purity: 73.11% @ 254 nm, 56.70% @ 214 nm.

### Step 2. Synthesis of 7-(trifluoromethyl)-1-(1-(2-(4-(4-(trifluoromethyl)piperidin-1-yl)acetyl)piperidin-4-yl)-1,3-dihydro-2H-benzo[d]imidazol-2-one (GEN1-285)

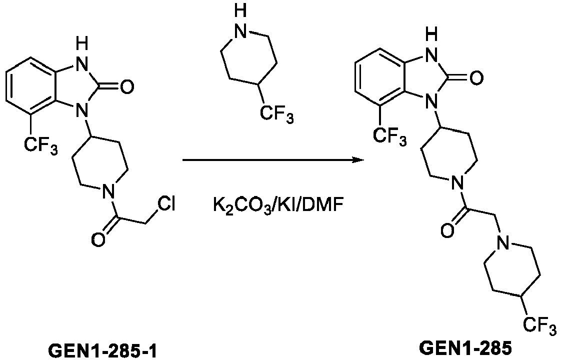

4-(trifluoromethyl)piperidine (58.44 mg, 0.382 mmol) was added to a suspension of 1-(1-(2-chloroacetyl)piperidin-4-yl)-7-(trifluoromethyl)-1,3-dihydro-2H-benzo[d]imidazol-2-one (115 mg, 0.318 mmol), K₂CO₃ (87.9 mg, 0.636 mmol), KI (5.3 mg, 0.032 mmol) in DMF (1 mL) at room temperature. The resulting mixture was then stirred at 90°C for 3 hours. The reaction mixture was diluted with water (30 mL), and extracted with EtOAc (30 mL×3). The combined organic phase was washed with brine (30 mL), dried over anhydrous Na₂SO₄ and filtered. The filtrate was concentrated to obtain the crude product, which was purified by reverse phase chromatography. Purification conditions: C18 silica gel column; mobile phase ACN: H₂O, gradient: 5% to 50%; 45 minutes, monitoring wavelength: 214 nm. The desired compound (48 mg, two steps yield: 31.55%) was obtained as a white solid. 1H NMR (400 MHz, DMSO-d6) δ 11.48 (s, 1H), 7.39 (d, J = 8.0 Hz, 1H), 7.30 (d, J = 7.7 Hz, 1H), 7.18 (t, J = 7.9 Hz, 1H), 4.56 (d, J = 11.9 Hz, 1H), 4.42 - 4.14 (m, 2H), 3.40 (s, 1H), 3.08 - 2.89 (m, 4H), 2.80-2.65 (m, 1H), 2.62-2.40 (m, 2H), 2.34 - 2.20 (m, 1H), 2.05 (q, J = 11.4 Hz, 2H), 1.87 - 1.66 (m, 4H), 1.66 - 1.37 (m, 2H). MS (ESI): Rt = 4.16 min, *m*/*z* 479.2 [M+H]⁺; Purity: 90.38% @ 254 nm, 98.87% @ 214 nm.

### Example 152: 1-(1-(2-(3-(ethoxyphenyl)acetyl)piperidin-4-yl)-1,3-dihydro-2H-benzo[d]imidazol-2-one (GEN1-287)

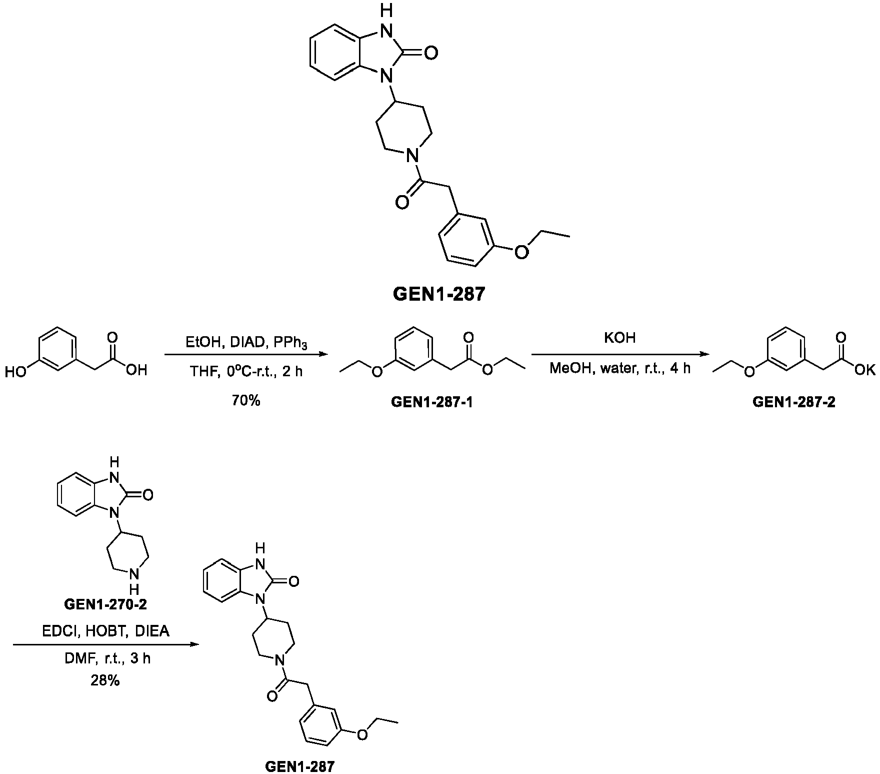

### Step 1. 2-(3-ethoxyphenyl) ethyl acetate (GEN1-287-1)

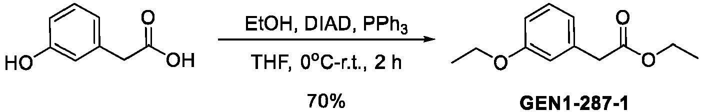

DIAD (1.99 g , 9.85 mmol) was added dropwise to a solution of 2-(3-hydroxyphenyl)acetic acid (500 mg, 3.29 mmol), EtOH (378 mg, 6.75 mmol), PPh₃ (2.58 g, 9.85 mmol) in THF (10 mL) at 0°C. The resulting mixture was stirred at room temperature under N₂ atmosphere for 2 hours. The reaction mixture was poured into water (30 mL), and extracted with EA (20 mL×3). The organic layer was washed with water (50 mL), brine (50 mL), dried over Na₂SO₄ and it was concentrated to obtain a pale yellow solid. The crude product was purified by silica gel column chromatography, eluted with (EA / (PE + EA) = 20%), to obtain the desired compound (480 mg, yield 70%) as a pale yellow oil. MS (ESI) Rt=1.61 min, m/z 209.2 [M+H]⁺, Purity: 100% @ 254 nm, 38% @ 214 nm

### Step 2. Potassium 2-(3-ethoxyphenyl) acetate (GEN1-287-2)

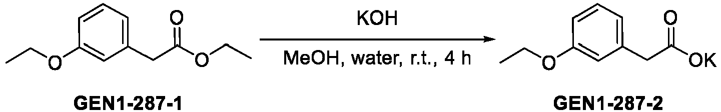

KOH (40 mg, 0.72 mmol) and water (0.3 mL) were added to a solution of 2-(3-ethoxyphenyl) ethyl acetate (150 mg, 0.72 mmol) in MeOH (3.0 mL). The resulting mixture was stirred at room temperature under N₂ atmosphere for 4 hours. The resulting mixture was concentrated under vacuum. The desired compound (104 mg crude product) was obtained and used directly in the next step without further purification. TLC: MeOH/(MeOH+DCM)=10%, Rf=0.2

### Step 3. 1-(1-(2-(3-(ethoxyphenyl)acetyl)piperidin-4-yl)-1,3-dihydro-2H-benzo[d]imidazol-2-one (GEN1-287)

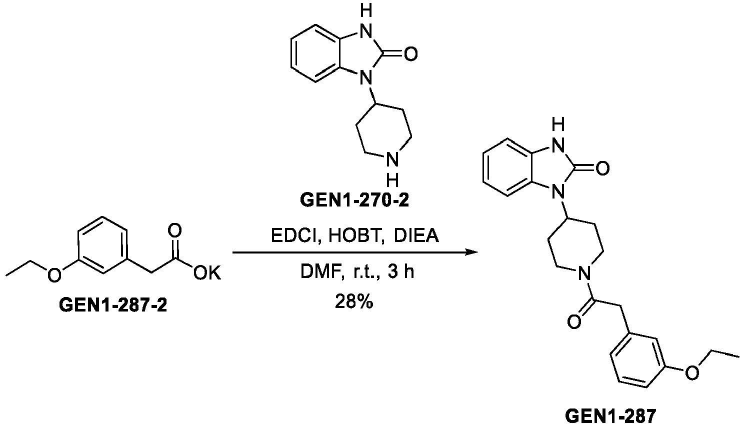

DIEA (298 mg, 2.31 mmol) was added to a solution of 1-(piperidin-4-yl)-1,3-dihydro-2H-benzo[d]imidazol-2-one (104 mg crude), potassium 2-(3-ethoxyphenyl)acetate (230 mg crude product), EDCI (166mg, 0.86mmol), HOBT (117mg, 0.87mmol) in DMF (3.0mL). The resulting mixture was stirred at room temperature for 3 hours. The reaction mixture was poured into water (15 mL), and extracted with EA (20 mL×3). The combined organic layer was washed with water (50 mL), brine (50 mL), dried over Na₂SO₄ and it was concentrated to obtain a yellow oil. The crude product was purified by reverse-phase flash chromatography (C₁₈ reverse silica column; mobile phase CH₃CN, water, 10% to 75% gradient, 50 minutes; detection, UV 214 nm) to obtain the desired compound (70 mg, yield 28%) as a white solid. ¹HNMR (400 MHz, DMSO-*d*₆) δ: 10.83 (s, 1H), 7.28-7.21 (m, 1H), 7.01-6.91 (m, 4H), 6.90-6.85 (m, 2H), 6.82-6.76 (m, 1H), 4.57 (d, *J*=13.1 Hz, 1H), 4.47-4.30 (m, 1H), 4.14-4.05 (m, 1H), 4.05-3.96 (m, 2H), 3.83-3.65 (m, 2H), 3.14 (t, *J*=13.0 Hz, 1H), 2.67 (t, *J*=12.7 Hz, 1H), 2.10-1.82 (m, 2H), 1.71-1.52 (m, 2H), 1.38-1.29 (m, 3H). MS (ESI) Rt=1.41 min, m/z 380.3 [M+H]⁺, Purity: 100% @ 254 nm, 100% @ 214 nm.

### Example 153: 7-fluoro-1-(1-(2-(2-(4-(trifluoromethyl)phenyl)acetyl)piperidin-4-yl)-1,3-dihydro-2H-benzo[d]imidazol-2-one (GEN1-289)

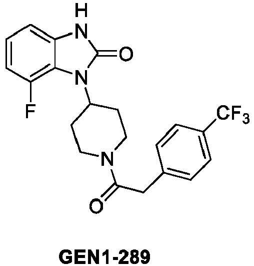

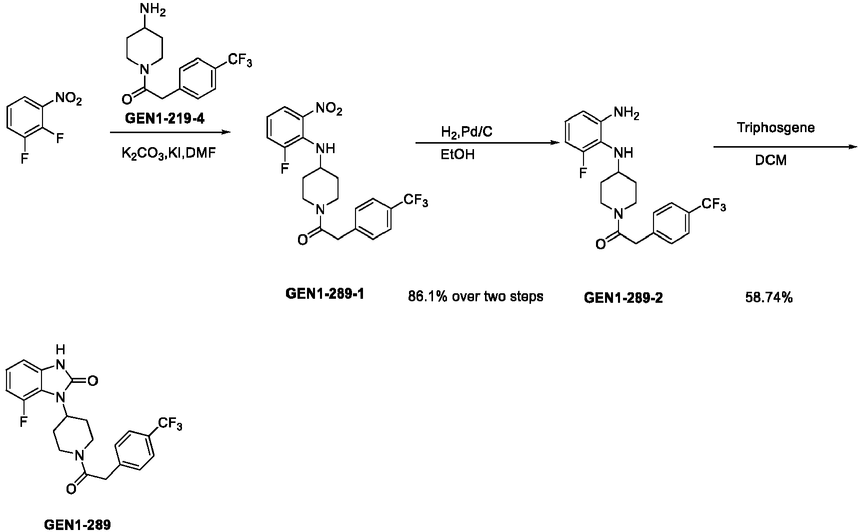

### Stpe 1. Synthesis of tert-butyl 4-((2-nitrophenyl)amino)piperidin-1-carboxylate (GEN1-122-1)

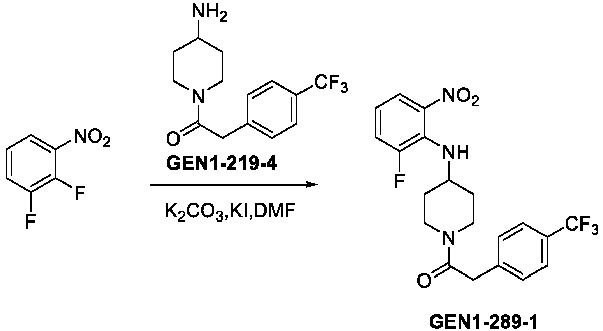

1-(4-aminopiperidin-1-yl)-2-(4-(trifluoromethyl)phenyl)ethan-1-one (85.89 mg, 0.3 mmol, the synthesis steps could be found in GEN1-219-4) was added to a suspension of 1,2-difluoro-3-nitrobenzene (47.73 mg, 0.3 mmol), K₂CO₃ (83 mg, 0.6 mmol) and KI (5 mg, 0.03 mmol) in DMF (2 ml) at room temperature. The resulting mixture was then stirred at 80°C for 3 hours. The reaction mixture was quenched by the addition of brine (30 mL). The aqueous phase was extracted with EtOAc (15 mL×3). The combined organic phase was washed with brine (20 mL×2), dried over anhydrous Na₂SO₄, and it was concentrated to obtain the desired compound (100 mg). The crude product was used directly in the next step without purification. MS (ESI): Rt = 1.69 min, *m*/*z* 426.1 [M+H]⁺; Purity: 63.13% @ 254 nm, 89.05% @ 214 nm.

### Step 2. Synthesis of 1-(4-((2-amino-6-fluorophenyl)amino)piperidin-1-yl)-2-(4-(trifluoromethyl)phenyl)ethan-1-one (GEN1-289-2)

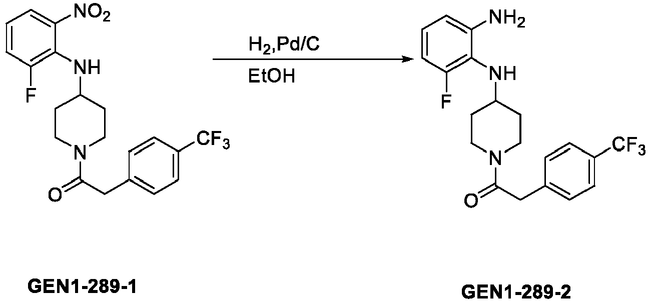

Pd/C (5%, 10mg) was added to a solution of 1-(4-((2-fluoro-6-nitrophenyl)amino)piperidin-1-yl)-2-(4-(trifluoromethyl)phenyl)ethan-1-one (100 mg , 0.235 mmol) in EtOH (20ml). The mixture was then replaced with hydrogen and stirred at room temperature under a hydrogen atmosphere for 16 hours. Then the reaction mixture was filtered, and the filtrate was concentrated to obtain a crude product. The crude product was purified by silica gel column chromatography (PE:EA = 1:1) to obtain the desired product (80mg, yield 86.1%). MS (ESI): Rt = 1.57 min, *m*/*z* 396.2 [M+H]⁺; Purity: 90.02% @ 254 nm.

### Step 3. Synthesis of 7-fluoro-1-(1-(2-(2-(4-(trifluoromethyl)phenyl)acetyl)piperidin-4-yl)-1,3-dihydro-2H-benzo[d]imidazol-2-one (GEN1-289)

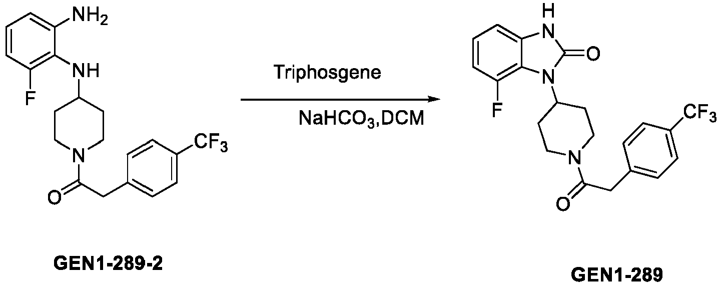

Triphosgene (24 mg, 0.081 mmol) was added to a suspension of 1-(4-(((2-amino-6-fluorophenyl)amino)piperidin-1-yl)-2-(4-(trifluoromethyl)phenyl)ethan-1-one (80 mg, 0.202 mmol), NaHCO₃ (170 mg, 2.02 mmol) in DCM (2 ml) at 0°C under nitrogen protection. The resulting mixture was then stirred at room temperature for 16 hours. Then the reaction mixture was filtered, and the filtrate was concentrated to obtain a crude product. The crude product was purified by reverse phase chromatography. Purification conditions: C18 silica gel column; mobile phase ACN: H₂O, gradient: 5% to 50%; 45 minutes, monitoring wavelength: 214 nm. The desired compound (50 mg, yield 58.74%) was obtained as a white solid. 1H NMR (400 MHz, DMSO-d6) δ 11.12 (s, 1H), 7.72 (d, J = 7.9 Hz, 2H), 7.52 (d, J = 7.9 Hz, 2H), 7.09 - 6.97 (m, 1H), 6.93 - 6.81 (m, 2H), 4.64-4.54 (m, 2H), 4.15 (d, J = 13.9 Hz, 1H), 4.00 - 3.85 (m, 2H), 3.22 (t, J = 13.1 Hz, 1H), 2.73 (t, J = 12.8 Hz, 1H), 2.02 - 1.91 (m, 2H), 1.1.82-1.69 (m, 2H). MS (ESI): Rt = 1.49 min, *m*/*z* 422.3 [M+H]⁺; Purity: 100% @ 254 nm, 100% @ 214 nm.

### Example 154: 7-bromo-1-(1-(1-(2-(4-(trifluoromethyl)phenyl)acetyl)piperidin-4-yl)-1,3-dihydro-2H-benzo[d]imidazol-2-one (GEN1-290)

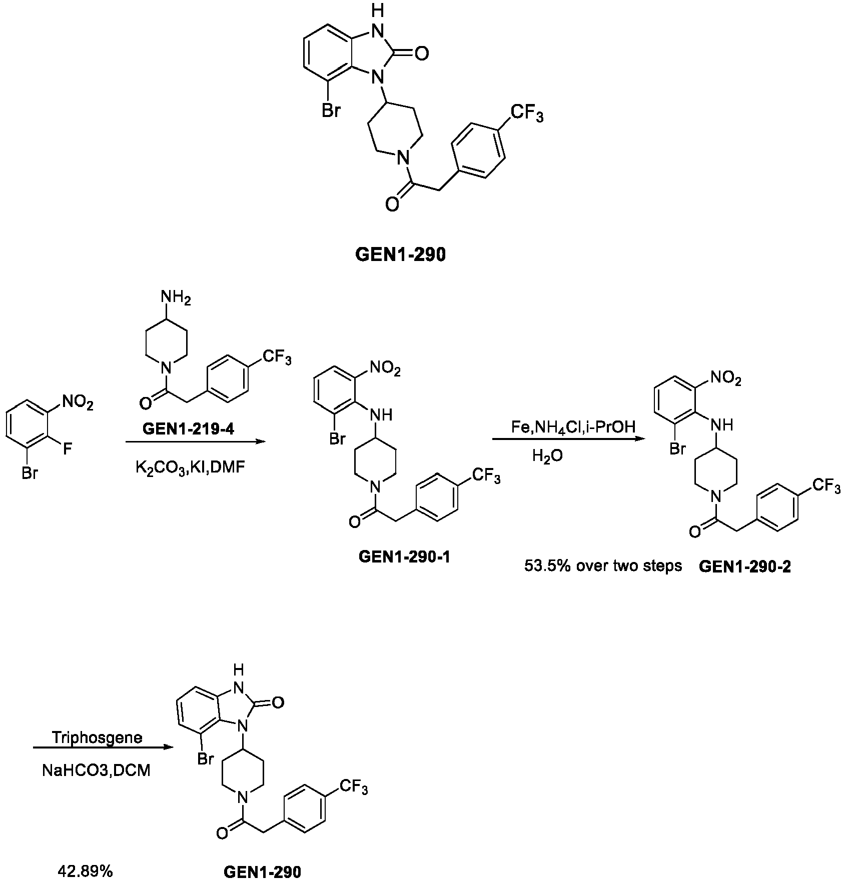

### Step 1. Synthesis of 1-(4-((2-bromo-6-nitrophenyl)amino)piperidin-1-yl)-2-(4-(trifluoromethyl)phenyl)ethan-1-one (GEN1-290-1)

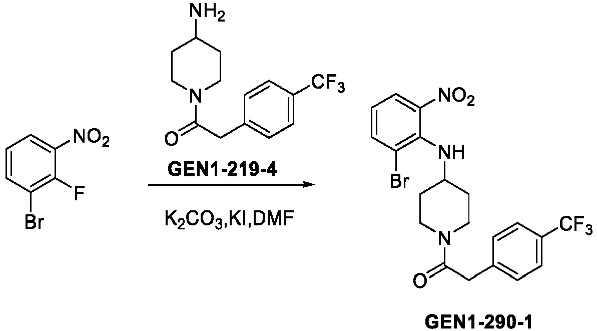

1-(4-aminopiperidin-1-yl)-2-(4-(trifluoromethyl)phenyl)ethan-1-one (85.89 mg, 0.3 mmol, the synthesis steps could be found in GEN1-219-4) was added to a suspension of 1-bromo-2-fluoro-3-nitrobenzene (66 mg, 0.3 mmol), K₂CO₃ (83 mg, 0.6 mmol) and KI (5 mg, 0.03 mmol) in DMF (2 ml) at room temperature. The resulting mixture was then stirred at 80°C for 3 hours. The reaction mixture was quenched by the addition of brine (30 mL). The aqueous phase was extracted with EtOAc (15 mL×3). The combined organic phase was washed with brine (20ml×2), dried over Na₂SO₄, and it was concentrated to obtain the desired compound (140mg). The crude product was used directly in the next step. MS (ESI): Rt = 1.72 min, *m*/*z* 488.0 [M+H]⁺; Purity: 93.41% @ 254 nm, 94.95% @ 214 nm.

### Step 2. Synthesis of 1-(4-((2-amino-6-bromophenyl)amino)piperidin-1-yl)-2-(4-(trifluoromethyl)phenyl)ethan-1-one (GEN1-290-2)

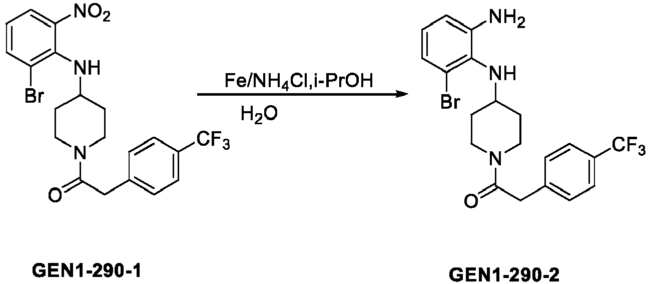

Fe (168 mg, 3 mmol) was added to a mixture of 1-(4-((2-bromo-6-nitrophenyl)amino)piperidin-1-yl)-2-(4-(trifluoromethyl)phenyl)ethan-1-one (140 mg, 0.288 mmol), NH₄Cl (321 mg, 0.6 mmol) in i-PrOH (5 ml) and H₂O (1 ml). The resulting mixture was stirred at room temperature for 16 hours. Then the reaction mixture was filtered, and the filtrate was concentrated to obtain a crude product. The crude product was purified by silica gel column chromatography (PE:EA = 1:1) to obtain the desired compound (70 mg, two steps yield: 53.3%). MS (ESI): Rt = 1.65 min, *m*/*z* 457.0 [M+H]⁺; Purity: 77.07% @ 254 nm, 66.17% @ 214 nm.

### Step 3. Synthesis of 7-bromo-1-(1-(1-(2-(4-(trifluoromethyl)phenyl)acetyl)piperidin-4-yl)-1,3-dihydro-2H-benzo[d]imidazol-2-one (GEN1-290)

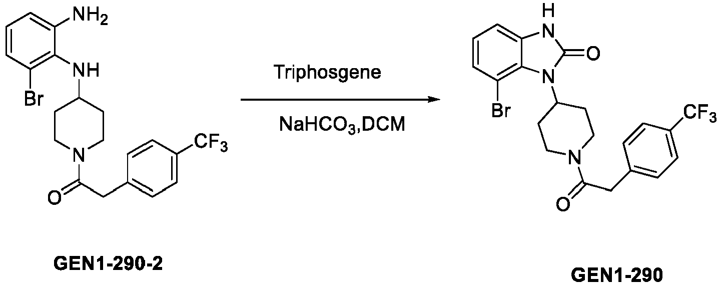

Triphosgene (17.2 mg, 0.058 mmol) was added to a suspension of 1-(4-((2-amino-6-bromophenyl)amino)piperidin-1-yl)-2-(4-(trifluoromethyl)phenyl)ethan-1-one (70 mg, 0.145 mmol), NaHCO₃ (121.8 mg, 1.45 mmol) in DCM (2 ml) at 0°C under nitrogen protection. The resulting mixture was then stirred at room temperature for 16 hours. The reaction mixture was filtered, and the filtrate was concentrated to obtain a crude product. The crude product was purified by reverse phase chromatography. Purification conditions: C18 silica gel column; mobile phase ACN: H₂O, gradient: 5% to 50%; 45 minutes, monitoring wavelength: 214 nm. The desired compound (30 mg, yield 42.89%) was obtained as a white solid. 1H NMR (400 MHz, DMSO-d6) δ 11.08 (s, 1H), 7.70 (d, J = 7.9 Hz, 2H), 7.51 (d, J = 7.8 Hz, 2H), 7.20 (d, J = 8.0 Hz, 1H), 7.05 - 6.86 (m, 2H), 5.30 (s, 1H), 4.60 (d, J = 13.0 Hz, 1H), 4.18 (d, J = 13.7 Hz, 1H), 4.04 - 3.80 (m, 2H), 3.12 (t, J = 13.4 Hz, 1H), 2.65 (t, J = 13.0 Hz, 1H), 2.52-2.38(m, 2H), 1.86-1.76 (m, 2H). MS (ESI): Rt = 1.68 min, *m*/*z* 484.2 [M+H]⁺; Purity: 100% @ 254 nm, 100% @ 214 nm.

### Example 155: 7-chloro-1-(1-(1-(2-(quinolin-6-yl)acetyl)piperidin-4-yl)-1,3-dihydro-2H-benzo[d]imidazol-2-one (GEN1-291)

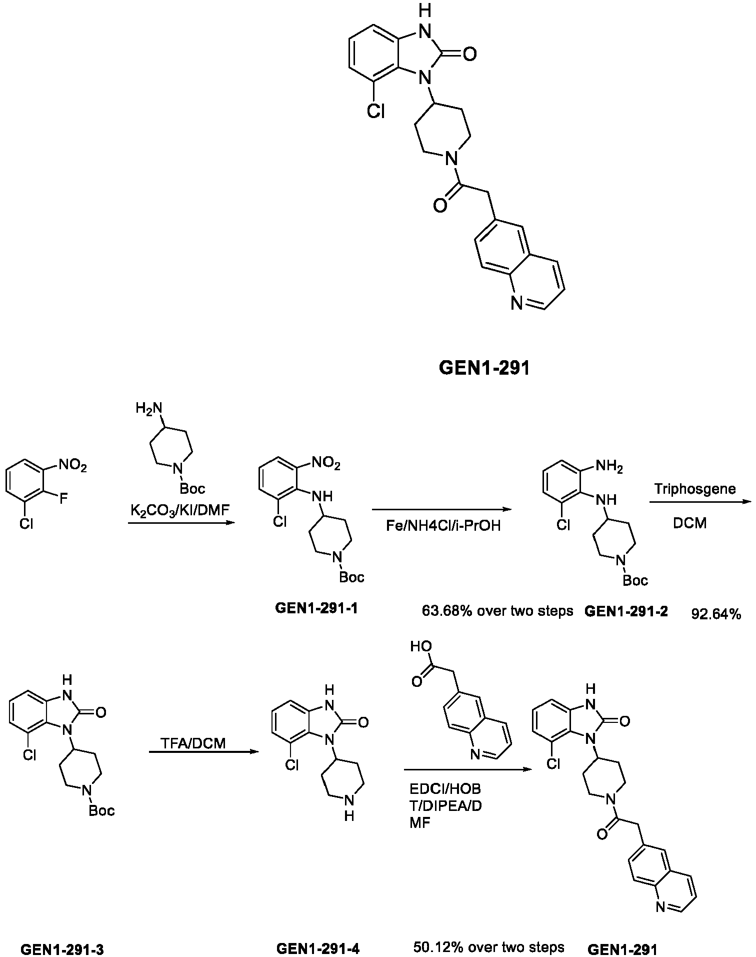

### Step 1. Synthesis of tert-butyl 4-((2-chloro-6-nitrophenyl)amino)piperidin-1-carboxylate (GEN1-291-1)

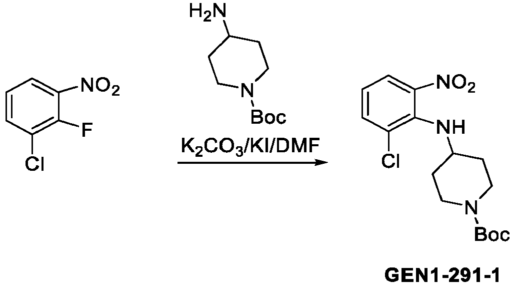

Tert-butyl 4-aminopiperidine-1-carboxylate (661 mg, 3.3 mmol) was added to a suspension of 1-chloro-2-fluoro-3-nitrobenzene (526.62 mg, 3 mmol), K₂CO₃ (828 mg, 6 mmol) and KI (50 mg, 0.3 mmol) in DMF (20 ml) at room temperature. The resulting mixture was then stirred at 80°C for 3 hours. The reaction mixture was quenched by the addition of brine (100 mL). It was extracted with EtOAc (30 mL×3). The combined organic phase was washed with brine (30 mL×2), dried over anhydrous Na₂SO₄, and it was concentrated to obtain the desired compound (900 mg). The crude product was used directly in the next step. MS (ESI): Rt = 1.81 min, *m*/*z* 378.0 [M+Na]⁺; Purity: 97.23% @ 254 nm, 96.42% @ 214 nm.

### Step 2. Synthesis of tert-butyl 4-((2-amino-6-chlorophenyl)amino)piperidin-1-carboxylate (GEN1-291-2)

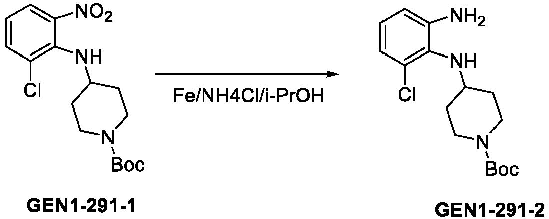

Fe (1.417 g, 25.3 mmol) was added to a mixed solution of tert-butyl 4-((2-chloro-6-nitrophenyl)amino)piperidin-1-carboxylate (900 mg, 2.53 mmol), NH₄Cl (2.706 g, 50.6 mmol) in i-PrOH (25 ml) and water (5 ml). The resulting mixture was stirred at room temperature for 16 hours. The reaction mixture was filtered, and the filtrate was concentrated to obtain a crude product. The crude product was purified by silica gel column chromatography (PE:EA = 1:1) to obtain the desired product (525 mg, two steps yield: 63.68%).

### Step 3. Synthesis of tert-butyl 4-(7-chloro-2-oxo-2,3-dihydro-1H-benzo[d]imidazol-1-yl)piperidin-1-carboxylate (GEN1-291- 3)

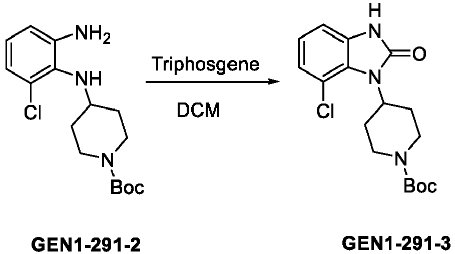

Triphosgene (91 mg, 0.307 mmol) was added to a suspension of tert-butyl 4-((2-amino-6-chlorophenyl)amino)piperidin-1-carboxylate (250 mg, 0.767 mmol), NaHCO₃ (644.3 mg, 7.67 mmol) in DCM (20 ml) at 0°C under nitrogen protection. The resulting mixture was then stirred at room temperature for 16 hours. The reaction mixture was filtered, and the filtrate was concentrated to obtain a crude product. The crude product was purified by silica gel column chromatography (PE:EA = 2:1) to obtain the desired compound (250 mg, yield 92.64%) as a white solid. MS (ESI): Rt = 1.62 min, *m*/*z* 350.0 [M-H]⁻; Purity: 64.8% @ 254 nm, 54.01% @ 214 nm.

### Step 4. Synthesis of 7-chloro-1-(piperidm-4-yl)-1,3-dihydro-2H-benzo[d]imidazol-2-one (GEN1-291-4)

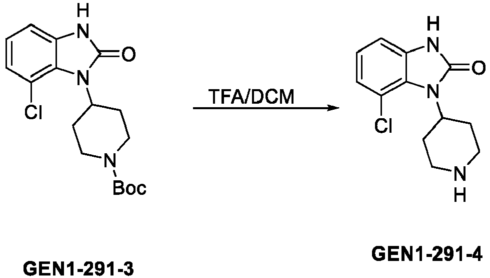

TFA (4 ml) was added to a solution of tert-butyl 4-(7-chloro-2-oxo-2,3-dihydro-1H-benzo[d]imidazol-1-yl)piperidin-1-carboxylate (250 mg, 0.71 mmol) in DCM (20ml) at 0°C. The resulting mixture was then stirred at room temperature for 1 h. The reaction mixture was concentrated to obtain (300 mg) a crude product. The crude product was used directly in the next step.

### Step 5. Synthesis of 7-chloro-1-(1-(1-(2-(quinolin-6-yl)acetyl)piperidin-4-yl)-1,3-dihydro-2H-benzo [d] imidazol-2-one (GEN1-291)

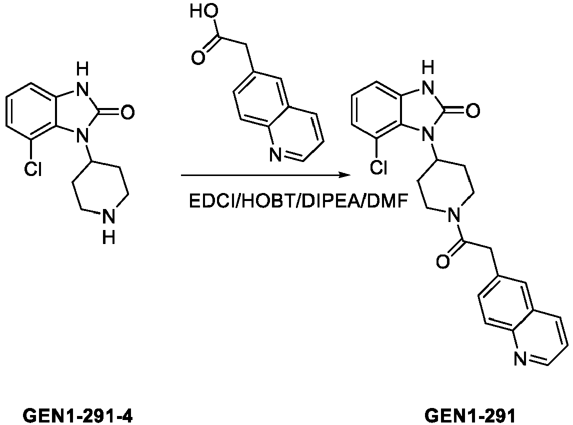

DIPEA (122.4 mg , 0.948 mmol) was added to a solution of 7-chloro-1-(piperidin-4-yl)-**1,3-dihydro-2H-benzo[d]imidazol-2-one** (100 mg, crude product, 0.237 mmol), 2-(quinolin-6-yl)acetic acid (53.16 mg, 0.284 mmol), HOBT (48 mg, 0.355 mmol), EDCI (68 mg, 0.355 mmol) in DMF (2mL). The resulting mixture was then stirred at room temperature for 1 h. The reaction mixture was diluted with water (30 mL), and extracted with EtOAc (20 mL×3). The combined organic phase was washed with brine (30 mL), dried over anhydrous Na₂SO₄ and filtered. The filtrate was concentrated to obtain a crude product. The crude product was purified by reverse phase chromatography. Purification conditions: C18 silica gel column; mobile phase ACN: H₂O, gradient: 5% to 50%; 45 minutes, monitoring wavelength: 214 nm. The desired compound (50 mg, yield 50.12%) was obtained as a white solid. 1H NMR (400 MHz, DMSO-d6) δ 11.23 (s, 1H), 8.92-8.87 (m, 1H), 8.36 (d, J = 8.3 Hz, 1H), 7.99 (d, J = 8.7 Hz, 1H), 7.85 (s, 1H), 7.75 - 7.63 (m, 1H), 7.59-7.49 (m, 1H), 7.09 - 6.89 (m, 3H), 5.13 (s, 1H), 4.63 (d, J = 13.2 Hz, 1H), 4.22 (d, J = 13.8 Hz, 1H), 4.10-3.95 (m, 2H), 3.17 (t, J = 13.3 Hz, 1H), 2.69 (t, J = 13.1 Hz, 1H), 2.50-2.34 (m, 2H), 1.90-1.75 (m, 2H). MS (ESI): Rt = 1.37 min, *m*/*z* 421.3 [M+H]⁺; Purity: 100% @ 254 nm, 100% @ 214 nm.

### Example 156: 7-methyl-1-(1-(2-(quinolin-6-yl)acetyl)piperidin-4-yl)-1,3-dihydro-2H-benzo[d]imidazol-2-one (GEN1-292)

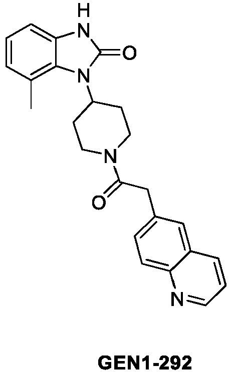

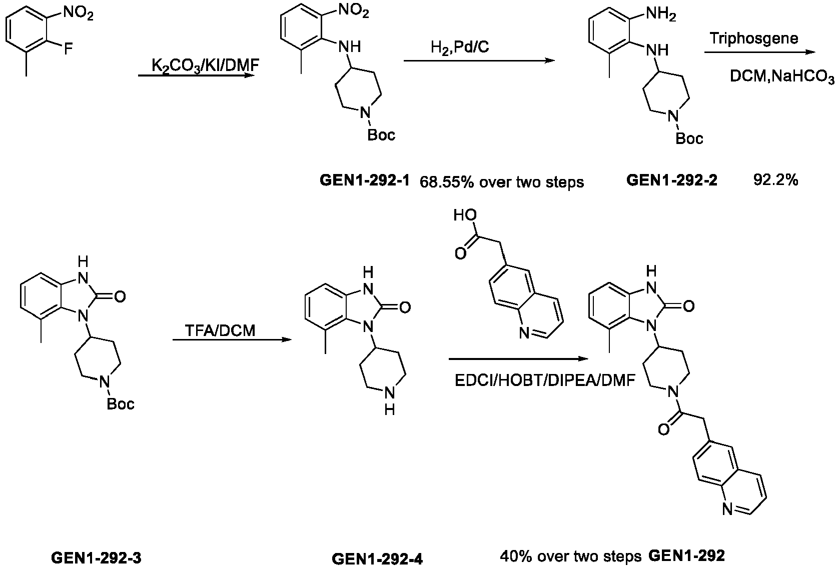

### Step 1. Synthesis of tert-butyl 4-((2-methyl-6-nitrophenyl)amino)piperidin-1-carboxylate (GEN1-292-1)

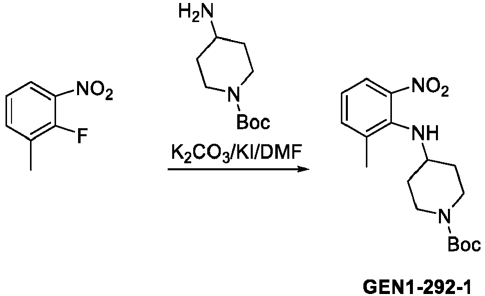

Tert-butyl 4-aminopiperidine-1-carboxylate (661 mg, 3.3 mmol) was added to a suspension of 2-fluoro-1-methyl-3-nitrobenzene (465 mg, 3 mmol), K₂CO₃ (828 mg, 6 mmol) and KI (50 mg, 0.3 mmol) in DMF (20 ml) at room temperature. The resulting mixture was then stirred at 80°C for 3 hours. The reaction mixture was quenched by the addition of brine (100 mL). It was extracted with EtOAc (30 mL×3). The combined organic phase was washed with brine (30 mL×2), dried over anhydrous Na₂SO₄, and it was concentrated to obtain the desired compound (800 mg, crude product). The crude product was used directly in the next step.

### Step 2. Synthesis of tert-butyl 4-((2-amino-6-methylphenyl)amino)piperidin-1-carboxylate (GEN1-292-2)

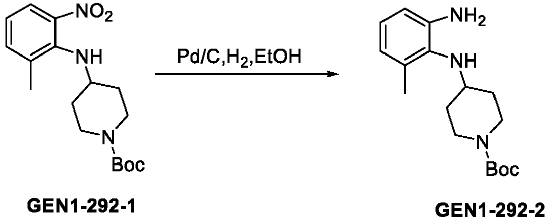

Pd/C (40 mg, 5% purity) was added to a solution of tert-butyl 4-((2-methyl-6-nitrophenyl)amino)piperidin-1-carboxylate (800 mg, crude product, 2.388 mmol) in EtOH (50 ml). The resulting mixture was then replaced with hydrogen and stirred at room temperature under a hydrogen atmosphere for 16 hours. The reaction mixture was filtered, and the filtrate was concentrated to obtain a crude product. The crude product was purified by silica gel column chromatography (PE:EA=1:1) to obtain the desired compound (500 mg, two steps yield: 68.55%).

### Step 3. Synthesis of tert-butyl 4-(7-methyl-2-oxo-2,3-dihydro-1H-benzo[d]imidazol-1-yl)piperidin-1-carboxylate (GEN1-292-3)

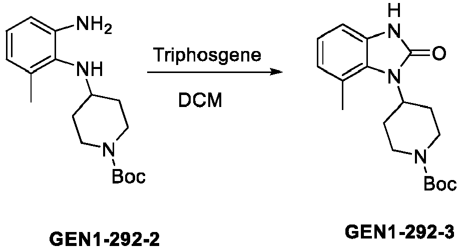

Phosgene (97.1 mg, 0.327 mmol) was added to a suspension of tert-butyl 4-((2-amino-6-methylphenyl)amino)piperidin-1-carboxylate (250 mg, 0.818 mmol), NaHCO₃ (687.6 mg, 8.18 mmol) in DCM (10 ml) under nitrogen protection at 0°C. The resulting mixture was then stirred at room temperature for 16 hours. The reaction mixture was filtered, and the filtrate was concentrated to obtain a crude product. The crude product was purified by silica gel column chromatography (PE:EA = 2:1) to obtain the desired compound (250 mg, yield 92.2%) as a white solid. MS (ESI): Rt = 1.54 min, *m*/*z* 330.1 [M-H]⁻; Purity: 97.23% @ 254 nm, 96.42% @ 214 nm.

### Step 4. Synthesis of 7-methyl-1-(piperidin-4-yl)-1,3-dihydro-2H-benzo[d]imidazol-2-one (GEN1-292-4)

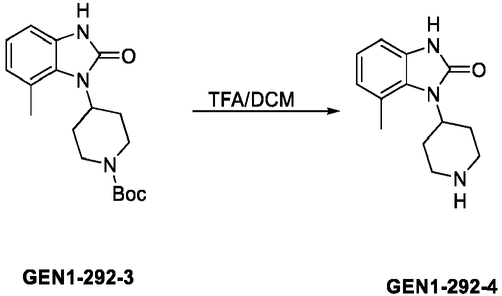

TFA (4 ml) was added to a solution of tert-butyl 4-(7-methyl-2-oxo-2,3-dihydro-1H-benzo[d]imidazol-1-yl)piperidin-1-carboxylate (250 mg, 0.754 mmol) in DCM (20 ml) at 0°C. The resulting mixture was then stirred at room temperature for 1 h. The reaction mixture was concentrated to obtain (300 mg) crude product. The crude product was used directly in the next step.

### Step 5. Synthesis of 7-methyl-1-(1-(2-(quinolin-6-yl)acetyl)piperidin-4-yl)-1,3-dihydro-2H-benzo [d] imidazol-2-one (GEN1-292)

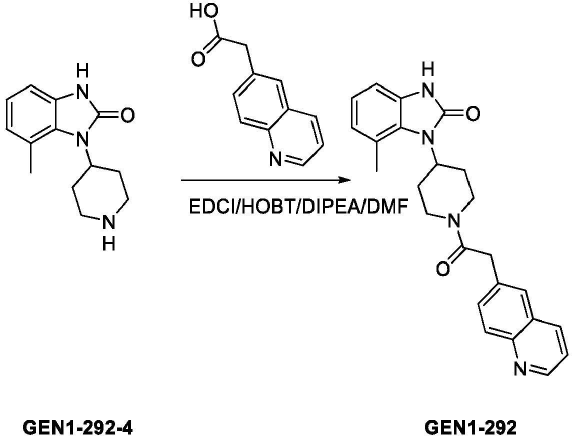

DIPEA (129.2 mg, 1.0 mmol) was added to a solution of 7-methyl-1-(piperidin-4-yl)-1,3-dihydro-2H-benzo[d]imidazol-2-one (100 mg, crude, 0.25 mmol), 2-(quinolin-6-yl)acetic acid (56.46 mg, 0.30 mmol), HOBT (50.6 mg, 0.375 mmol) and EDCI (71.9 mg, 0.375 mmol) in DMF (2 mL) at room temperature. The resulting mixture was then stirred at room temperature for 1 h. The reaction mixture was diluted with water (30 mL), and extracted with EtOAc (20 mL×3). The combined organic phase was washed with brine (30 mL), dried over anhydrous Na₂SO₄ and filtered. The filtrate was concentrated to obtain the crude product. The crude product was purified by reverse phase chromatography. Purification conditions: C18 silica gel column; mobile phase ACN: H₂O, gradient: 5% to 50%; 45 minutes, monitoring wavelength: 214 nm. The desired compound (40 mg, yield 40%) was obtained as a white solid. 1H NMR (400 MHz, DMSO-d6) 1H NMR (400 MHz, DMSO-d6) δ 10.81 (s, 1H), 8.92-8.87(m, 1H), 8.36 (d, J = 8.4 Hz, 1H), 8.00 (d, J = 8.7 Hz, 1H), 7.85 (s, 1H), 7.675-7.65 (m, 1H), 7.62 -7.48 (m, 1H), 6.98 - 6.67 (m, 3H), 4.66-4.52 (m, 2H), 4.25-4.14 (m, 1H), 4.11 - 3.90 (m, 2H), 3.17 (t, J = 13.1 Hz, 1H), 2.70 (t, J = 12.9 Hz, 1H), 2.57 (s, 3H), 2.2.50-2.30 (m, 2H), 1.87-1.76(m, 2H).MS (ESI): Rt = 3.317 min, *m*/*z* 401.2 [M+H]⁺; Purity: 93.5% @ 254 nm, 96.83% @ 214 nm.

### Example 157: 6-chloro-1-(1-(2-(quinolin-6-yl)acetyl)piperidin-4-yl)-1,3-dihydro-2H-benzo[d]imidazol-2-one (GEN1-293)

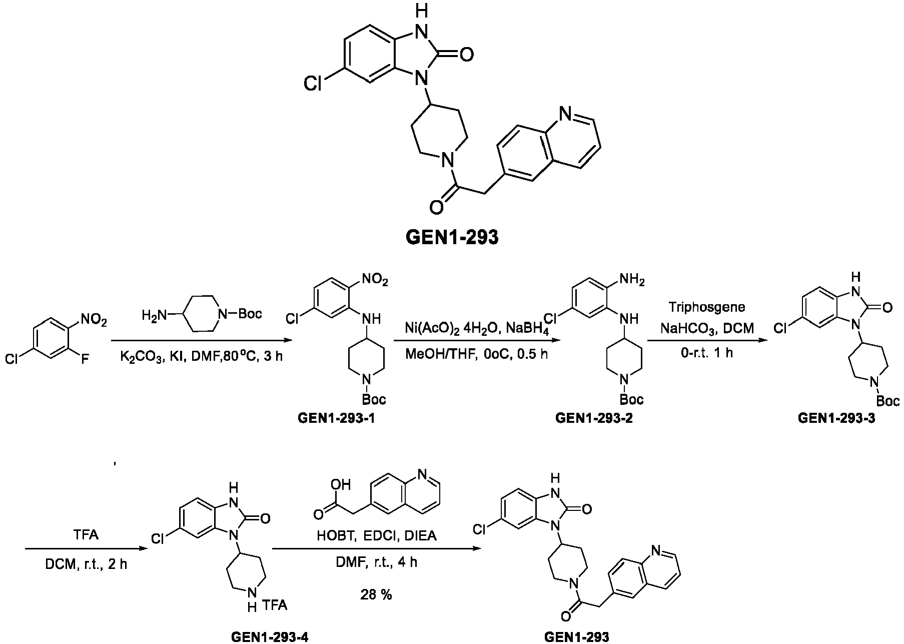

### Step 1. Tert-butyl 4-((5-chloro-2-nitrophenyl)amino)piperidin-1-carboxylate (GEN1-293-1)

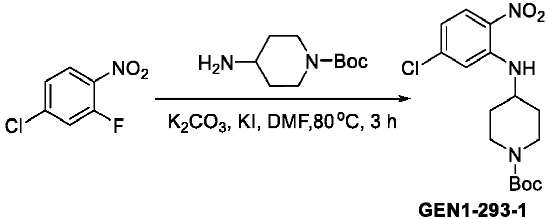

4-chloro-2-fluoro-1-nitrobenzene (500 mg, 2.86 mmol), tert-butyl 4-aminopiperidine-1-carboxylate (571 mg, 2.86 mmol), K₂CO₃ (491 mg, 4.28 mmol) and KI (47 mg, 0.28 mmol) were suspended in DMF (5.0 mL) and the suspension was stirred at 80°C for 3 hours. After it was cooled to room temperature, the resulting mixture was filtered, and the filter cake was washed with EA. The filtrate was concentrated under reduced pressure. The desired compound (1.27 g crude product) was obtained and used directly in the next step without further purification. MS (ESI) Rt=1.83 min, m/z 354.0 [M-H]⁻, Purity: 97% @ 254 nm, 98% @ 214 nm.

### Step 2. Tert-butyl 4-((2-amino-5-chlorophenyl)amino)piperidin-1-carboxylate (GEN1-293-2)

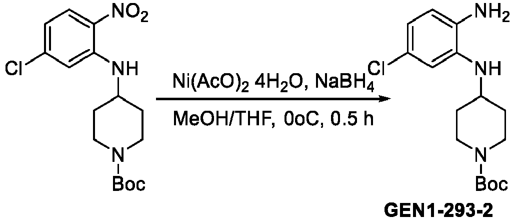

NaBH₄ (522 mg, 13.7 mmol) was added in batches to a mixed solution of tert-butyl 4-((5-chloro-2-nitrophenyl)amino)piperidin-1-carboxylate (1.22 g, 3.44 mmol) and Ni(AcO)₂ 4H₂O (1.71 g, 6.87 mmol) in MeOH (24 mL) and THF (12 mL) at 0°C. The resulting mixture was stirred at 0°C under N₂ atmosphere for 0.5 hour. The reaction mixture was poured into aqueous NH₄Cl solution (50 mL), and extracted with EA (65 mL×3). The organic layer was washed with water (150 mL), brine (150 mL), dried over Na₂SO₄ and it was concentrated to obtain the desired compound (990 mg crude product) which was used directly in the next step without further purification. MS (ESI) Rt=1.65 min, m/z 324.0 [M-H]⁻, Purity: 100% @ 254 nm, 97% @ 214 nm.

### Step 3. Tert-butyl 4-(6-chloro-2-oxo-2,3-dihydro-1H-benzo[d]imidazol-1-yl)piperidin-1-carboxylate (GEN1-293-3)

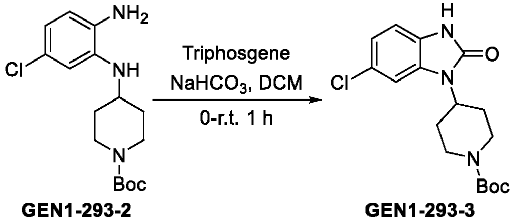

Triphosgene (895 mg, 3.04 mmol) was added in batches to a solution of tert-butyl 4-(((2-amino-5-chlorophenyl)amino)piperidin-1-carboxylate (990 mg, crude product) and NaHCO₃ (2.56 g, 30.5 mmol) in DCM (100 mL) at 0°C. The resulting mixture was stirred at room temperature under N₂ atmosphere overnight. The reaction mixture was poured into water (150 mL), and extracted with DCM (100 mL×3). The organic layer was washed with water (300 mL), brine (300 mL), dried over Na₂SO₄ and it was concentrated to obtain a pale yellow solid. The desired compound (1.1 g crude product) was obtained and used in the next step without further purification. MS (ESI) Rt=1.57 min, m/z 350.0 [M-H]⁻, Purity: 76% @ 254 nm, 98% @ 214 nm.

### Step 4. 6-chloro-1-(piperidin-4-yl)-1,3-dihydro-2H-benzo[d]imidazol-2-one trifluoroacetate (GEN1-293-4)

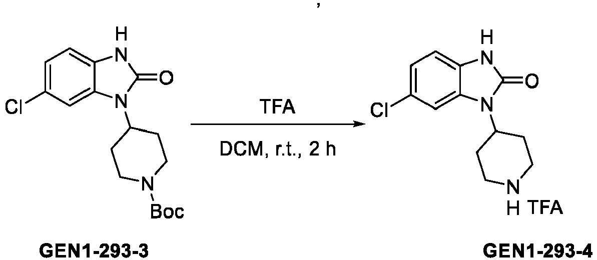

TFA (3.0 mL) was added dropwise to a solution of tert-butyl 4-(6-chloro-2-oxo-2,3-dihydro-1H-benzo[d]imidazol-1-yl)piperidin-1-carboxylate (300 mg crude product) in DCM (30 mL). The resulting mixture was stirred at room temperature under N₂ atmosphere for 2 hours. The resulting mixture was concentrated under vacuum. The desired compound (388 mg crude product) was obtained and used directly in the next step without further purification. TLC: MeOH/(DCM+MeOH)=5%, Rf=0.5

### Step 5. 6-chloro-1-(1-(2-(quinolin-6-yl)acetyl)piperidin-4-yl)-1,3-dihydro-2H-benzo[d]imidazol-2-one (GEN1-293)

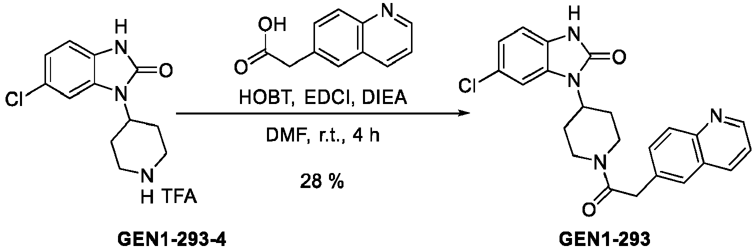

DIEA (441 mg, 3.42 mmol) was added dropwise to a solution of compound 2,2,2-trifluoroacetaldehyde and 6-chloro-1-(piperidin-4-yl)-1,3-dihydro-2H-benzo[d]imidazol-2-one (388 mg crude product), 2-(quinolin-6-yl)acetic acid (144 mg, 0.77 mmol), EDCI (1221 mg, 1.15 mmol), HOBT (156 mg, 1.15 mmol) in DMF (4.0 mL). The resulting mixture was stirred at room temperature for 4 hours. The reaction mixture was poured into water (30 mL), and extracted with EA (45 mL×3). The combined organic layer was washed with water (150 mL), brine (150 mL), dried over Na₂SO₄ and it was concentrated to obtain a yellow oil. The crude product was purified by reverse-phase flash chromatography (C₁₈ reverse silica column; mobile phase CH₃CN, water, 15% to 75% gradient, 50 minutes; detection, UV 214 nm) to obtain the desired compound (92 mg, yield 28%) as a white solid. ¹H NMR (400 MHz, DMSO-*d*₆) δ: 11.02 (s, 1H), 8.90-8.83 (m, 1H), 8.34 (d, *J*=8.3 Hz, 1H), 7.99 (d, *J*=8.6 Hz, 1H), 7.85 (s, 1H), 7.68 (d, *J*= 8.7, 1H), 7.54-7.48 (m, 1H), 7.28 (s, 1H), 7.02-6.91 (m, 2H), 4.61 (d, *J*=13.2 Hz, 1H), 4.48-4.35 (m, 1H), 4.16 (d, *J*=13.6 Hz, 1H), 4.01 (s, 2H), 3.17 (t, *J*=13.0 Hz, 1H), 2.70 (t, *J*=12.8 Hz, 1H), 2.24-2.09 (m, 2H), 1.77-1.62 (m, 2H). MS (ESI) Rt=1.35 min, m/z 421.3 [M+H]⁺, Purity: 100% @ 254 nm, 100% @ 214 nm.

### Example 158: 5-chloro-1-(1-(1-(2-(quinolin-6-yl)acetyl)piperidin-4-yl)-1,3-dihydro-2H-benzo[d]imidazol-2-one (GEN1-294)

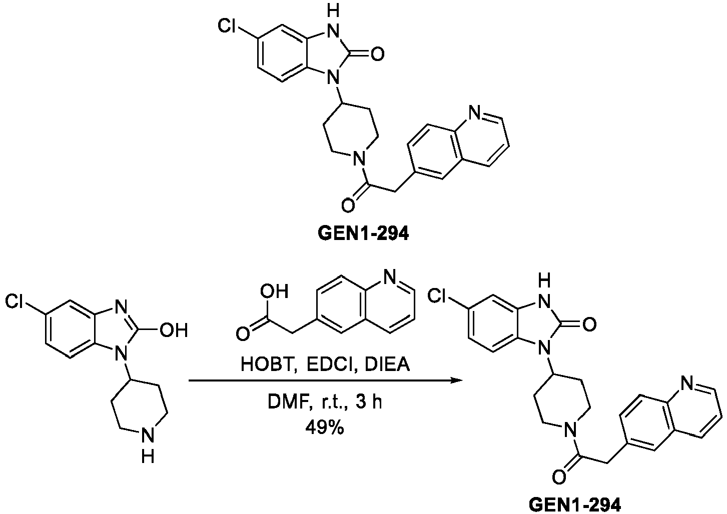

### Step 1. 5-chloro-1-(1-(1-(2-(quinolin-6-yl)acetyl)piperidin-4-yl)-1,3-dihydro-2H-benzo[d]imidazol-2-one (GEN1-294)

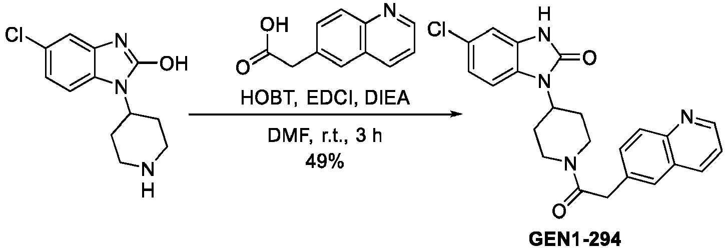

DIEA (154 mg, 1.19 mmol) was added dropwise to a solution of 5-chloro-1-(piperidin-4-yl)-1H-benzo[d]imidazol-2-ol (150 mg, 0.60 mmol), 2-(quinolin-6-yl)acetic acid (112 mg, 0.60 mmol), EDCI (138 mg, 0.72 mmol) and HOBT (97 mg, 0.72 mmol) in DMF (1.5 mL). The resulting mixture was stirred at room temperature for 3 hours. The reaction mixture was poured into water (10 mL), and extracted with EA (15 mL×3). The combined organic layer was washed with water (40 mL), brine (40 mL), dried over Na₂SO₄ and it was concentrated to obtain a yellow oil. The crude product was purified by reverse-phase flash chromatography (C₁₈ reverse silica column; mobile phase CH₃CN, water, 15% to 75% gradient, 50 minutes; detection, UV 214 nm) to obtain the desired compound (123 mg, yield 49%) as a white solid. ¹H NMR (400 MHz, DMSO-*d*₆): δ 11.03 (s, 1H), 8.90-8.83 (m, 1H), 8.36 (d, *J*=8.2 Hz, 1H), 8.00 (d, *J*=8.6 Hz, 1H), 7.87 (d, *J*=2.0 Hz, 1H), 7.70 (d, *J*=8.7, 1H), 7.57-7.47 (m, 1H), 7.01-6.93 (m, 2H), 6.92-6.83 (m, 1H), 4.60 (d, *J*=13.1 Hz, 1H), 4.49-4.34 (m, 1H), 4.16 (d, *J*=13.8 Hz, 1H), 4.10-3.91 (m, 2H), 3.18 (t, *J*=13.0 Hz, 1H), 2.71 (t, *J*=12.8 Hz, 1H), 2.13-1.87 (m, 2H), 1.76-1.55 (m, 2H). MS (ESI) Rt=1.35 min, m/z 421.3 [M+H]⁺, Purity: 100% @ 254 nm, 100% @ 214 nm.

### Example of Effect 1: Protein electrophoresis and blotting experiment

### Experimental Materials:

Standard protein electrophoresis and chemiluminescence imaging equipment (Bio-Rad);
Protein lysate RIPA (Lianke Biological; Cat#WB020); Western blot transfer membrane PVDF (Millipore; Cat#IPVH00010);
Protein antibody STAT5 Y694 phosphorylation antibody (Cell Signaling Tech. #9351), anti-glyceraldehyde-3-phosphate dehydrogenase (GAPDH) antibody, chemiluminescence imaging reagent (Immobilon Millipore)

### Experimental Process:

Jurkat cells (ATCC American Standard Culture Center, TIB152) were cultured in a 6-well plate at a density of 1x10⁶/ml with RPMI medium without 10% fetal bovine serum in a humidified incubator at 37°C and maintained at 5% carbon dioxide overnight. On the second day, the cells were pretreated with compounds of different concentrations (the mother liquor of the compound prepared by dimethyl sulfoxide was diluted in the medium at the required concentration) for 1 hour, and then stimulated with 20ng/ml human interferon beta for 30 minutes, it was mixed thoroughly and then placed in an incubator at 37°C environment. Then the cells were collected by centrifugation at a speed of 2000 rpm, resuspended in phosphate buffer (standard phosphate buffered saline sodium chloride 137 mmol, potassium chloride 2.7 mmol, sodium hydrogen phosphate 10 mmol, potassium dihydrogen phosphate 1.8 mmol) and washed once, and then the cells were collected by centrifugation. Then an appropriate volume of RIPA Lysis Solution was added, it was mixed well, and placed on ice for 1 hour, inverted and mixed several times in the middle. Then, the cell lysate was centrifuged at 12000 rpm at 4°C for 10 minutes, and the supernatant was transferred to another test tube, aliquoted and stored at - 20°C until use.

An equal volume of protein lysate was mixed with an appropriate 6-fold protein loading buffer (Biyun Tian Biochemical Company P0015F), and the mixture was heated at 95 degrees for 10 minutes, after full denaturation, the protein sample was added to the polyacrylamide gel (self-made, raw material purchased from Soleibao Biotechnology Company A1010, 30% acrylamide solution), and electrophoresis was performed at the appropriate voltage for 1 hour, until the strips were fully separated. It was transferred to a polyvinylidene fluoride membrane according to standard procedures, and blocked with 5% skim milk for 1 hour, and the specified antibody was added, and it was diluted 1:1000 in 3% bovine serum albumin, and it was incubated overnight for four degrees. It was thoroughly rinsed, incubated with the secondary antibody, exposed to chemiluminescence and signals were collected. ImageJ software was used to quantify the obtained chemiluminescence results. The same area was selected, the gray values of STAT5 phosphorylation band and GAPDH band of each sample were analyzed, and the ratio of pSTAT5/GAPDH level was calculated. The value of the solvent-treated sample was 100%, and the remaining samples were converted into relative data in the same proportion, that is, the percentage corresponding to the solvent sample. Table 1 shows the data of inhibition rate of phosphorylation of STAT5 by Y694 (100%-exposure gray scale of the test compound group/exposure gray scale of the solvent control group). The solvent control group was the same as the test compound group except that DMSO was used instead of the DMSO solution of the test compound. N1 (Pimozide) was a specific positive control. Table 6 shows the data of the inhibition ability based on the observed results of pSTAT5 bands.

**Table 1 Quantitative data of inhibitory activity on cell biochemical level**

| Compound Number | Inhibiting Ratio (0% for vehicle control) | | |
|---|---|---|---|
| | 1µM | 10µM | 100µM |
| N1 | -0.6% | 5.9% | 95.4% |
| X3 | 17.4% | 4.7% | 87.5% |
| X4 | 21.2% | 5.3% | 87.8% |
| X7 | -27.9% | -13.6% | 88.5% |
| X8 | 6.9% | 31.4% | 95.6% |
| X9 | 6.0% | 44.3% | 100.0% |
| GEN 1-001 | 11.0% | 29.2% | 96.8% |
| GEN1-003 | -10.8% | 22.2% | 94.1% |
| GEN1-005 | 28.0% | 44.4% | 95.6% |
| GEN1-006 | -2.8% | 35.7% | 94.8% |
| GEN1-007 | 42.4% | 54.8% | 96.6% |
| GEN1-010 | -29.0% | 17.5% | 94.8% |
| GEN1-013 | 12.4% | 62.6% | 97.0% |
| GEN1-014 | 14.2% | 78.8% | 99.1 % |
| GEN1-015 | 9.7% | 43.4% | 95.7% |
| GEN1-018 | -36.5% | -6.2% | 98.6% |
| GEN1-021 | -39.8% | -26.3% | 97.6% |
| GEN1-022 | 15.8% | 29.5% | 81.2% |
| GEN1-023 | 9.4% | 33.3% | 93.6% |
| GEN1-028 | 14.5% | 32.6% | 93.3% |
| GEN1-029 | -74.1 % | -67.7% | 92.3% |
| GEN1-030 | 15.7% | 19.4% | 94.0% |
| GEN1-031 | 19.7% | 38.1% | 98.7% |
| GEN1-032 | -16.3% | 13.8% | 94.9% |
| GEN1-033 | 12.8% | 7.6% | 98.0% |
| GEN1-055 | 25.0% | 50.0% | 89.6% |

### Example of Effect 2: Evaluation of the therapeutic efficacy of X8 compound in a mouse collagen-induced arthritis model

Sixty male DBA/1J mice (9-10 weeks old, H-2q haplotype) were acclimatized in a standard animal room for one week and then randomly divided into 5 groups. Except for the healthy control group, the rest were inoculated with collagen antigen injection, and then received oral treatment with different compounds. The brief procedure was to mix bovine type II collagen (purchased from Chondrex, 20021) with an equal volume of Freund's complete adjuvant (purchased from Sigma, F5881), the concentration was 4 mg/ml, and it was treated with a homogenizer at a speed of 20,000 revolutions/min for 5-10 minutes. A needle was inserted between the left side of the dorsal caudal artery and the lateral vein of the mouse, and 50 microliters of modeling agent was injected subcutaneously. 21 days after the first injection, another injection was injected between the right side of the dorsal tail artery and the lateral vein of the mouse. After the first injection, oral administration was started. The drug was formulated with 30% PEG300, and it was prepared for immediate use. Table 2 shows the specific groups, and Table 3 shows the scoring standard. The total score was between 0-12 points, and the swelling of the foot after vaccination was not calculated. Tables 4 and 5 show the results of test.

**Table 2 Groups of animals and plans of treatment**

| **Group** | **Name** | **Amount** | **Concentration of the Drug** | **Route and Frequency of Administration** |
|---|---|---|---|---|
| G1 | Healthy control | 12 | None | None |
| G2 | Vehicle treatment | 10 | None | Once a day |
| G3 | Tofacitinib | 12 | 5 mg/kg | Once a day |
| G4 | Methotrexate treatment | 10 | 0.25 mg/kg | Once a week |
| G5 | X8 treatment | 12 | 1 mg/kg | Once a day |

**Table 3 Clinical scoring criteria for arthritis**

| Score | Degree of Inflammation |
|---|---|
| 0 | Normal |
| 1 | Inflammation and swelling of one toe |
| 2 | Inflammation and swelling of more than one but not the entire plantar, or mild swelling of the entire plantar |
| 3 | Inflammation and swelling of the entire plantar |
| 4 | Severe inflammation, swelling, or stiffness of the foot and plantar joints. If the joints are stiff, the mouse cannot grasp the cage lid grid. |

**Table 4 Data on changes in body weight of animals**

| Time/ day | Healthy control n=12 | | Vehicle treatment n=10 | | Methotrexate - 0.25mg/kg n=10 | | Tofacitinib -5mg/kg n=12 | | X8-1mg/kg n=12 | |
|---|---|---|---|---|---|---|---|---|---|---|
| | Mea n/g | Varia nce | Mean/ g | Varian ce | Mean/ g | Varianc e | Mea n/g | Varia nce | Mea n/g | Varia nce |
| 0 | 22.2 1 | 0.85 | 22.00 | 1.82 | 21.92 | 1.28 | 21.9 4 | 1.31 | 21.5 0 | 1.55 |
| 4 | 23.0 9 | 1.04 | 22.34 | 1.52 | 22.30 | 1.38 | 22.4 1 | 1.56 | 21.5 2 | 1.16 |
| 7 | 23.2 1 | 0.84 | 22.22 | 1.70 | 22.91 | 1.49 | 22.7 6 | 1.45 | 21.9 3 | 1.21 |
| 11 | 23.5 5 | 0.83 | 22.51 | 1.79 | 23.33 | 1.39 | 22.7 9 | 1.53 | 21.9 0 | 1.61 |
| 14 | 23.1 3 | 1.00 | 22.51 | 1.74 | 21.72 | 1.76 | 22.5 5 | 1.43 | 21.9 2 | 1.41 |
| 18 | 23.9 1 | 0.84 | 23.00 | 2.01 | 23.92 | 1.26 | 22.8 7 | 1.25 | 22.2 3 | 1.37 |
| 20 | 24.4 0 | 1.10 | 23.32 | 1.75 | 24.09 | 1.23 | 23.2 0 | 1.24 | 22.5 0 | 1.41 |
| 25 | 24.7 6 | 1.22 | 23.14 | 1.94 | 23.64 | 1.71 | 22.4 8 | 1.71 | 22.0 7 | 1.19 |
| 28 | 24.7 9 | 1.21 | 22.57 | 1.92 | 23.48 | 1.68 | 22.4 6 | 1.72 | 21.8 2 | 1.50 |
| 31 | 24.5 8 | 0.80 | 22.31 | 1.98 | 23.12 | 1.80 | 22.3 7 | 1.71 | 21.7 1 | 1.54 |
| 33 | 24.9 6 | 1.28 | 22.20 | 2.07 | 23.00 | 2.07 | 22.6 5 | 1.81 | 21.8 2 | 1.47 |
| 35 | 25.0 5 | 1.14 | 21.72 | 2.03 | 22.08 | 2.28 | 22.4 3 | 1.73 | 21.5 5 | 1.43 |
| 38 | 25.2 4 | 1.10 | 21.27 | 2.00 | 22.86 | 2.04 | 22.3 0 | 1.76 | 21.7 1 | 1.22 |
| 40 | 25.1 2 | 1.25 | 21.40 | 1.89 | 22.70 | 1.93 | 20.9 0 | 2.63 | 21.6 4 | 1.19 |
| 42 | 25.5 1 | 1.20 | 21.21 | 1.93 | 22.45 | 1.86 | 22.1 3 | 1.80 | 21.6 1 | 1.20 |
| 45 | 25.5 1 | 1.24 | 20.98 | 1.72 | 22.30 | 1.62 | 22.3 0 | 1.80 | 21.9 8 | 1.50 |

As shown in Table 4, X8 treatment had no significant effect on body weight of animals, and no drastic changes were seen. However, there were two sudden drops in the methotrexate group. There was a weight loss in the late stage of the experiment in tofacitinib group.

**Table 5 Evaluation data of the therapeutic effect of X8 on collagen-induced mouse arthritis model (the larger the value, the more severe the disease)**

| Time/ d ay | Healthy Control n=12 | | Vehicle Treatment n=10 | | MTX-0.25mg/kg n=10 | | Tofacitinib-5mg/kg n=12 | | X8-1mg/kg n=12 | |
|---|---|---|---|---|---|---|---|---|---|---|
| | Me an | Varian ce | Mean | Variance | Me an | Varian ce | Mea n | Varianc e | Me an | Varian ce |
| 0 | 0.0 0 | 0.00 | 0.00 | 0.00 | 0.00 | 0.00 | 0.00 | 0.00 | 0.0 0 | 0.00 |
| 21 | 0.0 0 | 0.00 | 0.00 | 0.00 | 0.00 | 0.00 | 0.00 | 0.00 | 0.0 0 | 0.00 |
| 25 | 0.0 0 | 0.00 | 0.36 | 0.76 | 0.00 | 0.00 | 0.00 | 0.00 | 0.0 0 | 0.00 |
| 28 | 0.0 0 | 0.00 | 1.20 | 1.67 | 0.40 | 0.97 | 0.81 | 1.01 | 0.9 8 | 1.50 |
| 31 | 0.0 0 | 0.00 | 1.80 | 1.93 | 1.32 | 1.59 | 1.83 | 1.51 | 1.6 6 | 1.94 |
| 33 | 0.0 0 | 0.00 | 2.81 | 2.39 | 1.55 | 1.61 | 2.19 | 1.67 | 2.3 9 | 2.05 |
| 35 | 0.0 0 | 0.00 | 3.13 | 2.27 | 2.05 | 1.67 | 2.52 | 1.95 | 2.6 4 | 2.14 |
| 38 | 0.0 0 | 0.00 | 4.67 | 1.54 | 2.41 | 1.63 | 2.77 | 2.11 | 2.8 7 | 1.88 |
| 40 | 0.0 0 | 0.00 | 5.30 | 1.32 | 2.60 | 1.99 | 2.62 | 1.94 | 2.8 7 | 1.76 |
| 42 | 0.0 0 | 0.00 | 6.11 | 2.06 | 3.66 | 2.24 | 3.14 | 1.93 | 3.1 7 | 1.46 |
| 45 | 0.0 0 | 0.00 | 6.07 | 2.57 | 4.61 | 2.24 | 2.90 | 1.73 | 3.3 9 | 1.58 |

As shown in Table 5, the X8 treatment group showed comparable effects to the tofacitinib treatment group, which significantly reduced the severity of the disease, and the dose was only 1/5 of tofacitinib.

### Example of Effect 3: HTRF experiment

### Experimental Materials:

Multifunctional microplate reader (TECAN; INFINITE F PLEX);
96-well cell culture plates (Bioyong), HTRF 384 microplate (HTRF 384 well low volume plates)(Cisbio; Cat#66PL384025);
Human Interferon β (IFN-β) (GenScript,; Cat#P01574), STAT5 Y694 phosphorylation detection kit (STAT5 phospho-Y694 kit) (Cisbio; Cat#64AT5PEG).

### Experimental Process:

Hela cells (ATCC American Standard Culture Center, CCL-2) were cultured in a 96-well plate at a density of 3×104 cells/well with DMEM medium containing 5 % fetal bovine serum and maintained at 37°C in a humidified incubator with 5% carbon dioxide overnight. On the second day, the original DMEM medium in the 96-well plate was sucked dry, and an equal volume of medium containing different concentrations of compounds (the mother liquor of the compound prepared by dimethyl sulfoxide was diluted in the medium at the required concentration) was added for pretreatment for 2 hours, and then it was placed in an incubator at 37°C. Then human interferon beta with a final concentration of 10 nM was added to stimulate, and it was gently shaken to mix well and then placed in an incubator at 37°C for 30 minutes.

The medium was removed, an appropriate volume of PBS was added to wash, and then it was sucked dry. Then 40 microliters of 1x Lysis Buffer was added to each well and it was stood for 30 minutes at room temperature (1ml 1x lysate preparation: 740 µl ddH₂O was added to 250 µl 4x lysate and 10 µl Blocking reagent and it was mixed thoroughly). After 30 minutes, the cells were repeatedly blown until the lysis solution was not viscous (avoiding blowing out bubbles) to achieve sufficient lysis.

4 microliters of a premixed solution of antibody (a premixed solution of antibody contained antibodies of Cryptate and d2, these two antibodies were diluted 20 times with detection buffer and mixed in equal volumes to prepare a premixed solution of antibody) and 16 microliters of cell lysate were added to each well of the HTRF 384 microtiter plate, and it was sealed with parafilm and incubated overnight at room temperature in the dark.

After overnight incubation, the signals of 665 nm and 620 nm were detected in a multifunctional microplate reader. The calculation formula for the detection of STAT5 Y694 phosphorylation is: Ratio =[(signal 665nm)/ (signal 620nm)]x10⁴. Treatment with dimethyl sulfoxide alone was the baseline control (Ratio_{base}), and the human interferon β stimulation without addition of the compound was the STAT5 Y694 full phosphorylation activation control Ratioₛₜᵢₘᵤₗₐₜᵢₒₙ), the inhibition rate of STAT5 Y694 phosphorylation of different samples was normalized: Inhibition rate = [1-(Ratio_{Experiment} - Ratio_{base})/(Ratioₛₜᵢₘᵤᵢₐₜᵢₒₙ - Ratio_{base})]* 100.

**Table 6 Quantitative data of inhibitory activity on cell biochemical level**

| **Compound Number** | **Example of Effect 1: Inhibiting Ability*^{a}* (Protein Electrophoresis and Blotting)** | **Example of Effect 3: Inhibiting Ratio@10 µM (HTRF Experiment)** |
|---|---|---|
| N1⁶ | + | |
| P1 | - | |
| P3 | - | |
| P4*^{b}* | X | |
| P8 | - | |
| P9 | - | |
| X2 | - | |
| X3 | - | |
| X4*^{b}* | - | |
| X6 | - | |
| X7 | - | |
| X8 | + | 22.60% |
| X9 | + | |
| GEN1-001 | + | 3.75% |
| GEN1-003 | + | |
| GEN1-005 | + | |
| GEN1-006 | + | |
| GEN1-007 | + | |
| GEN1-009 | + | |
| GEN1-010 | + | |
| GEN1-011 | + | 20.49% |
| GEN1-012 | + | |
| GEN1-013 | + | 20.29% |
| GEN1-014 | + | 16.12% |
| GEN1-015 | + | |
| GEN1-016 | + | 9.50% |
| GEN1-017 | - | |
| GEN1-018 | + | |
| GEN1-021 | + | |
| GEN1-022 | - | |
| GEN1-023 | + | |
| GEN1-024 | - | |
| GEN1-025 | - | |
| GEN1-027 | + | |
| GEN1-028 | - | |
| GEN1-029 | + | |
| GEN1-030 | - | |
| GEN1-031 | + | 0.13% |
| GEN 1-032 | + | |
| GEN1-033 | + | |
| GEN1-034 | - | |
| GEN1-041 | + | |
| GEN 1-042 | + | |
| GEN1-043 | - | |
| GEN1-044 | + | |
| GEN1-049 | - | 18.33% |
| GEN1-050 | + | 15.28% |
| GEN1-051 | + | |
| GEN1-052 | + | 8.83% |
| GEN1-054 | - | |
| GEN1-055 | + | 16.88% |
| GEN1-056 | | 13.93% |
| GEN1-057 | ++ | 66.22% |
| GEN1-059 | - | |
| GEN1-061 | + | |
| GEN 1-062 | | 5.12% |
| GEN1-065 | - | |
| GEN1-068 | - | |
| GEN 1-070*^{b}* | - | |
| GEN1-071 | - | |
| GEN1-072 | X | |
| GEN1-073 | + | |
| GEN1-075 | + | 10.79% |
| GEN1-077 | | 34.25% |
| GEN1-078 | + | |
| GEN1-083 | - | |
| GEN1-084 | + | |
| GEN1-085 | - | |
| GEN1-086 | - | |
| GEN1-088 | + | |
| GEN1-089 | + | 7.83% |
| GEN1-090 | + | |
| GEN1-091 | + | 17.60% |
| GEN1-094 | + | |
| GEN1-095 | - | |
| GEN1-096 | + | |
| GEN1-097 | + | |
| GEN1-098 | + | |
| GEN1-101 | + | |
| GEN1-102 | + | |
| GEN1-107 | + | 37.09% |
| GEN1-108 | + | 38.81% |
| GEN1-109 | X | |
| GEN1-110 | X | -10.49% |
| GEN1-115 | + | |
| GEN1-116 | + | 35.01% |
| GEN1-117 | ++ | 89.31% |
| GEN1-118 | + | 27.57% |
| GEN1-120 | - | |
| GEN1-122 | + | |
| GEN1-127 | + | |
| GEN1-128 | + | 13.88% |
| GEN1-133 | | 31.27% |
| GEN1-134 | - | 3.06% |
| GEN1-135 | | 22.91% |
| GEN1-137 | + | 19.51% |
| GEN1-139 | | 18.43% |
| GEN1-140 | + | 53.79% |
| GEN1-146 | + | |
| GEN1-149 | + | 0.49% |
| GEN1-150 | - | |
| GEN1-151 | + | 39.02% |
| GEN1-154 | - | 6.10% |
| GEN1-156 | + | 47.04% |
| GEN1-157 | + | 28.83% |
| GEN1-158 | - | 0.16% |
| GEN1-159 | + | 22.99% |
| GEN1-160 | + | 10.27% |
| GEN1-161 | + | 1.38% |
| GEN1-162 | + | 22.04% |
| GEN1-163 | - | |
| GEN1-164 | - | |
| GEN1-166 | + | 15.16% |
| GEN1-167 | + | 15.57% |
| GEN1-168 | + | 47.57% |
| GENI-169^{h} | + | 13.47% |
| GEN1-171 | - | 9.85% |
| GEN1-172 | - | 13.83% |
| GEN1-173 | + | 50.75% |
| GEN1-174 | + | 39.65% |
| GEN1-178 | | 8.67% |
| GEN1-179 | | 10.11% |
| GEN1-243 | + | |
| GEN1-265 | | 16.47% |
| GEN1-268 | | 58.21% |
| GEN1-270 | | 21.10% |
| GEN1-271 | | 9.33% |
| GEN1-276 | | 55.48% |
| GEN1-277 | | 6.52% |
| GEN1-278 | | 73.74% |
| GEN1-280 | | 20.53% |
| GEN1-281 | | 50.87% |
| GEN1-282 | | 49.93% |
| GEN1-287 | | 8.72% |
| GEN1-289 | | 6.86% |
| GEN1-290 | | 57.51% |
| GEN1-291 | | 70.98% |
| GEN1-292 | | 59.63% |
| GEN1-294 | | 1.85% |

^{a} Description of the inhibiting ability of the compound: ++, the band of pSTAT5 disappeared completely at 10 µM; +, the band of pSTAT5 weakened at 10 µM, the band of pSTAT5 disappeared completely at 100 µM; -, the band of pSTAT5 weakened at 100 µM; the band of pSTAT5 did not weaken at X, 100 µM.

### ^{b} Control compound.

Although the specific embodiments of the present invention are described above, a skilled person in the art should understand that these are only exemplary descriptions, and various changes or modifications can be made to these embodiments without departing from the principle and essence of the present invention. Therefore, the protection scope of the present invention is defined by the appended claims.

## Claims

1. A compound represented by formula **XI,** 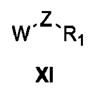 or a pharmaceutically acceptable salt, a solvate, or a solvate of a pharmaceutically acceptable salt thereof;
wherein, W is anyone of the following groups:
(1) 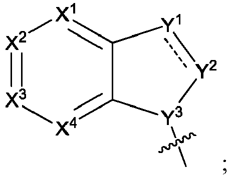
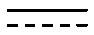 in 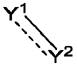 is a single bond or a double bond;
when 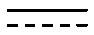 in 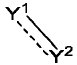 is a single bond, Y¹ is C(=O), O, S or N-R³, R³ is hydrogen, hydroxyl, CH₂COOH or C₁-C₄ alkyl, Y² is C(=N-CO-Ph), C(=N-CO-CH₃), NH, S(=O), S(=O)₂, C=O or C=S;
when 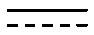 in 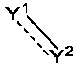 is a double bond, Y¹ is N, Y² is C-NH₂, C-NH-CN or C-O-CH₃;
Y³ is N or CH;
X¹ is N or CR², X² is N or CR⁴, X³ is N or CR⁵, X⁴ is N or CR⁶;
R², R⁴, R⁵ and R⁶ are independently hydrogen, halogen, hydroxyl, amino, carboxyl, cyano, nitro, C₁-C₄ alkyl, C₁-C₄ alkyl substituted with one or more halogens, C₂-C₄ alkenyl, C₂-C₄ alkynyl, C₁-C₄ alkoxy, C₃-C₆ cycloalkyl, phenyl, 5-7 membered heterocycloalkyl containing 1, 2, or 3 atoms independently selected from N, O and S, -S(=O)₂-R²⁻¹, -NH-S(=O)₂-R²⁻², -S(=O)₂-NR²⁻³R²⁻⁴, -C(=O)-R²⁻⁵, -NH-C(=O-R²⁻⁶, -C(=O)-NR²⁻⁷R²⁻⁸, -O(C=O-R²⁻⁹, or -C(=O)-O-R²⁻¹⁰;
R²⁻¹, R²⁻², R²⁻³, R²⁻⁴, R²⁻⁵, R²⁻⁶, R²⁻⁷, R²⁻⁸, R²⁻⁹ and R²⁻¹⁰ are independently hydrogen, C₁-C₄ alkyl, C₂-C₄ alkenyl, C₂-C₄ alkynyl, or C₁-C₄ alkyl substituted with one or more halogens;
(2) 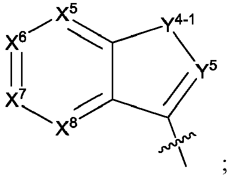
Y⁴⁻¹ is -NH-C(=O)-CH₂- or NH;
Y⁵ is N or C-R⁷, R⁷ is halogen;
X⁵ is N or CR⁸, X⁶ is N or CR⁹, X⁷ is N or CR¹⁰, X⁸ is N or CR¹¹;
R⁸, R⁹, R¹⁰ and R¹¹ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(3) 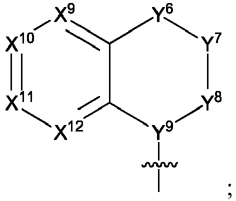
Y⁶ is N-C(=O)-CH₃, CH₂ or NH;
Y⁷ is NH, CH₂, C(=O), C(=S) or C(=N-OH);
Y⁸ is CH₂, CH₂CH₂, CH(CH₃) or C(=O);
Y⁹ is N or CH;
X⁹ is N or CR¹², X¹⁰ is N or CR¹³, X¹¹ is N or CR¹⁴, X¹² is N or CR¹⁵;
R¹², R¹³, R¹⁴ and R¹⁵ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(4) 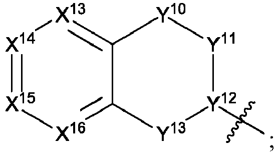
Y¹⁰ is NH;
Y¹¹ is C(=O);
Y¹² is N or CH;
Y¹³ is CH₂;
X¹³ is N or CR¹⁶, X¹⁴ is N or CR¹⁷, X¹⁵ is N or CR¹⁸, X¹⁶ is N or CR¹⁹;
R¹⁶, R¹⁷, R¹⁸ and R¹⁹ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(5) 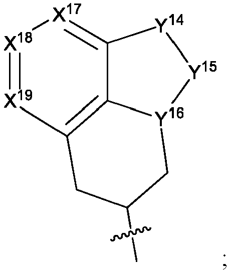
Y¹⁴ is NH;
Y¹⁵ is C(=O);
Y¹⁶ is N or CH;
X¹⁷ is N or CR²⁰, X¹⁸ is N or CR²¹, X¹⁹ is N or CR²²;
R²⁰, R²¹ and R²² are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(6) 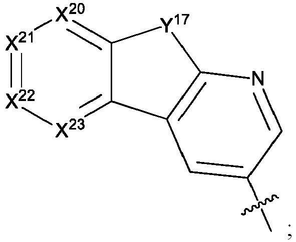
Y¹⁷ is NH;
X²⁰ is N or CR²³, X²¹ is N or CR²⁴, X²² is N or CR²⁵, X²³ is N or CR²⁶;
R²³, R²⁴, R²⁵ and R²⁶ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(7) 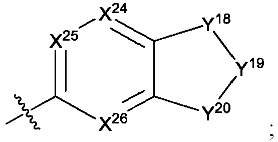
Y¹⁸ is NH or CH₂;
Y¹⁹ is C(=O) or NH;
Y²⁰ is CH₂ or C(=O);
X²⁴ is N or CR²⁷, X²⁵ is N or CR²⁸, X²⁶ is N or CR²⁹;
R²⁷, R²⁸ and R²⁹ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(8) 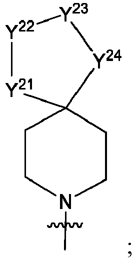
Y²¹ is C(=O);
Y²² is NH;
Y²³ is CH₂ or C(=O);
Y²⁴ is N-R³⁰, R³⁰ is hydrogen or phenyl;
(9) 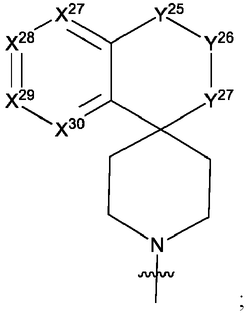
Y²⁵ is NH;
Y²⁶ is C(=O);
Y²⁷ is a single bond, O or NH;
X²⁷ is N or CR³¹, X²⁸ is N or CR³², X²⁹ is N or CR³³, X³⁰ is N or CR³⁴;
R³¹, R³², R³³ and R³⁴ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(10) 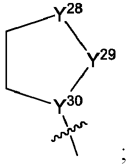
Y²⁸ is NH;
Y²⁹ is C(=O);
Y³⁰ is CH or N;
(11) 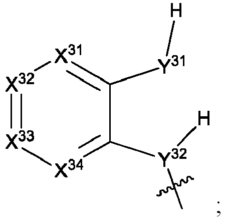
Y³¹ is O, NH or N-C(=O)-CH₃;
Y³² is CH or N;
X³¹ is N or CR³⁵, X³² is N or CR³⁶, X³³ is N or CR³⁷, X³⁴ is N or CR³⁸;
R³⁵, R³⁶, R³⁷ and R³⁸ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(12) 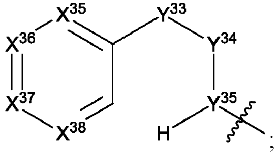
Y³³ is NH;
Y³⁴ is C(=O);
Y³⁵ is CH or N;
X³⁵ is N or CR³⁹, X³⁶ is N or CR⁴⁰, X³⁷ is N or CR⁴¹, X³⁸ is N or CR⁴²;
R³⁹, R⁴⁰, R⁴¹ and R⁴² are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(13) 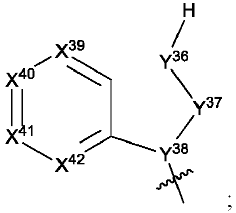
Y³⁶ is NH;
Y³⁷ is C(=O);
Y³⁸ is CH or N;
X³⁹ is N or CR⁴³, X⁴⁰ is N or CR⁴⁴, X⁴¹ is N or CR⁴⁵, X⁴² is N or CR⁴⁶;
R⁴³, R⁴⁴, R⁴⁵ and R⁴⁶ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂;
(14) 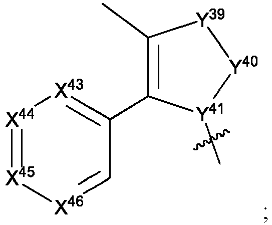
Y³⁹ is NH;
Y⁴⁰ is C(=O);
Y⁴¹ is CH or N;
X⁴³ is N or CR⁴⁷, X⁴⁴ is N or CR⁴⁸, X⁴⁵ is N or CR⁴⁹, X⁴⁶ is N or CR⁵⁰;
R⁴⁷, R⁴⁸, R⁴⁹ and R⁵⁰ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NH₂, or -C(=O)-NH₂; and
(15) 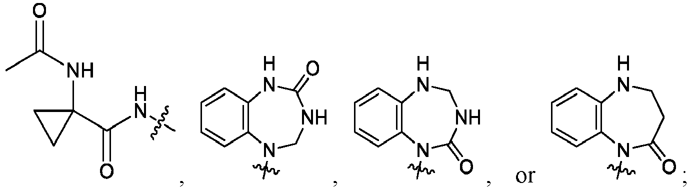
Z is anyone of the following fragments:
(1) 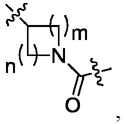 m is 1 or 2, n is 1, 2 or 3;
(2) 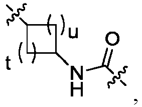 u is 1 or 2, t is 1 or 2;
(3) 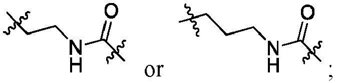
(4) 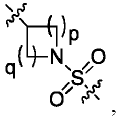 p is 1 or 2, q is 1 or 2;
(5) 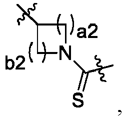 a2 is 1 or 2, b2 is 1 or 2;
(6) 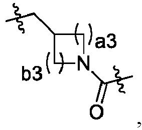 a3 is 1 or 2, b3 is 1 or 2;
(7) 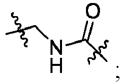
(8) -C(=O)-;
(9) 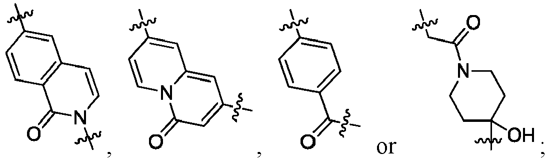
R¹ is C₂-C₄ alkyl, C₂-C₄ alkyl substituted with one or more halogens, -(CH₂)ₛ-R¹⁻¹, -NH-R¹⁻ 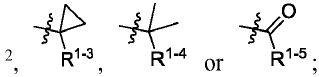 s is 0, 1 or 2;
R¹⁻¹, R¹⁻², R¹⁻³, R¹⁻⁴ and R¹⁻⁵ are independently C₇-C₁₀ cycloalkyl, one or more R¹⁻¹⁻³ substituted C₃-C₆ cycloalkyl, one or more R¹⁻¹⁻⁶ substituted 5-6 membered heterocycloalkyl containing 1, 2, or 3 atoms independently selected from N, O and S, one or more R¹⁻¹⁻¹ substituted C₆-C₁₀ aryl, C₃-C₆ cycloalkyl, cyclopentadienyl, one or more R¹⁻¹⁻⁴ substituted cyclopentadienyl, naphthyl, 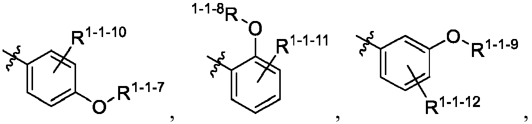 5, 10 membered heteroaryl containing 1, 2, or 3 atoms independently selected from N, O and S, pyridyl, 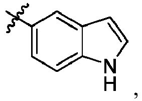 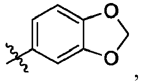 or one or more R¹⁻¹⁻² substituted 5-10 membered heteroaryl containing 1, 2, or 3 atoms independently selected from N, O and S;
R¹⁻¹⁻¹, R¹⁻¹⁻², R¹⁻¹⁻³, R¹⁻¹⁻⁴, and R¹⁻¹⁻⁶ are independently carboxy, hydroxy, cyano, halogen, C₁-C₄ alkyl, C₁-C₄ alkyl substituted with one or more halogens, C₁-C₄ alkoxy substituted with one or more halogens, C₃-C₆ cycloalkyl, 5-6 membered heterocycloalkyl containing 1, 2, or 3 atoms independently selected from N, O and S, -N(R¹⁻¹⁻¹⁻¹)(R¹⁻¹⁻¹⁻²), or phenyl;
R¹⁻¹⁻¹⁻¹ and R¹⁻¹⁻¹⁻² are independently hydrogen or C₁-C₄ alkyl;
R¹⁻¹⁻⁷, R¹⁻¹⁻⁸, and R¹⁻¹⁻⁹ are independently C₁-C₄ alkyl;
R¹⁻¹⁻¹ is independently C₁-C₄ alkoxy;
R¹⁻¹⁻¹¹ and R¹⁻¹⁻¹² are independently hydrogen or halogen.

2. The compound represented by formula **XI** of claim 1, or a pharmaceutically acceptable salt, a solvate, or a solvate of a pharmaceutically acceptable salt thereof, wherein the compound represented by formula **XI** is a compound represented by formula **I**; 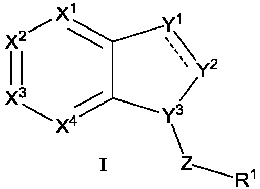
wherein, 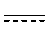 in 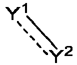 is a single bond or a double bond;
when 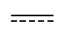 in 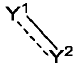 is a single bond, Y¹ is O, S or N-R³, R³ is hydrogen, hydroxyl, CH₂COOH or C₁-C₄ alkyl, Y² is C=O, C=S, S=O or S(=O)₂;
when 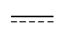 in 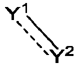 is a double bond, Y¹ is N, Y² is C-NH₂, C-NH-CN or C-O-CH₃;
Y³ is N or CH;
X¹ is N or CR², X² is N or CR⁴, X³ is N or CR⁵, X⁴ is N or CR⁶;
R², R⁴, R⁵ and R⁶ are independently hydrogen, halogen, hydroxyl, amino, carboxyl, cyano, nitro, C₁-C₄ alkyl, C₁-C₄ alkyl substituted with one or more halogens, C₂-C₄ alkenyl, C₂-C₄ alkynyl, C₁-C₄ alkoxy, C₃-C₆ cycloalkyl, phenyl, -S(=O)₂-R²⁻¹, -NH-S(=O)₂-R²⁻², -S(=O)₂-NR²⁻³R²⁻⁴, -C(=O)-R²⁻⁵, -NH-C(=O-R²⁻⁶, -C(=O)-NR²⁻⁷R²⁻⁸, -O-(C=O)-R²⁻⁹, or -C(=O)-O-R²⁻¹⁰;
R²⁻¹, R²⁻², R^{2-3,} R²⁻⁴, R²⁻⁵, R²⁻⁶, R²⁻⁷, R²⁻⁸, R²⁻⁹ and R²⁻¹⁰ are independently C₁-C₄ alkyl, C₁-C₄ alkyl substituted with one or more halogens, C₂-C₄ alkenyl, or C₂-C₄ alkynyl;
Z is 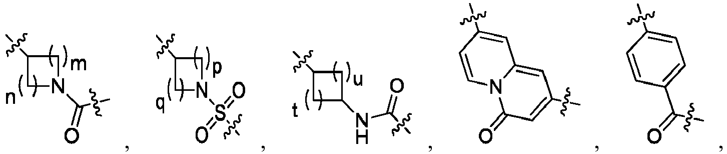 or 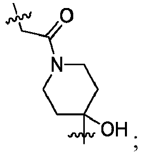 m is 1 or 2, n is 1 or 2; p is 1 or 2, q is 1 or 2; u is 1 or 2, t is 1 or 2;
R¹ is C₂-C₄ alkyl, C₂-C₄ alkyl substituted with one or more halogens, -(CH₂)ₛ-R¹⁻¹, or -NH-R¹⁻²; s is 0, 1 or 2;
R¹⁻¹ and R¹⁻² are independently C₆-C₁₀ aryl, one or more R¹⁻¹⁻¹ substituted C₆-C₁₀ aryl, 5-10 membered heteroaryl containing 1, 2, or 3 atoms independently selected from N, O and S, one or more R¹⁻¹⁻² substituted 5-10 membered heteroaryl containing 1, 2, or 3 atoms independently selected from N, O and S, C₃-C₆ cycloalkyl, one or more R¹⁻¹⁻³ substituted C₃-C₆ cycloalkyl, cyclopentadienyl, or one or more R¹⁻¹⁻⁴ substituted cyclopentadienyl;
R¹⁻¹⁻¹, R¹⁻¹⁻², R¹⁻¹⁻³ and R¹⁻¹⁻⁴ are independently hydroxyl, cyano, halogen, carboxyl, C₁-C₄ alkyl, C₁-C₄ alkyl substituted with one or more halogens, C₁-C₄ alkoxy, C₃-C₆ cycloalkyl, 5-6 membered heterocycloalkyl containing 1, 2, or 3 atoms independently selected from N, O and S, or phenyl.

3. The compound represented by formula **XI** of claim 2, or a pharmaceutically acceptable salt, a solvate, or a solvate of a pharmaceutically acceptable salt thereof, wherein 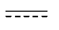 in 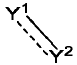 is a single bond or a double bond; when 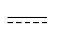 in 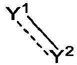 is a single bond, Y¹ is NH or N-OH, Y² is C=O; when 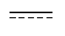 in 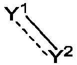 is a double bond, Y¹ is N, Y² is C-NH₂, C-NH-CN or C-O-CH₃;
Y³ is N;
X¹ is CR², X² is CR⁴, X³ is CR⁵, X⁴ is CR⁶;
R², R⁴, R⁵ and R⁶ are independently hydrogen, halogen, hydroxy, amino, carboxy, cyano, nitro, C₁-C₄ alkyl, C₁-C₄ alkyl substituted with one or more halogens, C₂-C₄ alkenyl, C₂-C₄ alkynyl, C₁-C₄ alkoxy, C₃-C₆ cycloalkyl, phenyl, -S(=O)₂-R²⁻¹, -NH-S(=O)₂-R²⁻², -S(=O)₂-NR²⁻³R²⁻⁴, -C(=O)-R²⁻⁵, -NH-C(=O-R²⁻⁶, or -C(=O)-NR²⁻⁷R²⁻⁸;
R²⁻¹, R²⁻², R²⁻³, R²⁻⁴, R²⁻⁵, R²⁻⁶, R²⁻⁷ and R²⁻⁸ are independently C₁-C₄ alkyl, or C₁-C₄ alkyl substituted with one or more halogens;
Z is 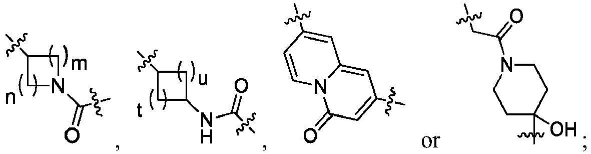 m is 1 or 2, n is 1 or 2; u is 1 or 2, t is 1 or 2;
R¹ is -(CH₂)ₛ-R¹⁻¹; s is 0, 1 or 2;
R¹⁻¹ is a C₆-C₁₀ aryl, one or more R¹⁻¹⁻¹ substituted C₆-C₁₀ aryl, 5-10 membered heteroaryl containing 1, 2, or 3 atoms independently selected from N, O and S, or one or more R¹⁻¹⁻² substituted 5-10 membered heteroaryl containing 1, 2, or 3 atoms independently selected from N, O and S;
R¹⁻¹⁻¹ and R¹⁻¹⁻² are independently C₁-C₄ alkyl, C₁-C₄ alkyl substituted with one or more halogens, C₃-C₆ cycloalkyl, or phenyl.

4. The compound represented by formula **XI** of claim 2, or a pharmaceutically acceptable salt, a solvate, or a solvate of a pharmaceutically acceptable salt thereof, wherein 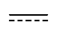 in 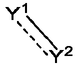 is a single bond or a double bond; when 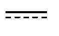 in 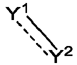 is a single bond, Y¹ is NH or N-OH, Y² is C=O; when 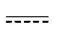 in 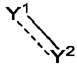 is a double bond, Y¹ is N, Y² is C-NH₂, C-NH-CN or C-O-CH₃;
and/or Z is 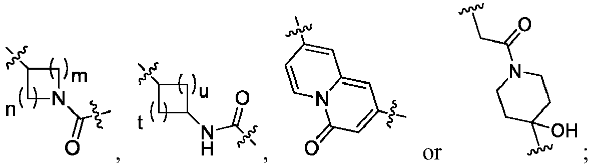 m is 1 or 2, n is 1 or 2; u is 1 or 2, t is 1 or 2;
and/or R¹⁻¹ and R¹⁻² are independently naphthyl, one or more R¹⁻¹⁻¹ substituted C₆-C₁₀ aryl, 10 membered heteroaryl containing 1, 2, or 3 atoms independently selected from N, O and S, one or more R¹⁻¹⁻² substituted 5-10 membered heteroaryl containing 1, 2, or 3 atoms independently selected from N, O and S, C₃-C₆ cycloalkyl, one or more R¹⁻¹⁻³ substituted C₃-C₆ cycloalkyl, cyclopentadienyl, or one or more R¹⁻¹⁻⁴ substituted cyclopentadienyl; R¹⁻¹⁻¹, R¹⁻¹⁻², R¹⁻¹⁻³ and R¹⁻¹⁻⁴ are independently hydroxy, cyano, halogen, carboxy, C₁-C₄ alkyl, C₁-C₄ alkyl substituted with one or more halogens, C₃-C₆ cycloalkyl, 5-6 membered heterocycloalkyl containing 1, 2, or 3 atoms independently selected from N, O and S, or phenyl.

5. The compound represented by formula **XI** of claim 1, or a pharmaceutically acceptable salt, a solvate, or a solvate of a pharmaceutically acceptable salt thereof, wherein the compound represented by formula **XI** is a compound represented by formula **III**; 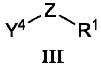
wherein, Y⁴ is 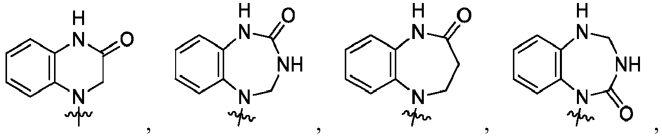 or 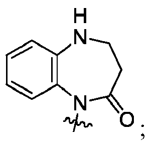
Z is 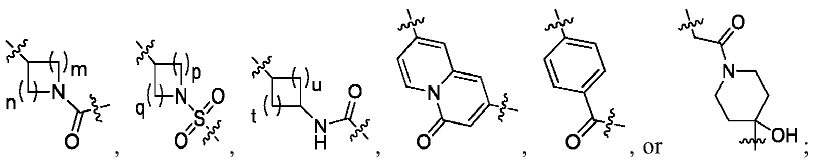 m is 1 or 2, n is 1 or 2; p is 1 or 2, q is 1 or 2; u is 1 or 2, t is 1 or 2;
R¹ is C₂-C₄ alkyl, C₂-C₄ alkyl substituted with one or more halogens, -(CH₂)ₛ-R¹⁻¹, or -NH-R¹⁻²; s is 0, 1 or 2;
R¹⁻¹ and R¹⁻² are independently C₆-C₁₀ aryl, one or more R¹⁻¹⁻¹ substituted C₆-C₁₀ aryl, 5-10 membered heteroaryl containing 1, 2, or 3 atoms independently selected from N, O and S, one or more R¹⁻¹⁻² substituted 5-10 membered heteroaryl containing 1, 2, or 3 atoms independently selected from N, O and S, C₃-C₆ cycloalkyl, one or more R¹⁻¹⁻³ substituted C₃-C₆ cycloalkyl, cyclopentadienyl, or one or more R¹⁻¹⁻⁴ substituted cyclopentadienyl;
R¹⁻¹⁻¹, R¹⁻¹⁻², R¹⁻¹⁻³ and R¹⁻¹⁻⁴ are independently hydroxyl, cyano, halogen, carboxyl, C₁-C₄ alkyl, C₁-C₄ alkyl substituted with one or more halogens, C₁-C₄ alkoxy, C₃-C₆ cycloalkyl, 5-6 membered heterocycloalkyl containing 1, 2, or 3 atoms independently selected from N, O and S, or phenyl.

6. The compound represented by formula **XI** of claim 1, or a pharmaceutically acceptable salt, a solvate, or a solvate of a pharmaceutically acceptable salt thereof, wherein the compound represented by formula **XI** is a compound represented by formula **V**; 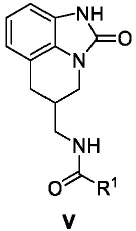
wherein, R¹ is C₂-C₄ alkyl,C₂-C₄ alkyl substituted with one or more halogens, -(CH₂)ₛ-R¹⁻¹, or -NH-R¹⁻²; s is 0, 1 or 2;
R¹⁻¹ and R¹⁻² are independently C₆-C₁₀ aryl, one or more R¹⁻¹⁻¹ substituted C₆-C₁₀ aryl, 5-10 membered heteroaryl containing 1, 2, or 3 atoms independently selected from N, O and S, one or more R¹⁻¹⁻² substituted 5-10 membered heteroaryl containing 1, 2, or 3 atoms independently selected from N, O and S, C₃-C₆ cycloalkyl, one or more R¹⁻¹⁻³ substituted C₃-C₆ cycloalkyl, cyclopentadienyl, or one or more R¹⁻¹⁻⁴ substituted cyclopentadienyl;
R¹⁻¹⁻¹, R¹⁻¹⁻², R¹⁻¹⁻³ and R¹⁻¹⁻⁴ are independently hydroxyl, cyano, halogen, carboxyl, C₁-C₄ alkyl, C₁-C₄ alkyl substituted with one or more halogens, C₁-C₄ alkoxy, C₃-C₆ cycloalkyl, 5-6 membered heterocycloalkyl containing 1, 2, or 3 atoms independently selected from N, O and S, or phenyl.

7. The compound represented by formula **XI** of claim 1, or a pharmaceutically acceptable salt, a solvate, or a solvate of a pharmaceutically acceptable salt thereof, wherein W is anyone of the following groups:
(1) 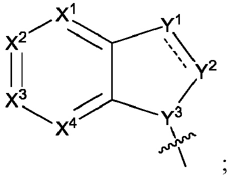 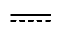
in 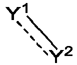 is a single bond or a double bond;
when 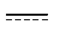 in 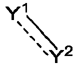 is a single bond, Y¹ is C(=O), O, or N-R³, R³ is hydrogen, hydroxyl, CH₂COOH or C₁-C₄ alkyl, Y² is C(=N-CO-Ph), C(=N-CO-CH₃), NH, S(=O)₂, C=O or C=S;
when 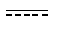 in 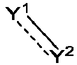 is a double bond, Y¹ is N, and Y² is C-NH₂ or C-NH-CN;
Y³ is N or CH;
X¹ is N or CR², X² is N or CR⁴, X³ is N or CR⁵, X⁴ is N or CR⁶;
R², R⁴, R⁵ and R⁶ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NR²⁻³R²⁻⁴, or -C(=O)-NR²⁻⁷R²⁻⁸;
R²⁻³, R²⁻⁴, R²⁻⁷ and R²⁻⁸ are independently hydrogen;
(2) 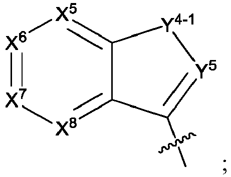
Y⁴⁻¹ is -NH-C(=O)-CH₂- or NH;
Y⁵ is N or C-R⁷, R⁷ is halogen;
X⁵ is CR⁸, X⁶ is CR⁹, X⁷ is CR¹⁰, X⁸ is CR¹¹;
R⁸, R⁹, R¹⁰ and R¹¹ are independently hydrosen;
(3) 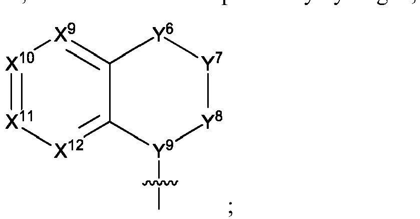
Y⁶ is N-C(=O)-CH₃, CH₂ or NH;
Y⁷ is NH, CH₂, C(=O), C(=S) or C(=N-OH);
Y⁸ is CH₂, CH₂CH₂, CH(CH₃) or C(=O);
Y⁹ is N;
X⁹ is CR¹², X¹⁰ is CR¹³, X¹¹ is CR¹⁴, X¹² is CR¹⁵;
R¹², R¹³, R¹⁴ and R¹⁵ are independently hydrogen, nitro, or C₁-C₄ alkyl substituted with one or more halogens;
(4) 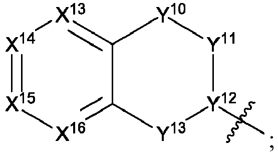
Y¹⁰ is NH;
Y¹¹ is C(=O);
Y¹² is N;
Y¹³ is CH₂;
X¹³ is CR¹⁶, X¹⁴ is CR¹⁷, X¹⁵ is CR¹⁸, X¹⁶ is CR¹⁹;
R¹⁶, R¹⁷, R¹⁸ and R¹⁹ are independently hydrogen;
(5) 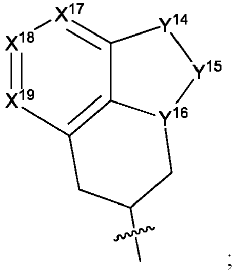
Y¹⁴ is NH;
Y¹⁵ is C(=O);
Y¹⁶ is N;
X¹⁷ is CR²⁰, X¹⁸ is CR²¹, X¹⁹ is CR²²;
R²⁰, R²¹ and R²² are independently hydrogen;
(6) 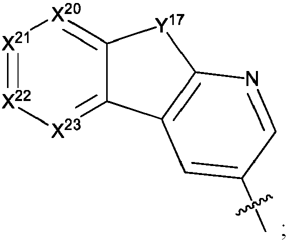
Y¹⁷ is NH;
X²⁰ is CR²³, X²¹ is CR²⁴, X²² is CR²⁵, X²³ is CR²⁶;
R²³, R²⁴, R²⁵ and R²⁶ are independently hydrogen;
(7) 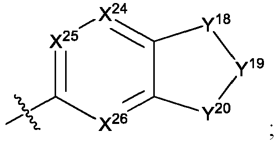
Y¹⁸ is NH or CH₂;
Y¹⁹ is C(=O) or NH;
Y²⁰ is CH₂ or C(=O);
X²⁴ is CR²⁷, X²⁵ is CR²⁸, X²⁶ is CR²⁹;
R²⁷, R²⁸ and R²⁹ are independently hydrogen;
(8) 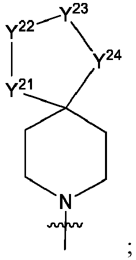
Y²¹ is C(=O);
Y²² is NH;
Y²³ is CH₂ or C(=O);
Y²⁴ is N-R³⁰, R³⁰ is hydrogen or phenyl;
(9) 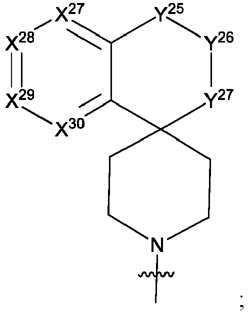
Y²⁵ is NH;
Y²⁶ is C(=O);
Y²⁷ is a single bond, O or NH;
X²⁷ is CR³¹, X²⁸ is CR³², X²⁹ is CR³³, X³⁰ is CR³⁴;
R³¹, R³², R³³ and R³⁴ are independently hydrogen;
(10) 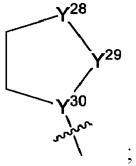
Y²⁸ is NH;
Y²⁹ is C(=O);
Y³⁰ is N;
(11) 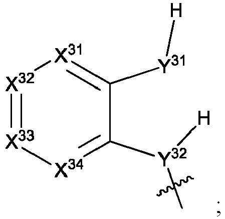
Y³¹ is O, NH or N-C(=O)-CH₃;
Y³² is N;
X³¹ is CR³⁵, X³² is CR³⁶, X³³ is CR³⁷, X³⁴ is CR³⁸;
R³⁵, R³⁶, R³⁷ and R³⁸ are independently hydrogen;
(12) 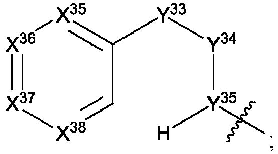
Y³³ is NH;
Y³⁴ is C(=O);
Y³⁵ is N;
X³⁵ is CR³⁹, X³⁶ is CR⁴⁰, X³⁷ is CR⁴¹, X³⁸ is CR⁴²;
R³⁹, R⁴⁰, R⁴¹ and R⁴² are independently hydrogen;
(13) 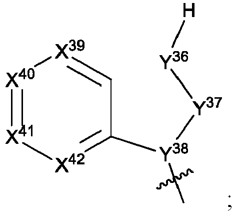
Y³⁶ is NH;
Y³⁷ is C(=O);
Y³⁸ is N;
X³⁹ is CR⁴³, X⁴⁰ is CR⁴⁴, X⁴¹ is CR⁴⁵, X⁴² is CR⁴⁶;
R⁴³, R⁴⁴, R⁴⁵ and R⁴⁶ are independently hydrogen;
(14) 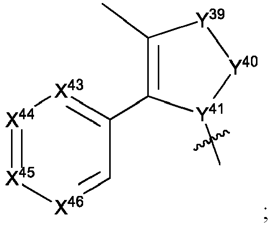
Y³⁹ is NH;
Y⁴⁰ is C(=O);
Y⁴¹ is N;
X⁴³ is CR⁴⁷, X⁴⁴ is CR⁴⁸, X⁴⁵ is CR⁴⁹, X⁴⁶ is CR⁵⁰;
R⁴⁷, R⁴⁸, R⁴⁹ and R⁵⁰ are independently hydrogen;
(15) 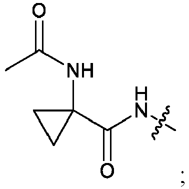
and/or R¹ is -(CH₂)ₛ-R¹⁻¹, -NH-R¹⁻², 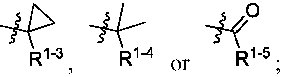 s is 0, 1 or 2;
R¹⁻¹, R¹⁻², R¹⁻³, R¹⁻⁴ and R¹⁻⁵ are independently one or more R¹⁻¹⁻³ substituted C₃-C₆ cycloalkyl, one or more R¹⁻¹⁻¹ substituted C₆-C₁₀ aryl, naphthyl, 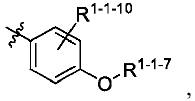 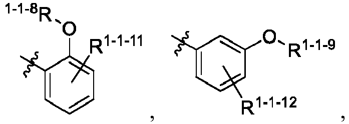 5, 10 membered heteroaryl containing 1, 2, or 3 atoms independently selected from N, O and S, pyridyl, 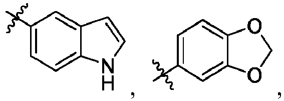 or one or more
R¹⁻¹⁻² substituted 5-10 membered heteroaryl containing 1, 2, or 3 atoms independently selected from N, O and S;
R¹⁻¹⁻¹, R¹⁻¹⁻², and R¹⁻¹⁻³ are independently carboxy, hydroxy, halogen, C₁-C₄ alkyl, C₁-C₄ alkyl substituted with one or more halogens, C₁-C₄ alkoxy substituted with one or more halogens, C₃-C₆ cycloalkyl, 5-6 membered heterocycloalkyl containing 1, 2, or 3 atoms independently selected from N, O and S, -N(R¹⁻¹⁻¹⁻¹)(R¹⁻¹⁻¹⁻²), or phenyl;
R¹⁻¹⁻¹⁻¹ and R¹⁻¹⁻¹⁻² are independently hydrogen or C₁-C₄ alkyl;
R¹⁻¹⁻⁷, R¹⁻¹⁻⁸, and R¹⁻¹⁻⁹ are independently C₁-C₄ alkyl;
R¹⁻¹⁻¹⁰ is independently C₁-C₄ alkoxy;
R¹⁻¹⁻¹¹ and R¹⁻¹⁻¹² are independently hydrogen or halogen.

8. The compound represented by formula **XI** of claim 1 or 7, or a pharmaceutically acceptable salt, a solvate, or a solvate of a pharmaceutically acceptable salt thereof, wherein when W is 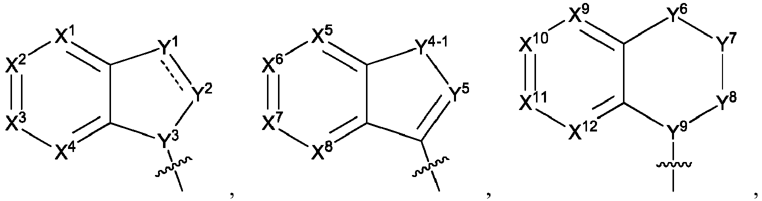 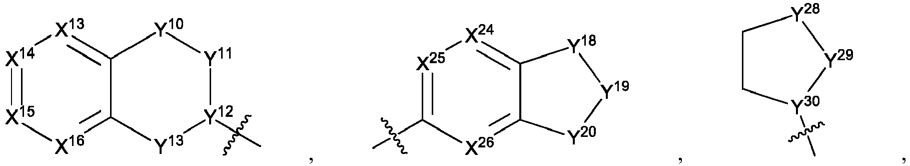 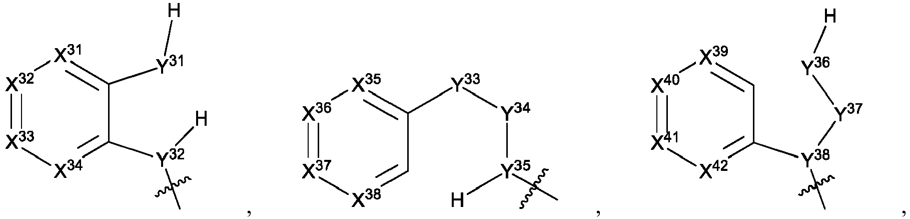 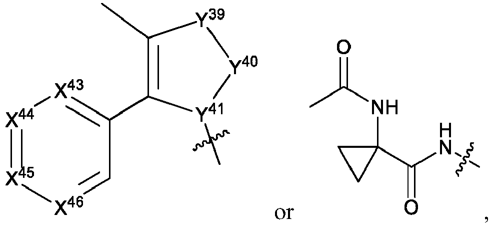 Z is anyone of the following fragments:
(1) 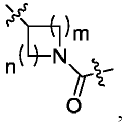 m is 1 or 2, n is 1, 2 or 3;
(2) 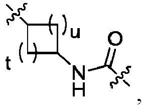 u is 1 or 2, t is 1 or 2;
(3) 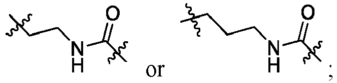
(4) 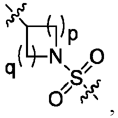 p is 1 or 2, q is 1 or 2;
(5) 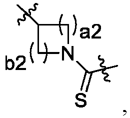 a2 is 1 or 2, b2 is 1 or 2;
(6) 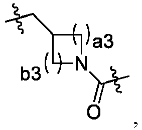 a3 is 1 or 2, b3 is 1 or 2;
(9) 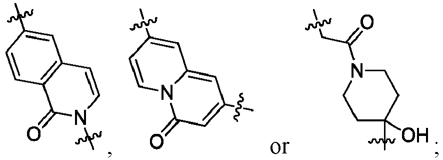 or when W is 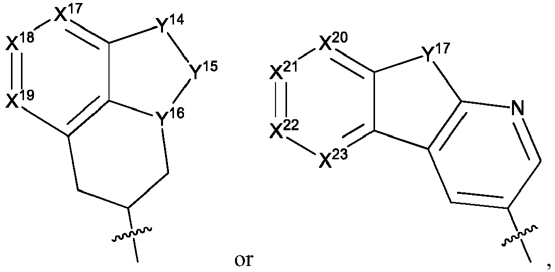 Z is 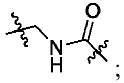 or when W is 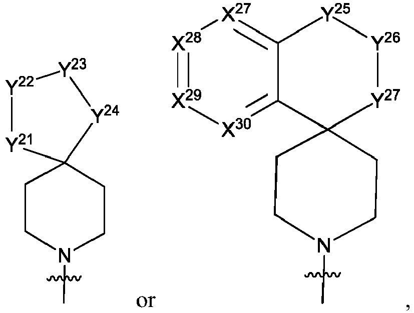 Z is -C(=O)-.

9. The compound represented by formula **XI** of claim 1, 7 or 8, or a pharmaceutically acceptable salt, a solvate, or a solvate of a pharmaceutically acceptable salt thereof, wherein the compound represented by formula **XI** has the definition in anyone of the following schemes; Scheme A: W is anyone of the following groups:
(1) 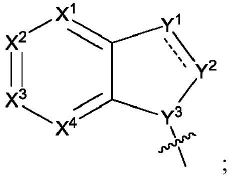 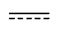
in 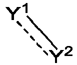 is a single bond or a double bond;
when 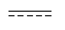 in 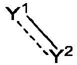 is a single bond, Y¹ is C(=O), O or N-R³, R³ is hydrogen, hydroxyl, CH₂COOH or C₁-C₄ alkyl, Y² is C(=N-CO-Ph), C(=N-CO-CH₃), NH, S(=O)₂, C=O or C=S;
when 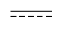 in 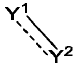 is a double bond, Y¹ is N, Y² is C-NH₂ or C-NH-CN;
Y³ is N or CH;
X¹ is N or CR², X² is N or CR⁴, X³ is N or CR⁵, X⁴ is N or CR⁶;
R², R⁴, R⁵ and R⁶ are independently hydrogen, halogen, hydroxyl, amino, carboxyl, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NR²⁻³R²⁻⁴ or -C(=O)-NR²⁻⁷R²⁻⁸;
R²⁻³, R²⁻⁴, R²⁻⁷ and R²⁻⁸ are independently hydrogen;
(2) 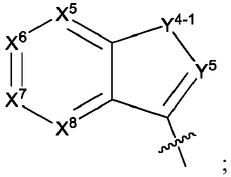
Y⁴⁻¹ is -NH-C(=O)-CH₂- or NH;
Y⁵ is N or C-R⁷, R⁷ is halogen;
X⁵ is CR⁸, X⁶ is CR⁹, X⁷ is CR¹⁰, X⁸ is CR¹¹;
R⁸, R⁹, R¹⁰ and R¹¹ are independently hydrogen;
(3) 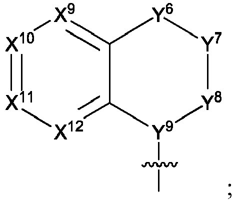
Y⁶ is N-C(=O)-CH₃, CH₂ or NH;
Y⁷ is NH, CH₂, C(=O), C(=S) or C(=N-OH);
Y⁸ is CH₂, CH₂CH₂, CH(CH₃) or C(=O);
Y⁹ is N;
X⁹ is CR¹², X¹⁰ is CR¹³, X¹¹ is CR¹⁴, X¹² is CR¹⁵;
R¹², R¹³, R¹⁴ and R¹⁵ are independently hydrogen, nitro, or C₁-C₄ alkyl substituted with one or more halogens;
(4) 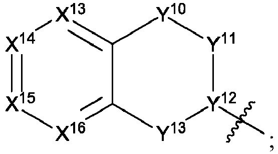
Y¹⁰ is NH;
Y¹¹ is C(=O);
Y¹² is N;
Y¹³ is CH₂;
X¹³ is CR¹⁶, X¹⁴ is CR¹⁷, X¹⁵ is CR¹⁸, X¹⁶ is CR¹⁹;
R¹⁶, R¹⁷ R¹⁸ and R¹⁹ are independently hydrogen;
(5) 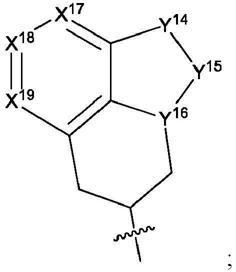
Y¹⁴ is NH;
Y¹⁵ is C(=O);
Y¹⁶ is N;
X¹⁷ is CR²⁰, X¹⁸ is CR²¹, X¹⁹ is CR²²;
R²⁰, R²¹ and R²² are independently hydrogen;
(6) 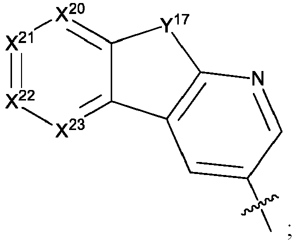
Y¹⁷ is NH;
X²⁰ is CR²³, X²¹ is CR²⁴, X²² is CR²⁵, X²³ is CR²⁶;
R²³, R²⁴, R²⁵ and R²⁶ are independently hydrogen;
(7) 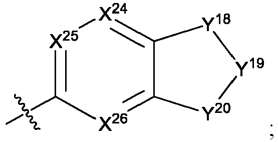
Y¹⁸ is NH or CH₂;
Y¹⁹ is C(=O) or NH;
Y²⁰ is CH₂ or C(=O);
X²⁴ is CR²⁷, X²⁵ is CR²⁸, X²⁶ is CR²⁹;
R²⁷, R²⁸ and R²⁹ are independently hydrogen;
(8) 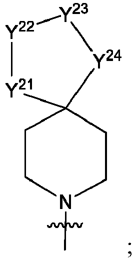
Y²¹ is C(=O);
Y²² is NH;
Y²³ is CH₂ or C(=O);
Y²⁴ is N-R³⁰, R³⁰ is hydrogen or phenyl;
(9) 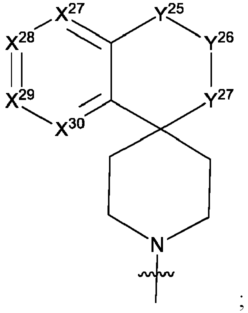
Y²⁵ is NH;
Y²⁶ is C(=O);
Y²⁷ is a single bond, O or NH;
X²⁷ is CR³¹, X²⁸ is CR³², X²⁹ is CR³³, X³⁰ is CR³⁴;
R³¹, R³² R³³ and R³⁴ are independently hydrogen;
(10) 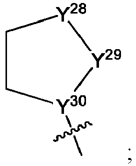
Y²⁸ is NH;
Y²⁹ is C(=O);
Y³⁰ is N;
(11) 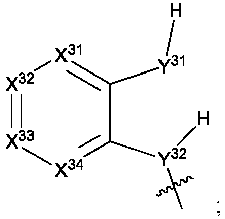
Y³¹ is O, NH or N-C(=O)-CH₃;
Y³² is N;
X³¹ is CR³⁵, X³² is CR³⁶, X³³ is CR³⁷, X³⁴ is CR³⁸;
R³⁵, R³⁶, R³⁷ and R³⁸ are independently hydrogen;
(12) 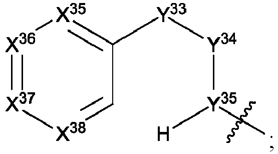
Y³³ is NH;
Y³⁴ is C(=O);
Y³⁵ is N;
X³⁵ is CR³⁹, X³⁶ is CR⁴⁰, X³⁷ is CR⁴¹, X³⁸ is CR⁴²;
R³⁹, R⁴⁰, R⁴¹ and R⁴² are independently hydrogen;
(13) 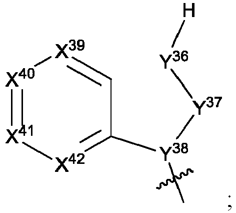
Y³⁶ is NH;
Y³⁷ is C(=O);
Y³⁸ is N;
X³⁹ is CR⁴³, X⁴⁰ is CR⁴⁴, X⁴¹ is CR⁴⁵, X⁴² is CR⁴⁶;
R⁴³, R⁴⁴, R⁴⁵ and R⁴⁶ are independently hydrogen;
(14) 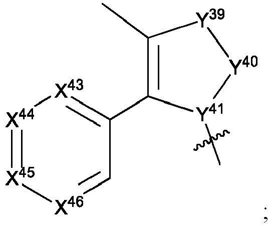
Y³⁹ is NH;
Y⁴⁰ is C(=O);
Y⁴¹ is N;
X⁴³ is CR⁴⁷, X⁴⁴ is CR⁴⁸, X⁴⁵ is CR⁴⁹, X⁴⁶ is CR⁵⁰;
R⁴⁷, R⁴⁸, R⁴⁹ and R⁵⁰ are independently hydrogen; and
(15) 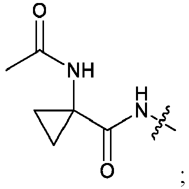
Z is anyone of the following fragments:
(1) 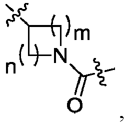 m is 1 or 2, n is 1, 2 or 3;
(2) 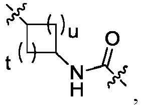 u is 1 or 2, t is 1 or 2;
(3) 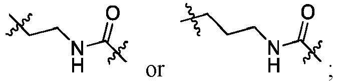
(4) 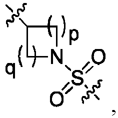 p is 1 or 2, q is 1 or 2;
(5) 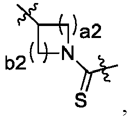 a2 is 1 or 2, b2 is 1 or 2;
(6) 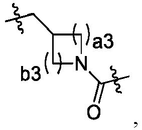 a3 is 1 or 2, b3 is 1 or 2;
(7) 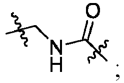
(8) -C(=O)-;
(9) 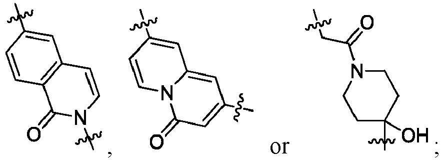
R¹ is C₂-C₄ alkyl, C₂-C₄ alkyl substituted with one or more halogens, -(CH₂)ₛ-R¹⁻¹, -NH-R¹⁻² 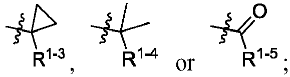 s is 0, 1 or 2;
R¹⁻¹, R¹⁻², R¹⁻³, R¹⁻⁴ and R¹⁻⁵ are independently C₇-C₁₀ cycloalkyl, one or more R¹⁻¹⁻³ substituted C₃-C₆ cycloalkyl, one or more R¹⁻¹⁻⁶ substituted 5-6 membered heterocycloalkyl containing 1, 2 or 3 atoms independently selected from N, O and S, one or more R¹⁻¹⁻¹ substituted C₆-C₁₀ aryl, naphthyl, 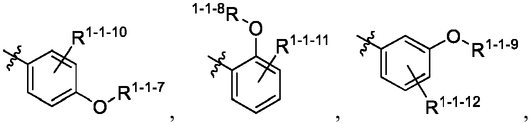 5, 10 membered heteroaryl containing 1, 2 or 3 atoms independently selected from N, O and S, pyridyl, 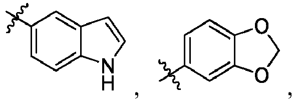 or one or more R¹⁻¹⁻² substituted 5-10 membered heteroaryl containing 1, 2 or 3 atoms independently selected from N, O and S;
R¹⁻¹⁻¹, R¹⁻¹⁻², R¹⁻¹⁻³, and R¹⁻¹⁻⁶ are independently carboxy, hydroxy, halogen, C₁-C₄ alkyl, C₁-C₄ alkyl substituted with one or more halogens, C₁-C₄ alkoxy substituted with one or more halogens, C₃-C₆ cycloalkyl, 5-6 membered heterocycloalkyl containing 1, 2, or 3 atoms independently selected from N, O and S, -N(R¹⁻¹⁻¹⁻¹)(R¹⁻¹⁻¹⁻²), or phenyl;
R¹⁻¹⁻¹⁻¹ and R¹⁻¹⁻¹⁻² are independently hydrogen or C₁-C₄ alkyl;
R¹⁻¹⁻⁷, R¹⁻¹⁻⁸, and R¹⁻¹⁻⁹ are independently C₁-C₄ alkyl;
R¹⁻¹⁻¹⁰ is independently C₁-C₄ alkoxy;
R¹⁻¹⁻¹¹ and R¹⁻¹⁻¹² are independently hydrogen or halogen;
Scheme B:
W is anyone of the following groups:
(1) 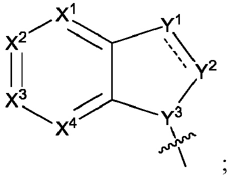 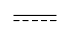
in 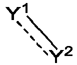 is a single bond or a double bond;
when 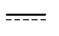 in 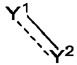 is a single bond, Y¹ is NH or N-OH, Y² is C(=N-CO-Ph), C(=N-CO-CH₃), S(=O)₂, C=O or C=S;
when 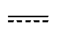 in 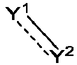 is a double bond, Y¹ is N, Y² is C-NH₂ or C-NH-CN;
Y³ is N or CH;
X¹ is N or CR², X² is N or CR⁴, X³ is N or CR⁵, X⁴ is N or CR⁶;
R², R⁴, R⁵ and R⁶ are independently hydrogen, halogen, hydroxyl, amino, cyano, nitro, C₁-C₄ alkyl, C₁-C₄ alkyl substituted with one or more halogens, C₁-C₄ alkoxy, C₃-C₆ cycloalkyl, phenyl, 5-7 membered heterocycloalkyl containing 1, 2, or 3 atoms independently selected from N, O and S, -S(=O)₂-NR²⁻³R²⁻⁴ or -C(=O)-NR²⁻⁷R²⁻⁸;
R²⁻³, R²⁻⁴, R²⁻⁷ and R²⁻⁸ are independently hydrogen;
(2) 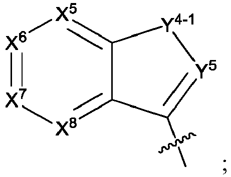
Y⁴⁻¹ is -NH-C(=O)-CH₂- or NH;
Y⁵ is N or C-R⁷, R⁷ is halogen;
X⁵ is CR⁸, X⁶ is CR⁹, X⁷ is CR¹⁰, X⁸ is CR¹¹;
R⁸, R⁹, R¹⁰ and R¹¹ are independently hydrogen;
(3) 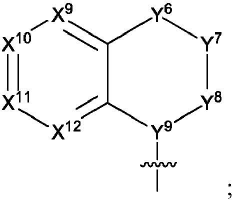
Y⁶ is N-C(=O)-CH₃ or NH;
Y⁷ is C(=O), C(=S) or C(=N-OH);
Y⁸ is CH₂ or C(=O);
Y⁹ is N;
X⁹ is CR¹², X¹⁰ is CR¹³, X¹¹ is CR¹⁴, X¹² is CR¹⁵;
R¹², R¹³, R¹⁴ and R¹⁵ are independently hydrogen, nitro, or C₁-C₄ alkyl substituted with one or more halogens;
(4) 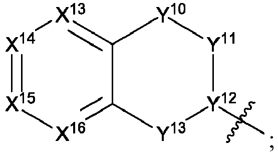
Y¹⁰ is NH;
Y¹¹ is C(=O);
Y¹² is N;
Y¹³ is CH₂;
X¹³ is CR¹⁶, X¹⁴ is CR¹⁷, X¹⁵ is CR¹⁸, X¹⁶ is CR¹⁹;
R¹⁶, R¹⁷ R¹⁸ and R¹⁹ are independently hydrogen;
(5) 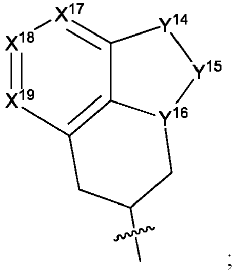
Y¹⁴ is NH;
Y¹⁵ is C(=O);
Y¹⁶ is N;
X¹⁷ is CR²⁰, X¹⁸ is CR²¹, X¹⁹ is CR²²;
R²⁰, R²¹ and R²² are independently hydrogen;
(7) 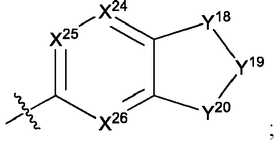
Y¹⁸ is NH or CH₂;
Y¹⁹ is C(=O) or NH;
Y²⁰ is CH₂ or C(=O);
X²⁴ is CR²⁷, X²⁵ is CR²⁸, X²⁶ is CR²⁹;
R²⁷, R²⁸ and R²⁹ are independently hydrogen;
(8) 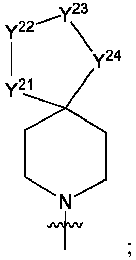
Y²¹ is C(=O);
Y²² is NH;
Y²³ is CH₂;
Y²⁴ is N-R³⁰, R³⁰ is phenyl;
(9) 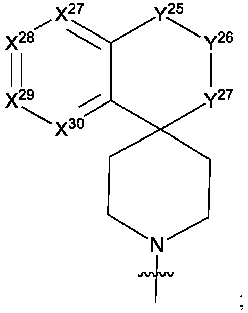
Y²⁵ is NH;
Y²⁶ is C(=O);
Y²⁷ is a single bond, O or NH;
X²⁷ is CR³¹, X²⁸ is CR³², X²⁹ is CR³³, X³⁰ is CR³⁴;
R³¹, R³² R³³ and R³⁴ are independently hydrogen;
(11) 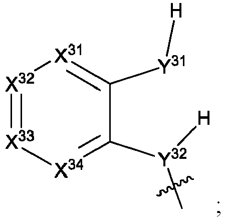
Y³¹ is O, NH or N-C(=O)-CH₃;
Y³² is N;
X³¹ is CR³⁵, X³² is CR³⁶, X³³ is CR³⁷, X³⁴ is CR³⁸; R³⁵, R³⁶, R³⁷ and R³⁸ are independently hydrogen;
(12) 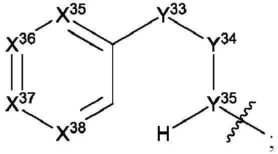
Y³³ is NH;
Y³⁴ is C(=O);
Y³⁵ is N;
X³⁵ is CR³⁹, X³⁶ is CR⁴⁰, X³⁷ is CR⁴¹, X³⁸ is CR⁴²;
R³⁹, R⁴⁰, R⁴¹ and R⁴² are independently hydrogen;
(13) 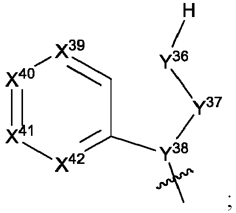
Y³⁶ is NH;
Y³⁷ is C(=O);
Y³⁸ is N;
X³⁹ is CR⁴³, X⁴⁰ is CR⁴⁴, X⁴¹ is CR⁴⁵, X⁴² is CR⁴⁶;
R⁴³, R⁴⁴, R⁴⁵ and R⁴⁶ are independently hydrogen; and
(14) 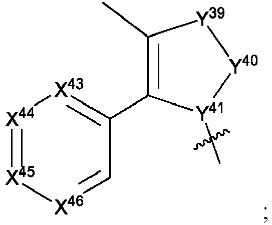
Y³⁹ is NH;
Y⁴⁰ is C(=O);
Y⁴¹ is N;
X⁴³ is CR⁴⁷, X⁴⁴ is CR⁴⁸, X⁴⁵ is CR⁴⁹, X⁴⁶ is CR⁵⁰;
R⁴⁷, R⁴⁸, R⁴⁹ and R⁵⁰ are independently hydrogen; Z is anyone of the following fragments:
(1) 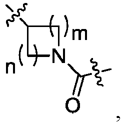 m is 1 or 2, n is 1, 2 or 3;
(2) 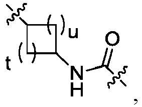 u is 1 or 2, t is 1 or 2;
(3) 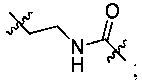
(4) 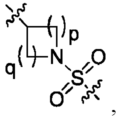 p is 1 or 2, q is 1 or 2;
(5) 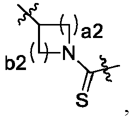 a2 is 1 or 2, b2 is 1 or 2;
(6) 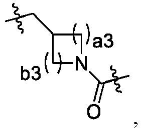 a3 is 1 or 2, b3 is 1 or 2;
(7) 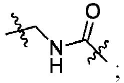
(8) -C(=O)-;
(9) 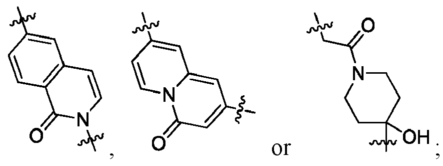
R¹ is -(CH₂)ₛ-R¹⁻¹, -NH-R¹⁻², 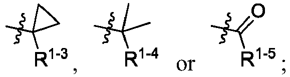 s is 0, 1 or 2;
R¹⁻¹, R¹⁻², R¹⁻³, R¹⁻⁴ and R¹⁻⁵ are independently one or more R¹⁻¹⁻³ substituted C₃-C₆ cycloalkyl, one or more R¹⁻¹⁻¹ substituted C₆-C₁₀ aryl, naphthyl, 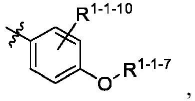 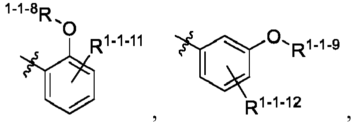 5, 10 membered heteroaryl containing 1, 2 or 3 atoms independently selected from N, O and S, pyridyl, 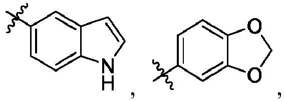 or one or more
R¹⁻¹⁻² substituted 5-10 membered heteroaryl containing 1, 2 or 3 atoms independently selected from N, O and S;
R¹⁻¹⁻¹, R¹⁻¹⁻², and R¹⁻¹⁻³ are independently carboxy, hydroxy, halogen, C₁-C₄ alkyl, C₁-C₄ alkyl substituted with one or more halogens, C₁-C₄ alkoxy substituted with one or more halogens, C₃-C₆ cycloalkyl, 5-6 membered heterocycloalkyl containing 1, 2, or 3 atoms independently selected from N, O and S, -N(R¹⁻¹⁻¹⁻¹)(R¹⁻¹⁻¹⁻²), or phenyl;
R¹⁻¹⁻¹⁻¹ and R¹⁻¹⁻¹⁻² are independently hydrogen or C₁-C₄ alkyl;
R¹⁻¹⁻⁷, R¹⁻¹⁻⁸, and R¹⁻¹⁻⁹ are independently C₁-C₄ alkyl;
R¹⁻¹⁻¹⁰ is independently C₁-C₄ alkoxy;
R¹⁻¹⁻¹¹ and R¹⁻¹⁻¹² are independently hydrogen or halogen.

10. The compound represented by formula **XI** of at least one of claims 1-9, or a pharmaceutically acceptable salt, a solvate, or a solvate of a pharmaceutically acceptable salt thereof, wherein when R³ is C₁-C₄ alkyl, said C₁-C₄ alkyl is methyl;
and/or when R², R⁴, R⁵ and R⁶ are independently halogen, said halogen is fluorine, chlorine or bromine;
and/or when R², R⁴, R⁵ and R⁶ are independently C₁-C₄ alkyl, said C₁-C₄ alkyl is methyl or tert-butyl;
and/or when R², R⁴, R⁵ and R⁶ are independently C₁-C₄ alkyl substituted with a plurality of halogens, said plurality of halogens are 2 or 3 halogens;
and/or when R², R⁴, R⁵ and R⁶ are independently C₁-C₄ alkyl substituted with one or more halogens, said halogen is fluorine;
and/or when R², R⁴, R⁵ and R⁶ are independently C₁-C₄ alkyl substituted with one or more halogens, said C₁-C₄ alkyl is methyl;
and/or when R², R⁴, R⁵ and R⁶ are independently C₁-C₄ alkoxy, said C₁-C₄ alkoxy is methoxy;
and/or when R², R⁴, R⁵ and R⁶ are independently C₃-C₆ cycloalkyl, said C₃-C₆ cycloalkyl is cyclopropyl;
and/or when R², R⁴, R⁵ and R⁶ are independently 5-7 membered heterocycloalkyl containing 1, 2 or 3 atoms independently selected from N, O and S, said 5-7 membered heterocycloalkyl containing 1, 2 or 3 atoms independently selected from N, O and S is morpholinyl;
and/or when R²⁻¹, R²⁻², R²⁻³, R²⁻⁴, R²⁻⁵, R²⁻⁶, R²⁻⁷ and R²⁻⁸ are independently C₁-C₄ alkyl, said C₁-C₄ alkyl is methyl or tert-butyl;
and/or when R²⁻¹, R²⁻², R²⁻³, R²⁻⁴, R²⁻⁵, R²⁻⁶, R²⁻⁷ and R²⁻⁸ are independently C₁-C₄ alkyl substituted with a plurality of halogens, said plurality of halogens are 2 or 3 halogens;
and/or when R²⁻¹, R²⁻², R²⁻³, R²⁻⁴, R²⁻⁵, R²⁻⁶, R²⁻⁷ and R²⁻⁸ are independently C₁-C₄ alkyl substituted with one or more halogens, said halogen is fluorine;
and/or when R²⁻¹, R²⁻², R²⁻³, R²⁻⁴, R²⁻⁵, R²⁻⁶, R²⁻⁷ and R²⁻⁸ are independently C₁-C₄ alkyl substituted with one or more halogens, said C₁-C₄ alkyl is methyl;
and/or when W is 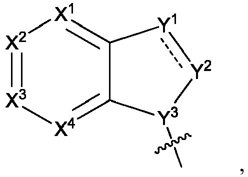 the 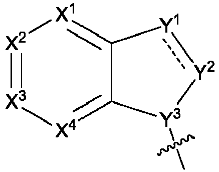 is 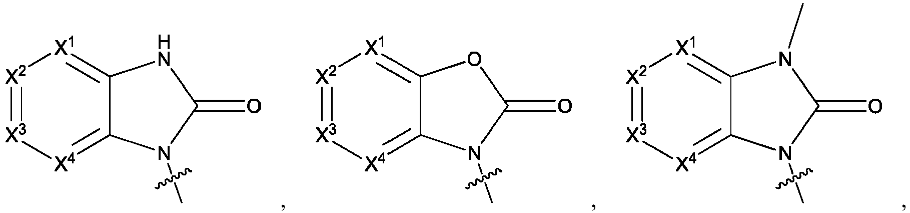 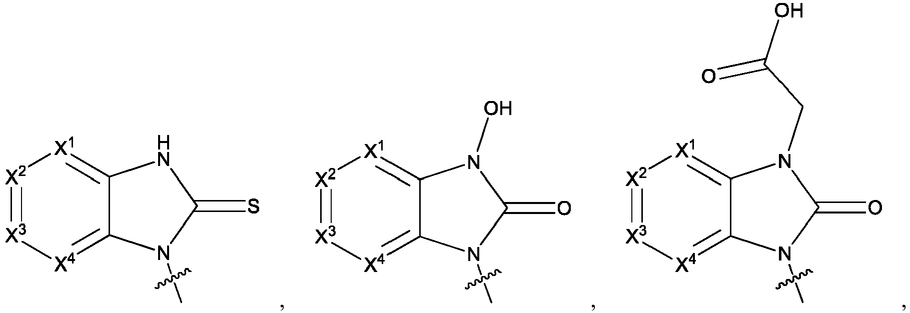 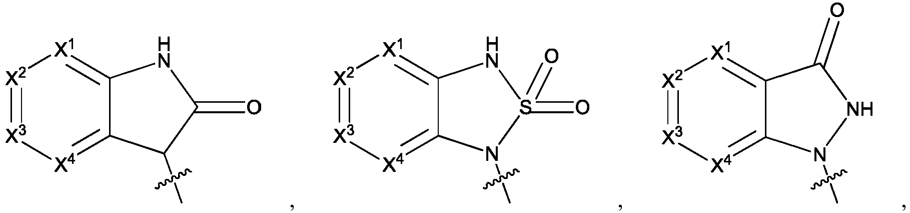 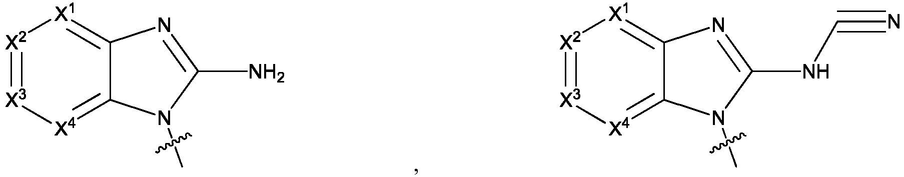 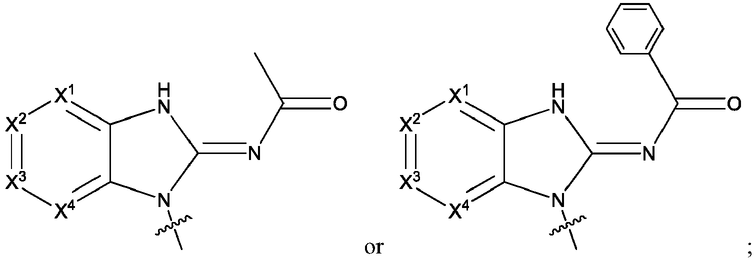
and/or when Y⁴⁻¹ is -NH-C(=O)-CH₂-, the -CH₂- end is connected with Y⁵;
and/or when W is 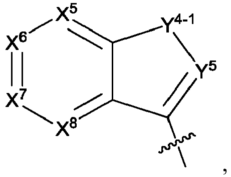 the 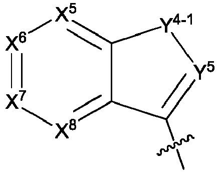 is 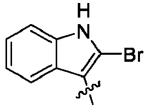 or 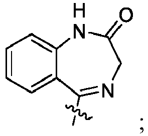
and/or when W is 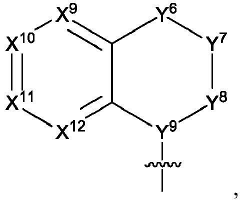 the 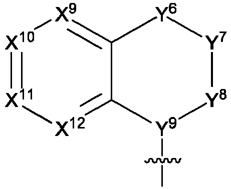 is 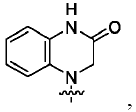 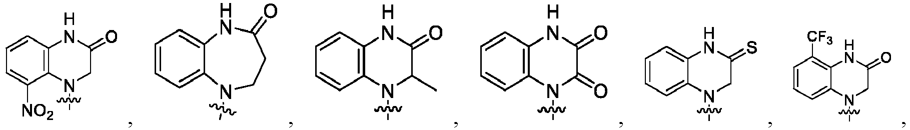 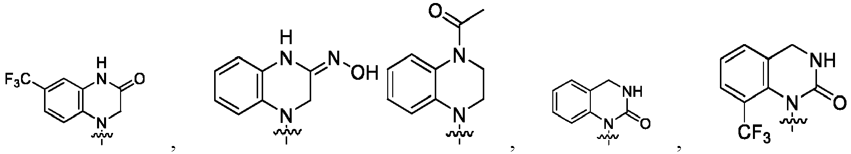 or 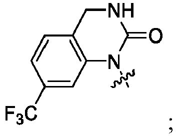
and/or when W is 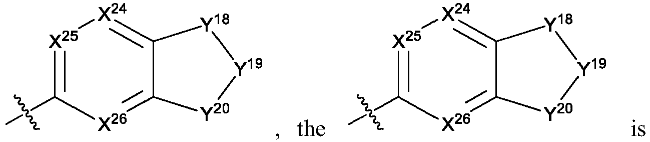 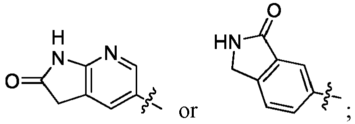
and/or when W is 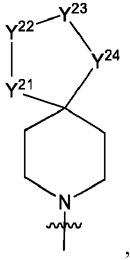 the 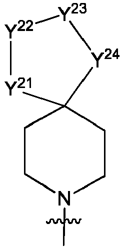 is 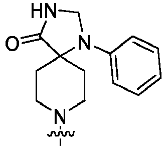 or 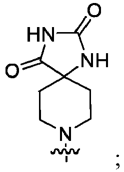
and/or when W is 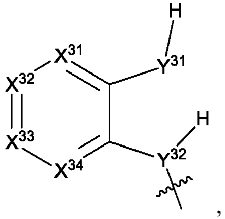 the 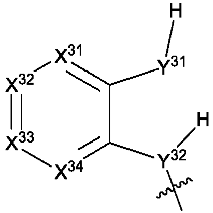 is 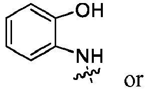 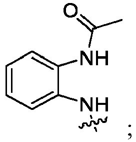
and/or when Z is 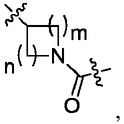 m is 1, n is 1, or m is 2, n is 2;
and/or when Z is 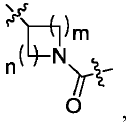 the 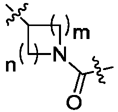 is 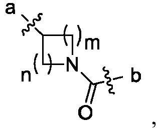 whose b end is connected with R¹;
and/or when Z is 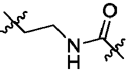 or 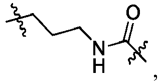 the carbonyl end is connected with R¹;
and/or when Z is 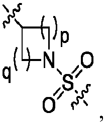 p is 2, q is 2;
and/or when Z is 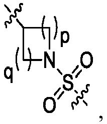 the 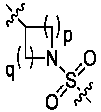 is 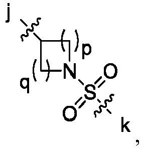 whose k end is connected with R¹;
and/or when Z is 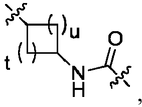 u is 1, t is 1, or u is 2, t is 2;
and/or when Z is 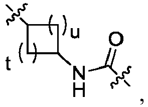 the 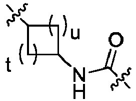 is 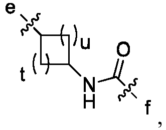 whose f end is connected with R¹;
and/or when Z is 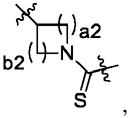 a2 is 2, b2 is 2;
and/or when Z is 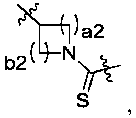 the 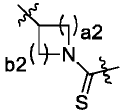 is 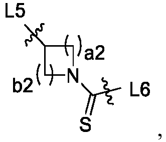 whose L6 end is connected with R¹;
and/or when Z is 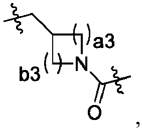 a3 is 2, b3 is 2;
and/or when Z is 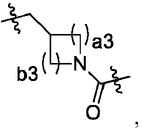 the 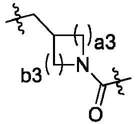 is 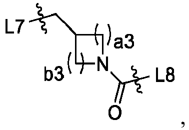 whose L8 end is connected with R¹;
and/or when Z is 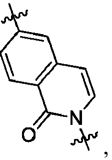 the 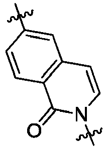 is 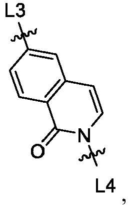 whose L4 end is connected with R¹;
and/or when Z is 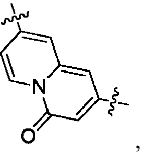 the 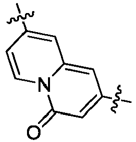 is 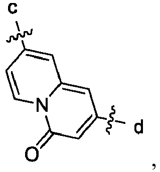 whose d end is connected with R¹;
and/or when Z is 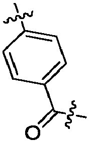 , the 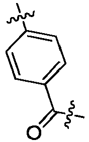 is 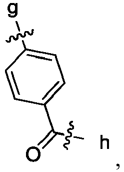 whose h end is connected with R¹;
and/or when Z is 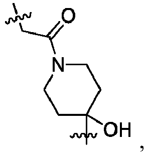 the 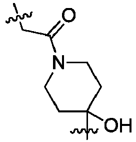 is 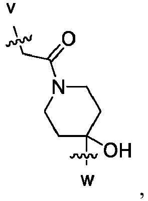 whose w end is connected with R¹;
and/or when R¹ is a C₂-C₄ alkyl, said C₂-C₄ alkyl is ethyl;
and/or when R¹ is a C₂-C₄ alkyl substituted with a plurality of halogens, said plurality of halogens are 2 or 3 halogens;
and/or when R¹ is a C₂-C₄ alkyl substituted with one or more halogens, said halogen is fluorine;
and/or when R¹ is a C₂-C₄ alkyl substituted with one or more halogens, the C₂-C₄ alkyl is ethyl;
and/or when R¹⁻¹, R¹⁻², R¹⁻³, R¹⁻⁴ and R¹⁻⁵ are independently one or more R¹⁻¹⁻¹ substituted C₆-C₁₀ aryl, said C₆-C₁₀ aryl is phenyl or naphthyl;
and/or when R¹⁻¹, R¹⁻², R¹⁻³, R¹⁻⁴ and R¹⁻⁵ are independently one or more R¹⁻¹⁻² substituted 5-10 membered heteroaryl containing 1, 2, or 3 atoms independently selected from N, O and S, said 5-10 membered heteroaryl containing 1, 2, or 3 atoms independently selected from N, O and S is 5-6 membered heteroaryl, indolyl, isoquinolyl or quinolyl containing one atom selected from N, O and S;
and/or when R¹⁻¹, R¹⁻², R¹⁻³, R¹⁻⁴ and R¹⁻⁵ are independently C₃-C₆ cycloalkyl, said C₃-C₆ cycloalkyl is cyclobutyl;
and/or when R¹⁻¹, R¹⁻², R¹⁻³, R¹⁻⁴ and R¹⁻⁵ are independently one or more R¹⁻¹⁻³ substituted C₃-C₆ cycloalkyl, said C₃-C₆ cycloalkyl is cyclobutyl or cyclohexyl;
and/or when R¹⁻¹, R¹⁻², R¹⁻³, R¹⁻⁴ and R¹⁻⁵ are independently one or more R¹⁻¹⁻⁶ substituted 5-6 membered heterocycloalkyl containing 1, 2 or 3 atoms independently selected from N, O, and S, said 5-6 membered heterocycloalkyl containing 1, 2 or 3 atoms independently selected from N, O, and S is piperidyl;
and/or when R¹⁻¹, R¹⁻², R¹⁻³, R¹⁻⁴ and R¹⁻⁵ are independently C₇-C₁₀ cycloalkyl, said C₇-C₁₀ cycloalkyl is C₇-C₁₀ bridged cycloalkyl;
and/or when R¹⁻¹, R¹⁻², R¹⁻³, R¹⁻⁴ and R¹⁻⁵ are independently naphthyl, said naphthyl is naphth-2-yl;
and/or when R¹⁻¹, R¹⁻², R¹⁻³, R¹⁻⁴ and R¹⁻⁵ are independently pyridyl, said pyridyl is pyridin-4-yl;
and/or when R¹⁻¹, R¹⁻², R¹⁻³, R¹⁻⁴ and R¹⁻⁵ are independently 5, 10 membered heteroaryl containing 1, 2 or 3 atoms independently selected from N, O and S, said 5, 10 membered heteroaryl containing 1, 2 or 3 atoms independently selected from N, O and S is 10 membered heteroaryl containing 1 or 2 atoms independently selected from N, O and S;
and/or when R¹⁻¹⁻¹, R¹⁻¹⁻², R¹⁻¹⁻³, and R¹⁻¹⁻⁶ are independently C₁-C₄ alkyl, said C₁-C₄ alkyl is methyl, ethyl, n-propyl, isopropyl, n-butyl, sec-butyl, isobutyl or tert-butyl;
and/or when R¹⁻¹⁻¹, R¹⁻¹⁻², R¹⁻¹⁻³, and R¹⁻¹⁻⁶ are independently C₁-C₄ alkyl substituted with a plurality of halogens, said plurality of halogens are 2 or 3 halogens;
and/or when R¹⁻¹⁻¹, R¹⁻¹⁻², R¹⁻¹⁻³, and R¹⁻¹⁻⁶ are independently C₁-C₄ alkyl substituted with one or more halogens, said halogen is fluorine;
and/or when R¹⁻¹⁻¹, R¹⁻¹⁻², R¹⁻¹⁻³, and R¹⁻¹⁻⁶ are independently C₁-C₄ alkyl substituted with one or more halogens, said C₁-C₄ alkyl is methyl or ethyl;
and/or when R¹⁻¹⁻¹, R¹⁻¹⁻², R¹⁻¹⁻³, and R¹⁻¹⁻⁶ are independently C₃-C₆ cycloalkyl, said C₃-C₆ cycloalkyl is cyclopropyl;
and/or when R¹⁻¹⁻¹, R¹⁻¹⁻², R¹⁻¹⁻³, and R¹⁻¹⁻⁶ are independently halogen, said halogen is fluorine, chlorine or bromine;
and/or when R¹⁻¹⁻¹, R¹⁻¹⁻², R¹⁻¹⁻³, and R¹⁻¹⁻⁶ are independently C₁-C₄ alkoxy substituted with a plurality of halogens, said plurality of halogens are 2 or 3 halogens;
and/or when R¹⁻¹⁻¹, R¹⁻¹⁻², R¹⁻¹⁻³, and R¹⁻¹⁻⁶ are independently C₁-C₄ alkoxy substituted with one or more halogens, said halogen is fluorine;
and/or when R¹⁻¹⁻¹, R¹⁻¹⁻², R¹⁻¹⁻³, and R¹⁻¹⁻⁶ are independently C₁-C₄ alkoxy substituted with one or more halogens, said C₁-C₄ alkoxy is methoxy or ethoxy;
and/or when R¹⁻¹⁻¹, R¹⁻¹⁻², R¹⁻¹⁻³, and R¹⁻¹⁻⁶ are independently 5-6 membered heterocycloalkyl containing 1, 2 or 3 atoms independently selected from N, O and S, said 5-6 membered heterocycloalkyl containing 1, 2 or 3 atoms independently selected from N, O and S is morpholinyl;
and/or when R¹⁻¹⁻¹⁻¹ and R¹⁻¹⁻¹⁻² are independently C₁-C₄ alkyl, said C₁-C₄ alkyl is methyl or ethyl;
and/or when R¹⁻¹⁻⁷, R¹⁻¹⁻⁸, and R¹⁻¹⁻⁹ are independently C₁-C₄ alkyl, said C₁-C₄ alkyl is methyl or ethyl;
and/or when R¹⁻¹⁻¹⁰ is independently C₁-C₄ alkoxy, said C₁-C₄ alkoxy is methoxy or ethoxy; and/or when R¹⁻¹⁻¹¹ and R¹⁻¹⁻¹² are independently halogen, said halogen is fluorine.

11. The compound represented by formula **XI** of at least one of claims 1-10, or a pharmaceutically acceptable salt, a solvate, or a solvate of a pharmaceutically acceptable salt thereof, wherein when R², R⁴, R⁵ and R⁶ are independently C₁-C₄ alkyl, said C₁-C₄ alkyl is tert-butyl;
and/or when R², R⁴, R⁵ and R⁶ are independently C₁-C₄ alkyl substituted with a plurality of halogens, said C₁-C₄ alkyl substituted with a plurality of halogens is trifluoromethyl;
and/or when R², R⁴, R⁵ and R⁶ are independently 5-7 membered heterocycloalkyl containing 1, 2 or 3 atoms independently selected from N, O and S, said 5-7 membered heterocycloalkyl containing 1, 2 or 3 atoms independently selected from N, O and S is morpholin-1-yl;
and/or when R²⁻¹, R²⁻², R²⁻³, R²⁻⁴, R²⁻⁵, R²⁻⁶, R²⁻⁷ and R²⁻⁸ are independently C₁-C₄ alkyl, said C₁-C₄ alkyl is methyl or tert-butyl;
and/or when R²⁻¹, R²⁻², R²⁻³, R²⁻⁴, R²⁻⁵, R²⁻⁶, R²⁻⁷ and R²⁻⁸ are independently C₁-C₄ alkyl substituted with a plurality of halogens, said C₁-C₄ alkyl substituted with a plurality of halogens is trifluoromethyl;
and/or when Z is 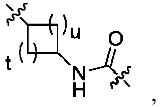 the 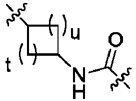 is 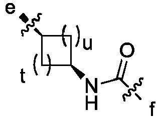 or 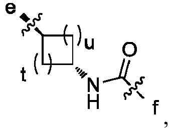 whose f end is connected with R¹;
and/or when R¹ is C₂-C₄ alkyl substituted with a plurality halogens, said C₂-C₄ alkyl substituted with a plurality halogens is 2,2,2-trifluoroethyl;
and/or when R¹⁻¹, R¹⁻², R¹⁻³, R¹⁻⁴ and R¹⁻⁵ are independently one or more R¹⁻¹⁻¹ substituted C₆-C₁₀ aryl, said C₆-C₁₀ aryl is phenyl or naphthyl;
and/or when R¹⁻¹, R¹⁻², R¹⁻³, R¹⁻⁴ and R¹⁻⁵ are independently one or more R¹⁻¹⁻¹ substituted phenyl, said R¹⁻¹⁻¹ and said -(CH₂)ₛ-, -NH-, 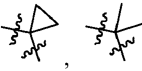 or carbonyl are located in a ortho, meta or para position relative to each other;
and/or when R¹⁻¹, R¹⁻², R¹⁻³, R¹⁻⁴ and R¹⁻⁵ are independently one or more R¹⁻¹⁻² substituted 5-10 membered heteroaryl containing 1, 2 or 3 atoms independently selected from N, O and S, said 5-10 membered heteroaryl containing 1, 2 or 3 atoms independently selected from N, O and S is furyl, thienyl or pyridyl;
and/or when R¹⁻¹, R¹⁻², R¹⁻³, R¹⁻⁴ and R¹⁻⁵ are independently one or more R¹⁻¹⁻² substituted 5-10 membered heteroaryl containing 1, 2 or 3 atoms independently selected from N, O and S, and said 5-10 membered heteroaryl containing 1, 2 or 3 atoms independently selected from N, O and S is a 5 membered heteroaryl containing one atom selected from N, O and S, said R¹⁻¹⁻²
and said -(CH₂)ₛ-, -NH-, 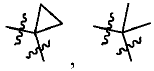 or carbonyl are located in a ortho or meta position relative to each other;
and/or when R¹⁻¹, R¹⁻², R¹⁻³, R¹⁻⁴ and R¹⁻⁵ are independently one or more R¹⁻¹⁻² substituted 5-10 membered heteroaryl containing 1, 2 or 3 atoms independently selected from N, O and S, and said 5-10 membered heteroaryl containing 1, 2 or 3 atoms independently selected from N, O and S is a 6 membered heteroaryl containing one N, said R¹⁻¹⁻² and said -(CH₂)ₛ-, -NH-, 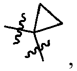 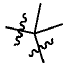 or carbonyl are located in a ortho, meta or para position relative to each other;
and/or when R¹⁻¹, R¹⁻², R¹⁻³, R¹⁻⁴ and R¹⁻⁵ are independently one or more R¹⁻¹⁻⁶ substituted 5-6 membered heterocycloalkyl containing 1, 2 or 3 atoms independently selected from N, O, and S, said 5-6 membered heterocycloalkyl containing 1, 2 or 3 atoms independently selected from N, O, and S is piperidin-1-yl;
and/or when R¹⁻¹, R¹⁻², R¹⁻³, R¹⁻⁴ and R¹⁻⁵ are independently C₇-C₁₀ cycloalkyl, said C₇-C₁₀ cycloalkyl is adamantyl;
and/or when R¹⁻¹, R¹⁻², R¹⁻³, R¹⁻⁴ and R¹⁻⁵ are independently 5, 10 membered heteroaryl containing 1, 2, or 3 atoms independently selected from N, O and S, said 5, 10 membered heteroaryl containing 1, 2, or 3 atoms independently selected from N, O and S is quinolinyl, 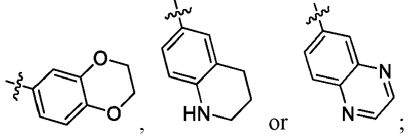
and/or when R¹⁻¹⁻¹, R¹⁻¹⁻², R¹⁻¹⁻³, and R¹⁻¹⁻⁶ are independently C₁-C₄ alkyl, said C₁-C₄ alkyl is methyl or tert-butyl;
and/or when R¹⁻¹⁻¹, R¹⁻¹⁻², R¹⁻¹⁻³, and R¹⁻¹⁻⁶ are independently C₁-C₄ alkyl substituted with a plurality of halogens, said C₁-C₄ alkyl substituted with a plurality of halogens is trifluoromethyl;
and/or when R¹⁻¹⁻¹, R¹⁻¹⁻², R¹⁻¹⁻³, and R¹⁻¹⁻⁶ are independently C₁-C₄ alkoxy substituted with a plurality of halogens, said C₁-C₄ alkoxy substituted with a plurality of halogens is trifluoromethoxy;
and/or when R¹⁻¹⁻¹, R¹⁻¹⁻², R¹⁻¹⁻³, and R¹⁻¹⁻⁶ are independently 5-6 membered heterocycloalkyl containing 1, 2 or 3 atoms independently selected from N, O and S, said 5-6 membered heterocycloalkyl containing 1, 2 or 3 atoms independently selected from N, O and S is morpholin-1-yl.

12. The compound represented by formula XI of at least one of claims 1-11, or a pharmaceutically acceptable salt, a solvate, or a solvate of a pharmaceutically acceptable salt thereof, wherein when R¹⁻¹, R¹⁻², R¹⁻³, R¹⁻⁴ and R¹⁻⁵ are independently one or more R¹⁻¹⁻¹ substituted C₆-C₁₀ aryl, said one or more R¹⁻¹⁻¹ substituted C₆-C₁₀ aryl is 4-trifluoromethylphenyl;
and/or when R¹⁻¹, R¹⁻², R¹⁻³, R¹⁻⁴ and R¹⁻⁵ are independently one or more R¹⁻¹⁻² substituted 5-10 membered heteroaryl containing 1, 2 or 3 atoms independently selected from N, O and S, said 5-10 membered heteroaryl containing 1, 2 or 3 atoms independently selected from N, O and S is pyridin-2-yl, pyridin-3-yl or pyridin-4-yl;
and/or when R¹⁻¹, R¹⁻², R¹⁻³, R¹⁻⁴ and R¹⁻⁵ are independently one or more R¹⁻¹⁻² substituted 5-10 membered heteroaryl containing 1, 2 or 3 atoms independently selected from of N, O, and S, and said 5-10 membered heteroaryl containing 1, 2 or 3 atoms independently selected from of N, O, and S is a 6 membered heteroaryl containing one N, said R¹⁻¹⁻² and said -(CH₂)ₛ-, -NH-, 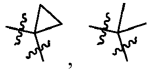 or carbonyl are located in a para position relative to each other;
and/or when R¹⁻¹, R¹⁻², R¹⁻³, R¹⁻⁴ and R¹⁻⁵ are independently 5, 10 membered heteroaryl containing 1, 2, or 3 atoms independently selected from N, O and S, said 5, 10 membered heteroaryl containing 1, 2, or 3 atoms independently selected from N, O and S is

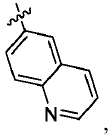

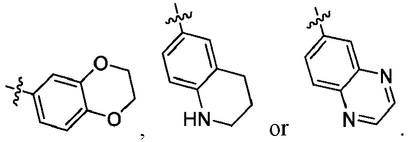

13. The compound represented by formula XI of at least one of claims 1-12, or a pharmaceutically acceptable salt, a solvate, or a solvate of a pharmaceutically acceptable salt thereof, wherein

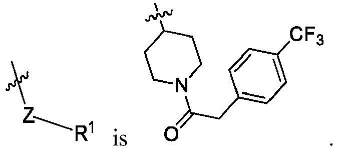

14. The compound represented by formula **XI** of at least one of claims 1-13, or a pharmaceutically acceptable salt, a solvate, or a solvate of a pharmaceutically acceptable salt thereof, wherein the compound represented by formula **XI** has anyone of the following structures:

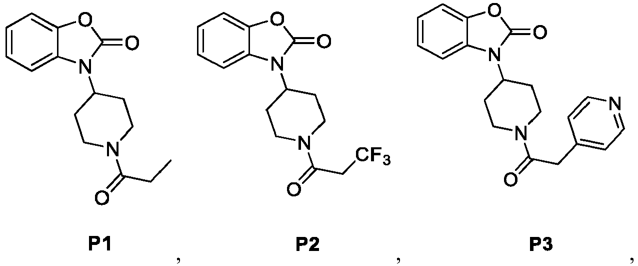

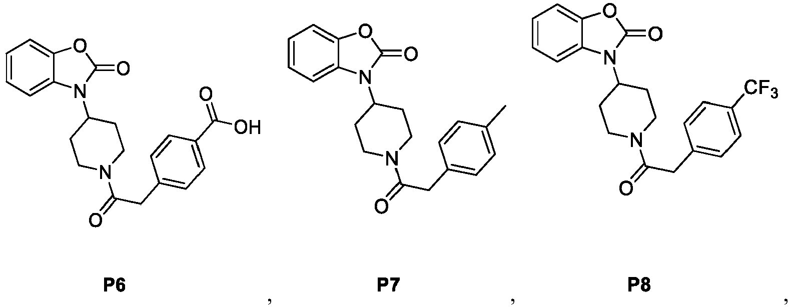

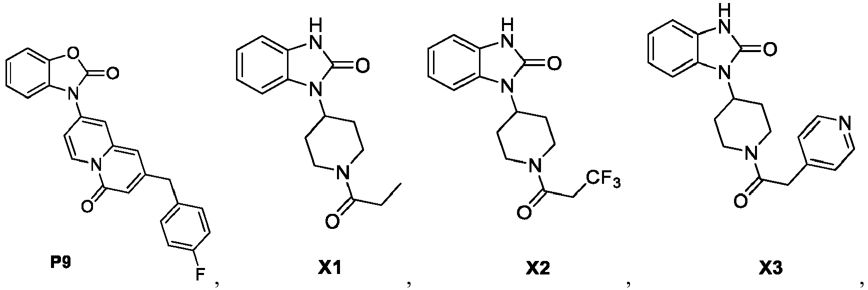

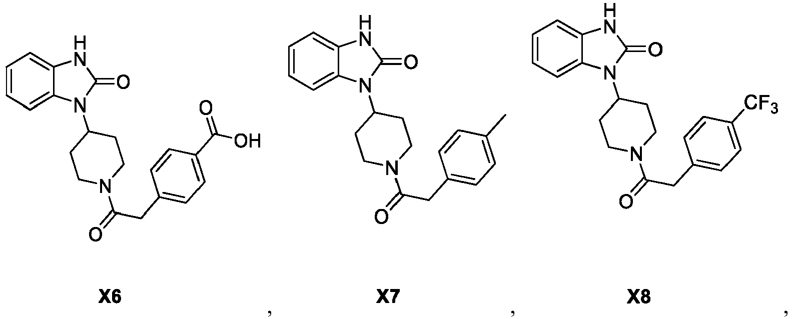

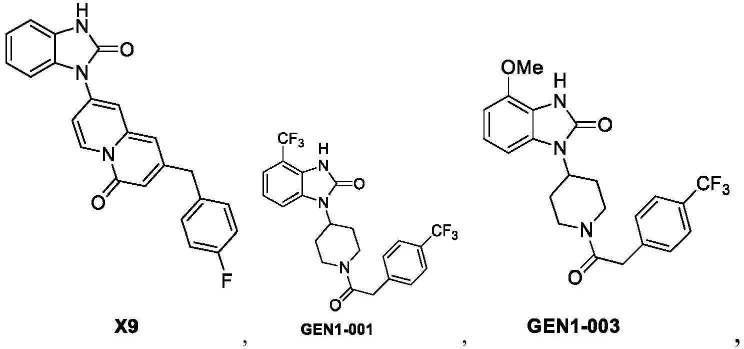

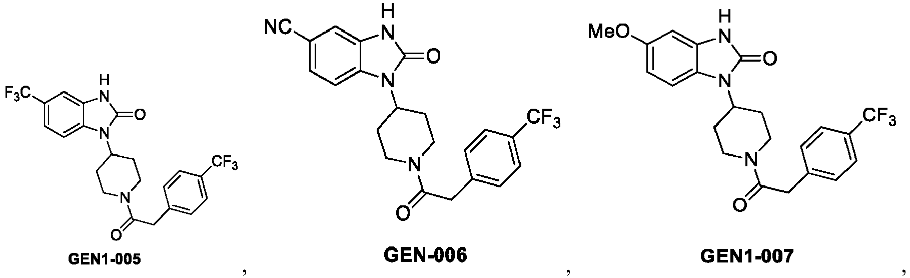

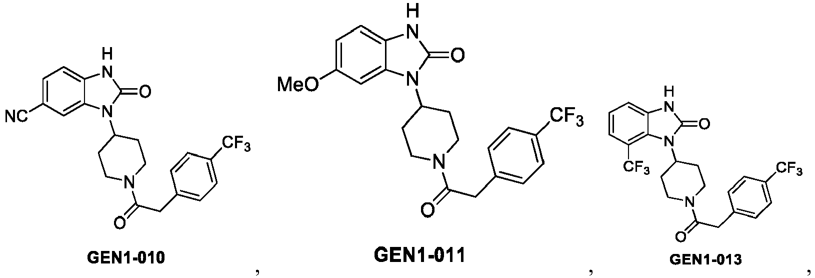

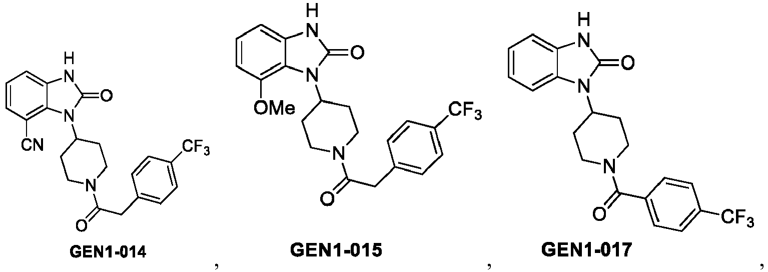

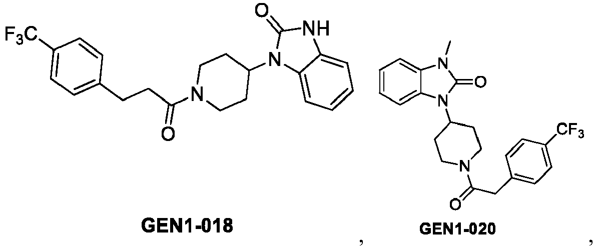

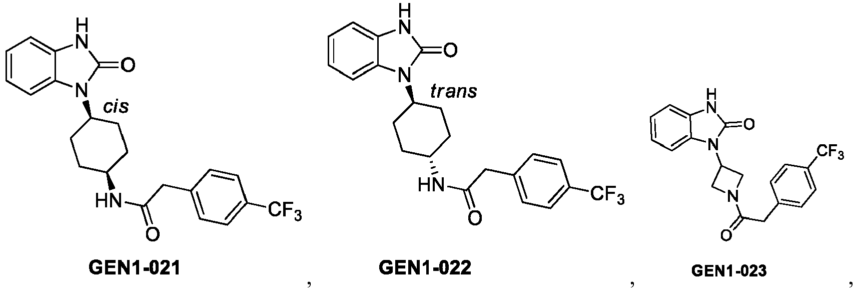

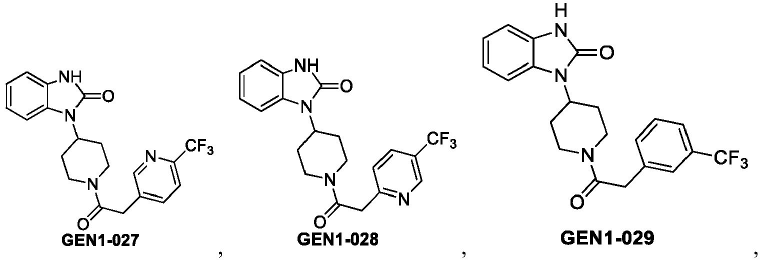

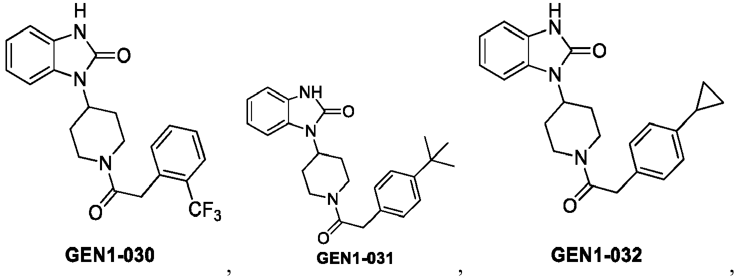

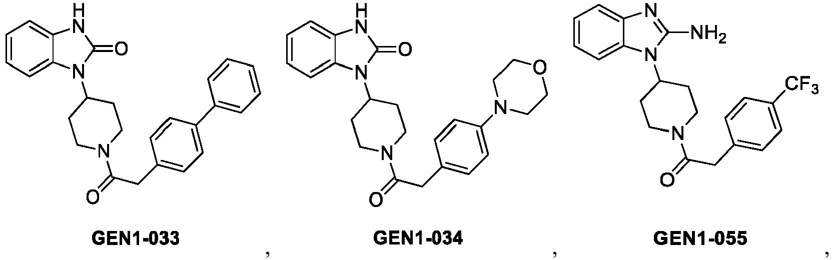

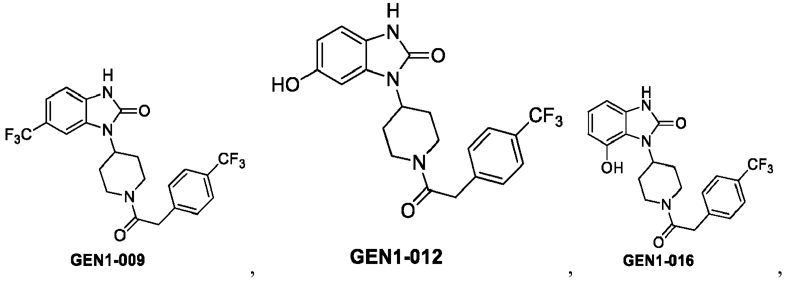

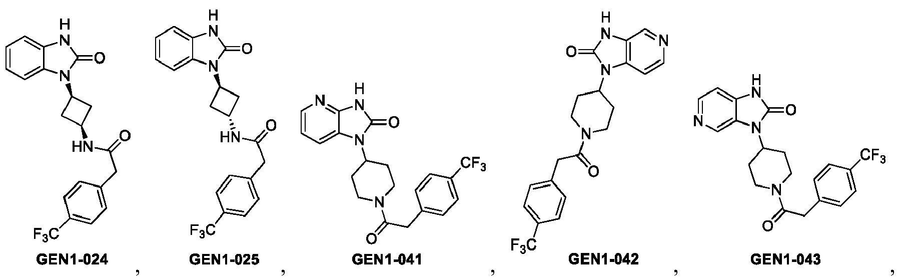

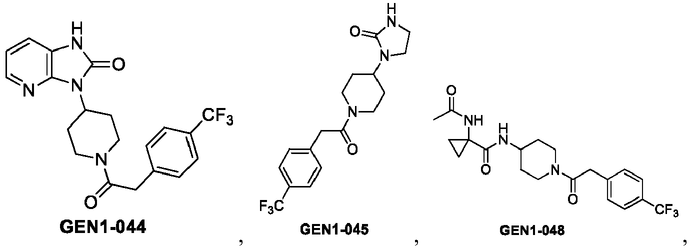

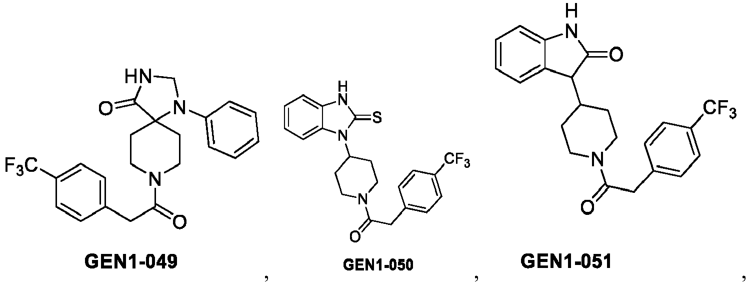

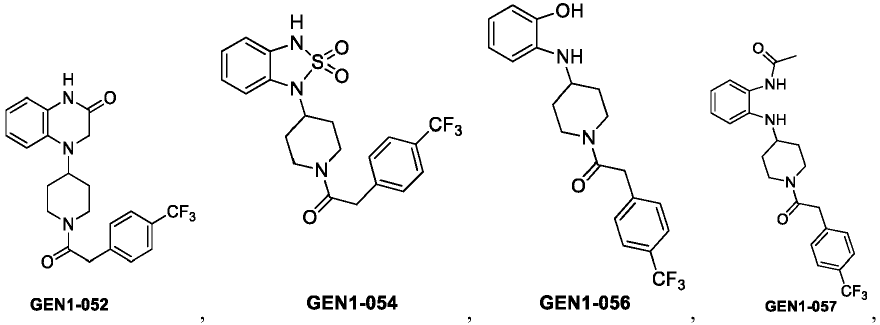

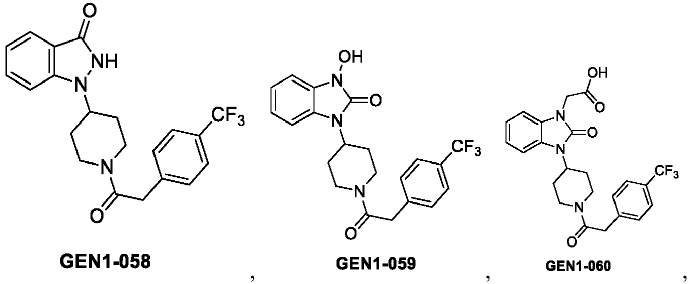

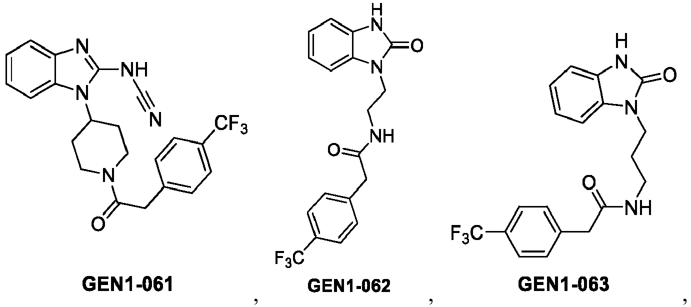

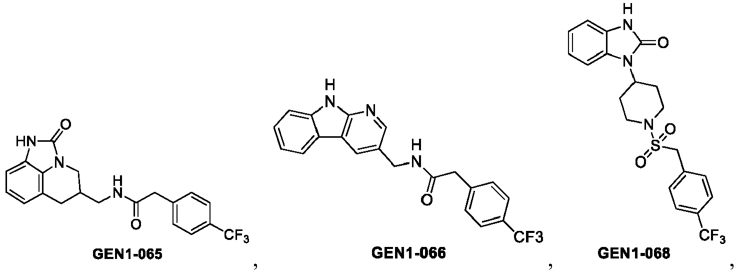

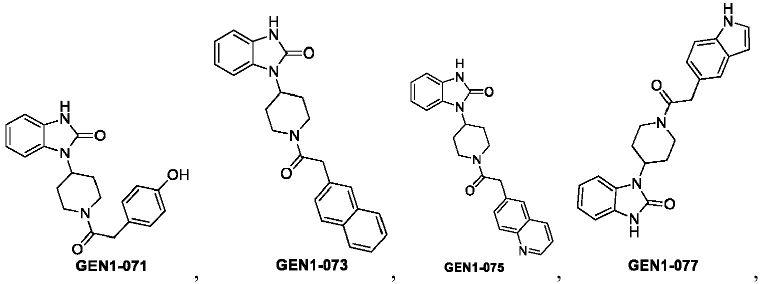

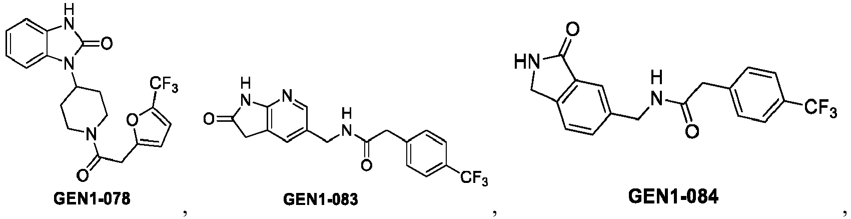

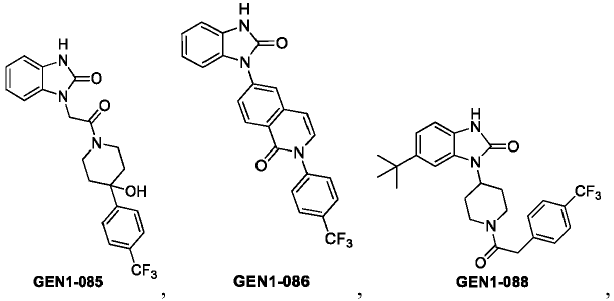

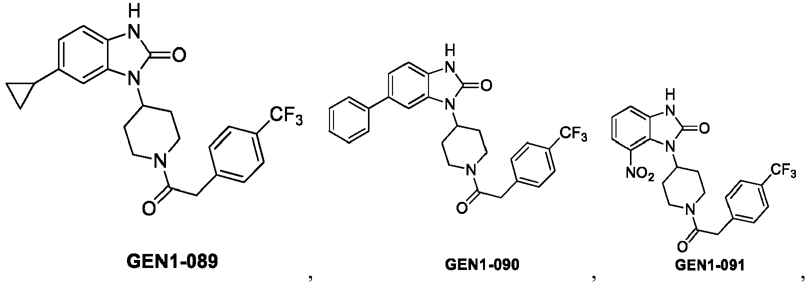

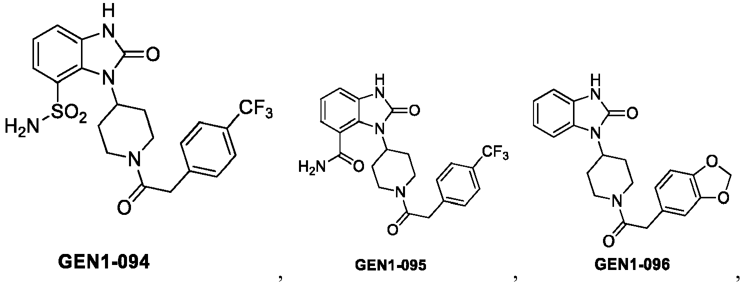

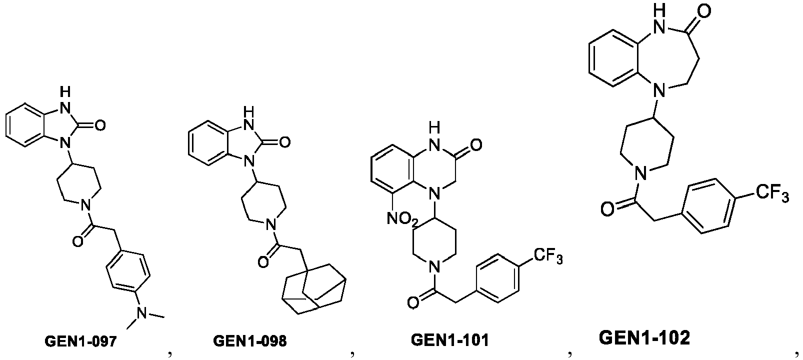

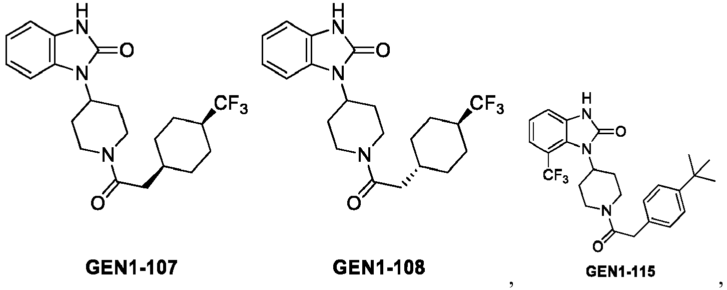

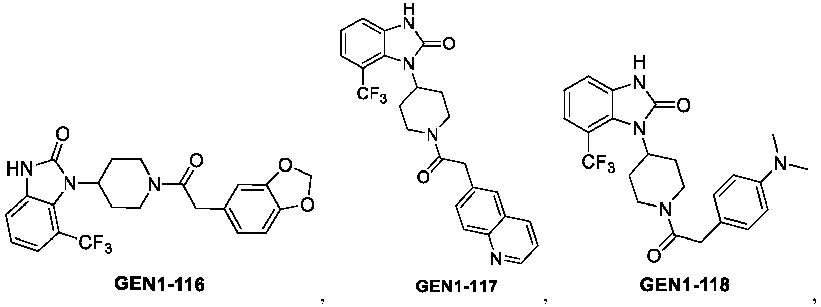

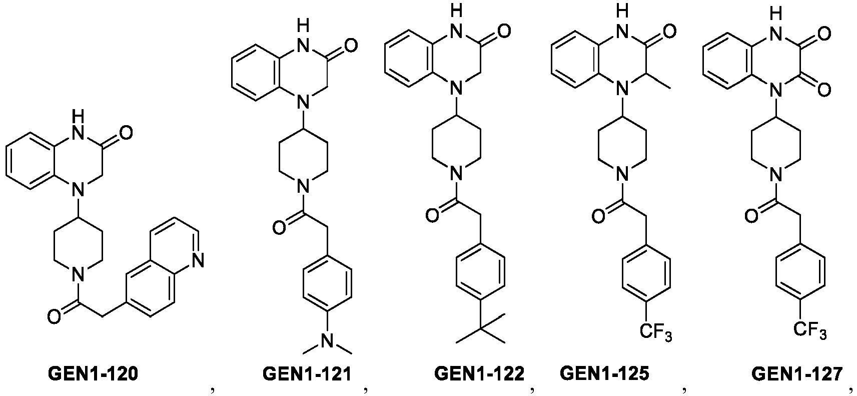

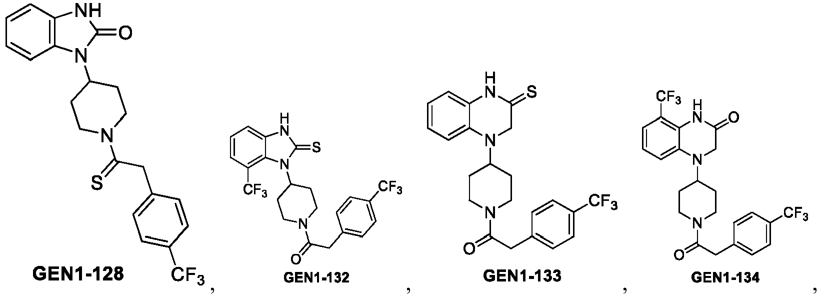

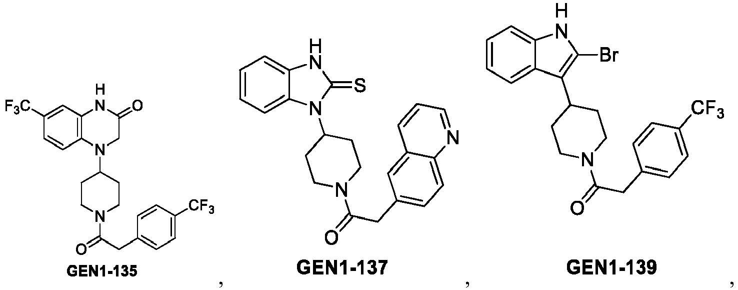

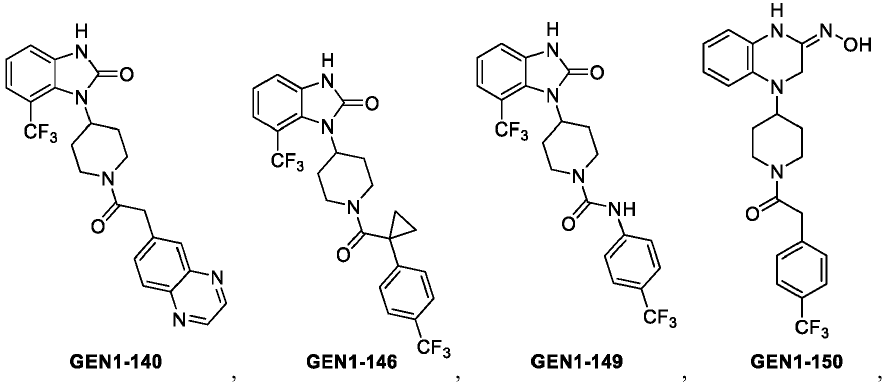

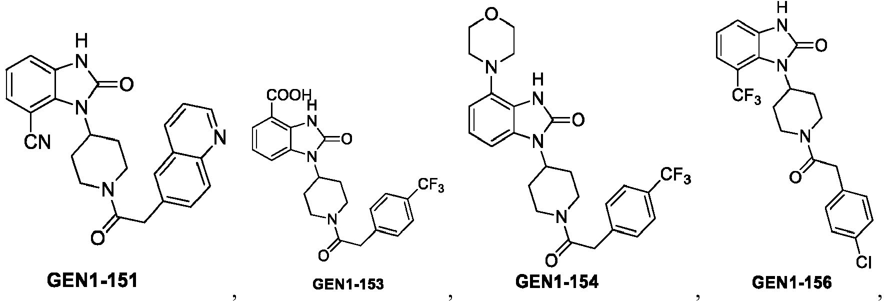

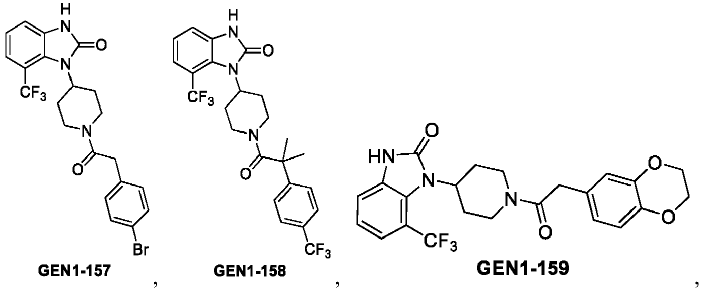

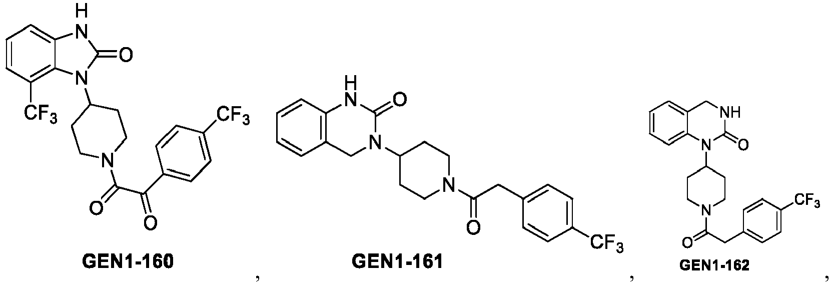

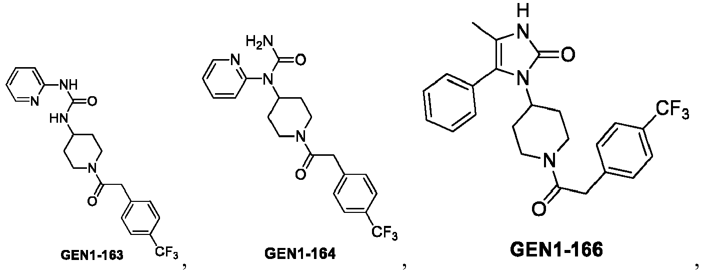

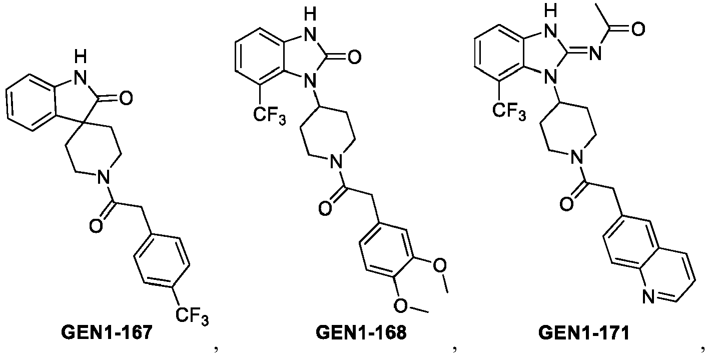

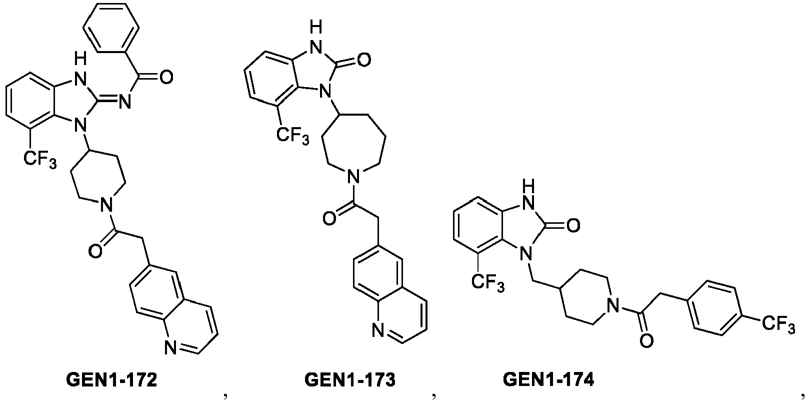

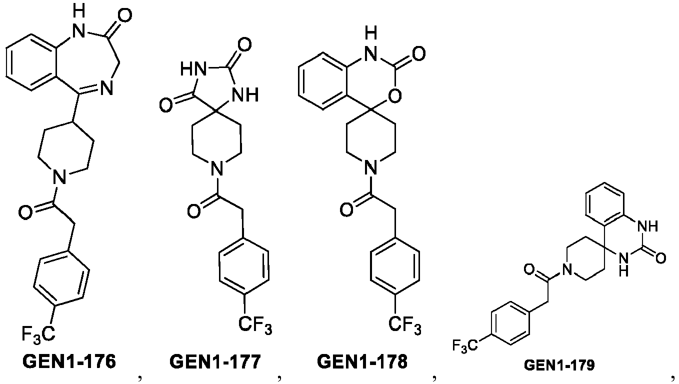

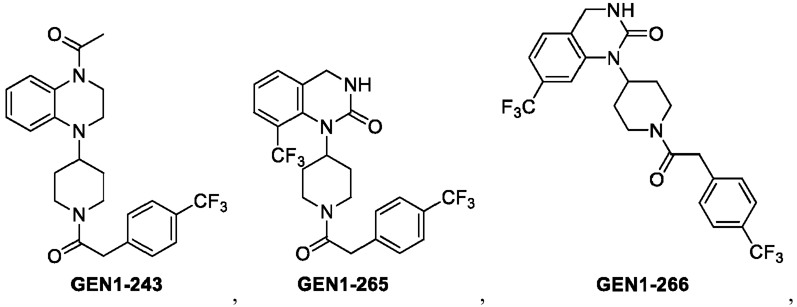

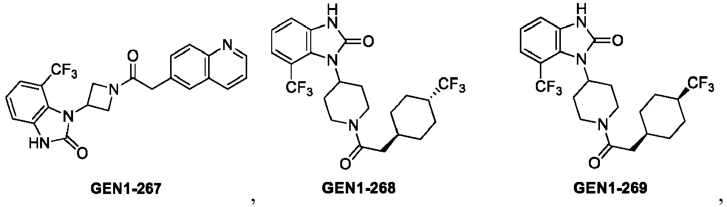

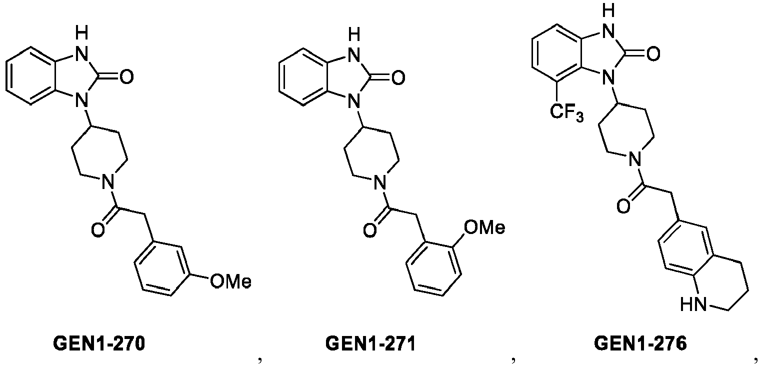

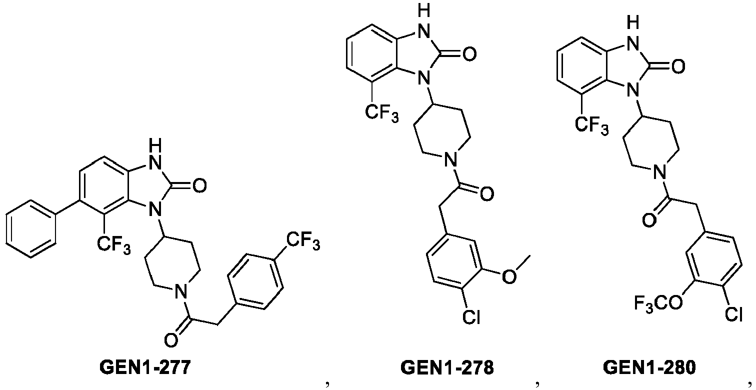

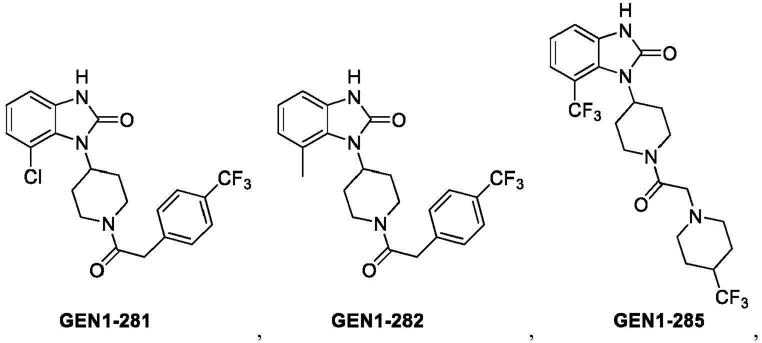

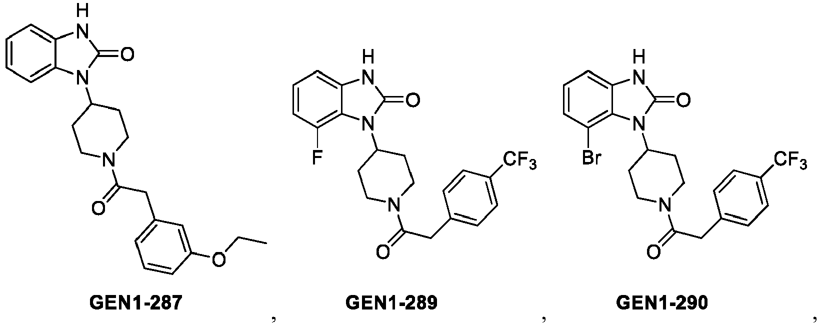

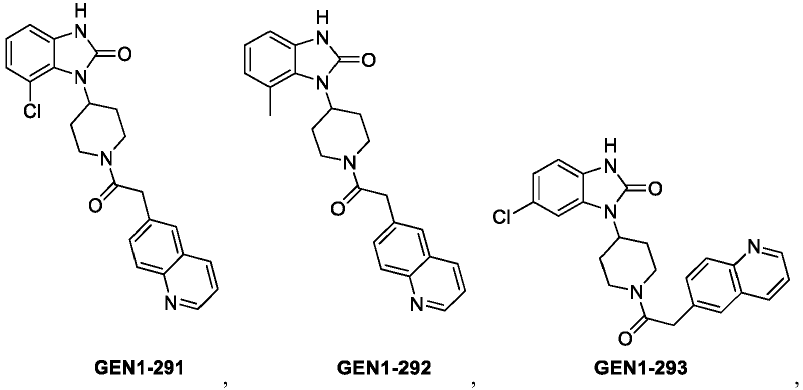

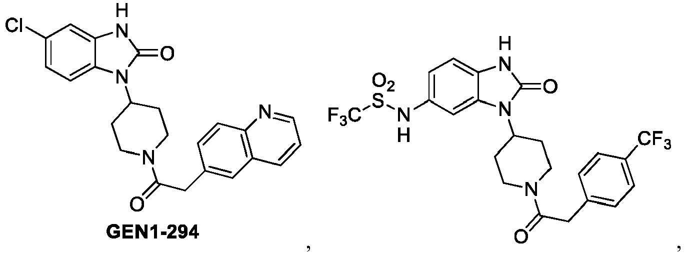

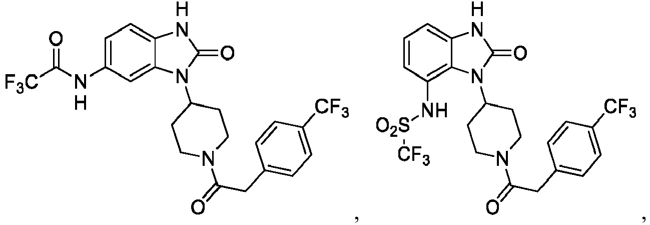

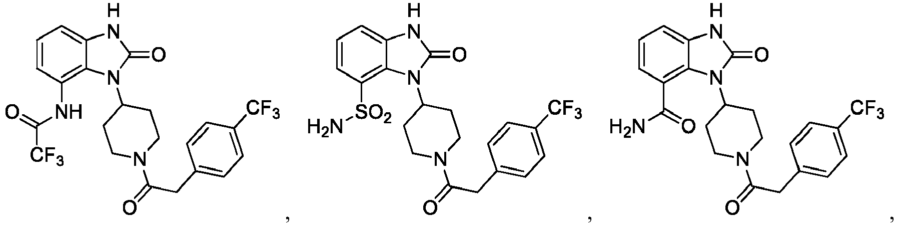

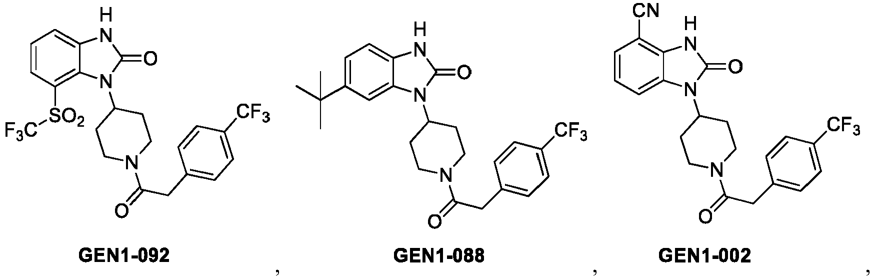

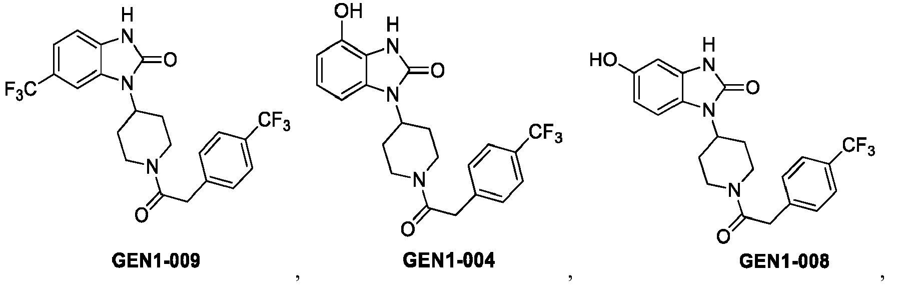

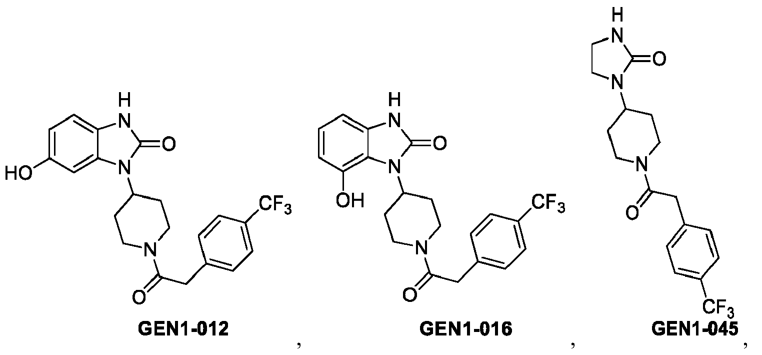

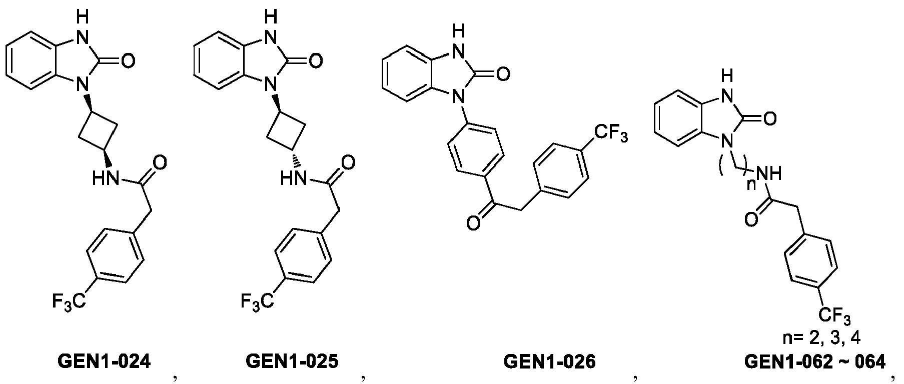

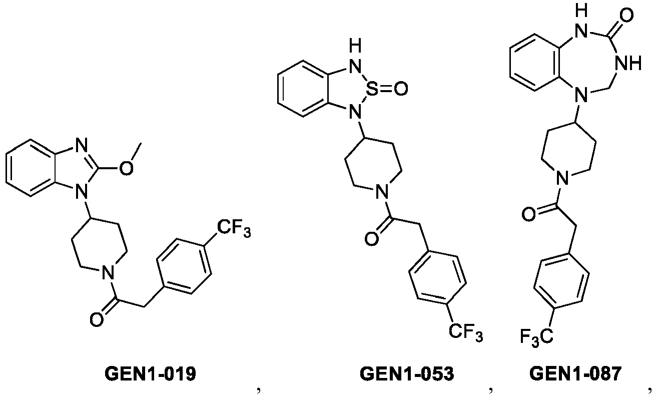

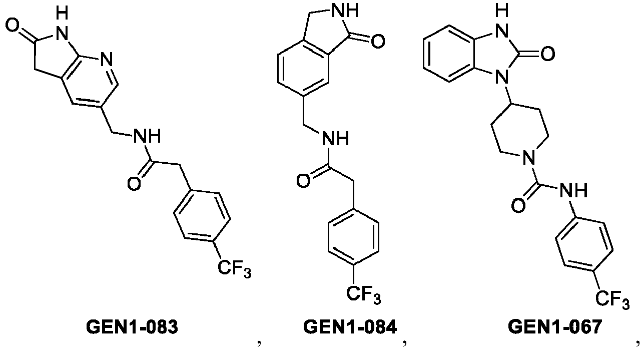

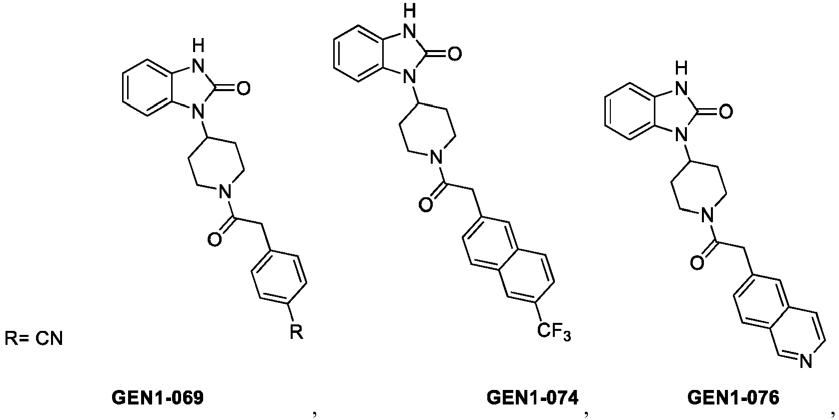

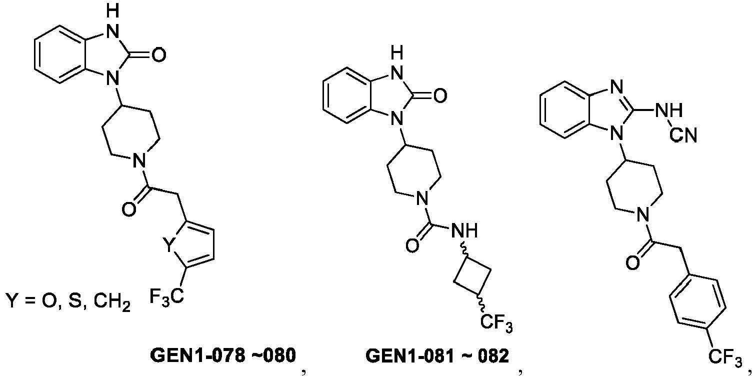

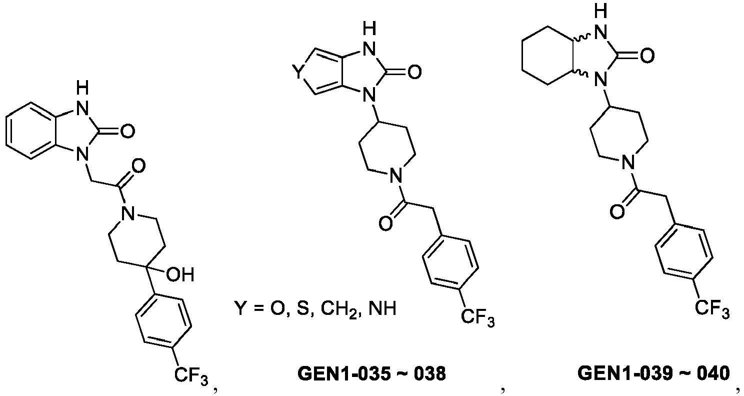

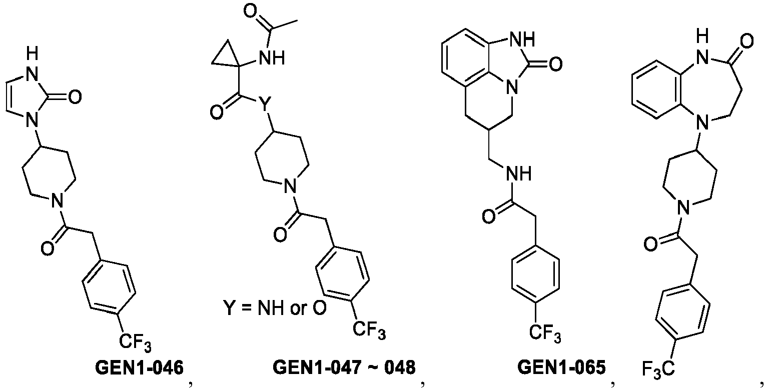

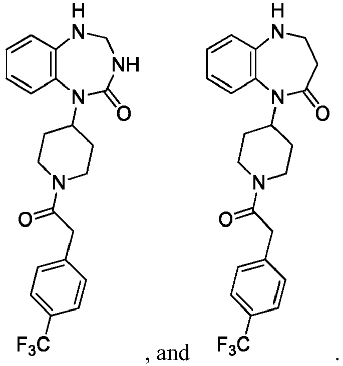

15. A pharmaceutical composition comprising a substance **X** and a pharmaceutical excipient; the substance **X** is the compound represented by formula **XI** of anyone of claims 1-14, or a pharmaceutically acceptable salt, a solvate, or a solvate of a pharmaceutically acceptable salt thereof.

16. Use of a substance **X** in the manufacture of a STAT5 inhibitor or a medicament;
the substance **X** is the compound represented by formula **XI** of anyone of claims 1-14, or a pharmaceutically acceptable salt, a solvate, or a solvate of a pharmaceutically acceptable salt thereof;
the medicament can be a medicament for the treatment and/or prevention of a disease related to STAT5, or the medicament can be a medicament for the treatment and/or prevention of rheumatoid arthritis.

17. A method of treating and/or preventing a disease related to STAT5 or rheumatoid arthritis, comprising administering to a patient a therapeutically effective amount of a substance **X;**
the substance **X** is the compound represented by formula **XI** of anyone of claims 1-14, or a pharmaceutically acceptable salt, a solvate, or a solvate of a pharmaceutically acceptable salt thereof.
